(19) Europäisches Patentamt
European Patent Office
Office européen des brevets

(11) EP 4 039 331 A1

(12) EUROPEAN PATENT APPLICATION
published in accordance with Art. 153(4) EPC

(43) Date of publication:
**10.08.2022 Bulletin 2022/32**

(21) Application number: **20871687.8**

(22) Date of filing: **29.09.2020**

(51) International Patent Classification (IPC):
***A61P 25/04*** *(2006.01)* ***A61P 43/00*** *(2006.01)*
***C07D 471/08*** *(2006.01)* ***A61P 17/04*** *(2006.01)*
***C07F 7/10*** *(2006.01)* ***C07D 519/00*** *(2006.01)*
***A61K 31/55*** *(2006.01)* ***A61K 31/695*** *(2006.01)*

(52) Cooperative Patent Classification (CPC):
**A61K 31/55; A61K 31/695; A61P 17/04; A61P 25/04; A61P 43/00; C07D 471/08; C07D 519/00; C07F 7/10;** Y02P 20/55

(86) International application number:
**PCT/JP2020/036868**

(87) International publication number:
**WO 2021/065898 (08.04.2021 Gazette 2021/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2019 JP 2019179727**

(71) Applicant: **Nippon Chemiphar Co., Ltd.**
**Tokyo 101-0032 (JP)**

(72) Inventors:
- **WATANABE Yoshikazu**
**Misato-shi, Saitama 341-0005 (JP)**
- **SAITO Daisuke**
**Misato-shi, Saitama 341-0005 (JP)**
- **HAYASHIDA Kohei**
**Misato-shi, Saitama 341-0005 (JP)**
- **YAMAMOTO Kohei**
**Misato-shi, Saitama 341-0005 (JP)**
- **NAMIKI Mayu**
**Misato-shi, Saitama 341-0005 (JP)**
- **MOGI Yuzo**
**Misato-shi, Saitama 341-0005 (JP)**
- **YATA Masahiro**
**Misato-shi, Saitama 341-0005 (JP)**

(74) Representative: **V.O.**
**P.O. Box 87930**
**2508 DH Den Haag (NL)**

(54) **AZEPAN DERIVATIVE**

(57) Provided are: an azepan derivative represented by general formula (I) and a pharmaceutically acceptable salt thereof (in the formula: $R^1$ represents a hydrogen atom, a $C_{1-6}$ alkyl group which may have a substituent, etc.; $R^2$ and $R^3$ are the same as or different from each other, and represent a hydrogen atom, a $C_{1-6}$ alkyl group which may have a substituent, etc.; $R^4$ and $R^5$ are the same as or different from each other, and represent a hydrogen atom, a $C_{1-6}$ alkyl group which may have a substituent, etc.; $R^6$ represents a hydrogen atom, a $C_{1-6}$ alkyl group which may have a substituent, etc.; $R^7$ and $R^8$ are the same as or different from each other, and represent a hydrogen atom, a $C_{1-6}$ alkyl group which may have a substituent, etc.; $R^9$ and $R^{10}$ are the same as or different from each other, and represent a hydrogen atom, a $C_{1-6}$ alkyl group which may have a substituent, etc.; $R^{11}$ represents a hydrogen atom, a $C_{1-6}$ alkyl group which may have a substituent, etc.; A and B differently represent a methyl group, a carbonyl group, etc.; and X represents a nitrogen atom or an N-oxide.).

EP 4 039 331 A1

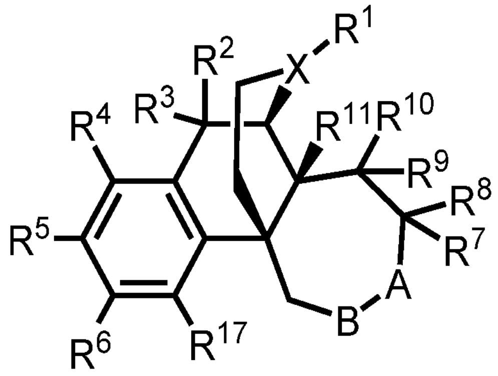

(I)

## Description

### Technical Field

The present invention relates to an azepane derivative having a κ opioid receptor agonistic effect.
The present application claims priority based on JP 2019-179727 A filed in Japan on September 30, 2019, the content of which is incorporated herein by reference.

### Background Art

Three types of opioid receptors are known: μ, δ, and κ. Morphine, which exhibits a high affinity for μ opioid receptors, has been used as an analgesic for a long time. However, μ opioid receptor agonists such as morphine are known to cause adverse events such as dependence and respiratory depression via μ opioid receptors.
Meanwhile, κ opioid receptor agonists are also known to exhibit an analgesic effect, but are not involved in such adverse events caused by morphine.
On the other hand, it is known that κ opioid receptor agonists generally exhibit a sedative effect and a drug-aversion effect. The only κ opioid receptor agonist from which drug-aversion has been separated is Nalfurafine (Patent Literature 1). However, since Nalfurafine exhibits a sedative effect in an analgesic dose, it has been approved as an antipruritic drug, but has not been approved as an analgesic. That is, there is still no κ opioid receptor-selective agonist approved as an analgesic.
Accordingly, a κ opioid receptor-selective agonist not exhibiting a sedative effect or a drug-aversion effect is expected as an excellent treating, improving or preventing drug for diseases or symptoms related to κ opioid receptors such as an analgesic.
Patent Literature 2 discloses that a compound represented by the following formula (A):

[Chemical Formula 1]

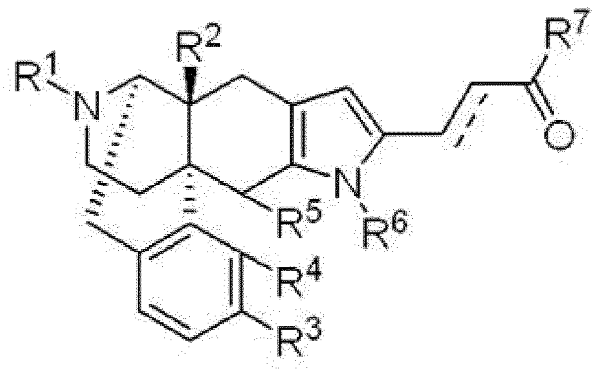

(A)

has a κ opioid receptor agonistic activity. However, the activity is not sufficient.
Furthermore, Patent Literature 3 reports a compound represented by the following formula (B):

[Chemical Formula 2]

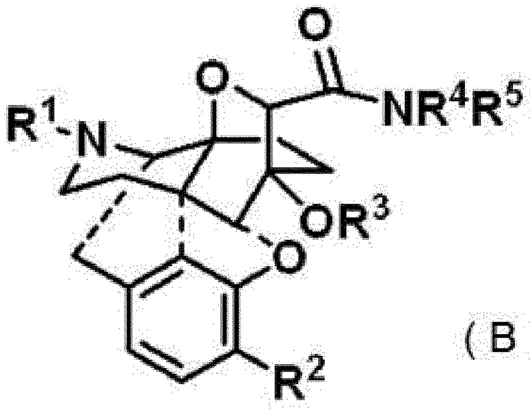

(B)

It is described that this compound has a κ opioid receptor selective binding effect and an analgesic effect. However, the analgesic activity is far from satisfactory.

In addition, Patent Literature 4 and Non Patent Literature 1 disclose that a compound (C) represented by the following formula:

[Chemical Formula 3]

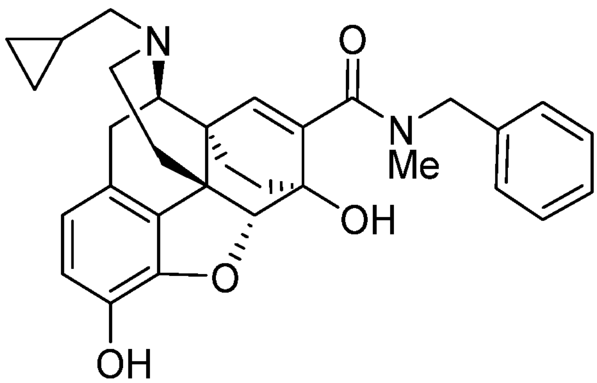

(C)

has extremely high activity. However, the compound has had insufficient *in vivo* stability. When the compound is unstable *in vivo,* it is difficult to develop the compound as a pharmaceutical product due to the fact that expected drug efficacy is not exhibited, the influence of the decomposed product is exerted on the living body, and the like. Accordingly, *in vivo* stability is an important requirement in the development of pharmaceutical products.

Among analgesics on the market, there is still no drug having high κ opioid receptor agonist activity.

Therefore, in order to aim for an analgesic, a compound is desired that exhibits selectivity and high activity for κ opioid receptors, is excellent in *in vivo* stability, and has little sedative effect and drug-aversion effect.

Citation List

Patent Literature

Patent Literature 1: WO 1993/5081 A
Patent Literature 2: JP 2008-179554 A
Patent Literature 3: JP 2525552 B2
Patent Literature 4: US 963460
Non Patent Literature

Non Patent Literature 1: Symposium Program and Abstract of 36th Annual Meeting of Japanese Narcotics

Research Conference (JNRC 2016), page 37, Japanese Narcotics Research Conference

Summary of Invention

Technical Problem

An object of the present invention is to provide a medicine effective for treatment, improvement, or prevention of various diseases and symptoms related to κ opioid receptors, in which the sedative effect and the drug-aversion effect are suppressed.

Solution to Problem

Under such circumstances, as a result of intensive studies, the present inventors have found that a specific azepane derivative exhibits potent agonist activity against κ opioid receptors and high *in vivo* stability. Furthermore, it has been revealed that the present azepane derivative exhibits a potent analgesic effect, and does not exhibit a sedative effect even at a dose higher than that of Nalfurafine (Patent Literature 1). As described above, the present inventors have found an azepane derivative that is a highly safe κ opioid receptor agonist intended for an analgesic, and completed the present invention.

[1] That is, the present invention relates to an azepane derivative represented by the following formula (I):

[Chemical Formula 4]

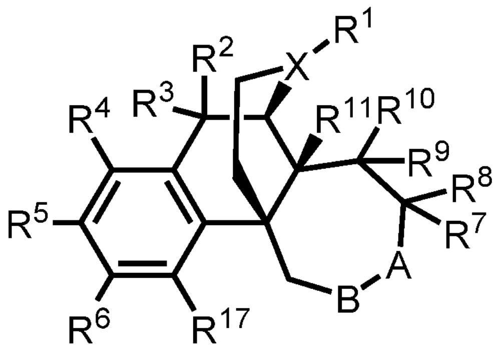

(I)

wherein $R^1$ represents a hydrogen atom, a $C_{1-6}$ alkyl group optionally having a substituent, a $C_{3-6}$ cycloalkyl group optionally having a substituent, a $C_{3-6}$ cycloalkyl $C_{1-6}$ alkyl group optionally having a substituent, a $C_{6-10}$ aryl group optionally having a substituent, a heteroaryl group optionally having a substituent, an aralkyl group optionally having a substituent, a heteroarylalkyl group optionally having a substituent, a $C_{2-6}$ alkenyl group optionally having a substituent, a $C_{2-6}$ alkynyl group optionally having a substituent, an acyl group optionally having a substituent, or an amino protecting group,

$R^2$ and $R^3$ are the same or different and each represent a hydrogen atom, a $C_{1-6}$ alkyl group optionally having a substituent, a $C_{1-6}$ alkoxy group optionally having a substituent, a halogen atom, an optionally protected hydroxy group, a carbonyl group in which $R^2$ and $R^3$ are together with each other, or a $C_{3-6}$ saturated hydrocarbon ring optionally having a substituent or a cyclic ketal optionally having a substituent in which $R^2$ and $R^3$ are bonded to each other,

$R^4$ and $R^5$ are the same or different and each represent a hydrogen atom, a $C_{1-6}$ alkyl group optionally having a substituent, a $C_{1-6}$ alkoxy group optionally having a substituent, a $C_{3-6}$ cycloalkyl group optionally having a substituent, an amino group optionally having a substituent, a protected amino group, a halogen atom, an optionally protected hydroxy group, a carboxy group, a carboxylic acid ester group, or a carbamoyl group optionally having a substituent,

$R^6$ represents a hydrogen atom, a $C_{1-6}$ alkoxy group optionally having a substituent, an amino group optionally having a substituent, a protected amino group, a halogen atom, an optionally protected hydroxy group, a tetrazolyloxy group optionally having a substituent, a cyano group, a carboxy group, a carboxylic acid ester group, a carbamoyl group optionally having a substituent, a $C_{6-10}$ aryl group optionally having a substituent, a heteroaryl group optionally having a substituent, a $C_{6-10}$ aryloxy group, or a saccharide,

$R^7$ and $R^8$ are the same or different and each represent a hydrogen atom, a $C_{1-6}$ alkyl group optionally having a substituent, a $C_{1-6}$ alkoxy group optionally having a substituent, a halogen atom, an optionally protected hydroxy group, a carbonyl group or a thiocarbonyl group in which $R^7$ and $R^8$ are together with each other, or a $C_{3-6}$ saturated hydrocarbon ring optionally having a substituent or a cyclic ketal optionally having a substituent in which $R^7$ and $R^8$ are bonded to each other,
$R^9$ and $R^{10}$ are the same or different and each represent a hydrogen atom, a $C_{1-6}$ alkyl group optionally having a substituent, a $C_{1-6}$ alkoxy group optionally having a substituent, a halogen atom, an optionally protected hydroxy group, a carbonyl group or a thiocarbonyl group in which $R^9$ and $R^{10}$ are together with each other, or a $C_{3-6}$ saturated hydrocarbon ring optionally having a substituent or a cyclic ketal optionally having a substituent in which $R^9$ and $R^{10}$ are bonded to each other,
$R^{11}$ represents a hydrogen atom, a $C_{1-6}$ alkyl group optionally having a substituent, a $C_{1-6}$ alkoxy group optionally having a substituent, an aralkyloxy group optionally having a substituent, an amino group optionally having a substituent, a protected amino group, a halogen atom, an optionally protected hydroxy group, or a cyano group,
A and B are different from each other and each represent $NR^{18}$ ($R^{18}$ represents a hydrogen atom or an amino protecting group), a methylene group, a carbonyl group, or a group represented by the following formula (II):

[Chemical Formula 5]

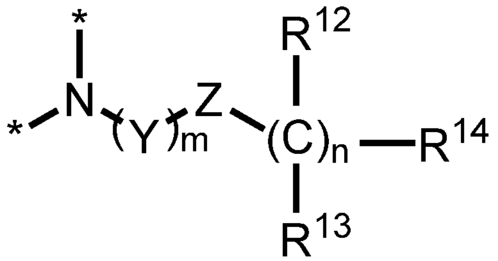

(II)

wherein $R^{12}$ and $R^{13}$ are the same or different and each represent a hydrogen atom, a halogen atom or a $C_{1-6}$ alkyl group optionally having a substituent, or a $C_{3-6}$ saturated hydrocarbon ring optionally having a substituent or a saturated heterocyclic ring optionally having a substituent in which $R^{12}$ and $R^{13}$ on the same carbon are bonded to each other, or may form a $C_{3-6}$ saturated hydrocarbon ring optionally having a substituent or a saturated heterocyclic ring optionally having a substituent in which a pair of adjacent $R^{12}$s are bonded to each other when n is 2 to 3,
$R^{14}$ represents a hydrogen atom, a $C_{1-6}$ alkyl group optionally having a substituent, a $C_{3-6}$ cycloalkyl group optionally having a substituent, a $C_{2-6}$ alkenyl group optionally having a substituent, a $C_{2-6}$ alkynyl group optionally having a substituent, a $C_{1-6}$ alkoxy group optionally having a substituent, an amino group optionally having a substituent, a protected amino group, a halogen atom, an optionally protected hydroxy group, a $C_{6-10}$ aryl group optionally having a substituent, a heteroaryl group optionally having a substituent, a saturated heterocyclic group optionally having a substituent, or a cyclic amino group optionally having a substituent, and * represents a bond, provided that one of A or B represents the formula (II),
X represents a nitrogen atom or an N-oxide,
Y represents a methylene group optionally having a substituent, a carbonyl group or a thiocarbonyl group,
Z represents $NR^{15}$, an oxygen atom, a bond, an ethenylene group optionally having a substituent, or an ethynylene group, provided that when m is 0, n does not represent 0 and Z does not represent $NR^{15}$,
$R^{15}$ represents a hydrogen atom, a $C_{1-6}$ alkyl group optionally having a substituent, or a nitrogen-containing saturated heterocyclic ring optionally having a substituent in which $R^{15}$ and $R^{14}$ are bonded to each other,
$R^{17}$ represents a hydrogen atom, a halogen atom, an optionally protected hydroxy group, an alkoxy group optionally having a substituent, or a tetrazolyloxy group optionally having a substituent,
m represents an integer of 0 to 1, and
n represents an integer of 0 to 3,
provided that when Z is a bond, m and n do not simultaneously represent 0,
and a pharmaceutically acceptable salt thereof.

[2] The present invention also relates to the azepane derivative and the pharmaceutically acceptable salt thereof according to [1], in which $R^6$ is a hydroxy group, a $C_{1-6}$ alkoxy group optionally having a substituent, or a carbamoyl group optionally having a substituent.

[3] The present invention also relates to the azepane derivative and the pharmaceutically acceptable salt thereof according to [1] or [2], in which $R^6$ is a hydroxy group.

[4] The present invention also relates to the azepane derivative and the pharmaceutically acceptable salt thereof according to [1] to [3], in which $R^1$ is a $C_{1-6}$ alkyl group optionally having a substituent or a $C_{3-6}$ cycloalkyl $C_{1-6}$ alkyl group optionally having a substituent.

[5] The present invention also relates to the azepane derivative and the pharmaceutically acceptable salt thereof according to [1] to [4], in which Z is a bond.

[6] The present invention also relates to the azepane derivative and the pharmaceutically acceptable salt thereof according to [1] to [5], in which n is 1 to 3.

[7] The present invention also relates to the azepane derivative and the pharmaceutically acceptable salt thereof according to [1] to [6], in which $R^{14}$ is a heteroaryl group optionally having a substituent.

[8] The present invention also relates to the azepane derivative and the pharmaceutically acceptable salt thereof according to [1] to [7], in which A represents a group represented by the formula (II) according to claim 1, and B represents a methylene group.

[9] The present invention also relates to the azepane derivative and the pharmaceutically acceptable salt thereof according to [1] to [8], in which $R^{11}$ is a hydroxy group.

[10] The present invention also relates to a pharmaceutical composition containing the azepane derivative and the pharmaceutically acceptable salt thereof according to any one of [1] to [9] as an active ingredient.

[11] The present invention also relates to a medicament according to [10], being a treating, improving, or preventing agent for a disease related to opioid κ receptors.

[12] The present invention also relates to the medicament according to [10] or [11], being an analgesic.

[13] The present invention also relates to the medicament according to [10] or [11], being an antipruritic drug.

Brief Description of Drawings

Fig. 1 is a diagram showing the results of a test for confirming a sedative effect by oral administration of a compound of Example 22 (500 μg/kg).
Fig. 2 is a diagram showing the results of a test for confirming a sedative effect by oral administration of a compound of Example 50 (7000 μg/kg).
Fig. 3 is a diagram showing the results of a test for confirming a sedative effect by oral administration of Nalfurafine (30, 100, 300 μg/kg).
Fig. 4 is a diagram showing the results of a test for confirming a sedative effect by subcutaneous administration of a compound of Example 77 (400 μg/kg).
Fig. 5 is a diagram showing the results of a test for confirming a sedative effect by subcutaneous administration of Nalfurafine (3, 10, 30 μg/kg).

Description of Embodiments

Next, a description is made of the present invention in more detail.

The azepane derivative represented by the above-mentioned formula (I) and a pharmaceutically acceptable salt thereof include tautomers, stereoisomers and solvates thereof.

In the $C_{1-6}$ alkyl groups optionally having a substituent represented by $R^1$ to $R^5$ and $R^7$ to $R^{15}$, the $C_{1-6}$ alkyl group includes a linear or branched alkyl group such as a methyl group, an ethyl group, a propyl group, an isopropyl group, a butyl group, an isobutyl group, a tert-butyl group, a pentyl group, or a hexyl group, preferably a methyl group, an ethyl group, or a propyl group, more preferably a methyl group.

In the $C_{1-6}$ alkyl groups optionally having a substituent, the substituent includes a halogen atom such as a

fluorine atom or a chlorine atom; a hydroxy group; an amino group optionally having a substituent such as a $C_{1-6}$ alkylamino group, a di $C_{1-6}$ alkylamino group, or an acylamino group; a protected amino group; an acyl group such as a formyl group, an acetyl group, a cyclopropylcarbonyl group, or a benzoyl group; a cyclic amino group such as an azetidinyl group, a pyrrolidinyl group, a piperazinyl group, or a morpholinyl group; a cyclic lactam group such as β-lactam, γ-lactam, or δ-lactam, a carboxy group, or a carboxylic acid ester group (the same as described in [0032]).

In the $C_{3-6}$ cycloalkyl groups optionally having a substituent represented by $R^1$, $R^4$, $R^5$, and $R^{14}$, the $C_{3-6}$ cycloalkyl group includes a cyclopropyl group, a cyclobutyl group, a cyclopentyl group, or a cyclohexyl group, preferably a cyclopropyl group.

In the $C_{3-6}$ cycloalkyl groups optionally having a substituent represented by $R^1$, $R^4$, $R^5$, and $R^{14}$, the substituent includes a $C_{1-6}$ alkyl group such as a methyl group, an ethyl group or a propyl group; a halogenated methyl group such as a fluoromethyl group, a difluoromethyl group, or a trifluoromethyl group; a halogen atom such as a fluorine atom or a chlorine atom; a hydroxy group; an amino group optionally having a substituent such as a $C_{1-6}$ alkylamino group, a di $C_{1-6}$ alkylamino group, or an acylamino group; a protected amino group; an acyl group such as a formyl group, an acetyl group, a cyclopropylcarbonyl group, or a benzoyl group; a cyclic amino group such as an azetidinyl group, a pyrrolidinyl group, a piperazinyl group, or a morpholinyl group; or a cyclic lactam group such as β-lactam, γ-lactam, or δ-lactam.

In the $C_{1-6}$ alkoxy groups optionally having a substituent represented by $R^2$ to $R^{11}$, $R^{14}$, and $R^{17}$, the $C_{1-6}$ alkoxy group includes a linear or branched alkoxy group such as a methoxy group, an ethoxy group, a propoxy group, an isopropoxy group, a butoxy group, or an isobutoxy group, preferably a methoxy group.

In the $C_{1-6}$ alkoxy groups optionally having a substituent, the substituent includes a $C_{1-6}$ alkoxy group such as a methoxy group or an ethoxy group; a phenoxy group; or a halogen atom such as a fluorine atom or a chlorine atom, preferably a fluorine atom, and examples thereof include a fluoromethoxy group, a difluoromethoxy group, a trifluoromethoxy group, or a 2,2,2-trifluoroethoxy group.

The $C_{6-10}$ aryl groups optionally having a substituent represented by $R^1$, $R^6$, and $R^{14}$, and the $C_{6-10}$ aryl group in the $C_{6-10}$ aryloxy group optionally having a substituent represented by $R^6$ include a phenyl group or a naphthyl group.

In the heteroaryl groups optionally having a substituent represented by $R^1$, $R^6$, and $R^{14}$, examples of the heteroaryl group include a 5-membered heteroaryl group such as a furanyl group, a thienyl group, a pyrrolyl group, an imidazolyl group, a pyrazolyl group, an oxazolyl group, an isoxazolyl group, a thiazolyl group, an isothiazolyl group, a triazolyl group, or a tetrazolyl group; a 6-membered heteroaryl group such as a pyridyl group, a pyridazinyl group, a pyrazinyl group, or a pyrimidyl group; or a monocyclic or bicyclic heteroaryl group containing 1 to 4 heteroatoms selected from a nitrogen atom, an oxygen atom, and a sulfur atom as a ring-constituting atom, such as a bicyclic heteroaryl group such as a quinolyl group, an isoquinolyl group, a quinazolyl group, a quinoxalyl group, an indolyl group, an indazolyl group, a benzimidazolyl group, a benzofuranyl group, a benzothienyl group, a benzoxazolyl group, a benzothiazolyl group, an imidazopyridinyl group, a pyrazolopyridinyl group, or an indazolyl group.

In addition, a tautomer may exist depending on the substituent on these heteroaryl groups. For example, when a hydroxy group is substituted on a pyridyl group, a 6-hydroxypyridin-2-yl group includes a 6-oxo-1,6-dihydropyridin-2-yl group as the tautomer thereof, and a 4-hydroxypyridin-2-yl group includes a 4-oxo-1,4-dihydropyridin-2-yl group as the tautomer thereof.

Preferable $R^1$ includes a 6-membered heteroaryl group such as a pyridyl group, a pyridazinyl group, a pyrazinyl group, or a pyrimidyl group.

Preferable $R^{14}$ includes a furanyl group, a thienyl group, a pyrrolyl group, an imidazolyl group, a pyrazolyl group, a thiazolyl group, a triazole group, a pyridyl group, a pyridazinyl group, a pyrazinyl group, a pyrimidyl group, a quinolyl group, an isoquinolyl group, an indolyl group, an indazolyl group, a benzoxazolyl group, a benzothiazolyl group, an imidazopyridinyl group, a pyrazolopyridinyl group, or an indazole group, more preferably a furan-2-yl group, a thiazol-2-yl group, a thiazol-4-yl group, a 1H-imidazol-4-yl group, a 1H-pyrazol-3-yl group, a 1H-pyrazol-1-yl group, a 2H-1,2,3-triazol-2-yl group, a pyridin-2-yl group, a pyrimidin-2-yl group, a pyrazin-2-yl group, a 1H-indazol-3-yl group, an imidazo[1,2-a]pyridin-2-yl group, a pyrazolo[1,5-a]1H-indazol-1-yl group, a quinolin-2-yl group, an isoquinolin-3-yl group, an indolin-1-yl group, or a benzo[d]thiazol-2-yl group.

The $C_{6-10}$ aryl groups optionally having a substituent represented by $R^1$, $R^6$, and $R^{14}$, and the heteroaryl groups optionally having a substituent represented by $R^1$, $R^6$, and $R^{14}$ may have 1 to 3 substituents on the ring, and the substituents include a linear or branched $C_{1-6}$ alkyl group such as a methyl group, an ethyl group, or a propyl group; a linear or branched halogenated alkyl group such as a fluoromethyl group, a chloromethyl group, a difluoromethyl group, a dichloromethyl group, or a trifluoromethyl group; a hydroxy alkyl group such as a hydroxymethyl group; a hydroxyethyl group, or a 1-hydroxypropyl group; a $C_{3-6}$ cycloalkyl group such as a cyclopropyl group, a cyclobutyl group, or a cyclopentyl group; a linear or branched $C_{1-6}$ alkoxy group such as a methoxy group, an ethoxy group, a propoxy group, an isopropoxy group, or a butoxy group; a linear or branched halogenated $C_{1-6}$ alkoxy group such as a trifluoromethoxy group or a 2,2,2-trifluoroethoxy group; a halogen atom such as a fluorine atom or a chlorine atom; a hydroxy group; a nitro group; a cyano group; an amino group optionally having a substituent such as a $C_{1-6}$ alkylamino group, a di $C_{1-6}$ alkylamino group, or an acylamino group; a protected amino group; an acyl group such as a formyl group, an acetyl group, a

cyclopropylcarbonyl group, or a benzoyl group; a cyclic amino group such as an azetidinyl group, a pyrrolidinyl group, a piperazinyl group, or a morpholinyl group; or a cyclic lactam group such as β-lactam, γ-lactam, or δ-lactam.

In the $C_{3-6}$ cycloalkyl $C_{1-6}$ alkyl group optionally having a substituent represented by $R^1$, the $C_{3-6}$ cycloalkyl $C_{1-6}$ alkyl group includes a cyclopropylmethyl group, a cyclopropylethyl group, a cyclopropylpropyl group, a cyclobutylmethyl group, a cyclobutylethyl group, a cyclopentylmethyl group, a cyclopentylethyl group, a cyclopentylpropyl group, a cyclohexylmethyl group, a cyclohexylethyl group, or a cyclohexylpropyl group, preferably a cyclopropylmethyl group, a cyclopropylethyl group, a cyclobutylmethyl group, a cyclobutylethyl group, a cyclopentylmethyl group, or a cyclopentylethyl group, more preferably a cyclopropylmethyl group, a cyclobutylmethyl group, or a cyclopentylmethyl group, most preferably a cyclopropylmethyl group.

In the $C_{3-6}$ cycloalkyl $C_{1-6}$ alkyl group optionally having a substituent, the substituent includes a fluorine atom, a halogen atom such as a chlorine atom, or a hydroxy group.

The aralkyl group optionally having a substituent represented by $R^1$, and the aralkyl in the aralkyloxy group optionally having a substituent represented by $R^{11}$ include one in which the carbon number of the aryl moiety is $C_{6-10}$ and the carbon number of the alkylene moiety is $C_{1-5}$, and examples thereof include a benzyl group, a phenylethyl group, or a 1-naphthylmethyl group, preferably a benzyl group.

In the heteroarylalkyl group optionally having a substituent represented by $R^1$, the heteroaryl moiety includes a heteroaryl containing 1 to 4 heteroatoms selected from a nitrogen atom, an oxygen atom, and a sulfur atom as a ring-constituting atom, and the alkyl moiety includes a $C_{1-6}$ alkyl group such as a methyl group, an ethyl group, or a propyl group, and examples thereof include a monocyclic heteroarylalkyl group such as a (pyridin-2-yl)methyl group, a (pyridin-3-yl)methyl group, a (pyridin-4-yl)methyl group, a 2-(pyridin-2-yl)ethyl group, a (furan-2-yl)methyl group, a (furan-3-yl)methyl group, an (imidazol-2-yl)methyl group, an (imidazol-4-yl)methyl group, an (imidazol-5-yl)methyl group, a (thiazol-2-yl)methyl group, a (thiazol-4-yl)methyl group, a (thiazol-5-yl)methyl group, a (thiophen-2-yl)methyl group, or a 2-(thiophen-2-yl)ethyl group, or a bicyclic heteroarylalkyl group such as a (quinolin-3-yl)methyl group or a (indol-3-yl)methyl group.

The aryl in the aralkyloxy group optionally having a substituent represented by $R^{11}$, and the aryl and heteroaryl in the aralkyl group optionally having a substituent and the heteroarylalkyl group optionally having a substituent represented by $R^1$ optionally have a substituent, and examples of the substituent include the same substituents as those in the $C_{6-10}$ aryl group optionally having a substituent described in paragraph [0020].

In the $C_{2-6}$ alkenyl groups optionally having a substituent represented by $R^1$ and $R^{14}$, the $C_{2-6}$ alkenyl group includes a $C_{2-6}$ linear or branched alkenyl group, and examples thereof include an alkenyl group such as an allyl group, a vinyl group, a 1-propenyl group, a 2-butenyl group, a 3-butenyl group, a 2-pentenyl group, a 3-pentenyl group, a 4-pentenyl group, a 2-hexenyl group, a 3-hexenyl group, a 4-hexenyl group, or a 5-hexenyl group.

In the $C_{2-6}$ alkynyl groups optionally having a substituent represented by $R^1$ and $R^{14}$, the $C_{2-6}$ alkynyl group represents a $C_{2-6}$ linear or branched alkynyl group, and examples thereof include an ethynyl group, a propynyl group, or a butynyl group.

A group that may be substituted into the alkenyl group and the alkynyl group includes a $C_{1-6}$ alkoxycarbonyl group such as a methoxycarbonyl group, an ethoxycarbonyl group, or a propoxycarbonyl group, or an aralkyl group such as a benzyl group, a 2-phenylethyl group, a 3-phenylpropyl group, or a 4-phenylbutyl group; a $C_{1-6}$ alkoxy group such as a methoxy group, an ethoxy group, a propoxy group, or a butoxy group; an aralkyloxy group such as a benzyloxy group or a 2-phenylethyloxy group; an amino group optionally substituted with a $C_{1-6}$ linear or branched alkyl group; a halogen atom such as a fluorine atom or a chlorine atom; a carboxy group or a hydroxy group.

The acyl group optionally having a substituent represented by $R^1$ includes a $C_{1-6}$ alkanoyl group such as a formyl group, an acetyl group, a propionyl group, a butanoyl group, a pentanoyl group, or a hexanoyl group; a $C_{4-7}$ cycloalkanoyl group such as a cyclopropylcarbonyl group, a cyclobutylcarbonyl group, or a cyclopentylcarbonyl group; an aroyl group such as a benzoyl group or a naphthoyl group; a 5 to 6 membered heteroaroyl group such as a furoyl group, a thiophene carbonyl group, a nicotinyl group, or an isonicotinoyl group.

In addition, the substituent in the $C_{1-6}$ alkanoyl group and the $C_{4-7}$ cycloalkanoyl group includes the same substituents as those described in paragraph [0015], and the substituent in the aroyl group and the heteroaroyl group includes the same substituents as those described in paragraph [0020].

The substituent in the carbamoyl groups optionally having a substituent represented by $R^4$ to $R^6$, the substituent in the amino groups optionally having a substituent represented by $R^4$ to $R^6$, $R^{11}$, and $R^{14}$, and the substituent in the tetrazolyloxy groups optionally having a substituent represented by $R^6$ and $R^{17}$ include a $C_{1-6}$ alkyl group optionally having a substituent such as a methyl group, an ethyl group, a propyl group, or an isopropyl group, or an aryl group such as a phenyl group. In the $C_{1-6}$ alkyl group optionally having a substituent, the substituent includes substituents described in paragraph [0015], and 1 or 2 of these substituents may be contained.

In the amino-protecting groups represented by $R^1$ and $R^{18}$ and the protected amino groups represented by $R^4$ to $R^6$, $R^{11}$, and $R^{14}$, the protecting group includes a carbamate-based protecting group such as a methoxycarbonyl group, an ethoxycarbonyl group, a tert-butoxycarbonyl group, a tert-amyloxycarbonyl group, a 2,2,2-trichloroethoxycarbonyl group, a benzyloxycarbonyl group, a p-chlorobenzyloxycarbonyl group, a p-methoxybenzylcarbonyl group, a p-nitrobenzyloxycarbonyl group, a p-methoxyphenylazobenzyloxycarbonyl group, a 3,5-dimethoxybenzyloxycarbonyl group, a 3,4,5-trimethoxybenzyloxycarbonyl group, a p-biphenylisopropyloxycarbonyl group, a diisopropylmethyloxycarbonyl group, a 2-(trimethylsilyl)ethoxycarbonyl group, or a 9-fluorenylmethyloxycarbonyl group; a sulfonamide-based protecting group such as a p-toluenesulfonyl group or a 2-nitrobenzenesulfonyl group; an imide-based protecting group such as a phthaloyl group; a $C_{7-19}$ aralkyl group such as a benzyl group, a phenylethyl group, a phenylpropyl group, a trityl group, or a naphthylmethyl group.

The halogen atoms represented by $R^2$ to $R^{14}$ and $R^{17}$ include a fluorine atom, a chlorine atom, a bromine atom, or an iodine atom, preferably a fluorine atom or a chlorine atom, more preferably a fluorine atom.

In the carboxylic acid ester groups represented by $R^4$ to $R^6$, the ester-forming group includes a linear or branched $C_{1-6}$ alkyl group such as a methyl group, an ethyl group, a propyl group, a 2-propyl group, a butyl group, an isobutyl group, a tert-butyl group, a pentyl group, or a hexyl group; a $C_{2-6}$ alkenyl group such as a vinyl group, an allyl group, a 1-propenyl group, a butenyl group, a pentenyl group, or a hexenyl group; an aralkyl group such as a benzyl group; an aryl group such as a phenyl group or a naphthyl group, or a $C_{1-6}$ alkanoyloxy $C_{1-4}$ lower alkyl group such as an acetoxymethyl group or a pivaloyloxymethyl group.

In the cyclic amino group optionally having a substituent represented by $R^{14}$, the cyclic amino group includes an azetidinyl group, a pyrrolidinyl group, an azepanyl group, a piperidinyl group, a piperazinyl group, a morphonyl group, a thiomorpholinyl group, an oxazabicyclooctyl group, an azasilinyl group, an indolinyl group or an isoindolyl group, preferably a 1-pyrrolidinyl group, a 1-piperidinyl group, a 2-oxa-5-azabicyclo[2.2.2]octan-5-yl group, a 3-oxa-8-azabicyclo[3.2.1]octan-8-yl group, an azasilinan-1-yl group, or a 1-indolinyl group, more preferably a 1-pyrrolidinyl group, a 1-piperidinyl group, an azasilinan-1-yl group, or a 1-indolinyl group.

The substituent includes a $C_{1-6}$ alkyl group optionally having a substituent such as a methyl group or an ethyl group; a $C_{1-6}$ alkoxy group optionally having a substituent such as a methoxy group, an ethoxy group, or a propoxy group; a halogen atom such as a fluorine atom or a chlorine atom; or a hydroxy group.

In the $C_{1-6}$ alkyl group optionally having a substituent, the substituent includes substituents described in paragraph [0015], and in the $C_{1-6}$ alkoxy group optionally having a substituent, the substituent includes substituents described in paragraph [0017].

In the $C_{3-6}$ saturated hydrocarbon ring optionally having a substituent formed by bonding of $R^2$ and $R^3$ or $R^7$ and $R^8$, the $C_{3-6}$ saturated hydrocarbon ring optionally having a substituent formed by bonding of $R^9$ and $R^{10}$, and the $C_{3-6}$ saturated hydrocarbon ring optionally having a substituent formed by bonding of $R^{12}$ and $R^{13}$ on the same carbon or bonding of a pair of adjacent $R^{12}$s when n is 2 to 3, the $C_{3-6}$ saturated hydrocarbon rings include a cyclopropane ring, a cyclobutane ring, a cyclopentane ring, or a cyclohexane ring.

The saturated heterocyclic ring optionally having a substituent formed by bonding of $R^{12}$ and $R^{13}$ on the same carbon or bonding of a pair of adjacent $R^{12}$s when n is 2 to 3, and the saturated heterocyclic ring optionally having a substituent represented by $R^{14}$ include a 3 to 6 membered saturated heterocyclic ring, and examples thereof include a cyclic amine such as aziridine, azetidine, pyrrolidine, piperidine, piperazine, morpholine, or thiomorpholine, a cyclic ether such as epoxide, oxetane, tetrahydrofuran, tetrahydropyran, or dioxane, or a cyclic thioether such as thietane, thiolane, or thiane.

When having a nitrogen atom as a constituting atom of the ring, the saturated heterocyclic ring optionally further has a linear or branched $C_{1-6}$ alkyl group, a substituent such as an acyl group, or an amino-protecting group on the nitrogen. These substituents and the like represent those described in paragraphs [0015], [0028], and [0030].

The nitrogen-containing saturated heterocyclic ring optionally having a substituent formed by bonding of $R^{14}$ and $R^{15}$ includes a 3 to 6 membered nitrogen-containing saturated heterocyclic ring, and examples thereof include a cyclic amino group such as aziridine, azetidine, pyrrolidine, piperidine, piperazine, morpholine, or thiomorpholine.

These nitrogen-containing saturated heterocyclic rings are optionally further condensed with a saturated hydrocarbon ring, a saturated heterocyclic ring, an unsaturated hydrocarbon ring, or an unsaturated heterocyclic ring, and examples thereof include decahydroquinoline, decahydroisoquinoline, indoline, isoindoline, 1,2,3,4-tetrahydroquinoline, 1,2,3,4-tetrahydroisoquinoline, benzomorpholine, or benzothiomorpholine.

When having a nitrogen atom as a constituting atom of the ring in addition to the nitrogen atom to which $R^{15}$ is bonded, the nitrogen-containing heterocyclic ring optionally further has a linear or branched $C_{1-6}$ alkyl group and a substituent such as an acyl group, or an amino-protecting group on the nitrogen atom. These substituents and the like represent those described in paragraphs [0015], [0028], and [0030].

In the $C_{3-6}$ saturated hydrocarbon ring optionally having a substituent formed by bonding of $R^{12}$ and $R^{13}$ on the same carbon, the saturated heterocyclic ring optionally having a substituent formed by bonding of $R^{12}$ and $R^{13}$ on the same carbon, the $C_{3-6}$ saturated hydrocarbon ring optionally having a substituent or the saturated heterocyclic ring optionally having a substituent formed by bonding of a pair of adjacent $R^{12}$s when n is 2 to 3, and the nitrogen-containing saturated heterocyclic ring optionally having a substituent formed by bonding of $R^{14}$ and $R^{15}$, the substituent includes the same substituents as those in the cyclic amino group described in paragraph [0033].

The cyclic ketals optionally having a substituent formed by bonding of $R^2$ and $R^3$, $R^7$ and $R^8$, and $R^9$ and $R^{10}$ include dioxolane or dioxane. The substituent includes a $C_{1-6}$ alkyl group such as a methyl group, an ethyl group, or a propyl group.

In the optionally protected hydroxy groups represented by $R^2$ to $R^{11}$, $R^{14}$, and $R^{17}$, the hydroxy-protecting group includes an acetal-based protecting group such as an aralkyl group optionally having a substituent such as a benzyl group, a 4-methoxybenzyl group, or a trityl group, an alkanoyl group such as an acetyl group, a trimethylsilyl group such as a methoxymethyl group, a 2-tetrahydropyranyl group, or an ethoxyethyl group, a silyl group having a substituent such as tert-butyldimethylsilyl, or a sulfonyl-based protecting group such as a methanesulfonyl group, a p-toluenesulfonyl group, or a trifluoromethanesulfonyl group.

The saccharide represented by $R^6$ includes glucuronic acid.

X represents a nitrogen atom or an N-oxide, preferably a nitrogen atom.

Y represents a methylene group optionally having a substituent, a carbonyl group, or a thiocarbonyl group, preferably a methylene group optionally having a substituent or a carbonyl group, particularly preferably a carbonyl group.

The substituent in the methylene group optionally having a substituent includes a $C_{1-6}$ alkyl group, an optionally protected hydroxy group (the protecting group includes protecting groups in paragraph [0039]), a halogen atom (such as a fluorine atom), or a $C_{1-6}$ alkyl group substituted with a halogen atom (such as a fluoromethyl group, a difluoromethyl group, a trifluoromethyl group, a chloromethyl group, or a dichloromethyl group).

Z represents $NR^{15}$, an oxygen atom, a bond, an ethenylene group, or an ethynylene group, preferably $NR^{15}$, an oxygen atom, a bond, or an ethenylene group optionally having a substituent.

In the ethenylene group optionally having a substituent, the substituent includes substituents described in paragraph [0027].

m represents an integer of 0 to 1, preferably 1. However, when n is 2, m is preferably 0.

n represents an integer of 0 to 3, preferably 1 or 2.

A preferable aspect of the compound (I) used in the present invention includes a case where $R^1$ is a $C_{1-6}$ alkyl group optionally having a substituent or a $C_{3-6}$ cycloalkyl $C_{1-6}$ alkyl group optionally having a substituent, $R^2$ and $R^3$ are the same or different and are each a hydrogen atom or a $C_{1-6}$ alkyl group optionally having a substituent, a $C_{1-6}$ alkoxy group optionally having a substituent, a halogen atom, or a hydroxy group, $R^4$ and $R^5$ are the same or different and are each a hydrogen atom, a $C_{1-6}$ alkyl group optionally having a substituent, a $C_{1-6}$ alkoxy group optionally having a substituent, an amino group optionally having a substituent, a halogen atom, or a hydroxy group, $R^6$ is a hydroxy group, a $C_{1-6}$ alkoxy group optionally having a substituent, or a carbamoyl group optionally having a substituent, $R^7$ and $R^8$ are the same or different and are each a hydrogen atom, a $C_{1-6}$ alkyl group optionally having a substituent, a $C_{1-6}$ alkoxy group optionally having a substituent, a halogen atom, or a hydroxy group, $R^9$ and $R^{10}$ are the same or different and are each a hydrogen atom, a $C_{1-6}$ alkyl group optionally having a substituent, a $C_{1-6}$ alkoxy group optionally having a substituent, a halogen atom, or a hydroxy group, $R^{11}$ is a hydrogen atom or a hydroxy group, $R^{12}$ and $R^{13}$ are the same or different and are each a hydrogen atom or a $C_{1-6}$ alkyl group optionally having a substituent, $R^{14}$ is a $C_{6-10}$ aryl group optionally having a substituent, a heteroaryl group optionally having a substituent, or a cyclic amino group optionally having a substituent, X is a nitrogen atom or an N-oxide, Y is a methylene group optionally having a substituent or a carbonyl group, Z is $NR^{15}$, an oxygen atom, a bond, an ethenylene group optionally having a substituent, or an ethynylene group, $R^{15}$ is a hydrogen atom or a $C_{1-6}$ alkyl group optionally having a substituent, $R^{17}$ is a hydrogen atom, a halogen atom, an optionally protected hydroxy group, or an alkoxy group optionally having a substituent, m is an integer of 0 or 1, and n is an integer of 0 to 3.

A preferable aspect of the compound (I) used in the present invention includes a case where $R^1$ is a $C_{1-6}$ alkyl group optionally having a substituent or a $C_{3-6}$ cycloalkyl $C_{1-6}$ alkyl group optionally having a substituent, $R^2$ and $R^3$ are the same or different and are each a hydrogen atom or a $C_{1-6}$ alkyl group optionally having a substituent, $R^4$ and $R^5$ are the same or different and are each a hydrogen atom or a $C_{1-6}$ alkyl group optionally having a substituent, $R^6$ is a hydroxy group, a $C_{1-6}$ alkoxy group optionally having a substituent, or a carbamoyl group optionally having a substituent, $R^7$ and $R^8$ are the same or different and are each a hydrogen atom, a $C_{1-6}$ alkyl group optionally having a substituent, or a $C_{1-6}$ alkoxy group optionally having a substituent, $R^9$ and $R^{10}$ are the same or different and are each a hydrogen atom or a $C_{1-6}$ alkyl group optionally having a substituent, $R^{11}$ is a hydrogen atom or a hydroxy group, $R^{12}$ and $R^{13}$ are the same or different and are each a hydrogen atom or a $C_{1-6}$ alkyl group optionally having a substituent, $R^{14}$ is a $C_{6-10}$ aryl group optionally having a substituent, a heteroaryl group optionally having a substituent, or a cyclic amino group optionally having a substituent, X is a nitrogen atom or an N-oxide, Y is a methylene group optionally having a substituent or a carbonyl group, Z is $NR^{15}$, an oxygen atom, a bond, or an ethenylene group optionally having a substituent, $R^{15}$ is a hydrogen atom or a $C_{1-6}$ alkyl group optionally having a substituent, $R^{17}$ is a hydrogen atom, an optionally protected hydroxy group, or an alkoxy group optionally having a substituent, m is an integer of 0 or 1, and n is an integer of 0 to 3.

Furthermore, a preferable aspect of the compound (I) used in the present invention includes a case where $R^1$ is a $C_{1-6}$ alkyl group optionally having a substituent or a $C_{3-6}$ cycloalkyl $C_{1-6}$ alkyl group optionally having a substituent, $R^2$ and $R^3$ are the same or different and are each a hydrogen atom or a $C_{1-6}$ alkyl group optionally having a substituent,

$R^4$ and $R^5$ are a hydrogen atom or a $C_{1-6}$ alkyl group optionally having a substituent, $R^6$ is a hydroxy group, $R^7$ and $R^8$ are the same or different and are each a hydrogen atom or a $C_{1-6}$ alkyl group optionally having a substituent, $R^9$ and $R^{10}$ are the same or different and are each a hydrogen atom or a $C_{1-6}$ alkyl group optionally having a substituent, $R^{11}$ is a hydrogen atom or a hydroxy group, $R^{12}$ and $R^{13}$ are the same or different and are each a hydrogen atom or a $C_{1-6}$ alkyl group optionally having a substituent, $R^{14}$ is a heteroaryl group optionally having a substituent, X is a nitrogen atom, Y is a methylene group optionally having a substituent or a carbonyl group, Z is a bond, $R^{17}$ is a hydrogen atom, m is 0, and n is 1 to 2.

In the azepane derivative represented by the formula (I) and the pharmaceutically acceptable salt thereof, the pharmaceutically acceptable salt preferably includes an acid addition salt, and examples of the acid addition salt include (i) a salt with a mineral acid such as hydrochloric acid, sulfuric acid, or phosphoric acid, (ii) a salt with an organic carboxylic acid such as formic acid, acetic acid, citric acid, trichloroacetic acid, trifluoroacetic acid, fumaric acid, maleic acid, or tartaric acid, or (iii) a salt with a sulfonic acid such as methanesulfonic acid, benzenesulfonic acid, p-toluenesulfonic acid, mesitylenesulfonic acid, or naphthalenesulfonic acid.

The azepane derivative represented by the formula (I) and the pharmaceutically acceptable salt thereof include a tautomer, or a stereoisomer such as a cis/trans isomer, a racemate, or an optical active material.

The azepane derivative represented by the formula (I) and the pharmaceutically acceptable salt thereof can also be present as a hydrate or a solvate. Accordingly, the compound of the present invention includes all crystal types and hydrates or solvates thereof.

Hereinafter, a reference is made of a method for producing the azepane derivative represented by the formula (I) and the pharmaceutically acceptable salt thereof.

(i) A production method in a case where in the formula (I), X represents a nitrogen atom, one of A or B represents NH, and the other represents $CH_2$.

(Method A) The compounds (f) and (g) of the present invention can be obtained by the method described below using a compound (a) as the starting material.

[Chemical Formula 6]

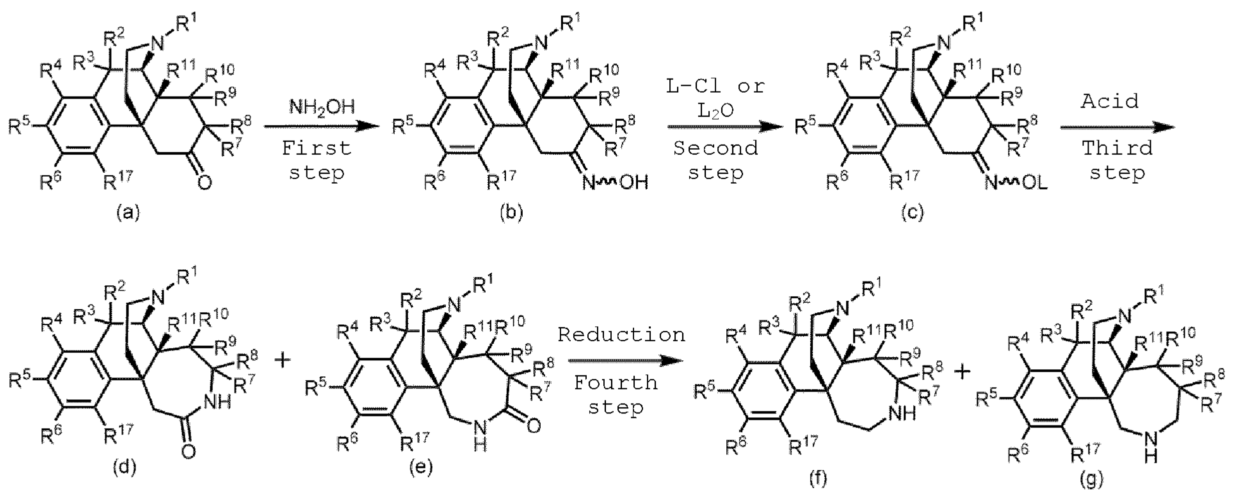


(a) (b) (c) (d) (e) (f) (g)

(wherein L represents a methanesulfonyl group, a p-toluenesulfonyl group, a trifluoromethanesulfonyl group, a 2-nitrobenzenesulfonyl group, or the like, and $R^1$ to $R^{11}$ and $R^{17}$ represent the same as those described above)

(First step)

A compound (b) can be synthesized by reacting the raw material (a) with hydroxylamine hydrochloride, hydroxylamine sulfate, or the like in an aromatic hydrocarbon such as benzene, toluene, or xylene; an ether such as diethyl ether, tetrahydrofuran, dioxane, monoglyme, or diglyme; an alcohol such as methanol or ethanol; a halogenated hydrocarbon such as dichloromethane, chloroform, or carbon tetrachloride; an aliphatic hydrocarbon such as pentane, hexane, heptane, or ligroin; a protic solvent such as water or acetic acid; an aprotic polar solvent such as acetonitrile, N,N-dimethylformamide, or dimethylsulfoxide, or in a mixed solvent thereof, in the presence or absence of a base such as potassium bis(trimethylsilyl)amide (KHMDS), lithium diisopropylamide (LDA), lithium hydride, sodium hydride, or potassium hydride; an organic base such as trimethylamine, triethylamine, tributylamine, N,N-diisopropylethylamine, pyridine, N,N-dimethylaniline, N,N-dimethylaminopyridine, N-methylpiperidine, N-methylmorpholine, diethylamine, cyclohexylamine, procaine, sodium methoxide, sodium ethoxide, sodium tert-butoxide, or potassium tert-butoxide; or an inorganic base such as lithium carbonate, sodium carbonate, potassium carbonate, sodium hydroxide, potassium hydroxide, or sodium acetate, at room temperature to heating under reflux for 1 to 24 hours.

(Second step)

A compound (c) can be synthesized by reacting the compound (b) with the reagent represented by L-Cl or $L_2O$ in an aromatic hydrocarbon such as benzene, toluene, or xylene; an ether such as diethyl ether, tetrahydrofuran, dioxane, monoglyme, or diglyme; a halogenated hydrocarbon such as dichloromethane, chloroform, or carbon tetrachloride; an aliphatic hydrocarbon such as pentane, hexane, heptane, or ligroin; or an aprotic polar solvent such as acetonitrile, N,N-dimethylformamide, or dimethylsulfoxide, in the presence of a base such as potassium bis(trimethylsilyl)amide (KHMDS), lithium diisopropylamide (LDA), lithium hydride, sodium hydride, or potassium hydride; an organic base such as trimethylamine, triethylamine, tributylamine, N,N-diisopropylethylamine, pyridine, N,N-dimethylaniline, N,N-dimethylaminopyridine, N-methylpiperidine, N-methylmorpholine, diethylamine, cyclohexylamine, procaine, sodium methoxide, sodium ethoxide, sodium tert-butoxide, or potassium tert-butoxide; or an inorganic base such as lithium carbonate, sodium carbonate, potassium carbonate, sodium hydroxide, or potassium hydroxide, at -78°C to heating under reflux for 1 to 24 hours.

(Third step)

Compounds (d) and (e) can be individually synthesized by reacting the compound (c) with a Brønsted acid such as hydrochloric acid, sulfuric acid, acetic acid, trifluoroacetic acid, methanesulfonic acid, or p-toluenesulfonic acid; or a Lewis acid such as a boron trifluoride-diethyl ether complex, aluminum chloride, iron (III) chloride, zinc chloride, or titanium (IV) chloride in an aromatic hydrocarbon such as benzene, toluene, or xylene; an ether such as diethyl ether, tetrahydrofuran, dioxane, monoglyme, or diglyme; an alcohol such as methanol or ethanol; a halogenated hydrocarbon such as dichloromethane, chloroform, or carbon tetrachloride; an aliphatic hydrocarbon such as pentane, hexane, heptane, or ligroin; a solvent such as water or acetic acid; an aprotic polar solvent such as acetonitrile, N,N-dimethylformamide, or dimethylsulfoxide, or in a mixed solvent thereof, or in the absence of a solvent, at room temperature to heating under reflux for 1 to 72 hours.

(Fourth step)

The compounds (f) and (g) of the present invention can be synthesized by reacting the compounds (d) and (e) with a reducing agent such as lithium aluminum hydride, a borane-tetrahydrofuran complex, or a borane-dimethyl sulfide complex, respectively, in an ether such as diethyl ether, tetrahydrofuran, dioxane, monoglyme, or diglyme, at 0°C to heating under reflux for 1 to 24 hours.

(Method B) The synthetic intermediates (d) and (e) described in Method A can also be synthesized from the compound (b) in one step as described below.

[Chemical Formula 7]

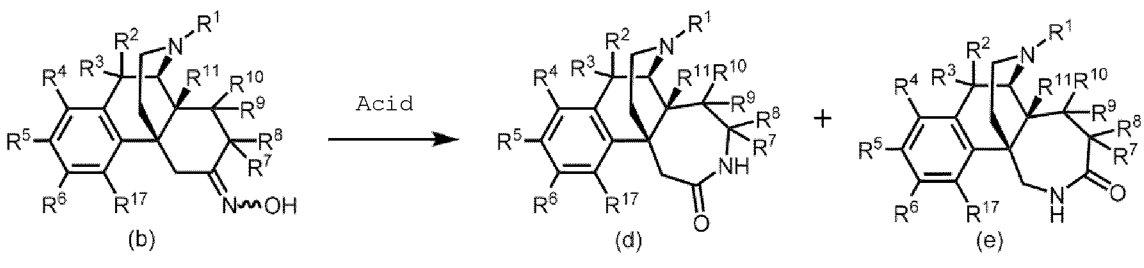


(wherein $R^1$ to $R^{11}$ and $R^{17}$ represent the same as those described above.)

Compounds (d) and (e) can be individually synthesized by reacting the compound (b) in an aromatic hydrocarbon such as benzene, toluene, or xylene; an ether such as diethyl ether, tetrahydrofuran, dioxane, monoglyme, or diglyme;

an alcohol such as methanol or ethanol; a halogenated hydrocarbon such as dichloromethane, chloroform, or carbon tetrachloride; an aliphatic hydrocarbon such as pentane, hexane, heptane, or ligroin; a solvent such as water or acetic acid; an aprotic polar solvent such as acetonitrile, N,N-dimethylformamide, or dimethylsulfoxide, or in a mixed solvent thereof, or in the absence of a solvent, in the presence or absence of an acid such as a Brønsted acid such as hydrochloric acid, sulfuric acid, acetic acid, or trifluoroacetic acid, or a Lewis acid such as a boron trifluoride-diethyl ether complex, aluminum chloride, iron chloride, or zinc chloride, at room temperature to heating under reflux for 1 to 72 hours.

(Method C) The synthetic intermediates (d) and (e) described in Method A can also be synthesized from the compound (a) in one step by using sodium azide or trimethylsilyl azide as described below.

[Chemical Formula 8]

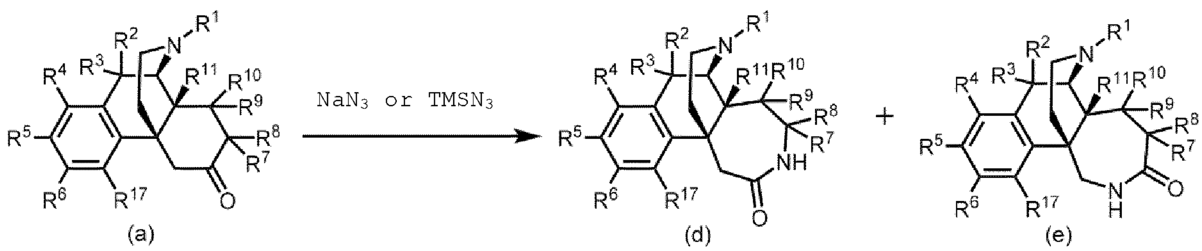


(a) (d) (e)

(wherein $R^1$ to $R^{11}$ and $R^{17}$ represent the same as those described above.)

Compounds (d) and (e) can be individually synthesized by reacting the raw material (a) with sodium azide ($NaN_3$) or trimethylsilyl azide ($TMSN_3$) in the presence or absence of a Brønsted acid such as hydrochloric acid, sulfuric acid, acetic acid, trifluoroacetic acid, trichloroacetic acid, polyphosphoric acid, an Eaton reagent, methanesulfonic acid, or p-toluenesulfonic acid; or a Lewis acid such as a boron trifluoride-diethyl ether complex, aluminum chloride, iron chloride, zinc chloride, or titanium (IV) chloride, in an aromatic hydrocarbon such as benzene, toluene, or xylene; an ether such as diethyl ether, tetrahydrofuran, dioxane, monoglyme, or diglyme; an alcohol such as methanol or ethanol; a halogenated hydrocarbon such as dichloromethane, chloroform, or carbon tetrachloride; an aliphatic hydrocarbon such as pentane, hexane, heptane, or ligroin; a solvent such as water; an aprotic polar solvent such as acetonitrile, N,N-dimethylformamide, or dimethylsulfoxide, or in a mixed solvent thereof, or in the absence of a solvent, at 0°C to heating under reflux for 1 to 24 hours.

(ii) A production method in a case where in the formula (I), X represents a nitrogen atom, one of A or B represents N-$R^{16}$ ($R^{16}$ is a $C_{1-10}$ alkyl group, an aralkyl group (the number of carbon atoms in the aryl moiety is $C_{6-10}$, and the number of carbon atoms in the alkylene moiety is $C_{1-5}$), or a heteroarylalkyl group optionally having a substituent, and the other represents a carbonyl group.

(Method D) The compounds (d-1) and (e-1) of the present invention can be individually synthesized by the method described below using a compound (a) as the starting material.

[Chemical Formula 9]

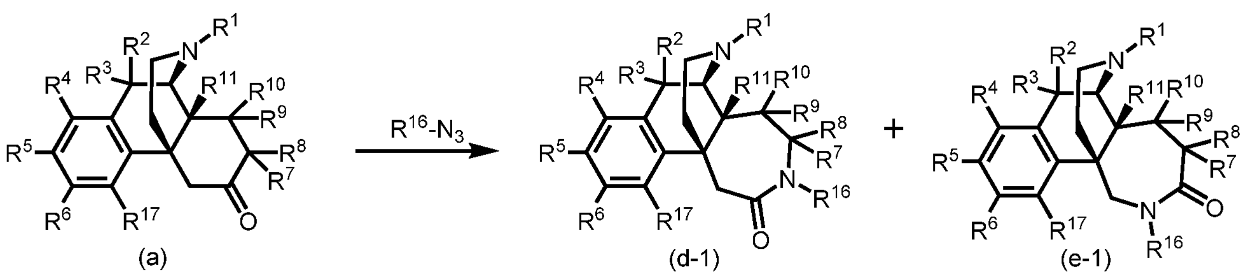


(a) (d-1) (e-1)

(wherein $R^{16}$ represents a $C_{1-10}$ alkyl group, an aralkyl group (the number of carbon atoms in the aryl moiety is $C_{6-10}$, and the number of carbon atoms in the alkylene moiety is $C_{1-5}$), or a heteroarylalkyl group optionally having a substituent, and $R^1$ to $R^{11}$ and $R^{17}$ represent the same as those described above.)

The compounds (d-1) and (e-1) of the present invention can be individually synthesized by reacting the raw material (a) with an azide represented by $R^{16}$-$N_3$ in the presence or absence of a Brønsted acid such as hydrochloric acid, sulfuric acid, acetic acid, trifluoroacetic acid, trichloroacetic acid, polyphosphoric acid, an Eaton reagent, methanesulfonic acid, or p-toluenesulfonic acid; or a Lewis acid such as a boron trifluoride-diethyl ether complex, aluminum chloride, iron chloride, zinc chloride, or titanium (IV) chloride, in an aromatic hydrocarbon such as benzene, toluene, or xylene; an ether such as diethyl ether, tetrahydrofuran, dioxane, monoglyme, or diglyme; an alcohol such as methanol or ethanol; a halogenated hydrocarbon such as dichloromethane, chloroform, or carbon tetrachloride; an aliphatic hydrocarbon such as pentane, hexane, heptane, or ligroin; a solvent such as water; an aprotic polar solvent such as acetonitrile, N,N-dimethylformamide, or dimethylsulfoxide, or in a mixed solvent thereof, or in the absence of a solvent, at 0°C to heating under reflux for 1 to 24 hours.

(iii) A production method in a case where in the formula (I), X represents a nitrogen atom, either A or B represents a carbonyl group, m represents 0, and Z represents a bond

(Method E) The compound (h) of the present invention can be synthesized by an alkylation reaction of the compound (d) or (e) (both are collectively referred to as (d-e)) obtained in the above (i).

[Chemical Formula 10]

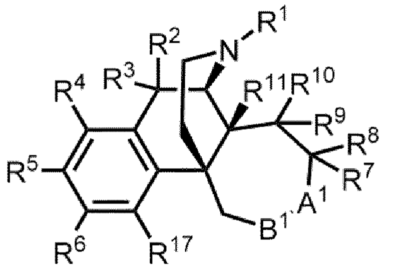

One of $A^1$ or $B^1$ represents NH, and the other represents a carbonyl group

(d-e)

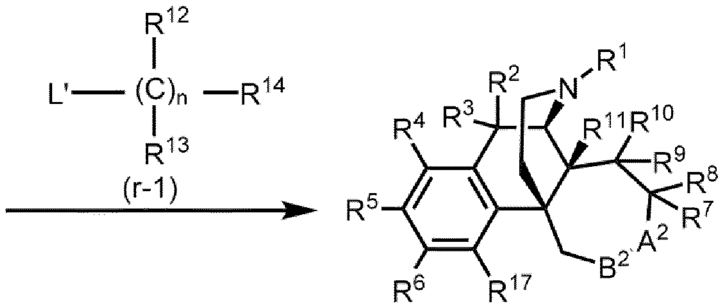

(h)

One of $A^2$ or $B^2$ represents a group 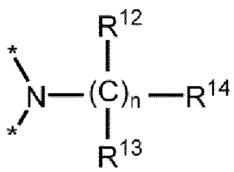, and the other represents a carbonyl group

(wherein L' represents a leaving group such as a methanesulfonyloxy group, a p-toluenesulfonyloxy group, or halogen, and $R^1$ to $R^{14}$, $R^{17}$, and n represent the same as those described above. * represents a bond.)

The compound (h) of the present invention can be synthesized by reacting the compound (d) or (e) (both are collectively referred to as (d-e)) obtained in the above (i) with an alkylating agent represented by (r-1) in an aromatic hydrocarbon such as benzene, toluene, or xylene; an ether such as diethyl ether, tetrahydrofuran, dioxane, monoglyme, or diglyme; an alcohol such as methanol or ethanol; a halogenated hydrocarbon such as dichloromethane, chloroform, or carbon tetrachloride; an aliphatic hydrocarbon such as pentane, hexane, heptane, or ligroin; or an aprotic polar solvent such as acetonitrile, N,N-dimethylformamide, or dimethylsulfoxide, in the presence of a base such as potassium bis(trimethylsilyl)amide (KHMDS), lithium diisopropylamide (LDA), lithium hydride, sodium hydride, or potassium hydride; an organic base such as trimethylamine, triethylamine, tributylamine, N,N-diisopropylethylamine, pyridine, N,N-dimethylaniline, N,N-dimethylaminopyridine, N-methylpiperidine, N-methylmorpholine, diethylamine, cyclohexylamine, procaine, sodium methoxide, sodium ethoxide, sodium tert-butoxide, or potassium tert-butoxide; or an inorganic base such as sodium bicarbonate, lithium carbonate, sodium carbonate, potassium carbonate, sodium hydroxide, or potassium hydroxide, in the presence or absence of potassium iodide or sodium iodide, at room temperature to heating under reflux for 1 to 24 hours.

(iv) A production method in a case where in the formula (I), X represents a nitrogen atom, Y represents a carbonyl group or a thiocarbonyl group, Z represents a bond, an ethenylene group optionally having a substituent, or an ethynylene group, and m represents 1,

(Method F) The compounds (i) and (j) of the present invention can be obtained by a reaction of the compound (f) or (g) (both are collectively referred to as (f-g)) of the present invention obtained in the above (i) with (r-2) or (r-3).

[Chemical Formula 11]

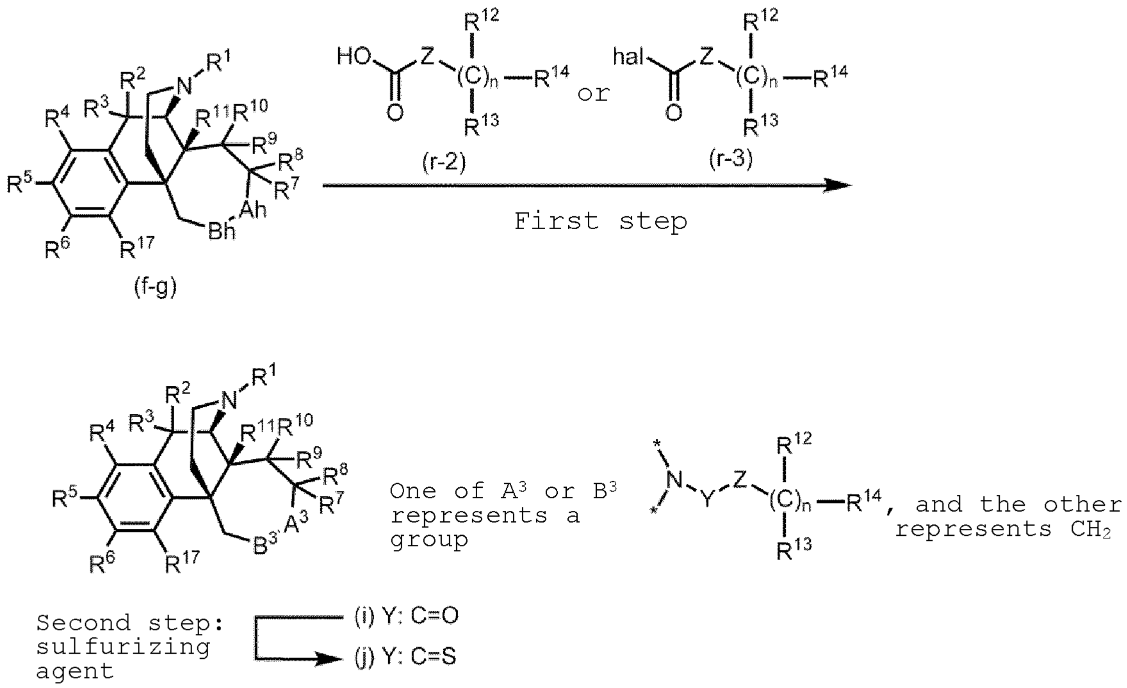


(wherein one of Ah or Bh represents NH, the other represents $CH_2$, hal represents a halogen atom, Z represents a bond and an ethenylene group optionally having a substituent or an ethynylene group, and n, $R^1$ to $R^{14}$, and $R^{17}$ represent the same as those described above. * represents a bond.)

(First step)

The compound (i) of the present invention can be synthesized by reacting the carboxylic acid represented by (r-2) or the acid halide represented by (r-3) with the compound (f) or (g) (both are collectively referred to as (f-g)) of the present invention obtained in the above (i) in an aromatic hydrocarbon such as benzene, toluene, or xylene; an ether such as diethyl ether, tetrahydrofuran, dioxane, monoglyme, or diglyme; a halogenated hydrocarbon such as dichloromethane, chloroform, or carbon tetrachloride; an alcohol such as methanol or ethanol; an aliphatic hydrocarbon such as pentane, hexane, heptane, or ligroin; or an aprotic polar solvent such as acetonitrile, N,N-dimethylformamide, or dimethylsulfoxide, in the presence of an organic base such as N,N-dimethylaminopyridine, trimethylamine, triethylamine, tributylamine, N,N-diisopropylethylamine, pyridine, N,N-dimethylaniline, N-methylpiperidine, N-methylmorpholine, diethylamine, cyclohexylamine, or procaine; or an inorganic base such as potassium carbonate or lithium carbonate, in the presence or absence of a condensing agent such as O-(7-azabenzotriazol-1-yl)-N,N,N',N'-tetramethyluronium hexafluorophosphate (HATU), O-(benzotriazol-1-yl)-N,N,N',N'-tetramethyluronium hexafluorophosphate (HBTU), N,N'-dicyclohexylcarbodiimide (DCC), 1-ethyl-3-(3-dimethylaminopropyl)carbodiimide hydrochloride (WSC), or 4-(4,6-dimethoxy-1,3,5-triazin-2-yl)-4-methylmorpholinium chloride n-hydrate (DMT-MM), at 0°C to heating under reflux for 1 to 12 hours.

(Second step)

The compound (i) of the present invention can be converted into the compound (j) of the present invention by a reaction in an aromatic hydrocarbon such as benzene, toluene, or xylene; an ether such as diethyl ether, tetrahydrofuran, dioxane, monoglyme, or diglyme; a halogenated hydrocarbon such as dichloromethane, chloroform, or carbon tetrachloride; or an aliphatic hydrocarbon such as pentane, hexane, heptane, or ligroin, using a sulfurizing agent such as diphosphorus pentasulfide, Lawesson's reagent, Davy reagent, Japanese reagent, or Belleau's reagent, at 0°C to heating under reflux for 1 to 12 hours.

(v) A production method in a case where in the formula (I), X represents a nitrogen atom, m represents 0, and Z represents a bond

(Method G) The compound (k) of the present invention can be obtained by a reductive amination reaction of the compound (f) or (g) (both are collectively referred to as (f-g)) of the present invention obtained in the above (i) with an aldehyde (r-4).

[Chemical Formula 12]

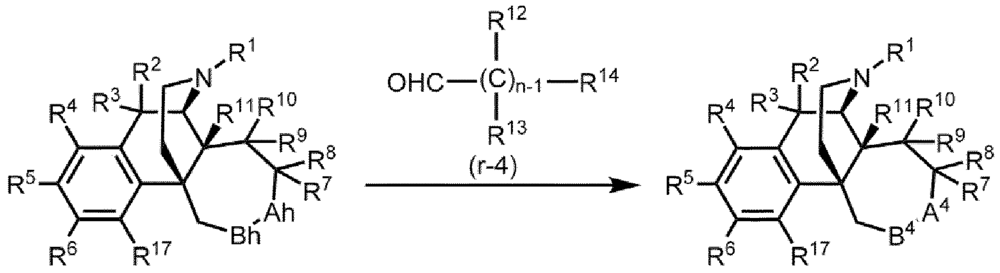


(f-g) (k)

One of $A^4$ or $B^4$ represents a group 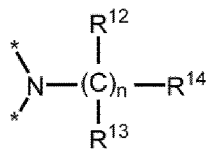 , and the other represents $CH_2$

(wherein one of Ah or Bh represents NH, the other represents $CH_2$, and $R^1$ to $R^{14}$, $R^{17}$, and n represent the same as those described above. * represents a bond.)

The compound (k) of the present invention can be synthesized by reacting the compound (f) or (g) (both are collectively referred to as (f-g)) of the present invention obtained in the above (i) with the aldehyde represented by (r-4) in a solvent such as an aromatic hydrocarbon such as benzene, toluene, or xylene; an ether such as diethyl ether, tetrahydrofuran, dioxane, monoglyme, or diglyme; a halogenated hydrocarbon such as dichloromethane, chloroform, or carbon tetrachloride; an alcohol such as methanol or ethanol; an aliphatic hydrocarbon such as pentane, hexane, heptane, or ligroin; an aprotic polar solvent such as acetonitrile, N N-dimethylformamide, or dimethylsulfoxide; or acetic acid, or a mixed solvent thereof, in the presence of a hydride reducing agent such as sodium borohydride, sodium cyanoborohydride, sodium triacetoxyborohydride, lithium tri(sec-butyl)borohydride, or potassium tri(sec-butyl)borohydride, at 0°C to heating under reflux for 1 to 12 hours.

(Method H) The compound (k) of the present invention can be obtained by an $S_N2$ reaction of the compound (f) or (g) (both are collectively referred to as (f-g)) of the present invention obtained in the above (i) with an alkylating agent (r-1).

[Chemical Formula 13]

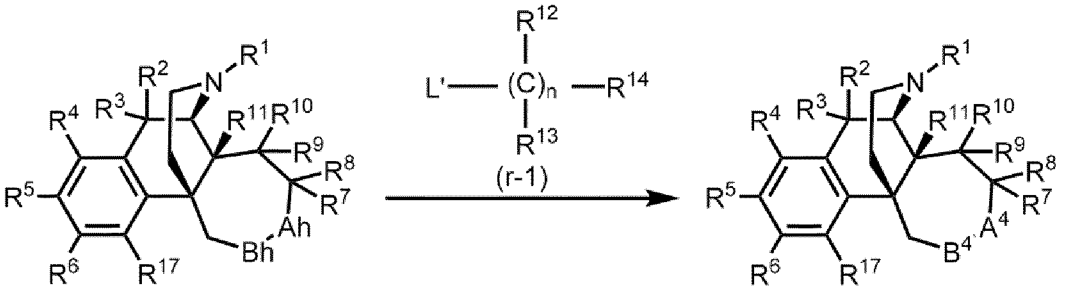


(f-g) (k)

One of $A^4$ or $B^4$ represents a group 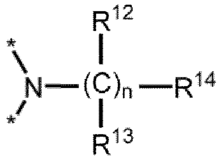, and the other represents $CH_2$

(wherein one of Ah or Bh represents NH, the other represents CH, and $R^1$ to $R^{14}$, $R^{17}$, L', and n represent the same as those described above. * represents a bond.)

The compound (k) of the present invention can be synthesized by reacting the compound (f) or (g) (both are collectively referred to as (f-g)) of the present invention obtained in the above (i) with an alkylating agent represented by (r-1) in an aromatic hydrocarbon such as benzene, toluene, or xylene; an ether such as diethyl ether, tetrahydrofuran, dioxane, monoglyme, or diglyme; an alcohol such as methanol or ethanol; a halogenated hydrocarbon such as dichloromethane, chloroform, or carbon tetrachloride; an aliphatic hydrocarbon such as pentane, hexane, heptane, or ligroin; an aprotic polar solvent such as acetonitrile, N,N-dimethylformamide, or dimethylsulfoxide, in the presence of a base such as potassium bis(trimethylsilyl)amide (KHMDS) or lithium diisopropylamide (LDA); an organic base such as trimethylamine, triethylamine, tributylamine, N,N-diisopropylethylamine, pyridine, N,N-dimethylaniline, N,N-dimethylaminopyridine, N-methylpiperidine, N-methylmorpholine, diethylamine, cyclohexylamine, procaine, sodium methoxide, sodium ethoxide, or potassium tert-butoxide; or an inorganic base such as sodium bicarbonate, lithium carbonate, sodium carbonate, potassium carbonate, sodium hydroxide, or potassium hydroxide, in the presence or absence of potassium iodide or sodium iodide, at room temperature to heating under reflux for 1 to 24 hours.

(Method I) The compound (k) of the present invention can be obtained by reducing the amide moiety of the compound (i) of the present invention obtained in the above (iv).

[Chemical Formula 14]

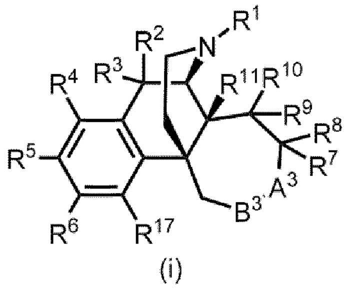

(i)

One of $A^3$ or $B^3$ represents a group

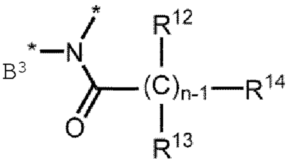

, and the other represents $CH_2$

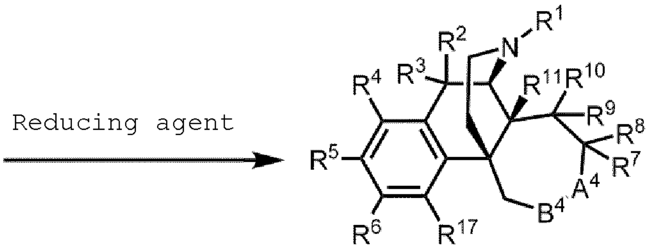


(k)

One of $A^4$ or $B^4$ represents a group

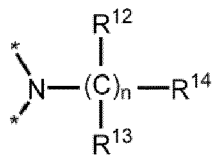

, and the other represents $CH_2$

(wherein $R^1$ to $R^{14}$ and $R^{17}$ or n represent the same as those described above. * represents a bond.)

The compounds (k) of the present invention can be synthesized by reacting the compound (i) of the present invention obtained in the above (iv) with a reducing agent such as lithium aluminum hydride, a borane-tetrahydrofuran complex, or a borane-dimethyl sulfide complex, in an ether such as diethyl ether, tetrahydrofuran, dioxane, monoglyme, or diglyme, at 0°C to heating under reflux for 1 to 24 hours.

(J method) The compound ($f^1$-$g^1$) of the present invention can also be synthesized by a reduction reaction of the compound (d -1) or (e-1) (both are collectively referred to as ($d^1$-$e^1$)) of the present invention obtained in the above (ii), when one of A or B represents N-$R^{16}$ ($R^{16}$ represents a $C_{1-10}$ alkyl group optionally having a substituent, an aralkyl group (the number of carbon atoms in the aryl moiety is $C_{6-10}$, and the number of carbon atoms in the alkylene moiety is $C_{1-5}$), or a heteroaryl group,) and the other represents $CH_2$.

[Chemical Formula 15]

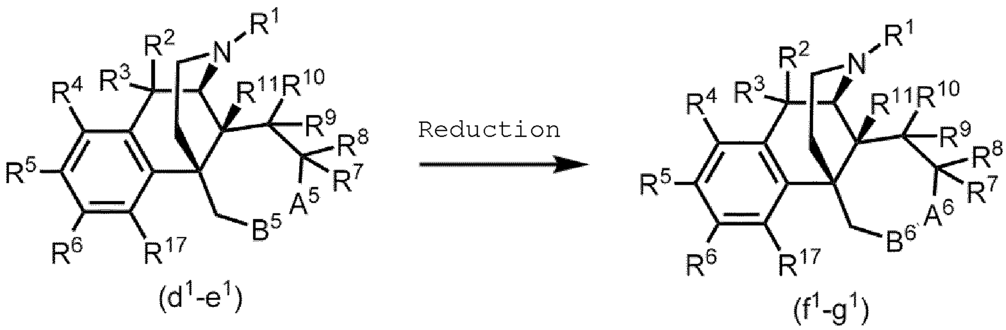


($d^1$-$e^1$) ($f^1$-$g^1$)

One of $A^5$ or $B^5$ represents N-$R^{16}$, and the other represents a carbonyl group

One of $A^6$ or $B^6$ represents N-$R^{16}$, and the other represents $CH_2$

(wherein $R^1$ to $R^{11}R^{16}$, and $R^{17}$ represent the same as those described above.)

The compound ($f^1$-$g^1$) of the present invention can be synthesized by performing the same reaction as in the fourth step of (Method A) on the compound (d-1) or (e-1) (both are collectively referred to as ($d^1$-$e^1$)) of the present invention obtained in the above (ii).

(vi) A production method in a case where in the formula (I), X represents a nitrogen atom, Y represents a carbonyl group, Z represents $NR^{15}$ or an oxygen atom, and m represents 1.

(Method K) The compound (n) of the present invention can be obtained by the method described below using the compound (f) or (g) (both are collectively referred to as (f-g)) of the present invention obtained in the above (i).

[Chemical Formula 16]

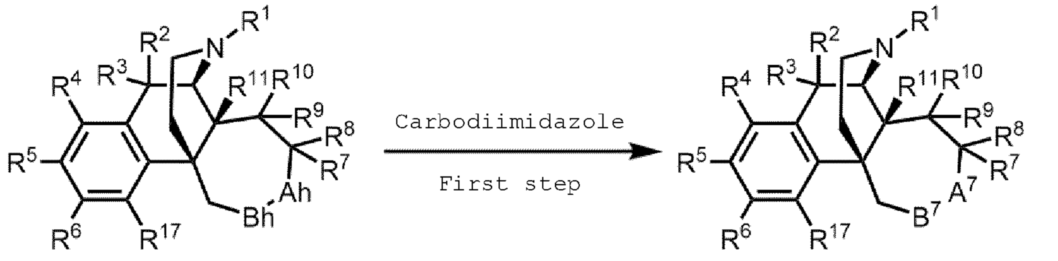


(f-g) (l)

One of $A^7$ or $B^7$ represents a group 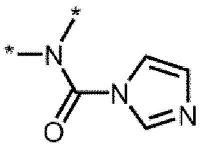 , and the other represents $CH_2$

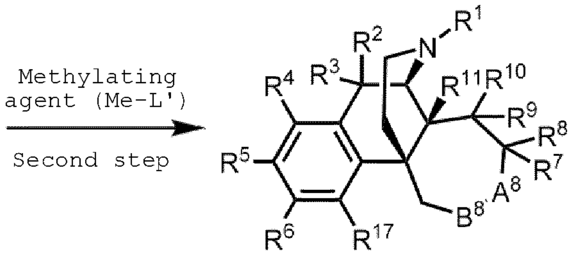


(m)

One of $A^8$ or $B^8$ represents a group 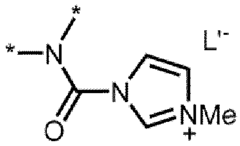 , and the other represents $CH_2$

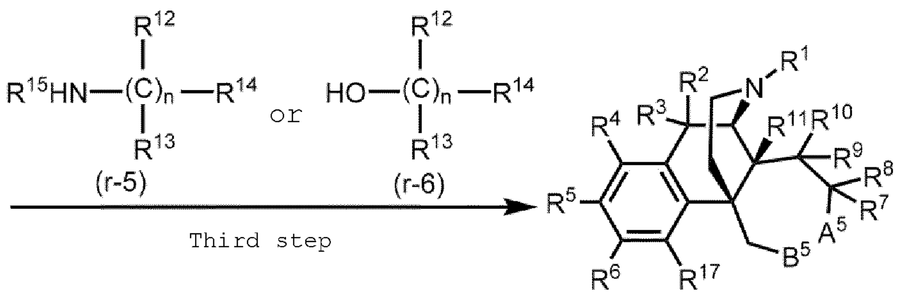


(n)

One of $A^5$ or $B^5$ represents a group 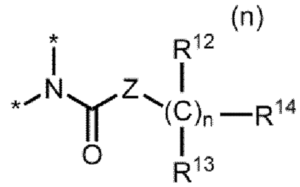 , and the other represents $CH_2$

(wherein one of Ah or Bh represents NH, the other represents $CH_2$, Z represents $NR^{15}$ or an oxygen atom, $R^1$ to $R^{15}$, $R^{17}$, L', and n represent the same as those described above, and L- represents the counter anion. * represents a bond.)

(First step)

The compound of the present invention (l) can be synthesized by reacting the compound (f) or (g) (both are collectively referred to as (f-g)) of the present invention obtained in the above (i) with carbodiimidazole in an aromatic hydrocarbon such as benzene, toluene, or xylene; an ether such as diethyl ether, tetrahydrofuran, dioxane, monoglyme, or diglyme; a halogenated hydrocarbon such as dichloromethane, chloroform, or carbon tetrachloride; an alcohol such as methanol or ethanol; an aliphatic hydrocarbon such as pentane, hexane, heptane, or ligroin; or an aprotic polar solvent such as acetonitrile, N,N-dimethylformamide, or dimethylsulfoxide, in the presence of an organic base such as N,N-dimethylaminopyridine, trimethylamine, triethylamine, tributylamine, N,N-diisopropylethylamine, pyridine, N,N-dimethylaniline, N-methylpiperidine, N-methylmorpholine, diethylamine, cyclohexylamine, or procaine; or an inorganic base such as potassium carbonate or lithium carbonate at 0°C to heating under reflux for 1 to 12 hours.

(Second step)

The compound (m) of the present invention can be synthesized by reacting the compound (1) of the present invention with a methylating agent such as methyl iodide, dimethyl sulfate, trifluoromethanesulfonylmethane, or a Meerwein reagent in an aromatic hydrocarbon such as benzene, toluene, or xylene; an ether such as diethyl ether, tetrahydrofuran, dioxane, monoglyme, or diglyme; a halogenated hydrocarbon such as dichloromethane, chloroform, or carbon tetrachloride; an alcohol such as methanol or ethanol; an aliphatic hydrocarbon such as pentane, hexane, heptane, or ligroin; or an aprotic polar solvent such as acetonitrile, N,N-dimethylformamide, or dimethylsulfoxide, in the presence or absence of an organic base such as N,N-dimethylaminopyridine, trimethylamine, triethylamine, tributylamine, N,N-diisopropylethylamine, pyridine, N,N-dimethylaniline, N-methylpiperidine, N-methylmorpholine, diethylamine, cyclohexylamine, or procaine; or an inorganic base such as potassium carbonate or lithium carbonate, at 0°C to heating under reflux for 12 to 48 hours.

(Third step)

The compound (n) of the present invention can be synthesized by reacting the compound (m) of the present invention with an amine represented by (r-5) or an alcohol represented by (r-6) in an aromatic hydrocarbon such as benzene, toluene, or xylene; an ether such as diethyl ether, tetrahydrofuran, dioxane, monoglyme, or diglyme; a halogenated hydrocarbon such as dichloromethane, chloroform, or carbon tetrachloride; an aliphatic hydrocarbon such as pentane, hexane, heptane, or ligroin; or an aprotic polar solvent such as acetonitrile, N,N-dimethylformamide, or dimethylsulfoxide, in the presence of an organic base such as N,N-dimethylaminopyridine, trimethylamine, triethylamine, N,N-diisopropylethylamine, tributylamine, pyridine, N,N-dimethylaniline, N-methylpiperidine, N-methylmorpholine, diethylamine, cyclohexylamine, or procaine; or an inorganic base such as potassium carbonate or lithium carbonate, at 0°C to heating under reflux for 1 to 12 hours.

(vii) A production method in a case where in the formula (I), $R^6$ represents a hydroxy group
(Method L) The compound (p) of the present invention can be synthesized by a dealkylation reaction of the compound (o) of the present invention obtained in the above (i) to (vi).

[Chemical Formula 17]

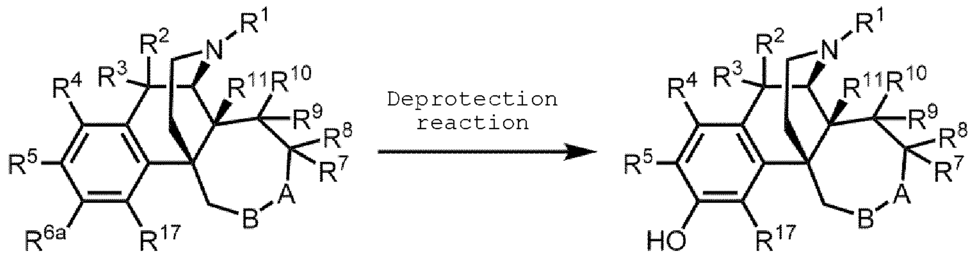


(o) (p)

One of A or B represents a group 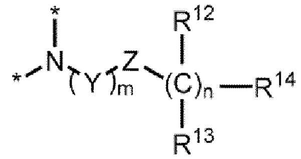 , and the other represents either $CH_2$ or a carbonyl group

(wherein R6a represents a $C_{1-10}$ alkoxy group, and $R^1$ to $R^{14}$, $R^{17}$, n, m, Y, and Z represent the same as those described above. * represents a bond.)

The compound (p) of the present invention can be synthesized by reacting the compound (o) of the present invention obtained in the above (i) to (vi) with boron tribromide, trimethylsilyl iodide, hydrogen bromide, pyridinium hydrochloride, or the like in a halogenated hydrocarbon such as dichloromethane, chloroform, or carbon tetrachloride; a protic organic solvent such as acetic acid; or an aprotic polar solvent such as acetonitrile or ethyl acetate, or in the absence of a solvent, at -30 to 180°C for 30 minutes to 24 hours, or reacting 1-propanethiol, 1-dodecanethiol, or the like in an aprotic polar solvent such as N,N-dimethylformamide, dimethylacetamide, N-methylpyrrolidone, or dimethylsulfoxide, in the presence of an organic base such as potassium tert-butoxide or sodium tert-butoxide, at 100°C to 180°C for 30 minutes to 24 hours.

(viii) A production method in a case where in the formula (I), X represents N-oxide

(Method M) The compound (s) of the present invention can be synthesized by an oxidation reaction of the compound (q) of the present invention obtained in the above (i) to (vii).

[Chemical Formula 18]

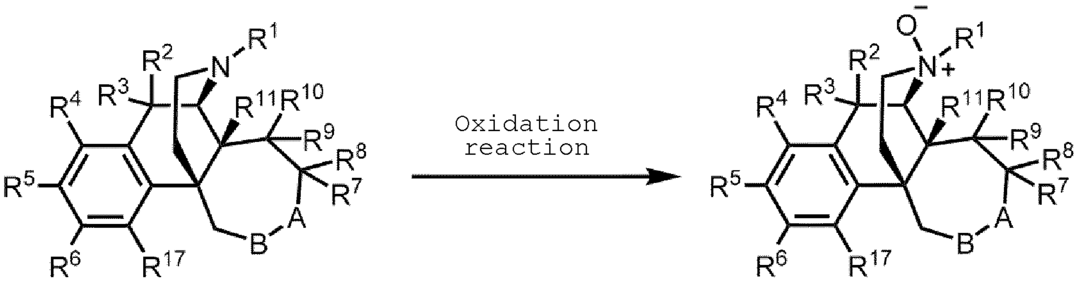


(q) (s)

One of A or B represents a group 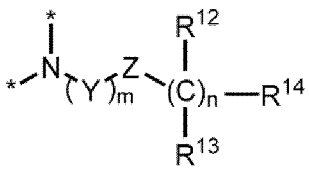 , and the other represents either $CH_2$ or a carbonyl group

(wherein $R^1$ to $R^{14}$, $R^{17}$, n, m, Y, and Z represent the same as those described above. * represents a bond.)

The compound (s) of the present invention can be synthesized by reacting the compound (q) of the present invention obtained in the above (i) to (vii) with 1 to 2 equivalents of an oxidizing agent such as a hydrogen peroxide ($H_2O_2$) aqueous solution or m-chloroperbenzoic acid (mCPBA), in a halogenated hydrocarbon solvent such as dichloromethane, chloroform, or carbon tetrachloride, at 0°C to room temperature for 30 minutes to 24 hours.

The compound obtained in each of the steps can be purified by, for example, silica gel column chromatography, if necessary.

Furthermore, if necessary, an acid addition salt can be formed by a normal method. For example, this formation is performed at room temperature or by appropriately heating the compound (I) of the present invention in an organic solvent such as ethyl acetate; an alcohol such as methanol or ethanol; or a polar solvent such as water, in the presence of a mineral acid such as hydrochloric acid, sulfuric acid, or phosphoric acid; an organic carboxylic acid such as formic acid, acetic acid, citric acid, trichloroacetic acid, trifluoroacetic acid, fumaric acid, or maleic acid; an organic sulfonic acid such as methanesulfonic acid, benzenesulfonic acid, p-toluenesulfonic acid, mesitylenesulfonic acid, or naphthalenesulfonic acid; or the like.

The azepane derivative represented by the formula (I) and the pharmaceutically acceptable salt thereof can be formulated into a composition together with a pharmaceutically acceptable carrier for parenteral administration, oral administration in a solid or liquid form, or the like to humans. In addition, the azepane derivative represented by the formula (I) and the pharmaceutically acceptable salt thereof can be used in combination with another analgesic.

The solid formulation for oral administration includes a capsule, a tablet, a pill, a powder, and a granule. In preparation of the solid formulation, an excipient, a disintegrator, a binder, a lubricant, a pigment, or the like can be used. Here, the excipient includes lactose, D-mannitol, crystalline cellulose, or glucose. The disintegrator includes starch or carboxymethylcellulose calcium (CMC-Ca). The lubricant includes magnesium stearate or talc. The binder includes hydroxypropyl cellulose (HPC), gelatin, or polyvinyl pyrrolidone (PVP). In the case of capsule, tablet, or pill, a buffer may be further used. The tablet and pill may be provided with an enteric coating.

The form of the composition of the present invention for injection includes a pharmaceutically acceptable sterile water, non-aqueous solution, suspension, or emulsion form. Examples of a suitable non-aqueous carrier, a diluent, a solvent or a vehicle include propylene glycol, polyethylene glycol, a vegetable oil such as an olive oil, or an injectable organic ester such as ethyl oleate. Such compositions may also contain an auxiliary such as an antiseptic, a wetting agent, an emulsifier, a soothing agent, a buffering agent, a preservative, or a dispersing agent.

These compositions can be sterilized, for example, by filtration through a bacteria-retaining filter, or by mixing a sterilizing agent, or a sterilizing agent in the form of a sterile solid composition capable of dissolving in some other sterilizable and injectable media just prior to use.

In preparation for ophthalmic administration, in addition to the compound of the present invention, a solubilizing auxiliary, a preservative, an isotonizing agent, a thickener, or the like can be preferably added.

A liquid formulation for oral administration includes an inert diluent commonly used by those skilled in the art, e.g., a water-containing pharmaceutically acceptable emulsion, solution, suspension, syrup, or elixir. In addition to such inert diluents, the composition can also include an auxiliary such as a wetting agent, an emulsifying agent, or a suspension; and a sweetening agent, a flavoring agent, or a perfuming agent.

The formulation for rectal administration may preferably contain, in addition to the compound of the present invention, an excipient, such as cocoa butter or a suppository wax.

In an adult, usually, the dosage of the azepane derivative represented by the formula (I), a tautomer of the compound, a stereoisomer of the compound, a pharmaceutically acceptable salt thereof, or a solvate thereof as an active ingredient is 0.01 $\mu$g to 1 g/day, preferably 0.0001 to 200 mg/day in injection, and 0.1 $\mu$g to 10 g/day, preferably 0.001 to 2000 mg/day in oral administration. However, the dosage can be increased or decreased depending on age, symptom, or the like. If desired, the daily dose can be divided in 2 or 4 doses for administration.

Examples of the diseases and symptoms related to $\kappa$ opioid receptors include cardiovascular system disorders, digestive system diseases, blood system diseases, respiratory system diseases, liver diseases, nervous system disorders, urinary system disorders, pain, cough, pruritus, and ischemic brain diseases. The compound of the present invention is effective for treatment, improvement, and prevention of these diseases and symptoms because of its $\kappa$ opioid receptor selectivity and strong agonist activity for $\kappa$ opioid receptors.

## Examples

Next, a description is made of the present invention in more detail with reference to Reference Examples, Examples, and Test Examples, but the present invention is not limited thereto.

(Reference Example 1)

Synthesis of (4bR,8aS,9R)-11-(cyclopropylmethyl)-8a-hydroxy-3-methoxy-6-oxo-6,7,8,8a,9, 10-hexahydro-5H-9,4b-(epiminoethano)phenanthren-4-yl trifluoromethanesulfonate

[Chemical Formula 19]

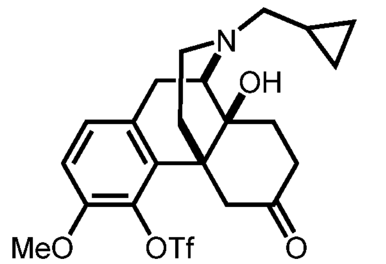

In N,N-dimethylformamide (250 mL) were dissolved (4bR,8aS,9R)-11-(cyclopropylmethyl)-8a-hydroxy-3-methoxy-8,8a,9,10-tetrahydro-5H-9,4b-(epiminoethano)phenanthrene-6(7H)-one (synthesized by the method described in Tetrahedron Lett., 2010, 51, 2359) (17.87 g, 50 mmol), N-phenylbis(trifluoromethanesulfonimide) (35.73 g, 100 mmol), and potassium carbonate (20.73 g, 150 mmol), followed by stirring at room temperature for 18 hours. To the reaction solution, chloroform (750 mL) was added, and the resultant solution was washed with a 1 M aqueous sodium hydroxide solution and water, dried over sodium sulfate, and then concentrated under reduced pressure. The obtained crude product was recrystallized from ethyl acetate to yield the title compound (21.31 g) as a white crystal. The recrystallization mother liquor (2.63 g) was purified by silica gel column chromatography (0 to 10% methanol/chloroform), followed by recrystallization from a mixed solvent of ethyl acetate and heptane to yield the title compound 4 (1.245 g, 92% in total) as a white crystal.

$^1$H-NMR (400 MHz, $CDCl_3$)$\delta$ (ppm): 0.09 - 0.18 (m, 2H), 0.52 - 0.59 (m, 2H), 0.81 - 0.93 (m, 1H), 1.65 - 1.87 (m, 3H), 2.03 - 2.14 (m, 2H), 2.20 (ddd, J = 5, 13, 13 Hz, 1H), 2.39 (d, J = 7 Hz, 2H), 2.66 - 2.73 (m, 1H), 2.77 (ddd, J = 8, 13, 14 Hz, 1H), 2.88 (dd, J = 7, 19 Hz, 1H), 3.01 - 3.18 (m, 3H), 3.52 (dd, J = 2, 14 Hz, 1H), 3.80 (s, 3H), 4.75 (s, 1H), 6.85 (d, J = 8 Hz, 1H), 7.05 (d, J = 8 Hz, 1H).

(Reference Example 2)

Synthesis of (4bR,8aS,9R)-11-(cyclopropylmethyl)-8a-hydroxy-3-methoxy-8,8a,9,10-tetrahydro-5H-9,4b-(epiminoethano)phenanthren-6(7H)-one

[Chemical Formula 20]

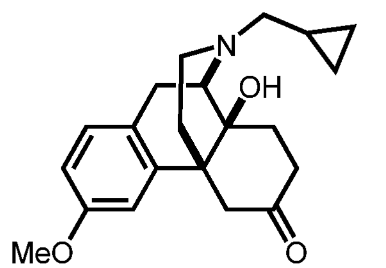

To the compound (22.55 g, 46 mmol) obtained in Reference Example 1, 1,3-bis(diphenylphosphino)propane (1.90 g, 4.6 mmol), and palladium acetate (1.03 g, 4.6 mmol) dissolved in N,N-dimethylformamide (230 mL) were added formic acid (5.22 mL, 138 mmol) and triethylamine (32.1 mL, 230 mmol), followed by stirring at 80°C for 30 minutes. To the reaction solution, a 1 M aqueous sodium hydroxide solution was added, then the resultant solution was extracted three times with chloroform, and the combined extracts were dried over sodium sulfate and then concentrated under reduced pressure. The obtained crude product was purified by silica gel column chromatography (0 to 10% methanol/chloroform) to yield the title compound (13.95 g, 89% in total) as a colorless solid.

$^1$H-NMR (400 MHz, $CDCl_3$)$\delta$ (ppm): 0.09 - 0.18 (m, 2H), 0.50 - 0.59 (m, 2H), 0.80 - 0.93 (m, 1H), 1.13 - 1.27 (m, 1H), 1.72 - 1.93 (m, 2H), 2.02 - 2.22 (m, 3H), 2.40 (d, J = 7 Hz, 2H), 2.55 - 2.63 (m, 1H), 2.70 - 2.86 (m, 3H), 3.01 - 3.17 (m, 3H), 3.77 (s, 3H), 4.87 (br s, 1H), 6.69 (d, J = 9 Hz, 1H), 6.80 (s, 1H), 6.99 (d, J = 9 Hz, 1H).

(Reference Example 3)

Synthesis of (4bR,8aS,9R)-11-(cyclopropylmethyl)-8a-hydroxy-3-methoxy-8,8a,9,10-tetrahydro-5H-9,4b-(epiminoethano)phenanthren-6(7H)-one oxime

[Chemical Formula 21]

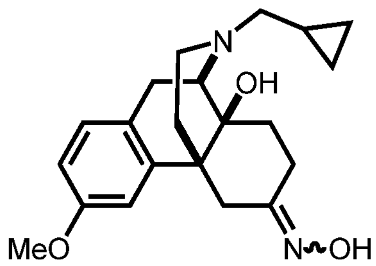

To a mixed solution of the compound (4.51 g, 13.2 mmol) obtained in Reference Example 2 in ethanol (111 mL) and water (28 mL) was added hydroxyamine hydrochloride (1.84 g, 26.4 mmol), followed by heating under reflux for 4 hours. After allowed to cool, the reaction solution was concentrated under reduced pressure, diluted with ethyl acetate, and then washed once with a saturated aqueous sodium bicarbonate solution and twice with a saturated saline solution. The organic layer was dried over sodium sulfate and then concentrated under reduced pressure. The obtained crude product was purified by silica gel column chromatography (2 to 25% methanol/ethyl acetate) to yield the title compound (4.1 g, 87%) as a colorless amorphous form.

$^1$H-NMR (400 MHz, $CDCl_3$)$\delta$ (ppm): 0.07 - 0.17 (m, 2H), 0.48 - 0.58 (m, 2H), 0.79 - 0.90 (m, 1H), 1.10 - 1.21 (m, 1H), 1.50 - 1.67 (m, 2.3H), 2.07 - 2.26 (m, 2.7H), 2.33 - 2.42 (m, 2.7H), 2.53 - 2.81 (m, 3.3H), 2.98 - 3.14 (m, 2.3H), 3.73 (d, J = 16 Hz, 0.7H), 3.76 (s, 0.9H), 3.77 (s, 2.1H), 4.80 (br s, 1H), 6.67 - 6.71 (m, 1H), 6.84 (d, J = 3 Hz, 0.3H), 6.92 - 7.02 (m, 2H), 7.10 (d, J = 3 Hz, 0.7H).

(Example 1)

Synthesis of mixture of (5aS,6R,11bR)-14-(cyclopropylmethyl)-5a-hydroxy-10-methoxy-3,4,5,5a,6,7-hexahydro-6,11b-(epiminoethano)naphtho[1,2-d]azepin-2(1H)-one (isomer A) and (5aS,6R,11bS)-14-(cyclopropylmethyl)-5a-hydroxy-10-methoxy-l,2,5,5a,6,7-hexahydro-6,11b-(epiminoethano)naphtho[1,2-c]azepin-3(4H)-one (isomer B)

[Chemical Formula 22]

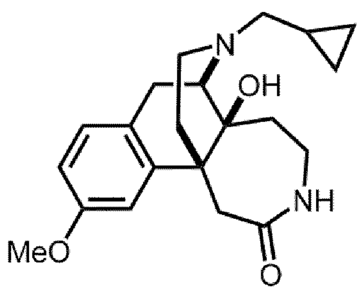

Isomer A

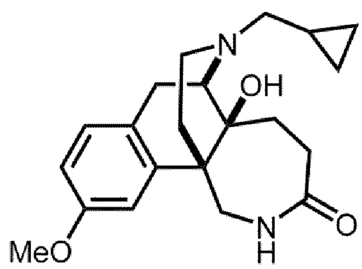

Isomer B

To a solution of the compound (4.06 g, 11.4 mmol) obtained in Reference Example 3 in chloroform (120 mL) were added triethylamine (3.33 mL, 23.9 mmol) and p-toluenesulfonyl chloride (4.34 g, 22.8 mmol) under ice cooling, followed by stirring at room temperature for 18 hours. p-Toluenesulfonyl chloride (2.0 g, 10.5 mmol) was added, followed by further stirring at room temperature for 3 and a half hours. After allowed to cool, the reaction solution was concentrated under reduced pressure, diluted with ethyl acetate, and then washed twice with a saturated aqueous sodium bicarbonate solution and once with a saturated saline solution. The organic layer was dried over sodium sulfate and then concentrated

under reduced pressure. To a solution of the obtained concentrated residue in tetrahydrofuran (80 mL) was added 2 M hydrochloric acid (40 mL), followed by stirring at room temperature for 2 hours. After tetrahydrofuran was distilled off under reduced pressure, the resultant solution was made basic with potassium carbonate, followed by extraction three times with chloroform. The combined extracts were dried over sodium sulfate and concentrated under reduced pressure. The obtained crude product was purified by silica gel column chromatography (0 to 5% methanol/chloroform) to yield a mixture of the title isomers A and B (2.95 g, 73%) as a slightly brown amorphous form.

$^1$H-NMR (400 MHz, $CDCl_3$)δ (ppm): 0.06 - 0.19 (m, 2H), 0.46 - 0.57 (m, 2H), 0.77 - 0.90 (m, 1H), 0.93 - 1.01 (m, 0.3H), 1.11 - 1.18 (m, 0.7H), 1.50 - 1.61 (m, 1H), 1.68 - 1.84 (m, 1H), 1.98 - 2.19 (m, 2.3H), 2.30 - 2.45 (m, 2H), 2.54 - 2.67 (m, 1.7H), 2.73 - 3.23 (m, 4H), 3.45 - 3.53 (m, 1H), 3.76 (s, 0.9H), 3.79 (s, 2.1H), 3.82 - 3.89 (m, 0.7H), 4.06 - 4.15 (m, 0.3H), 5.99 (br s, 0.7H), 6.20 (br s, 0.3H), 6.52 (d, J = 3 Hz, 0.3H), 6.68 - 6.72 (m, 1H), 6.96 (d, J = 8 Hz, 0.7H), 7.01 (d, J = 8 Hz, 0.3H), 7.14 - 7.16 (m, 0.7H).

(Example 2)

Synthesis of (5aS,6R, 11bS)-14-(cyclopropylmethyl)- 10-methoxy-2,3,4,5,6,7-hexahydro-6, 11b-(epiminoethano)naphtho[1,2-d]azepin-5a(1H)-ol (isomer C) and (5aS,6R,11bS)-14-(cyclopropylmethyl)-10-methoxy-2,3,4,5,6,7-hexahydro-6,11b-(epiminoethano)naphtho[1,2-c]azepin-5a(1H)-ol (isomer D)

[Chemical Formula 23]

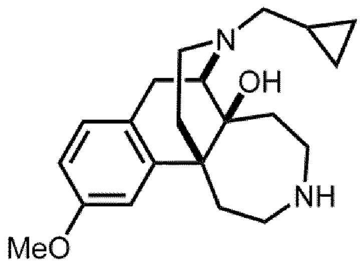

Isomer C

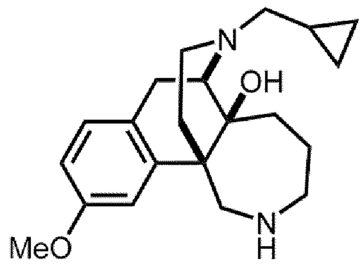

Isomer D

To a solution of the mixture of isomers A and B (662 mg, 1.86 mmol) obtained in Example 1 in tetrahydrofuran (8 mL) was added a 0.91 M borane-tetrahydrofuran complex-tetrahydrofuran solution (20.4 mL, 18.6 mmol), followed by heating under reflux for 14 hours. After allowed to cool, the reaction mixture was concentrated under reduced pressure. To the concentrated residue, 6 M hydrochloric acid (12 mL) was added, followed by heating under reflux for 16 hours. After allowed to cool, potassium carbonate was added to make the reaction mixture basic, followed by extraction three times with chloroform. The combined extracts were dried over sodium sulfate and then concentrated under reduced pressure. The obtained crude product was purified by silica gel column chromatography (amino group-supported silica gel, 0 to 10% methanol/chloroform) to individually yield the title isomer C (283 mg, 29%) and isomer D (236 mg, 24%) as colorless solids.

(Isomer C)

$^1$H-NMR (400 MHz, $CDCl_3$)δ (ppm): 0.06 - 0.15 (m, 2H), 0.46 - 0.55 (m, 2H), 0.80 - 0.87 (m, 1H), 1.04 - 1.11 (m, 1H), 1.57 (ddd, J = 4, 4, 14 Hz, 1H), 1.68 - 1.79 (m, 1H), 1.96 - 2.08 (m, 3H), 2.27 - 2.39 (m, 3H), 2.49 - 2.56 (m, 1H), 2.76 - 2.87 (m, 2H), 2.89 - 3.03 (m, 4H), 3.05 - 3.14 (m, 1H), 3.78 (s, 3H), 4.51 (br s, 1H), 6.69 - 6.74 (m, 2H), 7.02 (d, J = 8 Hz, 1H).

(Isomer D)

$^1$H-NMR (400 MHz, $CDCl_3$)δ (ppm): 0.06 - 0.16 (m, 2H), 0.45 - 0.56 (m, 2H), 0.77 - 0.91 (m, 1H), 0.94 - 1.01 (m, 1H), 1.46 - 1.72 (m, 3H), 1.91 (ddd, J = 5, 12, 12 Hz, 1H), 1, 98 (ddd, J = 3, 12, 12 Hz, 1H), 2.19 - 2.32 (m, 1H), 2.34 (dd, J = 7, 13 Hz, 1H), 2.38 (dd, J = 6, 13 Hz, 1H), 2.53 - 2.61 (m, 1H), 2.70 (ddd, J = 7, 9, 14 Hz, 1H), 2.80 (dd, J = 6, 18 Hz, 1H), 2.91 (d, J = 6 Hz, 1H), 2.94 - 3.02 (m, 1H), 2.98 (d, J = 18 Hz, 1H), 3.14 (d, J = 15 Hz, 1H), 3.18 (d, J = 15 Hz, 1H), 3.78 (s, 3H), 4.65 (br s, 1H), 6.73 (dd, J = 2, 8 Hz, 1H), 6.75 (d, J = 2 Hz, 1H), 7.03 (d, J = 8 Hz, 1H).

(Example 3)

Synthesis of (5aS,6R,11bS)-14-(cyclopropylmethyl)-2,3,4,5,6,7-hexahydro-6,11b-(epiminoethano)naphtho[1,2-d]azepine-5a,10(1H)-diol (compound E) and 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)ethan-1-one (compound F)

[Chemical Formula 24]

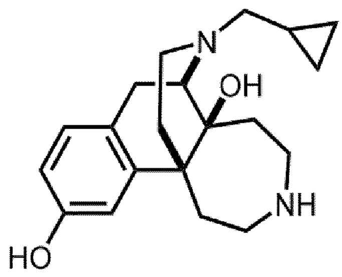

Compound E

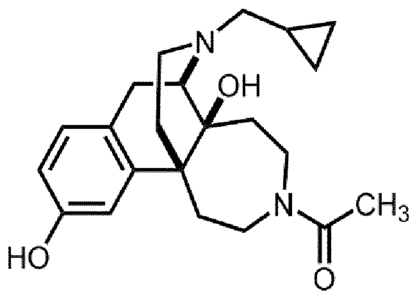

Compound F

To a solution of the isomer C (2.38 g, 6.9 mmol) obtained in Example 2 in N,N-dimethylacetamide (70 mL) were added 1-dodecanethiol (16.6 mL, 69 mmol) and potassium tert-butoxide (7.80 g, 69 mmol), followed by stirring at 160°C for 4 hours. After allowed to cool, the reaction solution was added to 1 M hydrochloric acid under ice cooling, followed by washing with heptane. Then, potassium carbonate was added to make the resultant solution basic, followed by extraction three times with a mixed solvent of chloroform/isopropanol (3/1). The combined extracts were dried over sodium sulfate and then concentrated under reduced pressure. The obtained crude product was purified by silica gel column chromatography (amino group-supported silica gel, 0 to 50% methanol/chloroform) to individually yield the title compound E (1.74 g, 76%) and compound F (606 mg, 24%) as colorless amorphous forms.

(Compound E)

$^1$H-NMR (400 MHz, $CDCl_3$)$\delta$ (ppm): 0.07 - 0.14 (m, 2H), 0.47 - 0.55 (m, 2H), 0.75 - 0.88 (m, 1H), 1.04 - 1.14 (m, 1H), 1.47 - 1.58 (m, 1H), 1.70 - 1.82 (m, 1H), 1.92 - 2.14 (m, 3H), 2.15 - 2.27 (m, 1H), 2.34 (d, J = 7 Hz, 2H), 2.48 - 2.59 (m, 1H), 2.67 - 2.86 (m, 3H), 2.89 (d, J = 6 Hz, 1H), 2.97 (d, J = 18 Hz, 1H), 3.07 (ddd, J = 4, 4, 14 Hz, 1H), 3.18 (ddd, J = 3, 13, 13 Hz, 1H), 4.54 (br s, 1H), 6.56 (d, J = 2 Hz, 1H), 6.63 (dd, J = 2, 8 Hz, 1H), 6.92 (d, J = 8 Hz, 1H).

(Compound F)

$^1$H-NMR (400 MHz, $CDCl_3$)$\delta$ (ppm): 0.04 - 0.18 (m, 2H), 0.45 - 0.58 (m, 2H), 0.75 - 2.41 (m, 13H), 2.49 - 3.07 (m, 5H), 3.22 - 3.98 (m, 2.3H), 4.35 (dd, J = 5, 15 Hz, 0.7H), 6.45 (dd, J = 3, 8 Hz, 0.3H), 6.60 (d, J = 3 Hz, 0.3H), 6.69 (dd, J = 3, 8 Hz, 0.7H), 6.86 (d, J = 8 Hz, 0.3H), 6.95 (d, J = 8 Hz, 0.7H), 7.00 (d, J = 2 Hz, 0.7H).

(Example 4)

Synthesis of ((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)(3-hydroxypyridin-2-yl)methanone

[Chemical Formula 25]

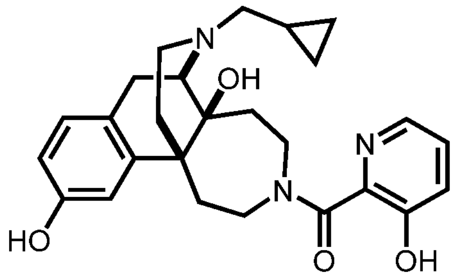

To a solution of the isomer C (12.2 mg, 0.036 mmol) obtained in Example 2 in N,N-dimethylformamide (1 mL) were added 3-hydroxypicolinic acid (14.9 mg, 0.11 mmol), N,N-dimethyl-4 aminopyridine (2.2 mg, 0.018 mmol), N,N-diisopropylethylamine (18.6 pL, 0.11 mmol), 1-hydroxybenzotriazole monohydrate (16.4 mg, 0.11 mmol), and 1-ethyl-3-(3-dimethylaminopropyl)carbodiimide (20.5 mg, 0.11 mmol), followed by stirring at room temperature for 3 hours. To the reaction mixture, a saturated aqueous sodium bicarbonate solution was added, followed by extraction once with ethyl acetate. The organic layer was washed with saturated saline and water, dried over sodium sulfate, and then concentrated under reduced pressure. To a solution of the obtained crude product (6 mg, 0.013 mmol) in chloroform (1 mL) was added a 1 M boron tribromide-dichloromethane solution (65 pL, 0.065 mmol) under ice cooling, followed by stirring at room temperature for 25 minutes. To the reaction mixture, a saturated sodium bicarbonate aqueous solution was added, followed by extraction three times with chloroform. The combined extracts were washed with saturated saline, dried over sodium sulfate, and then concentrated under reduced pressure. The obtained crude product was purified by preparative thin layer chromatography (chloroform : methanol = 5 : 1) to yield the title compound (4.5 mg, 28%) as a colorless solid.

$^1$H-NMR (400 MHz, $CD_3OD$)$\delta$ (ppm): 0.13 - 0.22 (m, 2H), 0.50 - 0.61 (m, 2H), 0.84 - 0.96 (m, 1H), 0.98 - 1.05 (m, 0.5H), 1.09 - 1.13 (m, 0.5H), 1.49 - 1.56 (m, 0.5H), 1.66 - 1.73 (m, 0.5H), 1.93 - 2.23 (m, 4H), 2.25 - 2.36 (m, 0.5H), 2.38 - 2.59 (m, 2.5H), 2.60 - 2.69 (m, 1H), 2.78 - 2.89 (m, 1H), 2.98 - 3.09 (m, 2H), 3.31 - 3.45 (m, 1.5H), 3.53 - 3.67 (m, 1H), 3.74 - 3.83 (m, 0.5H), 3.92 - 4.02 (m, 0.5H), 4.07 - 4.15 (m, 0.5H), 6.42 (d, J = 2 Hz, 0.5H), 6.58 (dd, J = 2, 8 Hz, 0.5H), 6.62 (dd, J = 2, 8 Hz, 0.5H), 6.68 (d, J = 2 Hz, 0.5H), 6.96 (d, J = 8 Hz, 0.5H), 6.98 (d, J = 8 Hz, 0.5H), 7.06 - 7.19 (m, 2H), 7.69 (d, J = 4 Hz, 0.5H), 7.74 (d, J = 4 Hz, 0.5H).

(Example 5)

Synthesis of ((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)(pyrimidin-2-yl)methanone

[Chemical Formula 26]

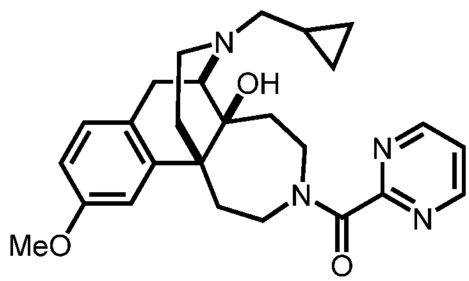

To a solution of the isomer C (15 mg, 0.044 mmol) obtained in Example 2 and pyrimidine-2-carboxylic acid (8.2 mg, 0.066 mmol) in N,N-dimethylformamide (1 mL) were added N,N-diisopropylethylamine (22 μL, 0.13 mmol) and O-(7-azabenzotriazol-1-yl)-N,N,N',N'-tetramethyluronium hexafluorophosphate (20 mg, 0.053 mmol), followed by stirring at room temperature for 2 hours. To the reaction mixture, a saturated aqueous sodium bicarbonate solution was added, followed by extraction three times with ethyl acetate. The combined extracts were dried over sodium sulfate and then concentrated under reduced pressure. The obtained crude product was purified by silica gel column chromatography (amino group-supported silica gel, 1 to 10% methanol/chloroform) to yield the title compound (16 mg, 80%) as a yellow

oily matter.

$^1$H-NMR (400 MHz, $CDCl_3$)δ (ppm): 0.03 - 0.18 (m, 2H), 0.41 - 0.58 (m, 2H), 0.73 - 0.91 (m, 1H), 0.98 - 1.16 (m, 1H), 1.21 - 1.80 (m, 1H), 1.84 - 2.12 (m, 4H), 2.16 - 2.63 (m, 4H), 2.71 - 3.07 (m, 3H), 3.22 - 3.52 (m, 2H), 3.60 - 3.83 (m, 3.5H), 3.86 - 3.93 (m, 1H), 4.24 - 4.34 (m, 0.5H), 6.36 (d, J = 2 Hz, 0.5H), 6.65 - 6.78 (m, 1.5H), 6.99 - 7.08 (m, 1H), 7.26 - 7.35 (m, 1H), 8.73 - 8.82 (m, 2H).

(Example 6)

Synthesis of ((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)(pyrimidin-2-yl)methanone

[Chemical Formula 27]

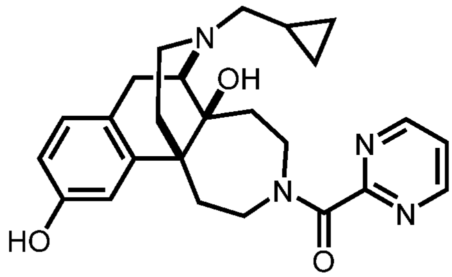

To a solution of the compound (16 mg, 0.035 mmol) in chloroform (1 mL) obtained in Example 5 was added a 1 M boron tribromide-dichloromethane solution (211 pL, 0.21 mmol) under ice cooling, followed by stirring at room temperature for 30 minutes. To the reaction mixture, a saturated aqueous sodium bicarbonate solution was added under ice cooling, followed by extraction three times with ethyl acetate. The combined extracts were dried over sodium sulfate and then concentrated under reduced pressure. The obtained crude product was purified by preparative thin layer chromatography (chloroform : 2 M ammonia-methanol solution = 10 : 1) to yield the title compound (11 mg, 71%) as a pale yellow oily matter.

$^1$H-NMR (400 MHz, $CDCl_3$)δ (ppm): 0.03 - 0.18 (m, 2H), 0.38 - 0.58 (m, 2H), 0.71 - 0.94 (m, 1H), 0.96 - 1.18 (m, 1H), 1.19 - 1.80 (m, 1H), 1.83 - 2.12 (m, 3.5H), 2.14 - 2.66 (m, 4.5H), 2.67 - 3.04 (m, 3H), 3.24 - 3.52 (m, 2H), 3.69 - 3.82 (m, 0.5H), 3.83 - 3.93 (m, 1H), 4.23 - 4.38 (m, 0.5H), 6.33 (d, J = 3 Hz, 0.5H), 6.67 - 6.69 (m, 1H), 6.78 - 6.84 (m, 0.5H), 6.91 - 7.00 (m, 1H), 7.28 - 7.34 (m, 1H), 8.75 - 8.83 (m, 2H).

(Example 7)

Synthesis of ((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)(oxazol-2-yl)methanone

[Chemical Formula 28]

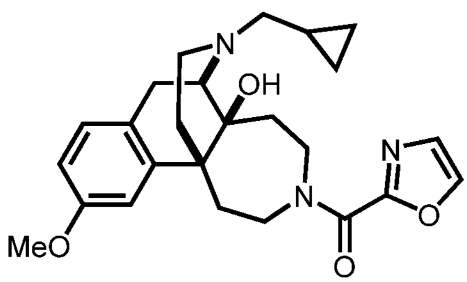

To the isomer C (13 mg, 0.038 mmol) obtained in Example 2 dissolved in N,N-dimethylformamide (1.0 mL) were added oxazole-2-carboxylic acid (7.0 mg, 0.062 mmol), N,N-diisopropylethylamine (33 μL, 0.19 mmol), 1-hydroxybenzotriazole monohydrate (10 mg, 0.076 mmol), 1-ethyl-3-(3-dimethylaminopropyl)carbodiimide hydrochloride (14 mg, 0.075 mmol), and N,N-dimethyl-4-aminopyridine (1.0 mg, 0.0080 mmol), followed by stirring at room temperature for 22 hours. To the reaction mixture, a saturated aqueous sodium bicarbonate solution was added under ice cooling to stop the reaction, followed by extraction with ethyl acetate. The combined extracts were washed with water and saturated saline, dried over sodium sulfate, and then concentrated under reduced pressure. The obtained crude product was purified by silica gel column chromatography (0 to 10% methanol/chloroform) to yield the title compound (12 mg, 74%) as a colorless oily matter.

$^1$H-NMR (400 MHz, $CDCl_3$)δ (ppm): 0.05 - 0.17 (m, 2H), 0.45 - 0.56 (m, 2H), 0.76 - 0.93 (m, 1H), 1.03 - 1.15 (m, 1H), 1.64 - 1.74 (m, 1H), 1.93 - 2.14 (m, 3.6H), 2.21 (dd, J = 5, 16 Hz, 0.4H), 2.28 - 2.68 (m, 4H), 2.83 (ddd, J = 6, 12, 18 Hz, 1H), 2.91 - 3.05 (m, 2H), 3.43 (dd, J = 12, 12 Hz, 0.4H), 3.64 (s, 1.8H), 3.67 - 3.84 (m, 1H), 3.79 (s, 1.2H), 3.96 - 4.04 (m, 0.6H), 4.06 - 4.23 (m, 2H), 6.46 - 6.55 (m, 0.6H)6.60 - 6.67 (m, 0.6H), 6.69 - 6.74 (m, 0.8H), 7.00 (d, J = 7 Hz, 0.6H), 7.02 (d, J = 8 Hz, 0.4H), 7.20 (s, 0.4H), 7.22 (s, 0.6H), 7.68 (s, 0.6H), 7.71 (s, 0.4H).

(Example 8)

Synthesis of ((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)(oxazol-2-yl)methanone

[Chemical Formula 29]

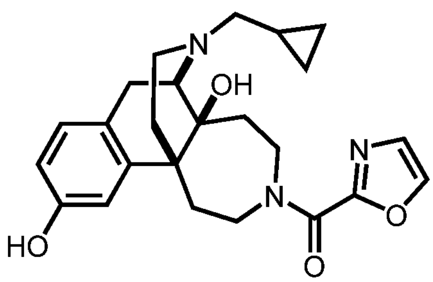

The title compound was obtained from the compound obtained in Example 7 according to the method described in Example 6.

$^1$H-NMR (400 MHz, $CDCl_3$)δ (ppm): 0.03 - 0.17 (m, 2H), 0.43 - 0.57 (m, 2H), 0.73 - 0.93 (m, 1H), 1.04 - 1.09 (m, 0.4H), 1.13 - 1.19 (m, 0.6H), 1.64 - 1.76 (m, 1H), 1.93 - 2.14 (m, 4H), 2.27 - 2.48 (m, 3H), 2.49 - 2.62 (m, 1H), 2.83 (dd, J = 6, 9 Hz, 0.6H), 2.78 (dd, J = 6, 9 Hz, 0.4H), 2.89 - 3.05 (m, 2H), 3.40 (dd, J = 12, 15 Hz, 0.6H), 3.72 - 3.81 (m, 0.8H), 3.98 (ddd, J = 4, 4, 14 Hz, 0.6H), 4.03 - 4.21 (m, 0.8H), 4.22 - 4.29 (m, 0.6H), 4.31 - 4.40 (m, 0.6H), 6.51 (d, J = 2 Hz, 0.4H), 6.57 (dd, J = 2, 8 Hz, 0.4H), 6.70 (dd, J = 2, 8 Hz, 0.6H), 6.93 (d, J = 8 Hz, 0.4H), 6.97 (d, J = 8 Hz, 0.6H), 7.00 (d, J = 2 Hz, 0.6H), 7.22 (s, 0.4H), 7.24 (s, 0.6H), 7.71 (s, 0.4H), 7.77 (s, 0.6H).

(Example 9)

Synthesis of ((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)(pyridin-3-yl)methanone

[Chemical Formula 30]

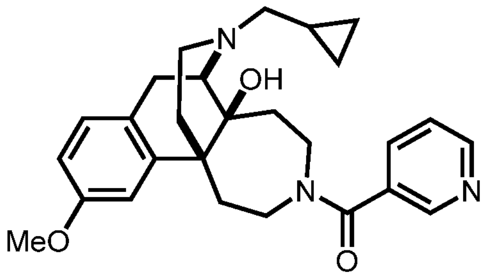

To a solution of the isomer C (9.5 mg, 0.029 mmol) obtained in Example 2 in tetrahydrofuran (1 mL) were added triethylamine (12.1 μL, 0.087 mmol) and nicotinic acid chloride hydrochloride (15.4 mg, 0.087 mmol), followed by stirring at room temperature for 30 minutes. To the reaction mixture, a saturated aqueous sodium bicarbonate solution was added, followed by extraction three times with ethyl acetate. The combined extracts were dried over sodium sulfate and then concentrated under reduced pressure. The obtained crude product was purified by preparative thin layer chromatography (chloroform : methanol = 10 : 1) to yield the title compound (12.8 mg, 99%) as a colorless solid.

$^1$H-NMR (400 MHz, $CDCl_3$)δ (ppm): 0.07 - 0.15 (m, 2H), 0.48 - 0.55 (m, 2H), 0.76 - 0.90 (m, 1H), 0.97 - 1.02 (m, 0.7H), 1.12 - 1.16 (m, 0.3H), 1.49 (ddd, J = 4, , 4, 15 Hz, 0.3H), 1.68 - 1.96 (m, 2.7H), 2.02 - 2.12 (m, 2H), 2.20 - 2.48 (m, 2.7H), 2.50 - 2.60 (m, 1H), 2.67 - 2.81 (m, 1H), 2.82 - 2.87 (m, 0.3H), 2.88 - 2.95 (m, 1H), 2.96 - 3.04 (m, 1H), 3.23 - 3.32 (m, 0.7H), 3.47 (s, 2.1H), 3.49 - 3.54 (m, 1.3H), 3.60 - 3.69 (m, 0.7H), 3.71 - 3.79 (m, 0.3H), 3.80 (s, 0.9H), 3.95 - 4.03 (m, 0.7H), 4.14 - 4.21 (m, 0.3H), 4.53 (br s, 1H), 6.20 (d, J = 3 Hz, 0.7H), 6.70 (dd, J = 3, 8 Hz, 0.7H), 6.72 - 6.77 (m, 0.6H), 7.03 (d, J = 8 Hz, 0.7H), 7.05 (d, J = 8 Hz, 0.3H), 7.15 - 7.25 (m, 1.4H), 7.28 - 7.32 (m, 0.3H), 7.71 (ddd, J = 2, 2, 8 Hz, 0.3H), 8.27 - 8.29 (m, 0.7H), 8.58 - 8.61 (m, 1.3H).

(Example 10)

Synthesis of ((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)(pyridin-3-yl)methanone

[Chemical Formula 31]

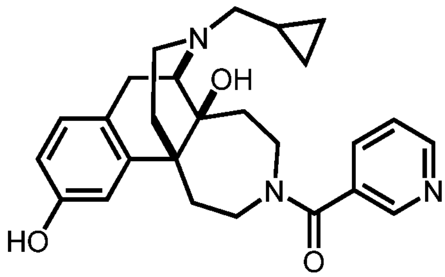

The title compound was obtained from the compound obtained in Example 9 according to the method described in Example 6.

$^1$H-NMR (400 MHz, $CDCl_3$)δ (ppm): 0.07 - 0.16 (m, 2H), 0.47 - 0.55 (m, 2H), 0.76 - 0.86 (m, 1H), 0.97 - 1.04 (m, 0.7H), 1.13 - 1.18 (m, 0.3H), 1.49 (ddd, J = 3, 3, 15 Hz, 0.3H), 1.69 - 1.77 (m, 1.4H), 1.88 - 2.16 (m, 3.3H), 2.20 - 2.41 (m, 2.7H), 2.49 - 2.59 (m, 1H), 2.70 - 2.87 (m, 1.3H), 2.87 - 2.94 (m, 1H), 2.95 - 3.04 (m, 1H), 3.25 - 3.33 (m, 0.7H), 3.38 - 3.47 (m, 1.3H), 3.61 - 3.80 (m, 1H), 4.05 - 4.11 (m, 1H), 4.58 (br s, 1H), 6.19 (d, J = 2 Hz, 0.7H), 6.67 (dd, J = 2, 8 Hz, 0.7H), 6.68 - 6.70 (m, 0.3H), 6.85 (d, J = 2 Hz, 0.3H), 6.96 (d, J = 8 Hz, 0.7H), 6.97 (d, J = 8 Hz, 0.3H), 7.29 - 7.34 (m, 1H), 7.41 (ddd, J = 2, 2, 8 Hz, 0.7H), 7.73 (ddd, J = 2, 2, 8 Hz, 0.3H), 8.01 (d, J = 1 Hz, 0.7 Hz), 8.50 (dd, J = 1, 5 Hz, 0.7H), 8.59 - 8.62 (m, 0.6H).

(Example 11)

Synthesis of ((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)(imidazo[1,2-a]pyrimidin-3-yl)methanone

[Chemical Formula 32]

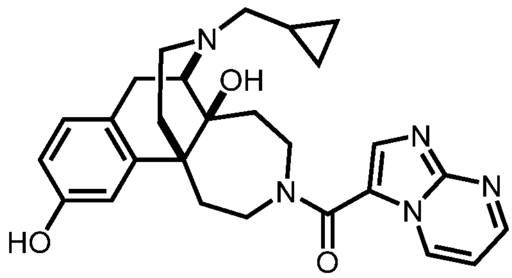

The title compound was obtained from the compound E obtained in Example 3 and imidazo[1,2-a]pyridine-3-carboxylic acid according to the method described in Example 5.

$^1$H-NMR (400 MHz, $CDCl_3$)δ (ppm): 0.05 - 0.19 (m, 2H), 0.44 - 0.61 (m, 2H), 0.76 - 0.93 (m, 1H), 1.02 - 1.19 (m, 1H), 1.58 - 1.78 (m, 2H), 1.90 - 2.14 (m, 3H), 2.24 - 2.44 (m, 3H), 2.47 - 2.59 (m, 1H), 2.81 - 3.08 (m, 4H), 3.39 - 3.56 (m, 1H), 3.73 - 3.87 (m, 1H), 3.91 - 4.05 (m, 1H), 4.70 - 4.93 (m, 1H), 6.38 - 6.50 (m, 1H), 6.59 (s, 1H), 6.86 - 7.02 (m, 2H), 7.68 (s, 1H), 8.26 - 8.39 (m, 1H), 8.61 (s, 1H).

(Example 12)

Synthesis of (5aS,6R,11bS)-3-benzyl-14-(cyclopropylmethyl)-10-methoxy-2,3,4,5,6,7-hexahydro-6,11b-(epiminoethano)naphtho[1,2-d]azepin-5a(1H)-ol

[Chemical Formula 33]

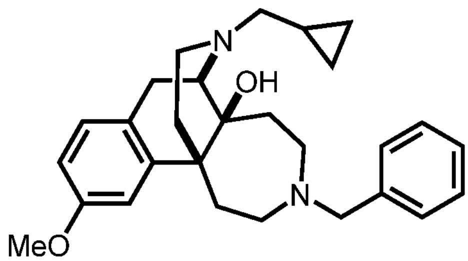

To a solution of the isomer C (5 mg, 0.015 mmol) obtained in Example 2 and benzaldehyde (5 μL, 0.045 mmol) in dichloromethane (0.5 mL) was added sodium triacetoxyborohydride (13 mg, 0.06 mmol) under ice cooling, followed by stirring at room temperature for 16 hours. The reaction solution was diluted with ethyl acetate, washed with a saturated aqueous sodium bicarbonate solution, water, and saturated saline, dried over sodium sulfate, and then concentrated under reduced pressure. The obtained crude product was purified by preparative thin layer chromatography (chloroform : 2 M ammonia-methanol solution = 90 : 10) to yield the title compound (5 mg, 77%) as a colorless amorphous form.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.03 (d, J = 13 Hz, 1H), 1.45 - 1.55 (m, 1H), 1.80 - 1.90 (m, 2H), 1.99 (ddd, J = 3, 11, 12 Hz, 1H), 2.10 (ddd, J = 5, 13, 13 Hz, 1H), 2.30 - 2.70 (m, 6H), 2.75 - 2.90 (m, 2H), 2.90 - 3.00 (m, 2H), 3.00 - 3.10 (m, 1H), 3.54 (s, 2H), 3.68 (s, 3H), 4.80 (br s, 1H), 6.61 (d, J = 2 Hz, 1H), 6.71 (dd, J = 2, 8 Hz, 1H), 7.00 - 7.10 (m, 3H), 7.15 - 7.25 (m, 3H).

(Example 13)

Synthesis of (5aS,6R,11bS)-3-benzyl-14-(cyclopropylmethyl)-2,3,4,5,6,7-hexahydro-6,11b-(epiminoethano)naphtho[1,2-d]azepine-5a,10(1H)-diol dihydrochloride

[Chemical Formula 34]

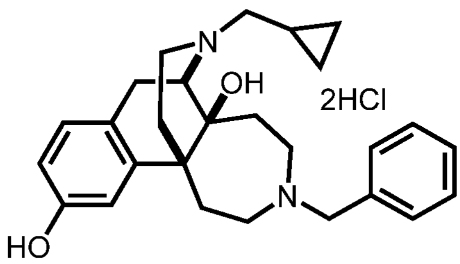

A free form of the title compound (3.4 mg) was obtained as a colorless amorphous form from the compound obtained in Example 12 according to the method described in Example 6. To the obtained free form dissolved in methanol (0.5 mL), a 1 M hydrochloric acid-diethyl ether solution (50 μL) was added, followed by concentration under reduced pressure. To the obtained concentrated residue, water (0.2 mL) was added, and the resultant solution was frozen, followed by freeze-drying, to yield the title compound (4.0 mg, 54%) as a white solid.

$^1$H-NMR (400 MHz, $CD_3OD$)δ (ppm): 0.40 - 0.60 (m, 2H), 0.70 - 1.00 (m, 4H), 1.00 - 1.15 (m, 1H), 1.20 - 1.45 (m, 2H), 1.80 - 2.70 (m, 4H), 2.80 - 3.00 (m, 1H), 3.00 - 3.60 (m, 6H), 3.60 - 3.90 (m, 2H), 4.10 - 4.50 (m, 2H), 6.61 (s, 1H), 6.77 (d, J = 8 Hz, 1H), 7.13 (d, J = 8 Hz, 1H), 7.40 - 7.60 (m, 5H).

(Example 14)

Synthesis of (5aS,6R,11bS)-14-(cyclopropylmethyl)-10-methoxy-3-(pyridin-2-ylmethyl)-2,3,4,5,6,7-hexahydro-6,11b-(epiminoethano)naphtho[1,2-d]azepin-5a(1H)-ol

[Chemical Formula 35]

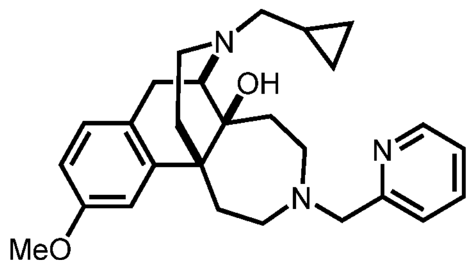

The title compound was obtained from the isomer C obtained in Example 2 and picolinaldehyde according to the method described in Example 12.

$^1$H-NMR (400 MHz, $CDCl_3$)δ (ppm): 0.03 - 0.17 (m, 2H), 0.44 - 0.56 (m, 2H), 0.78 - 0.91 (m, 1H), 1.01 - 1.09 (m, 1H), 1.51 (ddd, J = 2, 5, 14 Hz, 1H), 1.81 - 1.97 (m, 2H), 1.98 - 2.03 (m, 1H), 2.10 (ddd, J = 5, 13, 13 Hz, 1H), 2.34 (dd, J = 6.13 Hz, 1H), 2.36 (dd, J = 6, 13 Hz, 1H), 2.44 - 2.59 (m, 3H), 2.68 (ddd, J = 4, 4, 13 Hz, 1H), 2.79 - 2.95 (m, 2H), 2.95 (d, J = 6 Hz, 1H), 2.99 (d, J = 18 Hz, 1H), 3.19 (ddd, J = 2, 12, 14 Hz, 1H), 3.68 (s, 3H), 3.70 (d, J = 14 Hz, 1H), 3.74 (d, J = 14 Hz, 1H), 6.62 (d, J = 2 Hz, 1H), 6.72 (dd, J = 2, 8 Hz, 1H), 6.92 (d, J = 8 Hz, 1H), 7.02 - 7.06 (m, 1H), 7.07 (ddd, J = 1, 5, 7 Hz, 1H), 7.47 (ddd, J = 2, 7, 7 Hz, 1H), 8.45 (ddd, J = 1, 2, 5 Hz, 1H).

(Example 15)

Synthesis of (5aS,6R,11bS)-14-(cyclopropylmethyl)-3-(pyridin-2-ylmethyl)-2,3,4,5,6,7-hexahydro-6,11b-(epiminoethano)naphtho[1,2-d]azepine-5a,10(1H)-diol

[Chemical Formula 36]

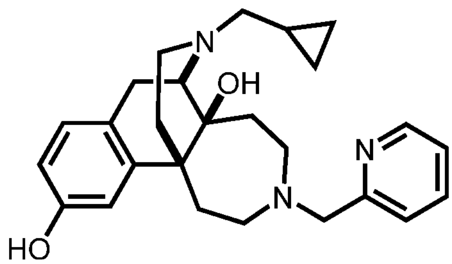

The title compound was obtained from the compound obtained in Example 14 according to the method described in Example 6.

$^1$H-NMR (400 MHz, $CDCl_3$)$\delta$ (ppm): 0.03 - 0.15 (m, 2H), 0.42 - 0.57 (m, 2H), 0.76 - 0.89 (m, 1H), 0.91 - 1.00 (m, 1H), 1.46 (ddd, J = 2, 5, 14 Hz, 1H), 1.53 (ddd, J = 2, 5, 16 Hz, 1H), 1.85 (ddd, J = 3, 12, 14 Hz, 1H), 1.93 - 2.07 (m, 2H), 2.28 - 2.40 (m, 3H), 2.47 - 2.81 (m, 5H), 2.88 (d, J = 6 Hz, 1H), 2.93 (d, J = 18 Hz, 1H), 3.22 (ddd, J = 1, 11, 13 Hz, 1H), 3.76 (s, 2H), 4.73 (br s, 1H), 6.25 (d, J = 2 Hz, 1H), 6.56 (dd, J = 2, 8 Hz, 1H), 6.90 (d, J = 8 Hz, 1H), 7.12 (ddd, J = 1, 5, 8 Hz, 1H), 7.16 - 7.21 (m, 1H), 7.57 (ddd, J = 2, 8, 8 Hz, 1H), 8.48 (ddd, J = 1, 2, 5 Hz, 1H).

Synthesis of (5aS,6R,11bS)-14-(cyclopropylmethyl)-10-methoxy-3-(pyridin-4-ylmethyl)-2,3,4,5,6,7-hexahydro-6,11b-(epiminoethano)naphtho[1,2-d]azepin-5a(1H)-ol

[Chemical Formula 37]

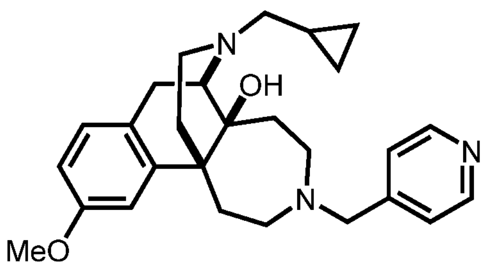

The title compound was obtained from the isomer C obtained in Example 2 and isonicotinaldehyde according to the method described in Example 12.

$^1$H-NMR (400 MHz, $CDCl_3$)$\delta$ (ppm): 0.06 - 0.17 (m, 2H), 0.45 - 0.56 (m, 2H), 0.78 - 0.91 (m, 1H), 1.02 - 1.10 (m, 1H), 1.49 (ddd, J = 2, 5, 14 Hz, 1H), 1.77 - 1.84 (m, 1H), 1.88 (ddd, J = 3, 12, 15 Hz, 1H), 1.99 (ddd, J = 2, 13, 13 Hz, 1H), 2.03 (ddd, J = 4, 13, 13 Hz, 1H), 2.35 (dd, J = 6, 13 Hz, 1H), 2.35 (dd, J = 6, 13 Hz, 1H), 2.43 (ddd, J = 4, 4, 13 Hz, 1H), 2.47 - 2.60 (m, 3H), 2.85 - 2.95 (m, 1H), 2.86 (dd, J = 6, 18 Hz, 1H), 2.94 (d, J = 6 Hz, 1H), 3.00 (d, J = 18 Hz, 1H), 3.11 (ddd, J = 1, 11, 13 Hz, 1H), 3.51 (d, J = 15 Hz, 1H), 3.56 (d, J = 15 Hz, 1H), 3.69 (s, 3H), 6.61 (d, J = 3 Hz, 1H), 6.75 (dd, J = 3, 8 Hz, 1H), 6.86 - 6.91 (m, 2H), 7.06 (d, J = 8 Hz, 1H), 8.35 - 8.41 (m, 2H).

(Example 17)

Synthesis of (5aS,6R,11bS)-14-(cyclopropylmethyl)-3-(pyridin-4-ylmethyl)-2,3,4,5,6,7-hexahydro-6,11b-(epiminoethano)naphtho[1,2-d]azepine-5a,10(1H)-diol

[Chemical Formula 38]

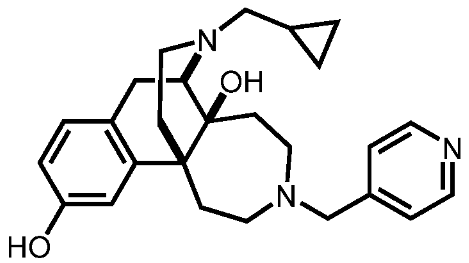

The title compound was obtained from the compound obtained in Example 16 according to the method described in Example 6.

$^1$H-NMR (400 MHz, $CDCl_3$)δ (ppm): 0.04 - 0.17 (m, 2H), 0.44 - 0.57 (m, 2H), 0.78 - 0.88 (m, 1H), 1.00 - 1.07 (m, 1H), 1.54 (dd, J = 4, 14 Hz, 1H), 1.65 (ddd, J = 2, 5, 16 Hz, 1H), 1.98 (ddd, J = 3, 12, 14 Hz, 1H), 1.99 - 2.10 (m, 2H), 2.32 - 2.40 (m, 1H), 2.33 (dd, J = 6, 13 Hz, 1H), 2.36 (dd, J = 6, 13 Hz, 1H), 2.45 - 2.61 (m, 3H), 2.81 - 2.91 (m, 2H), 2.94 (d, J = 6 Hz, 1H), 2.97 (d, J = 18 Hz, 1H), 3.19 (dd, J = 12, 12 Hz, 1H), 3.52 (d, J = 15 Hz, 1H), 3.66 (d, J = 15 Hz, 1H), 6.48 (d, J = 2 Hz, 1H), 6.70 (dd, J = 2, 8 Hz, 1H), 6.84 - 6.91 (m, 2H), 6.96 (d, J = 8 Hz, 1H), 8.30 - 8.42 (m, 2H).

(Example 18)

Synthesis of (5aS,6R,11bS)-14-(cyclopropylmethyl)-10-methoxy-3-(pyrimidin-2-ylmethyl)-2,3,4,5,6,7-hexahydro-6,11b-(epiminoethano)naphtho[1,2-d]azepin-5a(1H)-ol

[Chemical Formula 39]

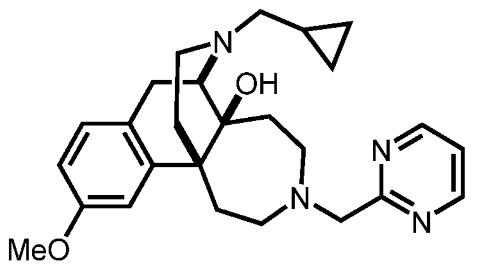

The title compound was obtained from the isomer C obtained in Example 2 and pyrimidine-2-carbaldehyde according to the method described in Example 12.

$^1$H-NMR (400 MHz, $CDCl_3$)δ (ppm): 0.03 - 0.16 (m, 2H), 0.42 - 0.56 (m, 2H), 0.79 - 0.92 (m, 1H), 0.98 - 1.10 (m, 1H), 1.48 (ddd, J = 2, 5, 14 Hz, 1H), 1.84 - 2.03 (m, 3H), 2.12 (ddd, J = 5, 13, 13 Hz, 1H), 2.33 - 2.44 (m, 3H), 2.56 (ddd, J = 2, 5, 12 Hz, 1H), 2.73 - 2.84 (m, 2H), 2.85 - 3.02 (m, 4H), 3.32 (ddd, J = 3, 11, 13 Hz, 1H), 3.75 (s, 3H), 3.92 (d, J = 15 Hz, 1H), 3.96 (d, J = 15 Hz, 1H), 6.63 (d, J = 3 Hz, 1H), 6.69 (dd, J = 3, 8 Hz, 1H), 7.00 (d, J = 8 Hz, 1H), 7.13 (t, J = 5 Hz, 1H), 8.68 (d, J = 5 Hz, 2H).

(Example 19)

Synthesis of (5aS,6R,11bS)-14-(cyclopropylmethyl)-3-(pyrimidin-2-ylmethyl)-2,3,4,5,6,7-hexahydro-6,11b-(epiminoethano)naphtho[1,2-d]azepine-5a,10(1H)-diol

[Chemical Formula 40]

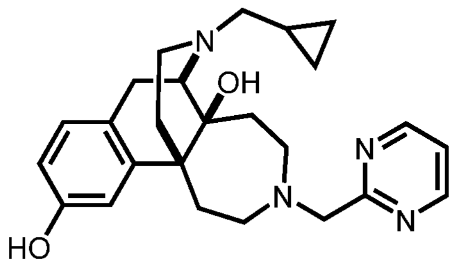

The title compound was obtained from the compound obtained in Example 18 according to the method described in Example 6.

$^1$H-NMR (400 MHz, $CDCl_3$)δ (ppm): 0.05 - 0.14 (m, 2H), 0.46 - 0.53 (m, 2H), 0.78 - 0.92 (m, 1H), 0.96 - 1.03 (m, 1H), 1.47 (ddd, J = 2, 5, 15 Hz, 1H), 1.75 (dd, J = 5, 15 Hz, 1H), 1.87 (ddd, J = 3, 11, 14 Hz, 1H), 1.98 (ddd, J = 3, 12, 12 Hz, 1H), 2.08 (ddd, J = 4, 12, 12 Hz, 1H), 2.29 - 2.40 (m, 3H), 2.55 (dd, J = 3, 11 Hz, 1H), 2.74 (dd, J = 5, 18 Hz, 1H), 2.80 - 2.98 (m, 5H), 3.32 (ddd, J = 2, 11, 13 Hz, 1H), 3.95 (d, J = 14 Hz, 1H), 4.00 (d, J = 14 Hz, 1H), 6.44 (d, J = 3 Hz, 1H), 6.57 (dd, J = 3, 8 Hz, 1H), 6.89 (d, J = 8 Hz, 1H), 7.15 (t, J = 5 Hz, 1H), 8.69 (d, J = 5 Hz, 2H).

(Example 20)

Synthesis of 1-((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(pyrimidin-2-yl)ethan-1-one

[Chemical Formula 41]

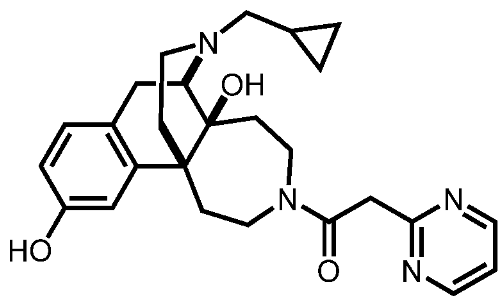

The title compound was obtained from the isomer C obtained in Example 2 and 2-(pyrimidin-2-yl)acetic acid according to the method described in Example 4.

$^1$H-NMR (400 MHz, $CDCl_3$)δ (ppm): 0.05 - 0.15 (m, 2H), 0.47 - 0.55 (m, 2H), 0.76 - 0.92 (m, 1H), 1.00 - 1.13 (m, 1H), 1.53 - 1.66 (m, 1H), 1.81 (ddd, J = 3, 12, 15 Hz, 0.4H), 1.86 - 2.10 (m, 3.6H), 2.18 - 2.24 (m, 1H), 2.26 - 2.40 (m, 2H), 2.50 - 2.63 (m, 1H), 2.68 - 3.09 (m, 2.6H), 2.74 (ddd, J = 6, 16, 16 Hz, 1H), 3.33 - 3.45 (m, 1.4H), 3.63 - 3.76 (m, 0.4H), 3.84 (d, J = 15 Hz, 0.4H), 3.90 - 4.01 (m, 1H), 3.96 (d, J = 15 Hz, 0.4H), 4.11 (s, 1.2H), 4.26 - 4.35 (m, 0.6H), 4.48 (br s, 1H), 6.57 - 6.64 (m, 1.4H), 6.83 (d, J = 2 Hz, 0.6H), 6.91 (d, J = 8 Hz, 1H), 7.17 (t, J = 5 Hz, 0.4H), 7.18 (t, J = 5 Hz, 0.6H), 8.67 (d, J = 5 Hz, 0.8H), 8.70 (d, J = 5 Hz, 1.2H).

(Example 21)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-2-(pyridin-2-yl)ethan-1-one

[Chemical Formula 42]

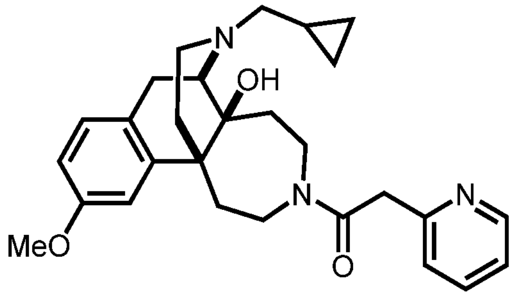

The title compound was obtained from the isomer C obtained in Example 2 and 2-(pyridin-2-yl)acetic acid hydrochloride according to the method described in Example 7.

$^1$H-NMR (400 MHz, $CDCl_3$)$\delta$ (ppm): 0.03 - 0.16 (m, 2H), 0.43 - 0.56 (m, 2H), 0.76 - 0.87 (m, 1H), 1.00 - 1.12 (m, 1H), 1.55 (ddd, J = 3, 4, 15 Hz, 0.5H), 1.63 (ddd, J = 2, 5, 15 Hz, 0.5H), 1.75 (ddd, J = 4, 12, 14 Hz, 0.5H), 1.82 (ddd, J = 2, 12, 14 Hz, 0.5H), 1.92 - 2.11 (m, 2.5H), 2.12 (ddd, J = 2, 5, 16 Hz, 0.5H), 2.24 - 2.40 (m, 2.5H), 2.46 (ddd, J = 6, 10, 16 Hz, 0.5H), 2.51 - 2.60 (m, 1H), 2.70 (dd, J = 6, 18 Hz, 0.5H), 2.80 (dd, J = 6, 18 Hz, 0.5H), 2.86 (d, J = 6 Hz, 0.5H), 2.92 (d, J = 6 Hz, 0.5H), 2.96 (d, J = 18 Hz, 0.5H), 2.98 (d, J = 18 Hz, 0.5H), 3.23 (ddd, J = 2, 12, 14 Hz, 0.5H), 3.48 (ddd, J = 4, 4, 13 Hz, 0.5H), 3.53 (ddd, J = 2, 12, 14 Hz, 0.5H), 3.57 - 3.67 (m, 1H), 3.68 (d, J = 15 Hz, 0.5H), 3.76 (s, 1.5H), 3.78 (s, 1.5H), 3.80 - 3.94 (m, 2.5H), 4.10 (ddd, J = 3, 6, 15 Hz, 0.5H), 4.47 (br s, 1H), 6.63 (d, J = 2 Hz, 0.5H), 6.66 - 6.73 (m, 1.5H), 6.99 (d, J = 9 Hz, 0.5H), 7.01 (d, J = 9 Hz, 0.5H), 7.09 - 7.15 (m, 1.5H), 7.18 - 7.22 (m, 0.5H), 7.55 (ddd, J = 2, 6, 8 Hz, 0.5H), 7.57 (ddd, J = 2, 6, 8 Hz, 0.5H), 8.49 (ddd, J = 1, 2, 5 Hz, 0.5H), 8.49 (ddd, J = 1, 2, 5 Hz, 0.5H).

(Example 22)

Synthesis of 1-((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(pyridin-2-yl)ethan-1-one

[Chemical Formula 43]

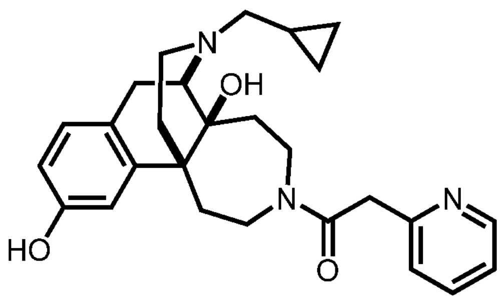

The title compound was obtained from the compound obtained in Example 21 according to the method described in Example 6.

$^1$H-NMR (400 MHz, $CDCl_3$)$\delta$ (ppm): 0.06 - 0.15 (m, 2H), 0.43 - 0.56 (m, 2H), 0.75 - 0.88 (m, 1H), 1.00 - 1.12 (m, 1H), 1.53 (ddd, J = 4, 4, 15 Hz, 0.5H), 1.59 (ddd, J = 1, 5, 15 Hz, 0.5H), 1.72 - 2.07 (m, 3.5H), 2.13 (ddd, J = 2, 6, 16 Hz, 0.5H), 2.20 - 2.39 (m, 2.5H), 2.47 (dd, J = 6, 12 Hz, 0.5H), 2.49 - 2.58 (m, 1H), 2.65 (dd, J = 6, 18 Hz, 0.5H), 2.76 (dd, J = 6, 19 Hz, 0.5H), 2.83 (d, J = 6 Hz, 0.5H), 2.90 (d, J = 6 Hz, 0.5H), 2.92 (d, J = 19 Hz, 0.5H), 2.95 (d, J = 18 Hz, 0.5H), 3.28 (ddd, J = 2, 12, 14 Hz, 0.5H), 3.32 - 3.43 (m, 1H), 3.48 (ddd, J = 3, 3, 14 Hz, 0.5H), 3.69 (d, J = 15 Hz, 0.5H), 3.72 - 3.81 (m, 0.5H), 3.78 (d, J = 15 Hz, 0.5H), 3.82 - 3.94 (m, 1H), 3.88 (d, J = 15 Hz, 0.5H), 3.93 (d, J = 15 Hz, 0.5H), 4.08 (ddd, J = 2, 5, 15 Hz, 0.5H), 4.44 (br s, 0.5H), 4.51 (br s, 0.5H), 6.63 (dd, J = 2, 8 Hz, 0.5H), 6.64 (dd, J = 2, 8 Hz, 0.5H), 6.71 (d, J = 2 Hz, 0.5H), 6.81 (d, J = 2 Hz, 0.5H), 6.89 (d, J = 8 Hz, 0.5H), 6.90 (d, J = 8 Hz, 0.5H), 7.13 (ddd, J = 1, 5, 8 Hz, 0.5H), 7.15 - 7.21 (m, 1H), 7.22 - 7.26 (m, 0.5H), 7.57 (ddd, J = 2, 8, 8 Hz, 0.5H), 7.62 (ddd, J = 2, 8, 8 Hz, 0.5H), 8.46 - 8.50 (m, 1H).

(Example 23)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-2-(thiophen-2-yl)ethan-1-one

[Chemical Formula 44]

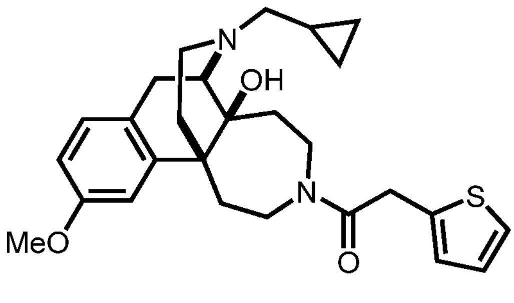

The title compound was obtained from the isomer C obtained in Example 2 and 2-(thiophen-2-yl)acetic acid according to the method described in Example 7.

$^1$H-NMR (400 MHz, $CD_3Cl$)$\delta$ (ppm): 0.06 - 0.17 (m, 2H), 0.45 - 0.55 (m, 2H), 0.76 - 0.89 (m, 1H), 1.01 - 1.13 (m, 1H), 1.57 - 1.67 (m, 1H), 1.76 - 1.85 (m, 1H), 1.92 - 2.40 (m, 6H), 2.52 - 2.61 (m, 1.5H), 2.68 - 3.03 (m, 2.5H), 3.11 - 3.18 (m, 0.5H), 3.36 - 3.53 (m, 1.5H), 3.60 (dd, J = 1, 16 Hz, 0.5H), 3.68 - 3.82 (m, 1H), 3.78 (s, 3H), 3.86 - 3.95 (m, 2H), 4.11 - 4.20 (m, 0.5H), 4.50 (br s, 1H), 6.64 (d, J = 3 Hz, 0.5H), 6.68 - 6.74 (m, 2H), 6.78 - 6.80 (m, 0.5H), 6.87 - 6.91 (m, 1H), 6.99 - 7.02 (m, 1H), 7.13 - 7.17 (m, 1H).

(Example 24)

Synthesis of (5aS,6R,11bS)-14-(cyclopropylmethyl)-10-methoxy-3-(2-(thiophen-2-yl)ethyl)-2,3,4,5,6,7-hexahydro-6,11b-(epiminoethano)naphtho[1,2-d]azepin-5a(1H)-ol

[Chemical Formula 45]

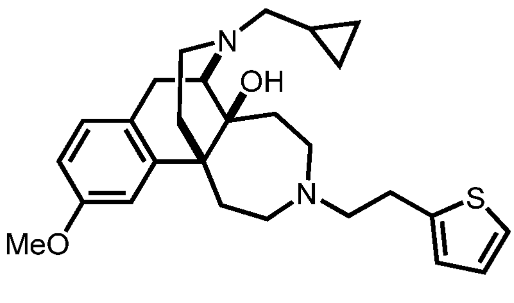

To a solution of the compound (24.3 mg, 0.052 mmol) obtained in Example 23 in tetrahydrofuran (3 mL) was added a 0.9 M borane-tetrahydrofuran complex-tetrahydrofuran solution (578.6 $\mu$L, 0.52 mmol), followed by heating under reflux for 2 hours. After allowed to cool, the reaction mixture was concentrated under reduced pressure. To the concentrated residue, 2 M hydrochloric acid (3 mL) was added, followed by heating under reflux for 2 hours. After allowed to cool, the reaction mixture was poured into a saturated aqueous sodium bicarbonate solution to be basic, followed by extraction three times with ethyl acetate. The combined extracts were dried over sodium sulfate and then concentrated under reduced pressure. The obtained crude product was purified by silica gel column chromatography (amino group-supported silica gel, 0 to 2% methanol/ethyl acetate) to yield the title compound (21.1 mg, 89%) as a colorless oily matter.

$^1$H-NMR (400 MHz, $CD_3Cl$)$\delta$ (ppm): 0.05 - 0.16 (m, 2H), 0.44 - 0.58 (m, 2H), 0.78 - 0.92 (m, 1H), 1.05 - 1.08 (m, 1H), 1.45 - 1.54 (m, 1H), 1.76 - 2.14 (m, 5H), 2.31 - 2.42 (m, 3H), 2.48 - 2.60 (m, 2H), 2.67 - 2.79 (m, 3H), 2.80 - 3.02 (m, 5H), 3.12 - 3.21 (m, 1H), 3.77 (s, 3H), 4.67 (br s, 1H), 6.66 - 6.74 (m, 3H), 6.82 - 6.87 (m, 1H), 6.97 - 7.06 (m, 2H).

(Example 25)

Synthesis of (5aS,6R,11bS)-14-(cyclopropylmethyl)-3-(2-(thiophen-2-yl)ethyl)-2,3,4,5,6,7-hexahydro-6,11b-(epiminoethano)naphtho[1,2-d]azepine-5a,10(1H)-diol

[Chemical Formula 46]

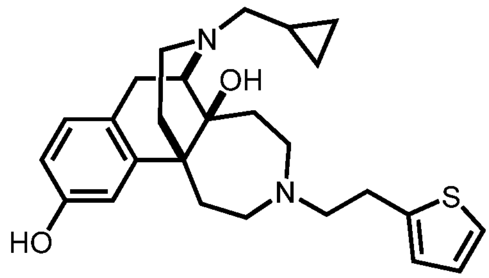

The title compound was obtained from the compound obtained in Example 24 according to the method described in Example 6.

$^1$H-NMR (400 MHz, $CD_3Cl$)δ (ppm): 0.04 - 0.15 (m, 2H), 0.45 - 0.53 (m, 2H), 0.78 - 0.90 (m, 1H), 0.99 - 1.07 (m, 1H), 1.47 - 1.54 (d, J = 4, 15 Hz, 1H), 1.77 - 1.87 (m, 2H), 1.97 - 2.08 (m, 2H), 2.30 - 2.39 (m, 3H), 2.48 - 2.58 (m, 1H), 2.68 - 3.08 (m, 10H), 3.21 (dd, J = 11, 11 Hz, 1H), 4.82 (br s, 1H), 6.42 (d, J = 2 Hz, 1H), 6.56 (dd, J = 2, 8 Hz, 1H), 6.77 (d, J = 3 Hz, 1H), 6.87 - 6.91 (m, 2H), 7.09 (dd, J = 1, 5 Hz, 1H).

(Example 26)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(1H-pyrazol-3-yl)ethan-1-one

[Chemical Formula 47]

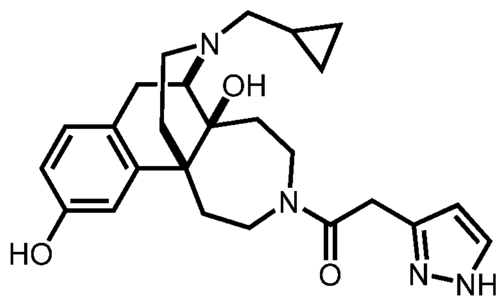

To a solution of the compound E (20 mg, 0.061 mmol) obtained in Example 3, 2-(1H-pyrazol-1-yl)acetic acid (12 mg, 0.17 mmol) and N,N-diisopropylethylamine (31 pL, 0.18 mmol) in N,N-dimethylformamide (0.5 mL) was added O-(7-azabenzotriazol-3-yl)-N,N,N',N'-tetramethyluronium hexafluorophosphate (24 mg, 0.064 mmol) under ice cooling, followed by stirring at room temperature for 16 hours. To the reaction mixture were added potassium carbonate (83 mg, 0.61 mmol) and methanol (1 mL), followed by further stirring for 1 hour. The reaction solution was diluted with ethyl acetate, washed with a saturated aqueous sodium bicarbonate solution, water, and saturated saline, dried over sodium sulfate, and then concentrated under reduced pressure. The obtained crude product was purified by preparative thin layer chromatography (chloroform : methanol = 10 : 1) to yield the title compound (27 mg, 100%) as a white solid.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.01 (d, J = 10 Hz, 0.5H), 1.10 (d, J = 13 Hz, 0.5H), 1.50 - 2.40 (m, 7.5H), 2.40 - 2.60 (m, 1.5H), 2.72 (ddd, J = 4, 12, 12 Hz, 1H), 2.80 - 3.00 (m, 2H), 3.28 (t, J = 12 Hz, 1H), 3.37 (t, J = 12 Hz, 0.5H), 3.45 - 3.55 (m, 1H), 3.60 - 4.00 (m, 3.5H), 6.11 (s, 1H), 6.55 - 6.70 (m, 1.5H), 6.76 (s, 0.5H), 6.87 (d, J = 8 Hz, 0.5H), 6.91 (d, J = 8 Hz, 0.5H), 7.44 (s, 0.5H), 7.49 (s, 0.5H).

(Example 27)

Synthesis of (5aS,6R,11bS)-3-(2-(1H-pyrazol-3-yl)ethyl)-14-(cyclopropylmethyl)-2,3,4,5,6,7-hexahydro-6,11b-(epiminoethano)naphtho[1,2-d]azepine-5a,10(1H)-diol

[Chemical Formula 48]

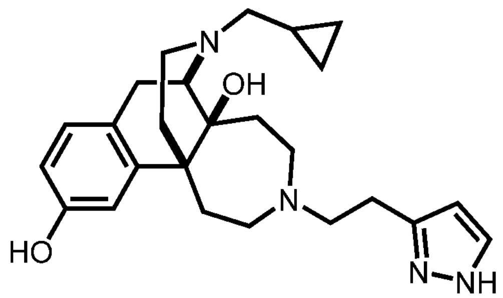

The title compound was obtained from the compound obtained in Example 26 according to the method described in Example 24.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.00 - 1.15 (m, 1H), 1.20 - 1.40 (m, 2H), 1.57 (d, J = 14 Hz, 1H), 1.80 - 2.00 (m, 1H), 2.00 - 2.10 (m, 3H), 2.20 - 2.40 (m, 3H), 2.40 - 3.10 (m, 9H), 3.16 (ddd, J = 7, 19, 19 Hz, 1H), 5.98 (s, 1H), 6.67 (d, J = 8 Hz, 1H), 6.73 (s, 1H), 6.97 (d, J = 8 Hz, 1H), 7.43 (s, 1H).

(Example 28)

Synthesis of (E)-1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-3-(furan-3-yl)prop-2-en-1-one

[Chemical Formula 49]

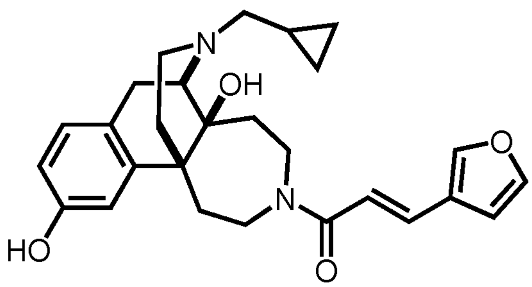

The title compound was obtained from the isomer C obtained in Example 2 and (E)-3-(furan-3-yl)acrylic acid according to the method described in Example 4.

$^1$H-NMR (400 MHz, $CDCl_3$)δ (ppm): 0.05 - 0.18 (m, 2H), 0.43 - 0.58 (m, 2H), 0.76 - 0.90 (m, 1H), 1.04 - 1.09 (m, 0.2H), 1.16 - 1.23 (m, 0.8H), 1.61 - 1.71 (m, 1H), 1.83 - 2.48 (m, 7H), 2.55 (dd, J = 4, 12 Hz, 1H), 2.79 (dd, J = 6, 18 Hz, 1H), 2.88 - 2.96 (m, 1.2H), 2.99 - 3.10 (m, 1.6H), 3.48 (ddd, J = 3, 3, 12 Hz, 0.8H), 3.52 - 3.68 (m, 0.4H), 3.76 - 3.85 (m, 0.2H), 3.91 (ddd, J = 3, 3, 14 Hz, 0.2H), 4.11 (ddd, J = 3, 12, 12 Hz, 0.8H), 4.51 (dd, J = 6, 14 Hz, 0.8H), 6.51 (d, J = 16 Hz, 0.2H), 6.55 (dd, J = 3, 8 Hz, 0.2H), 6.57 (s, 0.8H), 6.64 (d, J = 15 Hz, 0.8H), 6.65 - 6.68 (m, 0.2H), 6.71 (dd, J = 2, 8 Hz, 0.8H), 6.89 (d, J = 8 Hz, 0.2H), 6.97 (d, J = 8 Hz, 0.8H), 7.10 - 7.15 (m, 0.8H), 7.24 - 7.32 (m, 0.4H), 7.38 (s, 0.2H), 7.40 (s, 0.8H), 7.54 (d, J = 15 Hz, 0.8H), 7.55 (s, 0.2H), 7.65 (s, 0.8H).

(Example 29)

Synthesis of ((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)(phenyl)methanone

[Chemical Formula 50]

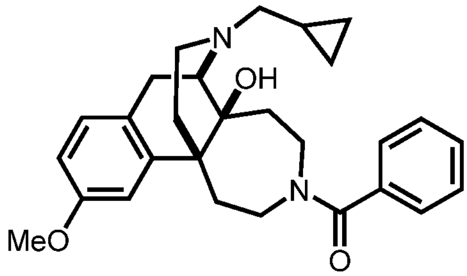

A solution of the isomer C (14 mg, 0.040 mmol) obtained in Example 2 in dichloromethane (1.0 mL) was cooled to 0°C, and to the solution were added triethylamine (17 pL, 0.12 mmol) and benzoic anhydride (18 mg, 0.079 mmol), followed by stirring at room temperature for 1 hour. Water was added to the reaction mixture to stop the reaction, followed by extraction twice with chloroform. The combined extracts were washed with saturated saline, dried over sodium sulfate, and then concentrated under reduced pressure. The residue was purified by silica gel column chromatography (0 to 10% methanol/chloroform) to yield the title compound (17 mg, 96%) as a colorless oily matter.

$^1$H-NMR (400 MHz, $CDCl_3$)δ (ppm): 0.06 - 0.17 (m, 2H), 0.45 - 0.58 (m, 2H), 0.75 - 0.91 (m, 1H), 0.96 - 1.02 (m, 0.7H), 1.10 - 1.16 (m, 0.3H), 1.46 (ddd, J = 3, 3, 14 Hz, 0.3H), 1.71 (dd, J = 4, 14 Hz, 0.7H), 1.74 - 2.14 (m, 3.7H), 2.21 (dd, J = 5, 15 Hz, 0.3H), 2.27 - 2.61 (m, 3.3H), 2.69 (ddd, J = 6, 12, 17 Hz, 0.7H), 2.76 (dd, J = 6, 18 Hz, 0.3H), 2.83 - 3.04 (m, 2.7H), 3.26 - 3.36 (m, 0.6H), 3.40 - 3.67 (m, 2.1H), 3.42 (s, 2.1H), 3.71 - 3.82 (m, 0.3H), 3.80 (s, 0.9H), 4.01 (ddd, J = 2, 4, 14 Hz, 0.7H), 4.17 (ddd, J = 1, 5, 14 Hz, 0.3H), 6.20 (d, J = 2 Hz, 0.7H), 6.66 - 6.78 (m, 1.3H), 6.92 - 6.98 (m, 1.3H), 7.02 (d, J = 8 Hz, 0.7H), 7.04 (d, J = 8 Hz, 0.3H), 7.24 - 7.40 (m, 3.7H).

(Example 30)

Synthesis of ((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)(phenyl)methanone

[Chemical Formula 51]

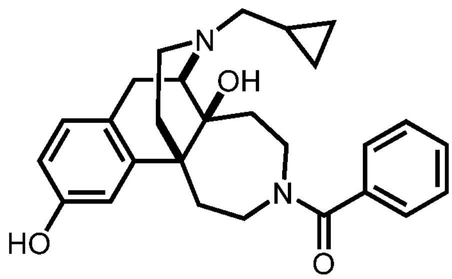

The title compound was obtained from the compound obtained in Example 29 according to the method described in Example 6.

$^1$H-NMR (400 MHz, $CDCl_3$)δ (ppm): 0.03 - 0.15 (m, 2H), 0.42 - 0.55 (m, 2H), 0.74 - 0.92 (m, 1H), 0.93 - 1.00 (m, 0.4H), 1.15 - 1.23 (m, 0.6H), 1.48 (ddd, J = 4, 4, 15 Hz, 0.6H), 1.66 - 1.79 (m, 0.8H), 1.88 - 2.13 (m, 3.4H), 2.26 - 2.66 (m, 4.4H), 2.73 (dd, J = 6, 18 Hz, 0.6H), 2.79 - 3.04 (m, 2.4H), 3.24 - 3.36 (m, 1.2H), 3.38 - 3.60 (m, 1H), 3.81 (ddd, J = 4, 13, 13 Hz, 0.6H), 3.93 - 4.01 (m, 0.4H), 4.29 - 4.38 (m, 0.6H), 6.20 (d, J = 2 Hz, 0.4H), 6.61 (dd, J = 2, 8 Hz, 0.4H), 6.65 (dd, J = 2, 8 Hz, 0.6H), 6.94 (d, J = 8 Hz, 0.6H), 6.95 (d, J = 8 Hz, 0.4H), 6.97 - 7.02 (m, 0.6H), 7.06 (d, J = 2 Hz,

0.6H), 7.29 - 7.45 (m, 4.4H).

(Example 31)

Synthesis of (5aS,6R,11bS)-14-(cyclopropylmethyl)-3-(2-(thiazol-2-yl)ethyl)-2,3,4,5,6,7-hexahydro-6,11b-(epiminoethano)naphtho[1,2-d]azepine-5a,10(1H)-diol

[Chemical Formula 52]

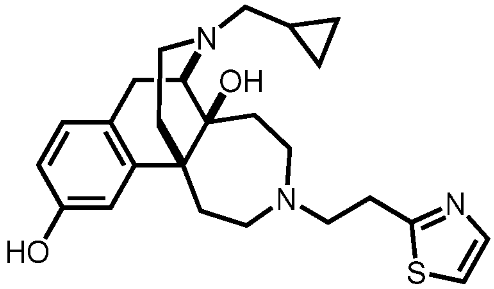

To the compound E (30 mg, 0.091 mmol) obtained in Example 3 and 2-(thiazol-2-yl)ethyl methanesulfonate (synthesized by the method described in WO 2010105179) (23 mg, 0.11 mmol) dissolved in acetonitrile (1 mL) was added N,N-diisopropylethylamine (47 μL, 0.27 mmol), followed by stirring at 80°C for 12 hours and then stirring at room temperature for 4 days. To the reaction mixture, a saturated aqueous sodium bicarbonate solution was added, followed by extraction twice with ethyl acetate. The combined extracts were dried over sodium sulfate and then concentrated under reduced pressure. The obtained crude product was purified by preparative thin layer chromatography (chloroform : 2 M ammonia-methanol solution = 85 : 15) to yield the title compound (26 mg, 65%) as a colorless amorphous form.

$^1$H-NMR (400 MHz, $CDCl_3$)δ (ppm): 0.04 - 0.17 (m, 2H), 0.45 - 0.56 (m, 2H), 0.77 - 0.92 (m, 1H), 0.97 - 1.08 (m, 1H), 1.17 - 1.73 (m, 1H), 1.76 - 1.93 (m, 2H), 1.94 - 2.11 (m, 2H), 2.28 - 3.00 (m, 12H), 3.01 - 3.23 (m, 3H), 4.64 (br s, 1H), 6.53 - 6.59 (m, 1H), 6.61 (dd, J = 3, 8 Hz, 1H), 6.93 (d, J = 8 Hz, 1H), 7.09 (d, J = 3 Hz, 1H), 7.61 (d, J = 4 Hz, 1H).

(Example 32)

Synthesis of (5aS,6R,11bS)-14-(cyclopropylmethyl)-3-(2-(furan-2-yl)ethyl)-2,3,4,5,6,7-hexahydro-6,11b-(epiminoethano)naphtho[1,2-d]azepine-5a,10(1H)-diol

[Chemical Formula 53]

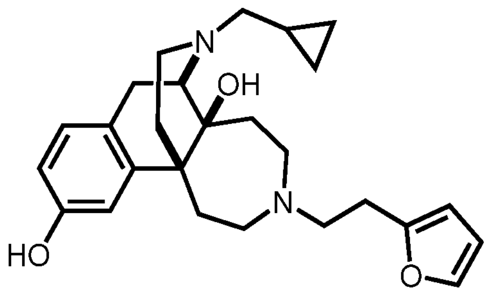

To a solution of 2-(furan-2-yl)ethan-1-ol (7 mg, 0.06 mmol) in dichloromethane (1 mL) was added triethylamine (18 pL, 0.12 mmol), then was added mesylic anhydride (11 mg, 0.066 mmol) under ice cooling, followed by stirring for 1 hour under ice cooling. Under ice cooling, the reaction mixture was poured into a saturated aqueous sodium bicarbonate solution, followed by extraction three times with chloroform. The combined extracts were dried over sodium sulfate and then concentrated under reduced pressure to yield a crude product of 2-(furan-2-yl)ethyl methanesulfonate. A solution of the obtained crude product in N,N-dimethylformamide (0.25 mL) was added dropwise to a solution of the compound E (10 mg, 0.03 mmol) obtained in Example 3 and N,N-diisopropylethylamine (21 pL, 0.12 mmol) in N,N-dimethylformamide

(0.5 mL) under ice cooling, followed by stirring at room temperature for 16 hours. The reaction solution was diluted with ethyl acetate, washed with a saturated aqueous sodium bicarbonate solution, water, and saturated saline, dried over sodium sulfate, and then concentrated under reduced pressure. The obtained crude product was purified by preparative thin layer chromatography (chloroform : 2 M ammonia-methanol solution = 90 : 10) to yield the title compound (2.1 mg, 17%) as a colorless amorphous form.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.04 (d, J = 11 Hz, 1H), 1.40 - 1.60 (m, 1H), 1.80 - 1.95 (m, 2H), 1.95 - 2.10 (m, 3H), 2.20 - 2.40 (m, 3H), 2.50 - 2.60 (m, 1H), 2.70 - 3.00 (m, 10H), 3.20 - 3.45 (m, 1H), 5.96 (d, J = 3 Hz, 1H), 6.20 - 6.25 (m, 1H), 6.50 - 6.55 (m, 1H), 6.58 (dd, J = 3, 8 Hz, 1H), 6.90 (d, J = 8 Hz, 1H)

(Example 33)

Synthesis of 1-((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(thiazol-4-yl)ethan-1-one

[Chemical Formula 54]

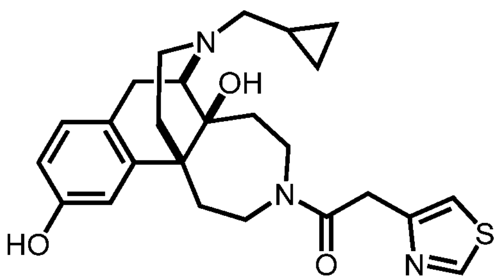

The title compound was obtained from the compound E obtained in Example 3 and 2-(thiazol-4-yl)acetic acid according to the method described in Example 7.

$^1$H-NMR (400 MHz, $CDCl_3$)δ (ppm): 0.04 - 0.14 (m, 2H), 0.43 - 0.56 (m, 2H), 0.76 - 0.87 (m, 1H), 1.01 - 1.15 (m, 1H), 1.53 - 1.65 (m, 1H), 1.77 - 2.10 (m, 3H), 2.16 - 2.40 (m, 3H), 2.48 - 2.60 (m, 1.4H), 2.69 (dd, J = 6, 18 Hz, 0.6H), 2.77 (dd, J = 6, 18 Hz, 0.4H), 2.86 (d, J = 6 Hz, 0.6H), 2.91 (d, J = 6 Hz, 0.4H), 2.92 (d, J = 18 Hz, 0.4H), 2.97 (d, J = 18 Hz, 0.6H), 3.12 - 3.30 (m, 1.4H), 3.37 - 3.46 (m, 0.6H), 3.49 (ddd, J = 4, 4, 13 Hz, 0.6H), 3.67 (d, J = 16 Hz, 0.4H), 3.72 - 3.83 (m, 0.4H), 3.84 (d, J = 16 Hz, 0.4H), 3.85 - 3.99 (m, 1H), 3.90 (d, J = 16 Hz, 0.6H), 4.00 (d, J = 16 Hz, 0.6H), 4.11 - 4.19 (m, 0.6H), 6.61 (dd, J = 3, 8 Hz, 0.4H), 6.63 - 6.68 (m, 1H), 6.85 (d, J = 3 Hz, 0.6H), 6.90 (d, J = 8 Hz, 0.4H), 6.92 (d, J = 8 Hz, 0.6H), 7.07 - 7.11 (m, 1H), 8.73 (d, J = 2 Hz, 0.6H), 8.74 (d, J = 2 Hz, 0.4H).

(Example 34)

Synthesis of (5aS,6R,11bS)-14-(cyclopropylmethyl)-3-(2-(thiazol-4-yl)ethyl)-2,3,4,5,6,7-hexahydro-6,b-(epiminoethano)naphtho[1,2-d]azepine-5a,10(1H)-diol

[Chemical Formula 55]

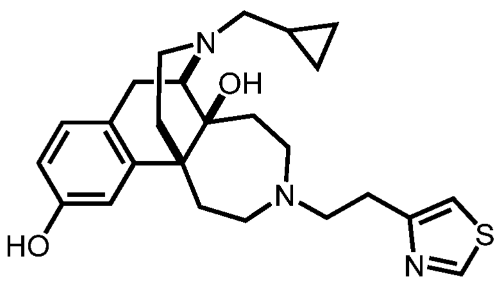

The title compound was obtained from the compound obtained in Example 33 according to the method described in Example 24.

$^1$H-NMR (400 MHz, $CDCl_3$)$\delta$ (ppm): 0.03 - 0.16 (m, 2H), 0.42 - 0.56 (m, 2H), 0.76 - 0.88 (m, 1H), 0.98 - 1.08 (m, 1H), 1.49 (dd, J = 5, 14 Hz, 1H), 1.75 - 1.86 (m, 2H), 1.94 - 2.08 (m, 2H), 2.28 - 2.41 (m, 3H), 2.49 - 2.58 (m, 1H), 2.68 - 2.80 (m, 2H), 2.73 (dd, J = 6, 18 Hz, 1H), 2.85 - 3.05 (m, 7H), 3.16 (dd, J = 12, 12 Hz, 1H), 4.72 (br s, 1H), 6.46 (s, 1H), 6.59 (d, J = 8 Hz, 1H), 6.84 (s, 1H), 6.89 (d, J = 8 Hz, 1H), 8.68 (s, 1H).

(Example 35)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-2-(1-methyl-5-(trifluoromethyl)-1H-pyrazol-3-yl)ethan-1-one

[Chemical Formula 56]

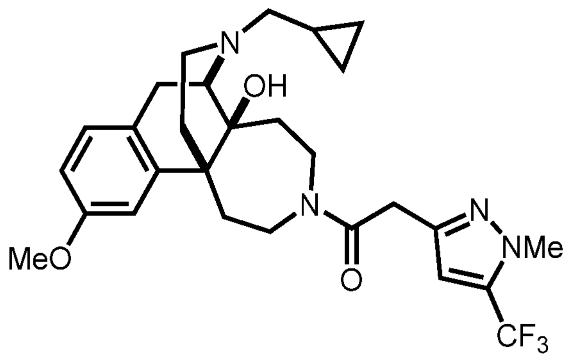

The title compound was obtained from the isomer C obtained in Example 2 and 2-(1-methyl-5-(trifluoromethyl)-1H-pyrazol-3-yl)acetic acid according to the method described in Example 5.

$^1$H-NMR (400 MHz, $CDCl_3$)$\delta$ (ppm): 0.03 - 0.20 (m, 2H), 0.45 - 0.61 (m, 2H), 0.76 - 0.92 (m, 1H), 1.01 - 1.10 (m, 1H), 1.21 - 1.91 (m, 2.5H), 1.93 - 2.44 (m, 5.5H), 2.47 - 2.62 (m, 1.5H), 2.69 - 3.07 (m, 3H), 3.08 - 3.20 (m, 0.5H), 3.36 - 3.97 (m, 10.5H), 4.06 - 4.17 (m, 0.5H), 6.41 (s, 0.5H), 6.54 (s, 0.5H), 6.62 - 6.75 (m, 2H), 6.97 - 7.06 (m, 1H).

(Example 36)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(l-methyl-5-(trifluoromethyl)-1H-pyrazol-3-yl)ethan-1-one

[Chemical Formula 57]

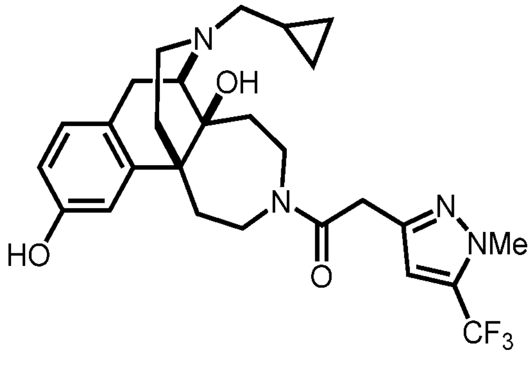

The title compound was obtained from the compound obtained in Example 35 according to the method described in Example 6.

$^1$H-NMR (400 MHz, $CDCl_3$)$\delta$ (ppm): 0.03 - 0.18 (m, 2H), 0.47 - 0.59 (m, 2H), 0.75 - 0.94 (m, 1H), 0.99 - 1.17 (m, 1H), 1.22 - 2.40 (m, 8H), 2.47 - 2.64 (m, 1.4H), 2.66 - 2.83 (m, 1H), 2.84 - 3.07 (m, 2H), 3.09 - 3.27 (m, 0.6H), 3.35 - 3.59 (m, 2H), 3.62 - 3.81 (m, 1.6H), 3.83 - 4.01 (m, 4H), 4.07 - 4.22 (m, 0.4H), 4.38 - 4.57 (m, 1H), 6.45 (s, 0.4H), 6.53 (s, 0.6H), 6.56 - 6.67 (m, 1.4H), 6.79 (d, J = 2 Hz, 0.6H), 6.88 - 6.97 (m, 1H).

(Example 37)

Synthesis of (5aS,6R,11bS)-14-(cyclopropylmethyl)-3-(2-(1-methyl-5-(trifluoromethyl)-1H-pyrazol-3-yl)ethyl)-2,3,4,5,6,7-hexahydro-6,11b-(epiminoethano)naphtho[1,2-d]azepine-5a,10(1H)-diol

[Chemical Formula 58]

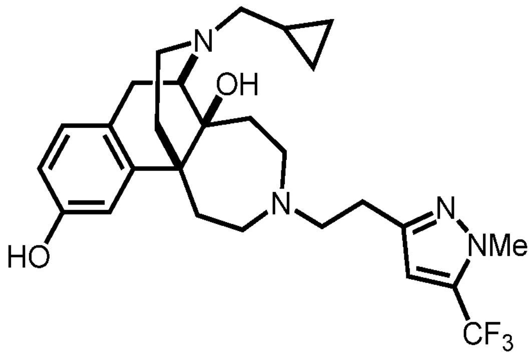

The title compound was obtained from the compound obtained in Example 36 according to the method described in Example 24.

$^1$H-NMR (400 MHz, $CDCl_3$)$\delta$ (ppm): 0.03 - 0.18 (m, 2H), 0.41 - 0.57 (m, 2H), 0.74 - 0.91 (m, 1H), 0.97 - 2.15 (m, 6H), 2.26 - 2.42 (m, 3H), 2.45 - 2.60 (m, 1H), 2.67 - 3.02 (m, 10H), 3.10 - 3.25 (m, 1H), 3.88 (s, 3H), 4.74 (br s, 1H), 6.35 (s, 1H), 6.45 (d, J = 2 Hz, 1H), 6.55 (dd, J = 2, 8 Hz, 1H), 6.89 (d, J = 8 Hz, 1H).

(Example 38)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-2-(1H-imidazol-4-yl)ethan-1-one

[Chemical Formula 59]

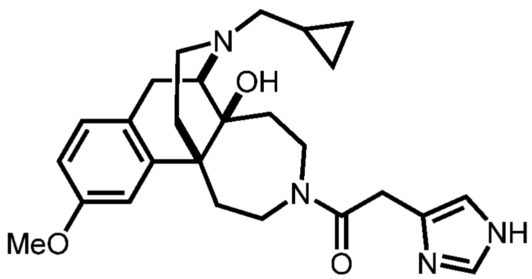

The title compound was obtained from the isomer C obtained in Example 2 and 2-(1H-imidazol-4-yl)acetic acid according to the method described in Example 5.

$^1$H-NMR (400 MHz, $CDCl_3$)$\delta$ (ppm): 0.06 - 0.17 (m, 2H), 0.46 - 0.58 (m, 2H), 0.77 - 0.90 (m, 1H), 1.01 - 1.17 (m, 1H), 1.48 - 3.19 (m, 13H), 3.36 - 4.82 (m, 8H), 6.56 - 6.88 (m, 3H), 6.97 - 7.09 (m, 1H), 7.48 - 7.54 (m, 1H).

(Example 39)

Synthesis of 1-((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(1H-imidazol-4-yl)ethan-1-one

[Chemical Formula 60]

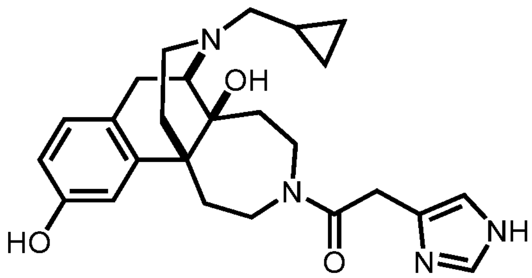

The title compound was obtained from the compound obtained in Example 38 according to the method described in Example 6.

$^1$H-NMR (400 MHz, $CDCl_3$)δ (ppm): 0.05 - 0.15 (m, 2H), 0.43 - 0.55 (m, 2H), 0.72 - 0.89 (m, 1H), 0.98 - 1.13 (m, 1H), 1.51 - 1.64 (m, 1H), 1.69 - 3.00 (m, 11H), 3.23 - 3.91 (m, 6H), 6.56 - 6.69 (m, 3H), 6.85 (d, J = 8 Hz, 0.6H), 6.97 (d, J = 8 Hz, 0.4H), 7.37 (s, 0.6H), 7.40 (s, 0.4H).

(Example 40)

Synthesis of 1-((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(1-methyl-1H-imidazol-4-yl)ethan-1-one

[Chemical Formula 61]

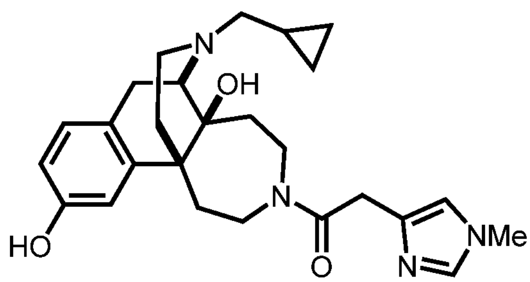

The title compound was obtained from the compound E obtained in Example 3 and 2-(1-methyl-1H-imidazol-4-yl)acetic acid according to the method described in Example 5.

$^1$H-NMR (400 MHz, $CDCl_3$)δ (ppm): 0.04 - 0.15 (m, 2H), 0.45 - 0.54 (m, 2H), 0.73 - 0.92 (m, 1H), 0.99 - 1.14 (m, 1H), 1.51 - 1.65 (m, 1H), 1.71 - 2.60 (m, 8H), 2.73 (ddd, J = 2, 8, 8 Hz, 1H), 2.82 - 3.03 (m, 2H), 3.14 - 3.25 (m, 0.4H), 3.28 - 3.99 (m, 8.2H), 4.04 - 4.17 (m, 0.4H), 6.63 (dd, J = 3, 8 Hz, 0.6H), 6.67 (dd, J = 3, 8 Hz, 0.4H), 6.70 - 6.77 (m, 1.6H), 6.82 (d, J = 3 Hz, 0.4H), 6.87 (d, J = 8 Hz, 0.6H), 6.91 (d, J = 8 Hz, 0.4H), 7.31 (s, 0.4H), 7.36 (s, 0.6H).

(Example 41)

Synthesis of (5aS,6R,11bS)-14-(cyclopropylmethyl)-3-(2-(1-methyl-1H-imidazol-4-yl)ethyl)-2,3,4,5,6,7-hexahydro-6,11b-(epiminoethano)naphtho[1,2-d]azepine-5a,10(1H)-diol

[Chemical Formula 62]

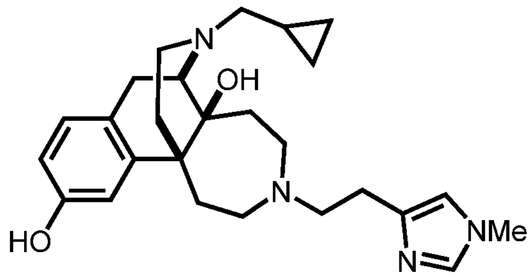

The title compound was obtained from the compound obtained in Example 40 according to the method described in Example 24.

$^1$H-NMR (400 MHz, $CDCl_3$)δ (ppm): 0.04 - 0.15 (m, 2H), 0.41 - 0.58 (m, 2H), 0.72 - 1.56 (m, 3H), 1.74 - 2.12 (m, 4H), 2.24 - 2.39 (m, 3H), 2.47 - 2.58 (m, 1H), 2.66 - 3.02 (m, 10H), 3.17 - 3.30 (m, 1H), 3.58 (s, 3H), 6.51 (s, 1H), 6.54 (s, 1H), 6.60 (d, J = 8 Hz, 1H), 6.87 (d, J = 8 Hz, 1H), 7.29 (s, 1H).

(Example 42)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-2-(1H-pyrazol-1-yl)ethan-1-one

[Chemical Formula 63]

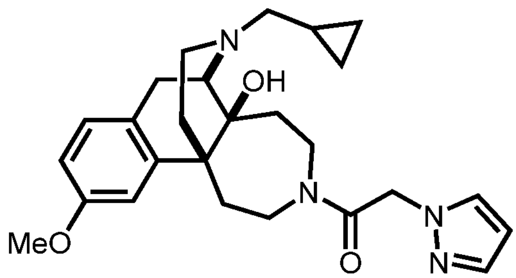

The title compound was obtained from the isomer C obtained in Example 2 and 2-(1H-pyrazol-1-yl)acetic acid according to the method described in Example 5.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.00 - 1.15 (m, 1H), 1.50 - 1.70 (m, 2H), 1.75 - 1.90 (m, 1H), 1.90 - 2.40 (m, 3H), 2.50 - 2.65 (m, 1.4H), 2.70 - 3.10 (m, 4.6H), 3.30 - 3.60 (m, 1.4H), 3.60 - 3.70 (m, 0.6H), 3.75 - 3.95 (m, 4.6H), 4.10 - 4.20 (m, 0.4H), 4.49 (br s, 1H), 4.59 (d, J = 16 Hz, 0.6H), 4.80 - 5.05 (m, 1.4H), 6.20 - 6.30 (m, 1H), 6.65 - 6.80 (m, 2H), 7.00 - 7.10 (m, 1H), 7.40 - 7.55 (m, 2H).

(Example 43)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(1H-pyrazol-1-yl)ethan-1-one

[Chemical Formula 64]

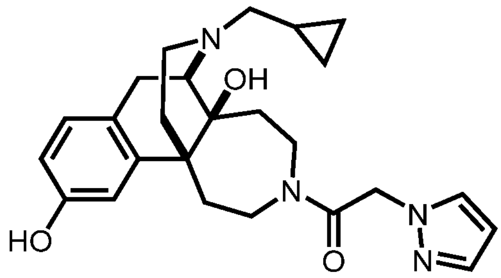

To a solution of the compound E (7.6 mg, 0.023 mmol) obtained in Example 3 in N,N-dimethylformamide (1.0 mL) were added 2-(1H-pyrazol-1-yl)acetic acid (8.8 mg, 0.069 mmol), N,N-diisopropylethylamine (12.1 μL, 0.069 mmol), 1-hydroxybenzotriazole monohydrate (10.6 mg, 0.069 mmol), 1-ethyl-3-(3-dimethylaminopropyl)carbodiimide hydrochloride (13.3 mg, 0.069 mmol), and N,N-dimethyl-4-aminopyridine (1.4 mg, 0.012 mmol), followed by stirring at room temperature for 2 hours. To the reaction mixture, a 2 M aqueous sodium hydroxide solution (1 mL) was added, followed by stirring at room temperature for 17 hours. The reaction mixture was diluted with water, followed by extraction three times with ethyl acetate. The combined extracts were dried over sodium sulfate and then concentrated under reduced pressure. The obtained crude product was purified by preparative thin layer chromatography (chloroform : 2 M ammonia-methanol = 10 : 1) to yield the title compound (9.9 mg, 98%) as a colorless oily matter.

$^1$H-NMR (400 MHz, $CDCl_3$)δ (ppm): 0.06 - 0.13 (m, 2H), 0.47 - 0.54 (m, 2H), 0.76 - 0.86 (m, 1H), 1.03 - 1.10 (m, 1H), 1.52 - 1.63 (m, 1H), 1.76 - 2.25 (m, 4.5H), 2.26 - 2.39 (m, 2H), 2.50 - 2.65 (m, 1.5H), 2.68 - 2.79 (m, 1H), 2.86 - 2.95 (m, 1.5H), 2.98 (d, J = 18 Hz, 0.5H), 3.09 - 3.19 (m, 0.5H), 3.25 - 3.39 (m, 1.5H), 3.62 - 3.73 (m, 0.5H), 3.81 - 3.89 (m, 1H), 4.01 - 4.09 (m, 0.5H), 4.52 (br s, 1H), 4.66 (d, J = 16 Hz, 0.5H), 4.81 (d, J = 16 Hz, 0.5H), 4.95 (d, J = 16 Hz, 0.5H), 4.99 (d, J = 16 Hz, 0.5H), 6.26 - 6.29 (m, 1H), 6.62 - 6.68 (m, 2H), 6.90 - 6.95 (m, 1H), 7.33 (dd, J = 2, 2 Hz, 0.5H), 7.41 (d, J = 2 Hz, 0.5H), 7.55 (dd, J = 2, 4 Hz, 1H).

(Example 44)

Synthesis of (5aS,6R,11bS)-3-(2-(1H-pyrazol-1-yl)ethyl)-14-(cyclopropylmethyl)-10-methoxy-2,3,4,5,6,7-hexahydro-6,11b-(epiminoethano)naphtho[1,2-d]azepin-5a(1H)-ol

[Chemical Formula 65]

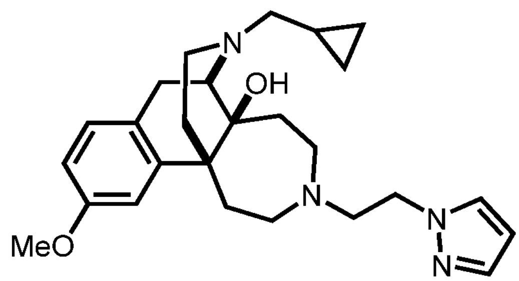

The title compound was obtained from the compound obtained in Example 42 according to the method described in Example 24.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.03 (d, J = 13 Hz, 1H), 1.40 - 1.50 (m, 1H), 1.75 (ddd, J = 3, 11, 14 Hz, 1H), 1.86 (ddd, J = 4, 4, 12 Hz, 1H), 1.90 - 2.10 (m, 2H), 2.34 (d, J = 6 Hz, 2H), 2.40 - 2.50 (m, 2H), 2.50 - 2.60 (m, 1H), 2.64 (dt, J = 13, 4 Hz, 1H), 2.70 - 2.90 (m, 3H), 2.90 - 3.00 (m, 3H), 3.14 (ddd, J = 1, 11, 12 Hz, 1H), 3.76 (s, 3H), 4.00 - 4.10 (m, 2H), 4.59 (br s, 1H), 6.04 (t, J = 2 Hz, 1H), 6.68 (d, J = 2 Hz, 1H), 6.72 (dd, J = 2, 8 Hz, 1H), 6.91 (d, J = 2 Hz, 1H), 7.03 (d, J = 8 Hz, 1H), 7.40 (d, J = 2 Hz, 1H).

(Example 45)

Synthesis of (5aS,6R,11bS)-3-(2-(1H-pyrazol-1-yl)ethyl)-14-(cyclopropylmethyl)-2,3,4,5,6,7-hexahydro-6,11b-(epiminoethano)naphtho[1,2-d]azepine-5a,10(1H)-diol

[Chemical Formula 66]

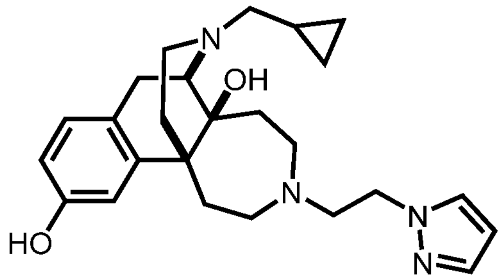

The title compound was obtained from the compound obtained in Example 44 according to the method described in Example 6.

$^1$H-NMR (400 MHz, $CDCl_3$)δ (ppm): 0.00 - 0.15 (m, 2H), 0.45 - 0.55 (m, 2H), 0.75 - 0.85 (m, 1H), 1.00 (d, J = 11 Hz, 1H), 1.44 (dd, J = 3, 15 Hz, 1H), 1.70 - 1.85 (m, 2H), 1.90 - 2.10 (m, 3H), 2.30 - 2.45 (m, 3H), 2.45 - 2.55 (m, 2H), 2.61 (dt, J = 13, 4 Hz, 1H), 2.75 (dd, J = 6, 18 Hz, 1H), 2.80 - 3.00 (m, 4H), 3.10 - 3.20 (m, 1H), 4.05 - 4.15 (m, 2H), 6.09 (t, J = 2 Hz, 1H), 6.55 (d, J = 2 Hz, 1H), 6.61 (dd, J = 2, 8 Hz, 1H), 6.93 (d, J = 8 Hz, 1H), 7.05 (d, J = 2 Hz, 1H), 7.41 (d, J = 2 Hz, 1H).

(Example 46)

Synthesis of 1-((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(3-methyl-1H-pyrazol-1-yl)ethan-1-one

[Chemical Formula 67]

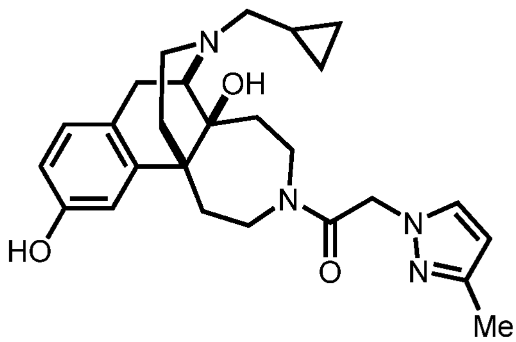

The title compound was obtained from the compound E obtained in Example 3 and 2-(4-methyl-1H-pyrazol-1-yl)acetic acid according to the method described in Example 5.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.07 (d, J = 12 Hz, 1H), 1.50 - 1.65 (m, 1H), 1.70 - 2.40 (m, 9.4H), 2.50 - 2.80 (m, 2.6H), 2.80 - 3.15 (m, 2.6H), 3.20 - 3.40 (m, 1.4H), 3.60 - 3.75 (m, 0.6H), 3.75 - 3.90 (m, 1H), 4.00 - 4.15 (m, 0.4H), 4.53 (br s, 1H), 4.60 (d, J = 16 Hz, 0.6H), 4.70 (d, J = 16 Hz, 0.6H), 4.85 (d, J = 16 Hz, 0.4H), 4.90 (d, J = 16 Hz, 0.4H), 6.05 (s, 1H), 6.60 - 6.70 (m, 2H), 6.85 - 7.00 (m, 1H), 7.20 - 7.35 (m, 1H).

(Example 47)

Synthesis of (5aS,6R,11bS)-14-(cyclopropylmethyl)-3-(2-(3-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 68]

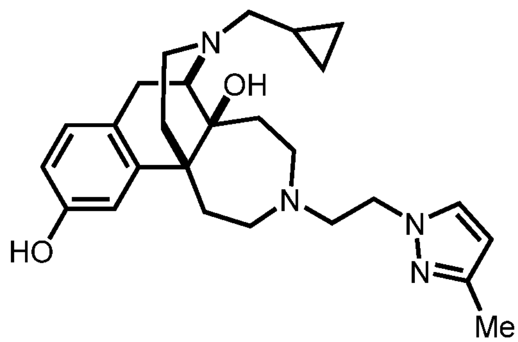

The title compound was obtained from the compound obtained in Example 46 according to the method described in Example 24.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.03 (d, J = 11 Hz, 1H), 1.48 (dd, J = 8.14 Hz, 1H), 1.70 - 1.85 (m, 2H), 1.90 - 2.10 (m, 2H), 2.23 (s, 3H), 2.30 - 2.45 (m, 3H), 2.50 - 2.70 (m, 3H), 2.70 - 3.00 (m, 6H), 3.10 - 3.25 (m, 1H), 3.95 - 4.10 (m, 2H), 5.88 (d, J = 2 Hz, 1H), 6.55 (d, J = 2 Hz, 1H), 6.62 (dd, J = 2, 8 Hz, 1H), 6.93 (d, J = 8 Hz, 1H), 6.99 (s, 1H).

(Example 48)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-2-(4-methyl-1H-pyrazol-1-yl)ethan-1-one

[Chemical Formula 69]

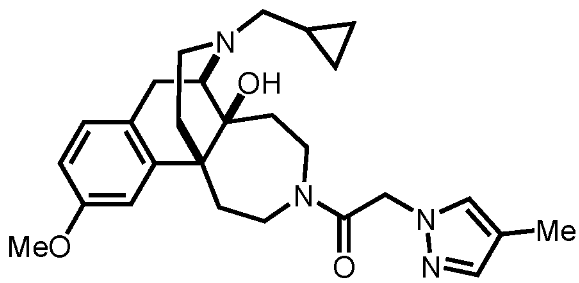

The title compound was obtained from the isomer C obtained in Example 2 and 2-(4-methyl-1H-pyrazol-1-yl)acetic acid according to the method described in Example 5.

$^1$H-NMR (400 MHz, $CDCl_3$)δ (ppm): 0.07 - 0.15 (m, 2H), 0.47 - 0.55 (m, 2H), 0.77 - 0.88 (m, 1H), 1.04 - 1.12 (m, 1H), 1.56 - 1.63 (m, 1H), 1.77 - 1.87 (m, 1H), 1.92 - 2.02 (m, 1.5H), 2.04 (s, 1.5H), 2.07 (s, 1.5H), 2.09 - 2.22 (m, 1.5H), 2.27 - 2.40 (m, 2H), 2.52 - 2.62 (m, 1H), 2.713.00 (m, 3.5H), 3.02 - 3.10 (m, 0.5H), 3.35 (ddd, J = 4, 4, 13 Hz, 0.5H), 3.39 - 3.47 (m, 0.5H), 3.48 - 3.54 (m, 0.5H), 3.59 - 3.72 (m, 0.5H), 3.77 (s, 1.5H), 3.79 (s, 1.5H), 3.80 - 3.91 (m, 1.5H), 4.10 - 4.17 (m, 0.5H), 4.51 (d, J = 16 Hz, 0.5H), 4.83 (d, J = 16 Hz, 0.5H), 4.87 (d, J = 16 Hz, 0.5H), 4.93 (d, J = 16 Hz, 0.5H), 6.67 (d, J = 6 Hz, 0.5H), 6.68 (d, J = 6 Hz, 0.5H), 6.70 - 6.75 (m, 1H), 7.01 - 7.05 (m, 1.5H), 7.20 (s, 0.5H), 7.27 (s, 0.5H), 7.30 (s, 0.5H).

(Example 49)

Synthesis of 1-((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(4-methyl-1H-pyrazol-1-yl)ethan-1-one

[Chemical Formula 70]

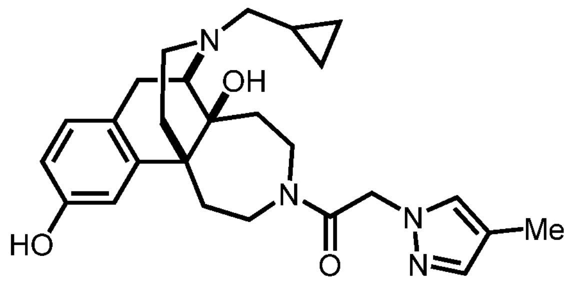

The title compound was obtained from the compound obtained in Example 48 according to the method described in Example 6.

$^1$H-NMR (400 MHz, $CDCl_3$)δ (ppm): 0.07 - 0.14 (m, 2H), 0.48 - 0.54 (m, 2H), 0.77 - 0.86 (m, 1H), 1.02 - 1.11 (m, 1H), 1.55 - 1.62 (m, 1H), 1.75 - 1.88 (m, 1.5H), 1.91 - 2.08 (m, 2.5H), 2.04 (s, 1.5H), 2.06 (s, 1.5H), 2.10 - 2.40 (m, 3.5H), 2.50 - 2.65 (m, 1.5H), 2.85 - 3.00 (m, 2H), 3.09 - 3.16 (m, 0.5H), 3.22 - 3.30 (m, 1H), 3.31 - 3.39 (m, 0.5H), 3.64 - 3.74 (m, 0.5H), 3.78 - 3.87 (m, 1H), 4.00 - 4.07 (m, 0.5H), 4.56 (d, J = 16 Hz, 0.5H), 4.74 (d, J = 16 Hz, 0.5H), 4.86 (d, J = 16 Hz, 0.5H), 4.91 (d, J = 16 Hz, 0.5H), 6.62 - 6.68 (m, 2H), 6.90 - 6.95 (m, 1H), 7.11 (s, 0.5H), 7.16 (s, 0.5H), 7.29 (m, 0.5H), 7.30 (s, 0.5H).

(Example 50)

Synthesis of (5aS,6R,11bS)-14-(cyclopropylmethyl)-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 71]

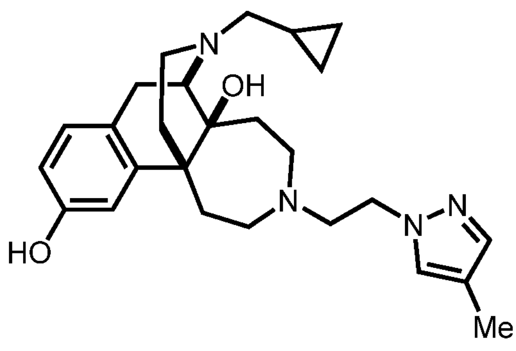

The title compound was obtained from the compound obtained in Example 49 according to the method described in Example 24.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.02 (d, J = 11 Hz, 1H), 1.45 (dd, J = 3, 14 Hz, 1H), 1.70 - 1.85 (m, 2H), 1.90 - 2.10 (m, 2H), 2.00 (s, 3H), 2.30 - 2.40 (m, 3H), 2.50 - 2.70 (m, 3H), 2.75 (dd, J = 6, 18 Hz, 1H), 2.80 - 3.00 (m, 5H), 3.13 - 3.25 (m, 1H), 4.00 - 4.15 (m, 2H), 6.54 (d, J = 3 Hz, 1H), 6.61 (dd, J = 3, 8 Hz, 1H), 6.85 - 6.95 (m, 2H), 7.24 (s, 1H).

(Example 51)

Synthesis of 1-((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(5-methyl-1H-pyrazol-1-yl)ethan-1-one

[Chemical Formula 72]

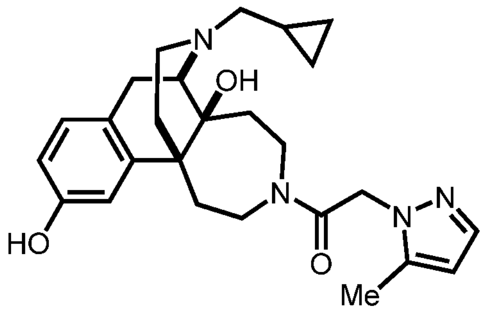

The title compound was obtained from the compound E obtained in Example 3 and 2-(5-methyl-1H-pyrazol-1-yl)acetic acid according to the method described in Example 5.

$^1$H-NMR (400 MHz, $CDCl_3$)δ (ppm): 0.00 - 0.15 (m, 2H), 0.45 - 0.55 (m, 2H), 0.80 - 0.90 (m, 1H), 1.06 (d, J = 11 Hz, 1H), 1.50 - 1.90 (m, 3H), 1.90 - 2.10 (m, 2H), 2.01 (s, 1.2H), 2.08 (s, 1.8H), 2.20 - 2.40 (m, 4H), 2.50 - 2.80 (m, 2H), 2.80 - 3.00 (m, 1.6H), 3.15 - 3.50 (m, 1.4H), 3.60 - 4.00 (m, 2H), 4.53 (d, J = 16 Hz, 0.6H), 4.72 (d, J = 16 Hz, 0.6H), 4.85 (d, J = 16 Hz, 0.4H), 4.90 (d, J = 16 Hz, 0.4H), 5.99 (s, 0.4H), 6.02 (s, 0.6H), 6.60 - 6.75 (m, 2H), 6.90 - 7.00 (m, 1H), 7.38 (s, 0.4H), 7.41 (s, 0.6H).

(Example 52)

Synthesis of (5aS,6R,11bS)-14-(cyclopropylmethyl)-3-(2-(5-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 73]

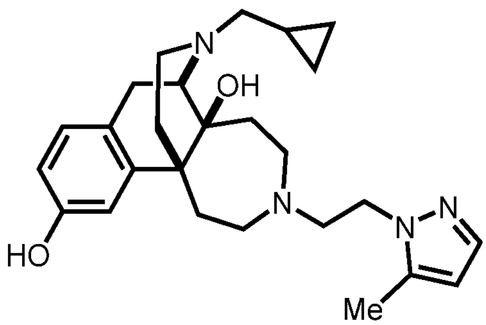

The title compound was obtained from the compound obtained in Example 51 according to the method described in Example 24.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.02 (d, J = 10 Hz, 1H), 1.40 - 1.50 (m, 1H), 1.75 - 1.90 (m, 2H), 1.95 - 2.10 (m, 3H), 2.20 (s, 3H), 2.20 - 2.40 (m, 3H), 2.50 - 2.80 (m, 4H), 2.80 - 3.00 (m, 4H), 3.15 - 3.35 (m, 1H), 4.07 (t, J = 8 Hz, 2H), 5.95 (s, 1H), 6.54 (s, 1H), 6.60 - 6.65 (m, 1H), 6.91 (d, J = 8 Hz, 1H), 7.36 (s, 1H).

(Example 53)

Synthesis of 1-((5aS,6R, 11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(3-(trifluoromethyl)-1H-pyrazol-1-yl)ethan-1-one

[Chemical Formula 74]

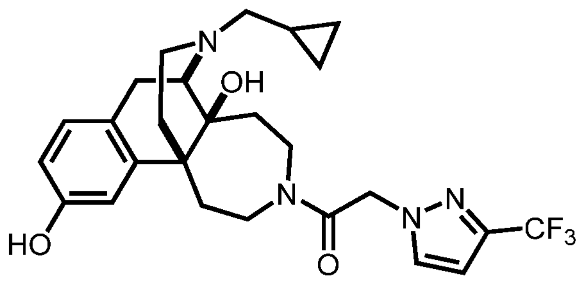

The title compound was obtained from the compound E obtained in Example 3 and 2-(3-(trifluoromethyl)-1H-pyrazol-1-yl)acetic acid according to the method described in Example 5.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.05 - 1.15 (m, 1H), 1.50 - 2.40 (m, 7H), 2.50 - 2.70 (m, 1.5H), 2.70 - 2.85 (m, 1.5H), 2.85 - 3.05 (m, 2H), 3.05 - 3.15 (m, 0.5H), 3.30 - 3.50 (m, 1.5H), 3.60 - 3.75 (m, 0.5H), 3.80 - 3.90 (m, 1H), 4.00 - 4.15 (m, 0.5H), 4.72 (d, J = 16 Hz, 0.5H), 4.89 (d, J = 16 Hz, 0.5H), 5.01 (d, J = 16 Hz, 0.5H), 5.02 (d, J = 16 Hz, 0.5H), , 6.51 (d, J = 2 Hz, 0.5H), 6.54 (d, J = 2 Hz, 0.5H), 6.60 - 6.70 (m, 2H), 6.95 (s, 0.5H), 6.97 (s, 0.5H), 7.37 (d, J = 1 Hz, 0.5H), 7.50 (d, J = 1 Hz, 0.5H).

(Example 54)

Synthesis of (5aS,6R,11bS)-14-(cyclopropylmethyl)-3-(2-(3-(trifluoromethyl)-1H-pyrazol-1-yl)ethyl)-2,3,4,5,6,7-hexahydro-6,11b-(epiminoethano)naphtho[1,2-d]azepine-5a,10(1H)-diol

[Chemical Formula 75]

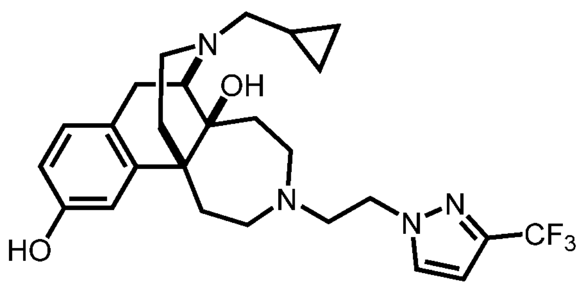

The title compound was obtained from the compound obtained in Example 53 according to the method described in Example 24.

$^1$H-NMR (400 MHz, $CDCl_3$)δ (ppm): 0.00 - 0.15 (m, 2H), 0.45 - 0.55 (m, 2H), 0.70 - 1.00 (m, 1H), 1.02 (d, J = 13 Hz, 1H), 1.40 - 1.55 (m, 1H), 1.65 - 1.85 (m, 2H), 1.90 - 2.10 (m, 2H), 2.30 - 2.65 (m, 6H), 2.75 - 3.05 (m, 6H), 3.05 - 3.20 (m, 1H), 4.00 - 4.15 (m, 2H), 6.26 (s, 1H), 6.60 - 6.70 (m, 2H), 6.86 (s, 1H), 6.98 (d, J = 8 Hz, 1H).

(Example 55)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(3,5-dimethyl-1H-pyrazol-1-yl)ethan-1-one

[Chemical Formula 76]

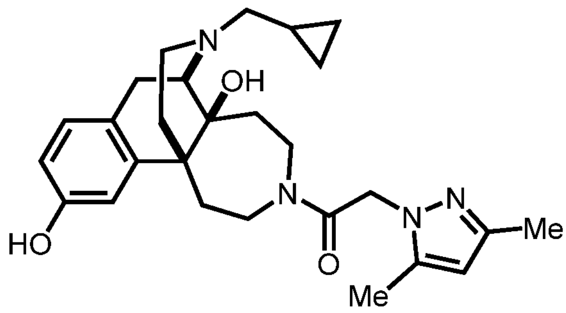

The title compound was obtained from the compound E obtained in Example 3 and 2-(3,5-dimethyl-1H-pyrazol-1-yl)acetic acid according to the method described in Example 5.

$^1$H-NMR (400 MHz, $CDCl_3$)δ (ppm): 0.00 - 0.15 (m, 2H), 0.45 - 0.55 (m, 2H), 0.80 - 0.90 (m, 1H), 1.00 - 1.10 (m, 1H), 1.45 - 1.70 (m, 1.6H), 1.75 - 1.90 (m, 1.4H), 1.90 - 2.40 (m, 4.6H), 1.94 (s, 1.2H), 2.01 (s, 1.8H), 2.17 (s, 1.2H), 2.22 (s, 1.8H), 2.45 - 3.00 (m, 4.4H), 3.15 - 3.45 (m, 2H), 3.60 - 4.00 (m, 2H), 4.47 (d, J = 16 Hz, 0.6H), 4.62 (d, J = 16 Hz, 0.6H), 4.75 (d, J = 16 Hz, 0.4H), 4.82 (d, J = 16 Hz, 0.4H), 5.77 (s, 0.4H), 5.80 (s, 0.6H), 6.60 - 6.70 (m, 2H), 6.85 - 6.95 (m, 1H).

(Example 56)

Synthesis of (5aS,6R,11bS)-14-(cyclopropylmethyl)-3-(2-(3,5-dimethyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 77]

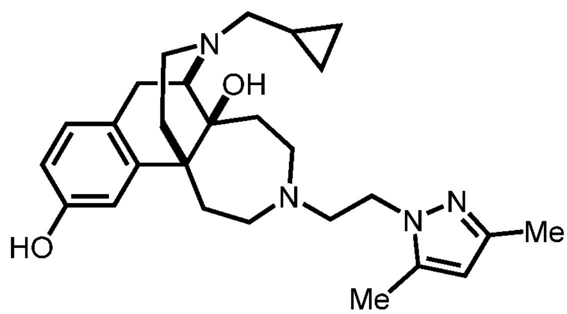

The title compound was obtained from the compound obtained in Example 55 according to the method described in Example 24.

$^1$H-NMR (400 MHz, $CDCl_3$)δ (ppm): 0.00 - 0.15 (m, 2H), 0.45 - 0.55 (m, 2H), 0.70 - 0.90 (m, 1H), 1.00 - 1.10 (m, 1H), 1.40 - 1.50 (m, 1H), 1.70 - 1.90 (m, 2H), 1.95 - 2.10 (m, 2H), 2.14 (s, 3H), 2.19, (s, 3H), 2.20 - 2.40 (m, 3H), 2.50 - 2.60 (m, 1H), 2.60 - 3.00 (m, 8H), 3.20 - 3.30 (m, 1H), 4.00 (t, J = 7 Hz, 2H), 5.74 (s, 1H), 6.53 (s, 1H), 6.61 (d, J = 8 Hz, 1H), 6.90 (d, J = 8 Hz, 1H).

(Example 57)

Synthesis of 2-(3,5-bis(difluoromethyl)-1H-pyrazol-1-yl)-1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)ethan-1-one

[Chemical Formula 78]

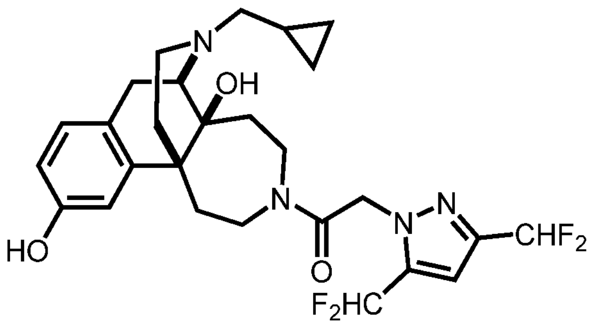

The title compound was obtained from the compound E obtained in Example 3 and 2-(3,5-bis(difluoromethyl)-1H-pyrazol-1-yl)acetic acid according to the method described in Example 5.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.10 (d, J = 12 Hz, 1H), 1.75 - 1.90 (m, 1H), 1.90 - 2.40 (m, 6H), 2.50 - 3.10 (m, 5H), 3.15 - 3.25 (m, 0.5H), 3.30 - 3.55 (m, 1.5H), 3.65 - 3.75 (m, 0.5H), 3.80 - 3.90 (m, 1H), 3.95 - 4.05 (m, 0.5H), 4.72 (d, J = 16 Hz, 0.5H), 4.98 (d, J = 16 Hz, 0.5H), 5.00 (d, J = 16 Hz, 0.5H), 5.15 (d, J = 16 Hz, 0.5H), 6.40 - 6.80 (m, 5H), 6.90 - 7.00 (m, 1H).

(Example 58)

Synthesis of (5aS,6R,11bS)-3-(2-(3,5-bis(difluoromethyl)-1H-pyrazol-1-yl)ethyl)-14-(cyclopropylmethyl)-2,3,4,5,6,7-hexahydro-6,11b-(epiminoethano)naphtho[1,2-d]azepine-5a,10(1H)-diol

[Chemical Formula 79]

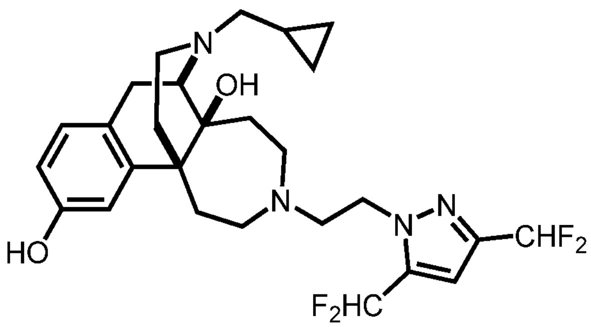

The title compound was obtained from the compound obtained in Example 57 according to the method described in Example 24.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.00 (d, J = 10 Hz, 1H), 1.40 - 1.50 (m, 1H), 1.65 - 2.10 (m, 4H), 2.20 - 2.40 (m, 2H), 2.45 - 2.60 (m, 2H), 2.62 (ddd, J = 4, 5, 13 Hz, 1H), 2.75 (dd, J = 6, 8 Hz, 1H), 2.80 - 3.00 (m, 5H), 3.18 (ddd, J = 2, 12, 12 Hz, 1H), 3.55 - 3.80 (m, 1H), 4.16 (t, J = 6 Hz, 2H), 6.50 - 6.90 (m, 5H), 6.93 (d, J = 8 Hz, 1H).

(Example 59)

Synthesis of 2-(4-bromo-1H-pyrazol-1-yl)-1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)ethan-1-one

[Chemical Formula 80]

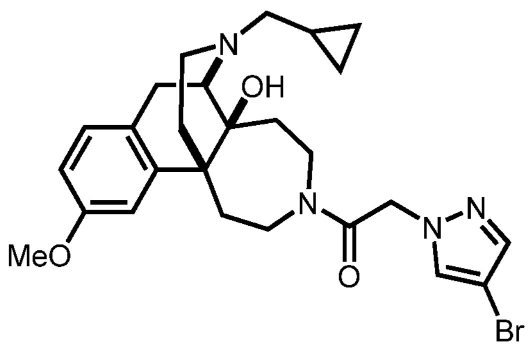

The title compound was obtained from the isomer C obtained in Example 2 and 2-(4-bromo-1H-pyrazol-1-yl)acetic acid according to the method described in Example 5.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.00 - 1.15 (m, 1H), 1.55 - 1.65 (m, 1H), 1.75 - 2.40 (m, 7H), 2.50 - 3.10 (m, 4.5H), 3.30 - 3.55 (m, 1.5H), 3.60 - 3.75 (m, 0.5H), 3.75 - 3.90 (m, 1H), 3.78 (s, 1.5H), 3.79 (s, 1.5H), 4.10 - 4.20 (m, 0.5H), 4.40 - 4.60 (br s, 1H), 4.54 (d, J = 16 Hz, 0.5H), 4.84 (d, J = 16 Hz, 0.5H), 4.94 (d, J = 16 Hz, 0.5H), 4.94 (d, J = 16 Hz, 0.5H), 6.65 - 6.70 (m, 1H), 6.70 - 6.75 (m, 1H), 7.00 - 7.05 (m, 1H), 7.25 (s, 0.5H), 7.42 (s, 0.5H), 7.45 (s, 0.5H), 7.47 (s, 0.5H).

(Example 60)

Synthesis of 2-(4-bromo-1H-pyrazol-1-yl)-1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)ethan-1-one

[Chemical Formula 81]

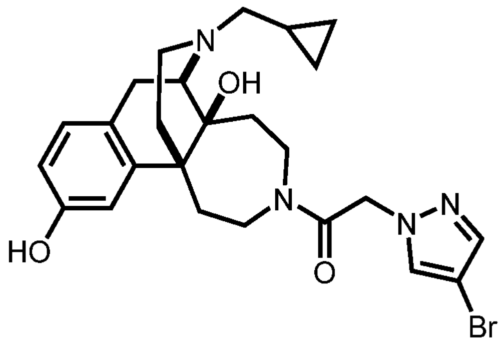

The title compound was obtained from the compound E obtained in Example 3 and 2-(4-bromo-1H-pyrazol-1-yl)acetic acid according to the method described in Example 5.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.00 - 1.15 (m, 1H), 1.50 - 1.70 (m, 1H), 1.70 - 2.45 (m, 7H), 2.50 - 2.85 (m, 2H), 2.85 - 3.00 (m, 2H), 3.12 - 3.22 (m, 0.5H), 3.30 - 3.45 (m, 1.5H), 3.65 - 3.75 (m, 0.5H), 3.75 - 3.90 (m, 1H), 3.95 - 4.05 (m, 0.5H), 4.61 (d, J = 16 Hz, 0.5H), 4.79 (d, J = 16 Hz, 0.5H), 4.92 (s, 1H), 6.60 - 6.70 (m, 2H), 6.90 - 7.00 (m, 1H), 7.33 (s, 0.5H), 7.43 (s, 1H), 7.46 (s, 0.5H).

(Example 61)

Synthesis of (5aS,6R,11bS)-3-(2-(4-bromo-1H-pyrazol-1-yl)ethyl)-14-(cyclopropylmethyl)-2,3,4,5,6,7-hexahydro-6,11b-(epiminoethano)naphtho[1,2-d]azepine-5a,10(1H)-diol

[Chemical Formula 82]

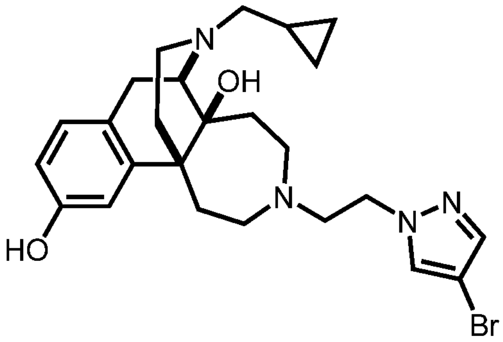

The title compound was obtained from the compound obtained in Example 60 according to the method described in Example 24.

$^1$H-NMR (400 MHz, $CDCl_3$)δ (ppm): 0.00 - 0.15 (m, 2H), 0.45 - 0.65 (m, 2H), 0.75 - 0.90 (m, 1H), 1.02 (d, J = 10 Hz, 1H), 1.40 - 1.50 (m, 1H), 1.65 - 1.85 (m, 2H), 1.90 - 2.10 (m, 2H), 2.25 - 2.40 (m, 3H), 2.40 - 2.60 (m, 3H), 2.70 - 3.20 (m, 7H), 3.80 - 3.95 (m, 1H), 3.95 - 4.05 (m, 1H), 6.60 - 6.70 (m, 2H), 6.71 (s, 1H), 7.03 (d, J = 8 Hz, 1H), 7.35 (s, 1H).

(Example 62)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(1H-imidazol-1-yl)ethan-1-one

[Chemical Formula 83]

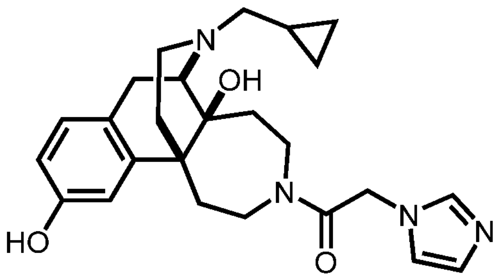

The title compound was obtained from the compound E obtained in Example 3 and 2-(1H-imidazol-1-yl)acetic acid according to the method described in Example 5.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.10 - 1.25 (m, 2H), 1.80 - 2.00 (m, 2.8H), 2.00 - 2.15 (m, 2.2H), 2.25 - 2.45 (m, 2H), 2.50 - 2.65 (m, 0.8H), 2.70 - 2.95 (m, 3.2H), 3.00 - 3.10 (m, 0.2H), 3.13 - 3.22 (m, 0.8H), 3.25 - 3.35 (m, 0.2H), 3.40 (dd, J = 8, 14 Hz, 0.8H), 3.70 - 3.80 (m, 0.2H), 3.85 - 4.05 (m, 1.8H), 4.43 (d, J = 16 Hz, 0.8H), 4.67 (d, J = 16 Hz, 0.2H), 4.75 - 4.90 (m, 1H), 6.57 (d, J = 2 Hz, 0.2H), 6.65 - 6.75 (m, 1H), 6.81 (dd, J = 2, 8 Hz, 0.8H), 6.85 - 6.95 (m, 1.8H), 6.95 - 7.05 (m, 1.2H), 7.35 (s, 0.8H), 7.48 (s, 0.2H).

(Example 63)

Synthesis of (5aS,6R,11bS)-3-(2-(1H-imidazol-1-yl)ethyl)-14-(cyclopropylmethyl)-2,3,4,5,6,7-hexahydro-6,11b-(epiminoethano)naphtho[1,2-d]azepine-5a,10(1H)-diol

[Chemical Formula 84]

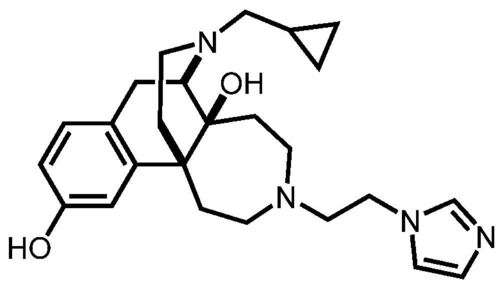

The title compound was obtained from the compound obtained in Example 62 according to the method described in Example 24.

$^1$H-NMR (400 MHz, $CDCl_3$)δ (ppm): 0.00 - 0.15 (m, 2H), 0.45 - 0.55 (m, 2H), 0.70 - 0.90 (m, 1H), 1.04 (d, J = 10 Hz, 1H), 1.51 (dd, J = 5, 14 Hz, 1H), 1.65 - 1.75 (m, 1H), 1.82 (ddd, J = 3, 11, 14 Hz, 1H), 1.95 - 2.05 (m, 2H), 2.30 - 2.45 (m, 3H), 2.45 - 2.55 (m, 3H), 2.60 - 2.75 (m, 2H), 2.80 - 3.00 (m, 4H), 3.08 - 3.18 (m, 1H), 3.78 (t, J = 6 Hz, 2H), 6.60 (s, 1H), 6.64 (d, J = 3 Hz, 1H), 6.73 (dd, J = 3, 8 Hz, 1H), 6.91 (s, 1H), 6.97 (d, J = 8 Hz, 1H), 7.08 (s, 1H).

(Example 64)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-2-(2H-1,2,3-triazol-2-yl)ethan-1-one

[Chemical Formula 85]

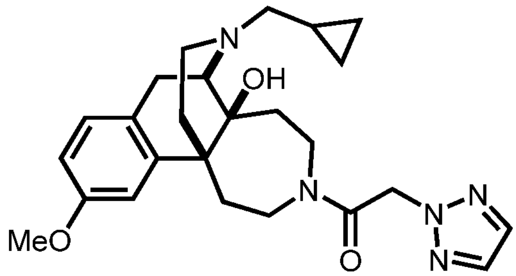

The title compound was obtained from the isomer C obtained in Example 2 and 2-(2H-1,2,3-triazol-2-yl)acetic acid according to the method described in Example 5.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.00 - 1.10 (m, 1H), 1.50 - 1.70 (m, 1H), 1.75 - 1.85 (m, 0.4H), 1.90 - 2.20 (m, 3.6H), 2.25 - 2.45 (m, 2H), 2.50 - 2.70 (m, 1.4H), 2.70 - 3.10 (m, 4.6H), 3.20 - 3.35 (m, 0.4H), 3.35 - 3.50 (m, 0.6H), 3.60 - 3.70 (m, 0.4H), 3.76 (s, 1.8H), 3.81 (s, 1.2H), 3.85 - 3.95 (m, 1H), 4.20 - 4.30 (m, 0.6H), 4.50 (br s, 1H), 4.94 (d, J = 16 Hz, 0.4H), 5.19 (d, J = 16 Hz, 0.4H), 5.28 (d, J = 16 Hz, 0.6H), 5.38 (d, J = 16 Hz, 0.6H), 6.60 - 6.80 (m, 2H), 7.00 - 7.10 (m, 1H), 7.61 (s, 0.8H), 7.66 (s, 1.2H).

(Example 65)

Synthesis of (5aS,6R,11bS)-3-(2-(2H-1,2,3-triazol-2-yl)ethyl)-14-(cyclopropylmethyl)-10-methoxy-2,3,4,5,6,7-hexahydro-6,11b-(epiminoethano)naphtho[1,2-d]azepin-5a(1H)-ol

[Chemical Formula 86]

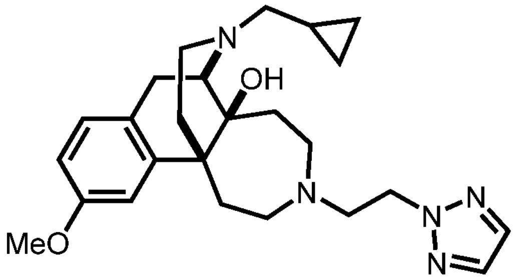

The title compound was obtained from the compound obtained in Example 64 according to the method described in Example 24.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.02 (d, J = 11 Hz, 1H), 1.40 - 1.50 (m, 1H), 1.70 - 2.10 (m, 4H), 2.20 - 2.40 (m, 3H), 2.50 - 2.60 (m, 2H), 2.65 - 3.10 (m, 7H), 3.11 - 3.23 (m, 1H), 3.77 (s, 3H), 4.35 - 4.50 (m, 2H), 6.63 (d, J = 2 Hz, 1H), 6.68 (dd, J = 2, 8 Hz, 1H), 6.98 (d, J = 8 Hz, 1H), 7.52 (s, 2H).

(Example 66)

Synthesis of (5aS,6R,11bS)-3-(2-(2H-1,2,3-triazol-2-yl)ethyl)-14-(cyclopropylmethyl)-2,3,4,5,6,7-hexahydro-6,11b-(epiminoethano)naphtho[1,2-d]azepine-5a,10(1H)-diol

[Chemical Formula 87]

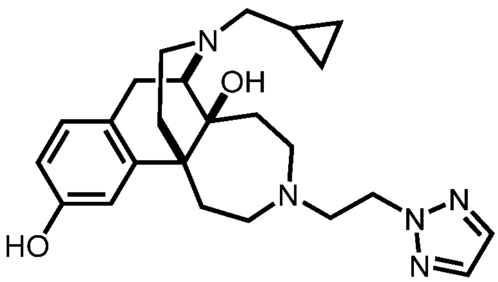

The title compound was obtained from the compound obtained in Example 65 according to the method described in Example 6.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.03 (d, J = 11 Hz, 1H), 1.20 - 1.30 (m, 1H), 1.40 - 1.50 (m, 1H), 1.70 - 1.90 (m, 2H), 1.90 - 2.10 (m, 3H), 2.20 - 2.40 (m, 4H), 2.50 - 3.25 (m, 7H), 4.40 - 4.60 (m, 2H), 6.51 (d, J = 2 Hz, 1H), 6.59 (dd, J = 2, 8 Hz, 1H), 6.91 (d, J = 8 Hz, 1H), 7.54 (s, 2H).

(Example 67)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-3-(pyridin-2-yl)propan-1-one

[Chemical Formula 88]

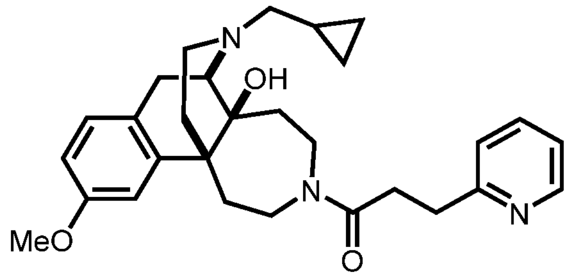

The title compound was obtained from the isomer C obtained in Example 2 and 3-(pyridin-2-yl)propanoic acid according to the method described in Example 7.

$^1$H-NMR (400 MHz, $CDCl_3$)$\delta$ (ppm): 0.05 - 0.16 (m, 2H), 0.45 - 0.57 (m, 2H), 0.76 - 0.88 (m, 1H), 1.01 - 1.12 (m, 1H), 1.54 - 1.65 (m, 1H), 1.78 (ddd, J = 2, 12, 15 Hz, 0.5H), 1.87 (ddd, J = 4, 12, 16 Hz, 0.5H), 1.91 - 2.23 (m, 3.5H), 2.29 (dd, J = 6, 12 Hz, 0.5H), 2.30 - 2.40 (m, 1.5H), 2.41 - 2.60 (m, 2H), 2.66 - 3.18 (m, 7H), 3.34 (ddd, J = 4, 4, 14 Hz, 0.5H), 3.39 - 3.49 (m, 0.5H), 3.50 (ddd, J = 4, 4, 14 Hz, 0.5H), 3.61 - 3.73 (m, 0.5H), 3.70 (s, 1.5H), 3.77 (s, 1.5H), 3.80 - 3.92 (m, 1H), 4.17 (ddd, J = 3, 5, 15 Hz, 0.5H), 4.48 (br s, 1H), 6.63 (d, J = 2 Hz, 0.5H), 6.64 - 6.73 (m, 1.5H), 7.00 (d, J = 8 Hz, 0.5H), 7.01 (d, J = 8 Hz, 0.5H), 7.04 - 7.10 (m, 1H), 7.13 (d, J = 8 Hz, 0.5H), 7.20 (d, J = 8 Hz, 0.5H), 7.51 - 7.59 (m, 1H), 8.46 - 8.51 (m, 1H).

(Example 68)

Synthesis of (5aS,6R,11bS)-14-(cyclopropylmethyl)-10-methoxy-3-(3-(pyridin-2-yl)propyl)-2,3,4,5,6,7-hexahydro-6,11b-(epiminoethano)naphtho[1,2-d]azepin-5a(1H)-ol

[Chemical Formula 89]

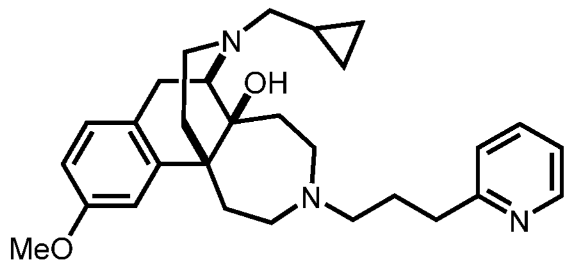

A solution of the compound (14 mg, 0.030 mmol) obtained in Example 67 in tetrahydrofuran (1.0 mL) was cooled to -20°C, and to the cooled solution, lithium aluminum hydride (3.1 mg, 0.082 mmol) was added, followed by stirring at -20°C for 2 hours, and then heating to 0°C and stirring for 1 hour. Thereafter, lithium aluminum hydride (3.1 mg, 0.082 mmol) was added, followed by stirring at 0°C for 25 hours. Thereafter, lithium aluminum hydride (3.1 mg, 0.082 mmol) was further added, followed by stirring at room temperature for 1 hour. After the reaction mixture was cooled to 0°C, a saturated Rochelle's salt aqueous solution was added to stop the reaction, and the temperature was raised to room temperature. Subsequently, the resultant mixture was stirred overnight and extracted three times with ethyl acetate. The combined extracts were washed with saturated saline, dried over sodium sulfate, and then concentrated under reduced pressure. The obtained crude product was purified by silica gel column chromatography (18 to 60% 2 M ammonia-methanol solution/ethyl acetate) to yield the title compound (3.0 mg, 22%) as a colorless oily matter.

$^1$H-NMR (400 MHz, $CDCl_3$)$\delta$ (ppm): 0.05 - 0.16 (m, 2H), 0.44 - 0.55 (m, 2H), 0.79 - 0.91 (m, 1H), 1.00 - 1.09 (m, 1H), 1.50 (ddd, J = 3, 5, 15 Hz, 1H), 1.74 - 2.03 (m, 5H), 2.09 (ddd, J = 5, 13, 13 Hz, 1H), 2.29 - 2.52 (m, 6H), 2.55 (ddd, J = 2, 6, 11 Hz, 1H), 2.59 - 2.69 (m, 3H), 2.75 - 2.86 (m, 2H), 2.95 (d, J = 6 Hz, 1H), 2.98 (d, J = 18 Hz, 1H), 3.09 (ddd, J = 2, 10, 13 Hz, 1H), 3.76 (s, 3H), 6.64 - 6.71 (m, 2H), 6.97 - 7.01 (m, 1H), 7.00 (d, J = 8 Hz, 1H), 7.06 (ddd, J = 1, 5, 7 Hz, 1H), 7.54 (ddd, J = 2, 7, 7 Hz, 1H), 8.49 (ddd, J = 1, 2, 5 Hz, 1H).

(Example 69)

Synthesis of (5aS,6R,11bS)-14-(cyclopropylmethyl)-3-(3-(pyridin-2-yl)propyl)-2,3,4,5,6,7-hexahydro-6,11b-(epiminoethano)naphtho[1,2-d]azepine-5a,10(1H)-diol

[Chemical Formula 90]

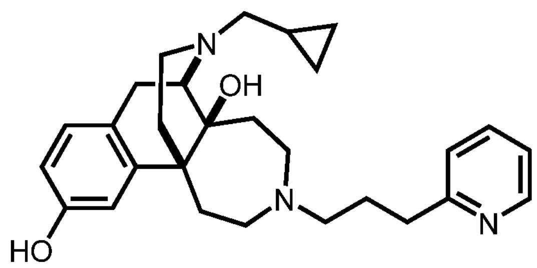

The title compound was obtained from the compound obtained in Example 68 according to the method described in Example 6.

$^1$H-NMR (400 MHz, $CDCl_3$)δ (ppm): 0.06 - 0.17 (m, 2H), 0.45 - 0.55 (m, 2H), 0.77 - 0.88 (m, 1H), 0.97 - 1.07 (m, 1H), 1.47 (ddd, J = 1, 6, 14 Hz, 1H), 1.73 - 1.90 (m, 4H), 1.95 - 2.09 (m, 3H), 2.32 - 2.68 (m, 9H), 2.76 (dd, J = 6, 18 Hz, 1H), 2.90 (d, J = 6 Hz, 1H), 2.94 - 3.08 (m, 2H), 2.95 (d, J = 18 Hz, 1H), 4.73 (br s, 1H), 6.59 (dd, J = 2, 8 Hz, 1H), 6.62 (d, J = 2 Hz, 1H), 6.89 (d, J = 8 Hz, 1H), 7.07 - 7.12 (m, 1H), 7.11 (dd, J = 5, 8 Hz, 1H), 7.58 (ddd, J = 2, 8, 8 Hz, 1H), 8.48 - 8.51 (m, 1H).

(Example 70)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-3-(pyridin-3-yl)propan-1-one

[Chemical Formula 91]

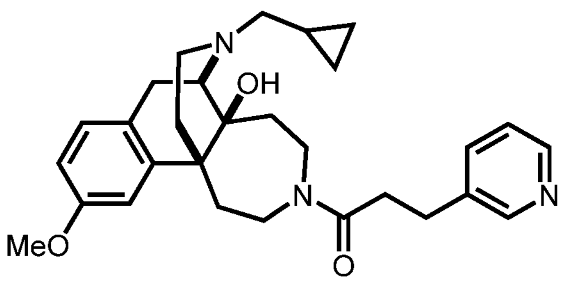

The title compound was obtained from the isomer C obtained in Example 2 and 3-(pyridin-3-yl)propanoic acid according to the method described in Example 7.

$^1$H-NMR (400 MHz, $CDCl_3$)δ (ppm): 0.06 - 0.16 (m, 2H), 0.46 - 0.57 (m, 2H), 0.77 - 0.88 (m, 1H), 1.01 - 1.12 (m, 1H), 1.57 (ddd, J = 3, 3, 14 Hz, 0.5H), 1.63 (ddd, J = 2, 5, 15 Hz, 0.5H), 1.78 (ddd, J = 2, 12, 14 Hz, 0.5H), 1.84 - 2.23 (m, 4.5H), 2.24 - 2.86 (m, 7H), 2.87 - 3.05 (m, 3.5H), 3.24 (ddd, J = 4, 4, 13 Hz, 0.5H), 3.32 - 3.43 (m, 1H), 3.65 - 3.76 (m, 0.5H), 3.70 (s, 1.5H), 3.79 (s, 1.5H), 3.83 (ddd, J = 3, 13, 13 Hz, 0.5H), 3.91 (ddd, J = 3, 4, 14 Hz, 0.5H), 4.22 (ddd, J = 2, 5, 14 Hz, 0.5H), 6.62 (d, J = 2 Hz, 0.5H), 6.67 (dd, J = 2, 8 Hz, 0.5H), 6.69 - 6.74 (m, 1H), 7.01 (d, J = 8 Hz, 0.5H), 7.03 (d, J = 8 Hz, 0.5H), 7.18 (dd, J = 5, 8 Hz, 1H), 7.45 (ddd, J = 2, 2, 8 Hz, 0.5H), 7.53 (ddd, J = 2, 2, 8 Hz, 0.5H), 8.38 - 8.49 (m, 2H).

(Example 71)

Synthesis of (5aS,6R,11bS)-14-(cyclopropylmethyl)-10-methoxy-3-(3-(pyridin-3-yl)propyl)-2,3,4,5,6,7-hexahydro-6,11b-(epiminoethano)naphtho[1,2-d]azepin-5a(1H)-ol

[Chemical Formula 92]

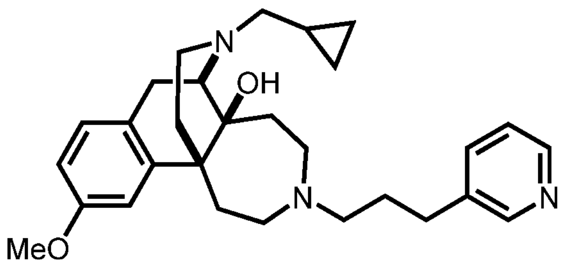

The title compound was obtained from the compound obtained in Example 70 according to the method described in Example 24.

$^1$H-NMR (400 MHz, $CDCl_3$)δ (ppm): 0.05 - 0.16 (m, 2H), 0.45 - 0.56 (m, 2H), 0.79 - 0.91 (m, 1H), 1.02 - 1.09 (m, 1H), 1.51 (ddd, J = 3, 4, 14 Hz, 1H), 1.59 - 1.72 (m, 2H), 1.84 (ddd, J = 4, 11, 14 Hz, 1H), 1.90 (ddd, J = 4, 4, 16 Hz, 1H), 1.98 (ddd, J = 3, 11, 12 Hz, 1H), 2.09 (ddd, J = 4, 12, 12 Hz, 1H), 2.32 - 2.46 (m, 8H), 2.52 - 2.62 (m, 2H), 2.78 - 2.90 (m, 2H), 2.96 (d, J = 6 Hz, 1H), 2.99 (d, J = 18 Hz, 1H), 3.06 (ddd, J = 2, 12, 13 Hz, 1H), 3.75 (s, 3H), 6.67 (dd, J = 2, 8 Hz, 1H), 6.71 (d, J = 2 Hz, 1H), 7.01 (d, J = 8 Hz, 1H), 7.16 (dd, J = 5, 8 Hz, 1H), 7.35 (ddd, J = 2, 2, 8 Hz, 1H), 8.29 - 8.31 (m, 1H), 8.38 - 8.41 (m, 1H).

(Example 72)

Synthesis of (5aS,6R,11bS)-14-(cyclopropylmethyl)-3-(3-(pyridin-3-yl)propyl)-2,3,4,5,6,7-hexahydro-6,11b-(epiminoethano)naphtho[1,2-d]azepine-5a,10(1H)-diol

[Chemical Formula 93]

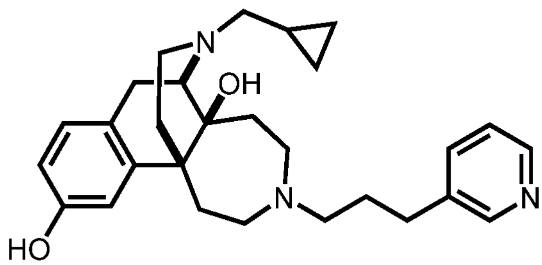

The title compound was obtained from the compound obtained in Example 71 according to the method described in Example 6.

$^1$H-NMR (400 MHz, $CDCl_3$)δ (ppm): 0.05 - 0.16 (m, 2H), 0.44 - 0.55 (m, 2H), 0.77 - 0.89 (m, 1H), 0.98 - 1.06 (m, 1H), 1.51 (dd, J = 5, 14 Hz, 1H), 1.59 - 1.77 (m, 3H), 1.78 - 1.87 (m, 1H), 1.96 - 2.07 (m, 2H), 2.28 - 2.47 (m, 7H), 2.49 - 2.58 (m, 3H), 2.78 (dd, J = 6, 18 Hz, 1H), 2.84 - 2.95 (m, 1H), 2.90 (d, J = 6 Hz, 1H), 2.95 (d, J = 18 Hz, 1H), 2.98 - 3.07 (m, 1H), 4.72 (br s, 1H), 6.56 - 6.61 (m, 2H), 6.91 (d, J = 8 Hz, 1H), 7.18 (dd, J = 5, 7 Hz, 1H), 7.41 (d, J = 7 Hz, 1H), 8.18 (s, 1H), 8.38 (d, J = 5 Hz, 1H).

(Example 73)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-3-(pyridin-4-yl)propan-1-one

[Chemical Formula 94]

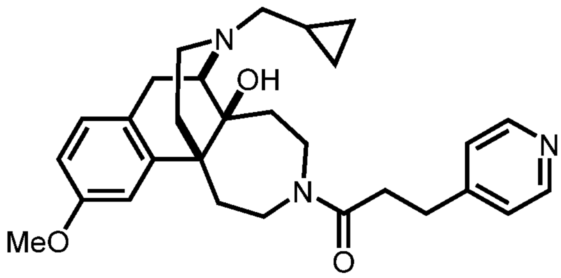

The title compound was obtained from the isomer C obtained in Example 2 and 3-(pyridin-4-yl)propanoic acid according to the method described in Example 7.

$^1$H-NMR (400 MHz, $CDCl_3$)δ (ppm): 0.03 - 0.16 (m, 2H), 0.45 - 0.58 (m, 2H), 0.76 - 0.91 (m, 1H), 1.01 - 1.13 (m, 1H), 1.58 (ddd, J = 4, 4, 14 Hz, 0.5H), 1.63 (ddd, J = 2, 5, 15 Hz, 0.5H), 1.77 (ddd, J = 3, 12, 15 Hz, 0.5H), 1.84 - 2.23 (m, 4H), 2.25 - 3.06 (m, 11H), 3.25 (ddd, J = 4, 4, 13 Hz, 0.5H), 3.36 (ddd, J = 1, 12, 14 Hz, 0.5H), 3.41 (ddd, J = 3, 6, 14 Hz, 0.5H), 3.66 - 3.80 (m, 0.5H), 3.69 (s, 1.5H), 3.78 (s, 1.5H), 3.83 (ddd, J = 3, 13, 13 Hz, 0.5H), 3.91 (ddd, J = 3, 5, 14 Hz, 0.5H), 4.22 (ddd, J = 3, 5, 14 Hz, 0.5H), 6.63 (d, J = 3 Hz, 0.5H), 6.67 (dd, J = 3, 8 Hz, 0.5H), 6.69 - 6.75 (m, 1H), 6.99 - 7.06 (m, 2H), 7.11 - 7.14 (m, 1H), 8.44 - 8.48 (m, 2H).

(Example 74)

Synthesis of (5aS,6R,11bS)-14-(cyclopropylmethyl)-10-methoxy-3-(3-(pyridin-4-yl)propyl)-2,3,4,5,6,7-hexahydro-6,11b-(epiminoethano)naphtho[1,2-d]azepin-5a(1H)-ol

[Chemical Formula 95]

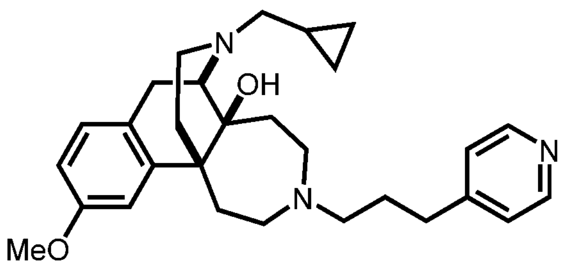

The title compound was obtained from the compound obtained in Example 73 according to the method described in Example 24.

$^1$H-NMR (400 MHz, $CDCl_3$)δ (ppm): 0.05 - 0.17 (m, 2H), 0.45 - 0.56 (m, 2H), 0.78 - 0.90 (m, 1H), 1.01 - 1.09 (m, 1H), 1.51 (ddd, J = 3, 5, 14 Hz, 1H), 1.55 - 1.72 (m, 2H), 1.84 (ddd, J = 4, 11, 14 Hz, 1H), 1.89 (ddd, J = 4, 4, 16 Hz, 1H), 1.98 (ddd, J = 3, 11, 11 Hz, 1H), 2.09 (ddd, J = 5, 13, 13 Hz, 1H), 2.29 - 2.46 (m, 8H), 2.52 - 2.61 (m, 2H), 2.82 (dd, J = 6, 18 Hz, 1H), 2.82 - 2.91 (m, 1H), 2.95 (d, J = 6 Hz, 1H), 2.99 (d, J = 18 Hz, 1H), 3.04 (ddd, J = 2, 12, 14 Hz, 1H), 3.75 (s, 3H), 6.66 (dd, J = 2, 8 Hz, 1H), 6.71 (d, J = 2 Hz, 1H), 6.94 - 6.98 (m, 2H), 7.01 (d, J = 8 Hz, 1H), 8.41 - 8.45 (m, 2H).

(Example 75)

Synthesis of (5aS,6R,11bS)-14-(cyclopropylmethyl)-3-(3-(pyridin-4-yl)propyl)-2,3,4,5,6,7-hexahydro-6,11b-(epiminoethano)naphtho[1,2-d]azepine-5a,10(1H)-diol

[Chemical Formula 96]

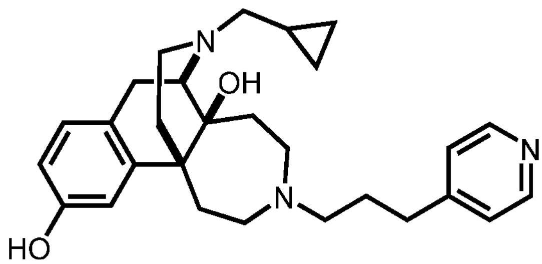

The title compound was obtained from the compound obtained in Example 74 according to the method described in Example 6.

$^1$H-NMR (400 MHz, $CDCl_3$)δ (ppm): 0.05 - 0.16 (m, 2H), 0.45 - 0.55 (m, 2H), 0.78 - 0.88 (m, 1H), 0.95 - 1.05 (m, 1H), 1.51 (dd, J = 5, 14 Hz, 1H), 1.63 - 1.81 (m, 3H), 1.83 (ddd, J = 3, 12, 14 Hz, 1H), 1.95 - 2.08 (m, 2H), 2.29 - 2.50 (m, 7H), 2.51 - 2.62 (m, 3H), 2.77 (dd, J = 6, 18 Hz, 1H), 2.85 - 2.94 (m, 1H), 2.90 (d, J = 6 Hz, 1H), 2.94 (d, J = 18 Hz, 1H), 2.99 - 3.08 (m, 1H), 4.74 (br s, 1H), 6.53 (d, J = 2 Hz, 1H), 6.56 (dd, J = 2, 8 Hz, 1H), 6.88 (d, J = 8 Hz, 1H), 6.97 - 7.00 (m, 2H), 8.41 - 8.44 (m, 2H).

(Example 76)

Synthesis of (5aS,6R,11bS)-14-(cyclopropylmethyl)-10-methoxy-3-(2-(pyridin-2-yl)ethyl)-2,3,4,5,6,7-hexahydro-6,11b-(epiminoethano)naphtho[1,2-d]azepin-5a(1H)-ol

[Chemical Formula 97]

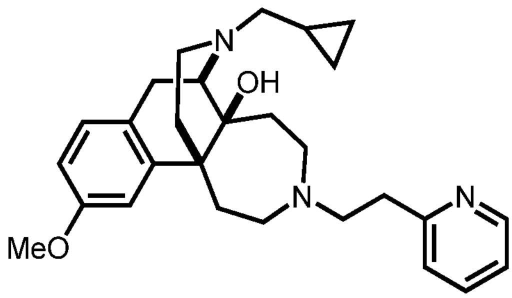

(Method 1)

The title compound was obtained from the isomer C obtained in Example 2 and 2-(pyridin-2-yl)ethyl 4-methylbenzenesulfonate (synthesized by the method described in Journal of Medicinal Chemistry, 2015, 58, 5842) according to the method described in Example 31.

(Method 2)

To a solution of the isomer C (257.7 mg, 0.75 mmol) obtained in Example 2 in ethanol (10 mL) were added acetic acid (430.1 μL, 7.52 mmol) and 2-vinylpyridine (807.3 μL, 7.52 mmol), followed by heating under reflux for 3.5 hours. After allowed to cool, the reaction solution was concentrated under reduced pressure. The obtained concentrated

residue was purified by silica gel column chromatography (amino group-supported silica gel, 0 to 3% methanol/chloroform) to yield the title compound (357.3 mg, quantitative) as a pale yellow oily matter.

$^1$H-NMR (400 MHz, $CDCl_3$)δ (ppm): 0.05 - 0.13 (m, 2H), 0.44 - 0.55 (m, 2H), 0.79 - 0.90 (m, 1H), 1.00 - 1.07 (m, 1H), 1.49 (ddd, J = 3, 4, 14 Hz, 1H), 1.81 (ddd, J = 4, 11, 15 Hz, 1H), 1.91 - 2.02 (m, 2H), 2.08 (ddd, J = 4, 12, 12 Hz, 1H), 2.28 - 2.40 (m, 3H), 2.50 - 2.61 (m, 2H), 2.71 - 2.91 (m, 7H), 2.94 (d, J = 6 Hz, 1H), 2.97 (d, J = 18 Hz, 1H), 3.20 (ddd, J = 3, 10, 13 Hz, 1H), 3.77 (s, 3H), 4.68 (br s, 1H), 6.66 - 6.71 (m, 2H), 6.98 - 7.04 (m, 2H), 7.05 (dd, J = 5, 8 Hz, 1H), 7.50 (ddd, J = 2, 8, 8 Hz, 1H), 8.45 - 8.49 (m, 1H).

(Example 77)

Synthesis of (5aS,6R,11bS)-14-(cyclopropylmethyl)-3-(2-(pyridin-2-yl)ethyl)-2,3,4,5,6,7-hexahydro-6,11b-(epiminoethano)naphtho[1,2-d]azepine-5a,10(1H)-diol

[Chemical Formula 98]

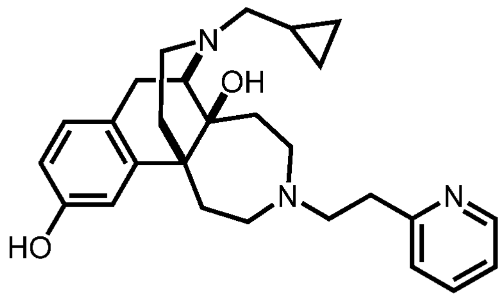

The title compound was obtained from the compound obtained in Example 76 according to the method described in Example 6.

$^1$H-NMR (400 MHz, $CDCl_3$)δ (ppm): 0.05 - 0.15 (m, 2H), 0.44 - 0.54 (m, 2H), 0.76 - 0.89 (m, 1H), 1.00 - 1.08 (m, 1H), 1.50 (ddd, J = 2, 6, 15 Hz, 1H), 1.78 - 1.89 (m, 2H), 1.96 - 2.08 (m, 2H), 2.25 - 2.39 (m, 3H), 2.48 - 2.58 (m, 1H), 2.72 - 2.84 (m, 2H), 2.73 (dd, J = 6, 18 Hz, 1H), 2.86 - 3.00 (m, 7H), 3.21 (ddd, J = 2, 11, 11 Hz, 1H), 4.73 (br s, 1H), 6.48 (d, J = 2 Hz, 1H), 6.57 (dd, J = 2, 8 Hz, 1H), 6.87 (d, J = 8 Hz, 1H), 7.08 - 7.13 (m, 2H), 7.56 (ddd, J = 2, 8, 8 Hz, 1H), 8.46 - 8.49 (m, 1H).

(Example 78)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-2-(pyridin-3-yl)ethan-1-one

[Chemical Formula 99]

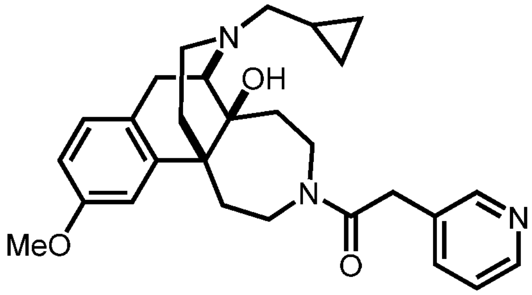

The title compound was obtained from the isomer C obtained in Example 2 and 2-(pyridin-3-yl)acetic acid according to the method described in Example 5.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.03 (d, J = 13 Hz, 1H), 1.45 - 1.55 (m, 1H), 1.80 - 1.90 (m, 2H), 1.99 (ddd, J = 4, 11, 11 Hz, 1H), 2.10 (ddd, J = 3, 13, 13 Hz, 1H), 2.30 - 2.70 (m, 6H), 2.70 - 2.90 (m, 2H), 2.90 - 3.00 (m, 2H), 3.07 (ddd, J = 3, 11, 13 Hz, 1H), 3.54 (s, 2H), 3.68 (s, 3H), 4.78 (br s, 1H), 6.62 (d, J = 2 Hz, 1H), 6.71 (dd, J = 2, 8 Hz, 1H), 7.00 - 7.10 (m, 3H), 7.10 - 7.25 (m, 2H).

(Example 79)

Synthesis of (5aS,6R,11bS)-14-(cyclopropylmethyl)-10-methoxy-3-(2-(pyridin-3-yl)ethyl)-2,3,4,5,6,7-hexahydro-6,11b-(epiminoethano)naphtho[1,2-d]azepin-5a(1H)-ol

[Chemical Formula 100]

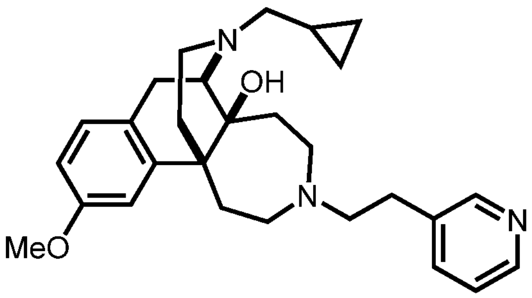

The title compound was obtained from the compound obtained in Example 78 according to the method described in Example 24.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.00 - 1.10 (m, 1H), 1.20 - 1.30 (m, 1H), 1.65 - 1.80 (m, 2H), 1.80 - 1.95 (m, 2H), 1.95 - 2.10 (m, 1H), 2.30 - 2.45 (m, 2H), 2.50 - 2.55 (m, 1H), 2.60 - 2.85 (m, 7H), 2.85 - 3.10 (m, 3H), 3.10 - 3.25 (m, 1H), 3.77 (s, 3H), 6.55 - 6.65 (m, 2H), 7.00 - 7.05 (m, 2H), 7.05 - 7.10 (m, 1H), 7.10 - 7.15 (m, 1H), 7.25 - 7.40 (m, 1H).

(Example 80)

Synthesis of (5aS,6R,11bS)-14-(cyclopropylmethyl)-3-(2-(pyridin-3-yl)ethyl)-2,3,4,5,6,7-hexahydro-6,11b-(epiminoethano)naphtho[1,2-d]azepine-5a,10(1H)-diol

[Chemical Formula 101]

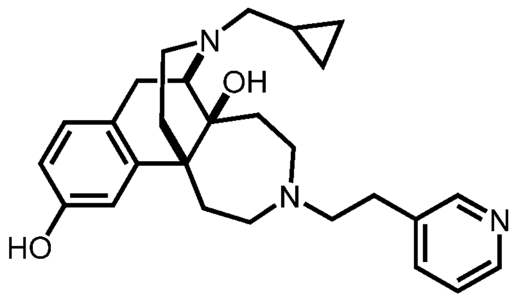

The title compound was obtained from the compound obtained in Example 79 according to the method described in Example 6.

$^1$H-NMR (400 MHz, $CDCl_3$)δ (ppm): 0.00 - 0.15 (m, 2H), 0.45 - 0.55 (m, 2H), 0.80 - 0.90 (m, 1H), 1.04 (d, J = 11 Hz, 1H), 1.50 (dd, J = 5, 14 Hz, 1H), 1.70 - 1.90 (m, 2H), 1.95 - 2.10 (m, 3H), 2.30 - 2.40 (m, 3H), 2.50 - 2.60 (m, 1H), 2.60 - 2.80 (m, 6H), 2.85 - 3.00 (m, 3H), 3.15 - 3.25 (m, 1H), 6.56 (d, J = 2 Hz, 1H), 6.63 (dd, J = 2, 8 Hz, 1H), 6.93

(d, J = 8 Hz, 1H), 7.16 (dd, J = 6, 8 Hz, 1H), 7.42 (ddd, J = 2, 2, 6 Hz, 1H), 8.35 (d, J = 2 Hz, 1H), 8.40 (dd, J = 2, 6 Hz, 1H).

(Example 81)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-2-(pyridin-4-yl)ethan-1-one

[Chemical Formula 102]

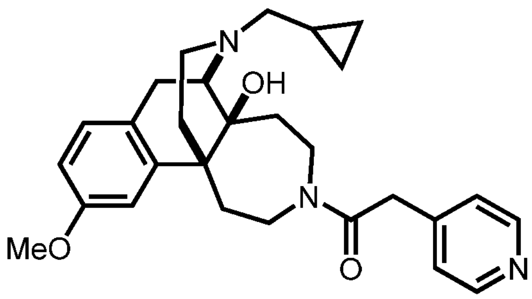

The title compound was obtained from the isomer C obtained in Example 2 and 2-(pyridin-4-yl)acetic acid according to the method described in Example 5.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.00 - 1.10 (m, 1H), 1.50 - 1.70 (m, 1H), 1.70 - 1.90 (m, 1H), 1.90 - 2.50 (m, 5H), 2.50 - 2.75 (m, 1.5H), 2.75 - 3.05 (m, 2.5H), 3.10 - 3.25 (m, 0.5H), 3.25 - 3.35 (m, 0.5H), 3.35 - 3.50 (m, 2H), 3.65 - 4.00 (m, 3.5H), 3.75 (s, 1.5H), 3.79 (s, 1.5H), 4.05 - 4.20 (m, 0.5H), 4.50 (br s, 1H), 6.61 (d, J = 3 Hz, 0.5H), 6.65 - 6.75 (m, 1.5H), 6.90 - 7.00 (m, 1H), 7.03 (d, J = 8 Hz, 1H), 7.08 (d, J = 6 Hz, 1H), 8.40 - 8.50 (m, 2H).

Example 82

Synthesis of (5aS,6R,11bS)-14-(cyclopropylmethyl)-3-(2-(pyridin-4-yl)ethyl)-2,3,4,5,6,7-hexahydro-6,11b-(epiminoethano)naphtho[1,2-d]azepine-5a, 10(1H)-diol

[Chemical Formula 103]

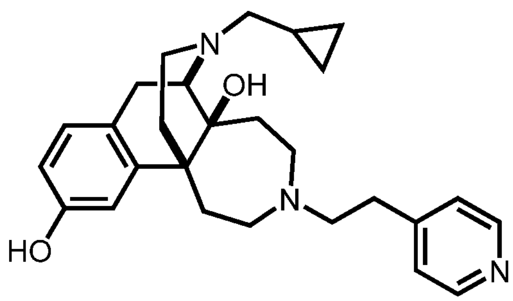

The title compound was obtained from the compound obtained in Example 81 according to the method described in Example 24.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.00 - 1.10 (m, 1H), 1.45 - 1.55 (m, 1H), 1.70 - 1.90 (m, 2H), 1.90 - 2.10 (m, 3H), 2.20 - 2.50 (m, 3H), 2.50 - 2.85 (m, 7H), 2.85 - 3.15 (m, 4H), 4.65 (br s, 1H), 6.52 - 6.65 (m, 2H), 6.93 (d, J = 8 Hz, 1H), 6.98 (d, J = 8 Hz, 2H), 8.37 (d, J = 5 Hz, 2H).

(Example 83)

Synthesis of (5aS,6R,1l1bS)-14-(cyclopropylmethyl)-3-(2-(pyrimidin-2-yl)ethyl)-2,3,4,5,6,7-hexahydro-6,11b-(epiminoethano)naphtho[1,2-d]azepine-5a,10(1H)-diol

[Chemical Formula 104]

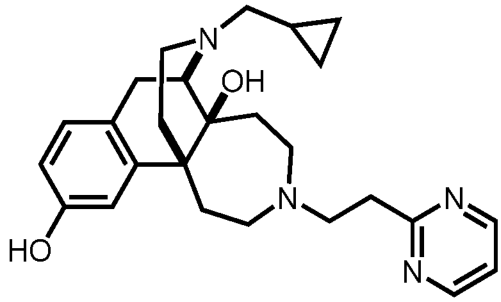

The title compound was obtained from the compound E obtained in Example 3 and 2-(pyrimidin-2-yl)ethan-1-ol according to the method described in Example 32.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.05 (d, J = 10 Hz, 1H), 1.51 (dd, J = 6, 15 Hz, 1H), 1.80 - 2.10 (m, 4H), 2.25 - 2.40 (m, 3H), 2.54 (d, J = 7 Hz, 1H), 2.73 (dd, J = 6, 17 Hz, 1H), 2.80 - 3.00 (m, 5H), 3.10 - 3.25 (m, 4H), 3.25 - 3.40 (t, J = 11 Hz, 1H), 6.52 (s, 1H), 6.55 (d, J = 8 Hz, 1H), 6.86 (d, J = 8 Hz, 1H), 7.10 (t, J = 5 Hz, 1H), 8.62 (d, J = 5 Hz, 2H).

(Example 84)

Synthesis of (5aS,6R,11bS)-14-(cyclopropylmethyl)-3-(2-(pyrazin-2-yl)ethyl)-2,3,4,5,6,7-hexahydro-6,11b-(epiminoethano)naphtho[1,2-d]azepine-5a,10(1H)-diol

[Chemical Formula 105]

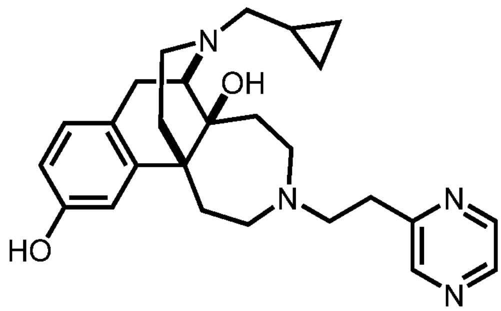

The title compound was obtained from the compound E obtained in Example 3 and 2-(pyrazin-2-yl)ethyl methanesulfonate (synthesized by the method described in WO 2017223239) according to the method described in Example 31.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.02 (d, J = 9 Hz, 1H), 1.50 (dd, J = 4, 14 Hz, 1H), 1.70 - 1.90 (m, 2H), 1.90 - 2.10 (m, 2H), 2.30 - 2.40 (m, 3H), 2.50 - 2.60 (m, 1H), 2.70 - 2.85 (m, 3H), 2.85 - 3.05 (m, 7H), 3.24 (t, J = 12 Hz, 1H), 6.49 (d, J = 2 Hz, 1H), 6.57 (dd, J = 2, 8 Hz, 1H), 6.89 (d, J = 8 Hz, 1H), 8.30 - 8.40 (m, 2H), 8.40 - 8.50 (m, 1H).

(Example 85)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(6-methoxypyridin-2-yl)ethan-1-one

[Chemical Formula 106]

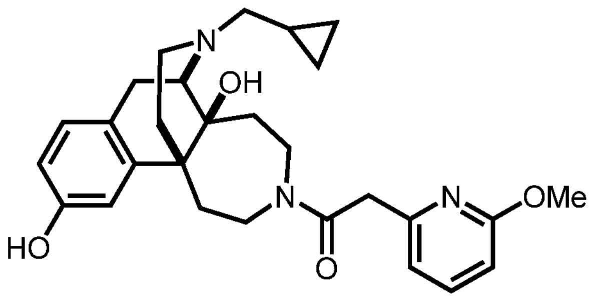

A solution of the compound E (7.8 mg, 0.0237 mmol) obtained in Example 3, 2-(6-methoxypyridin-2-yl)acetic acid (10 mg, 0.0609 mmol), N,N-dimethylaminopyridine (1.9 mg, 0.0152 mmol), 1-hydroxybenztriazole monohydrate (6.0 mg, 0.0395 mmol), N,N-diisopropylethylamine (26 μL, 0.152 mmol), and 1-ethyl-3-(3-dimethylaminopropyl)carbodiimide hydrochloride (7.6 mg, 0.0395 mmol) in N,N-dimethylformamide (1 mL) was stirred at room temperature overnight. Then, to the solution, a suspension of potassium carbonate (50 mg, 0.36 mmol) in methanol (1 mL) was added, followed by stirring at room temperature for 4 hours. The reaction mixture was extracted four times with ethyl acetate, and then the combined extracts were dried over sodium sulfate and concentrated under reduced pressure. The obtained crude product was purified by silica gel column chromatography (3 to 15% methanol/chloroform) to yield the title compound (11 mg, 97%) as a white powder.

$^1$H-NMR (400 MHz, $CDCl_3$)δ (ppm): 0.04 - 0.18 (m, 2H), 0.43 - 0.58 (m, 2H), 0.75 - 0.93 (m, 1H), 0.98 - 1.15 (m, 1H), 1.17 - 2.21 (m, 5.5H), 2.24 - 3.04 (m, 6.5H), 3.25 - 3.34 (m, 0.5H), 3.40 - 3.99 (m, 7.5H), 4.02 - 4.18 (m, 1H), 6.51 - 6.84 (m, 4H), 6.88 - 6.98 (m, 1H), 7.39 - 7.50 (m, 1H).

(Example 86)

Synthesis of (5aS,6R,11bS)-14-(cyclopropylmethyl)-3-(2-(6-methoxypyridin-2-yl)ethyl)-2,3,4,5,6,7-hexahydro-6,11b-(epiminoethano)naphtho[1,2-d]azepine-5a,10(1H)-diol

[Chemical Formula 107]

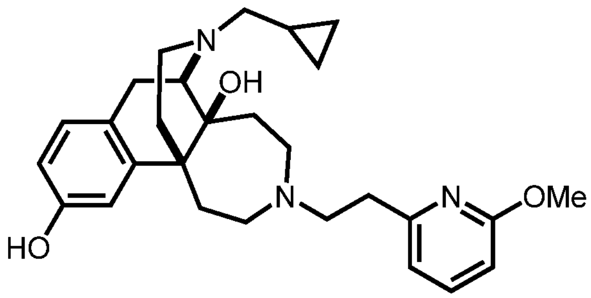

The title compound was obtained from the compound obtained in Example 85 according to the method described in Example 24.

$^1$H-NMR (400 MHz, $CDCl_3$)δ (ppm): 0.03 - 0.17 (m, 2H), 0.41 - 0.56 (m, 2H), 0.76 - 0.91 (m, 1H), 0.94 - 2.11 (m, 6H), 2.26 - 2.40 (m, 3H), 2.47 - 2.58 (m, 1H), 2.67 - 3.03 (m, 10H), 3.13 - 3.25 (m, 1H), 3.89 (s, 3H), 4.73 (br s, 1H), 6.44 - 6.53 (m, 2H), 6.56 (dd, J = 2, 8 Hz, 1H)6.65 (d, J = 7 Hz, 1H), 6.89 (d, J = 8 Hz, 1H), 7.37 - 7.45 (m, 1H).

(Example 87)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(6-(trifluoromethyl)pyridin-2-yl)ethan-1-one

[Chemical Formula 108]

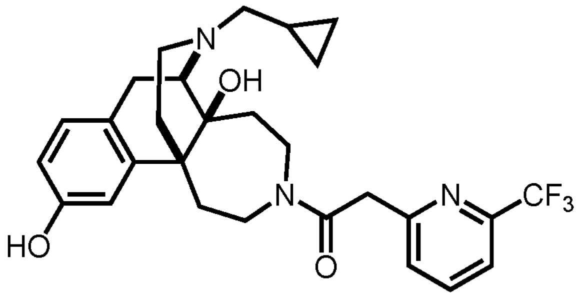

The title compound was obtained from the compound E obtained in Example 3 and 2-(6-(trifluoromethyl)pyridin-2-yl)acetic acid according to the method described in Example 85.

$^1$H-NMR (400 MHz, $CDCl_3$)δ (ppm): 0.04 - 0.17 (m, 2H), 0.44 - 0.57 (m, 2H), 0.74 - 0.93 (m, 1H), 1.01 - 1.13 (m, 1H), 1.19 - 1.69 (m, 1H), 1.70 - 1.89 (m, 1H), 1.90 - 2.19 (m, 3H), 2.22 - 2.42 (m, 2.6H), 2.45 - 2.59 (m, 1.4H), 2.61 - 3.02 (m, 3H), 3.24 - 3.48 (m, 1H), 3.49 - 3.61 (m, 1H), 3.64 - 4.08 (m, 4H), 4.28 - 4.60 (m, 1H), 6.58 - 6.68 (m, 1.4H), 6.73 (d, J = 2 Hz, 0.6H), 6.87 - 6.97 (m, 1H), 7.37 - 7.45 (m, 1H), 7.49 - 7.56 (m, 1H), 7.75 (dd, J = 8, 8 Hz, 1H).

(Example 88)

Synthesis of (5aS,6R,11bS)-14-(cyclopropylmethyl)-3-(2-(6-(trifluoromethyl)pyridin-2-yl)ethyl)-2,3,4,5,6,7-hexahydro-6,11b-(epiminoethano)naphtho[1,2-d]azepine-5a,10(1H)-diol

[Chemical Formula 109]

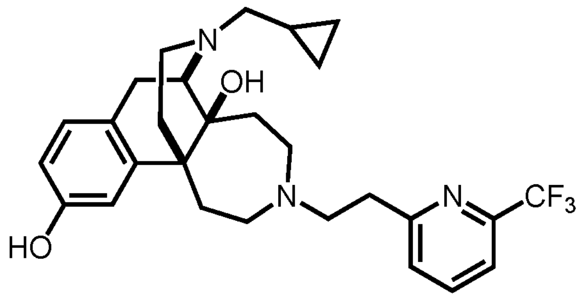

The title compound was obtained from the compound obtained in Example 87 according to the method described in Example 24.

$^1$H-NMR (400 MHz, $CDCl_3$)δ (ppm): 0.02 - 0.16 (m, 2H), 0.41 - 0.56 (m, 2H), 0.75 - 0.91 (m, 1H), 0.95 - 1.07 (m, 1H), 1.15 - 2.11 (m, 5H), 2.26 - 2.42 (m, 3H), 2.46 - 2.57 (m, 1H), 2.66 (ddd, J = 3, 5, 13 Hz, 1H), 2.69 - 2.80 (m, 2H), 2.85 - 3.03 (m, 7H), 3.10 - 3.22 (m, 1H), 4.68 (br s, 1H), 6.48 - 6.60 (m, 2H), 6.94 (d, J = 8 Hz, 1H), 7.20 (d, J = 8 Hz, 1H), 7.44 (d, J = 8 Hz, 1H), 7.65 (dd, J = 8, 8 Hz, 1H).

(Example 89)

Synthesis of (5aS,6R,11bS)-14-(cyclopropylmethyl)-10-methoxy-3-phenethyl-2,3,4,5,6,7-hexahydro-6,11b-(epiminoethano)naphtho[1,2-d]azepin-5a(1H)-ol

[Chemical Formula 110]

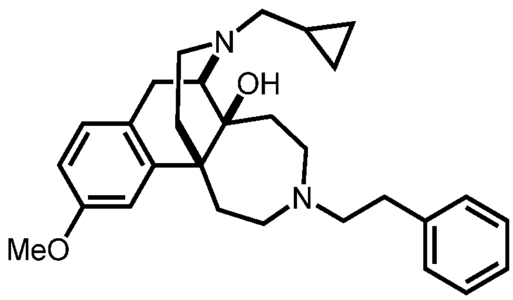

The title compound was obtained from the isomer C obtained in Example 2 and phenethyl 4-methylbenzenesulfonate (synthesized by the method described in Journal of the American Chemical Society, 2012, 134, 11408) according to the method described in Example 31.

$^{1}$H-NMR (400 MHz, $CDCl_3$)$\delta$ (ppm): 0.05 - 0.15 (m, 2H), 0.45 - 0.55 (m, 2H), 0.79 - 0.91 (m, 1H), 1.01 - 1.08 (m, 1H), 1.50 (ddd, J = 3, 5, 15 Hz, 1H), 1.84 (ddd, J = 4, 12, 15 Hz, 1H), 1.92 - 2.03 (m, 2H), 2.09 (ddd, J = 5, 13, 13 Hz, 1H), 2.29 - 2.38 (m, 3H), 2.52 - 2.60 (m, 2H), 2.62 - 2.83 (m, 6H), 2.87 (ddd, J = 2, 11, 13 Hz, 1H), 2.94 (d, J = 6 Hz, 1H), 2.98 (d, J = 18 Hz, 1H), 3.21 (ddd, J = 3, 12, 13 Hz, 1H), 3.77 (s, 3H), 4.72 (br s, 1H), 6.68 - 6.73 (m, 2H), 6.99 - 7.04 (m, 1H), 7.07 - 7.17 (m, 3H), 7.19 - 7.25 (m, 2H).

(Example 90)

Synthesis of (5aS,6R,11bS)-14-(cyclopropylmethyl)-3-phenethyl-2,3,4,5,6,7-hexahydro-6,11b-(epiminoethano)naphtho[1,2-d]azepine-5a,10(1H)-diol

[Chemical Formula 111]

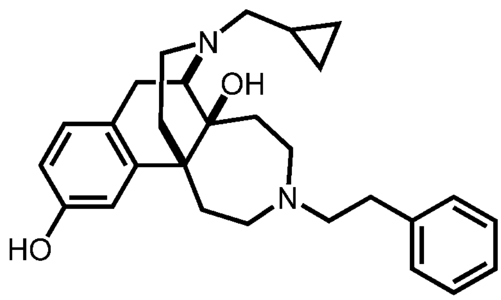

The title compound was obtained from the compound obtained in Example 89 according to the method described in Example 6.

$^{1}$H-NMR (400 MHz, $CDCl_3$)$\delta$ (ppm): 0.03 - 0.13 (m, 2H), 0.43 - 0.54 (m, 2H), 0.75 - 0.87 (m, 1H), 0.97 - 1.06 (m, 1H), 1.41 - 1.52 (m, 1H), 1.70 - 1.85 (m, 2H), 1.92 - 2.04 (m, 2H), 2.16 - 2.37 (m, 3H), 2.45 - 2.55 (m, 1H), 2.62 - 2.97 (m, 10H), 3.07 - 3.21 (m, 1H), 4.79 (br s, 1H), 6.42 (s, 1H), 6.53 (d, J = 8 Hz, 1H), 6.84 (d, J = 8 Hz, 1H), 7.10 - 7.19 (m, 3H), 7.23 (d, J = 8 Hz, 2H).

(Example 91)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-3-phenylpropan-1-one

[Chemical Formula 112]

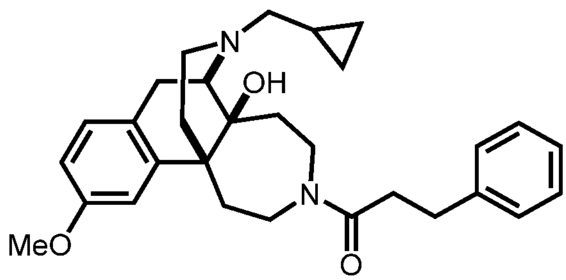

The title compound was obtained from the isomer C obtained in Example 2 and 3-phenylpropanoic acid according to the method described in Example 7.

$^1$H-NMR (400 MHz, $CDCl_3$)δ (ppm): 0.05 - 0.16 (m, 2H), 0.46 - 0.56 (m, 2H), 0.76 - 0.88 (m, 1H), 1.01 - 1.12 (m, 1H), 1.56 (ddd, J = 3, 3, 15 Hz, 0.5H), 1.63 (ddd, J = 2, 5, 15 Hz, 0.5H), 1.79 (ddd, J = 3, 12, 15 Hz, 0.5H), 1.83 - 2.03 (m, 2H), 2.08 (ddd, J = 5, 13, 13 Hz, 0.5H), 2.15 - 2.20 (m, 1H), 2.26 - 2.66 (m, 5.5H), 2.68 (ddd, J = 4, 6, 10 Hz, 0.5H), 2.73 - 3.05 (m, 5.5H), 3.25 (ddd, J = 4, 4, 13 Hz, 0.5H), 3.34 - 3.45 (m, 1H), 3.64 - 3.74 (m, 0.5H), 3.69 (s, 1.5H), 3.79 (s, 1.5H), 3.83 (ddd, J = 3, 12, 12 Hz, 0.5H), 3.91 (ddd, J = 2, 4, 14 Hz, 0.5H), 4.21 (ddd, J = 3, 5, 14 Hz, 0.5H), 6.62 (d, J = 3 Hz, 0.5H), 6.68 (dd, J = 3, 8 Hz, 0.5H), 6.70 - 6.74 (m, 1H), 7.01 (d, J = 8 Hz, 0.5H), 7.02 (d, J = 8 Hz, 0.5H), 7.12 - 7.32 (m, 5H).

(Example 92)

Synthesis of (5aS,6R,11bS)-14-(cyclopropylmethyl)-10-methoxy-3-(3-phenylpropyl)-2,3,4,5,6,7-hexahydro-6,11b-(epiminoethano)naphtho[1,2-d]azepin-5a(1H)-ol

[Chemical Formula 113]

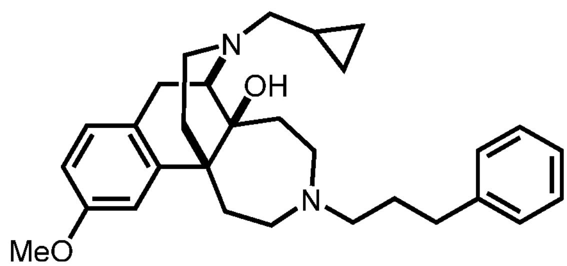

The title compound was obtained from the compound obtained in Example 91 according to the method described in Example 24.

$^1$H-NMR (400 MHz, $CDCl_3$)δ (ppm): 0.05 - 0.16 (m, 2H), 0.45 - 0.54 (m, 2H), 0.80 - 0.91 (m, 1H), 1.01 - 1.09 (m, 1H), 1.50 (ddd, J = 3, 4, 15 Hz, 1H), 1.62 - 1.72 (m, 2H), 1.84 (ddd, J = 4, 12, 15 Hz, 1H), 1.89 - 2.02 (m, 2H), 2.10 (ddd, J = 5, 13, 13 Hz, 1H), 2.29 - 2.48 (m, 8H), 2.55 (ddd, J = 2, 5, 11 Hz, 1H), 2.62 (ddd, J = 4, 4, 13 Hz, 1H), 2.76 - 2.85 (m, 2H), 2.96 (d, J = 6 Hz, 1H), 2.98 (d, J = 18 Hz, 1H), 3.07 (ddd, J = 3, 11, 13 Hz, 1H), 3.76 (s, 3H), 4.79 (br s, 1H), 6.67 (dd, J = 3, 8 Hz, 1H), 6.70 (d, J = 3 Hz, 1H), 7.00 (d, J = 8 Hz, 1H), 7.06 - 7.10 (m, 2H), 7.14 (tt, J = 2, 7 Hz, 1H), 7.21 - 7.27 (m, 2H).

(Example 93)

Synthesis of (5aS,6R,11bS)-14-(cyclopropylmethyl)-3-(3-phenylpropyl)-2,3,4,5,6,7-hexahydro-6,11b-(epiminoethano)naphtho[1,2-d]azepine-5a,10(1H)-diol

[Chemical Formula 114]

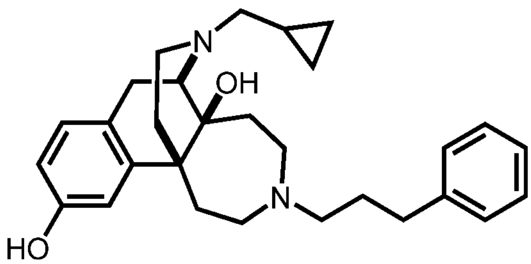

The title compound was obtained from the compound obtained in Example 92 according to the method described in Example 6.

$^1$H-NMR (400 MHz, $CDCl_3$)δ (ppm): 0.06 - 0.17 (m, 2H), 0.46 - 0.54 (m, 2H), 0.78 - 0.88 (m, 1H), 0.95 - 1.03 (m, 1H), 1.48 (ddd, J = 2, 6, 15 Hz, 1H), 1.71 - 1.88 (m, 4H), 1.98 - 2.04 (m, 2H), 2.25 - 2.39 (m, 3H), 2.48 - 2.64 (m, 5H), 2.66 - 2.77 (m, 3H), 2.83 - 2.92 (m, 1H), 2.88 (d, J = 6 Hz, 1H), 2.93 (d, J = 18 Hz, 1H), 3.05 - 3.15 (m, 1H), 4.80 (br s, 1H), 6.42 (d, J = 3 Hz, 1H), 6.53 (dd, J = 3, 8 Hz, 1H), 6.87 (d, J = 8 Hz, 1H), 7.11 - 7.18 (m, 3H), 7.21 - 7.28 (m, 2H).

(Example 94)

Synthesis of (5aS,6R,11bS)-14-(cyclopropylmethyl)-3-(2-fluorophenethyl)-10-methoxy-2,3,4,5,6,7-hexahydro-6,11b-(epiminoethano)naphtho[1,2-d]azepin-5a(1H)-ol

[Chemical Formula 115]

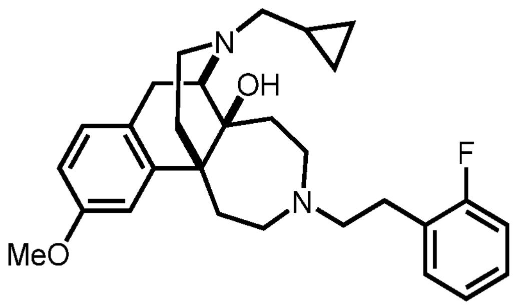

The title compound was obtained from the isomer C obtained in Example 2 and 2-(2-fluorophenyl)ethan-1-ol according to the method described in Example 32.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.04 (d, J = 14 Hz, 1H), 1.45 - 1.55 (m, 1H), 1.75 - 1.90 (m, 1H), 1.90 - 2.15 (m, 3H), 2.25 - 2.40 (m, 2H), 2.50 - 2.60 (m, 2H), 2.60 - 3.00 (m, 9H), 3.15 - 3.25 (m, 1H), 3.77 (s, 3H), 3.80 - 3.90 (m, 1H), 4.70 (br s, 1H), 6.65 - 6.75 (m, 2H), 6.90 - 7.20 (m, 5H).

(Example 95)

Synthesis of (5aS,6R,11bS)-14-(cyclopropylmethyl)-3-(2-fluorophenethyl)-2,3,4,5,6,7-hexahydro-6,11b-(epiminoethano)naphtho[1,2-d]azepine-5a,10(1H)-diol

[Chemical Formula 116]

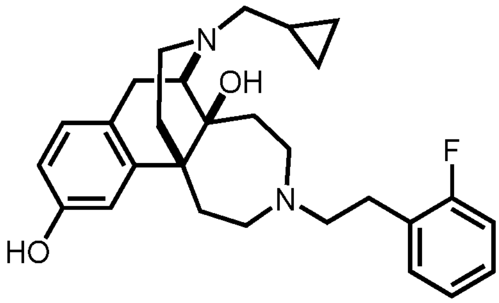

The title compound was obtained from the compound obtained in Example 94 according to the method described in Example 6.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.04 (d, J = 11 Hz, 1H), 1.52 (dd, J = 4, 15 Hz, 1H), 1.80 - 1.90 (m, 2H), 1.95 - 2.10 (m, 2H), 2.25 - 2.40 (m, 3H), 2.50 - 2.60 (m, 1H), 2.70 - 3.10 (m, 10H), 3.18 - 3.33 (m, 1H), 4.75 (br s, 1H), 6.47 (d, J = 3 Hz, 1H), 6.57 (dd, J = 3, 8 Hz, 1H), 6.89 (d, J = 8 Hz, 1H), 6.95 - 7.05 (m, 2H), 7.10 - 7.20 (m, 2H).

(Example 96)

Synthesis of (5aS,6R,11bS)-14-(cyclopropylmethyl)-3-(2-methoxyphenethyl)-2,3,4,5,6,7-hexahydro-6,11b-(epiminoethano)naphtho[1,2-d]azepine-5a,10(1H)-diol

[Chemical Formula 117]

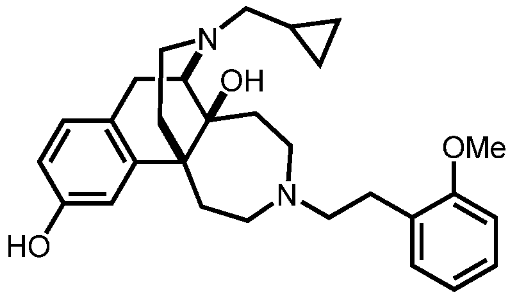

The title compound was obtained from the compound E obtained in Example 3 and 2-methoxyphenethyl 4-methylbenzenesulfonate (synthesized by the method described in WO 2010021149) according to the method described in Example 31.

$^1$H-NMR (400 MHz, $CDCl_3$)δ (ppm): 0.05 - 0.15 (m, 2H), 0.45 - 0.54 (m, 2H), 0.77 - 0.88 (m, 1H), 1.00 - 1.07 (m, 1H), 1.52 (ddd, J = 2, 6, 14 Hz, 1H), 1.78 - 1.88 (m, 2H), 1.96 - 2.09 (m, 2H), 2.27 - 2.41 (m, 3H), 2.46 - 2.61 (m, 1H), 2.69 - 3.04 (m, 10H), 3.26 (dd, J = 12, 12 Hz, 1H), 3.78 (s, 3H), 4.79 (br s, 1H), 6.47 (d, J = 2 Hz, 1H), 6.56 (dd, J = 2, 8 Hz, 1H), 6.79 - 6.90 (m, 3H), 7.10 (dd, J = 1, 7 Hz, 1H), 7.16 (ddd, J = 1, 8, 8 Hz, 1H).

(Example 97)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(3-methoxypyridin-2-yl)ethan-1-one

[Chemical Formula 118]

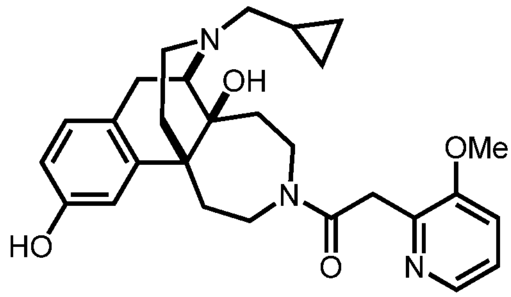

The title compound was obtained from the compound E obtained in Example 3 and 2-(3-methoxypyridin-2-yl)acetic acid according to the method described in Example 7.

$^1$H-NMR (400 MHz, $CDCl_3$)$\delta$ (ppm): 0.04 - 0.16 (m, 2H), 0.43 - 0.57 (m, 2H), 0.76 - 0.87 (m, 1H), 1.00 - 1.11 (m, 1H), 1.52 (ddd, J = 4, 4, 15 Hz, 0.6H), 1.52 - 1.61 (m, 0.4H), 1.76 - 1.90 (m, 1H), 1.91 - 2.08 (m, 2.4H), 2.09 - 2.18 (m, 0.6H), 2.19 - 2.43 (m, 2.6H), 2.47 - 2.59 (m, 1.4H), 2.69 (dd, J = 6, 18 Hz, 0.6H), 2.76 (dd, J = 6, 18 Hz, 0.4H), 2.84 (d, J = 6 Hz, 0.6H), 2.88 - 2.95 (m, 0.8H), 2.96 (d, J = 18 Hz, 0.6H), 3.08 - 3.23 (m, 0.6H), 3.27 - 3.45 (m, 1.4H), 3.64 (d, J = 16 Hz, 0.4H), 3.64 - 3.76 (m, 0.4H), 3.73 (s, 3H), 3.80 (d, J = 16 Hz, 0.4H), 3.85 - 3.98 (m, 2.2H), 4.22 (ddd, J = 2, 5, 14 Hz, 0.6H), 6.59 (dd, J = 2, 8 Hz, 0.6H), 6.61 (dd, J = 2, 8 Hz, 0.4H), 6.66 (d, J = 2 Hz, 0.4H), 6.80 (d, J = 2 Hz, 0.6H), 6.88 (d, J = 8 Hz, 0.4H), 6.89 (d, J = 8 Hz, 0.6H), 7.07 - 7.17 (m, 2H), 8.06 - 8.10 (m, 1H).

(Example 98)

Synthesis of (5aS,6R,11bS)-14-(cyclopropylmethyl)-3-(2-(3-methoxypyridin-2-yl)ethyl)-2,3,4,5,6,7-hexahydro-6,11b-(epiminoethano)naphtho[1,2-d]azepine-5a,10(1H)-diol

[Chemical Formula 119]

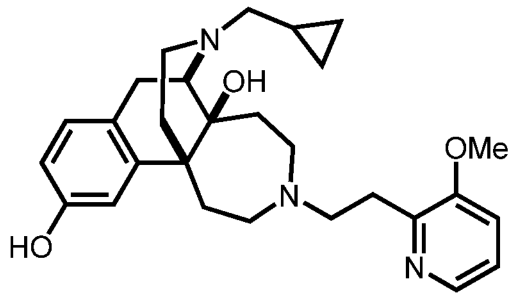

The title compound was obtained from the compound obtained in Example 97 according to the method described in Example 24.

$^1$H-NMR (400 MHz, $CDCl_3$)$\delta$ (ppm): 0.04 - 0.14 (m, 2H), 0.44 - 0.54 (m, 2H), 0.77 - 0.88 (m, 1H), 1.01 - 1.07 (m, 1H), 1.51 (ddd, J = 2, 5, 14 Hz, 1H), 1.80 - 1.92 (m, 2H), 1.96 - 2.09 (m, 2H), 2.26 - 2.39 (m, 3H), 2.50 - 2.55 (m, 1H), 2.72 (dd, J = 6, 18 Hz, 1H), 2.81 - 3.10 (m, 9H), 3.21 - 3.30 (m, 1H), 3.78 (s, 3H), 4.78 (br s, 1H), 6.47 (d, J = 2 Hz, 1H), 6.55 (dd, J = 2, 8 Hz, 1H), 6.85 (d, J = 8 Hz, 1H), 7.08 (dd, J = 2, 8 Hz, 1H), 7.10 (dd, J = 4, 8 Hz, 1H), 8.07 (dd, J = 2, 4 Hz, 1H).

(Example 99)

Synthesis of (5aS,6R,11bS)-14-(cyclopropylmethyl)-3-(2-(3-fluoropyridin-2-yl)ethyl)-10-methoxy-2,3,4,5,6,7-hexahydro-6,11b-(epiminoethano)naphtho[1,2-d]azepin-5a(1H)-ol

[Chemical Formula 120]

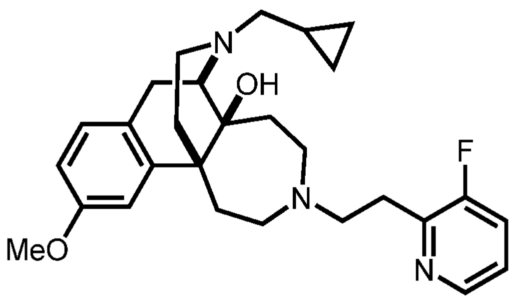

The title compound was obtained from the isomer C obtained in Example 2 and 2-(3-fluoropyridin-2-yl)ethyl 4-methylbenzenesulfonate according to the method described in Example 31.

$^1$H-NMR (400 MHz, $CDCl_3$)δ (ppm): 0.04 - 0.14 (m, 2H), 0.44 - 0.55 (m, 2H), 0.78 - 0.90 (m, 1H), 0.98 - 1.07 (m, 1H), 1.51 (ddd, J = 3, 4, 15 Hz, 1H), 1.82 (ddd, J = 4, 12, 15 Hz, 1H), 1.92 - 2.01 (m, 2H), 2.08 (ddd, J = 5, 13, 13 Hz, 1H), 2.25 - 2.40 (m, 3H), 2.54 (ddd, J = 2, 4, 11 Hz, 1H), 2.61 (ddd, J = 4, 4, 13 Hz, 1H), 2.72 - 3.02 (m, 9H), 3.20 (ddd, J = 3, 12, 13 Hz, 1H), 3.77 (s, 3H), 4.67 (br s, 1H), 6.65 - 6.69 (m, 2H), 6.98 (d, J = 9 Hz, 1H), 7.09 (ddd, J = 4, 4, 8 Hz, 1H), 7.26 (ddd, J = 1, 8, 10 Hz, 1H), 8.29 (ddd, J = 1, 1, 4 Hz, 1H).

(Example 100)

Synthesis of (5aS,6R,11bS)-14-(cyclopropylmethyl)-3-(2-(3-fluoropyridin-2-yl)ethyl)-2,3,4,5,6,7-hexahydro-6,11b-(epiminoethano)naphtho[1,2-d]azepine-5a,10(1H)-diol

[Chemical Formula 121]

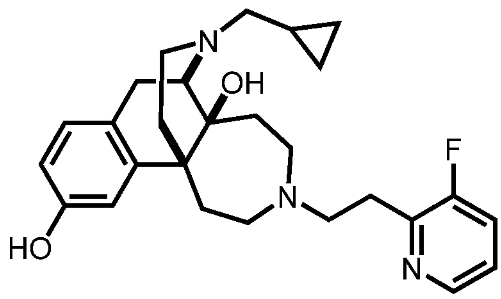

The title compound was obtained from the compound obtained in Example 99 according to the method described in Example 6.

$^1$H-NMR (400 MHz, $CDCl_3$)δ (ppm): 0.03 - 0.16 (m, 2H), 0.43 - 0.54 (m, 2H), 0.77 - 0.88 (m, 1H), 0.99 - 1.09 (m, 1H), 1.51 (ddd, J = 2, 5, 15 Hz, 1H), 1.78 - 1.90 (m, 2H), 1.96 - 2.09 (m, 2H), 2.27 - 2.39 (m, 3H), 2.49 - 2.59 (m, 1H), 2.73 (dd, J = 6, 18 Hz, 1H), 2.82 (ddd, J = 3, 6, 13 Hz, 1H), 2.85 - 3.09 (m, 8H), 3.19 - 3.28 (m, 1H), 4.73 (br s, 1H), 6.45 (d, J = 3 Hz, 1H), 6.54 (dd, J = 3, 8 Hz, 1H), 6.86 (d, J = 8 Hz, 1H), 7.12 (ddd, J = 4, 4, 8 Hz, 1H), 7.29 (ddd, J = 1, 8, 10 Hz, 1H), 8.29 (ddd, J = 1, 1, 4 Hz, 1H).

(Example 101)

Synthesis of (5aS,6R,11bS)-14-(cyclopropylmethyl)-10-methoxy-3-(2-(3-methylpyridin-2-yl)ethyl)-2,3,4,5,6,7-hexahydro-6,11b-(epiminoethano)naphtho[1,2-d]azepin-5a(1H)-ol

[Chemical Formula 122]

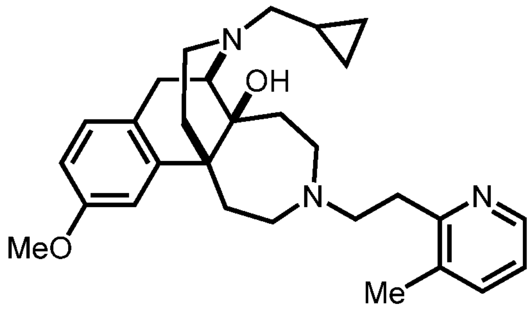

The title compound was obtained from the isomer C obtained in Example 2 and 2-(3-methylpyridine-2-yl)ethan-1-ol according to the method described in Example 32.

$^1$H-NMR (400 MHz, $CDCl_3$)δ (ppm): 0.05 - 0.16 (m, 2H), 0.45 - 0.55 (m, 2H), 0.79 - 0.90 (m, 1H), 1.01 - 1.09 (m, 1H), 1.50 (ddd, J = 3, 5, 15 Hz, 1H), 1.84 (ddd, J = 4, 12, 15 Hz, 1H), 1.93 - 2.03 (m, 2H), 2.10 (ddd, J = 5, 13, 13 Hz, 1H), 2.27 (s, 3H), 2.29 - 2.39 (m, 3H), 2.52 - 2.59 (m, 1H), 2.64 (ddd, J = 4, 4, 13 Hz, 1H), 2.73 - 3.04 (m, 9H), 3.28 (ddd, J = 3, 12, 13 Hz, 1H), 3.77 (s, 3H), 4.75 (br s, 1H), 6.66 - 6.72 (m, 2H), 6.97 - 7.03 (m, 2H), 7.34 - 7.39 (m, 1H), 8.32 (dd, J = 1, 5 Hz, 1H).

(Example 102)

Synthesis of (5aS,6R, 11bS)-14-(cyclopropylmethyl)-3-(2-(3-methylpyridin-2-yl)ethyl)-2,3,4,5,6,7-hexahydro-6,11b-(epiminoethano)naphtho[1,2-d]azepine-5a,10(1H)-diol

[Chemical Formula 123]

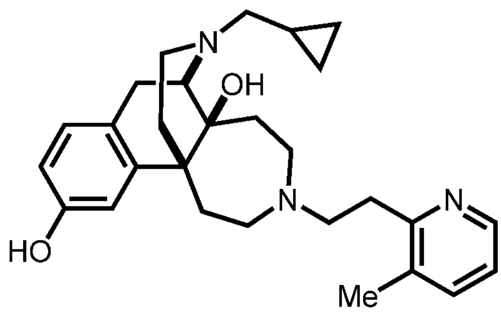

The title compound was obtained from the compound obtained in Example 101 according to the method described in Example 6.

$^1$H-NMR (400 MHz, $CDCl_3$)δ (ppm): 0.04 - 0.16 (m, 2H), 0.43 - 0.54 (m, 2H), 0.77 - 0.89 (m, 1H), 0.98 - 1.09 (m, 1H), 1.51 (ddd, J = 2, 5, 15 Hz, 1H), 1.80 - 1.93 (m, 2H), 1.95 - 2.11 (m, 2H), 2.23 (s, 3H), 2.26 - 2.40 (m, 3H), 2.48 - 2.59 (m, 1H), 2.73 (dd, J = 6, 18 Hz, 1H), 2.83 - 3.04 (m, 9H), 3.23 - 3.32 (m, 1H), 4.76 (br s, 1H), 6.49 (d, J = 2 Hz, 1H), 6.58 (dd, J = 2, 8 Hz, 1H), 6.77 (d, J = 8 Hz, 1H), 7.05 (dd, J = 5, 8 Hz, 1H), 7.38 - 7.43 (m, 1H), 8.33 (dd, J = 1, 5 Hz, 1H).

(Example 103)

Synthesis of (5aS,6R,11bS)-14-(cyclopropylmethyl)-10-methoxy-3-(2-(6-methylpyridin-2-yl)ethyl)-2,3,4,5,6,7-hexahydro-6,11b-(epiminoethano)naphtho[1,2-d]azepin-5a(1H)-ol

[Chemical Formula 124]

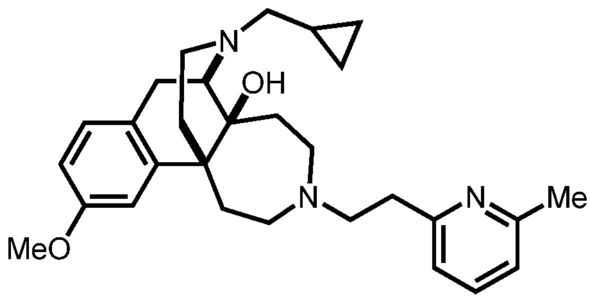

The title compound was obtained from the isomer C obtained in Example 2 and 2-(6-methylpyridin-2-yl)ethyl 4-methylbenzenesulfonate (synthesized by the method described in WO 9905095) according to the method described in Example 31.

$^1$H-NMR (400 MHz, $CDCl_3$)δ (ppm): 0.05 - 0.14 (m, 2H), 0.45 - 0.54 (m, 2H), 0.79 - 0.90 (m, 1H), 0.99 - 1.07 (m, 1H), 1.49 (ddd, J = 3, 4, 15 Hz, 1H), 1.82 (ddd, J = 4, 11, 15 Hz, 1H), 1.90 - 2.03 (m, 2H), 2.09 (ddd, J = 5, 13, 13 Hz, 1H), 2.28 - 2.40 (m, 3H), 2.49 (s, 3H), 2.51 - 2.61 (m, 2H), 2.71 - 2.90 (m, 7H), 2.94 (d, J = 6 Hz, 1H), 2.97 (d, J = 18 Hz, 1H), 3.18 (ddd, J = 3, 11, 13 Hz, 1H), 3.76 (s, 3H), 4.69 (br s, 1H), 6.67 - 6.71 (m, 2H), 6.82 (d, J = 8 Hz, 1H), 6.91 (d, J = 8 Hz, 1H), 6.98 - 7.02 (m, 1H), 7.38 (dd, J = 8, 8 Hz, 1H).

(Example 104)

Synthesis of (5aS,6R,11bS)-14-(cyclopropylmethyl)-3-(2-(6-methylpyridin-2-yl)ethyl)-2,3,4,5,6,7-hexahydro-6,11b-(epiminoethano)naphtho[1,2-d]azepine-5a,10(1H)-diol

[Chemical Formula 125]

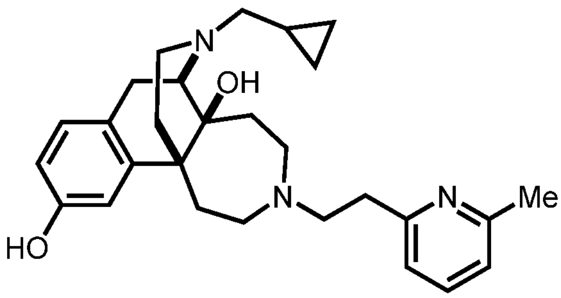

The title compound was obtained from the compound obtained in Example 103 according to the method described in Example 6.

$^1$H-NMR (400 MHz, $CDCl_3$)δ (ppm): 0.04 - 0.16 (m, 2H), 0.44 - 0.55 (m, 2H), 0.77 - 0.89 (m, 1H), 0.98 - 1.11 (m, 1H), 1.49 (ddd, J = 2, 5, 14 Hz, 1H), 1.78 - 1.89 (m, 2H), 1.96 - 2.09 (m, 2H), 2.24 - 2.39 (m, 3H), 2.49 - 2.56 (m, 1H), 2.51 (s, 3H), 2.65 - 2.97 (m, 10H), 3.16 - 3.26 (m, 1H), 4.73 (br s, 1H), 6.48 (d, J = 2 Hz, 1H), 6.56 (dd, J = 2, 8 Hz, 1H), 6.86 (d, J = 8 Hz, 1H), 6.90 (d, J = 8 Hz, 1H), 6.96 (d, J = 8 Hz, 1H), 7.45 (dd, J = 8, 8 Hz, 1H).

(Reference Example 4)

Synthesis of (E)-2-(2-methoxyvinyl)-4-methylpyridine (isomer G) and (Z)-2-(2-methoxyvinyl)-4-methylpyridine (isomer H)

[Chemical Formula 126]

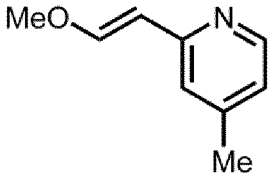 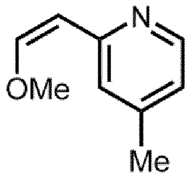

Isomer G Isomer H

To 4-methylpicolinaldehyde (363.4 mg, 3.0 mmol) and (methoxymethyl) triphenylphosphonium chloride (3.09 g, 9.0 mmol) dissolved in tetrahydrofuran (60 mL) was added sodium hydride (55% oil dispersion) (600 mg, 13.8 mmol) at -20°C, followed by stirring at -20°C for 30 minutes and at room temperature for 18 hours. The reaction mixture was poured into ice water, followed by extraction three times with ethyl acetate. The combined extracts were washed with saturated saline, then dried over sodium sulfate, and concentrated under reduced pressure. The obtained crude product was purified by silica gel column chromatography (10 to 100% ethyl acetate/heptane) to individually yield the title isomers G (70.2 mg, 16%) and H (73.9 mg, 17%). (Isomer G)

$^1$H-NMR (400 MHz, $CDCl_3$)δ (ppm): 2.29 (s, 3H), 3.73 (s, 3H), 5.83 (d, J = 13 Hz, 1H), 6.84 (dd, J = 1, 5 Hz, 1H), 6.90 (d, J = 1 Hz, 1H), 7.56 (d, J = 13 Hz, 1H), 8.29 (d, J = 5 Hz, 1H).

(Isomer H)

$^1$H-NMR (400 MHz, $CDCl_3$)δ (ppm): 2.33 (s, 3H), 3.84 (s, 3H), 5.47 (d, J = 7 Hz, 1H), 6.32 (d, J = 7 Hz, 1H), 6.85 (dd, J = 1, 5 Hz, 1H), 7.70 (d, J = 1 Hz, 1H), 8.36 (d, J = 5 Hz, 1H).

(Example 105)

Synthesis of (5aS,6R,11bS)-14-(cyclopropylmethyl)-3-(2-(4-methylpyridin-2-yl)ethyl)-2,3,4,5,6,7-hexahydro-6,11b-(epiminoethano)naphtho[1,2-d]azepine-5a,10(1H)-diol

[Chemical Formula 127]

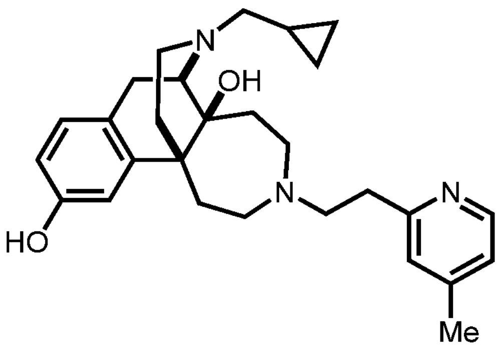

To a solution of the isomer G (14.9 mg, 0.10 mmol) obtained in Reference Example 4 in methanol (1 mL) was added 2 M hydrochloric acid (1 mL), followed by heating under reflux for 8 hours. After allowed to cool, a saturated aqueous sodium bicarbonate solution was added to make the reaction solution basic, followed by extraction three times with chloroform. The combined extracts were dried over sodium sulfate and then concentrated under reduced pressure to yield a crude product of 2-(4-methylpyridin-2-yl)acetaldehyde. The title compound was obtained from the compound E obtained in Example 3 and the crude product of 2-(4-methylpyridin-2-yl)acetaldehyde according to the method described in Example 12.

$^1$H-NMR (400 MHz, $CDCl_3$)δ (ppm): 0.04 - 0.17 (m, 2H), 0.42 - 0.56 (m, 2H), 0.76 - 1.12 (m, 2H), 1.42 - 2.11 (m, 5H), 2.20 - 2.41 (m, 6H), 2.46 - 2.60 (m, 1H), 2.66 - 3.01 (m, 10H), 3.17 - 3.27 (m, 1H), 4.74 (br s, 1H), 6.48 (d, J = 2 Hz, 1H), 6.57 (dd, J = 2, 8 Hz, 1H), 6.87 (d, J = 8 Hz, 1H), 6.90 - 6.97 (m, 2H), 8.33 (d, J = 5 Hz, 1H).

(Example 106)

Synthesis of (5aS,6R,11bS)-14-(cyclopropylmethyl)-3-(2,2-difluoro-2-(pyridin-2-yl)ethyl)-2,3,4,5,6,7-hexahydro-6,11b-(epiminoethano)naphtho[1,2-d]azepine-5a,10(1H)-diol

[Chemical Formula 128]

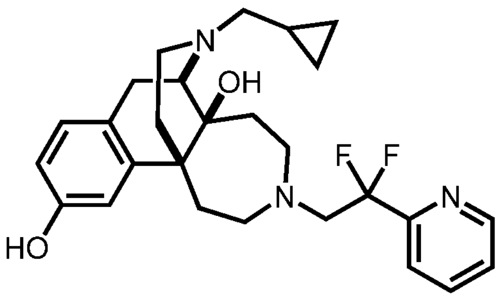

The title compound was obtained from the isomer C obtained in Example 2 and 2,2-difluoro-2-(pyridin-2-yl)ethyl trifluoromethanesulfonate (synthesized by the method described in WO 2011045383) according to the method described in Example 31.

$^1$H-NMR (400 MHz, $CDCl_3$)$\delta$ (ppm): 0.04 - 0.12 (m, 2H), 0.45 - 0.51 (m, 2H), 0.76 - 0.84 (m, 1H), 0.94 - 1.00 (m, 1H), 1.26 - 1.33 (m, 1H), 1.46 (ddd, J = 3, 11, 15 Hz, 1H), 1.71 (ddd, J = 3, 3, 16 Hz, 1H), 1.90 - 2.02 (m, 2H), 2.23 - 2.33 (m, 3H), 2.46 - 2.52 (m, 1H), 2.56 (ddd, J = 4, 4, 13 Hz, 1H), 2.62 (dd, J = 6, 18 Hz, 1H), 2.76 - 2.83 (m, 2H), 2.90 (d, J = 18 Hz, 1H), 2.94 - 3.02 (m, 1H), 3.13 - 3.21 (m, 1H), 3.23 - 3.42 (m, 2H), 4.64 (br s, 1H), 6.51 (d, J = 2 Hz, 1H), 6.60 (dd, J = 2, 8 Hz, 1H), 6.89 (d, J = 8 Hz, 1H), 7.24 - 7.30 (m, 2H), 7.63 (ddd, J = 2, 8, 8 Hz, 1H), 8.57 - 8.62 (m, 1H).

(Example 107)

Synthesis of ((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)(1-(pyridin-2-yl)cyclopropyl)methanone

[Chemical Formula 129]

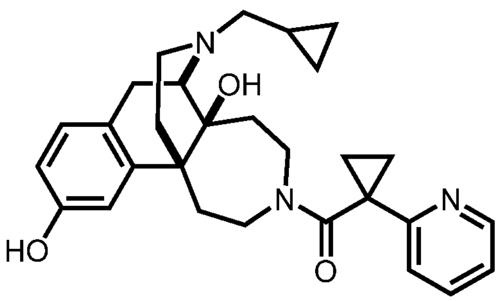

The title compound was obtained from the compound E obtained in Example 3 and 1-(pyridin-2-yl)cyclopropane-1-carboxylic acid according to the method described in Example 85.

$^1$H-NMR (400 MHz, $CDCl_3$)$\delta$ (ppm): 0.03 - 0.15 (m, 2H), 0.38 - 0.54 (m, 2.4H), 0.74 - 0.97 (m, 1.8H), 1.14 - 1.30 (m, 1.2H), 1.38 (ddd, J = 3, 3, 15 Hz, 0.6H), 1.41 - 1.49 (m, 0.6H), 1.52 - 1.80 (m, 2.8H), 1.83 - 2.11 (m, 2.6H), 2.19 - 2.58 (m, 4.6H), 2.62 (dd, J = 6, 18 Hz, 0.6H), 2.76 (dd, J = 6, 18 Hz, 0.4H), 2.78 (d, J = 6 Hz, 0.6H), 2.87 (d, J = 6 Hz, 0.4H), 2.90 (d, J = 18 Hz, 0.4H), 2.94 - 3.04 (m, 0.4H), 2.96 (d, J = 18 Hz, 0.6H), 3.08 (ddd, J = 4, 4, 14 Hz, 0.6H), 3.21 (dd, J = 12, 14 Hz, 0.6H), 3.40 (ddd, J = 2, 12, 14 Hz, 0.4H), 3.78 (ddd, J = 4, 14, 14 Hz, 0.4H), 3.90 - 4.01 (m, 1H), 4.25 - 4.38 (m, 1H), 4.51 (br s, 1H), 6.55 (d, J = 3 Hz, 0.4H), 6.63 (dd, J = 3, 8 Hz, 0.4H), 6.69 (d, J = 2, 8 Hz, 0.6H), 6.89 (d, J = 8 Hz, 0.4H), 6.93 (d, J = 8 Hz, 0.6H), 7.01 - 7.09 (m, 1.2H), 7.10 (d, J = 2 Hz, 0.6H), 7.13 (ddd, J = 1, 5, 7 Hz, 0.4H),

7.19 - 7.23 (m, 0.4H), 7.48 (ddd, J = 2, 8, 8 Hz, 0.6H), 7.60 (ddd, J = 2, 8, 8 Hz, 0.4H), 8.44 (ddd, J = 1, 2, 5 Hz, 0.6H), 8.45 (ddd, J = 1, 2, 5 Hz, 0.4H).

(Example 108)

Synthesis of (5aS,6R,11bS)-14-(cyclopropylmethyl)-3-((1-(pyridin-2-yl)cyclopropyl)methyl)-2,3,4,5,6,7-hexahydro-6,11b-(epiminoethano)naphtho[1,2-d]azepine-5a,10(1H)-diol

[Chemical Formula 130]

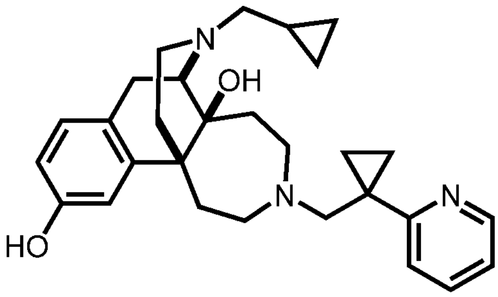

The title compound was obtained from the compound obtained in Example 107 according to the method described in Example 24.

$^1$H-NMR (400 MHz, $CDCl_3$)δ (ppm): 0.03 - 0.14 (m, 2H), 0.45 - 0.51 (m, 2H), 0.74 - 0.87 (m, 2H), 0.92 - 0.98 (m, 1H), 1.15 - 1.23 (m, 2H), 1.38 - 1.45 (m, 1H), 1.65 - 1.77 (m, 1H), 1.90 - 2.04 (m, 2H), 2.20 - 2.29 (m, 1H), 2.31 (d, J = 7 Hz, 2H), 2.46 - 2.53 (m, 1H), 2.62 - 2.73 (m, 3H), 2.78 (d, J = 13 Hz, 1H), 2.78 - 2.84 (m, 1H), 2.85 (d, J = 5 Hz, 1H), 2.88 (d, J = 18 Hz, 1H), 2.91 (d, J = 13 Hz, 1H), 2.95 - 3.04 (m, 1H), 3.67 - 3.74 (m, 2H), 6.33 (d, J = 3 Hz, 1H), 6.50 (dd, J = 3, 8 Hz, 1H), 6.83 (d, J = 8 Hz, 1H), 6.93 (ddd, J = 1, 5, 8 Hz, 1H), 7.23 - 7.28 (m, 1H), 7.38 (ddd, J = 2, 8, 8 Hz, 1H), 8.35 - 8.38 (m, 1H).

(Example 109)

Synthesis of 1-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-2-(1H-indol-2-yl)ethan-1-one

[Chemical Formula 131]

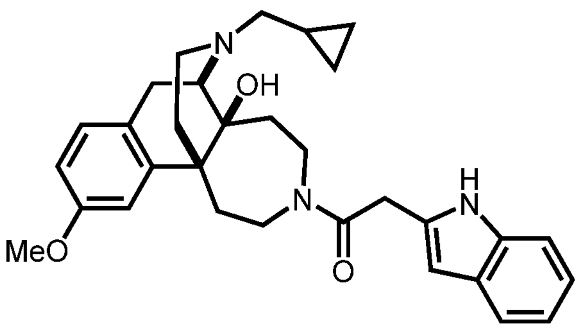

The title compound was obtained from the isomer C obtained in Example 2 and 2-(1H-indol-2-yl)acetic acid according to the method described in Example 7.

$^1$H-NMR (400 MHz, $CDCl_3$)δ (ppm): 0.07 - 0.15 (m, 2H), 0.49 - 0.55 (m, 2H), 0.78 - 0.90 (m, 1H), 1.04 - 1.11 (m, 1H), 1.56 - 1.67 (m, 1.4H), 1.78 - 1.90 (m, 0.6H), 1.91 - 2.08 (m, 3H), 2.12 - 2.40 (m, 3H), 2.46 - 2.58 (m, 1.4H), 2.68

(dd, J = 6, 18 Hz, 0.6H), 2.81 (dd, J = 7, 19 Hz, 0.4H), 2.88 - 2.96 (m, 1.6H), 2.97 - 3.00 (m, 0.4H), 3.20 (ddd, J = 3, 11, 14 Hz, 0.6H), 3.49 (ddd, J = 4, 4, 13 Hz, 0.6H), 3.54 - 3.64 (m, 1H), 3.65 - 3.72 (m, 0.4H), 3.74 - 3.76 (m, 0.4H), 3.76 (s, 1.2H), 3.79 (s, 1.8H), 3.80 - 3.90 (m, 1.6H), 3.97 (ddd, J = 3, 12, 12 Hz, 0.6H), 4.06 - 4.14 (m, 0.4H), 6.21 - 6.25 (m, 1H), 6.62 - 6.70 (m, 2H), 6.90 (d, J = 8 Hz, 0.6H), 6.99 - 7.13 (m, 2.4H), 7.26 - 7.30 (m, 1H), 7.49 - 7.53 (m, 1H), 8.94 (br s, 0.4H), 9.23 (br s, 0.6H).

(Example 110)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(1H-indol-2-yl)ethan-1-one

[Chemical Formula 132]

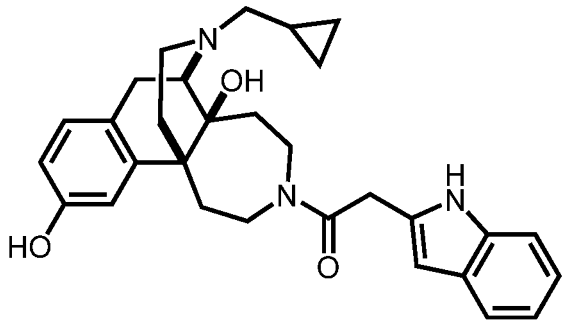

The title compound was obtained from the compound obtained in Example 109 according to the method described in Example 6.

$^1$H-NMR (400 MHz, $CDCl_3$)$\delta$ (ppm): 0.05 - 0.15 (m, 2H), 0.47 - 0.57 (m, 2H), 0.78 - 0.90 (m, 1H), 1.00 - 1.12 (m, 1H), 1.56 - 1.67 (m, 1.2H), 1.79 - 2.12 (m, 3.8H), 2.13 - 2.40 (m, 3.8H), 2.44 - 2.56 (m, 1.2H), 2.62 (dd, J = 5, 18 Hz, 0.8H), 2.77 (dd, J = 6, 18 Hz, 0.2H), 2.86 - 2.99 (m, 2H), 3.12 - 3.30 (m, 0.8H), 3.41 - 3.59 (m, 1.2H), 3.65 - 3.75 (m, 0.2H), 3.79 - 3.92 (m, 1.8H), 3.96 - 4.17 (m, 1H), 4.82 (br s, 1H), 6.17 (s, 0.2H), 6.27 (s, 0.8H), 6.49 (d, J = 8 Hz, 0.2H), 6.60 - 6.72 (m, 1H), 6.73 - 6.81 (m, 0.8H), 6.86 - 6.92 (m, 1H), 6.99 - 7.13 (m, 2H), 7.15 - 7.20 (m, 0.2H), 7.21 - 7.29 (m, 0.8H), 7.43 - 7.52 (m, 1H), 8.83 (br s, 0.2H), 9.49 (br s, 0.8H).

(Reference Example 5)

Synthesis of tert-butyl 3-(2-hydroxyethyl)-1H-indazole-1-carboxylate

[Chemical Formula 133]

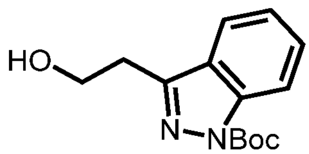

To a solution of 2-(1H-indazol-3-yl)ethan-1-ol (13 mg, 0.077 mmol) in chloroform (2 mL) were added triethylamine (32 pL, 0.23 mmol), di-tert-butyl dicarbonate (20 mg, 0.093 mmol) and N,N-dimethylaminopyridine (1 mg, 0.0077 mmol), followed by stirring at room temperature for 17 hours. Water was added to the reaction mixture, followed by extraction three times with ethyl acetate. The combined extracts were dried over sodium sulfate and then concentrated under reduced pressure. The obtained crude product was purified by silica gel column chromatography (60 to 80% ethyl acetate/heptane) to yield the title compound (16 mg, 80%) as a colorless oily matter.

$^1$H-NMR (400 MHz, $CDCl_3$)$\delta$ (ppm): 1.72 (s, 9H), 2.63 (t, J = 6 Hz, 1H), 3.23 (t, J = 6 Hz, 2H), 4.13 (dt, J = 6,

6 Hz, 2H), 7.32 (t, J = 8 Hz, 1H), 7.51 - 7.56 (m, 1H), 7.70 (d, J = 8 Hz, 1H), 8.12 (d, J = 9 Hz, 1H).

(Reference Example 6)

Synthesis of tert-butyl 3-(2-(tosyloxy)ethyl)-1H-indazole-1-carboxylate

[Chemical Formula 134]

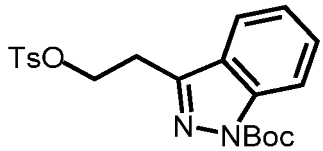

To a solution of the compound (16 mg, 0.060 mmol) obtained in Reference Example 5 in chloroform (1 mL) were added triethylamine (25 μL, 0.18 mmol) and p-toluenesulfonyl chloride (14 mg, 0.072 mmol) under ice cooling, followed by stirring at room temperature for 17 hours. To the reaction mixture, a saturated aqueous sodium bicarbonate solution was added under ice cooling, followed by extraction three times with ethyl acetate. The combined extracts were dried over sodium sulfate and then concentrated under reduced pressure. The obtained crude product was purified by silica gel column chromatography (15 to 35% ethyl acetate/heptane) to yield the title compound (25 mg, 99%) as a pale yellow oily matter.

$^1$H-NMR (400 MHz, $CDCl_3$)δ (ppm): 1.72 (s, 9H), 2.41 (s.3H), 3.56 (t, J = 7 Hz, 2H), 4.47 (t, J = 7 Hz, 2H), 7.17 - 7.34 (m, 3H), 7.52 (ddd, J = 1, 7, 9 Hz, 1H), 7.61 - 7.68 (m, 3H), 8.05 (d, J = 9 Hz, 1H).

(Example 111)

Synthesis of tert-butyl 3-(2-((5aS,6R,11bS)-14-(cyclopropylmethyl)-5a-hydroxy-10-methoxy-1,2,5,5a,6,7-hexahydro-6,11b-(epiminoethano)naphtho[1,2-d]azepin-3(4H)-yl)ethyl)-1H-indazole-1-carboxylate

[Chemical Formula 135]

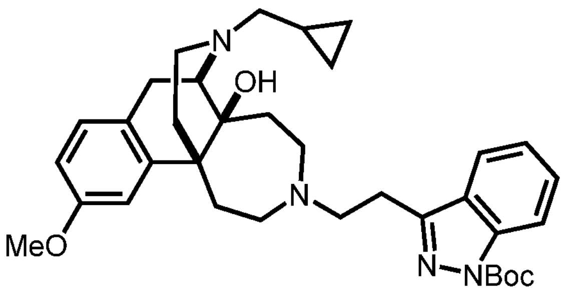

The title compound was obtained from the isomer C obtained in Example 2 and the compound obtained in Reference Example 6 according to the method described in Example 5.

$^1$H-NMR (400 MHz, $CDCl_3$)δ (ppm): 0.04 - 0.18 (m, 2H), 0.46 - 0.55 (m, 2H), 0.77 - 0.92 (m, 1H), 0.99 - 1.10 (m, 1H), 1.20 - 1.65 (m, 1H), 1.72 (s, 9H), 1.79 - 2.14 (m, 4H), 2.30 - 2.45 (m, 3H), 2.50 - 2.59 (m, 1H), 2.61 - 2.71 (m, 1H), 2.73 - 3.12 (m, 9H), 3.19 - 3.33 (m, 1H), 3.76 (s, 3H), 6.62 - 6.69 (m, 2H), 6.98 (d, J = 9 Hz, 1H), 7.21 - 7.31 (m, 1H), 7.48 (ddd, J = 1, 7, 8 Hz, 1H), 7.63 (d, J = 8 Hz, 1H), 8.05 (d, J = 8 Hz, 1H).

(Example 112)

Synthesis of (5aS,6R,11bS)-3-(2-(1H-indazol-3-yl)ethyl)-14-(cyclopropylmethyl)-2,3,4,5,6,7-hexahydro-6,11b-(epiminoethano)naphtho[1,2-d]azepine-5a,10(1H)-diol

[Chemical Formula 136]

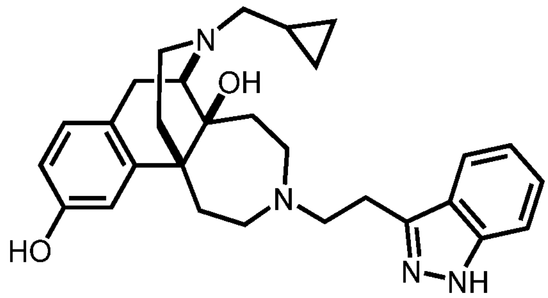

The title compound was obtained from the compound obtained in Example 111 according to the method described in Example 6.

$^1$H-NMR (400 MHz, $CDCl_3$)$\delta$ (ppm): 0.05 - 0.17 (m, 2H), 0.44 - 0.56 (m, 2H), 0.77 - 0.95 (m, 1H), 0.99 - 1.08 (m, 1H), 1.18 - 2.13 (m, 5H), 2.24 - 2.39 (m, 3H), 2.49 - 2.58 (m, 1H), 2.59 - 2.69 (m, 1H), 2.70 - 2.81 (m, 2H), 2.83 - 3.02 (m, 5H), 3.06 - 3.17 (m, 2H), 3.19 - 3.32 (m, 1H), 4.79 (br s, 1H), 6.56 - 6.66 (m, 2H), 6.93 (d, J = 9 Hz, 1H), 7.07 - 7.14 (m, 1H), 7.30 - 7.38 (m, 1H), 7.43 - 7.52 (m, 1H), 7.66 (d, J = 8 Hz, 1H).

(Reference Example 7)

Synthesis of 2-(1-methyl-1H-indazol-3-yl)ethyl methanesulfonate

[Chemical Formula 137]

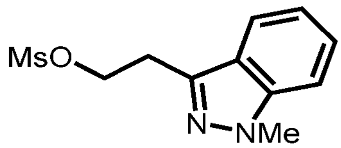

To a solution of 2-(1-methyl-1H-indazol-3-yl)ethan-1-ol (6.9 mg, 0.039 mmol) in chloroform (1 mL) were added triethylamine (16 μL, 0.12 mmol) and methanesulfonic anhydride (10 mg, 0.059 mmol) under ice cooling, followed by stirring at the same temperature for 1 hour. To the reaction mixture, a saturated aqueous sodium bicarbonate solution was added under ice cooling, followed by extraction three times with ethyl acetate. The combined extracts were dried over sodium sulfate and then concentrated under reduced pressure to yield a crude product of the title compound (8.9 mg, 89%) as a pale yellow oily matter.

$^1$H-NMR (400 MHz, $CDCl_3$)$\delta$ (ppm): 2.92 (s, 3H), 3.43 (t, J = 7 Hz, 2H), 4.03 (s, 3H), 4.63 (t, J = 7 Hz, 2H), 7.16 (ddd, J = 1, 6, 8 Hz, 1H), 7.33 - 7.44 (m, 2H), 7.70 (d, J = 8 Hz, 1H).

(Example 113)

Synthesis of (5aS,6R,11bS)-14-(cyclopropylmethyl)-3-(2-(1-methyl-1H-indazol-3-yl)ethyl)-2,3,4,5,6,7-hexahydro-6,11b-(epiminoethano)naphtho[1,2-d]azepine-5a,10(1H)-diol

[Chemical Formula 138]

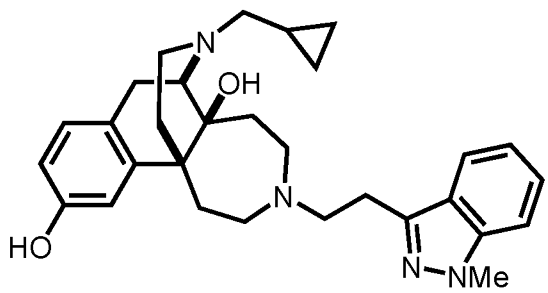

The title compound was obtained from the compound E obtained in Example 3 and the compound obtained in Reference Example 7 according to the method described in Example 31.

$^1$H-NMR (400 MHz, $CDCl_3$)δ (ppm): 0.03 - 0.17 (m, 2H), 0.43 - 0.56 (m, 2H), 0.75 - 0.93 (m, 1H), 0.95 - 1.08 (m, 1H), 1.12 - 2.10 (m, 5H), 2.25 - 2.41 (m, 3H), 2.45 - 2.59 (m, 1H), 2.74 (dd, J = 6, 18 Hz, 1H), 2.80 - 3.07 (m, 7H), 3.10 - 3.33 (m, 3H), 3.98 (s, 3H), 4.75 (br s, 1H), 6.43 - 6.61 (m, 2H), 6.89 (d, J = 8 Hz, 1H), 7.03 - 7.12 (m, 1H), 7.27 - 7.39 (m, 2H), 7.63 (d, J = 8 Hz, 1H).

(Example 114)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(1H-indazol-3-yl)ethan-1-one

[Chemical Formula 139]

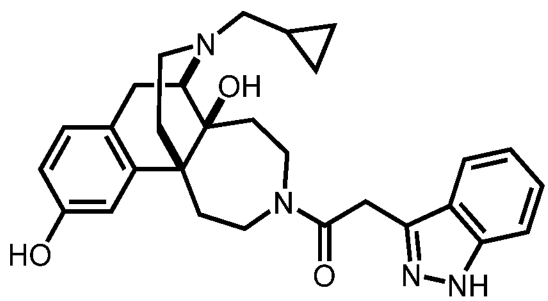

The title compound was obtained from the compound E obtained in Example 3 and 2-(1H-indazol-3-yl)acetic acid according to the method described in Example 85.

$^1$H-NMR (400 MHz, $CDCl_3$)δ (ppm): 0.03 - 0.19 (m, 2H), 0.43 - 0.59 (m, 2H), 0.74 - 1.12 (m, 2H), 1.13 - 2.18 (m, 6H), 2.23 - 2.44 (m, 2H), 2.46 - 2.81 (m, 2H), 2.83 - 3.02 (m, 2H), 3.11 - 3.30 (m, 1H), 3.46 - 4.08 (m, 5H), 6.58 - 6.76 (m, 2H), 6.88 (d, J = 8 Hz, 1H), 7.02 - 7, 13 (m, 1H), 7.24 - 7.45 (m, 2H), 7.63 (d, J = 8 Hz, 0.4H), 7.72 (d, J = 8 Hz, 0.6H).

(Example 115)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(imidazo[1,2-a]pyridin-2-yl)ethan-1-one

[Chemical Formula 140]

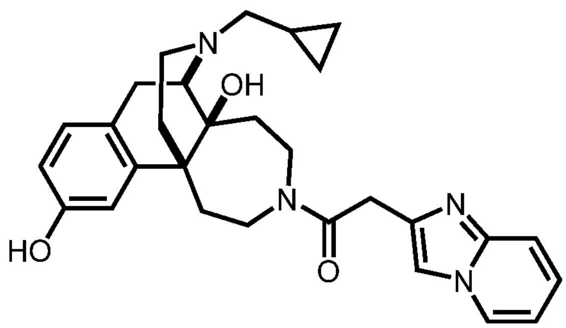

The title compound was obtained from 2-(imidazo[1,2-a]pyridin-2-yl)acetic acid according to the method described in Example 5.

$^1$H-NMR (400 MHz, $CDCl_3$)δ (ppm): 0.03 - 0.18 (m, 2H), 0.41 - 0.59 (m, 2H), 0.71 - 0.92 (m, 1H), 0.96 - 1.10 (m, 1H), 1.49 - 1.66 (m, 1H), 1.73 - 2.14 (m, 5H), 2.23 - 2.97 (m, 6H), 3.26 - 4.01 (m, 6H), 4.52 (br s, 1H), 6.59 (dd, J = 2, 8 Hz, 0.6H), 6.64 (dd, J = 3, 8 Hz, 0.4H), 6.68 - 6.82 (m, 2.4H), 6.86 (d, J = 8 Hz, 0.6H), 7.07 - 7.19 (m, 1H), 7.38 (s, 0.4H), 7.43 (s, 0.6H), 7.48 (d, J = 9 Hz, 0.4H), 7.52 (d, J = 9 Hz, 0.6H), 7.94 - 8.04 (m, 1H).

(Example 116)

Synthesis of (5aS,6R,11bS)-14-(cyclopropylmethyl)-3-(2-(imidazo[1,2-a]pyridin-2-yl)ethyl)-2,3,4,5,6,7-hexahydro-6,11b-(epiminoethano)naphtho[1,2-d]azepine-5a,10(1H)-diol

[Chemical Formula 141]

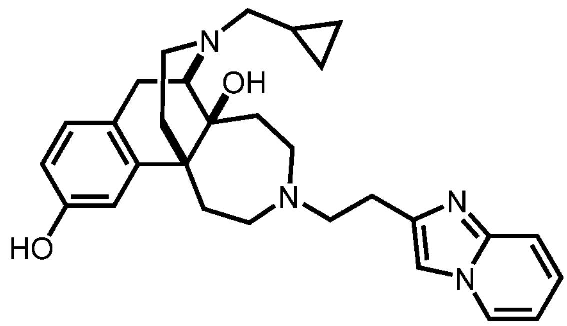

The title compound was obtained from the compound obtained in Example 115 according to the method described in Example 24.

$^1$H-NMR (400 MHz, $CDCl_3$)δ (ppm): 0.03 - 0.15 (m, 2H), 0.42 - 0.53 (m, 2H), 0.74 - 1.04 (m, 2H), 1.42 - 1.54 (m, 1H), 1.67 - 1.91 (m, 2H), 1.92 - 2.09 (m, 2H), 2.26 - 2.38 (m, 3H), 2.43 - 2.99 (m, 11H), 3.10 - 3.26 (m, 1H), 6.54 (d, J = 2 Hz, 1H), 6.65 (dd, J = 2, 8 Hz, 1H), 6.70 (ddd, J = 1, 1, 7 Hz, 1H), 6.89 (d, J = 8 Hz, 1H), 7.09 (ddd, J = 1, 7, 9 Hz, 1H), 7.19 (s, 1H), 7.47 (d, J = 8 Hz, 1H), 7.98 - 8.04 (m, 1H).

(Example 117)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(pyrazolo[1,5-a]pyridin-2-yl)ethan-1-one

[Chemical Formula 142]

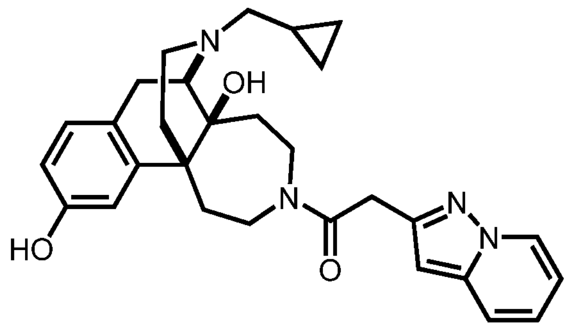

The title compound was obtained from the compound E obtained in Example 3 and 2-(pyrazolo[1,5-a]pyridin-2-yl)acetic acid according to the method described in Example 85.

$^1$H-NMR (400 MHz, $CDCl_3$)$\delta$ (ppm): 0.00 - 0.15 (m, 2H), 0.45 - 0.55 (m, 2H), 0.75 - 0.90 (m, 1H), 0.95 - 1.10 (m, 1H), 1.50 - 1.65 (m, 1H), 1.70 - 1.90 (m, 3H), 1.90 - 2.05 (m, 2H), 2.20 - 2.40 (m, 3H), 2.45 - 2.65 (m, 2H), 2.70 - 3.00 (m, 2H), 3.20 - 3.55 (m, 2H), 3.60 - 4.00 (m, 3H), 4.05 - 4.15 (m, 1H), 6.32 (s, 0.5H), 6.33 (s, 0.5H), 6.50 - 6.75 (m, 2.5H), 6.80 - 6.95 (m, 1.5H), 7.00 - 7.10 (m, 1H), 7.40 (d, J = 3 Hz, 1H), 8.30 - 8.40 (m, 1H).

(Example 118)

Synthesis of (5aS,6R, 11bS)-14-(cyclopropylmethyl)-3-(2-(pyrazolo[1,5-a]pyridin-2-yl)ethyl)-2,3,4,5,6,7-hexahydro-6,11b-(epiminoethano)naphtho[1,2-d]azepine-5a,10(1H)-diol

[Chemical Formula 143]

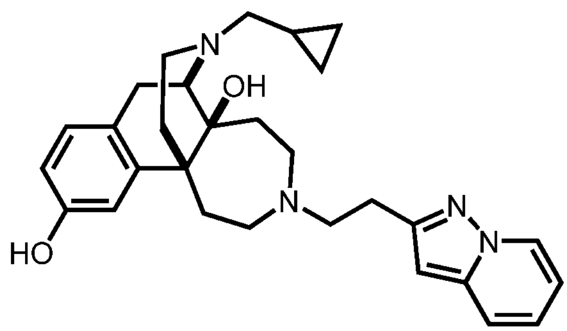

The title compound was obtained from the compound obtained in Example 117 according to the method described in Example 24.

$^1$H-NMR (400 MHz, $CDCl_3$)$\delta$ (ppm): 0.00 - 0.20 (m, 2H), 0.45 - 0.60 (m, 2H), 0.75 - 0.90 (m, 1H), 0.95 - 1.10 (m, 1H), 1.40 - 1.60 (m, 1H), 1.75 - 1.90 (m, 2H), 1.95 - 2.05 (m, 2H), 2.30 - 2.40 (m, 3H), 2.45 - 2.55 (m, 1H), 2.70 - 2.85 (m, 3H), 2.85 - 3.05 (m, 7H), 3.15 - 3.28 (m, 1H), 4.74 (br s, 1H), 6.23 (s, 1H), 6.51 (d, J = 3 Hz, 1H), 6.57 (dd, J = 3, 8 Hz, 1H), 6.65 (ddd, J = 1, 6, 6 Hz, 1H), 6.89 (d, J = 8 Hz, 1H), 7.00 - 7.10 (m, 1H), 7.39 (d, J = 8 Hz, 1H), 8.33 (d, J = 6 Hz, 1H).

(Example 119)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(2H-indazol-2-yl)ethan-1-one

[Chemical Formula 144]

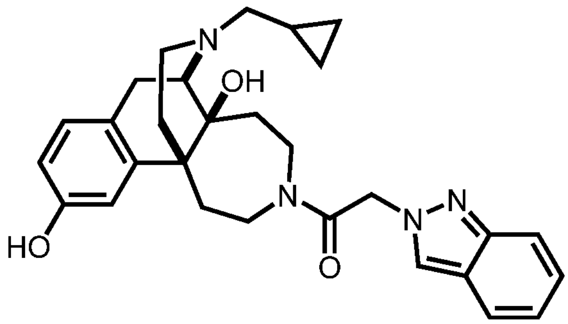

The title compound was obtained from the compound E obtained in Example 3 and 2-(2H-indazol-2-yl)acetic acid according to the method described in Example 26.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.04 (d, J = 10 Hz, 1H), 1.50 - 1.90 (m, 2H), 1.90 - 2.10 (m, 2H), 2.20 - 2.40 (m, 2H), 2.50 - 2.70 (m, 2H), 2.70 - 3.00 (m, 2H), 3.10 - 3.25 (m, 2.6H), 3.37 (ddd, J = 4, 4, 12 Hz, 0.4H), 3.77 (ddd, J = 5, 12, 12 Hz, 0.6H), 3.80 - 4.00 (m, 2.4H), 4.80 (d, J = 16 Hz, 0.6H), 5.02 (d, J = 16 Hz, 0.6H), 5.19 (s, 0.8H), 6.50 - 6.75 (m, 2H), 6.88 (d, J = 8 Hz, 0.4H), 6.97 (d, J = 8 Hz, 0.6H), 7.10 - 7.35 (m, 4H), 7.68 (d, J = 8 Hz, 1H).

(Example 120)

Synthesis of (5aS,6R,11bS)-3-(2-(2H-indazol-2-yl)ethyl)-14-(cyclopropylmethyl)-2,3,4,5,6,7-hexahydro-6,11b-(epiminoethano)naphtho[1,2-d]azepine-5a,10(1H)-diol

[Chemical Formula 145]

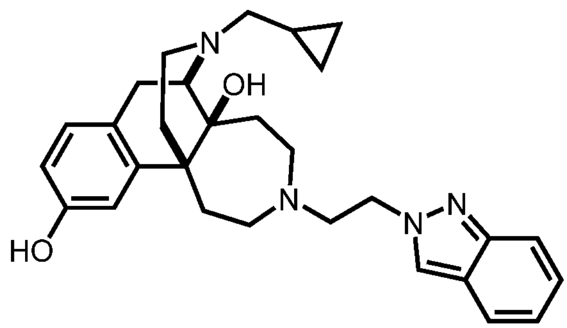

The title compound was obtained from the compound obtained in Example 119 according to the method described in Example 24.

$^1$H-NMR (400 MHz, $CDCl_3$)δ (ppm): 0.00 - 0.15 (m, 2H), 0.45 - 0.55 (m, 2H), 0.80 - 0.90 (m, 1H), 1.01 (d, J = 9 Hz, 1H), 1.35 - 1.45 (m, 1H), 1.60 - 1.80 (m, 2H), 1.90 - 2.10 (m, 3H), 2.30 - 2.65 (m, 5H), 2.77 (dd, J = 6, 18 Hz, 1H), 2.85 - 3.10 (m, 5H), 3.10 - 3.20 (m, 1H), 4.20 - 4.40 (m, 2H), 6.60 - 6.70 (m, 2H), 7.00 - 7.10 (m, 2H), 7.20 - 7.30 (m, 1H), 7.36 (s, 1H), 7.56 (d, J = 8 Hz, 1H), 7.63 (d, J = 8 Hz, 1H).

(Example 121)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(1H-indazol-1-yl)ethan-1-one

[Chemical Formula 146]

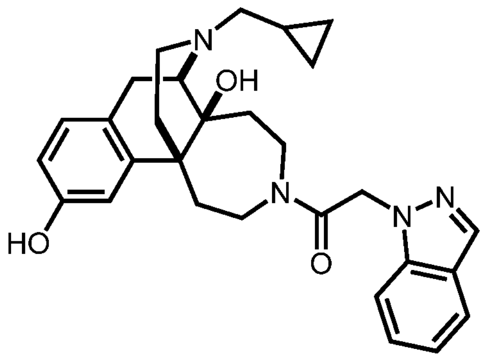

The title compound was obtained from the compound E obtained in Example 3 and 2-(1H-indazol-1-yl)acetic acid according to the method described in Example 26.

$^1$H-NMR (400 MHz, $CDCl_3$)δ (ppm): 0.00 - 0.15 (m, 2H), 0.45 - 0.55 (m, 2H), 0.80 - 0.90 (m, 1H), 1.00 - 1.05 (m, 1H), 1.50 - 2.10 (m, 5H), 2.15 - 2.40 (m, 3H), 2.50 - 2.70 (m, 2H), 2.70 - 3.00 (m, 2.6H), 3.10 - 3.35 (m, 1.4H), 3.35 - 3.45 (m, 0.4H), 3.60 - 3.70 (m, 0.6H), 3.80 - 3.90 (m, 0.6H), 3.90 - 4.00 (m, 0.4H), 4.87 (d, J = 16 Hz, 0.6H), 5.03 (d, J = 16 Hz, 0.6H), 5.20 (d, J = 16 Hz, 0.4H), 5.26 (d, J = 16 Hz, 0.4H), 6.60 - 6.70 (m, 2H), 6.85 - 6.95 (m, 1H), 7.00 - 7.10 (m, 1H), 7.10 - 7.20 (m, 1H), 7.60 - 7.70 (m, 2H), 7.84 (s, 0.6H), 7.92 (s, 0.4H).

(Example 122)

Synthesis of (5aS,6R,11bS)-3-(2-(1H-indazol-1-yl)ethyl)-14-(cyclopropylmethyl)-2,3,4,5,6,7-hexahydro-6,11b-(epiminoethano)naphtho[1,2-d]azepine-5a,10(1H)-diol

[Chemical Formula 147]

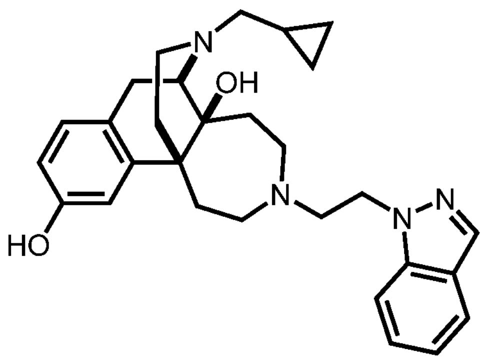

The title compound was obtained from the compound obtained in Example 121 according to the method described in Example 24.

$^1$H-NMR (400 MHz, $CDCl_3$)δ (ppm): 0.00 - 0.15 (m, 2H), 0.45 - 0.55 (m, 2H), 0.75 - 0.90 (m, 1H), 1.01 (d, J = 9 Hz, 1H), 1.20 - 1.90 (m, 3H), 1.90 - 2.10 (m, 3H), 2.25 - 2.45 (m, 3H), 2.50 - 2.80 (m, 3H), 2.80 - 3.10 (m, 5H), 3.15 - 3.30 (m, 1H), 4.30 - 4.50 (m, 2H), 6.50 - 6.70 (m, 2H), 6.91 (d, J = 8 Hz, 1H), 7.11 (dd, J = 6, 6 Hz, 1H), 7.20 - 7.40 (m, 2H), 7.69 (d, J = 8 Hz, 1H), 7.96 (s, 1H).

(Example 123)

Synthesis of (5aS,6R,11bS)-14-(cyclopropylmethyl)-3-(2-(isoquinolin-3-yl)ethyl)-2,3,4,5,6,7-hexahydro-6,11b-(epiminoethano)naphtho[1,2-d]azepine-5a,10(1H)-diol

[Chemical Formula 148]

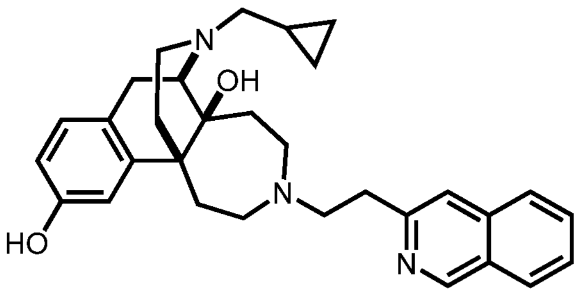

The title compound was obtained from 2-(isoquinolin-3-yl)acetaldehyde and the compound E obtained in Example 3 according to the method described in Reference Example 4 and Example 105.

$^1$H-NMR (400 MHz, $CDCl_3$)δ (ppm): 0.03 - 0.15 (m, 2H), 0.40 - 0.55 (m, 2H), 0.74 - 0.92 (m, 1H), 0.97 - 1.09 (m, 1H), 1.51 (dd, J = 3, 15 Hz, 1H), 1.80 - 2.10 (m, 4H), 2.26 - 2.40 (m, 3H), 2.44 - 2.56 (m, 1H), 2.72 (dd, J = 2, 18 Hz, 1H), 2.81 - 3.17 (m, 9H), 3.22 - 3.33 (m, 1H), 4.75 (br s, 1H), 6.48 (d, J = 3 Hz, 1H), 6.55 (dd, J = 3, 8 Hz, 1H), 6.84 (d, J = 8 Hz, 1H), 7.47 (s, 1H), 7.48 - 7.56 (m, 1H), 7.60 - 7.68 (m, 1H), 7.72 (d, J = 8 Hz, 1H), 7.90 (d, J = 8 Hz, 1H), 9.15 (s, 1H).

(Example 124)

Synthesis of (5aS,6R, 11bS)-14-(cyclopropylmethyl)-3-(2-(quinolin-2-yl)ethyl)-2,3,4,5,6,7-hexahydro-6,11b-(epiminoethano)naphtho[1,2-d]azepine-5a,10(1H)-diol

[Chemical Formula 149]

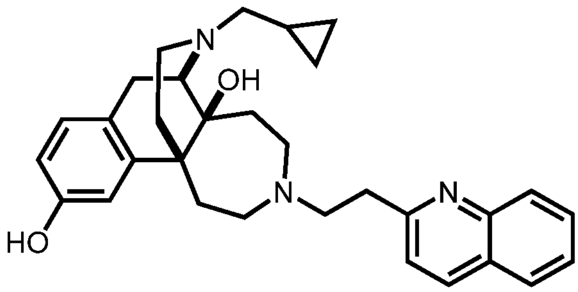

The title compound was obtained from 2-(quinolin-2-yl)acetaldehyde and the compound E obtained in Example 3 according to the method described in Reference Example 4 and Example 105.

$^1$H-NMR (400 MHz, $CDCl_3$)δ (ppm): 0.03 - 0.15 (m, 2H), 0.44 - 0.55 (m, 2H), 0.75 - 0.90 (m, 1H), 0.95 - 1.07 (m, 1H), 1.42 - 1.60 (m, 1H), 1.75 - 1.90 (m, 2H), 1.94 - 2.09 (m, 2H), 2.26 - 2.41 (m, 3H), 2.49 - 2.59 (m, 1H), 2.73 (dd, J = 6, 18 Hz, 1H), 2.77 - 3.18 (m, 9H), 3.22 - 3.33 (m, 1H), 6.51 (d, J = 2 Hz, 1H), 6.59 (dd, J = 3, 8 Hz, 1H), 6.86 (d, J = 8 Hz, 1H), 7.22 (d, J = 8 Hz, 1H), 7.45 - 7.53 (m, 1H), 7.62 - 7.70 (m, 1H), 7.77 (dd, J = 1, 8 Hz, 1H), 8.03 (d, J = 8 Hz, 2H).

(Example 125)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(1H-indol-1-yl)ethan-1-one

[Chemical Formula 150]

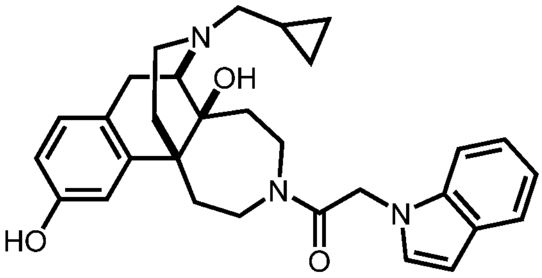

The title compound was obtained from the compound E obtained in Example 3 and 2-(1H-indol-1-yl)acetic acid according to the method described in Example 26.

$^1$H-NMR (400 MHz, $CDCl_3$)δ (ppm): 0.00 - 0.15 (m, 2H), 0.45 - 0.60 (m, 2H), 0.75 - 0.90 (m, 1H), 1.00 - 1.10 (m, 1H), 1.40 - 1.50 (m, 1H), 1.60 - 1.85 (m, 2H), 1.90 - 2.15 (m, 2H), 2.15 - 2.40 (m, 3H), 2.50 - 3.00 (m, 4.4H), 3.10 - 3.35 (m, 1.8H), 3.72 (ddd, J = 6, 13, 13 Hz, 0.4H), 3.84 (ddd, J = 3, 13, 13 Hz, 0.4H), 3.90 - 4.00 (m, 1H), 4.51 (d, J = 16 Hz, 0.6H), 4.64 (d, J = 16 Hz, 0.6H), 4.82 (d, J = 16 Hz, 0.4H), 4.90 (d, J = 16 Hz, 0.4H), 6.48 (d, J = 8 Hz, 0.4H), 6.50 (d, J = 8 Hz, 0.6H), 6.54 (d, J = 2 Hz, 0.4H), 6.59 (d, J = 2 Hz, 0.6H), 6.65 (ddd, J = 3, 8, 8 Hz, 1H), 6.88 (d, J = 3 Hz, 1H), 6.90 - 7.00 (m, 2H), 7.00 - 7.20 (m, 2H), 7.59 (t, J = 8 Hz, 1H).

(Example 126)

Synthesis of (5aS,6R,11bS)-14-(cyclopropylmethyl)-3-(2-(indolin-1-yl)ethyl)-2,3,4,5,6,7-hexahydro-6,11b-(epiminoethano)naphtho[1,2-d]azepine-5a,10(1H)-diol

[Chemical Formula 151]

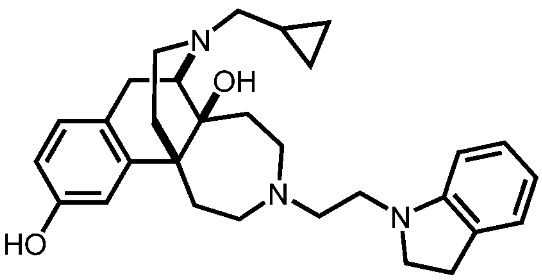

The title compound was obtained from the compound obtained in Example 125 according to the method described in Example 24.

$^1$H-NMR (400 MHz, $CDCl_3$)δ (ppm): 0.00 - 0.15 (m, 2H), 0.45 - 0.60 (m, 2H), 0.75 - 1.00 (m, 2H), 1.40 - 1.90 (m, 5H), 1.90 - 2.05 (m, 2H), 2.20 - 2.40 (m, 3H), 2.45 - 2.60 (m, 1H), 2.65 - 3.00 (m, 9H), 3.10 - 3.40 (m, 4H), 4.72 (br s, 1H), 6.45 (d, J = 8 Hz, 1H), 6.50 (d, J = 3 Hz, 1H), 6.56 (dd, J = 3, 8 Hz, 1H), 6.61 (dd, J = 8, 8 Hz, 1H), 6.89 (d, J = 8 Hz, 1H), 7.00 - 7.05 (m, 2H).

(Example 127)

Synthesis of (5aS,6R,11bS)-3-(2-(benzo[d]thiazol-2-yl)ethyl)-14-(cyclopropylmethyl)-10-methoxy-2,3,4,5,6,7-hexahydro-6,11b-(epiminoethano)naphtho[1,2-d]azepin-5a(1H)-ol

[Chemical Formula 152]

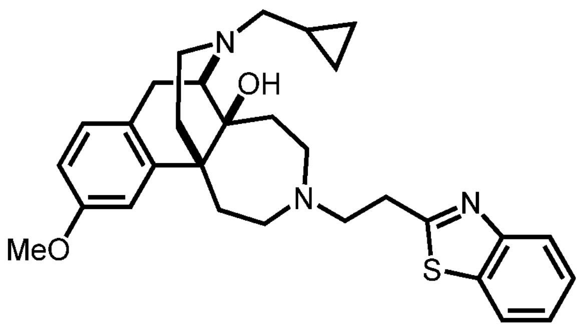

The title compound was obtained from the isomer C obtained in Example 2 and 2-(benzo[d]thiazol-2-yl)ethan-1-ol according to the method described in Example 32.

$^1$H-NMR (400 MHz, $CDCl_3$)δ (ppm): 0.03 - 0.17 (m, 2H), 0.45 - 0.55 (m, 2H), 0.78 - 0.90 (m, 1H), 1.01 - 1.08 (m, 1H), 1.55 (ddd, J = 2, 5, 14 Hz, 1H), 1.86 - 2.03 (m, 3H), 2.09 (ddd, J = 5, 13, 13 Hz, 1H), 2.35 (dd, J = 6, 13 Hz, 1H), 2.367 (dd, J = 6, 13 Hz, 1H), 2.45 - 2.58 (m, 2H), 2.61 (ddd, J = 3, 5, 13 Hz, 1H), 2.72 (ddd, J = 4, 4, 13 Hz, 1H), 2.78 - 2.91 (m, 3H), 2.95 (d, J = 6 Hz, 1H), 2.98 (d, J = 18 Hz, 1H), 3.04 (ddd, J = 3, 12, 12 Hz, 1H), 3.12 - 3.21 (m, 3H), 3.72 (s, 3H), 6.68 - 6.72 (m, 2H), 6.99 - 7.03 (m, 1H), 7.31 (ddd, J = 1, 7, 8 Hz, 1H), 7.40 (ddd, J = 1, 7, 8 Hz, 1H), 7.75 - 7.79 (m, 1H), 7.88 - 7.92 (m, 1H).

(Example 128)

Synthesis of (5aS,6R,11bS)-3-(2-(benzo[d]thiazol-2-yl)ethyl)-14-(cyclopropylmethyl)-2,3,4,5,6,7-hexahydro-6,11b-(epiminoethano)naphtho[1,2-d]azepine-5a,10(1H)-diol

[Chemical Formula 153]

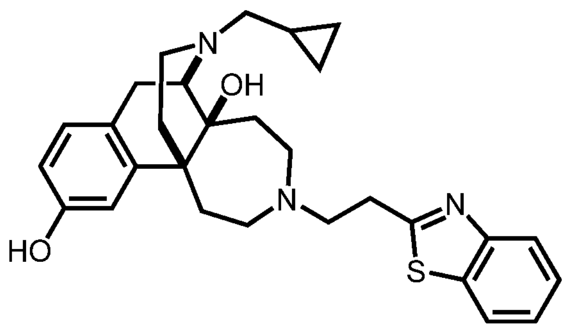

The title compound was obtained from the compound obtained in Example 127 according to the method described in Example 6.

$^1$H-NMR (400 MHz, $CDCl_3$)δ (ppm): 0.03 - 0.16 (m, 2H), 0.42 - 0.56 (m, 2H), 0.76 - 0.88 (m, 1H), 0.96 - 1.06 (m, 1H), 1.51 (dd, J = 4, 14 Hz, 1H), 1.71 - 1.80 (m, 1H), 1.89 (ddd, J = 3, 12, 14 Hz, 1H), 1.95 - 2.09 (m, 2H), 2.34 (dd, J = 6, 12 Hz, 1H), 2.34 (dd, J = 6, 12 Hz, 1H), 2.42 (ddd, J = 4, 12, 16 Hz, 1H), 2.48 - 2.58 (m, 1H), 2.59 - 2.70 (m, 2H), 2.76 (dd, J = 6, 18 Hz, 1H), 2.83 - 2.98 (m, 5H), 3.10 - 3.22 (m, 3H), 4.70 (br s, 1H), 6.55 (d, J = 2 Hz, 1H), 6.62 (dd, J = 2, 8 Hz, 1H), 6.91 (d, J = 8 Hz, 1H), 7.30 (dd, J = 8, 8 Hz, 1H), 7.39 (dd, J = 8, 8 Hz, 1H), 7.78 (d, J = 8 Hz, 1H), 7.91 (d, J = 8 Hz, 1H).

(Example 129)

Synthesis of (5aS,6R, 11bS)-14-(cyclopropylmethyl)-10-methoxy-3-(2-(piperidin-1-yl)ethyl)-2,3,4,5,6,7-hexahydro-6,11b-(epiminoethano)naphtho[1,2-d]azepin-5a(1H)-ol

[Chemical Formula 154]

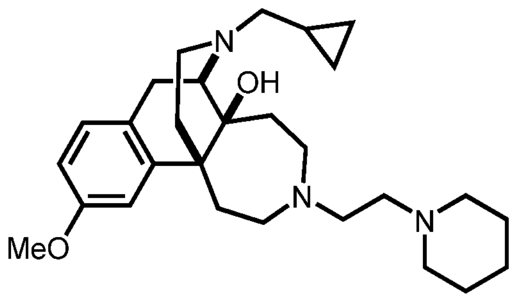

The title compound was obtained from the isomer C obtained in Example 2 and 1-(2-chloroethyl)piperidine hydrochloride according to the method described in Example 31.

$^{1}$H-NMR (400 MHz, $CDCl_3$)δ (ppm): 0.04 - 0.17 (m, 2H), 0.44 - 0.55 (m, 2H), 0.77 - 0.94 (m, 1H), 0.98 - 1.09 (m, 1H), 1.19 - 1.71 (m, 10 Hz), 1.75 - 2.15 (m, 3H), 2.22 - 2.42 (m, 8H), 2.46 - 2.88 (m, 6H), 2.90 - 3.02 (m, 2H), 3.06 - 3.21 (m, 1H), 3.77 (s, 3H), 6.63 - 6.71 (m, 2H), 6.99 (d, J = 8 Hz, 1H).

(Example 130)

Synthesis of (5aS,6R, 11bS)-14-(cyclopropylmethyl)-3-(2-(piperidin-1-yl)ethyl)-2,3,4,5,6,7-hexahydro-6,11b-(epiminoethano)naphtho[1,2-d]azepine-5a,10(1H)-diol

[Chemical Formula 155]

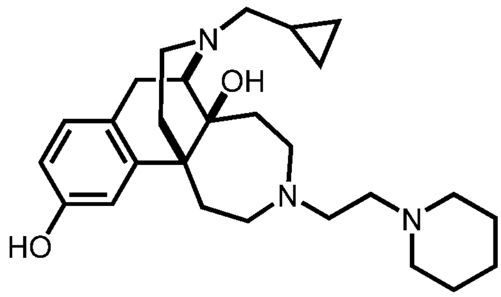

To a solution of the compound (5.3 mg, 0.0117 mmol) in chloroform (1 mL) obtained in Example 129 was added a 1 M boron tribromide-dichloromethane solution (70 pL, 0.070 mmol) under ice cooling, followed by stirring at room temperature for 1 hour. To the reaction mixture, a saturated aqueous sodium bicarbonate solution was added under ice cooling, followed by extraction three times with ethyl acetate. The combined extracts were dried over sodium sulfate and then concentrated under reduced pressure. To the obtained concentrated residue dissolved in methanol (1 mL), 10% palladium-activated carbon (55% wet) (3.4 mg) was added, followed by stirring under a hydrogen atmosphere at room temperature for 15 hours. The reaction mixture was filtered through a membrane filter, and the filtrate was concentrated under reduced pressure. The obtained crude product was purified by preparative thin layer chromatography (chloroform : 2 M ammonia-methanol solution = 4 : 1) to yield the title compound (4.0 mg, 78%) as a pale yellow solid substance.

$^{1}$H-NMR (400 MHz, $CDCl_3$)δ (ppm): 0.04 - 0.17 (m, 2H), 0.44 - 0.55 (m, 2H), 0.76 - 0.92 (m, 1H), 0.95 - 1.08 (m, 1H), 1.14 - 1.91 (m, 10H), 1.93 - 2.10 (m, 2H), 2.14 - 3.02 (m, 17H), 3.07 - 3.22 (m, 1H), 4.75 (br s, 1H), 6.46 (d, J

= 2 Hz, 1H), 6.55 (dd, J = 2, 8 Hz, 1H), 6.89 (d, J = 8 Hz, 1H).

(Example 131)

Synthesis of 2-((5aS,6R, 11bS)-14-(cyclopropylmethyl)-5a,10-dihydroxy-1,2,5,5a,6,7-hexahydro-6,11b-(epiminoethano)naphtho[1,2-d]azepin-3(4H)-yl)-1-(piperidin-1-yl)ethan-1-one

[Chemical Formula 156]

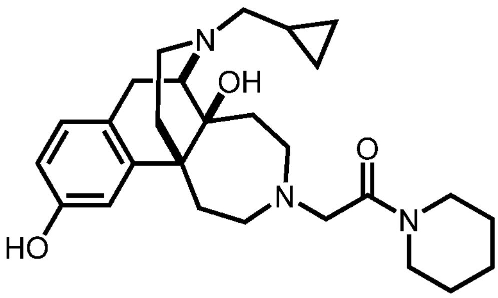

The title compound was obtained from the compound E obtained in Example 3 and 2-chloro-1-(piperidin-1-yl)ethan-1-one (synthesized by the method described in WO 2018148576) according to the method described in Example 31.

$^1$H-NMR (400 MHz, $CDCl_3$)$\delta$ (ppm): 0.03 - 0.17 (m, 2H), 0.43 - 0.56 (m, 2H), 0.75 - 0.91 (m, 1H), 0.96 - 1.06 (m, 1H), 1.08 - 2.15 (m, 12H), 2.33 (d, J = 6 Hz, 1H), 2.42 - 2.61 (m, 4H), 2.72 - 2.84 (m, 1H), 2.85 - 3.20 (m, 6H), 3.15 (d, J = 13 Hz, 1H), 3.23 (d, J = 13 Hz, 1H), 3.33 - 3.51 (m, 2H), 4.63 (br s, 1H), 6.57 - 6.70 (m, 2H), 6.91 (d, J = 8 Hz, 1H).

(Example 132)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(4,4-dimethyl-1,4-azasilinan-1-yl)ethan-1-one

[Chemical Formula 157]

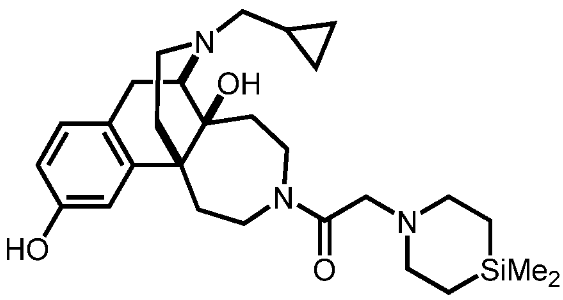

To a solution of the compound E (10 mg, 0.030 mmol) obtained in Example 3 in chloroform (1 mL) were added N,N-diisopropylethylamine (21 pL, 0.12 mmol) and chloroacetyl chloride (4.8 pL, 0.061 mmol) under ice cooling, followed by stirring at room temperature for 1 hour. Next, to the reaction mixture were added 4,4-dimethyl-1,4-azasilinane (16 mg, 0.12 mmol), sodium iodide (10 mg, 0.061 mmol), and acetonitrile (1 mL), followed by stirring at 60°C for 6 hours. Thereafter, a suspension of potassium carbonate (28 mg, 0.204 mmol) in methanol (1 mL) was added, followed by stirring at room temperature for 18 hour. Water was added to the reaction mixture, followed by extraction three times with ethyl acetate. The combined extracts were dried over sodium sulfate and then concentrated under reduced pressure. The obtained crude product was purified by silica gel column chromatography (amino group-supported silica gel, 0 to 5% methanol/chloroform) to yield the title compound (14 mg, 95%) as a pale yellow solid substance.

$^1$H-NMR (400 MHz, $CDCl_3$)δ (ppm): 0.00 - 0.15 (m, 8H), 0.46 - 0.56 (m, 2H), 0.61 - 0.92 (m, 5H), 1.01 - 1.13 (m, 1H), 1.22 - 1.86 (m, 2H), 1.89 - 2.19 (m, 3H), 2.25 - 2.41 (m, 2.6H), 2.48 - 2.69 (m, 5.4H), 2.71 - 2.84 (m, 1H), 2.86 - 3.27 (m, 4H), 3.28 - 3.48 (m, 1H), 3.51 - 3.71 (m, 1.4H), 3.72 - 3.85 (m, 1H), 3.89 - 4.00 (m, 0.6H), 4.49 - 4.534.51 (m, 1H), 6.54 - 6.69 (m, 1.4H), 6.76 - 6.81 (m, 0.6H), 6.90 - 6.96 (m, 1H).

(Example 133)

Synthesis of (5aS,6R, 11bS)-14-(cyclopropylmethyl)-3-(2-(4,4-dimethyl-1,4-azasilinan-1-yl)ethyl)-2,3,4,5,6,7-hexahydro-6,11b-(epiminoethano)naphtho[1,2-d]azepine-5a,10(1H)-diol

[Chemical Formula 158]

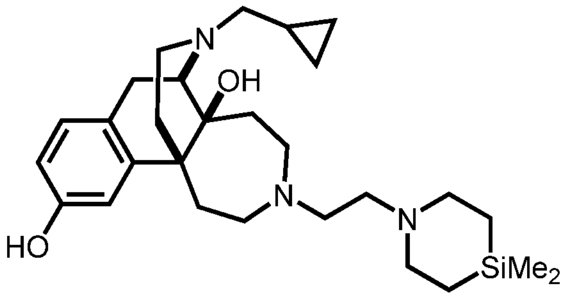

The title compound was obtained from the compound obtained in Example 132 according to the method described in Example 24.

$^1$H-NMR (400 MHz, $CDCl_3$)δ (ppm): 0.00 - 0.19 (m, 8H), 0.43 - 0.55 (m, 2H), 0.66 - 0.90 (m, 5H), 0.95 - 1.07 (m, 1H), 1.16 - 2.11 (m, 5H), 2.23 - 2.41 (m, 3H), 2.45 - 2.98 (m, 15H), 3.09 - 3.22 (m, 1H), 4.76 (br s, 1H), 6.46 (d, J = 2 Hz, 1H), 6.56 (dd, J = 2, 8 Hz, 1H), 6.89 (d, J = 8 Hz, 1H).

(Example 134)

Synthesis of mixture of (5aR,6R,11bS)-14-(cyclopropylmethyl)-10-methoxy-1,2,5,5a,6,7-hexahydro-6,11b-(epiminoethano)naphtho[1,2-c]azepin-3(4H)-one (isomer G) and (5aR,6R,11bS)-14-(cyclopropylmethyl)-10-methoxy-3,4,5,5a,6,7-hexahydro-6,11b-(epiminoethano)naphtho[1,2-d]azepin-2(1H)-one (isomer H)

[Chemical Formula 159]

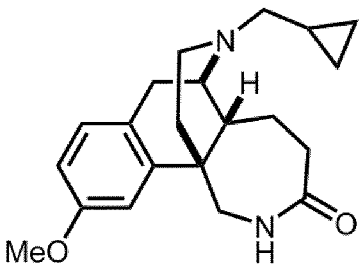

Isomer G

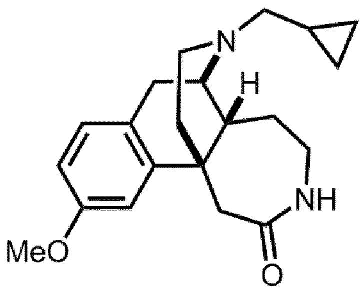

Isomer H

A mixture of the title isomers G and H was obtained as a colorless amorphous form using (4bS,8aR,9R)-11-(cyclopropylmethyl)-3-methoxy-8,8a,9,10-tetrahydro-5H-9,4b-(epiminoethano)phenanthren-6(7H)-one (synthesized by the method described in Bioorganic Medicinal Chemistry, 2012, 20, 949) according to the method described in Reference Example 3 and Example 1.

$^1$H-NMR (400 MHz, $CDCl_3$)δ (ppm): 0.00 - 0.20 (m, 2H), 0.45 - 0.60 (m, 2H), 0.75 - 0.90 (m, 1H), 1.20 - 1.80 (m, 3H), 1.86 (ddd, J = 5, 12, 12 Hz, 1H), 1.90 - 2.10 (m, 2H), 2.25 - 2.35 (m, 1H), 2.35 - 2.70 (m, 3H), 2.70 - 2.90 (m, 2.5H), 3.00 - 3.05 (m, 0.5H), 3.05 - 3.15 (m, 1.5H), 3.30 - 3.40 (m, 0.5H), 3.40 - 3.60 (m, 1H), 3.76 (s, 1.5H), 3.79 (s, 1.5H), 5.81 (br s, 0.5H), 5.92 (br s, 0.5H), 6.52 (d, J = 3 Hz, 0.5H), 6.68 (dd, J = 3, 8 Hz, 1H), 6.94 (d, J = 8 Hz, 0.5H), 6.99 (d, J = 8 Hz, 0.5H), 7.10 (d, J = 3 Hz, 0.5H).

(Example 135)

Synthesis of (5aR,6R,11bS)-14-(cyclopropylmethyl)-10-methoxy-1,2,3,4,5,5a,6,7-octahydro-6,11b-(epiminoethano)naphtho[1,2-c]azepine

[Chemical Formula 160]

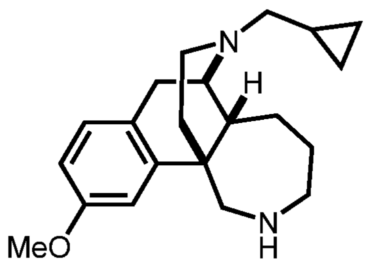

The title compound was obtained as a colorless amorphous form from the isomers G and H described in Example 134 according to the method described in Example 2.

$^1$H-NMR (400 MHz, $CDCl_3$)δ (ppm): 0.00 - 0.20 (m, 2H), 0.45 - 0.55 (m, 2H), 0.75 - 0.90 (m, 1H), 1.20 - 1.50 (m, 3H), 1.65 - 2.20 (m, 6H), 2.30 (dd, J = 7, 12 Hz, 1H), 2.55 (dd, J = 6, 13 Hz, 1H), 2.60 - 2.70 (m, 2H), 2.70 - 2.90 (m, 3H), 3.04 (dd, J = 5, 13 Hz, 1H), 3.18 (br s, 1H), 3.45 (d, J = 14 Hz, 1H), 3.78 (s, 3H), 6.50 - 6.70 (m, 2H), 7.02 (d, J = 8 Hz, 1H).

(Example 136)

Synthesis of (5aS,6R, 11bS)-14-(cyclopropylmethyl)-10-methoxy-2-(2-(pyridin-2-yl)ethyl)-2,3,4,5,6,7-hexahydro-6,11b-(epiminoethano)naphtho[1,2-c]azepin-5a(1H)-ol

[Chemical Formula 161]

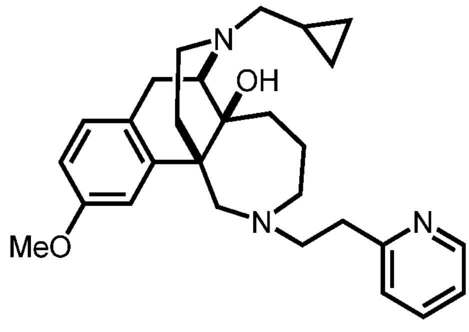

The title compound was obtained from the isomer D obtained in Example 2 and 2-(pyridin-2-yl)ethyl 4-methylbenzenesulfonate according to the method described in Example 31.

$^1$H-NMR (400 MHz, $CDCl_3$)δ (ppm): 0.03 - 0.16 (m, 2H), 0.43 - 0.55 (m, 2H), 0.78 - 1.00 (m, 2H), 1.45 - 1.69 (m, 3H), 1.71 - 1.82 (m, 1H), 1.91 - 2.14 (m, 3H), 2.28 - 2.40 (m, 2H), 2.51 - 2.85 (m, 5H), 2.86 - 3.31 (m, 6H), 3.73 (s, 3H), 6.67 (dd, J = 3, 8 Hz, 1H), 6.78 (d, J = 3 Hz, 1H), 6.96 (d, J = 8 Hz, 1H), 7.08 (dd, J = 5, 8 Hz, 1H), 7.14 - 7.18 (m, 1H), 7.54 (ddd, J = 2, 8, 8 Hz, 1H), 8.52 (ddd, J = 1, 2, 5 Hz, 1H).

(Example 137)

Synthesis of (5aS,6R, 11bS)-14-(cyclopropylmethyl)-2-(2-(pyridin-2-yl)ethyl)-2,3,4,5,6,7-hexahydro-6,11b-(epiminoethano)naphtho[1,2-c]azepine-5a,10(1H)-diol

[Chemical Formula 162]

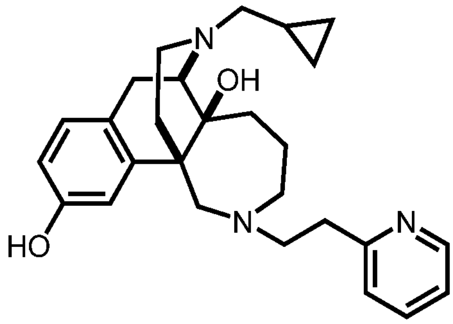

The title compound was obtained from the compound obtained in Example 136 according to the method described in Example 6.

$^{1}H$-NMR (400 MHz, $CDCl_3$)δ (ppm): 0.05 - 0.18 (m, 2H), 0.44 - 0.55 (m, 2H), 0.78 - 0.99 (m, 2H), 1.39 - 1.47 (m, 1H), 1.56 - 1.65 (m, 1H), 1.69 (ddd, J = 2, 12, 13 Hz, 1H), 1.88 (ddd, J = 6, 13, 13 Hz, 1H), 2.14 (ddd, J = 4, 12, 12 Hz, 1H), 2.23 - 2.42 (m, 3H), 2.54 - 2.81 (m, 6H), 2.84 - 3.09 (m, 5H), 3.44 (d, J = 15 Hz, 1H), 6.72 (dd, J = 2, 8 Hz, 1H), 6.88 (d, J = 2 Hz, 1H), 6.93 (d, J = 8 Hz, 1H), 7.08 - 7.13 (m, 1H), 7.18 (ddd, J = 1, 5, 8 Hz, 1H), 7.63 (ddd, J = 2, 8, 8 Hz, 1H), 8.53 - 8.58 (m, 1H).

(Example 138)

Synthesis of 1-((5aS,6R,11bS)-14-(cyclopropylmethyl)-5a-hydroxy-10-methoxy-3,4,5,5a,6,7-hexahydro-6,11b-(epiminoethano)naphtho[1,2-c]azepin-2(1H)-yl)-2-(pyrimidin-2-yl)ethan-1-one

[Chemical Formula 163]

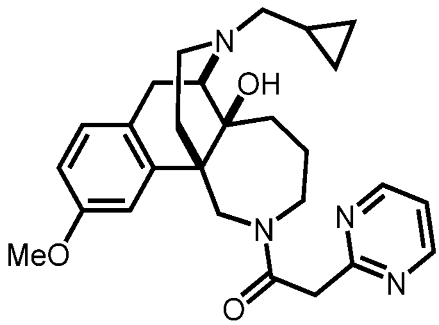

The title compound was obtained from the isomer D obtained in Example 2 and 2-(pyrimidin-2-yl)acetic acid according to the method described in Example 7.

$^{1}H$-NMR (400 MHz, $CDCl_3$)δ (ppm): 0.05 - 0.17 (m, 2H), 0.45 - 0.57 (m, 2H), 0.77 - 0.92 (m, 1H), 1.06 - 1.13 (m, 1H), 1.55 - 1.72 (m, 2H), 1.76 (dd, J = 6, 13 Hz, 1H), 1.92 - 2.10 (m, 2H), 2.23 - 2.42 (m, 3H), 2.55 - 2.63 (m, 1H), 2.79 (dd, J = 6, 18 Hz, 1H), 2.90 - 3.06 (m, 3H), 3.64 (d, J = 15 Hz, 1H), 3.69 - 3.81 (m, 1H), 3.71 (s, 3H), 3.95 (d, J = 15 Hz, 1H), 4.09 (d, J = 15 Hz, 1H), 4.79 (d, J = 15 Hz, 1H), 6.69 (dd, J = 3, 8 Hz, 1H), 6.89 (d, J = 3 Hz, 1H), 6.94 (d, J = 8 Hz, 1H), 7.15 (t, J = 5 Hz, 1H), 8.65 (d, J = 5 Hz, 2H).

(Example 139)

Synthesis of 1-((5aS,6R,11bS)-14-(cyclopropylmethyl)-5a,10-dihydroxy-3,4,5,5a,6,7-hexahydro-6,11b-(epiminoethano)naphtho[1,2-c]azepin-2(1H)-yl)-2-(pyrimidin-2-yl)ethan-1-one

[Chemical Formula 164]

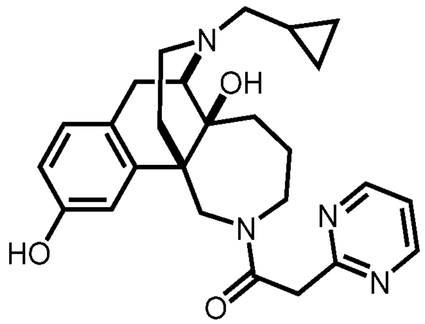

The title compound was obtained from the compound obtained in Example 138 according to the method described in Example 6.

$^1$H-NMR (400 MHz, $CDCl_3$)δ (ppm): 0.06 - 0.17 (m, 2H), 0.45 - 0.58 (m, 2H), 0.78 - 0.93 (m, 1H), 1.10 - 1.17 (m, 1H), 1.52 - 1.63 (m, 1H), 1.65 - 1.74 (m, 1H), 1.78 (dd, J = 6, 13 Hz, 1H), 2.02 (ddd, J = 4, 12, 12 Hz, 1H), 2.11 (ddd, J = 3, 12, 12 Hz, 1H), 2.19 - 2.32 (m, 1H), 2.35 (dd, J = 6, 13 Hz, 1H), 2.36 (dd, J = 7, 13 Hz, 1H), 2.55 - 2.63 (m, 1H), 2.65 - 2.74 (m, 1H), 2.78 (dd, J = 7, 18 Hz, 1H), 2.98 (d, J = 7 Hz, 1H), 3.00 (d, J = 18 Hz, 1H), 3.54 (dd, J = 4, 12 Hz, 1H), 3.78 (d, J = 15 Hz, 1H), 3.89 (d, J = 16 Hz, 1H), 3.96 (d, J = 16 Hz, 1H), 4.62 (d, J = 15 Hz, 1H), 6.65 (d, J = 3 Hz, 1H), 6.74 (dd, J = 3, 8 Hz, 1H), 6.97 (d, J = 8 Hz, 1H), 7.29 (t, J = 5 Hz, 1H), 8.76 (d, J = 5 Hz, 2H).

(Example 140)

Synthesis of (5aS,6R, 11bR)-14-(cyclopropylmethyl)-5a-hydroxy-10-methoxy-3,4,5,5a,6,7-hexahydro-6,11b-(epiminoethano)naphtho[1,2-d]azepin-2(1H)-one

[Chemical Formula 165]

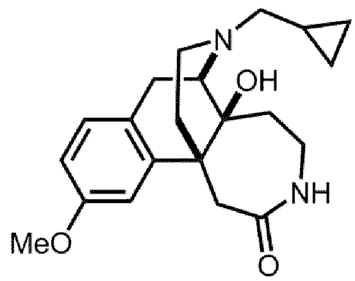

Single isomer A

To a solution of the compound (14 g, 39.3 mmol) obtained in Reference Example 3 in chloroform (400 mL) were added triethylamine (16.4 mL, 117.8 mmol) and p-toluenesulfonyl chloride (22.5 g, 117.8 mmol) under ice cooling, followed by stirring at room temperature for 17 hours. The reaction solution was concentrated under reduced pressure, and to a solution of the obtained concentrated residue in tetrahydrofuran (250 mL) was added 2 M hydrochloric acid (160 mL), followed by stirring at room temperature for 71 hours. After the reaction solution was washed three times with a mixed solution of ethyl acetate and heptane (2/1), the aqueous layer was made basic with potassium carbonate, then extracted twice with ethyl acetate, and extracted once with chloroform. The combined extracts were dried over sodium

sulfate and concentrated under reduced pressure. The obtained crude product was suspended in ethyl acetate (50 mL), followed by stirring for 1 hour. Subsequently, insoluble matter was collected by filtration to yield 4-methylbenzenesulfonate (5.15 g, 25%) of the title compound as a colorless solid. Subsequently, a reaction similar to the method described in Example 24 was performed on 4-methylbenzenesulfonate of the title compound to yield the title compound (509.3 mg, 10% recovery) as a colorless amorphous form as an unreacted recovery raw material.

$^1$H-NMR (400 MHz, $CDCl_3$)δ (ppm): 0.08 - 0.16 (m, 2H), 0.50 - 0.57 (m, 2H), 0.78 - 0.92 (m, 1H), 1.10 - 1.18 (m, 1H), 1.23 - 1.33 (m, 1H), 1.78 (ddd, J = 2, 11, 14 Hz, 1H), 2.07 - 2.13 (m, 2H), 2.34 - 2.42 (m, 2H), 2.56 - 2.63 (m, 2H), 2.72 - 2.82 (m, 1H), 2.83 - 2.97 (m, 2H), 3.02 (d, J = 18 Hz, 1H), 3.51 (d, J = 15 Hz, 1H), 3.80 (s, 3H), 3.87 (ddd, J = 3, 11, 15 Hz, 1H), 5.62 - 5.71 (m, 1H), 6.71 (dd, J = 3, 8 Hz, 1H), 6.97 (d, J = 8 Hz, 1H), 7.15 (d, J = 3 Hz, 1H).

(Example 141)

Synthesis of (5aS,6R,11bR)-3-benzyl-14-(cyclopropylmethyl)-5a-hydroxy-10-methoxy-3,4,5,5a,6,7-hexahydro-6,11b-(epiminoethano)naphtho[1,2-d]azepin-2(1H)-one

[Chemical Formula 166]

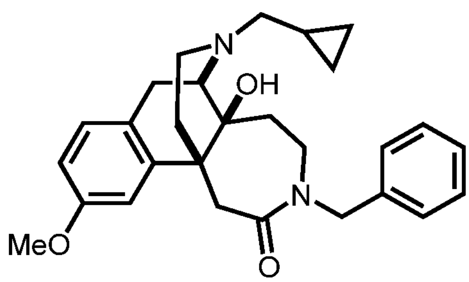

To a solution of the compound (14.3 mg, 0.04 mmol) obtained in Example 140 in N,N-dimethylformamide (1 mL) was added sodium hydride (55% oil dispersion, 16 mg, 0.37 mmol), followed by stirring at room temperature for 10 minutes. Benzyl bromide (47.5 pL, 0.4 mmol) was then added, followed by stirring at room temperature for 1 hour. To the reaction solution, a saturated sodium bicarbonate aqueous solution was added, followed by extraction three times with chloroform. The combined extracts were dried over sodium sulfate and then concentrated under reduced pressure. The obtained crude product was purified by silica gel column chromatography (0 to 10% methanol/chloroform) to yield the title compound (14.4 mg, 79%) as a colorless amorphous form.

$^1$H-NMR (400 MHz, $CDCl_3$)δ (ppm): 0.05 - 0.15 (m, 2H), 0.44 - 0.53 (m, 2H), 0.72 - 0.91 (m, 1H), 1.15 - 1.49 (m, 3H), 2.00 - 2.15 (m, 2H), 2.33 (d, J = 7 Hz, 2H), 2.52 - 2.66 (m, 2H), 2.77 (d, J = 15 Hz, 1H), 2.78 - 2.92 (m, 2H), 2.94 (d, J = 18 Hz, 1H), 3.62 (d, J = 15 Hz, 1H), 3.82 (d, J = 15 Hz, 1H), 3.85 (s, 3H), 4.06 (dd, J = 11, 15 Hz, 1H), 5.05 (d, J = 15 Hz, 1H), 6.62 (d, J = 8 Hz, 2H), 6.79 (dd, J = 3, 8 Hz, 1H), 6.97 (d, J = 9 Hz, 1H), 7.01 (t, J = 8 Hz, 2H), 7.09 (t, J = 7 Hz, 1H), 7.32 (d, J = 3 Hz, 1H).

(Example 142)

Synthesis of (5aS,6R, 11bR)-3-benzyl-14-(cyclopropylmethyl)-5a, 10-dihydroxy-3,4,5,5a,6,7-hexahydro-6,11b-(epiminoethano)naphtho[1,2-d]azepin-2(1H)-one

[Chemical Formula 167]

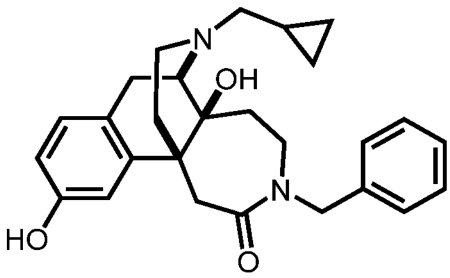

The title compound was obtained from the compound obtained in Example 141 according to the method described in Example 6.

$^1$H-NMR (400 MHz, $CDCl_3$)δ (ppm): 0.05 - 0.12 (m, 2H), 0.44 - 0.55 (m, 2H), 0.71 - 0.93 (m, 1H), 1.08 - 1.50 (m, 3H), 2.02 - 2.12 (m, 2H), 2.33 (d, J = 6 Hz, 2H), 2.50 - 2.64 (m, 2H), 2.71 (d, J = 15 Hz, 1H), 2.78 - 2.98 (m, 3H), 3.60 (d, J = 15 Hz, 1H), 3.84 (d, J = 15 Hz, 1H), 4.07 (dd, J = 11, 15 Hz, 1H), 5.03 (d, J = 14 Hz, 1H), 6.65 (d, J = 8 Hz, 2H), 6.78 (dd, J = 3, 8 Hz, 1H), 6.95 (d, J = 8 Hz, 1H), 7.03 (t, J = 7 Hz, 2H), 7.11 (t, J = 7 Hz, 1H), 7.18 (d, J = 2 Hz, 1H).

(Example 143)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(4-(trifluoromethyl)-1H-pyrazol-1-yl)ethan-1-one

[Chemical Formula 168]

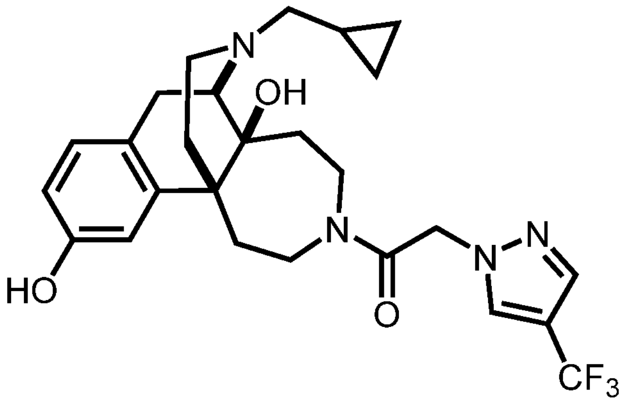

The title compound was obtained from the compound E obtained in Example 3 according to the method described in Example 26.

$^1$H-NMR (400 MHz, $CDCl_3$)δ (ppm): 0.05 - 0.20 (m, 2H), 0.45 - 0.60 (m, 2H), 0.75 - 0.90 (m, 1H), 1.05 - 1.15 (m, 1H), 1.30 - 2.50 (m, 7H), 2.50 - 2.85 (m, 2.5H), 2.90 - 3.15 (m, 2.5H), 3.30 - 3.40 (m, 1H), 3.40 - 3.50 (m, 1H), 3.70 - 3.80 (m, 0.5H), 3.80 - 3.95 (m, 1H), 4.05 - 4.15 (m, 0.5H), 4.66 (d, J = 16 Hz, 0.5H), 4.86 (d, J = 16 Hz, 0.5H), 4.94 (d, J = 16 Hz, 0.5H), 5.00 (d, J = 16 Hz, 0.5H), 6.60 - 6.70 (m, 2H), 6.90 - 7.00 (m, 1H), 7.59 (s, 0.5H), 7.66 (s, 0.5H), 7.69 (m, 0.5H), 7.78 (s, 0.5H).

(Example 144)

Synthesis of (5aS,6R, 11bS)-14-(cyclopropylmethyl)-3-(2-(4-(trifluoromethyl)-1H-pyrazol-1-yl)ethyl)-2,3,4,5,6,7-hexahydro-6,11b-(epiminoethano)naphtho[1,2-d]azepine-5a,10(1H)-diol

[Chemical Formula 169]

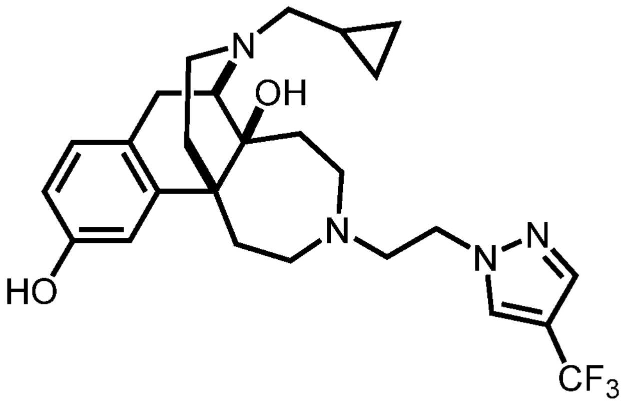

The title compound was obtained from the compound obtained in Example 143 according to the method described in Example 24.

$^1$H-NMR (400 MHz, $CDCl_3$)δ (ppm): 0.05 - 0.20 (m, 2H), 0.45 - 0.60 (m, 2H), 0.75 - 1.00 (m, 1H), 1.00 - 1.10 (m, 1H), 1.40 - 2.20 (m, 4H), 2.25 - 2.50 (m, 5H), 2.50 - 2.70 (m, 2H), 2.75 - 3.15 (m, 6H), 3.15 - 3.30 (m, 1H), 3.90 - 4.05 (m, 1H), 4.10 - 4.20 (m, 1H), 6.60 - 6.70 (m, 2H), 6.99 (d, J = 8 Hz, 1H), 7.17 (s, 1H), 7.62 (s, 1H).

(Example 145)

Synthesis of (5aS,6R, 11bR)-3-(2-cyclohexylethyl)-14-(cyclopropylmethyl)-5a-hydroxy-10-methoxy-3,4,5,5a,6,7-hexahydro-6,11b-(epiminoethano)naphtho[1,2-d]azepin-2(1H)-one

[Chemical Formula 170]

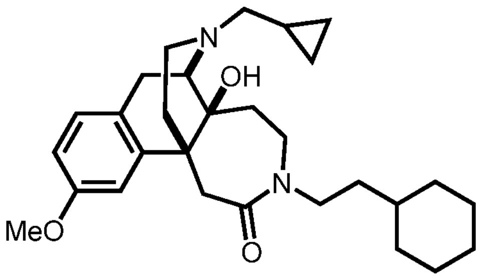

The title compound was obtained from the compound obtained in Example 140 and (2-bromoethyl)cyclohexane according to the method described in Example 141.

$^1$H-NMR (400 MHz, $CDCl_3$)δ (ppm): 0.05 - 0.15 (m, 2H), 0.45 - 0.55 (m, 2H), 0.65 - 1.77 (m, 17H), 1.99 - 2.12 (m, 2H), 2.35 (dd, J = 7, 13 Hz, 1H), 2.36 (dd, J = 7, 13 Hz, 1H), 2.53 - 2.61 (m, 1H), 2.66 (d, J = 15 Hz, 1H), 2.74 (dd, J = 6, 18 Hz, 1H), 2.83 - 2.97 (m, 3H), 3.01 (d, J = 18 Hz, 1H), 3.46 - 3.64 (m, 2H), 3.81 (s, 3H), 4.06 (dd, J = 11, 15 Hz, 1H), 6.68 (dd, J = 3, 8 Hz, 1H), 6.94 (d, J = 8 Hz, 1H), 7.20 (d, J = 3 Hz, 1H).

(Example 146)

Synthesis of (5aS,6R, 11bR)-3-(2-cyclohexylethyl)-14-(cyclopropylmethyl)-5a, 10-dihydroxy-3,4,5,5a,6,7-hexahydro-6,11b-(epiminoethano)naphtho[1,2-d]azepin-2(1H)-one

[Chemical Formula 171]

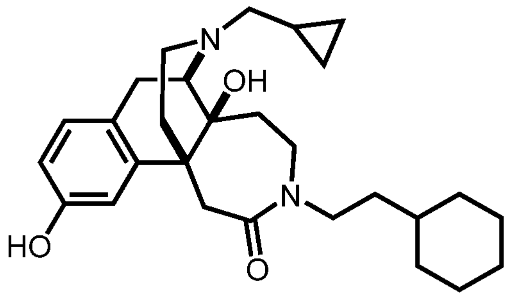

The title compound was obtained from the compound obtained in Example 145 according to the method described in Example 6.

$^1$H-NMR (400 MHz, $CDCl_3$)$\delta$ (ppm): 0.05 - 0.14 (m, 2H), 0.46 - 0.55 (m, 2H), 0.64 - 0.90 (m, 4H), 0.97 - 1.30 (m, 6H), 1.35 - 1.46 (m, 1H), 1.47 - 1.65 (m, 5H), 1.67 - 1.79 (m, 1H), 1.96 - 2.12 (m, 2H), 2.35 (dd, J = 7, 13 Hz, 1H), 2.36 (dd, J = 7, 13 Hz, 1H), 2.51 - 2.61 (m, 1H), 2.66 (d, J = 15 Hz, 1H), 2.73 (dd, J = 6, 18 Hz, 1H), 2.84 - 3.04 (m, 4H), 3.44 - 3.58 (m, 2H), 4.07 (dd, J = 11, 15 Hz, 1H), 6.68 (dd, J = 2, 8 Hz, 1H), 6.91 (d, J = 8 Hz, 1H), 7.16 (d, J = 2 Hz, 1H).

(Example 147)

Synthesis of 2-chloro-1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)ethan-1-one

[Chemical Formula 172]

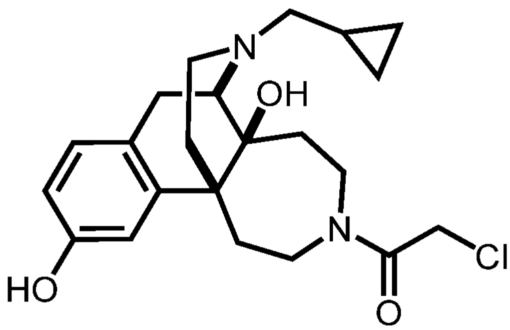

To the compound E (40 mg, 0.12 mmol) obtained in Example 3 and N,N-diisopropylethylamine (125 pL, 0.73 mmol) dissolved in tetrahydrofuran (1.5 mL) was added chloroacetyl chloride (32 $\mu$L, 0.40 mmol) under ice cooling, followed by stirring for 1 hour under a nitrogen atmosphere. After the reaction solution was diluted with ethyl acetate and then washed with a saturated aqueous sodium bicarbonate solution, the obtained organic layer was dried over sodium sulfate and then concentrated under reduced pressure. The obtained crude product was purified by silica gel column chromatography (amino group-supported silica gel, 1 to 20% methanol/chloroform) to yield the title compound (40 mg, 81%) as a yellow oily matter.

$^1$H-NMR (400 MHz, $CDCl_3$)$\delta$ (ppm): 0.05 - 0.20 (m, 2H), 0.45 - 0.60 (m, 2H), 0.75 - 1.00 (m, 1H), 1.08 (d, J = 12 Hz, 0.3H), 1.16 (d, J = 12 Hz, 0.7H), 1.40 - 2.50 (m, 8H), 2.55 - 2.70 (m, 1.3H), 2.75 - 2.85 (m, 1H), 2.90 - 3.15 (m, 2.7H), 3.30 - 4.00 (m, 4.3H), 4.17 (s, 1H), 4.20 - 4.30 (m, 0.7H), 6.55 - 6.65 (m, 0.6H), 6.71 (dd, J = 3, 8 Hz, 0.7H), 6.90 (d, J = 3 Hz, 0.7H), 6.94 (d, J = 8 Hz, 0.3H), 6.98 (d, J = 8 Hz, 0.7H).

(Example 148)

Synthesis of 2-(4-(tert-butyl)-1H-pyrazol-1-yl)-1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)ethan-1-one

[Chemical Formula 173]

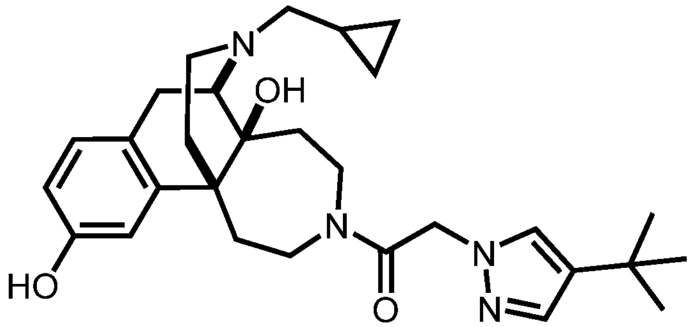

The compound (20 mg, 0.049 mmol) obtained in Example 147, potassium carbonate (102 mg, 0.735 mmol), 4-tert-butylpyrazole (61 mg, 0.490 mmol), and sodium iodide (9 mg, 0.059 mmol) were dissolved in acetonitrile (1 mL), followed by stirring at 60°C for 16 hours under a nitrogen atmosphere. After the reaction solution was diluted with ethyl acetate and then washed with a saturated aqueous sodium bicarbonate solution, the obtained organic layer was dried over sodium sulfate and then concentrated under reduced pressure. The obtained crude product was purified by silica gel column chromatography (amino group-supported silica gel, 1 to 20% methanol/chloroform) to yield the title compound (10 mg, 41%) as a yellow oily matter.

$^1$H-NMR (400 MHz, $CDCl_3$)$\delta$ (ppm): 0.05 - 0.20 (m, 2H), 0.45 - 0.60 (m, 2H), 0.75 - 1.00 (m, 1H), 1.00 - 1.10 (m, 1H), 1.22 (s, 6.3H), 1.24 (s, 2.7H), 1.40 - 2.20 (m, 8H), 2.25 - 2.50 (m, 1.3H), 2.50 - 2.70 (m, 1H), 2.70 - 2.90 (m, 0.7H), 2.90 - 3.30 (m, 3H), 3.30 - 3.40 (m, 0.3H), 3.70 - 3.80 (m, 0.7H), 3.80 - 3.95 (m, 0.7H), 4.05 - 4.15 (m, 0.3H), 4.57 (d, J = 16 Hz, 0.7H), 4.69 (d, J = 16 Hz, 0.7H), 4.88 (s, 0.6H), 6.60 - 6.70 (m, 2H), 6.94 (d, J = 8 Hz, 0.7H), 6.98 (d, J = 8 Hz, 0.3H), 7.16 (s, 0.7H), 7.24 (s, 0.3H), 7.36 (s, 0.7H), 7.37 (s, 0.3H).

(Example 149)

Synthesis of (5aS,6R, 11bS)-3-(2-(4-(tert-butyl)-1H-pyrazol-1-yl)ethyl)-14-(cyclopropylmethyl)-2,3,4,5,6,7-hexahydro-6,11b-(epiminoethano)naphtho[1,2-d]azepine-5a,10(1H)-diol

[Chemical Formula 174]

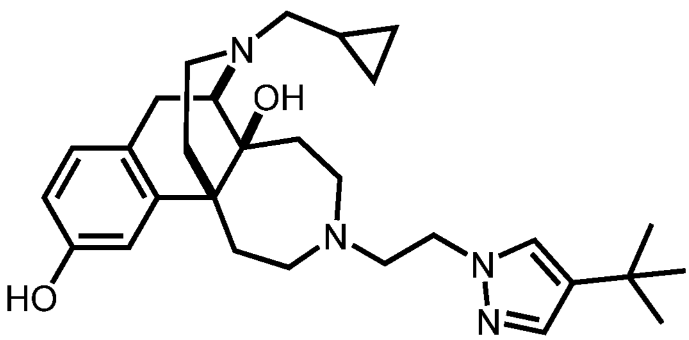

The title compound was obtained from the compound obtained in Example 148 according to the method described in Example 24.

$^1$H-NMR (400 MHz, $CDCl_3$)δ (ppm): 0.05 - 0.20 (m, 2H), 0.45 - 0.60 (m, 2H), 0.75 - 1.00 (m, 1H), 1.03 (d, J = 11 Hz, 1H), 1.21 (s, 9H), 1.40 - 2.10 (m, 5H), 2.20 - 2.40 (m, 3H), 2.50 - 2.70 (m, 3H), 2.74 (dd, J = 6, 18 Hz, 1H), 2.80 - 3.00 (m, 5H), 3.10 - 3.30 (m, 1H), 4.00 - 4.10 (m, 2H), 6.54 (d, J = 3 Hz, 1H), 6.60 (dd, J = 3, 8 Hz, 1H), 6.91 (d, J = 8 Hz, 1H), 7.10 (s, 1H), 7.25 (s, 1H).

(Example 150)

Synthesis of 2,2,2-trichloroethyl (5aS,6R,11bR)-14-(cyclopropylmethyl)-5a-hydroxy-10-methoxy-1,2,5,5a,6,7-hexahydro-6,11b-(epiminoethano)naphtho[1,2-d]azepine-3(4H)-carboxylate

[Chemical Formula 175]

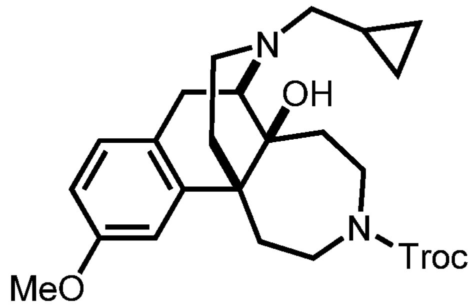

To a solution of the isomer C (342 mg, 1.0 mmol) obtained in Example 2 in tetrahydrofuran (10 mL) were added N,N-diisopropylethylamine (348 μL, 2.0 mmol) and 2,2,2-trichloroethyl chloroformate (165 pL, 1.2 mmol), followed by stirring at room temperature for 1 hour. To the reaction mixture, a saturated sodium bicarbonate aqueous solution was added, followed by extraction three times with chloroform. The organic layers were combined, dried over sodium sulfate, and then concentrated under reduced pressure. The obtained crude product was purified by silica gel column chromatography (0 to 20% methanol/chloroform) to yield the title compound (481 mg, 93%) as a colorless amorphous form.

$^1$H-NMR (400 MHz, $CDCl_3$)δ (ppm): 0.04 - 0.17 (m, 2H), 0.45 - 0.55 (m, 2H), 0.76 - 0.88 (m, 1H), 0.96 - 2.12 (m, 6H), 2.26 - 2.59 (m, 4H), 2.73 - 3.05 (m, 3H), 3.51 - 3.77 (m, 4H), 3.77 (s, 1.5H), 3.79 (s, 1.5H), 4.47 (d, J = 12 Hz, 0.5H), 4.52 (br s, 1H), 4.62 (s, 1H), 4.79 (d, J = 12 Hz, 0.5H), 6.64 - 6.71 (m, 2H), 6.95 - 7.02 (m, 1H).

(Example 151)

Synthesis of 2,2,2-trichloroethyl (5aS,6R,11bR)-5a-acetoxy-14-(cyclopropylmethyl)-10-methoxy-1,2,5,5a,6,7-hexahydro-6,11b-(epiminoethano)naphtho[1,2-d]azepine-3(4H)-carboxylate

[Chemical Formula 176]

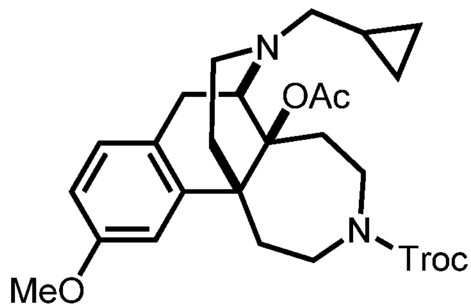

Acetic anhydride (10 mL) was added to the compound (481 mg, 0.93 mmol) obtained in Example 150, followed by heating under reflux for 30 minutes. The reaction mixture was concentrated under reduced pressure, and to the concentrated reaction mixture, a saturated aqueous sodium bicarbonate solution was added, followed by extraction

three times with chloroform. The organic layers were combined, dried over sodium sulfate, and then concentrated under reduced pressure. The obtained crude product was purified by silica gel column chromatography (0 to 20% methanol/chloroform) to yield the title compound (410 mg, 79%) as a colorless amorphous form.

$^1$H-NMR (400 MHz, $CDCl_3$)δ (ppm): 0.02 - 0.11 (m, 2H), 0.38 - 0.52 (m, 2H), 0.68 - 0.91 (m, 1H), 1.05 - 3.80 (m, 15H), 2.09 (s, 1.5H), 2.13 (s, 1.5H), 3.79 (s, 3H), 3.86 - 3.99 (m, 1H), 4.22 (d, J = 6 Hz, 0.5H), 4.27 (d, J = 6 Hz, 0.5H), 4.62 (d, J = 12 Hz, 0.5H), 4.69 (d, J = 12 Hz, 0.5H), 4.71 (d, J = 12 Hz, 0.5H), 4.90 (d, J = 12 Hz, 0.5H), 6.69 - 6.76 (m, 2H), 6.97 - 7.07 (m, 1H).

(Example 152)

Synthesis of 2,2,2-trichloroethyl (5aS,6R,11bR)-14-acetyl-5a-hydroxy-10-methoxy-1,2,5,5a,6,7-hexahydro-6,11b-(epiminoethano)naphtho[1,2-d]azepine-3(4H)-carboxylate

[Chemical Formula 177]

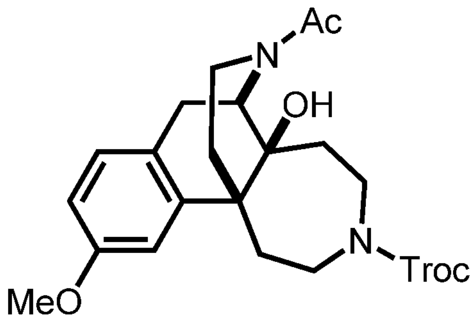

To a solution of the compound (410 mg, 0.73 mmol) obtained in Example 151 in toluene (7 mL) was added diisopropyl azodicarboxylate (40% toluene solution, about 1.9 mol/L, 768 pL, 1.46 mmol), followed by stirring at 100°C for 90 minutes. The reaction mixture was concentrated under reduced pressure, and to the concentrated reaction mixture, ethanol (14 mL) and pyridine hydrochloride (888 mg, 7.7 mmol) were added, followed by stirring at 40°C for 18 hours. To the reaction mixture, water and potassium carbonate were added to adjust the pH to 11, followed by extraction three times with chloroform. The organic layers were combined, dried over sodium sulfate, and then concentrated under reduced pressure. The obtained crude product was purified by silica gel column chromatography (50 to 100% ethyl acetate/heptane) to yield the title compound (346 mg, 93%) as a colorless amorphous form.

$^1$H-NMR (400 MHz, $CDCl_3$)δ (ppm): 1.04 - 1.30 (m, 1H), 1.52 - 4.84 (m, 19H), 3.79 (s, 1.5H), 3.80 (s, 1.5H), 6.67 - 6.77 (m, 2H), 6.96 - 7.15 (m, 1H).

(Example 153)

Synthesis of 1-((5aS,6R,11bS)-5a-hydroxy-10-methoxy-1,2,3,4,5,5a,6,7-octahydro-6,11b-(epiminoethano)naphtho[1,2-d]azepin-14-yl)ethan-1-one

[Chemical Formula 178]

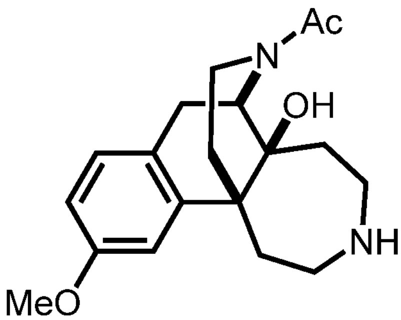

To a solution of the compound (346 mg, 0.68 mmol) obtained in Example 152 in acetic acid (30 mL) was added a zinc powder (895 mg, 13.7 mmol), followed by stirring at room temperature for 4 hours. The reaction mixture was filtered through celite to remove zinc, followed by concentration under reduced pressure. To the obtained crude product, a saturated sodium bicarbonate aqueous solution was added, followed by extraction three times with chloroform. The organic layers were combined, dried over sodium sulfate, and then concentrated under reduced pressure. The obtained crude product was purified by silica gel column chromatography (amino group-supported silica gel, 0 to 50% methanol/chloroform) to yield the title compound (187 mg, 83%) as a colorless amorphous form.

$^1$H-NMR (400 MHz, $CDCl_3$)$\delta$ (ppm): 1.04 - 1.14 (m, 1H), 1.50 - 2.47 (m, 6H), 2.11 (s, 1.5H), 2.20 (s, 1.5H), 2.53 - 3.00 (m, 4H), 3.02 - 3.14 (m, 1H), 3.24 - 3.36 (m, 1H), 3.42 - 3.50 (m, 0.5H), 3.80 (s, 3H), 3.89 (d, J = 7 Hz, 0.5H), 4.33 - 4.40 (m, 0.5H), 4.87 (d, J = 6 Hz, 0.5H), 6.76 (dd, J = 2, 8 Hz, 1H), 6.80 - 6.83 (m, 1H), 7.05 (d, J = 8 Hz, 0.5H), 7.06 (d, J = 8 Hz, 0.5H).

(Example 154)

Synthesis of 1-((5aS,6R,11bS)-5a-hydroxy-10-methoxy-3-(2-(pyridin-2-yl)ethyl)-1,2,3,4,5,5a,6,7-octahydro-6,11b-(epiminoethano)naphtho[1,2-d]azepin-14-yl)ethan-1-one

[Chemical Formula 179]

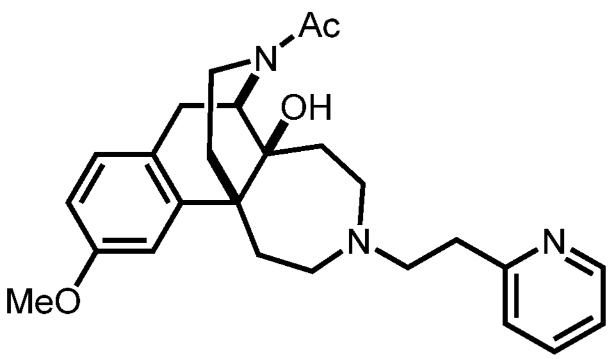

The title compound was obtained from the compound obtained in Example 153 according to the method described in Example 76 (Method 1).

$^1$H-NMR (400 MHz, $CDCl_3$)$\delta$ (ppm): 0.97 - 1.11 (m, 1H), 1.50 - 3.15 (m, 15H), 2.09 (s, 1.5H), 2.18 (s, 1.5H), 3.19 - 3.33 (m, 1H), 3.39 - 3.48 (m, 0.5H), 3.79 (s, 1.5H), 3.79 (s, 1.5H), 3.85 (d, J = 6 Hz, 0.5H), 4.31 - 4.38 (m, 0.5H), 4.87 (d, J = 6 Hz, 0.5H), 6.71 - 6.77 (m, 1H), 6.78 - 6.82 (m, 1H), 7.01 - 7.07 (m, 1H), 7.10 - 7.20 (m, 2H), 7.57 - 7.65 (m, 1H), 8.49 - 8.56 (m, 1H).

(Example 155)

Synthesis of (5aS,6R,11bR)-10-methoxy-3-(2-(pyridin-2-yl)ethyl)-2,3,4,5,6,7-hexahydro-6,11b-(epiminoethano)naphtho[1,2-d]azepin-5a(1H)-ol

[Chemical Formula 180]

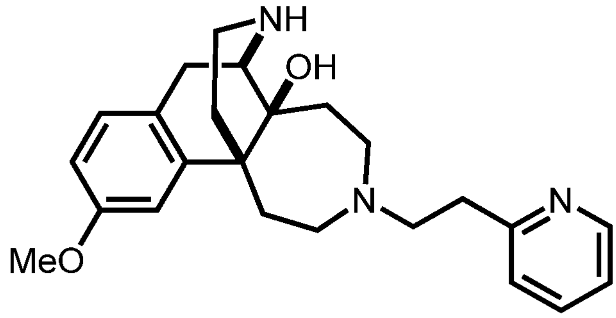

To a solution of the compound (87.0 mg, 0.20 mmol) obtained in Example 154 in ethanol (5 mL) was added 2 M aqueous sodium hydroxide solution (5 mL, 10 mmol), followed by heating under reflux for 40 hours. After allowed to cool, to the resultant solution was added water, followed by extraction three times with chloroform. The organic layers were combined, dried over sodium sulfate, and then concentrated under reduced pressure. The obtained crude product was purified by silica gel column chromatography (amino group-supported silica gel, 0 to 50% methanol/chloroform) to yield the title compound (69.3 mg, 88%) as a colorless amorphous form.

$^1$H-NMR (400 MHz, $CDCl_3$)δ (ppm): 0.92 - 1.00 (m, 1H), 1.48 (dd, J = 7, 14 Hz, 1H), 1.72 - 1.93 (m, 3H), 2.28 (dd, J = 4, 15 Hz, 1H), 2.40 - 2.71 (m, 5H), 2.82 - 3.00 (m, 7H), 3.19 (dd, J = 7, 18 Hz, 1H), 3.79 (s, 3H), 4.81 (br s, 1H), 6.72 (dd, J = 3, 8 Hz, 1H), 6.75 (d, J = 3 Hz, 1H), 7.06 (d, J = 8 Hz, 1H), 7.12 - 7.20 (m, 2H), 7.63 (ddd, J = 2, 7, 7 Hz, 1H), 8.54 - 8.57 (m, 1H).

(Example 156)

Synthesis of (5aS,6R,11bR)-3-(2-(pyridin-2-yl)ethyl)-2,3,4,5,6,7-hexahydro-6,11b-(epiminoethano)naphtho[1,2-d]azepine-5a, 10(1H)-diol (compound I) and (5aS,6R, 11bS)-14-methyl-3-(2-(pyridin-2-yl)ethyl)-2,3,4,5,6,7-hexahydro-6,11b-(epiminoethano)naphtho[1,2-d]azepine-5a,10(1H)-diol (compound J)

[Chemical Formula 181]

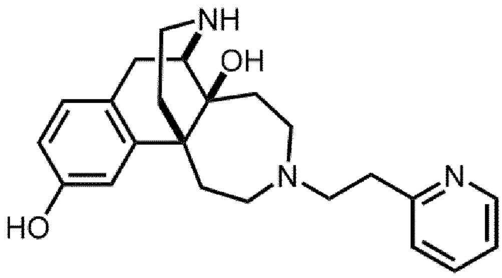

Compound I

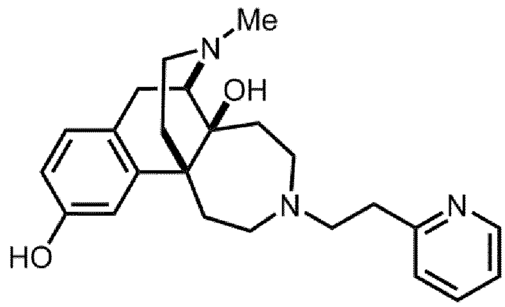

Compound J

The title compounds I and J were individually obtained from the compound obtained in Example 155 according to the method described in Example 6.

(Compound I)

$^1$H-NMR (400 MHz, $CDCl_3$)δ (ppm): 0.93 - 1.03 (m, 1H), 1.47 (dd, J = 7, 15 Hz, 1H), 1.60 - 1.71 (m, 1H), 1.77 - 1.92 (m, 2H), 2.19 (dd, J = 4, 15 Hz, 1H), 2.32 - 2.63 (m, 5H), 2.75 - 3.01 (m, 7H), 3.18 (dd, J = 7, 18 Hz, 1H), 6.64 (dd, J = 2, 8 Hz, 1H), 6.69 (d, J = 2 Hz, 1H), 6.96 (d, J = 8 Hz, 1H), 7.14 - 7.22 (m, 2H), 7.66 (ddd, J = 2, 8, 8 Hz, 1H), 8.53 - 8.58 (m, 1H).

(Compound J)

$^1$H-NMR (400 MHz, $CDCl_3$)δ (ppm): 0.96 - 1.08 (m, 1H), 1.41 - 1.54 (m, 1H), 1.76 - 2.09 (m, 4H), 2.16 - 2.38 (m, 2H), 2.33 (s, 3H), 2.61 (d, J = 6 Hz, 1H), 2.63 - 2.96 (m, 8H), 3.03 (d, J = 18 Hz, 1H), 3.11 - 3.23 (m, 1H), 4.58 (br s, 1H), 6.53 (d, J = 2 Hz, 1H), 6.59 (dd, J = 2, 8 Hz, 1H), 6.91 (d, J = 8 Hz, 1H), 7.07 - 7.14 (m, 2H), 7.56 (ddd, J = 2, 7, 7 Hz, 1H), 8.46 - 8.51 (m, 1H).

(Example 157)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-3-(1H-pyrazol-1-yl)propan-1-one

[Chemical Formula 182]

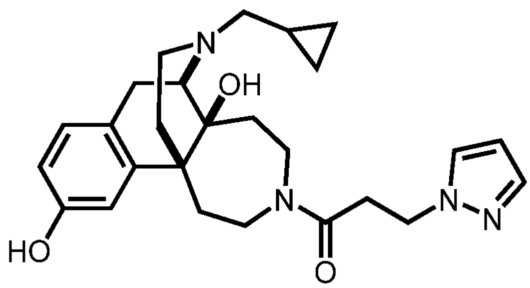

The title compound was obtained from the compound E obtained in Example 3 and 3-(1H-pyrazol-1-yl)propanoic acid hydrochloride according to the method described in Example 26.

$^1$H-NMR (400 MHz, $CDCl_3$)δ (ppm): 0.07 - 0.15 (m, 2H), 0.48 - 0.53 (m, 2H), 0.78 - 0.88 (m, 1H), 1.06 - 1.14 (m, 1H), 1.52 - 1.65 (m, 1.3H), 1.78 - 1.93 (m, 1.7H), 1.94 - 2.20 (m, 2.3H), 2.26 - 2.48 (m, 2.7H), 2.50 - 2.58 (m, 1H), 2.68 - 2.81 (m, 1.3H), 2.82 - 2.99 (m, 3.7H), 3.00 - 3.22 (m, 1.3H), 3.26 - 3.33 (m, 0.7H), 3.67 - 3.82 (m, 1H), 3.86 - 3.99 (m, 1.3H), 4.07 (br s, 0.3H), 4.20 - 4.29 (m, 0.7H), 4.40 - 4.48 (m, 1H), 4.53 (br s, 0.7H), 6.17 (s, 0.3H), 6.22 (s, 0.7H), 6.65 - 6.76 (m, 1.3H), 6.77 - 6.81 (m, 0.7H), 6.91 - 6.98 (m, 1H), 7.37 (s, 0.7H), 7.42 (s, 0.3H), 7.47 (s, 0.3H), 7.56 (s, 0.7H), 7.89 (br s, 0.7H).

(Example 158)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(thiazol-2-yl)ethan-1-one

[Chemical Formula 183]

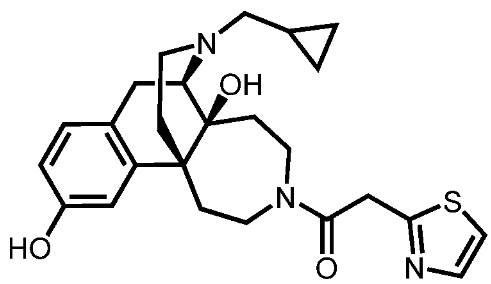

The title compound was obtained from the compound E obtained in Example 3 and 2-(thiazol-2-yl)acetic acid according to the method described in Example 5.

$^1$H-NMR (400 MHz, $CDCl_3$)δ (ppm): 0.08 - 0.12 (m, 2H), 0.49 - 0.53 (m, 2H), 0.77 - 0.88 (m, 1H), 1.05 - 1.10 (m, 1H), 1.23 - 1.28 (m, 1H), 1.60 - 1.64 (m, 1H), 1.79 - 2.15 (m, 4H), 2.24 - 2.39 (m, 2H), 2.52 - 2.64 (m, 1H), 2.69 -

2.80 (m, 1H), 2.88 - 3.00 (m, 2H), 3.25 - 3.50 (m, 2H), 3.82 - 4.15 (m, 4H), 6.60 (dd, J = 2, 8 Hz, 0.6H), 6.65 (dd, J = 2, 8 Hz, 0.4H), 6.69 (d, J = 2 Hz, 0.6H), 6.72 (d, J = 2 Hz, 0.4H), 6.88 - 6.92 (m, 1H), 7.25 - 7.28 (m, 1H), 7.67 - 7.69 (m, 1H).

(Example 159)

Synthesis of (5aS,6R,11bS)-14-propyl-3-(2-(pyridin-2-yl)ethyl)-2,3,4,5,6,7-hexahydro-6,11b-(epiminoethano)naphtho[1,2-d]azepine-5a,10(1H)-diol

[Chemical Formula 184]

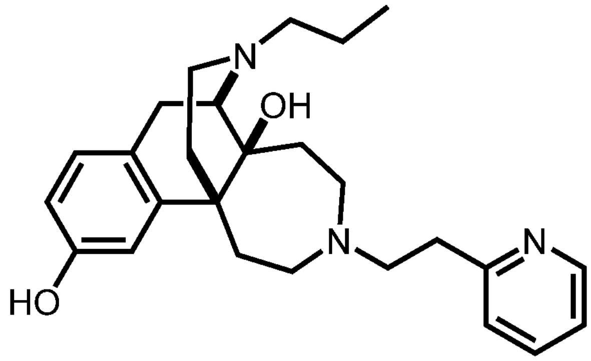

To a solution of the compound I (10 mg, 0.026 mmol) obtained in Example 156 in N,N-dimethylformamide (1 mL) were added 1-bromopropane (4.6 pL, 0.051 mmol) and N,N-diisopropylethylamine (13.2 pL, 0.076 mmol), followed by stirring at room temperature for 18 hours. To the reaction mixture, a saturated sodium bicarbonate aqueous solution was added, followed by extraction three times with chloroform. The organic layers were combined, dried over sodium sulfate, and then concentrated under reduced pressure. The obtained crude product was used for the next reaction without purification.

To a solution of the obtained crude product in chloroform (2 mL) was added a 1 M boron tribromide-dichloromethane solution (500 μL, 0.50 mmol) under ice cooling, followed by stirring at room temperature for 30 minutes. To the reaction mixture, a 28% aqueous ammonia solution was added under ice cooling, followed by extraction three times with chloroform. The organic layers were combined, dried over sodium sulfate, and then concentrated under reduced pressure. The obtained crude product was purified by preparative thin layer chromatography (chloroform : 2 M ammonia-methanol solution = 10 : 1) to yield the title compound (4.9 mg, 45%) as a colorless amorphous form.

$^1$H-NMR (400 MHz, $CDCl_3$)δ (ppm): 0.90 (t, J = 7 Hz, 3H), 0.98 - 1.05 (m, 1H), 1.41 - 1.52 (m, 3H), 1.79 - 2.05 (m, 4H), 2.27 - 2.44 (m, 4H), 2.66 (d, J = 6 Hz, 1H), 2.75 (dd, J = 6, 18 Hz, 1H), 2.80 - 3.02 (m, 8H), 3.32 (dd, J = 11, 11 Hz, 1H), 6.49 (d, J = 3 Hz, 1H), 6.58 (dd, J = 3, 8 Hz, 1H), 6.89 (d, J = 8 Hz, 1H), 7.07 - 7.14 (m, 2H), 7.55 (ddd, J = 2, 8, 8 Hz, 1H), 8.45 - 8.48 (m, 1H).

(Example 160)

Synthesis of 2-(4-cyclopropyl-1H-pyrazol-1-yl)-1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)ethan-1-one

[Chemical Formula 185]

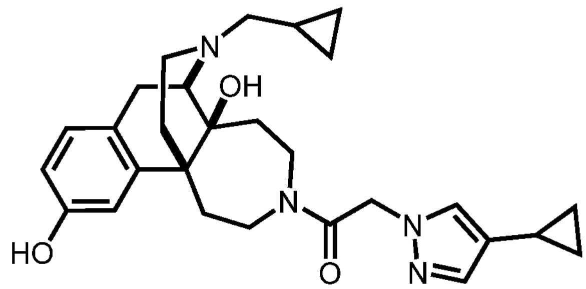

The title compound was obtained from the compound E obtained in Example 3 and 2-(4-cyclopropyl-1H-pyrazol-1-yl)acetic acid according to the method described in Example 26.

$^1$H-NMR (400 MHz, $CDCl_3$)δ (ppm): 0.05 - 0.20 (m, 2H), 0.45 - 0.60 (m, 4H), 0.75 - 0.95 (m, 3H), 1.00 - 1.15 (m, 1H), 1.50 - 2.40 (m, 8.4H), 2.50 - 2.65 (m, 1.6H), 2.65 - 2.85 (m, 1H), 2.85 - 3.05 (m, 2H), 3.05 - 3.40 (m, 2H), 3.60 - 3.75 (m, 0.6H), 3.75 - 3.90 (m, 1H), 4.00 - 4.10 (m, 0.4H), 4.55 (d, J = 16 Hz, 0.6H), 4.70 (d, J = 16 Hz, 0.6H), 4.85 (d, J = 16 Hz, 0.4H), 4.89 (d, J = 16 Hz, 0.4H), 6.60 - 6.70 (m, 2H), 6.85 - 7.00 (m, 1H), 7.10 (s, 0.6H), 7.16 (s, 0.4H), 7.26 (s, 1H).

(Example 161)

Synthesis of (5aS,6R,11bS)-14-(cyclobutylmethyl)-10-methoxy-3-(2-(pyridin-2-yl)ethyl)-2,3,4,5,6,7-hexahydro-6,11b-(epiminoethano)naphtho[1,2-d]azepin-5a(1H)-ol

[Chemical Formula 186]

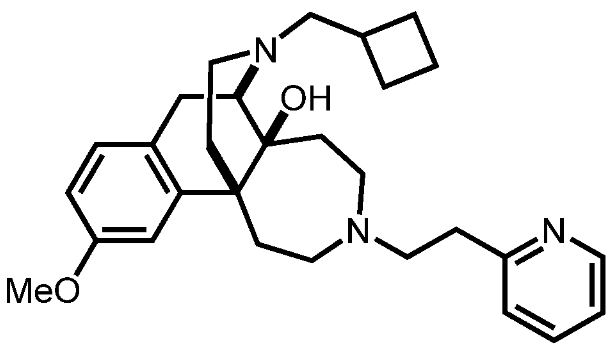

To a solution of the compound (10 mg, 0.025 mmol) obtained in Example 155 in N,N-dimethylformamide (1.0 mL) were added (bromomethyl)cyclobutane (4.6 pL, 0.042 mmol) and N,N-diisopropylethylamine (13.2 pL, 0.076 mmol), followed by stirring at room temperature for 18 hours and then stirring at 80°C for 6 hours. After allowed to cool, to the reaction mixture was added a saturated sodium bicarbonate aqueous solution, followed by extraction three times with chloroform. The organic layers were combined, dried over sodium sulfate, and then concentrated under reduced pressure. The obtained crude product was purified by preparative thin layer chromatography (chloroform : 2 M ammonia-methanol solution = 10 : 1) to yield the title compound (7.1 mg, 62%) as a colorless amorphous form.

$^1$H-NMR (400 MHz, $CDCl_3$)δ (ppm): 0.94 - 1.07 (m, 1H), 1.20 - 1.32 (m, 1H), 1.40 - 1.51 (m, 1H), 1.58 - 2.11 (m, 10H), 2.27 - 2.68 (m, 7H), 2.71 - 2.95 (m, 6H), 3.01 (d, J = 18 Hz, 1H), 3.14 - 3.26 (m, 1H), 3.76 (s, 3H), 4.63 (br s, 1H), 6.66 (d, J = 2 Hz, 1H), 6.69 (dd, J = 3, 8 Hz, 1H), 6.98 - 7.08 (m, 3H), 7.49 (ddd, J = 2, 8, 8 Hz, 1H), 8.44 - 8.47 (m, 1H).

(Example 162)

Synthesis of (5aS,6R, 11bS)-14-(cyclobutylmethyl)-3-(2-(pyridin-2-yl)ethyl)-2,3,4,5,6,7-hexahydro-6,11b-(epiminoethano)naphtho[1,2-d]azepine-5a,10(1H)-diol

[Chemical Formula 187]

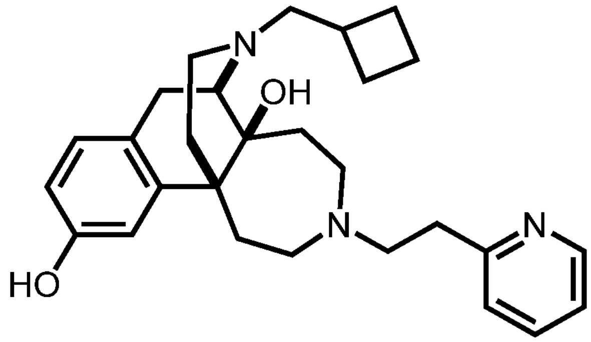

The title compound was obtained from the compound obtained in Example 161 according to the method described in Example 6.

$^1$H-NMR (400 MHz, $CDCl_3$)δ (ppm): 0.96 - 1.03 (m, 1H), 1.20 - 1.28 (m, 1H), 1.41 - 1.52 (m, 1H), 1.56 - 1.69 (m, 2H), 1.75 - 2.10 (m, 8H), 2.28 - 2.50 (m, 5H), 2.60 (d, J = 6 Hz, 1H), 2.73 (dd, J = 6, 18 Hz, 1H), 2.80 - 3.04 (m, 7H), 3.31 (dd, J = 12, 12 Hz, 1H), 6.47 (d, J = 2 Hz, 1H), 6.58 (dd, J = 2, 8 Hz, 1H), 6.88 (d, J = 8 Hz, 1H), 7.07 - 7.15 (m, 2H), 7.56 (ddd, J = 2, 8, 8 Hz, 1H), 8.44 - 8.47 (m, 1H).

(Example 163)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(4-methyl-3-(trifluoromethyl)-1H-pyrazol-1-yl)ethan-1-one

[Chemical Formula 188]

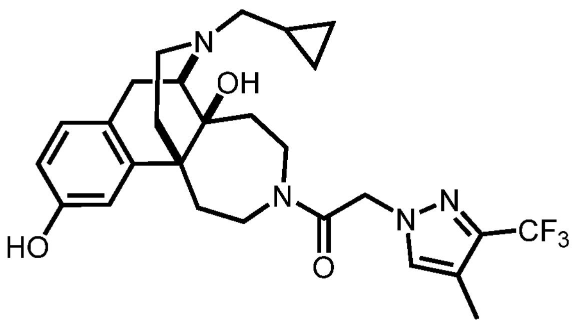

The title compound was obtained from the compound E obtained in Example 3 and 2-(4-methyl3-(trifluoromethyl)-1H-pyrazol-1-yl)acetic acid according to the method described in Example 26.

$^1$H-NMR (400 MHz, $CDCl_3$)δ (ppm): 0.08 - 0.13 (m, 2H), 0.49 - 0.54 (m, 2H), 0.79 - 0.84 (m, 1H), 1.07 - 1.10 (m, 1H), 1.23 - 1.28 (m, 1H), 1.56 - 2.07 (m, 5H), 2.11 (s, 1.5H), 2.14 (s, 1.5H), 2.20 - 2.39 (m, 2H), 2.53 - 2.67 (m, 1H), 2.75 (ddd, J = 6, 19, 19 Hz, 1H), 2.88 - 3.02 (m, 2H), 3.12 - 3.18 (m, 0.5H), 3.32 - 3.42 (m, 1.5H), 3.65 - 3.75 (m, 0.5H), 3.79 - 3.86 (m, 1H), 4.02 - 4.07 (m, 0.5H), 4.64 (d, J = 16 Hz, 0.5H), 4.79 (d, J = 16 Hz, 0.5H), 4.90 (d, J = 16 Hz, 0.5H), 4.95 (d, J = 16 Hz, 0.5H), 5.90 (br s, 0.5H), 6.62 - 6.67 (m, 2H), 6.95 (dd, J = 2, 8 Hz, 1H), 7.16 (s, 0.5H).

(Example 164)

Synthesis of (5aS,6R, 11bS)-14-(cyclopropylmethyl)-3-(2-(4-methyl-3-(trifluoromethyl)-1H-pyrazol-1-yl)ethyl)-2,3,4,5,6,7-hexahydro-6,11b-(epiminoethano)naphtho[1,2-d]azepine-5a,10(1H)-diol

[Chemical Formula 189]

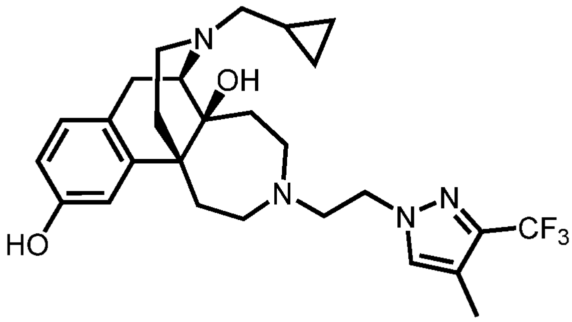

The title compound was obtained from the compound obtained in Example 163 according to the method described in Example 24.

$^1$H-NMR (400 MHz, $CDCl_3$)δ (ppm): 0.09 - 0.13 (m, 2H), 0.49 - 0.53 (m, 2H), 0.81 - 0.89 (m, 1H), 1.01 - 1.04 (m, 1H), 1.22 - 1.28 (m, 1H), 1.46 (dd, J = 4, 14 Hz, 1H), 1.72 - 1.85 (m, 2H), 2.00 - 2.05 (m, 2H), 2.05 (s, 3H), 2.37 - 3.03 (m, 11H), 3.20 (dd, J = 12, 12 Hz, 1H), 3.99 - 4.13 (m, 2H), 6.60 (s, 1H), 6.62 (d, J = 8 Hz, 1H), 6.83 (s, 1H), 6.97 (d, J = 8 Hz, 1H).

(Example 165)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(4-isopropyl-1H-1,2,3-triazol-1-yl)ethan-1-one

[Chemical Formula 190]

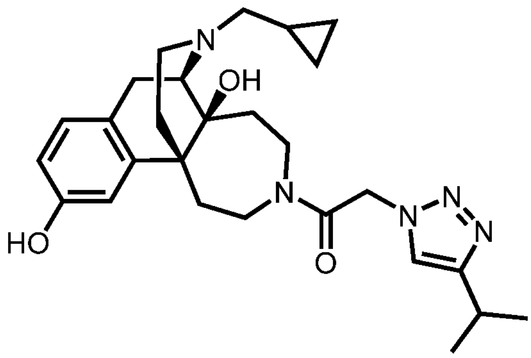

The title compound was obtained from the compound E obtained in Example 3 and 2-(4-isopropyl-1H-1,2,3-triazol-1-yl)acetic acid (synthesized by the method described in Journal of Medicinal Chemistry 2018, 61, 8797) according to the method described in Example 26.

$^1$H-NMR (400 MHz, $CDCl_3$)δ (ppm): 0.09 - 0.14 (m, 2H), 0.49 - 0.55 (m, 2H), 0.79 - 0.87 (m, 1H), 1.09 - 1.12 (m, 1H), 1.20 - 1.28 (m, 6H), 1.58 - 1.66 (m, 1H), 1.81 - 2.11 (m, 4H), 2.22 - 2.39 (m, 2.2H), 2.54 - 2.80 (m, 3H), 2.90 - 3.07 (m, 3.2H), 3.30 - 3.42 (m, 1.6H), 3.85 - 3.97 (m, 1.6H), 4.12 - 4.15 (m, 0.4H), 4.56 (br s, 1H), 4.67 (d, J = 16 Hz, 0.6H), 5.12 (d, J = 16 Hz, 0.4H), 5.25 (d, J = 16 Hz, 0.4H), 5.29 (d, J = 16 Hz, 0.6H), 6.65 - 6.69 (m, 1H), 6.78 (s, 0.6H), 6.91 - 6.95 (m, 1.4H), 7.26 (s, 0.6H), 7.39 (s, 0.4H).

(Reference Example 8)

Synthesis of 1-(2-((tert-butyldimethylsilyl)oxy)ethyl)-3-methyl- 1H-pyrrole

[Chemical Formula 191]

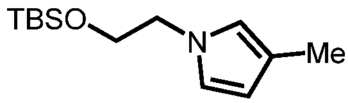

To 3-methyl-1H-pyrrole (97 mg, 1.20 mmol) dissolved in N,N-dimethylformamide (3 mL) were added sodium hydride (55% oil dispersion) (156.5 mg, 3.59 mmol) and (2-bromoethoxy)(tert-butyl)dimethylsilane (765.6 μL, 3.59 mmol) under ice cooling, followed by stirring at room temperature for 30 minutes. To the reaction solution, a saturated aqueous sodium bicarbonate solution was added, followed by extraction with ethyl acetate. The organic layer was washed twice with a saturated aqueous sodium bicarbonate solution, then dried over sodium sulfate, and concentrated under reduced pressure. The obtained crude product was purified by silica gel column chromatography (2 to 12% ethyl acetate/heptane) to yield the title compound (314 mg, quantitative) as a yellow liquid.
$^1$H-NMR (400 MHz, $CDCl_3$)δ (ppm): 0.90 (s, 9H), 0.93 (s, 3H), 1.59 (s, 3H), 2.10 (s, 3H), 3.78 - 3.85 (m, 2H), 3.89 - 3.95 (m, 2H), 5.94 - 5.98 (m, 1H), 6.43 - 6.47 (m, 1H), 6.56 - 6.59 (m, 1H).

(Reference Example 9)

Synthesis of 2-(3-methyl-1H-pyrrol-1-yl)ethan-1-ol

[Chemical Formula 192]

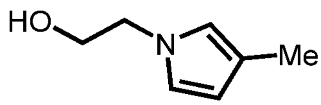

To the compound (304.9 mg, 1.27 mmol) obtained in Reference Example 8 dissolved in tetrahydrofuran (1 mL), a 1 M tetrabutylammonium fluoride-tetrahydrofuran solution (3.82 mL, 3.82 mmol) was added, followed by stirring at room temperature for 10 minutes. To the reaction solution, a saturated aqueous sodium bicarbonate solution was added, followed by extraction with ethyl acetate. The organic layer was washed once with a saturated aqueous sodium bicarbonate solution, twice with saturated saline, and once with water, then dried over sodium sulfate, and concentrated under reduced pressure. The obtained crude product was purified by silica gel column chromatography (34 to 54% ethyl acetate/heptane) to yield the title compound (69.5 mg, 44%) as a pale yellow oily matter.
$^1$H-NMR (400 MHz, $CDCl_3$)δ (ppm): 2.10 (s, 3H), 3.83 (t, J = 5 Hz, 2H), 3.95 (t, J = 5 Hz, 2H), 5.98 - 6.02 (m, 1H), 6.46 - 6.49 (m, 1H), 6.58 - 6.61 (m, 1H).

(Reference Example 10)

Synthesis of 2-(3-methyl-1H-pyrrol-1-yl)ethyl 4-methylbenzenesulfonate

[Chemical Formula 193]

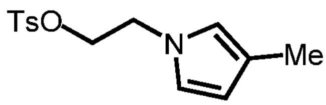

The title compound was obtained from the compound obtained in Reference Example 9 according to the method described in Reference Example 6.
$^1$H-NMR (400 MHz, $CDCl_3$)δ (ppm): 2.04 (s, 3H), 2.44 (s, 3H), 4.05 (t, J = 6 Hz, 2H), 4.20 (t, J = 6 Hz, 2H), 5.90 - 5.94 (m, 1H), 6.30 - 6.33 (m, 1H), 6.44 - 6.48 (m, 1H), 7.29 (d, J = 8 Hz, 2H), 7.66 (d, J = 8 Hz, 2H).

(Example 166)

Synthesis of (5aS,6R, 11bS)-14-(cyclopropylmethyl)-3-(2-(3-methyl-1H-pyrrol-1-yl)ethyl)-2,3,4,5,6,7-hexahydro-6,11b-(epiminoethano)naphtho[1,2-d]azepine-5a,10(1H)-diol

[Chemical Formula 194]

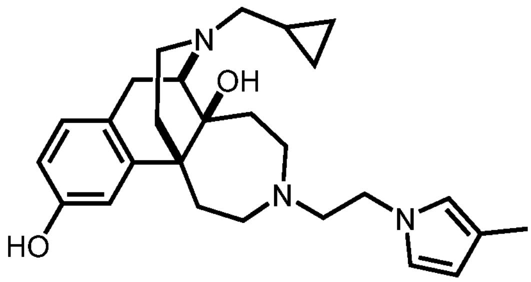

The title compound was obtained from the compound E obtained in Example 3 and the compound obtained in Reference Example 10 according to the method described in Example 31.

$^1$H-NMR (400 MHz, $CDCl_3$)δ (ppm): 0.06 - 0.16 (m, 2H), 0.46 - 0.54 (m, 2H), 0.76 - 0.90 (m, 1H), 0.98 - 1.07 (m, 1H), 1.42 - 1.51 (m, 1H), 1.74 - 1.84 (m, 2H), 1.95 - 2.06 (m, 2H), 2.05 (s, 3H), 2.28 - 2.39 (m, 3H), 2.49 - 2.57 (m, 1H), 2.62 - 2.98 (m, 8H), 3.13 - 3.22 (m, 1H), 3.85 (t, J = 7 Hz, 2H), 4.72 (br s, 1H), 5.89 (dd, J = 2, 2 Hz, 1H), 6.30 - 6.34 (m, 1H), 6.42 - 6.45 (m, 1H), 6.45 - 6.48 (m, 1H), 6.58 (dd, J = 2, 8 Hz, 1H), 6.92 (d, J = 8 Hz, 1H).

(Example 167)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2,2,2-trifluoroethan-1-one

[Chemical Formula 195]

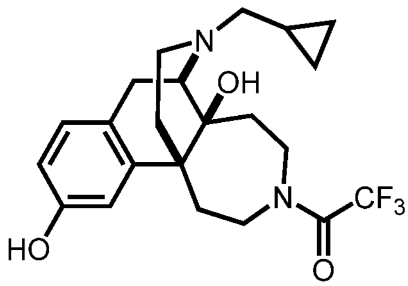

To a solution of the compound E (20 mg, 0.061 mmol) obtained in Example 3 in chloroform (1 mL) were added N,N-diisopropylethylamine (49 pL, 0.29 mmol) and trifluoroacetic anhydride (16 pL, 0.12 mmol) under ice cooling, followed by stirring at room temperature for 23 hours. To the reaction mixture were added potassium carbonate (60 mg, 0.43 mmol) and methanol (1 mL), followed by further stirring for 1 hour. Water was added to the reaction mixture under ice cooling, followed by extraction three times with ethyl acetate. The organic layer was dried over sodium sulfate and then concentrated under reduced pressure. The obtained crude product was purified by silica gel chromatography (1 to 10% methanol/chloroform) to yield the title compound (14 mg, 54%) as a pale yellow gumlike substance.

$^1$H-NMR (400 MHz, $CDCl_3$)δ (ppm): 0.03 - 0.19 (m, 2H), 0.43 - 0.60 (m, 2H), 0.75 - 0.95 (m, 1H), 1.01 - 1.78 (m, 3H), 1.79 - 2.24 (m, 4H), 2.27 - 2.47 (m, 3H), 2.48 - 2.66 (m, 1H), 2.70 - 2, 85 (m, 1H), 2.86 - 3.06 (m, 2H), 3.27 - 3.36 (m, 0.5H), 3.42 - 3.69 (m, 1.5H), 3.69 - 3.89 (m, 1H), 3.91 - 4.09 (m, 1H), 6.58 (d, J = 2 Hz, 0.5H), 6.60 - 6.70 (m, 1.5H), 6.91 - 7.02 (m, 1H).

(Reference Example 11)

Synthesis of 3-(chloromethyl)-5-methylisothiazole

[Chemical Formula 196]

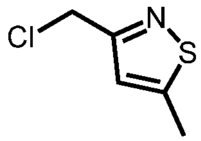

To a solution of (5-methylisothiazol-3-yl) methanol (103 mg, 0.797 mmol) in toluene (3 mL) was added thionyl chloride (0.12 mL, 1.7 mmol), followed by stirring at 80°C for 5 hours. After allowed to cool to room temperature, to the reaction mixture was added a saturated aqueous sodium bicarbonate solution, followed by extraction three times with ethyl acetate. The combined extracts were washed with saturated saline, dried over sodium sulfate, and then concentrated under reduced pressure. The obtained crude product was purified by silica gel column chromatography (4 to 25% ethyl acetate/heptane) to yield the title compound (69.7 mg, 59%) as a pale brown oily matter.
$^1$H-NMR (400 MHz, $CDCl_3$)$\delta$ (ppm): 2.58 (s, 3H), 4.62 (s, 2H), 7.05 (s, 1H).

(Reference Example 12)

Synthesis of 2-(5-methylisothiazol-3-yl)acetonitrile

[Chemical Formula 197]

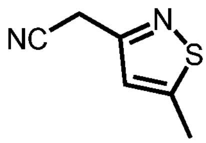

To a solution of the compound (69.7 mg, 0.472 mmol) obtained in Reference Example 11 in dimethyl sulfoxide (1.5 mL) was added sodium cyanide (69.4 mg, 1.42 mmol), followed by stirring at room temperature for 1.5 hours and at 50°C for 17 hours. After allowed to cool to room temperature, to the reaction mixture was added water, followed by extraction three times with tert-butyl methyl ether. The combined extracts were washed with saturated saline, dried over sodium sulfate, and then concentrated under reduced pressure. The obtained crude product was purified by silica gel column chromatography (19 to 40% ethyl acetate/heptane) to yield the title compound (42.9 mg, 66%) as a colorless oily matter.
$^1$H-NMR (400 MHz, $CDCl_3$)$\delta$ (ppm): 2.59 (s, 3H), 3.86 (s, 2H), 7.01 (s, 1H).

(Reference Example 13)

Synthesis of 2-(5-methylisothiazol-3-yl)acetic acid

[Chemical Formula 198]

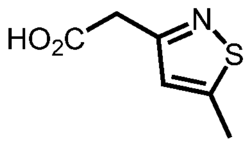

(YM1911191crude)

To a solution of the compound (42.9 mg, 0.310 mmol) obtained in Reference Example 12 in acetic acid (1 mL) was added 6 M hydrochloric acid (0.5 mL), followed by stirring at 90°C for 4.5 hours. After allowed to cool to room temperature, the reaction solution was concentrated under reduced pressure. Water was added to the obtained residue, followed by extraction three times with ethyl acetate. The combined extracts were washed with saturated saline, dried over sodium sulfate, and then concentrated under reduced pressure to yield the title compound (42.9 mg, 88%) as a pale brown crystal.

$^1$H-NMR (400 MHz, $CDCl_3$)δ (ppm): 2.59 (s, 3H), 3.88 (s, 2H), 6.91 (s, 1H).

(Example 168)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-2-(5-methylisothiazol-3-yl)ethan-1-one

[Chemical Formula 199]

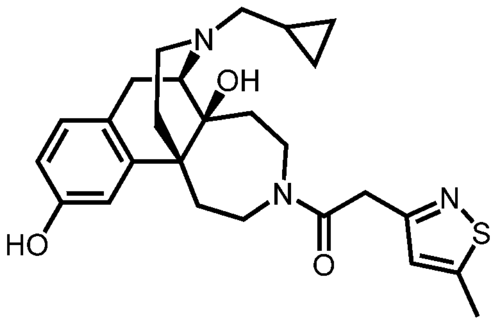

The title compound was obtained from the compound E obtained in Example 3 and the compound obtained in Reference Example 13 according to the method described in Example 26.

$^1$H-NMR (400 MHz, $CDCl_3$)δ (ppm): 0.08 - 0.12 (m, 2H), 0.48 - 0.53 (m, 2H), 0.78 - 0.85 (m, 1H), 1.02 - 1.10 (m, 1H), 1.53 - 1.62 (m, 1H), 1.72 - 2.15 (m, 4H), 2.26 - 2.38 (m, 2.6H), 2.47 - 2.54 (m, 4.4H), 2.67 (dd, J = 6, 18 Hz, 0.6H), 2.77 (dd, J = 6, 18 Hz, 0.4H), 2.85 - 2.98 (m, 2H), 3.33 - 3.41 (m, 1.4H), 3.45 - 3.49 (m, 1.2H), 3.57 (d, J = 15 Hz, 0.4H), 3.72 (d, J = 15 Hz, 0.4H), 3.73 - 3.78 (m, 0.4H), 3.78 (d, J = 15 Hz, 0.6H), 3.85 - 3.88 (m, 1H), 3.89 (d, J = 15 Hz, 0.6H), 3.99 - 4.02 (m, 0.4H), 4.46 - 4.52 (m, 0.6H), 6.61 - 6.68 (m, 1.5H), 6.79 - 6.83 (m, 1.5H), 6.89 - 6.92 (m, 1H).

(Example 169)

Synthesis of 1-((5aS,6R,11bR)-14-(cyclopropylmethyl)-5a, 10-dihydroxy-1,2,5,5a,6,7-hexahydro-6,11b-(epiminoethano)naphtho[1,2-d]azepin-3(4H)-yl)-3-(4-(trifluoromethyl)-1H-pyrazol-1-yl)propan-1-one

[Chemical Formula 200]

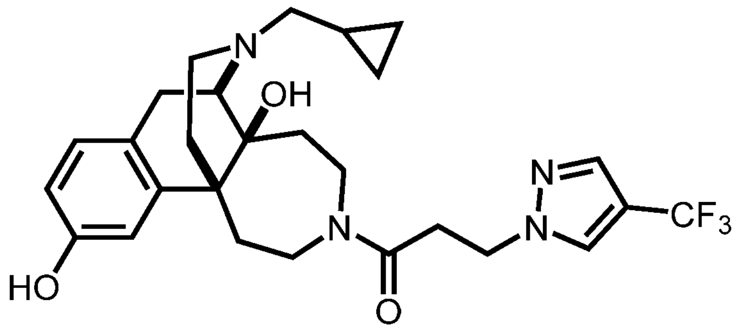

The title compound was obtained from the compound E obtained in Example 3 and 3-(4-(trifluoromethyl)-1H-pyrazol-1-yl)propanoic acid according to the method described in Example 85.

$^1$H-NMR (400 MHz, $CDCl_3$)δ (ppm): 0.05 - 0.15 (m, 2H), 0.48 - 0.56 (m, 2H), 0.76 - 0.89 (m, 1H), 1.04 - 1.15 (m, 1H), 1.55 - 1.63 (m, 1H), 1.78 (ddd, J = 3, 12, 14 Hz, 0.5H), 1.87 - 2.25 (m, 3.5H), 2.29 (dd, J = 6, 13 Hz, 0.5H), 2.32 - 2.39 (m, 1.5H), 2.51 - 3.09 (m, 7H), 3.24 (ddd, J = 4, 4, 13 Hz, 0.5H), 3.26 - 3.34 (m, 0.5H), 3.36 (ddd, J = 2, 6, 14 Hz, 0.5H), 3.63 - 3.90 (m, 1.5H), 4.03 - 4.18 (m, 1.5H), 4.32 (ddd, J = 7, 7, 14 Hz, 0.5H), 4.47 (t, J = 7 Hz, 1H), 6.61 (dd, J = 2, 8 Hz, 0.5H), 6.64 - 6.69 (m, 1H), 6.75 - 6.80 (m, 0.5H), 6.91 (d, J = 8 Hz, 0.5H), 6.96 (d, J = 8 Hz, 0.5H), 7.66 (s, 0.5H), 7.69 (s, 0.5H), 7.73 (s, 0.5H), 7.81 (s, 0.5H).

(Example 170)

Synthesis of 2,2,2-trichloroethyl (5aS,6R,11bR)-5a-(benzoyloxy)-14-(cyclopropylmethyl)-10-methoxy-1,2,5,5a,6,7-hexahydro-6,11b-(epiminoethano)naphtho[1,2-d]azepine-3(4H)-carboxylate

[Chemical Formula 201]

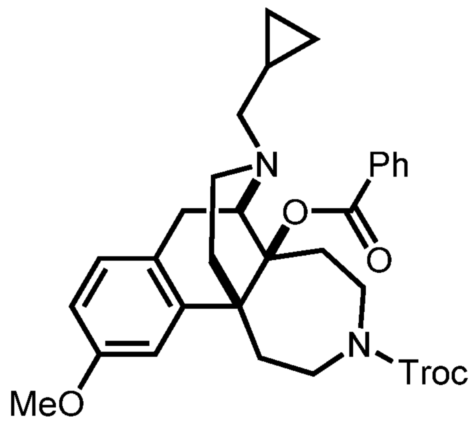

To a solution of the compound (270 mg, 0.52 mmol) obtained in Example 150 in xylene (10 mL) were added benzoic anhydride (236 mg, 1.04 mmol), 4-dimethylaminopyridine (127 mg, 1.04 mmol), and N,N-diisopropylethylamine (182 μL, 1.04 mmol), followed by stirring at 140°C for 18 hours. Thereafter, xylene (10 mL), benzoic anhydride (236 mg, 1.04 mmol), 4-dimethylaminopyridine (127 mg, 1.04 mmol), and N,N-diisopropylethylamine (182 μL, 1.04 mmol) were added, followed by stirring at 140°C for 42 hours. After allowed to cool, a saturated sodium bicarbonate aqueous solution was added, followed by extraction three times with chloroform. The combined extracts were dried over sodium sulfate and then concentrated under reduced pressure. The obtained crude product was purified by silica gel column chromatography (25 to 100% ethyl acetate/heptane) to yield the title compound (213 mg, 66%) as a white amorphous form.

$^1$H-NMR (400 MHz, $CDCl_3$)δ (ppm): - 0.18 - 0.00 (m, 2H), 0.29 - 0.43 (m, 2H), 0.58 - 0.70 (m, 1H), 0.83 - 2.84 (m, 11H), 3.02 - 3.35 (m, 3H), 3.63 - 3.78 (m, 1H), 3.81 (s, 1.2H), 3.81 (s, 1.8H), 3.88 - 4.03 (m, 1.6H), 4.40 - 4.51 (m, 1.4H), 4.53 (d, J = 12 Hz, 0.6H), 4.63 (d, J = 12 Hz, 0.4H), 6.72 - 6.79 (m, 2H), 7.05 - 7.11 (m, 1H), 7.28 - 7.60 (m, 3H),

8.01 - 8.08 (m, 2H).

(Example 171)

Synthesis of 2,2,2-trichloroethyl (5aS,6R,11bR)-14-benzoyl-5a-hydroxy-10-methoxy-1,2,5,5a,6,7-hexahydro-6,11b-(epiminoethano)naphtho[1,2-d]azepine-3(4H)-carboxylate

[Chemical Formula 202]

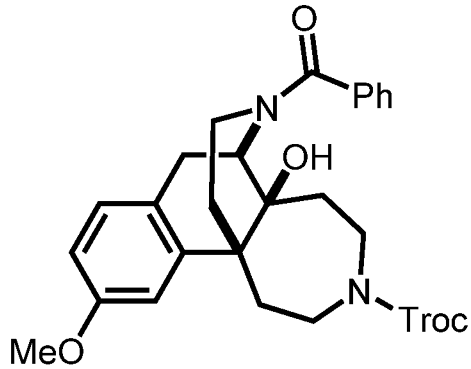

The title compound was obtained from the compound obtained in Example 170 according to the method described in Example 152.

$^1$H-NMR (400 MHz, $CDCl_3$)δ (ppm): 0.88 - 1.39 (m, 1H), 1.60 - 3.02 (m, 9H), 3.30 - 3.90 (m, 5H), 3.79 (s, 1.2H), 3.80 (s, 1.8H), 4.37 - 4.96 (m, 3H), 6.67 - 6.78 (m, 2H), 6.99 - 7.12 (m, 1H), 7.34 - 7.51 (m, 5H).

(Example 172)

Synthesis of ((5aS,6R,11bS)-5a-hydroxy-10-methoxy-1,2,3,4,5,5a,6,7-octahydro-6,11b-(epiminoethano)naphtho[1,2-d]azepin-14-yl)(phenyl)methanone

[Chemical Formula 203]

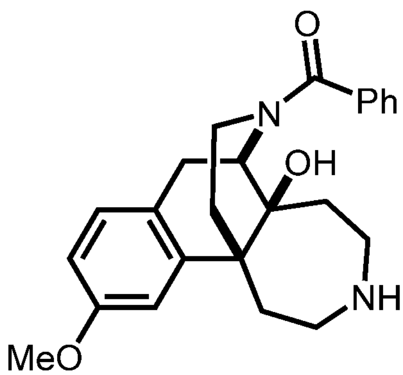

The title compound was obtained from the compound obtained in Example 171 according to the method described in Example 153.

$^1$H-NMR (400 MHz, $CDCl_3$)δ (ppm): 0.80 - 1.50 (m, 1H), 1.55 - 3.28 (m, 11H), 3.30 - 3.42 (m, 1H), 3.73 - 4.00 (m, 1H), 3.78 (s, 3H), 4.38 - 4.49 (m, 0.5H), 4.92 - 5.00 (m, 0.5H), 6.70 - 6.86 (m, 2H), 7.04 (d, J = 8 Hz, 0.5H), 7.11 (d, J = 8 Hz, 0.5H), 7.32 - 7.61 (m, 5H).

(Example 173)

Synthesis of 1-((5aS,6R,11bS)-14-benzoyl-5a-hydroxy-10-methoxy-1,2,5,5a,6,7-hexahydro-6,11b-(epiminoethano)naphtho[1,2-d]azepin-3(4H)-yl)-2-(4-methyl-1H-pyrazol-1-yl)ethan-1-one

[Chemical Formula 204]

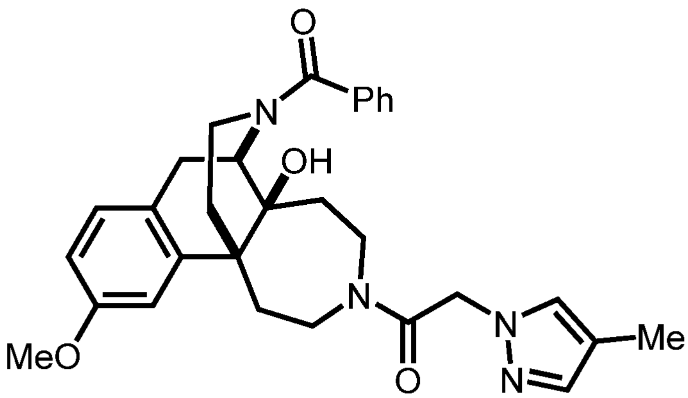

The title compound was obtained from the compound obtained in Example 172 and 2-(4-methyl-1H-pyrazol-1-yl)acetic acid according to the method described in Example 5.

$^1$H-NMR (400 MHz, $CDCl_3$)δ (ppm): 0.80 - 4.60 (m, 15H), 2.04 (s, 1.5H), 2.07 (s, 1.5H), 3.79 (s, 1.5H), 3.81 (s, 1.5H), 4.76 - 4.96 (m, 2H), 6.66 - 6.74 (m, 1H), 6.76 - 6.83 (m, 1H), 6.98 - 7.20 (m, 2H), 7.26 - 7.33 (m, 1H), 7.34 - 7.50 (m, 5H).

(Example 174)

Synthesis of (5aS,6R, 11bS)-14-benzyl-10-methoxy-3-(2-(4-methyl-1H-pyrazol-1-yl)ethyl)-2,3,4,5,6,7-hexahydro-6,11b-(epiminoethano)naphtho[1,2-d]azepin-5a(1H)-ol

[Chemical Formula 205]

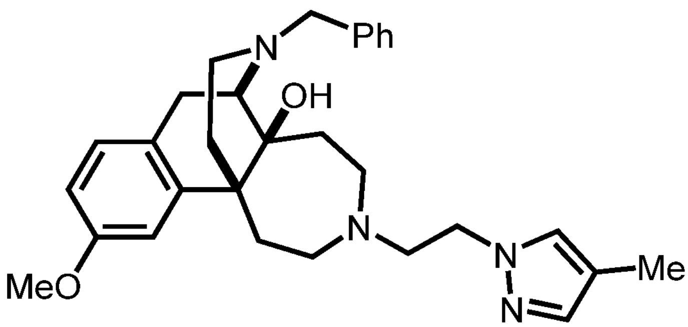

The title compound was obtained from the compound obtained in Example 173 according to the method described in Example 24.

$^1$H-NMR (400 MHz, $CDCl_3$)δ (ppm): 0.96 - 1.06 (m, 1H), 1.34 - 1.43 (m, 1H), 1.73 (ddd, J = 3, 11, 11 Hz, 1H), 1.84 (ddd, J = 4, 4, 16 Hz, 1H), 1.95 - 2.10 (m, 2H), 1.99 (s, 3H), 2.31 - 2.48 (m, 3H), 2.64 (ddd, J = 4, 4, 13 Hz, 1H), 2.73 (d, J = 6 Hz, 1H), 2.76 - 2.88 (m, 3H), 2.94 - 3.04 (m, 1H), 3.09 - 3.20 (m, 2H), 3.59 (d, J = 13 Hz, 1H), 3.64 (d, J = 13 Hz, 1H), 3.77 (s, 3H), 3.86 - 4.05 (m, 2H), 4.51 (br s, 1H), 6.69 (d, J = 2 Hz, 1H), 6.70 - 6.76 (m, 2H), 7.09 (d, J = 8 Hz, 1H), 7.17 - 7.35 (m, 6H).

(Example 175)

Synthesis of (5aS,6R,11bS)-14-benzyl-3-(2-(4-methyl-1H-pyrazol-1-yl)ethyl)-2,3,4,5,6,7-hexahydro-6,11b-(epiminoethano)naphtho[1,2-d]azepine-5a, 10(1H)-diol

[Chemical Formula 206]

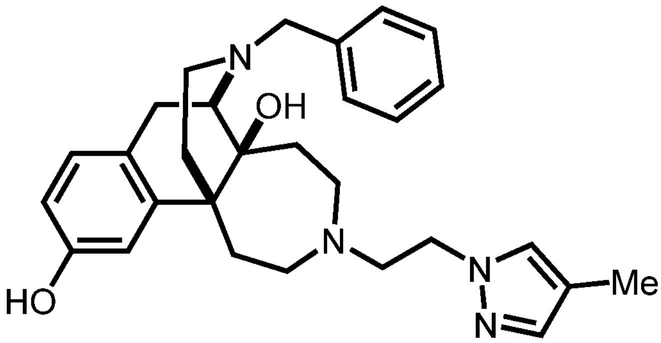

The title compound was obtained from the compound obtained in Example 174 according to the method described in Example 6.

$^1$H-NMR (400 MHz, $CDCl_3$)δ (ppm): 0.95 - 1.05 (m, 1H), 1.33 - 1.44 (m, 1H), 1.68 - 1.82 (m, 2H), 1.91 - 2.13 (m, 2H), 1.99 (s, 3H), 2.28 - 2.42 (m, 2H), 2.48 - 2.68 (m, 2H), 2.70 (d, J = 6 Hz, 1H), 2.79 (dd, J = 6, 18 Hz, 1H), 2.83 - 2.99 (m, 3H), 3.05 - 3.23 (m, 2H), 3.58 (d, J = 13 Hz, 1H), 3.64 (d, J = 13 Hz, 1H), 3.99 - 4.11 (m, 2H), 6.53 (d, J = 2 Hz, 1H), 6.63 (dd, J = 2, 8 Hz, 1H), 6.90 (s, 1H), 6.97 (d, J = 8 Hz, 1H), 7.20 - 7.38 (m, 6H).

(Example 176)

Synthesis of (5aS,6R, 11bR)-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 207]

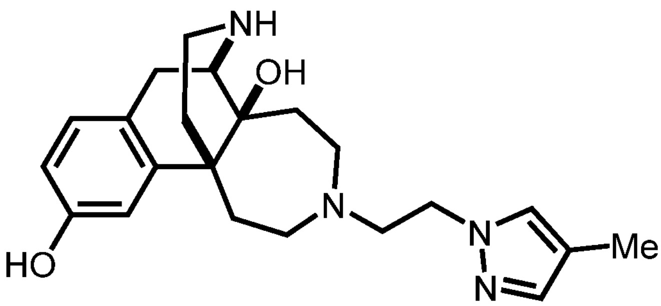

To the compound (4.7 mg, 0.010 mmol) obtained in Example 175 were added 10% palladium-activated carbon (4.7 mg) and methanol (500 μL), followed by stirring under a hydrogen atmosphere at room temperature for 18 hours. The reaction mixture was filtered through celite to remove palladium, followed by concentration under reduced pressure. The obtained crude product was purified by preparative thin layer chromatography (chloroform : 2 M ammonia-methanol solution = 5 : 1) to yield the title compound (2.2 mg, 58%) as a colorless amorphous form.

$^1$H-NMR (400 MHz, $CDCl_3$)δ (ppm): 0.92 - 1.00 (m, 1H), 1.47 (dd, J = 7, 15 Hz, 1H), 1.63 - 2.05 (m, 3H), 2.09 (s, 3H), 2.16 (dd, J = 5, 15 Hz, 1H), 2.31 - 2.64 (m, 5H), 2.82 - 2.98 (m, 5H), 3.17 (dd, J = 7, 18 Hz, 1H), 4.08 - 4.15 (m, 2H), 6.62 - 6.68 (m, 2H), 6.98 (d, J = 8 Hz, 1H), 7.15 (s, 1H), 7.35 (s, 1H).

(Example 177)

Synthesis of (5aS,6R, 11bS)-14-(cyclopropylmethyl)-3-(2-(5-methylisothiazol-3-yl)ethyl)-2,3,4,5,6,7-hexahydro-6,11b-(epiminoethano)naphtho[1,2-d]azepine-5a,10(1H)-diol

[Chemical Formula 208]

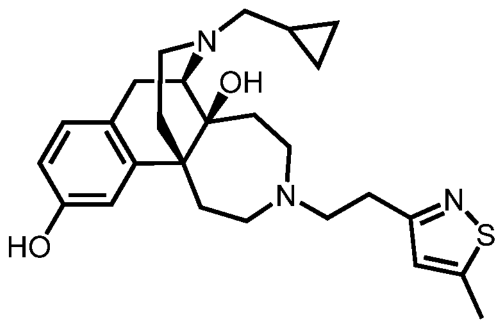

The title compound was obtained from the compound obtained in Example 168 according to the method described in Example 24.

$^1$H-NMR (400 MHz, $CDCl_3$)δ (ppm): 0.07 - 0.13 (m, 2H), 0.47 - 0.52 (m, 2H), 0.79 - 0.86 (m, 1H), 1.00 - 1.07 (m, 1H), 1.47 - 1.51 (m, 1H), 1.78 - 1.85 (m, 2H), 1.96 - 2.07 (m, 2H), 2.30 - 2.35 (m, 3H), 2.50 (s, 3H), 2.50 - 2.54 (m, 1H)2.71 - 2.80 (m, 3H), 2.87 - 2.96 (m, 7H), 3.15 - 3.23 (m, 1H), 4.74 (br s, 1H), 6.44 - 6.49 (m, 1H), 6.57 (dd, J = 2, 8 Hz, 1H), 6.71 (s, 1H), 6.89 (d, J = 8 Hz, 1H).

(Example 178)

Synthesis of (5aS,6R,11bS)-14-(cyclopropylmethyl)-3-(2,2,2-trifluoroethyl)-2,3,4,5,6,7-hexahydro-6,11b-(epiminoethano)naphtho[1,2-d]azepine-5a,10(1H)-diol

[Chemical Formula 209]

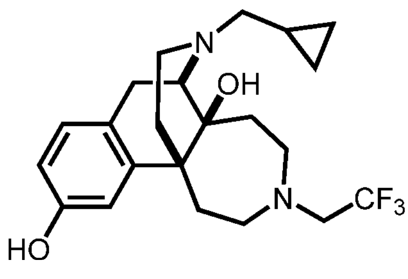

The title compound was obtained from the compound obtained in Example 167 according to the method described in Example 24.

$^1$H-NMR (400 MHz, $CDCl_3$)δ (ppm): 0.04 - 0.18 (m, 2H), 0.42 - 0.58 (m, 2H), 0.75 - 0.91 (m, 1H), 0.98 - 1.13 (m, 1H), 1.19 - 1.90 (m, 3H), 1.92 - 2.11 (m, 2H), 2.25 - 2.42 (m, 3H), 2.48 - 2.63 (m, 1H), 2.72 - 2.85 (m, 2H), 2.86 - 3.18 (m, 6H), 3.23 - 3.31 (m, 1H), 4.64 (br s, 1H), 6.54 - 6.66 (m, 2H), 6.96 (d, J = 8 Hz, 1H).

(Example 179)

Synthesis of (5aS,6R,11bS)-14-(cyclopropylmethyl)-3-(3-(4-(trifluoromethyl)-1H-pyrazol-1-yl)propyl)-2,3,4,5,6,7-hexahydro-6,11b-(epiminoethano)naphtho[1,2-d]azepme-5a,10(1H)-diol

[Chemical Formula 210]

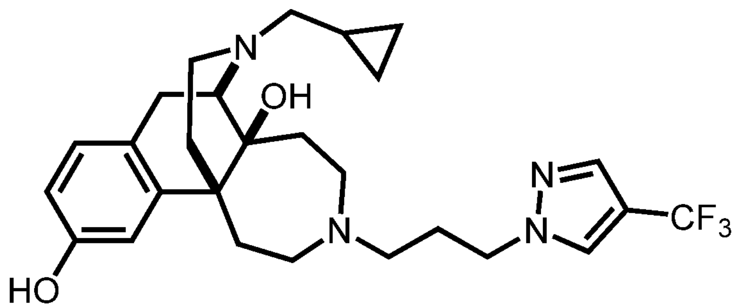

The title compound was obtained from the compound obtained in Example 169 according to the method described in Example 24.

$^1$H-NMR (400 MHz, $CDCl_3$)δ (ppm): 0.06 - 0.16 (m, 2H), 0.46 - 0.56 (m, 2H), 0.77 - 0.89 (m, 1H), 1.02 - 1.08 (m, 1H), 1.46 - 1.53 (m, 1H), 1.75 - 1.89 (m, 4H), 1.96 - 2.10 (m, 2H), 2.16 - 2.43 (m, 6H), 2.49 - 2.60 (m, 2H), 2.84 (dd, J = 6, 18 Hz, 1H), 2.91 - 3.04 (m, 4H), 3.76 (ddd, J = 7, 7, 14 Hz, 1H), 3.82 (ddd, J = 7, 7, 14 Hz, 1H), 4.65 (br s, 1H), 6.58 (dd, J = 2, 8 Hz, 1H), 6.72 (d, J = 2 Hz, 1H), 6.97 (d, J = 8 Hz, 1H), 7.19 (s, 1H), 7.64 (s, 1H).

(Example 180)

Synthesis of 2-chloro-1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)ethan-1-one

[Chemical Formula 211]

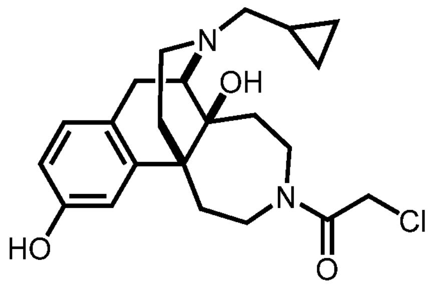

To a solution of the compound E (50 mg, 0.15 mmol) obtained in Example 3 in chloroform (1 mL) were added N,N-diisopropylethylamine (77 pL, 0.45 mmol) and chloroacetyl chloride (18 pL, 0.23 mmol) under ice cooling, followed by stirring at room temperature for 15 hours. Thereafter, potassium carbonate (207 mg, 1.5 mmol) and methanol (1 mL) were added, followed by stirring at room temperature for 1 hour. Water was added to the reaction mixture under ice cooling, followed by extraction three times with ethyl acetate. The organic layer was dried over sodium sulfate and then concentrated under reduced pressure. The obtained crude product was purified by silica gel column chromatography (amino group-supported silica gel, 1 to 100% methanol/chloroform) to yield the title compound (30 mg, 50%) as a pale yellow amorphous form.

$^1$H-NMR (400 MHz, $CDCl_3$)δ (ppm): 0.03 - 0.19 (m, 2H), 0.42 - 0.61 (m, 2H), 0.75 - 0.92 (m, 1H), 1.02 - 1.18 (m, 1H), 1.21 - 2.43 (m, 8H), 2.46 - 3.12 (m, 5H), 3.33 - 4.01 (m, 3H), 4.06 - 4.32 (m, 2H), 6.51 - 6.78 (m, 1.4H), 6.79 - 7.04 (m, 1.6H).

(Example 181)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(3,3-difluoropyrrolidin-1-yl)ethan-1-one

[Chemical Formula 212]

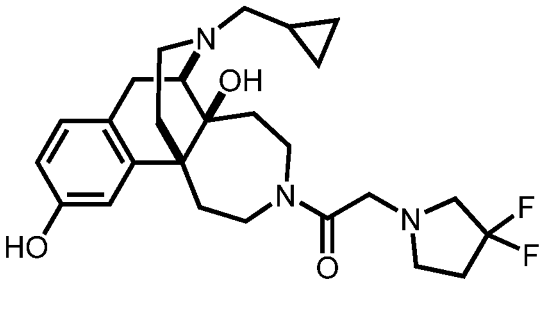

To a solution of the compound (15.3 mg, 0.038 mmol) obtained in Example 180 in acetonitrile (1 mL) were added N,N-diisopropylethylamine (65 pL, 0.38 mmol) and 3,3-difluoropyrrolidine (16 mg, 0.11 mmol), followed by stirring at room temperature for 42 hours. Water was added to the reaction mixture, followed by extraction three times with ethyl acetate. The combined extracts were dried over sodium sulfate and then concentrated under reduced pressure. The obtained crude product was purified by silica gel column chromatography (amino group-supported silica gel, 0 to 5% methanol/chloroform) to yield the title compound (16.6 mg, 92%) as a pale yellow gumlike substance.

$^1$H-NMR (400 MHz, $CDCl_3$)δ (ppm): 0.03 - 0.19 (m, 2H), 0.41 - 0.59 (m, 2H), 0.74 - 0.92 (m, 1H), 0.99 - 1.18 (m, 1H), 1.19 - 2.43 (m, 10H), 2.46 - 3.12 (m, 8.3H), 3.17 - 3.28 (m, 1H), 3.30 - 3.54 (m, 2.7H), 3.60 - 3.72 (m, 0.3H), 3.73 - 3.88 (m, 1H), 3.90 - 4.05 (m, 0.7H), 6.51 - 6.73 (m, 1.3H), 6.79 - 7.02 (m, 1.7H).

(Example 182)

Synthesis of (5aS,6R,11bS)-14-(cyclopropylmethyl)-3-(2-(3,3-difluoropyrrolidin-1-yl)ethyl)-2,3,4,5,6,7-hexahydro-6,11b-(epiminoethano)naphtho[1,2-d]azepine-5a,10(1H)-diol

[Chemical Formula 213]

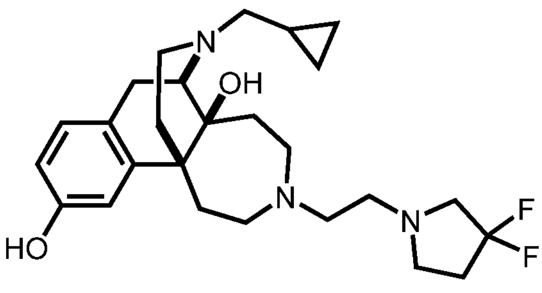

The title compound was obtained from the compound obtained in Example 181 according to the method described in Example 24.

$^1$H-NMR (400 MHz, $CDCl_3$)δ (ppm): 0.03 - 0.18 (m, 2H), 0.43 - 0.57 (m, 2H), 0.76 - 0.92 (m, 1H), 0.96 - 1.08 (m, 1H), 1.17 - 1.91 (m, 3H), 1.94 - 2.09 (m, 2H), 2.11 - 2.26 (m, 2H), 2.27 - 2.41 (m, 3H), 2.46 - 3.00 (m, 15H), 3.01 - 3.16 (m, 1H), 4.73 (br s, 1H), 6.48 (d, J = 2 Hz, 1H), 6.56 (dd, J = 2, 8 Hz, 1H), 6.90 (d, J = 8 Hz, 1H).

(Example 183)

Synthesis of (5aS,6R,11bS)-3-(2-(4-cyclopropyl-1H-pyrazol-1-yl)ethyl)-14-(cyclopropylmethyl)-2,3,4,5,6,7-hexahydro-6,11b-(epiminoethano)naphtho[1,2-d]azepine-5a,10(1H)-diol

[Chemical Formula 214]

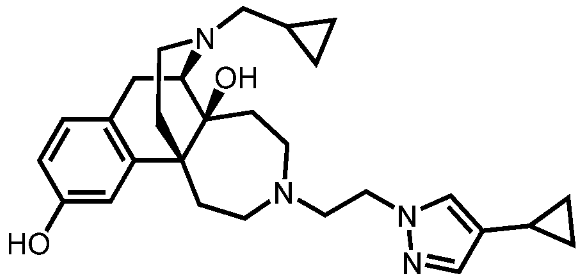

The title compound was obtained from the compound obtained in Example 160 according to the method described in Example 68.

$^1$H-NMR (400 MHz, $CDCl_3$)δ (ppm): 0.05 - 0.20 (m, 2H), 0.45 - 0.60 (m, 4H), 0.70 - 0.95 (m, 3H), 1.00 - 1.10 (m, 1H), 1.20 - 1.40 (m, 1H), 1.40 - 1.50 (m, 2H), 1.50 - 2.20 (m, 3H), 2.20 - 2.45 (m, 4H), 2.50 - 2.60 (m, 2H), 2.60 - 2.80 (m, 2H), 2.85 - 3.10 (m, 4H), 3.15 - 3.30 (m, 1H), 4.00 - 4.20 (m, 2H), 6.56 (d, J = 3 Hz, 1H), 6.63 (dd, J = 3, 8 Hz, 1H), 6.94 (d, J = 8 Hz, 1H), 7.00 (s, 1H), 7.22 (s, 1H).

(Example 184)

Synthesis of (5aS,6R,11bS)-14-(cyclopropylmethyl)-3-(2-(4-fluoro-1H-pyrazol-1-yl)ethyl)-2,3,4,5,6,7-hexahydro-6,11b-(epiminoethano)naphtho[1,2-d]azepine-5a,10(1H)-diol

[Chemical Formula 215]

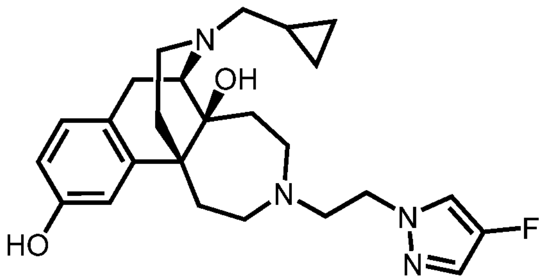

The title compound was obtained from the compound obtained in Example 180 and 4-fluoro-1H-pyrazole according to the methods described in Examples 181 and 182.

$^1$H-NMR (400 MHz, $CDCl_3$)δ (ppm): 0.05 - 0.20 (m, 2H), 0.45 - 0.60 (m, 2H), 0.75 - 1.00 (m, 1H), 0.95 - 1.05 (m, 1H), 1.40 - 1.90 (m, 3H), 1.90 - 2.10 (m, 3H), 2.25 - 2.65 (m, 6H), 2.70 - 2.85 (m, 3H), 2.85 - 3.05 (m, 2H), 3.05 - 3.15 (m, 1H), 3.80 - 4.00 (m, 2H), 6.60 - 6.70 (m, 3H), 6.97 (d, J = 8 Hz, 1H), 7.19 (d, J = 4 Hz, 1H).

(Reference Example 14)

Synthesis of 2-(5-methylisoxazol-3-yl)acetic acid

[Chemical Formula 216]

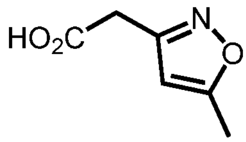

The title compound was obtained from 2-(5-methylisoxazol-3-yl)acetonitrile according to the method described in Reference Example 13.
$^1$H-NMR (400 MHz, $CDCl_3$)δ (ppm): 2.43 (s, 3H), 3.76 (s, 2H), 6.06 (s, 1H).

(Example 185)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(5-methylisoxazol-3-yl)ethan-1-one

[Chemical Formula 217]

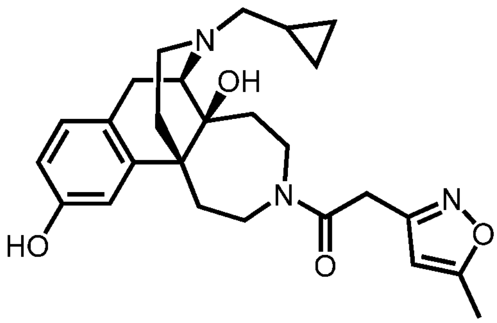

The title compound was obtained from the compound E obtained in Example 3 and the compound obtained in Reference Example 14 according to the method described in Example 26.
$^1$H-NMR (400 MHz, $CDCl_3$)δ (ppm): 0.08 - 0.13 (m, 2H), 0.50 - 0.54 (m, 2H), 0.78 - 0.85 (m, 1H), 1.04 - 1.13 (m, 1H), 1.55 - 1.64 (m, 1H), 1.78 - 2.08 (m, 3.4H), 2.16 - 2.38 (m, 6.6H), 2.51 - 2.60 (m, 1.4H), 2.72 (dd, J = 7, 19 Hz, 0.6H), 2.77 (dd, J = 6, 20 Hz, 0.4H), 2.87 - 3.01 (m, 2H), 3.16 - 3.22 (m, 0.6H), 3.36 - 3.48 (m, 2H), 3.59 (d, J = 15 Hz, 0.4H), 3.67 (d, J = 15 Hz, 0.6H), 3.63 - 3.97 (m, 2H), 4.48 (br s, 0.6H), 5.90 (s, 0.4H), 5.97 (s, 0.6H), 6.61 - 6.68 (m, 1.4H), 6.82 - 6.84 (m, 0.6H), 6.90 - 6.94 (m, 1H), 7.60 (br s, 0.4H).

(Example 186)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-3-(dimethylamino)propan-1-one

[Chemical Formula 218]

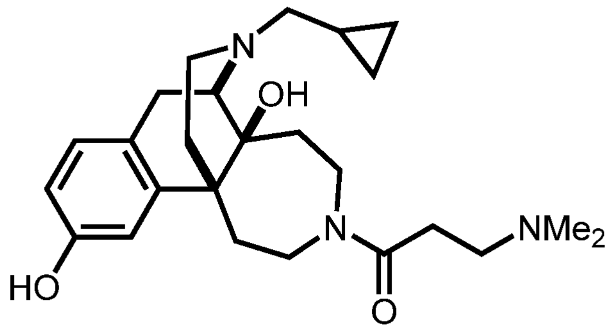

The title compound was obtained from the compound E obtained in Example 3 and 3-(dimethylamino)propanoic acid according to the method described in Example 85.

$^1$H-NMR (400 MHz, $CDCl_3$)δ (ppm): 0.02 - 0.17 (m, 2H), 0.43 - 0.57 (m, 2H), 0.76 - 0.88 (m, 1H), 1.02 - 1.14 (m, 1H), 1.52 - 1.65 (m, 1H), 1.77 (ddd, J = 2, 12, 14 Hz, 0.5H), 1.86 - 2.11 (m, 3H), 2.13 - 2.47 (m, 5.5H), 2.23 (s, 3H), 2.24 (s, 3H), 2.49 - 2.81 (m, 4H), 2.88 (d, J = 6 Hz, 0.5H), 2.92 (d, J = 19 Hz, 0.5H), 2.92 (d, J = 6 Hz, 0.5H), 3.00 (d, J = 18 Hz, 0.5H), 3.12 - 3.27 (m, 1H), 3.37 (ddd, J = 3, 3, 14 Hz, 0.5H), 3.40 (ddd, J = 2, 6, 14 Hz, 0.5H), 3.80 - 3.98 (m, 1.5H), 4.07 - 4.16 (m, 0.5H), 4.47 (br s, 0.5H), 4.53 (br s, 0.5H), 6.58 - 6.63 (m, 0.5H), 6.65 (dd, J = 2, 8 Hz, 0.5H), 6.68 - 6.71 (m, 0.5H), 6.81 (d, J = 2 Hz, 0.5H), 6.90 (d, J = 8 Hz, 0.5H), 6.94 (d, J = 8 Hz, 0.5H).

(Example 187)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-3-methoxypropan-1-one

[Chemical Formula 219]

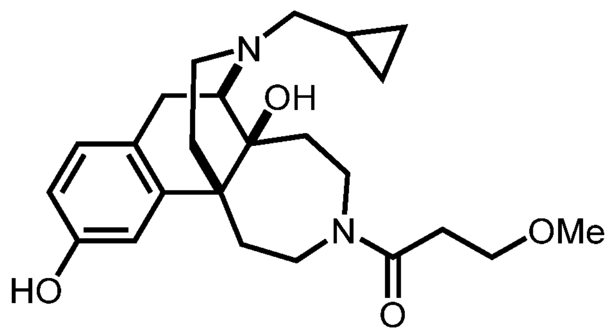

The title compound was obtained from the compound E obtained in Example 3 and 3-methoxypropanoic acid according to the method described in Example 85.

$^1$H-NMR (400 MHz, $CDCl_3$)δ (ppm): 0.03 - 0.17 (m, 2H), 0.44 - 0.57 (m, 2H), 0.74 - 0.90 (m, 1H), 0.91 - 0.98 (m, 0.3H), 1.02 - 1.11 (m, 0.7H), 1.53 - 1.63 (m, 1H), 1.72 - 2.12 (m, 4H), 2.23 - 2.57 (m, 6H), 2.74 (dd, J = 6, 18 Hz, 1H), 2.83 - 2.92 (m, 1.3H), 2.98 (d, J = 18 Hz, 0.7H), 3.27 (s, 2.1H), 3.29 (s, 0.9H), 3.30 - 3.88 (m, 6H), 4.49 (br s, 1H), 6.61 (dd, J = 2, 8 Hz, 0.3H), 6.65 (d, J = 2 Hz, 0.3H), 6.69 (dd, J = 2, 8 Hz, 0.7H), 6.77 - 6.82 (m, 0.7H), 6.86 (d, J = 8 Hz, 0.3H), 6.89 (d, J = 8 Hz, 0.7H).

(Example 188)

Synthesis of (5aS,6R,11bS)-14-(cyclopropylmethyl)-3-(2-(5-methylisoxazol-3-yl)ethyl)-2,3,4,5,6,7-hexahydro-6,11b-(epiminoethano)naphtho[1,2-d]azepine-5a,10(1H)-diol

[Chemical Formula 220]

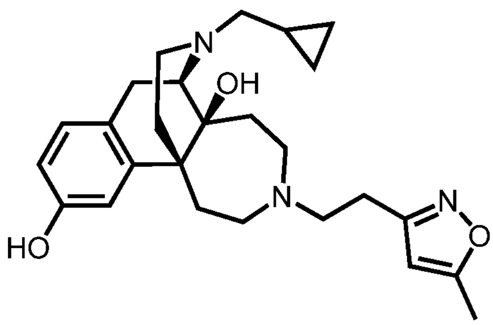

The title compound was obtained from the compound obtained in Example 185 according to the method described in Example 24.

$^1$H-NMR (400 MHz, $CDCl_3$)δ (ppm): 0.08 - 0.11 (m, 2H), 0.47 - 0.52 (m, 2H), 0.78 - 0.86 (m, 1H), 1.03 - 1.05 (m, 1H), 1.49 (dd, J = 4, 14 Hz, 1H), 1.68 - 1.71 (m, 1H), 1.78 - 1.84 (m, 2H), 1.99 - 2.03 (m, 2H), 2.32 (s, 3H), 2.32 - 2.35 (m, 2H)2.52 - 2.54 (m, 1H), 2.71 - 2.96 (m, 9H), 3.13 - 3.19 (m, 1H), 3.69 - 3.71 (m, 2H), 4.71 (br s, 1H), 5.72 (s, 1H), 6.45 (s, 1H), 6.54 - 6.59 (m, 1H), 6.89 (d, J = 8 Hz, 1H).

(Example 189)

Synthesis of 1-(2-((5aS,6R,11bS)-14-(cyclopropylmethyl)-5a,10-dihydroxy-1,2,5,5a,6,7-hexahydro-6,11b-(epiminoethano)naphtho[1,2-d]azepin-3(4H)-yl)ethyl)-1H-pyrazole-4-carbonitrile

[Chemical Formula 221]

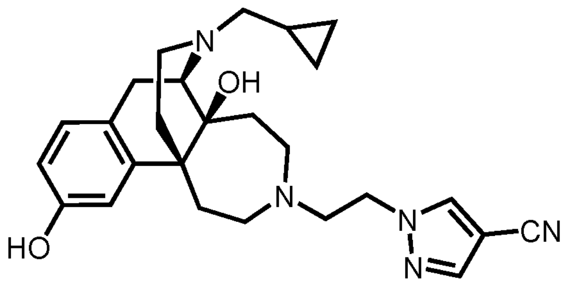

The target compound was obtained from 1-(2-hydroxyethyl)-1H-pyrazole-4-carbonitrile and the compound E obtained in Example 3 according to the methods described in Reference Example 6 and Example 31.

$^1$H-NMR (400 MHz, $CDCl_3$)δ (ppm): 0.05 - 0.20 (m, 2H), 0.45 - 0.60 (m, 2H), 0.75 - 0.90 (m, 1H), 1.03 (d, J = 12 Hz, 1H), 1.15 - 1.25 (m, 1H), 1.77 (dd, J = 5, 15 Hz, 1H), 1.90 - 2.10 (m, 2H), 2.15 - 2.25 (m, 1H), 2.25 - 2.40 (m, 2H), 2.45 - 2.70 (m, 3H), 2.70 - 3.05 (m, 5H), 3.05 - 3.20 (m, 2H), 3.65 - 3.80 (m, 1H), 3.85 - 4.05 (m, 2H), 6.65 - 6.80 (m, 3H), 7.12 (d, J = 8 Hz, 1H), 7.63 (s, 1H).

(Example 190)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-3-(pyridin-2-yl)propan-1-one

[Chemical Formula 222]

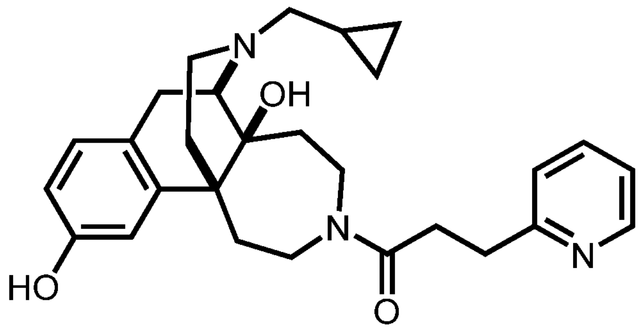

The title compound was obtained from the compound E obtained in Example 3 and 3-(pyridin-2-yl)propanoic acid according to the method described in Example 85.

$^1$H-NMR (400 MHz, $CDCl_3$)δ (ppm): 0.06 - 0.17 (m, 2H), 0.44 - 0.57 (m, 2H), 0.76 - 0.90 (m, 1H), 1.07 - 1.16 (m, 1H), 1.52 - 1.63 (m, 1H), 1.77 - 2.43 (m, 8H), 2.50 - 2.59 (m, 1H), 2.65 - 3.20 (m, 7H), 3.25 - 3.37 (m, 1H), 3.88 (ddd, J = 3, 13, 13 Hz, 0.3H), 3.94 - 4.07 (m, 1.4H), 4.22 (dd, J = 4, 15 Hz, 0.3H), 4.43 (br s, 0.3H), 4.55 (br s, 0.7H), 6.69 (dd, J = 2, 8 Hz, 0.3H), 6.71 - 6.76 (m, 0.7H), 6.83 (dd, J = 2, 2 Hz, 0.7H), 6.88 - 6.97 (m, 1.3H), 7.09 (dd, J = 5, 8 Hz, 0.3H), 7.16 - 7.23 (m, 1.7H), 7.56 (ddd, J = 2, 8, 8 Hz, 0.3H), 7.65 (ddd, J = 2, 8, 8 Hz, 0.7H), 8.47 - 8.53 (m, 1H).

(Reference Example 15)

Synthesis of (5-ethylisoxazol-3-yl)methanol

[Chemical Formula 223]

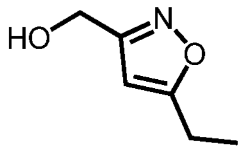

To a solution of ethyl 5-ethylisoxazole-3-carboxylate (synthesized by the method described in WO 2016040515) (410 mg, 2.42 mmol) in tetrahydrofuran (4.8 mL) was added a 4 M lithium borohydride-tetrahydrofuran solution (1.21 mL, 4.84 mmol) under ice cooling, followed by stirring at room temperature for 24.5 hours. Water was added to the reaction mixture, followed by extraction three times with ethyl acetate. The combined extracts were washed with saturated brine, dried over sodium sulfate, and then concentrated under reduced pressure to yield the title compound (195 mg, 63%) as a pale yellow oily matter.

$^1$H-NMR (400 MHz, $CDCl_3$)δ (ppm): 1.30 (t, J = 8 Hz, 3H), 2.77 (q, J = 8 Hz, 2H), 4.73 (s, 2H), 6.03 (s, 1H).

(Reference Example 16)

Synthesis of 3-(chloromethyl)-5-ethylisoxazole

[Chemical Formula 224]

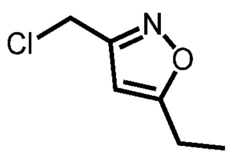

The title compound was obtained from the compound obtained in Reference Example 15 according to the method described in Reference Example 11.

$^1$H-NMR (400 MHz, $CDCl_3$)δ (ppm): 1.31 (t, J = 8 Hz, 3H), 2.77 (q, J = 8 Hz, 2H), 4.56 (s, 2H), 6.10 (s, 1H).

(Reference Example 17)

Synthesis of 2-(5-ethylisoxazol-3-yl)acetonitrile

[Chemical Formula 225]

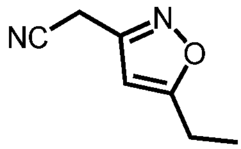

The title compound was obtained from the compound obtained in Reference Example 16 according to the method described in Reference Example 12.

$^1$H-NMR (400 MHz, $CDCl_3$)δ (ppm): 1.31 (t, J = 8 Hz, 3H), 2.79 (q, J = 8 Hz, 2H), 3.78 (s, 2H), 6.10 (s, 1H).

(Reference Example 18)

Synthesis of 2-(5-ethylisoxazol-3-yl)acetic acid

[Chemical Formula 226]

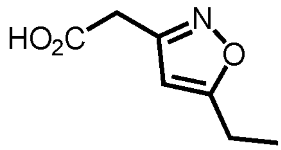

The title compound was obtained from the compound obtained in Reference Example 17 according to the method described in Reference Example 13.

$^1$H-NMR (400 MHz, $CDCl_3$)δ (ppm): 1.30 (t, J = 8 Hz, 3H), 2.77 (q, J = 8 Hz, 2H), 3.77 (s, 2H), 6.05 (s, 1H).

(Example 191)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(5-ethylisoxazol-3-yl)ethan-1-one

[Chemical Formula 227]

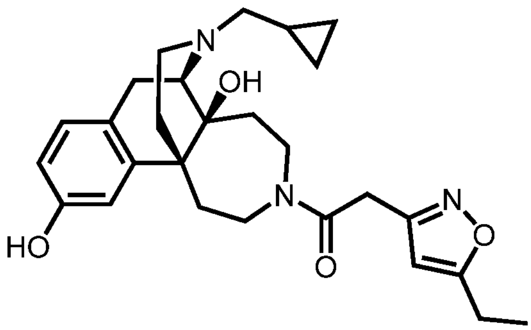

The title compound was obtained from the compound E obtained in Example 3 and the compound obtained in Reference Example 18 according to the method described in Example 26.

$^1$H-NMR (400 MHz, $CDCl_3$)δ (ppm): 0.08 - 0.13 (m, 2H), 0.48 - 0.54 (m, 2H), 0.80 - 0.86 (m, 1H), 1.04 - 1.13 (m, 1H), 1.23 - 1.32 (m, 3H), 1.59 - 1.63 (m, 1H), 1.70 - 2.10 (m, 3.4H), 2.20 - 2.23 (m, 1H), 2.28 - 2.39 (m, 2H), 2.52 - 2.60 (m, 1.4H), 2.68 - 2.81 (m, 3H), 2.89 - 3.02 (m, 2H), 3.16 (ddd, J = 4, 9, 13 Hz, 0.6H), 3.37 - 3.49 (m, 2H), 3.59 (d, J = 15 Hz, 0.4H), 3.68 (d, J = 15 Hz, 0.6H), 3.71 - 3.80 (m, 1H), 3.83 - 3.88 (m, 0.4H), 3.93 (ddd, J = 3, 12, 12 Hz, 0.6H), 4.12 - 4.18 (m, 0.6H), 5.92 (s, 0.4H), 5.99 (s, 0.6H), 6.61 - 6.67 (m, 1.4H), 6.80 - 6.82 (m, 0.6H), 6.91 - 6.95 (m, 1H).

(Example 192)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(4-ethyl-1H-pyrazol-1-yl)ethan-1-one

[Chemical Formula 228]

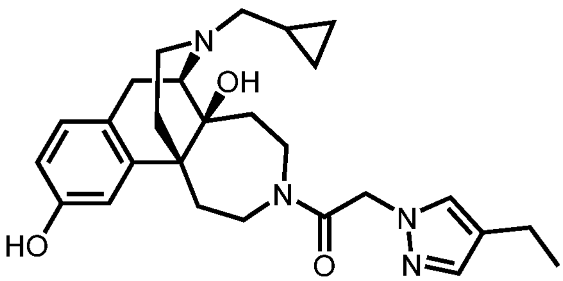

The title compound was obtained from the compound obtained in Example 180 and 4-ethyl-1H-pyrazole according to the methods described in Example 181.

$^1$H-NMR (400 MHz, $CDCl_3$)δ (ppm): 0.05 - 0.20 (m, 2H), 0.45 - 0.60 (m, 2H), 0.75 - 0.90 (m, 1H), 1.00 - 1.10 (m, 1H), 1.10 - 1.30 (m, 3H), 1.50 - 2.10 (m, 3H), 2.10 - 2.25 (m, 1H), 2.25 - 2.40 (m, 2H), 2.40 - 2.70 (m, 3H), 2.70 - 2.85 (m, 1H), 2.85 - 3.15 (m, 2.4H), 3.20 - 3.60 (m, 1.6H), 3.60 - 3.75 (m, 0.6H), 3.80 - 4.00 (m, 1.4H), 4.05 - 4.25 (m, 1H), 4.52 (br s, 1H), 4.60 (d, J = 16 Hz, 0.6H), 4.73 (d, J = 16 Hz, 0.6H), 4.88 (d, J = 16 Hz, 0.4H), 4.93 (d, J = 16 Hz, 0.4H), 6.60 - 6.80 (m, 2H), 6.90 - 7.00 (m, 1H), 7.14 (s, 0.6H), 7.21 (s, 0.4H), 7.34 (s, 0.6H), 7.42 (s, 0.4H).

(Example 193)

Synthesis of (5aS,6R,11bS)-14-(cyclopropylmethyl)-3-(2-(4-ethyl-1H-pyrazol-1-yl)ethyl)-2,3,4,5,6,7-hexahydro-6,11b-(epiminoethano)naphtho[1,2-d]azepme-5a,10(1H)-diol

[Chemical Formula 229]

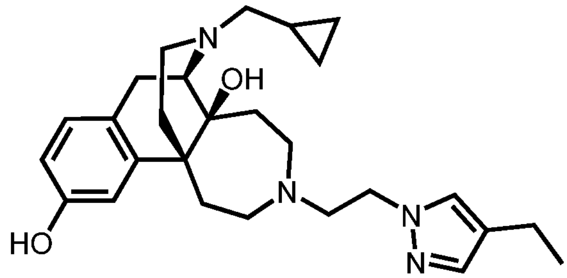

The title compound was obtained from the compound obtained in Example 192 according to the method described in Example 24.

$^1$H-NMR (400 MHz, $CDCl_3$)δ (ppm): 0.05 - 0.20 (m, 2H), 0.45 - 0.60 (m, 2H), 0.75 - 0.90 (m, 1H), 1.03 (d, J = 10 Hz, 1H), 1.15 (t, J = 8 Hz, 3H), 1.40 - 1.50 (m, 1H), 1.70 - 1.85 (m, 2H), 1.90 - 2.10 (m, 2H), 2.30 - 2.40 (m, 3H), 2.41 (q, J = 8 Hz, 2H), 2.50 - 2.70 (m, 3H), 2.70 - 2.80 (m, 1H), 2.80 - 3.00 (m, 5H), 3.15 - 3.30 (m, 1H), 4.00 - 4.15 (m, 2H), 6.55 (s, 1H), 6.61 (d, J = 8 Hz, 1H), 6.92 (d, J = 8 Hz, 1H), 6.96 (s, 1H), 7.28 (s, 1H).

(Example 194)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(4-isopropyl-1H-pyrazol-1-yl)ethan-1-one

[Chemical Formula 230]

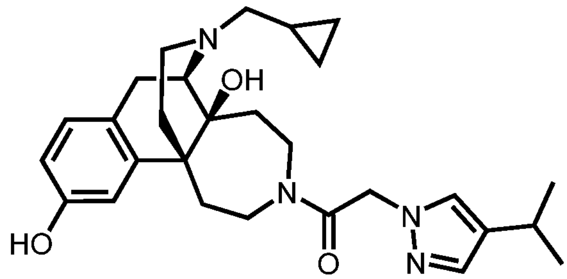

The title compound was obtained from the compound obtained in Example 180 and 4-isopropyl-1H-pyrazole according to the method described in Example 181.

$^1$H-NMR (400 MHz, $CDCl_3$)δ (ppm): 0.05 - 0.20 (m, 2H), 0.45 - 0.60 (m, 2H), 0.75 - 0.90 (m, 1H), 1.00 - 1.10 (m, 1H), 1.17 (d, J = 7 Hz, 3.6H), 1.18 (d, J = 7 Hz, 2.4H), 1.50 - 1.70 (m, 1H), 1.70 - 1.90 (m, 2H), 1.90 - 2.10 (m, 3H), 2.20 - 2.40 (m, 2H), 2.50 - 3.05 (m, 5H), 3.15 - 3.30 (m, 1H), 3.30 - 3.45 (m, 1H), 3.70 - 3.95 (m, 2H), 4.55 (br s, 1H), 4.57 (d, J = 16 Hz, 0.6H), 4.74 (d, J = 16 Hz, 0.6H), 4.78 (d, J = 16 Hz, 0.4H), 4.86 (d, J = 16 Hz, 0.4H), 6.60 - 6.70 (m, 2H), 6.90 (d, J = 8 Hz, 0.6H), 6.93 (d, J = 8 Hz, 0.4H), 7.15 (s, 0.4H), 7.16 (s, 0.6H), 7.34 (s, 0.4H), 7.36 (s, 0.6H).

(Example 195)

Synthesis of (5aS,6R,11bS)-14-(cyclopropylmethyl)-3-(2-(4-isopropyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 231]

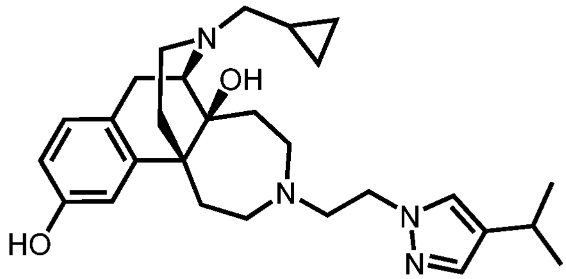

The title compound was obtained from the compound obtained in Example 194 according to the method described in Example 24.

$^1$H-NMR (400 MHz, $CDCl_3$)$\delta$ (ppm): 0.05 - 0.20 (m, 2H), 0.45 - 0.60 (m, 2H), 0.75 - 0.90 (m, 1H), 1.03 (d, J = 10 Hz, 1H), 1.16 (d, J = 7 Hz, 3H), 1.17 (d, J = 7 Hz, 3H), 1.44 (dd, J = 2, 15 Hz, 1H), 1.70 - 1.85 (m, 2H), 1.95 - 2.05 (m, 2H), 2.20 - 2.40 (m, 2H), 2.50 - 2.85 (m, 6H), 2.85 - 3.00 (m, 5H), 3.15 - 3.30 (m, 1H), 4.00 - 4.15 (m, 2H), 6.50 (d, J = 2 Hz, 1H), 6.60 (dd, J = 2, 8 Hz, 1H), 6.90 (d, J = 8 Hz, 1H), 7.05 (s, 1H), 7.31 (s, 1H).

(Example 196)

Synthesis of (5aS,6R,11bS)-14-allyl-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 232]

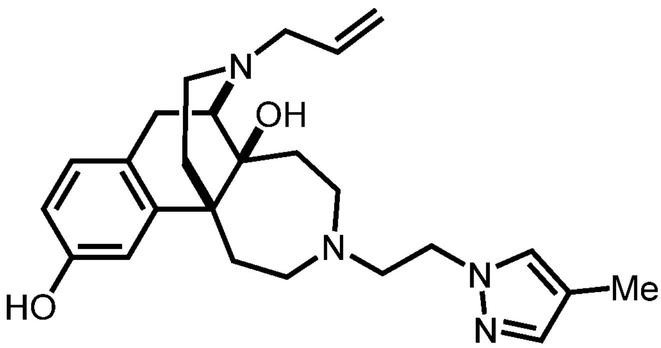

The title compound was obtained from the compound obtained in Example 176 and allyl bromide according to the method described in Example 161.

$^1$H-NMR (400 MHz, $CDCl_3$)$\delta$ (ppm): 0.95 - 1.10 (m, 1H), 1.35 - 1.50 (m, 1H), 1.70 - 1.85 (m, 2H), 1.95 - 2.08 (m, 2H), 2.00 (s, 3H), 2.28 - 2.53 (m, 3H), 2.54 - 2.62 (m, 1H), 2.68 - 3.02 (m, 6H), 3.07 - 3.19 (m, 3H), 3.94 - 4.08 (m, 2H), 5.08 - 5.21 (m, 2H), 5.72 - 5.85 (m, 1H), 6.56 (d, J = 2 Hz, 1H), 6.62 (dd, J = 2, 8 Hz, 1H), 6.86 (s, 1H), 6.95 (d, J = 8 Hz, 1H), 7.23 (s, 1H).

(Example 197)

Synthesis of (5aS,6R,11bS)-14-(cyclopropylmethyl)-3-(2-(5-ethylisoxazol-3-yl)ethyl)-2,3,4,5,6,7-hexahydro-6,1b-(epiminoethano)naphtho[1,2-d]azepine-5a,10(1H)-diol

[Chemical Formula 233]

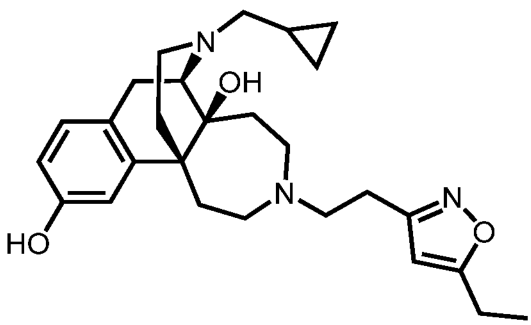

The title compound was obtained from the compound obtained in Example 191 according to the method described in Example 24.

$^1$H-NMR (400 MHz, $CDCl_3$)$\delta$ (ppm): 0.08 - 0.11 (m, 2H), 0.48 - 0.52 (m, 2H), 0.80 - 0.87 (m, 1H), 1.03 - 1.05 (m, 1H), 1.23 (t, J = 8 Hz, 3H), 1.46 - 1.49 (m, 1H), 1.77 - 1.87 (m, 2H), 1.96 - 2.08 (m, 2H), 2.31 - 2.38 (m, 3H), 2.53 - 2.55 (m, 1H), 2.65 - 2.83 (m, 9H), 2.88 - 2.97 (m, 3H), 3.13 - 3.19 (m, 1H), 4.69 (br s, 1H), 5.73 (s, 1H), 6.50 (d, J = 2 Hz, 1H), 6.58 (dd, J = 2, 8 Hz, 1H), 6.91 (d, J = 8 Hz, 1H).

(Example 198)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(dimethylamino)ethan-1-one

[Chemical Formula 234]

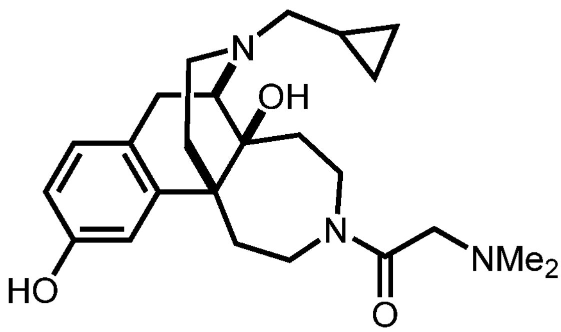

The title compound was obtained from the compound E obtained in Example 3 and dimethylglycine according to the method described in Example 85.

$^1$H-NMR (400 MHz, $CDCl_3$)$\delta$ (ppm): 0.05 - 0.13 (m, 2H), 0.46 - 0.54 (m, 2H), 0.77 - 0.87 (m, 1H), 1.01 - 1.07 (m, 0.3H), 1.09 - 1.15 (m, 0.7H), 1.56 - 1.64 (m, 1H), 1.78 - 2.12 (m, 3H), 2.16 - 2.39 (m, 3.7H), 2.21 (s, 4.2H), 2.25 (s, 1.8H), 2.49 - 2.62 (m, 1.3H), 2.76 (dd, J = 6, 18 Hz, 0.7H), 2.77 (dd, J = 6, 18 Hz, 0.3H), 2.84 - 3.17 (m, 4H), 3.20 - 3.29 (m, 0.7H), 3.30 - 3.39 (m, 0.3H), 3.41 - 3.51 (m, 1H), 3.76 (ddd, J = 4, 12, 14 Hz, 0.3H), 3.81 - 3.91 (m, 1H), 4.09 - 4.17 (m, 0.7H), 4.52 (br s, 1H), 6.54 (dd.J = 3, 8 Hz, 0.3H), 6.65 - 6.70 (m, 1H), 6.87 (d, J = 8 Hz, 0.3H), 6.92 (d, J = 8 Hz, 0.7H), 6.95 (d, J = 2 Hz, 0.7H).

(Example 199)

Synthesis of (5aS,6R,11bR)-3-(3-((tertbutyldimethylsilyl)oxy)propyl)-14-(cyclopropylmethyl)-5a-hydroxy-10-methoxy-3,4,5,5a,6,7-hexahydro-6,11b-(epiminoethano)naphtho[1,2-d]azepin-2(1H)-one

[Chemical Formula 235]

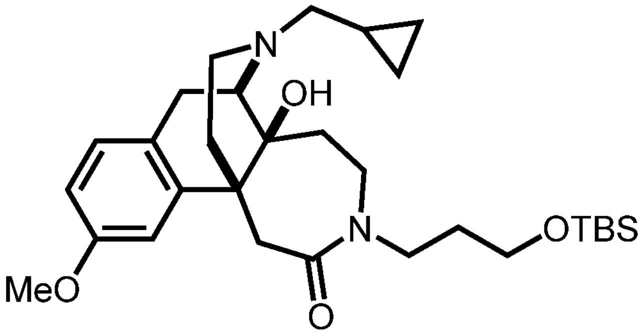

The title compound was obtained from the compound obtained in Example 140 and (3-bromopropoxy)(tert-butyl)dimethylsilane according to the method described in Example 141.

$^1$H-NMR (400 MHz, $CDCl_3$)δ (ppm): - 0.04 (s, 3H), - 0.03 (s, 3H), 0.02 - 0.16 (m, 2H), 0.46 - 0.55 (m, 2H), 0.80 - 0.95 (m, 1H), 0.84 (s, 9H), 1.08 - 1.18 (m, 1H), 1.33 - 1.82 (m, 4H), 1.99 - 2.12 (m, 2H), 2.28 - 2.45 (m, 2H), 2.53 - 2.60 (m, 1H), 2.64 (d, J = 15 Hz, 1H), 2.73 (dd, J = 6, 18 Hz, 1H), 2.88 - 2.99 (m, 2H), 3.00 (d, J = 18 Hz, 1H), 3.06 - 3.21 (m, 2H), 3.33 - 3.56 (m, 3H), 3.81 (s, 3H), 4.10 (dd, J = 11, 15 Hz, 1H), 4.72 (br s, 1H), 6.68 (dd, J = 2, 8 Hz, 1H), 6.93 (d, J = 9 Hz, 1H), 7.19 (d, J = 2 Hz, 1H).

(Example 200)

Synthesis of (5aS,6R,11bR)-14-(cyclopropylmethyl)-5a-hydroxy-3-(3-hydroxypropyl)-10-methoxy-3,4,5,5a,6,7-hexahydro-6,11b-(epiminoethano)naphtho[1,2-d]azepin-2(1H)-one

[Chemical Formula 236]

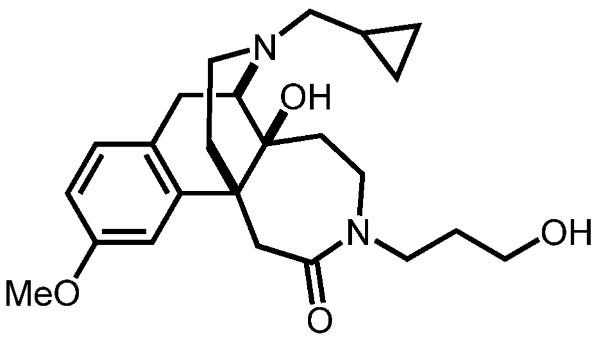

To a solution of the compound (138 mg, 0.26 mmol) obtained in Example 199 in tetrahydrofuran (30 mL) was added a 1 M tetrabutylammonium fluoride tetrahydrofuran solution (2.6 mL, 2.6 mmol), followed by stirring at room temperature for 3 hours. To the reaction mixture, a saturated sodium bicarbonate aqueous solution was added, followed by extraction three times with chloroform. The organic layers were combined, dried over sodium sulfate, and then concentrated under reduced pressure. The obtained crude product was purified by silica gel column chromatography (amino group-supported silica gel, 0 to 50% methanol/chloroform) to yield the title compound (99.5 mg, 92%) as a colorless amorphous form.

$^1$H-NMR (400 MHz, $CDCl_3$)δ (ppm): 0.07 - 0.18 (m, 2H), 0.46 - 0.58 (m, 2H), 0.77 - 0.90 (m, 1H), 1.10 - 1.39 (m, 2H), 1.44 - 1.80 (m, 3H), 2.00 - 2.15 (m, 2H), 2.35 (dd, J = 6, 12 Hz, 1H), 2.37 (dd, J = 6, 12 Hz, 1H), 2.53 - 2.67 (m, 2H), 2.71 (d, J = 15 Hz, 1H), 2.74 (dd, J = 7, 18 Hz, 1H), 2.84 - 2.96 (m, 2H), 3.00 (d, J = 18 Hz, 1H), 3.01 - 3.18 (m, 2H), 3.56 (d, J = 15 Hz, 1H), 3.68 - 3.88 (m, 2H), 3.83 (s, 3H), 4.17 (dd, J = 10, 15 Hz, 1H), 6.72 (dd, J = 2, 8 Hz, 1H), 6.95 (d, J = 9 Hz, 1H), 7.19 (d, J = 2 Hz, 1H).

(Example 201)

Synthesis of 3-((5aS,6R,11bR)-14-(cyclopropylmethyl)-5a-hydroxy-10-methoxy-2-oxo-1,2,5,5a,6,7-hexahydro-6,11b-(epiminoethano)naphtho[1,2-d]azepin-3(4H)-yl)propyl methanesulfonate

[Chemical Formula 237]

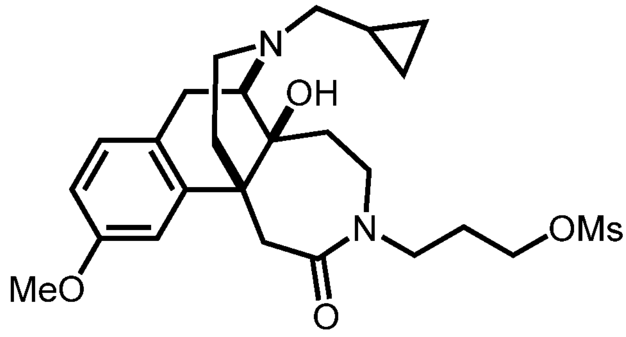

The title compound was obtained from the compound obtained in Example 200 according to the method described in Reference Example 7.

$^1$H-NMR (400 MHz, $CDCl_3$)δ (ppm): 0.06 - 0.18 (m, 2H), 0.46 - 0.58 (m, 2H), 0.76 - 0.92 (m, 1H), 1.08 - 1.21 (m, 1H), 1.55 - 3.18 (m, 16H), 2.94 (s, 3H), 3.26 - 3.86 (m, 3H), 3.80 (s, 3H), 4.15 (dd, J = 11, 15 Hz, 1H), 6.69 (dd, J = 2, 8 Hz, 1H), 6.97 (d, J = 9 Hz, 1H), 7.16 (d, J = 2 Hz, 1H).

(Example 202)

Synthesis of (5aS,6R,11bR)-14-(cyclopropylmethyl)-5a-hydroxy-10-methoxy-3-(3-(4-methyl-1H-pyrazol-1-yl)propyl)-3,4,5,5a,6,7-hexahydro-6,11b-(epiminoethano)naphtho[1,2-d]azepin-2(1H)-one

[Chemical Formula 238]

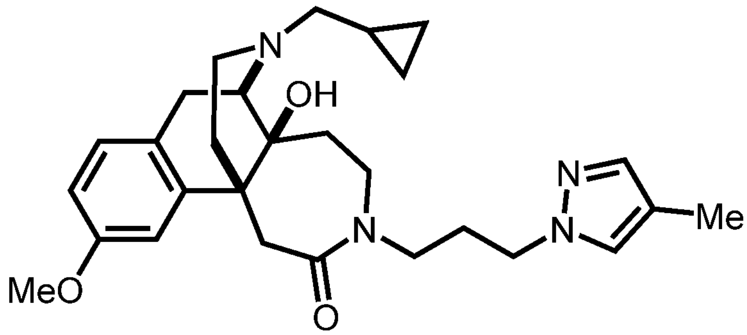

To a solution of the compound (9.8 mg, 0.020 mmol) obtained in Example 201 in tetrahydrofuran (1 mL) were added 4-methylpyrazole (16.5 pL, 0.20 mmol) and sodium hydride (55% oil dispersion, 7.0 mg, 0.16 mmol), followed by stirring at room temperature for 4 hours. To the reaction mixture, a saturated sodium bicarbonate aqueous solution was added, followed by extraction three times with chloroform. The combined extracts were dried over sodium sulfate and then concentrated under reduced pressure. The obtained crude product was purified by silica gel column chromatography (amino group-supported silica gel, 0 to 20% methanol/chloroform) to yield the title compound (8.0 mg, 84%) as a colorless amorphous form.

$^1$H-NMR (400 MHz, $CDCl_3$)δ (ppm): 0.07 - 0.15 (m, 2H), 0.48 - 0.56 (m, 2H), 0.76 - 0.90 (m, 1H), 1.10 - 1.20 (m, 1H), 1.56 - 1.77 (m, 3H), 1.83 - 1.96 (m, 1H), 2.00 - 2.14 (m, 2H), 2.04 (s, 3H), 2.35 (dd, J = 7, 12 Hz, 1H), 2.36 (dd, J = 7, 12 Hz, 1H), 2.53 - 2.62 (m, 1H), 2.68 (d, J = 15 Hz, 1H), 2.76 (dd, J = 6, 18 Hz, 1H), 2.83 - 2.95 (m, 3H), 3.01 (d,

J = 18 Hz, 1H), 3.44 (t, J = 7 Hz, 2H), 3.55 (d, J = 15 Hz, 1H), 3.58 - 3.69 (m, 1H), 3.78 (s, 3H), 4.09 (dd, J = 11, 15 Hz, 1H), 4.68 (br s, 1H), 6.63 (dd, J = 3, 8 Hz, 1H), 6.94 (d, J = 9 Hz, 1H), 6.99 (s, 1H), 7.22 (s, 1H), 7.24 (d, J = 3 Hz, 1H).

(Example 203)

Synthesis of (5aS,6R,11bR)-14-(cyclopropylmethyl)-5a,10-dihydroxy-3-(3-(4-methyl-1H-pyrazol-1-yl)propyl)-3,4,5,5a,6,7-hexahydro-6,11b-(epiminoethano)naphtho[1,2-d]azepin-2(1H)-one

[Chemical Formula 239]

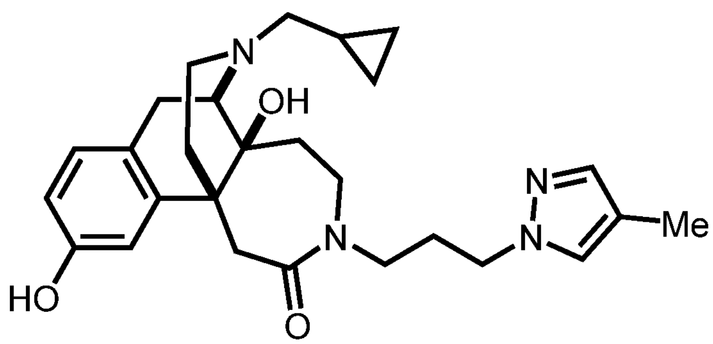

The title compound was obtained from the compound obtained in Example 202 according to the method described in Example 6.

$^1$H-NMR (400 MHz, DMSO - d6)δ (ppm): 0.08 - 0.19 (m, 2H), 0.46 - 0.58 (m, 2H), 0.82 - 0.92 (m, 1H), 1.06 - 1.38 (m, 1H), 1.58 - 1.89 (m, 4H), 1.99 - 2.19 (m, 2H), 2.04 (s, 3H), 2.28 - 2.45 (m, 2H), 2.52 - 2.62 (m, 2H), 2.75 - 2.88 (m, 2H), 2.94 - 3.07 (m, 3H), 3.26 - 3.36 (m, 1H), 3.37 - 3.46 (m, 1H), 3.56 (d, J = 15 Hz, 1H), 3.61 - 3.72 (m, 1H), 4.09 (dd, J = 11, 15 Hz, 1H), 6.52 (dd, J = 2, 8 Hz, 1H), 6.93 (d, J = 9 Hz, 1H), 6.97 (d, J = 3 Hz, 1H), 7.16 (s, 1H), 7.20 (s, 1H).

(Example 204)

Synthesis of (5aS,6R,11bR)-14-(cyclopropylmethyl)-5a-hydroxy-10-methoxy-3-(3-(pyrrolidin-1-yl)propyl)-3,4,5,5a,6,7-hexahydro-6,11b-(epiminoethano)naphtho[1,2-d]azepin-2(1H)-one

[Chemical Formula 240]

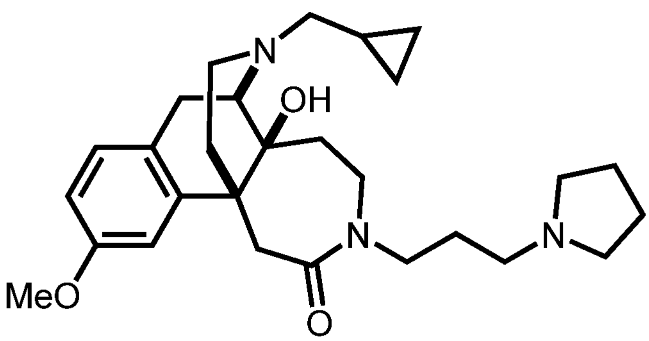

To a solution of the compound (9.8 mg, 0.020 mmol) obtained in Example 201 in tetrahydrofuran (1 mL) was added pyrrolidine (16.4 pL, 0.20 mmol), followed by stirring at 60°C for 1 hour. To the reaction mixture, a saturated sodium bicarbonate aqueous solution was added, followed by extraction three times with chloroform. The combined extracts were dried over sodium sulfate and then concentrated under reduced pressure. The obtained crude product was purified by silica gel column chromatography (amino group-supported silica gel, 0 to 20% methanol/chloroform) to

yield the title compound (5.0 mg, 53%) as a colorless amorphous form.

$^1$H-NMR (400 MHz, $CDCl_3$)δ (ppm): 0.05 - 0.16 (m, 2H), 0.46 - 0.56 (m, 2H), 0.75 - 0.90 (m, 1H), 1.06 - 1.18 (m, 1H), 1.34 - 1.89 (m, 9H), 1.99 - 2.16 (m, 3H), 2.17 - 2.42 (m, 6H), 2.50 - 2.77 (m, 3H), 2.86 - 3.04 (m, 4H), 3.52 (d, J = 15 Hz, 1H), 3.60 - 3.75 (m, 1H), 3.81 (s, 3H), 4.10 (dd, J = 11, 15 Hz, 1H), 4.70 (br s, 1H), 6.66 (dd, J = 2, 8 Hz, 1H), 6.93 (d, J = 8 Hz, 1H), 7.21 (d, J = 2 Hz, 1H).

(Example 205)

Synthesis of (5aS,6R,11bR)-14-(cyclopropylmethyl)-5a,10-dihydroxy-3-(3-(pyrrolidin-1-yl)propyl)-3,4,5,5a,6,7-hexahydro-6,11b-(epiminoethano)naphtho[1,2-d]azepin-2(1H)-one

[Chemical Formula 241]

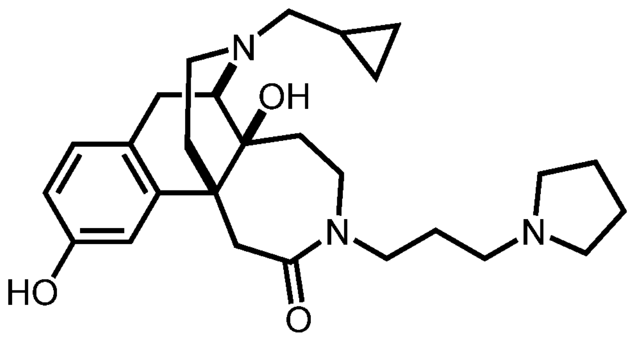

The title compound was obtained from the compound obtained in Example 204 according to the method described in Example 6.

$^1$H-NMR (400 MHz, $CDCl_3$)δ (ppm): 0.03 - 0.17 (m, 2H), 0.47 - 0.55 (m, 2H), 0.72 - 0.96 (m, 1H), 1.03 - 1.90 (m, 10H), 1.97 - 2.20 (m, 3H), 2.22 - 2.41 (m, 6H), 2.52 - 2.59 (m, 1H), 2.64 (d, J = 15 Hz, 1H), 2.71 (dd, J = 6, 18 Hz, 1H), 2.86 - 3.03 (m, 4H), 3.51 (d, J = 15 Hz, 1H), 3.64 - 3.75 (m, 1H), 4.09 (dd, J = 11, 15 Hz, 1H), 4.72 (br s, 1H), 6.64 (dd, J = 2, 8 Hz, 1H), 6.90 (d, J = 8 Hz, 1H), 7.12 (d, J = 2 Hz, 1H).

(Example 206)

Synthesis of (5aS,6R,11bR)-3-(3-(4-(tert-butyl)-1H-pyrazol-1-yl)propyl)-14-(cyclopropylmethyl)-5a-hydroxy-10-methoxy-3,4,5,5a,6,7-hexahydro-6,11b-(epiminoethano)naphtho[1,2-d]azepin-2(1H)-one

[Chemical Formula 242]

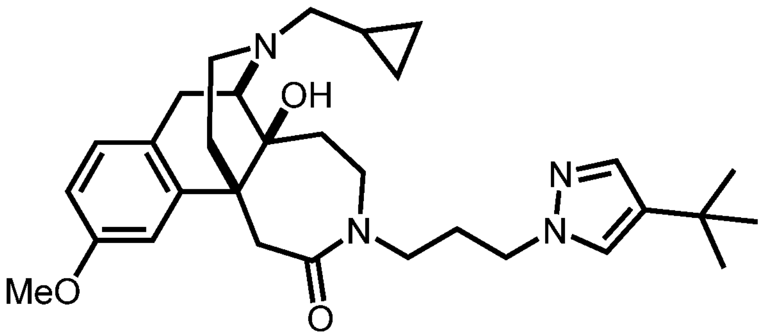

The title compound was obtained from the compound obtained in Example 201 and 4-tert-butyl-1H-pyrazole according to the methods described in Example 202.

$^1$H-NMR (400 MHz, $CDCl_3$)δ (ppm): 0.06 - 0.16 (m, 2H), 0.48 - 0.56 (m, 2H), 0.77 - 0.90 (m, 1H), 1.06 - 1.35

(m, 1H), 1.24 (s, 9H), 1.55 - 1.78 (m, 3H), 1.86 - 2.15 (m, 3H), 2.35 (dd, J = 7, 13 Hz, 1H), 2.36 (dd, J = 7, 13 Hz, 1H), 2.52 - 2.61 (m, 1H), 2.70 (d, J = 15 Hz, 1H), 2.76 (dd, J = 6, 19 Hz, 1H), 2.83 - 2.97 (m, 3H), 3.01 (d, J = 18 Hz, 1H), 3.34 - 3.53 (m, 2H), 3.55 (d, J = 15 Hz, 1H), 3.61 - 3.72 (m, 1H), 3.78 (s, 3H), 4.10 (dd, J = 11, 15 Hz, 1H), 4.74 (br s, 1H), 6.65 (dd, J = 2, 8 Hz, 1H), 6.94 (d, J = 8 Hz, 1H), 6.98 (s, 1H), 7.25 (d, J = 2 Hz, 1H), 7.30 (s, 1H).

(Example 207)

Synthesis of (5aS,6R,11bR)-3-(3-(4-(tert-butyl)-1H-pyrazol-1-yl)propyl)-14-(cyclopropylmethyl)-5a,10-dihydroxy-3,4,5,5a,6,7-hexahydro-6,11b-(epiminoethano)naphtho[1,2-d]azepin-2(1H)-one

[Chemical Formula 243]

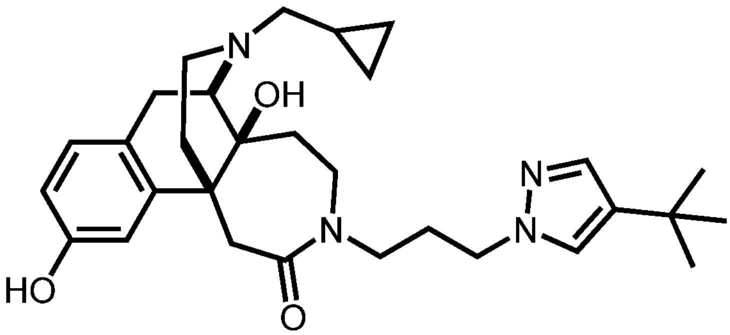

The title compound was obtained from the compound obtained in Example 206 according to the method described in Example 6.

$^1$H-NMR (400 MHz, $CDCl_3$)δ (ppm): 0.03 - 0.15 (m, 2H), 0.46 - 0.57 (m, 2H), 0.74 - 0.92 (m, 1H), 1.08 - 1.33 (m, 1H), 1.23 (s, 9H), 1.56 - 1.82 (m, 3H), 1.89 - 2.15 (m, 3H), 2.28 - 2.42 (m, 2H), 2.50 - 2.64 (m, 1H), 2.66 (d, J = 15 Hz, 1H), 2.74 (dd, J = 6, 18 Hz, 1H), 2.84 - 3.04 (m, 4H), 3.38 - 3.72 (m, 4H), 4.10 (dd, J = 11, 15 Hz, 1H), 6.64 (dd, J = 1, 8 Hz, 1H), 6.90 (d, J = 8 Hz, 1H), 6.98 (s, 1H), 7.22 (d, J = 1 Hz, 1H), 7.31 (s, 1H).

(Reference Example 19)

Synthesis of isothiazol-3-ylmethyl methanesulfonate

[Chemical Formula 244]

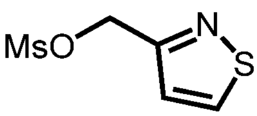

To a solution of isothiazol-3-ylmethanol (synthesized by the method described in WO 2018160878) (61.4 mg, 0.533 mmol) in tetrahydrofuran (2 mL) were added methanesulfonic anhydride (141 mg, 0.809 mmol) and N,N-diisopropylethylamine (183 pL, 1.06 mmol), followed by stirring at room temperature for 1.5 hours. To the reaction mixture, a saturated aqueous sodium bicarbonate solution was added, followed by extraction three times with ethyl acetate. The combined extracts were washed with saturated saline, dried over sodium sulfate, and then concentrated under reduced pressure. The obtained crude product was purified by silica gel column chromatography (49 to 70% ethyl acetate/heptane) to yield the title compound (71.0 mg, 69%) as a pale brown oily matter.

$^1$H-NMR (400 MHz, $CDCl_3$)δ (ppm): 3.06 (s, 3H), 5.40 (s, 2H), 7.39 (d, J = 5 Hz, 1H), 8.73 (d, J = 5 Hz, 1H).

(Reference Example 20)

Synthesis of 2-(isothiazol-3-yl)acetonitrile

[Chemical Formula 245]

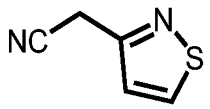

To a solution of the compound (71.0 mg, 0.367 mmol) obtained in Reference Example 19 in dimethyl sulfoxide (2 mL) was added sodium cyanide (53.4 mg, 1.09 mmol), followed by stirring at 70°C for 18.5 hours. After allowed to cool to room temperature, to the reaction mixture was added water, followed by extraction three times with tert-butyl methyl ether. The combined extracts were washed with saturated brine, dried over sodium sulfate, and then concentrated under reduced pressure to yield the title compound (30.0 mg, 66%) as a brown oily matter.

$^1$H-NMR (400 MHz, $CDCl_3$)$\delta$ (ppm): 3.98 (s, 2H), 7.32 (d, J = 5 Hz, 1H), 8.74 (d, J = 5 Hz, 1H).

(Reference Example 21)

Synthesis of 2-(isothiazol-3-yl)acetic acid

[Chemical Formula 246]

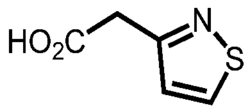

The title compound was obtained from the compound obtained in Reference Example 20 according to the method described in Reference Example 13.

$^1$H-NMR (400 MHz, $CDCl_3$)$\delta$ (ppm): 4.00 (s, 2H), 7.22 (d, J = 5 Hz, 1H), 8.71 (d, J = 5 Hz, 1H).

(Example 208)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(isothiazol-3-yl)ethan-1-one

[Chemical Formula 247]

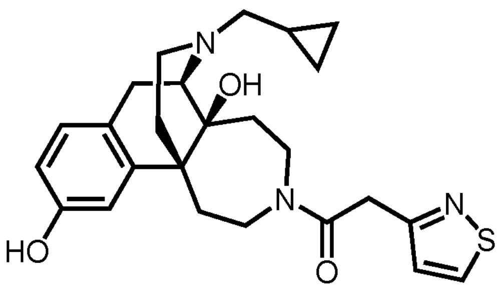

The title compound was obtained from the compound E obtained in Example 3 and the compound obtained in Reference Example 21 according to the method described in Example 5.

$^1$H-NMR (400 MHz, $CDCl_3$)δ (ppm): 0.07 - 0.13 (m, 2H), 0.49 - 0.53 (m, 2H), 0.79 - 0.86 (m, 1H), 1.04 - 1.12 (m, 1H), 1.55 - 1.63 (m, 1H), 1.75 - 2.10 (m, 3.6H), 2.14 - 2.40 (m, 3H), 2.44 - 2.57 (m, 1.4H), 2.69 (dd, J = 6, 18 Hz, 0.6H), 2.78 (dd, J = 6, 18 Hz, 0.4H), 2.88 - 2.99 (m, 2H), 3.24 - 3.31 (m, 0.6H), 3.39 - 3.48 (m, 1.4H), 3.70 - 3.77 (m, 0.4H), 3.71 (d, J = 16 Hz, 0.4H), 3.83 - 3.95 (m, 2H), 4.02 (d, J = 16 Hz, 0.6H), 4.06 - 4.12 (m, 0.6H), 6.61 - 6.66 (m, 1.4H), 6.81 (s, 0.6H), 6.91 - 6.93 (m, 1H), 7.12 - 7.13 (m, 1H), 8.54 - 8.56 (m, 1H).

(Example 209)

Synthesis of (5aS,6R,11bS)-14-(cyclopropylmethyl)-3-(2-(isothiazol-3-yl)ethyl)-2,3,4,5,6,7-hexahydro-6,11b-(epiminoethano)naphtho[1,2-d]azepine-5a,10(1H)-diol

[Chemical Formula 248]

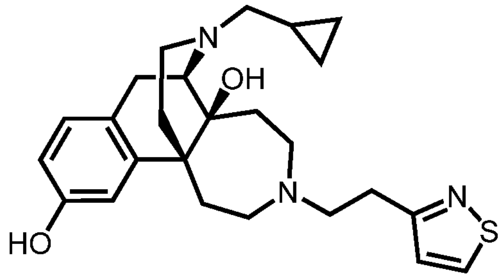

The title compound was obtained from the compound obtained in Example 208 according to the method described in Example 24.

$^1$H-NMR (400 MHz, $CDCl_3$)δ (ppm): 0.07 - 0.09 (m, 2H), 0.47 - 0.49 (m, 2H), 0.78 - 0.94 (m, 2H), 1.26 - 1.39 (m, 2H), 1.65 - 1.77 (m, 2H), 1.87 - 2.18 (m, 2H), 2.29 - 2.31 (m, 2H), 2.45 - 3.68 (m, 13H), 6.37 - 6.44 (m, 2H), 6.68 - 6.73 (m, 1H), 6.89 (d, J = 4 Hz, 1H), 8.42 (d, J = 4 Hz, 1H).

(Example 210)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-3-(thiazol-2-yl)propan-1-one

[Chemical Formula 249]

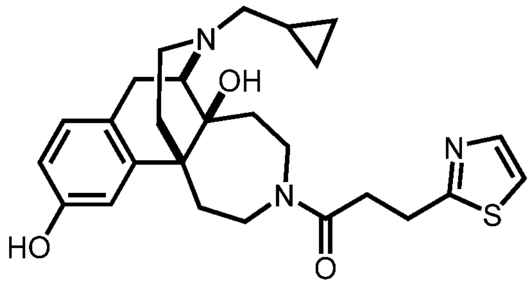

The title compound was obtained from the compound E obtained in Example 3 and 3-(thiazol-2-yl)propanoic acid according to the method described in Example 26.

$^1$H-NMR (400 MHz, $CDCl_3$)δ (ppm): 0.03 - 0.17 (m, 2H), 0.42 - 0.60 (m, 2H), 0.75 - 0.94 (m, 1H), 1.03 - 2.45 (m, 9H), 2.49 - 3.46 (m, 10H), 3.80 - 4.06 (m, 1.7H), 4.13 - 4.27 (m, 0.3H), 6.61 - 7.03 (m, 3H), 7.16 (d, J = 3 Hz, 0.3H), 7.21 (d, J = 3 Hz, 0.7H), 7.65 (d, J = 3 Hz, 0.3H), 7.72 (d, J = 3 Hz, 0.7H).

(Example 211)

Synthesis of (5aS,6R,11bS)-3-(3-(1H-pyrazol-1-yl)propyl)-14-(cyclopropylmethyl)-2,3,4,5,6,7-hexahydro-6,11b-(epiminoethano)naphtho[1,2-d]azepine-5a,10(1H)-diol

[Chemical Formula 250]

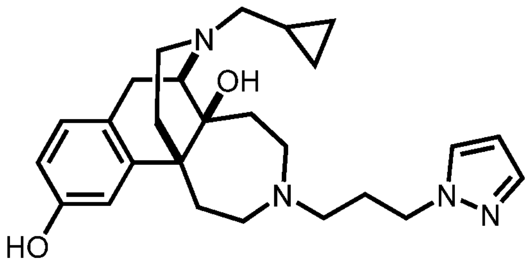

The title compound was obtained from the compound obtained in Example 157 according to the method described in Example 24.

$^1$H-NMR (400 MHz, $CDCl_3$)δ (ppm): 0.05 - 0.15 (m, 2H), 0.44 - 0.54 (m, 2H), 0.77 - 0.92 (m, 1H), 0.99 - 1.08 (m, 1H), 1.45 - 1.52 (m, 1H), 1.67 - 1.95 (m, 4H), 2.00 - 2.06 (m, 2H), 2.27 - 2.57 (m, 8H), 2.79 (dd, J = 6, 18 Hz, 1H), 2.87 - 3.02 (m, 4H), 3.77 - 3.94 (m, 2H), 4.69 (br s, 1H), 6.18 (dd, J = 1, 2 Hz, 1H), 6.57 (dd, J = 2, 8 Hz, 1H), 6.61 (d, J = 2 Hz, 1H), 6.90 (d, J = 8 Hz, 1H), 7.15 (d, J = 2 Hz, 1H), 7.48 (d, J = 1 Hz, 1H).

(Example 212)

Synthesis of (5aS,6R,11bR)-3-(3-(1H-pyrazol-1-yl)propyl)-14-(cyclopropylmethyl)-5a-hydroxy-10-methoxy-3,4,5,5a,6,7-hexahydro-6,11b-(epiminoethano)naphtho[1,2-d]azepin-2(1H)-one

[Chemical Formula 251]

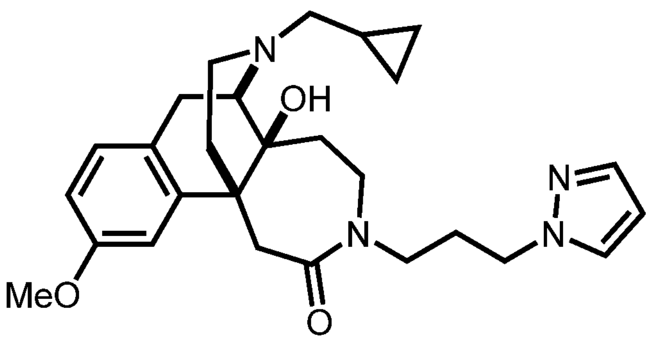

The title compound was obtained from the compound obtained in Example 201 and pyrazole according to the method described in Example 202.

$^1$H-NMR (400 MHz, $CDCl_3$)δ (ppm): 0.04 - 0.17 (m, 2H), 0.46 - 0.57 (m, 2H), 0.76 - 0.94 (m, 1H), 1.10 - 1.21 (m, 1H), 1.58 - 1.78 (m, 2H), 1.87 - 1.99 (m, 1H), 2.02 - 2.14 (m, 2H), 2.35 (dd, J = 6, 13 Hz, 1H), 2.36 (dd, J = 6, 13 Hz, 1H), 2.53 - 2.63 (m, 1H), 2.69 (d, J = 15 Hz, 1H), 2.77 (dd, J = 6, 18 Hz, 1H), 2.83 - 2.96 (m, 3H), 3.01 (d, J = 18 Hz, 1H), 3.43 - 3.60 (m, 3H), 3.62 - 3.72 (m, 1H), 3.77 (s, 3H), 4.11 (dd, J = 11, 15 Hz, 1H), 6.13 - 6.17 (m, 1H), 6.63 (dd, J = 2, 9 Hz, 1H), 6.94 (d, J = 8 Hz, 1H), 7.20 - 7.26 (m, 2H), 7.42 - 7.46 (m, 1H).

(Example 213)

Synthesis of (5aS,6R,11bR)-3-(3-(1H-pyrazol-1-yl)propyl)-14-(cyclopropylmethyl)-5a,10-dihydroxy-3,4,5,5a,6,7-hexahydro-6,11b-(epiminoethano)naphtho[1,2-d]azepin-2(1H)-one

[Chemical Formula 252]

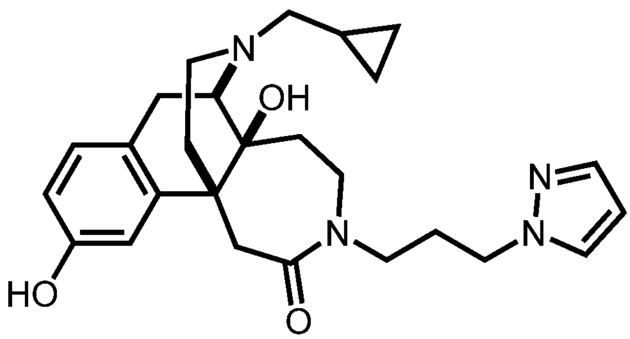

The title compound was obtained from the compound obtained in Example 212 according to the method described in Example 6.

$^1$H-NMR (400 MHz, $CDCl_3$)δ (ppm): 0.05 - 0.17 (m, 2H), 0.46 - 0.56 (m, 2H), 0.76 - 0.92 (m, 1H), 1.10 - 1.25 (m, 1H), 1.62 (dd, J = 6, 14 Hz, 1H), 1.66 - 1.82 (m, 2H), 1.90 - 2.16 (m, 3H), 2.35 (dd, J = 6, 13 Hz, 1H), 2.36 (dd, J = 6, 13 Hz, 1H), 2.52 - 2.62 (m, 1H), 2.67 (d, J = 15 Hz, 1H), 2.76 (dd, J = 6, 18 Hz, 1H), 2.85 - 2.95 (m, 3H), 3.00 (d, J = 18 Hz, 1H), 3.48 - 3.70 (m, 4H), 4.10 (dd, J = 11, 15 Hz, 1H), 6.11 - 6.15 (m, 1H), 6.62 (dd, J = 2, 8 Hz, 1H), 6.90 (d, J = 8 Hz, 1H), 7.23 - 7.28 (m, 2H), 7.42 - 7.46 (m, 1H).

(Example 214)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-2-(5-hydroxy-1H-pyrazol-1-yl)ethan-1-one

[Chemical Formula 253]

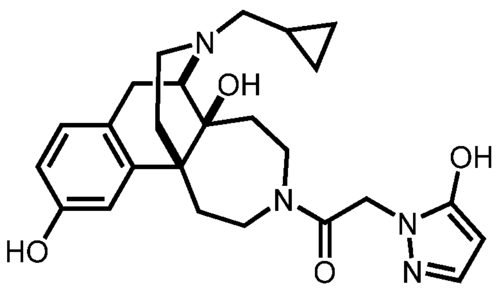

The title compound was obtained from the compound E obtained in Example 3 and 2,4-dihydro-3H-pyrazol-3-one according to the method described in Example 132.

$^1$H-NMR (400 MHz, $CDCl_3$)δ (ppm): 0.03 - 0.20 (m, 2H), 0.41 - 0.59 (m, 2H), 0.74 - 2.43 (m, 8H), 2.46 - 3.04 (m, 5H), 3.06 - 3.45 (m, 2H), 3.55 - 4.19 (m, 2.7H), 4.23 - 4.49 (m, 1H), 4.59 - 4.93 (m, 1.3H), 5.70 - 5.77 (m, 1H), 6.50 - 7.02 (m, 3H), 7.22 - 7.38 (m, 1H).

(Reference Example 22)

Synthesis of ethyl (E)-3-(5-methylisothiazol-3-yl)acrylate

[Chemical Formula 254]

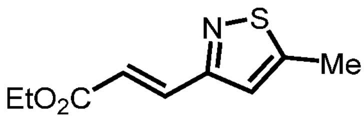

To a suspension of potassium tert-butoxide (106 mg, 0.94 mmol) in tetrahydrofuran (1 mL) was added dropwise triethyl phosphonoacetate (189 μL, 0.94 mmol) under ice cooling, followed by stirring at 0°C for 30 minutes. Thereafter, a solution of 5-methylisothiazole-3-carbaldehyde (100 mg, 0.79 mmol) in THF (1 mL) was added dropwise, followed by stirring at room temperature for 5 hours. Under ice cooling, to the reaction mixture was added a saturated aqueous ammonium chloride solution, followed by extraction three times with ethyl acetate. The combined extracts were dried over sodium sulfate and then concentrated under reduced pressure. The obtained crude product was purified by silica gel column chromatography (1 to 10% ethyl acetate/heptane) to yield the title compound (143 mg, 92%) as a colorless oily matter.

$^1$H-NMR (400 MHz, $CDCl_3$)δ (ppm): 1.33 (t, J = 7 Hz, 3H), 2.59 (s, 3H), 4.27 (q, J = 7 Hz, 2H), 6.56 (d, J = 16 Hz, 1H), 7.15 (s, 1H), 7.65 (d, J = 16 Hz, 1H).

(Reference Example 23)

Synthesis of (E)-3-(5-methylisothiazol-3-yl)acrylic acid

[Chemical Formula 255]

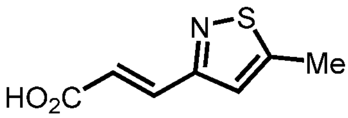

To a solution of the compound (143 mg, 0.73 mmol) obtained in Reference Example 22 in tetrahydrofuran (1 mL) was added a 2 M aqueous sodium hydroxide solution (0.73 mL, 1.45 mmol), followed by stirring at room temperature for 5 hours. To the reaction mixture, 2 M hydrochloric acid (0.73 mL, 1.45 mmol) was added, followed by extraction three times with ethyl acetate. The combined extracts were dried over sodium sulfate and then concentrated under reduced pressure to yield the title compound (122 mg, 99%) as a white powder.

$^1$H-NMR (400 MHz, DMSO - $d_6$)δ (ppm): 2.57 (s, 3H), 6.61 (d, J = 16 Hz, 1H), 7.42 (d, J = 16 Hz, 1H), 7, 58 (s, 1H), 12.71 (br s, 1H).

(Example 215)

Synthesis of (E)-1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-3-(5-methylisothiazol-3-yl)prop-2-en-1-one

[Chemical Formula 256]

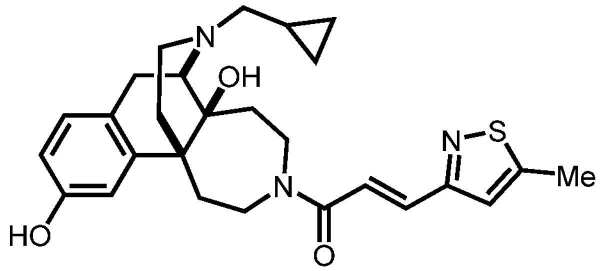

The title compound was obtained from the compound E obtained in Example 3 and the compound obtained in Reference Example 23 according to the method described in Example 26.

$^1$H-NMR (400 MHz, $CDCl_3$)$\delta$ (ppm): 0.03 - 0.18 (m, 2H), 0.41 - 0.57 (m, 2H), 0.75 - 0.93 (m, 1H), 1.02 - 2.16 (m, 5.6H), 2.18 - 2.42 (m, 3H), 2.46 - 3.08 (m, 7.4H), 3.11 - 3.24 (m, 0.6H), 3.38 - 3.62 (m, 1.4H), 3.85 - 4.00 (m, 0.6H), 4.04 - 4.17 (m, 0.8H), 4.29 - 4.40 (m, 0.6H), 6.58 (dd, J = 2, 8 Hz, 0.4H), 6.65 - 6.73 (m, 1H), 6.84 - 7.12 (m, 3H), 7.13 - 7.24 (m, 1H), 7.55 (d, J = 16 Hz, 0.6H).

(Example 216)

Synthesis of (5aS,6R,11bS)-14-(cyclopropylmethyl)-3-(3,3,3-trifluoro-2-phenylpropyl)-2,3,4,5,6,7-hexahydro-6,11b-(epiminoethano)naphtho[1,2-d]azepine-5a,10(1H)-diol

[Chemical Formula 257]

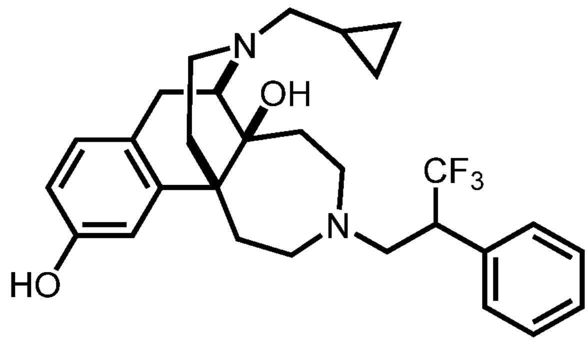

The title compound was obtained from the compound E obtained in Example 3 and 3,3,3-trifluoro-2 phenylpropanoic acid according to the methods described in Example 5 and Example 24.

$^1$H-NMR (400 MHz, $CDCl_3$)$\delta$ (ppm): 0.04 - 0.11 (m, 2H), 0.44 - 0.52 (m, 2H), 0.75 - 0.86 (m, 1H), 0.94 - 1.01 (m, 1H), 1.23 - 1.33 (m, 1H), 1.48 (ddd, J = 4, 11, 14 Hz, 1H), 1.73 (ddd, J = 4, 4, 15 Hz, 1H), 1.90 - 2.06 (m, 2H), 2.20 - 2.38 (m, 4H), 2.48 - 2.54 (m, 1H), 2.59 - 2.68 (m, 2H), 2.83 (d, J = 6 Hz, 1H), 2.86 - 2.97 (m, 1H), 2.90 (d, J = 18 Hz, 1H), 2.96 (dd, J = 9, 13 Hz, 1H), 3.09 (ddd, J = 2, 11, 13 Hz, 1H), 3.15 (dd, J = 5, 13 Hz, 1H), 3.26 (ddq, J = 5, 9, 9 Hz, 1H), 6.52 (d, J = 2 Hz, 1H), 6.60 (dd, J = 2, 8 Hz, 1H), 6.93 (d, J = 8 Hz, 1H), 7.02 - 7.08 (m, 2H), 7.21 - 7.31 (m, 3H).

(Example 217)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-3-(5-methylisothiazol-3-yl)propan-1-one

[Chemical Formula 258]

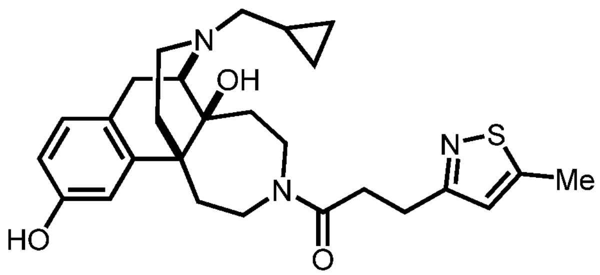

To a solution of the compound (14 mg, 0.029 mmol) obtained in Example 215 in methanol (1 mL) was added 10% palladium-activated carbon (55% wet, 8.5 mg), followed by stirring under a hydrogen atmosphere at room temperature for 10 days. The reaction mixture was filtered through a membrane filter, and the filtrate was concentrated under reduced pressure. The obtained crude product was purified by preparative thin layer chromatography (chloroform : 2 M ammonia-methanol solution = 10 : 1) to yield the title compound and a raw material compound (compound described in Example 215) (6.6 mg) as a colorless gumlike substance. To the obtained product dissolved in a methanol (1 mL) solution, 10% palladium-activated carbon (55% wet, 13.2 mg) was added, followed by stirring at room temperature for 19 hours under a hydrogen atmosphere. The reaction mixture was filtered through a membrane filter, and the filtrate was concentrated under reduced pressure. The obtained crude product was purified by silica gel column chromatography (amino group-supported silica gel, 0 to 5% methanol/chloroform) to yield the title compound (5.4 mg, 39%) as a white solid.

$^1$H-NMR (400 MHz, $CDCl_3$)δ (ppm): 0.04 - 0.19 (m, 2H), 0.43 - 0.59 (m, 2H), 0.76 - 3.27 (m, 22H), 3.28 - 3.44 (m, 1H), 3.80 - 4.04 (m, 1.4H), 4.16 - 4.33 (m, 0.6H), 6.57 - 7.11 (m, 4H).

(Example 218)

Synthesis of (5aS,6R,11bS)-14-((2,2-difluorocyclopropyl)methyl)-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 259]

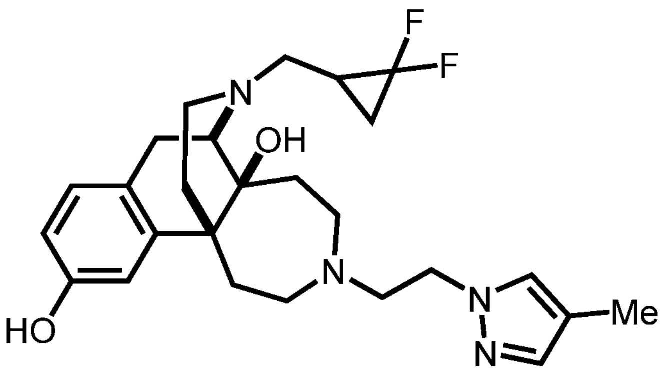

The title compound was obtained from the compound obtained in Example 176 and 2-(bromomethyl)-1,1-difluorocyclopropane according to the method described in Example 161.

$^1$H-NMR (400 MHz, $CDCl_3$)δ (ppm): 0.95 - 1.10 (m, 2H), 1.38 - 1.53 (m, 2H), 1.58 - 1.86 (m, 3H), 1.96 - 2.20 (m, 2H), 2.00 (s, 3H), 2.28 - 3.02 (m, 12H), 3.09 - 3.19 (m, 1H), 3.95 - 4.09 (m, 2H), 6.57 (d, J = 3 Hz, 1H), 6.63 (dd, J = 2, 8 Hz, 1H), 6.86 - 6.88 (m, 1H), 6.94 (d, J = 8 Hz, 0.5H), 6.95 (d, J = 8 Hz, 0.5H), 7.24 (s, 1H).

(Example 219)

Synthesis of (5aS,6R,11bS)-14-(((1S,2S)-2-fluorocyclopropyl)methyl)-3-(2-(4-methyl-1H-pyrazol-1-yl)ethyl)-2,3,4,5,6,7-hexahydro-6,11b-(epiminoethano)naphtho[1,2-d]azepine-5a, 10(1H)-diol

[Chemical Formula 260]

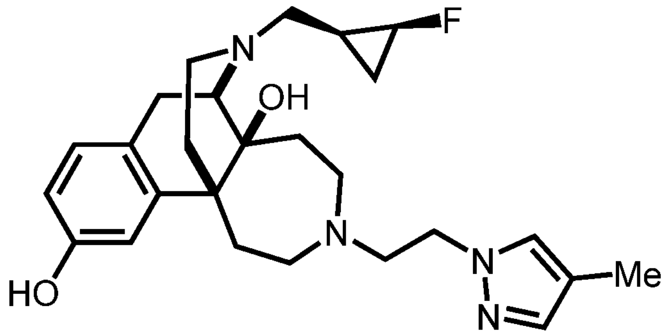

To the compound (15.3 mg, 0.040 mmol) obtained in Example 176 were added (1S,2S)-2 fluorocyclopropane-1-carboxylic acid (5.0 mg, 0.048 mmol), O-(7-azabenzotriazol-1-yl)-N,N,N',N'-tetramethyluronium hexafluorophosphate (HATU) (18.3 mg, 0.048 mmol), N,N-dimethylformamide (1 mL), and N,N-diisopropylethylamine (17 μL, 0.10 mmol), followed by stirring at room temperature for 1 hour. To the reaction mixture, a saturated sodium bicarbonate aqueous solution was added, followed by extraction three times with chloroform. The combined extracts were dried over sodium sulfate and then concentrated under reduced pressure. The obtained crude product was used for the next reaction without purification.

To a solution of the obtained crude product in tetrahydrofuran (0.5 mL) was added a 0.91 M borane-tetrahydrofuran complex-tetrahydrofuran solution (0.44 mL, 0.40 mmol), followed by heating under reflux for 6 hours. After allowed to cool, the reaction mixture was concentrated under reduced pressure. To the concentrated residue, 6 M hydrochloric acid (2 mL) was added, followed by heating under reflux for 1 hour. After allowed to cool, water and potassium carbonate were added to make the reaction mixture basic, followed by extraction three times with chloroform. The combined extracts were dried over sodium sulfate and then concentrated under reduced pressure. The obtained crude product was purified by preparative thin layer chromatography (chloroform : 2 M ammonia-methanol solution = 10 : 1) to yield the title compound (9.0 mg, 49%) as a colorless amorphous form.

$^1$H-NMR (400 MHz, $CDCl_3$)δ (ppm): 0.60 (dddd, J = 3, 7, 7, 23 Hz, 1H), 0.73 - 0.85 (m, 1H), 0.90 - 1.10 (m, 2H), 1.38 - 1.48 (m, 1H), 1.72 - 1.85 (m, 2H), 1.96 - 2.09 (m, 2H), 1.99 (s, 3H), 2.29 - 2.40 (m, 1H), 2.49 - 2.66 (m, 4H), 2.68 (dd, J = 6, 13 Hz, 1H), 2.75 - 3.04 (m, 6H), 3.16 (dd, J = 11, 11 Hz, 1H), 3.95 - 4.09 (m, 2H), 4.55 (dddd, J = 3, 6, 6, 65 Hz, 1H), 6.53 (d, J = 3 Hz, 1H), 6.61 (dd, J = 3, 8 Hz, 1H), 6.87 (s, 1H), 6.93 (d, J = 8 Hz, 1H), 7.24 (s, 1H).

(Example 220)

Synthesis of 1-((5aS,6R,11bR)-14-(cyclopropylmethyl)-5a,10-dihydroxy-1,2,5,5a,6,7-hexahydro-6,11b-(epiminoethano)naphtho[1,2-d]azepin-3(4H)-yl)-3-(4-methyl-1H-pyrazol-1-yl)propan-1-one

[Chemical Formula 261]

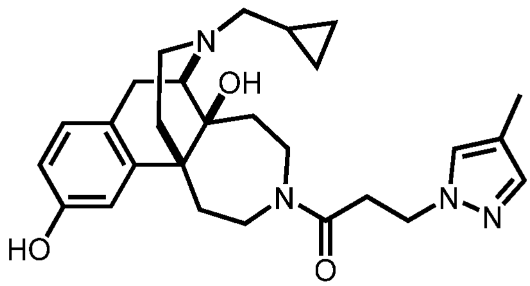

The title compound was obtained from the compound obtained in Example 3 and 3-(4-methyl-1H-pyrazol-1-yl)propanoic acid hydrochloride according to the method described in Example 26.

$^1$H-NMR (400 MHz, $CDCl_3$)δ (ppm): 0.07 - 0.14 (m, 2H), 0.47 - 0.53 (m, 2H), 0.76 - 0.88 (m, 1H), 1.05 - 1.13 (m, 1H), 1.51 - 1.61 (m, 1H), 1.76 - 1.99 (m, 2H), 2.00 - 2.23 (m, 2.3H), 2.03 (s, 0.9H), 2.04 (s, 2.1H), 2.27 - 2.45 (m,

2.7H), 2.49 - 2.59 (m, 1H), 2.66 - 3.02 (m, 5H), 3.10 - 3.23 (m, 1.3H), 3.27 - 3.33 (m, 0.7H), 3.60 - 3.69 (m, 0.7H), 3.70 - 3.79 (m, 0.3H), 3.85 - 4.06 (m, 1.7H), 4.11 - 4.20 (m, 0.6H), 4.31 - 4.37 (m, 0.7H), 4.43 (br s, 0.3H), 4.53 (br s, 0.7H), 6.67 (dd, J = 3, 8 Hz, 0.3H), 6.72 (dd, J = 3, 8 Hz, 0.7H), 6.75 (d, J = 3 Hz, 0.3H), 6.79 (d, J = 3 Hz, 0.7H), 6.92 (d, J = 8 Hz, 0.7H), 6.93 (d, J = 8 Hz, 0.3H), 7.15 (s, 0.7H), 7.19 (s, 0.3H), 7.25 - 7.28 (m, 0.3H), 7.34 (s, 0.7H).

(Example 221)

Synthesis of (5aS,6R,11bS)-3-(2-(4-methyl-1H-pyrazol-1-yl)ethyl)-14-((1-methylcyclopropyl)methyl)-2,3,4,5,6,7-hexahydro-6,11b-(epiminoethano)naphtho[1,2-d]azepine-5a,10(1H)-diol

[Chemical Formula 262]

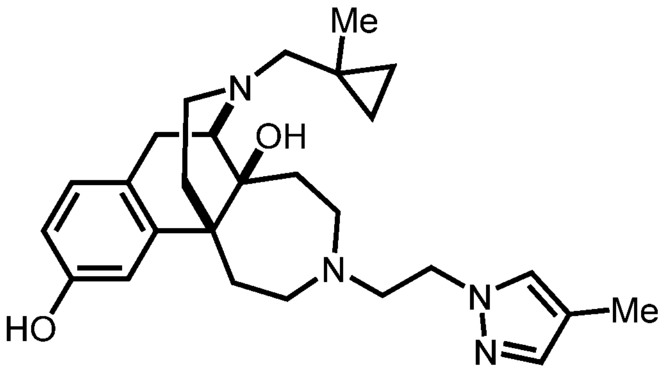

The title compound was obtained from the compound obtained in Example 176 and 1-methylcyclopropane-1 carboxylic acid according to the method described in Example 219.

$^1$H-NMR (400 MHz, $CDCl_3$)δ (ppm): 0.02 - 0.32 (m, 4H), 0.99 - 1.10 (m, 1H), 1.07 (s, 3H), 1.39 - 1.49 (m, 1H), 1.70 - 1.81 (m, 2H), 1.90 - 2.08 (m, 2H), 1.99 (s, 3H), 2.21 (d, J = 12 Hz, 1H), 2.35 - 2.45 (m, 1H), 2.38 (d, J = 12 Hz, 1H), 2.48 - 2.65 (m, 3H), 2.70 - 3.00 (m, 6H), 3.11 - 3.21 (m, 1H), 3.95 - 4.09 (m, 2H), 6.54 (d, J = 3 Hz, 1H), 6.61 (dd, J = 3, 8 Hz, 1H), 6.85 (s, 1H), 6.92 (d, J = 8 Hz, 1H), 7.23 (s, 1H).

(Example 222)

Synthesis of diastereomric mixture (1:1) of ethyl (S)-3-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-4,4,4-trifluorobutanoate and ethyl (R)-3-((5aS,6R,11bR)-14-(cyclopropylmethyl)-5a-hydroxy-10-methoxy-1,2,5,5a,6,7-hexahydro-6,11b-(epiminoethano)naphtho[1,2-d]azepin-3(4H)-yl)-4,4,4-trifluorobutanoate

[Chemical Formula 263]

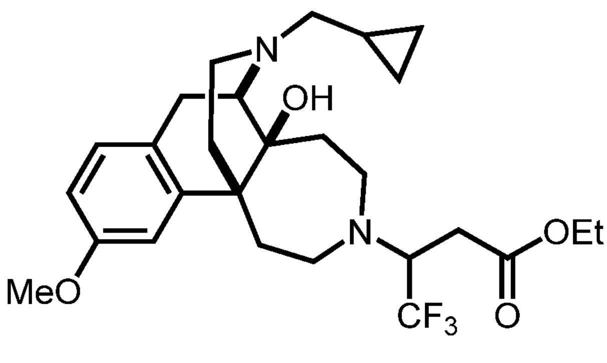

To a solution of the compound C (98.2 mg, 0.287 mmol) obtained in Example 2 in acetonitrile (3.0 mL) were

added ethyl (E)-4,4,4-trifluorocrotonate (210 pL, 1.46 mmol) and cesium carbonate (467 mg, 1.43 mmol), followed by stirring at 85°C for 2 hours. Water (10 mL) was added to the reaction mixture under ice cooling, followed by extraction three times with ethyl acetate. The organic layers were combined, washed with saturated saline, dried over sodium sulfate, and then concentrated under reduced pressure. The obtained crude product was purified by silica gel column chromatography (5 to 20% methanol/ethyl acetate) to yield the title compound (154 mg, quantitative) as a yellow amorphous form.

$^1$H-NMR (400 MHz, $CDCl_3$)$\delta$ (ppm): 0.05 - 0.14 (m, 2H), 0.44 - 0.56 (m, 2H), 0.76 - 0.89 (m, 1H), 0.97 - 1.04 (m, 1H), 1.15 - 1.37 (m, 2H), 1.46 - 1.56 (m, 1H), 1.73 (ddd, J = 3, 11, 14 Hz, 0.5H), 1.79 (ddd, J = 3, 10, 14 Hz, 0.5H), 1.84 - 2.11 (m, 3H), 2.28 - 2.73 (m, 6.5H), 2.74 - 3.10 (m, 6H), 3.24 (dd, J = 11, 11 Hz, 0.5H), 3.34 (dd, J = 12, 12 Hz, 0.5H), 3.55 - 3.75 (m, 1H), 3.78 (s, 1.5H), 3.78 (s, 1.5H), 3.81 - 4.07 (m, 1.5H), 4.16 - 4.25 (m, 1H), 6.62 - 6.65 (m, 1H), 6.67 (dd, J = 3, 8 Hz, 0.5H), 6.68 (dd, J = 2, 8 Hz, 0.5H), 6.98 (d, J = 8 Hz, 0.5H), 6.99 (d, J = 8 Hz, 0.5H).

(Example 223)

Synthesis of (5aS,6R,11bR)-14-(cyclopropylmethyl)-3-(1,1,1-trifluoro-4-(4-methyl-1H-pyrazol-1-yl)butan-2-yl)-2,3,4,5,6,7-hexahydro-6,11b-(epiminoethano)naphtho[1,2-d]azepine-5a,10(1H)-diol (diastereomer I) and (5aS,6R,11bR)-14-(cyclopropylmethyl)-3-(1,1,1-trifluoro-4-(4-methyl-1H-pyrazol-1-yl)butan-2-yl)-2,3,4,5,6,7-hexahydro-6,11b-(epiminoethano)naphtho[1,2-d]azepine-5a,10(1H)-diol (diastereomer II)

[Chemical Formula 264]

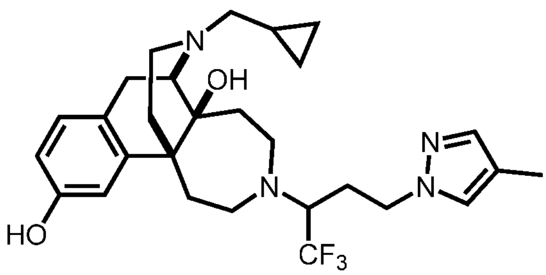

diastereomer I

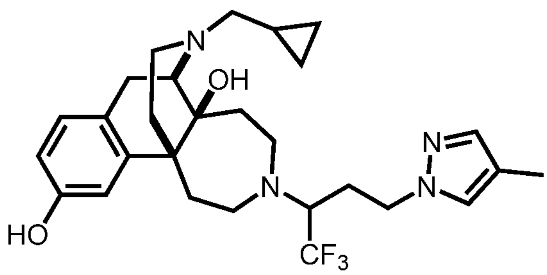

diastereomer II

To a solution of the compound (154 mg, 0.301 mmol) obtained in Example 222 in tetrahydrofuran (3.0 mL) was added lithium borohydride (4 M tetrahydrofuran solution, 380 pL, 1.52 mmol), followed by stirring at room temperature for 5 hours. Thereafter, lithium borohydride (4 M tetrahydrofuran solution, 380 pL, 1.52 mmol) was added, followed by stirring at room temperature for 2 hours. Water (10 mL) was added to the reaction mixture under ice cooling, followed by extraction three times with ethyl acetate. The organic layers were combined, washed with saturated saline, dried over sodium sulfate, and then concentrated under reduced pressure. To a solution of the obtained crude product in tetrahydrofuran (3.0 mL) was added lithium borohydride (4 M tetrahydrofuran solution, 800 pL, 3.20 mmol), followed by stirring at room temperature for 18 hours. Under ice cooling, to the reaction mixture were added water (3 mL) and a saturated aqueous ammonium chloride solution (10 mL), followed by extraction three times with ethyl acetate. The organic layers were combined, washed with saturated saline, dried over sodium sulfate, and then concentrated under reduced pressure.

To a solution of the obtained crude product in chloroform (1.0 mL) were added N,N-diisopropylethylamine (31.2 pL, 179 pmol) and methanesulfonic anhydride (16.0 mg, 91.9 pmol) at 0°C, followed by stirring at 0°C for 2.5 hours. Thereafter, methanesulfonic anhydride (6.0 mg, 34.4 pmol) was added, followed by stirring at 0°C for 40 minutes. To the reaction mixture were added a saturated aqueous sodium bicarbonate solution (3 mL) and water (2 mL), followed by extraction three times with ethyl acetate. The organic layers were combined, washed with saturated saline, dried over sodium sulfate, and then concentrated under reduced pressure.

To a solution of the obtained crude product in acetonitrile (1.0 mL) were added N,N-diisopropylethylamine (15.6 pL, 89.6 pmol) and 4-methyl-1H-pyrazole (5.6 pL, 67.7 pmol), followed by stirring at room temperature for 18 hours. Thereafter, N,N-diisopropylethylamine (15.6 pL, 89.6 pmol) and 4-methylpyrazole (6.0 pL, 72.6 pmol) were added, followed by stirring at 70°C for 6.5 hours. After cooled to room temperature, to the reaction mixture was added a saturated aqueous sodium bicarbonate solution (3 mL), followed by extraction three times with ethyl acetate. The organic layers were combined, washed with saturated saline, dried over sodium sulfate, and then concentrated under reduced pressure.

The title compounds (diastereomer I (4.3 mg, 4 steps 16%) and diastereomer II (1.8 mg, 4 steps 7%)) were individually obtained from the obtained crude product according to the method described in Example 6.

(Diastereomer I)

$^1$H-NMR (400 MHz, $CDCl_3$)δ (ppm): 0.06 - 0.16 (m, 2H), 0.46 - 0.57 (m, 2H), 0.77 - 0.89 (m, 1H), 1.03 - 1.10 (m, 1H), 1.53 (dd, J = 4, 13 Hz, 1H), 1.74 - 2.12 (m, 6H), 2.04 (s, 3H), 2.33 (dd, J = 7, 13 Hz, 1H), 2.34 (dd, J = 6, 13 Hz, 1H), 2.43 - 2.68 (m, 3H), 2.69 - 2.81 (m, 2H), 2.85 (dd, J = 6, 18 Hz, 1H), 2.96 (d, J = 6 Hz, 1H), 3.00 (d, J = 18 Hz, 1H), 3.05 - 3.20 (m, 2H), 3.34 - 3.45 (m, 2H), 4.55 (br s, 1H), 6.52 (dd, J = 2, 8 Hz, 1H), 6.77 (d, J = 2 Hz, 1H), 6.78 (s, 1H), 6.93 (d, J = 8 Hz, 1H), 7.03 (s, 1H).

(Diastereomer II)

$^1$H-NMR (400 MHz, $CDCl_3$)δ (ppm): 0.06 - 0.17 (m, 2H), 0.48 - 0.54 (m, 2H), 0.78 - 0.89 (m, 1H), 1.02 - 1.12 (m, 1H), 1.51 - 1.72 (m, 2H), 1.82 (ddd, J = 2, 6, 16 Hz, 1H), 1.87 - 1.97 (m, 1H), 1.98 - 2.09 (m, 3H), 2.06 (s, 3H), 2.34 (dd, J = 7, 13 Hz, 1H), 2.34 (dd, J = 7, 13 Hz, 1H), 2.49 - 2.68 (m, 3H), 2.78 - 2.89 (m, 2H), 2.92 - 3.07 (m, 2H), 2.94 (d, J = 6 Hz, 1H), 3.00 (d, J = 18 Hz, 1H), 3.18 - 3.26 (m, 1H), 3.34 (ddd, J = 5, 10, 19 Hz, 1H), 3.46 - 3.54 (m, 1H), 4.53 (br s, 1H), 6.61 (dd, J = 2, 8 Hz, 1H), 6.78 (d, J = 2 Hz, 1H), 6.94 (d, J = 8 Hz, 1H), 6.96 (s, 1H), 7.28 (s, 1H).

(Example 224)

Synthesis of (5aS,6R,11bS)-3-(2-(4-methyl-1H-pyrazol-1-yl)ethyl)-14-(3-methylbut-2-en-1-yl)-2,3,4,5,6,7-hexahydro-6,11b-(epiminoethano)naphtho[1,2-d]azepine-5a,10(1H)-diol

[Chemical Formula 265]

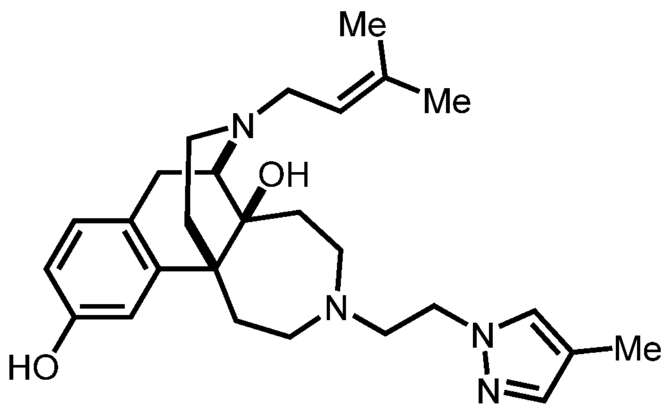

The title compound was obtained from the compound obtained in Example 176 and 1-bromo-3-methylbut-2-ene according to the method described in Example 31.

$^1$H-NMR (400 MHz, $CDCl_3$)δ (ppm): 0.96 - 1.04 (m, 1H), 1.38 - 1.46 (m, 1H), 1.64 (s, 3H), 1.67 - 1.81 (m, 2H), 1.72 (s, 3H), 1.93 - 2.06 (m, 2H), 1.99 (s, 3H), 2.29 - 2.43 (m, 2H), 2.46 - 2.63 (m, 2H), 2.67 - 3.08 (m, 8H), 3.09 - 3.19 (m, 1H), 3.94 - 4.07 (m, 2H), 4.56 (br s, 1H), 5.12 - 5.19 (m, 1H), 6.54 (d, J = 3 Hz, 1H), 6.61 (dd, J = 3, 8 Hz, 1H), 6.85 (s, 1H), 6.92 (d, J = 8 Hz, 1H), 7.23 (s, 1H).

(Example 225)

Synthesis of (5aS,6R,11bS)-14-((1-hydroxycyclopropyl)methyl)-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 266]

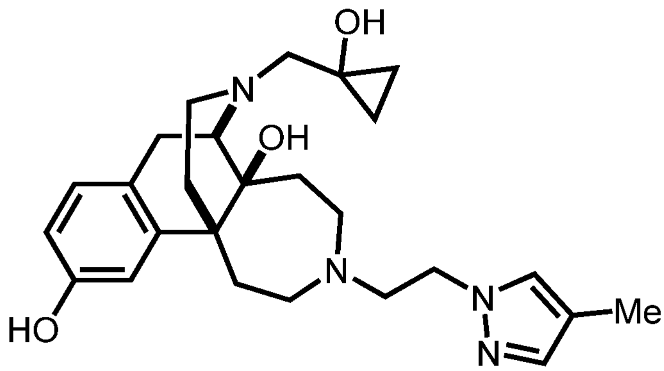

The title compound was obtained from the compound obtained in Example 176 and 1-hydroxycyclopropane-1 carboxylic acid according to the method described in Example 219.

$^1$H-NMR (400 MHz, $CDCl_3$)δ (ppm): 0.35 - 0.42 (m, 2H), 0.78 - 0.84 (m, 2H), 1.02 - 1.09 (m, 1H), 1.46 - 1.55 (m, 1H), 1.76 - 1.89 (m, 1H), 1.90 - 2.23 (m, 4H), 1.99 (s, 3H), 2.42 - 3.00 (m, 11H), 3.09 (ddd, J = 4, 12, 12 Hz, 1H), 4.00 - 4.09 (m, 2H), 6.58 - 6.66 (m, 2H), 6.94 (d, J = 8 Hz, 1H), 6.96 (s, 1H), 7.25 - 7.28 (m, 1H).

(Example 226)

Synthesis of (5aS,6R,11bS)-14-((1-fluorocyclopropyl)methyl)-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 267]

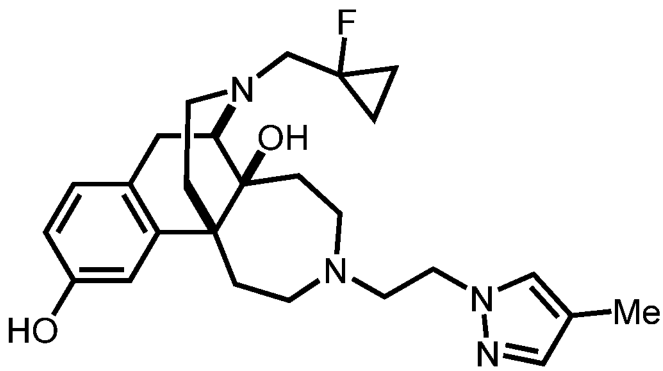

The title compound was obtained from the compound obtained in Example 176 and 1-fluorocyclopropane-1 carboxylic acid according to the method described in Example 219.

$^1$H-NMR (400 MHz, $CDCl_3$)δ (ppm): 0.52 - 0.67 (m, 2H), 0.97 - 1.13 (m, 3H), 1.40 - 1.52 (m, 1H), 1.71 - 1.82 (m, 2H), 2.00 (s, 3H), 2.06 (ddd, J = 5, 13, 13 Hz, 1H), 2.17 (ddd, J = 3, 12, 12 Hz, 1H), 2.37 (ddd, J = 4, 11, 11 Hz, 1H), 2.42 - 2.65 (m, 3H), 2.68 - 3.05 (m, 8H), 3.10 - 3.20 (m, 1H), 3.95 - 4.09 (m, 2H), 4.52 (br s, 1H), 6.55 (d, J = 2 Hz, 1H), 6.62 (dd, J = 2, 8 Hz, 1H), 6.87 (s, 1H), 6.95 (d, J = 8 Hz, 1H), 7.24 (s, 1H).

(Reference Example 24)

Synthesis of 3-(4-methyl-1H-pyrazol-1-yl)cyclobutan-1-one

[Chemical Formula 268]

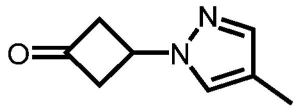

To a solution of 4-methylpyrazole (81.9 mg, 0.997 mmol) in N,N-dimethylformamide (2.5 mL) was added sodium hydride (55% oil dispersion, 43.8 mg, 1.00 mmol), followed by stirring at 70°C for 1 hour. After allowed to cool to room temperature, to the resultant solution was added a solution of 3-bromocyclobutan-1-one (175 mg, 1.17 mmol) in N,N-dimethylformamide (1.0 mL), followed by stirring at room temperature for 20 hours. Water was added to the reaction mixture, followed by extraction three times with ethyl acetate. The combined extracts were washed with saturated saline, dried over sodium sulfate, and then concentrated under reduced pressure. The obtained crude product was used for the next reaction without purification.

$^1$H-NMR (400 MHz, $CDCl_3$)δ (ppm): 2.09 (s, 3H), 3.48 - 3.57 (m, 2H), 3.71 - 3.80 (m, 2H), 4.92 - 4.99 (m, 1H), 7.29 (s, 1H), 7.39 (s, 1H).

(Example 227)

Synthesis of (5aS,6R,11bS)-14-(cyclopropylmethyl)-3-(3-(4-methyl-1H-pyrazol-1-yl)cyclobutyl)-2,3,4,5,6,7-hexahydro-6,11b-(epiminoethano)naphtho[1,2-d]azepine-5a,10(1H)-diol(diastereomer I) and (5aS,6R,11bS)-14-(cyclopropylmethyl)-3-(3-(4-methyl-1H-pyrazol-1-yl)cyclobutyl)-2,3,4,5,6,7-hexahydro-6, 11b-(epiminoethano)naphtho[1,2-d]azepine-5a, 10(1H)-diol(diastereomer II)

[Chemical Formula 269]

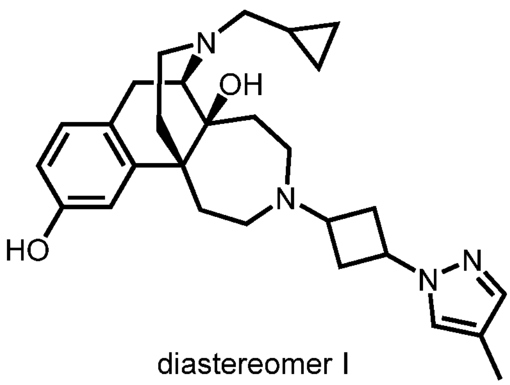

diastereomer I

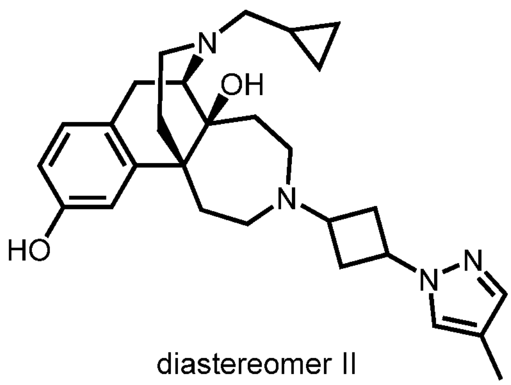

diastereomer II

The title compound was obtained from the compound E obtained in Example 3 and the compound obtained in Reference Example 24 according to the method described in Example 12.

(Diastereomer I)

$^1$H-NMR (400 MHz, $CDCl_3$)δ (ppm): 0.06 - 0.12 (m, 2H), 0.47 - 0.52 (m, 2H), 0.78 - 0.88 (m, 1H), 0.99 - 1.03 (m, 1H), 1.46 - 1.52 (m, 1H), 1.76 - 1.85 (m, 2H), 1.94 - 2.09 (m, 5H), 2.17 - 2.39 (m, 4H), 2.51 - 2.55 (m, 1H), 2.58 - 2.79 (m, 6H), 2.88 - 3.00 (m, 3H), 3.05 - 3.11 (m, 1H), 4.35 - 4.43 (m, 1H), 4.75 (br s, 1H), 6.54 (d, J = 2 Hz, 1H), 6.61 (dd, J = 2, 8 Hz, 1H), 6.92 (d, J = 8 Hz, 1H), 7.17 (s, 1H), 7.25 (s, 1H).

(Diastereomer II)

$^1$H-NMR (400 MHz, $CDCl_3$)δ (ppm): 0.07 - 0.11 (m, 2H), 0.47 - 0.52 (m, 2H), 0.79 - 0.90 (m, 3H), 1.01 - 1.04 (m, 1H), 1.48 (dd, J = 4, 15 Hz, 1H), 1.78 - 1.83 (m, 2H), 1.96 - 2.08 (m, 5H), 2.28 - 2.37 (m, 3H), 2.42 - 2.55 (m, 2H), 2.59 - 2.68 (m, 2H), 2.72 - 2.79 (m, 2H), 2.89 (d, J = 6 Hz, 1H), 2.95 (d, J = 18 Hz, 1H), 3.03 - 3.09 (m, 1H), 3.45 - 3.52 (m, 1H), 4.52 - 4.58 (m, 1H), 4.76 (br s, 1H), 6.55 (d, J = 2 Hz, 1H), 6.59 (dd, J = 2, 8 Hz, 1H), 6.91 (d, J = 8 Hz, 1H), 7.17 (s, 1H), 7.34 (s, 1H).

(Reference Example 25)

Synthesis of (R)-1-(4-methyl-1H-pyrazol-1-yl)propan-2-ol

[Chemical Formula 270]

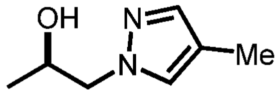

To a suspension of sodium hydride (55% oil dispersion, 79 mg, 1.8 mmol) in N,N-dimethylformamide (5 mL) was added dropwise (R)-2-methyloxirane (71 pL, 1.0 mmol) under ice cooling, followed by stirring at room temperature for 30 minutes. Thereafter, 4-methyl-1H-pyrazole (100 μL, 1.2 mmol) was added dropwise under ice cooling, followed by stirring at room temperature for 18 hours. Under ice cooling, to the reaction mixture was added a saturated aqueous ammonium chloride solution, followed by extraction three times with ethyl acetate. The combined extracts were washed with water and saturated saline, dried over sodium sulfate, and then concentrated under reduced pressure. The obtained crude product was purified by silica gel column chromatography (20 to 40% ethyl acetate/heptane) to yield the title compound (35 mg, 25%) as a pale yellow oily matter.

$^1$H-NMR (400 MHz, $CDCl_3$)δ (ppm): 1.21 (d, J = 6 Hz, 3H), 2.08 (s, 3H), 3.57 (d, J = 3 Hz, 1H), 3.92 (dd, J = 8, 14 Hz, 1H), 4.05 - 4.23 (m, 2H), 7.18 (s, 1H), 7.33 (s, 1H).

(Example 228)

Synthesis of (5aS,6R,11bS)-14-(cyclopropylmethyl)-3-((S)-1-(4-methyl-1H-pyrazol-1-yl)propan-2-yl)-2,3,4,5,6,7-hexahydro-6,11b-(epiminoethano)naphtho[1,2-d]azepine-5a,10(1H)-diol

[Chemical Formula 271]

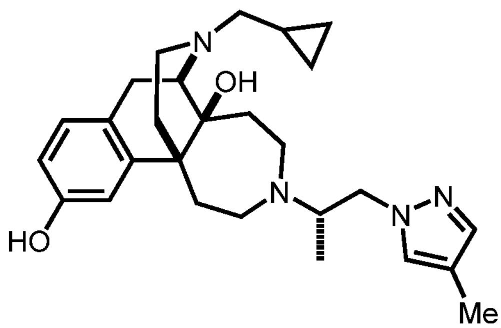

The title compound was obtained from the compound E obtained in Example 3 and the compound obtained in Reference Example 25 according to the method described in Example 32.

$^1$H-NMR (400 MHz, $CDCl_3$)δ (ppm): 0.02 - 0.17 (m, 2H), 0.42 - 0.57 (m, 2H), 0.75 - 0.93 (m, 4H), 0.97 - 1.07 (m, 1H), 1.45 - 1.56 (m, 1H), 1.74 (ddd, J = 3, 11, 14 Hz, 1H), 1.82 - 2.12 (m, 6H), 2.24 - 2.40 (m, 3H), 2.43 - 2.59 (m, 2H), 2.62 - 3.23 (m, 7H), 3.77 (dd, J = 7, 14 Hz, 1H), 3.98 (dd, J = 6, 14 Hz, 1H), 6.57 - 6.66 (m, 2H), 6.84 (s, 1H), 6.95 (d, J = 8 Hz, 1H), 7.23 (s, 1H).

(Reference Example 26)

Synthesis of (S)-1-(4-methyl-1H-pyrazol-1-yl)propan-2-ol

[Chemical Formula 272]

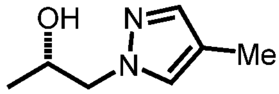

The title compound was obtained from (S)-2-methyloxirane and 4-methyl- 1H-pyrazole according to the method described in Reference Example 25.

$^1$H-NMR (400 MHz, $CDCl_3$)δ (ppm): 1.20 (d, J = 6 Hz, 3H), 2.08 (s, 3H), 3.58 (br s, 1H), 3.92 (dd, J = 8, 14 Hz, 1H), 4.05 - 4.22 (m, 2H), 7.18 (s, 1H), 7.33 (s, 1H).

(Example 229)

Synthesis of (5aS,6R,11bS)-14-(cyclopropylmethyl)-3-((R)-1-(4-methyl-1H-pyrazol-1-yl)propan-2-yl)-2,3,4,5,6,7-hexahydro-6,11b-(epiminoethano)naphtho[1,2-d]azepine-5a,10(1H)-diol

[Chemical Formula 273]

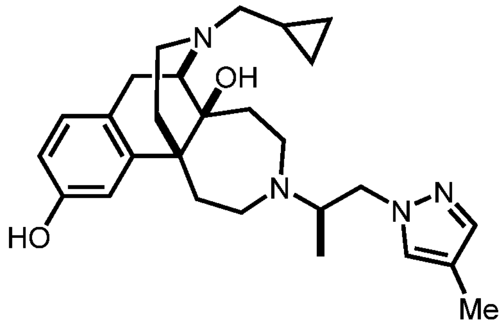

The title compound was obtained from the compound E obtained in Example 3 and the compound obtained in Reference Example 26 according to the method described in Example 32.

$^1$H-NMR (400 MHz, $CDCl_3$)δ (ppm): 0.04 - 0.18 (m, 2H), 0.44 - 0.57 (m, 2H), 0.76 - 0.93 (m, 4H), 0.97 - 1.06 (m, 1H), 1.49 - 1.83 (m, 2H), 1.88 - 2.13 (m, 6H), 2.21 - 2.41 (m, 3H), 2.47 - 2.61 (m, 2H), 2.68 - 2.85 (m, 3H), 2.88 - 3.17 (m, 4H), 3.79 (dd, J = 7, 14 Hz, 1H), 4.02 (dd, J = 7, 14 Hz, 1H), 4.61 (br s, 1H), 6.56 - 6.69 (m, 2H), 6.89 - 7.02 (m, 2H), 7.21 (s, 1H).

(Example 230)

Synthesis of (5aS,6R,11bS)-14-(cyclopropylmethyl)-3-(3-(4-methyl-1H-pyrazol-1-yl)propyl)-2,3,4,5,6,7-hexahydro-6,11b-(epiminoethano)naphtho[1,2-d]azepine-5a,10(1H)-diol

[Chemical Formula 274]

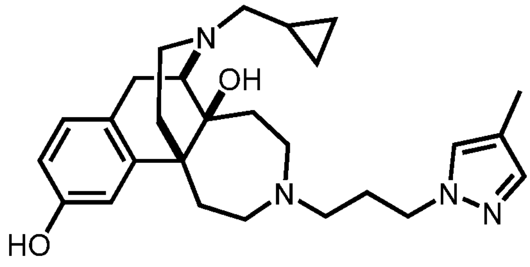

The title compound was obtained from the compound obtained in Example 220 according to the method de-

scribed in Example 24.

$^1$H-NMR (400 MHz, $CDCl_3$)δ (ppm): 0.05 - 0.15 (m, 2H), 0.44 - 0.54 (m, 2H), 0.77 - 0.92 (m, 1H), 0.99 - 1.08 (m, 1H), 1.48 (dd, J = 4, 14 Hz, 1H), 1.70 - 1.92 (m, 4H), 1.99 - 2.06 (m, 2H), 2.04 (s, 3H), 2.29 - 2.57 (m, 8H), 2.78 (dd, J = 6, 18 Hz, 1H), 2.88 - 3.04 (m, 4H), 3.67 - 3.76 (m, 1H), 3.77 - 3.86 (m, 1H), 4.68 (br s, 1H), 6.59 (dd, J = 2, 8 Hz, 1H), 6.64 (d, J = 2 Hz, 1H), 6.90 (d, J = 8 Hz, 1H), 6.98 (s, 1H), 7.27 (s, 1H).

(Reference Example 27)

Synthesis of (S)-1-(benzyloxy)-3-(4-methyl-1H-pyrazol-1-yl)propan-2-ol

[Chemical Formula 275]

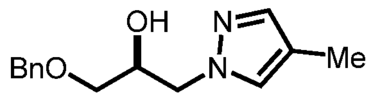

The title compound was obtained from (S)-2-((benzyloxy)methyl)oxirane and 4-methyl-1H-pyrazole according to the method described in Reference Example 25.

$^1$H-NMR (400 MHz, $CDCl_3$)δ (ppm): 2.06 (s, 3H), 3.34 (dd, J = 6, 9 Hz, 1H), 3.46 (dd, J = 5, 9 Hz, 1H), 3.67 (br s, 1H), 4.08 - 4.30 (m, 3H), 4.51 (d, J = 12 Hz, 1H), 4.55 (d, J = 12 Hz, 1H), 7.14 (s, 1H), 7.26 - 7.42 (m, 6H).

(Example 231)

Synthesis of (5aS,6R,11bS)-3-((R)-1-(benzyloxy)-3-(4-methyl-1H-pyrazol-1-yl)propan-2-yl)-14-(cyclopropylmethyl)-2,3,4,5,6,7-hexahydro-6,11b-(epiminoethano)naphtho[1,2-d]azepine-5a,10(1H)-diol

[Chemical Formula 276]

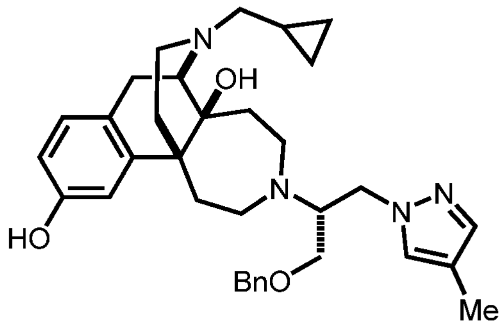

The title compound was obtained from the compound E obtained in Example 3 and the compound obtained in Reference Example 27 according to the method described in Example 32.

$^1$H-NMR (400 MHz, $CDCl_3$)δ (ppm): 0.03 - 0.16 (m, 2H), 0.42 - 0.55 (m, 2H), 0.76 - 0.93 (m, 1H), 0.94 - 1.05 (m, 1H), 1.39 - 1.74 (m, 2H), 1.77 - 1.88 (m, 1H), 1.92 - 2.11 (m, 5H), 2.28 - 2.43 (m, 3H), 2.46 - 2.61 (m, 2H), 2.68 - 2.83 (m, 2H), 2.85 - 3.01 (m, 3H), 3.03 - 3.25 (m, 2H), 3.35 (d, J = 6 Hz, 2H), 3.91 (dd, J = 7, 14 Hz, 1H), 4.01 (dd, J = 6, 14 Hz, 1H), 4.37 (d, J = 12 Hz, 1H), 4.40 (d, J = 12 Hz, 1H), 6.53 - 6.71 (m, 3H), 6.95 (d, J = 8 Hz, 1H), 7.17 - 7.39 (m, 6H).

(Example 232)

Synthesis of (5aS,6R,11bS)-14-(cyclopropylmethyl)-3-((R)-1-hydroxy-3-(4-methyl-1H-pyrazol-1-yl)propan-2-yl)-2,3,4,5,6,7-hexahydro-6,11b-(epiminoethano)naphtho[1,2-d]azepine-5a,10(1H)-diol

[Chemical Formula 277]

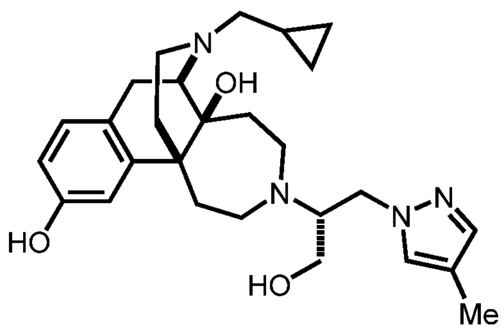

The title compound was obtained from the compound obtained in Example 231 according to the method described in Example 6.

$^1$H-NMR (400 MHz, $CDCl_3$)δ (ppm): 0.03 - 0.17 (m, 2H), 0.43 - 0.57 (m, 2H), 0.74 - 0.92 (m, 1H), 0.96 - 1.12 (m, 1H), 1.50 - 1.84 (m, 3H), 1.90 - 2.12 (m, 5H), 2.24 - 2.68 (m, 6H), 2.76 - 3.23 (m, 8H), 3.81 (dd, J = 6, 14 Hz, 1H), 4.07 (dd, J = 6, 14 Hz, 1H), 6.59 (dd, J = 2, 8 Hz, 1H), 6.65 (d, J = 2 Hz, 1H), 6.95 (d, J = 8 Hz, 1H), 7.04 (s, 1H), 7.27 (s, 1H).

(Reference Example 28)

Synthesis of (R)-1-(benzyloxy)-3-(4-methyl-1H-pyrazol-1-yl)propan-2-ol

[Chemical Formula 278]

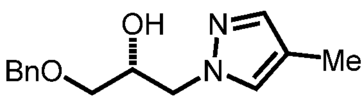

The title compound was obtained from (R)-2-((benzyloxy)methyl)oxirane and 4-methyl-1H-pyrazole according to the method described in Reference Example 25.

$^1$H-NMR (400 MHz, $CDCl_3$)δ (ppm): 2.06 (s, 3H), 3.34 (dd, J = 6, 9 Hz, 1H), 3.46 (dd, J = 5, 9 Hz, 1H), 3.67 (br s, 1H), 4.08 - 4.28 (m, 3H), 4.51 (d, J = 12 Hz, 1H), 4.55 (d, J = 12 Hz, 1H), 7.14 (s, 1H), 7.26 - 7.41 (m, 6H).

(Example 233)

Synthesis of (5aS,6R,11bS)-3-((S)-1-(benzyloxy)-3-(4-methyl-1H-pyrazol-1-yl)propan-2-yl)-14-(cyclopropylmethyl)-2,3,4,5,6,7-hexahydro-6,11b-(epiminoethano)naphtho[1,2-d]azepine-5a,10(1H)-diol

[Chemical Formula 279]

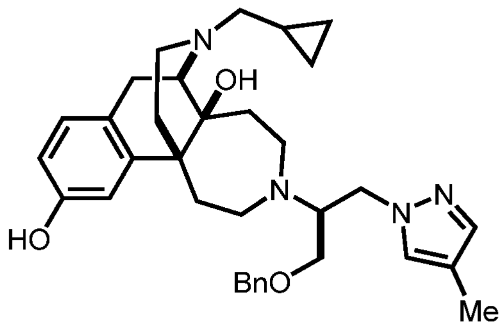

The title compound was obtained from the compound E obtained in Example 3 and the compound obtained in Reference Example 28 according to the method described in Example 32.

$^1$H-NMR (400 MHz, $CDCl_3$)δ (ppm): 0.02 - 0.17 (m, 2H), 0.43 - 0.55 (m, 2H), 0.76 - 0.92 (m, 1H), 0.94 - 1.05 (m, 1H), 1.40 - 1.79 (m, 2H), 1.81 - 2.11 (m, 6H), 2.27 - 2.41 (m, 3H), 2.46 - 2.67 (m, 2H), 2.69 - 3.20 (m, 7H), 3.24 - 3.40 (m, 2H), 4.01 (dd, J = 8, 14 Hz, 1H), 4.10 (dd, J = 6, 14 Hz, 1H), 4.33 - 4.45 (m, 2H), 6.54 - 6.63 (m, 2H), 6.89 - 6.98 (m, 2H), 7.19 (s, 1H), 7.20 - 7.38 (m, 5H).

(Example 234)

Synthesis of (5aS,6R,11bS)-14-(cyclopropylmethyl)-3-((S)-1-hydroxy-3-(4-methyl-1H-pyrazol-1-yl)propan-2-yl)-2,3,4,5,6,7-hexahydro-6,11b-(epiminoethano)naphtho[1,2-d]azepine-5a,10(1H)-diol

[Chemical Formula 280]

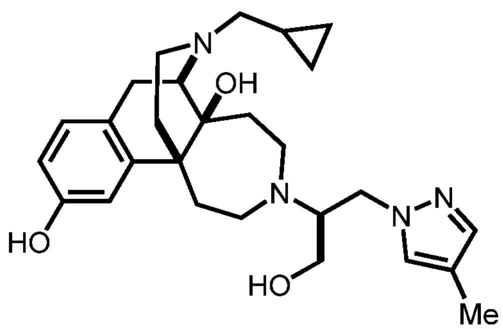

The title compound was obtained from the compound obtained in Example 233 according to the method described in Example 6.

$^1$H-NMR (400 MHz, $CDCl_3$)δ (ppm): 0.02 - 0.17 (m, 2H), 0.43 - 0.56 (m, 2H), 0.74 - 0.91 (m, 1H), 0.96 - 1.11 (m, 1H), 1.43 - 1.60 (m, 1H), 1.73 - 2.10 (m, 7H), 2.23 - 2.38 (m, 3H), 2.41 - 2.65 (m, 3H), 2.71 - 3.07 (m, 5H), 3.15 - 3.42 (m, 3H), 3.82 (dd, J = 6, 14 Hz, 1H), 4.10 (dd, J = 8, 14 Hz, 1H), 6.55 - 6.68 (m, 2H), 6.95 (d, J = 8 Hz, 1H), 7.05 (s, 1H), 7.26 (s, 1H).

(Reference Example 29)

Synthesis of (5aS,6R,11bS)-14-(cyclopropylmethyl)-3-(2-(4-methyl-1H-pyrazol-1-yl)ethyl)-10-((1-phenyl-1H-tetrazol-5-yl)oxy)-2,3,4,5,6,7-hexahydro-6,11b-(epiminoethano)naphtho[1,2-d]azepin-5a(1H)-ol

[Chemical Formula 281]

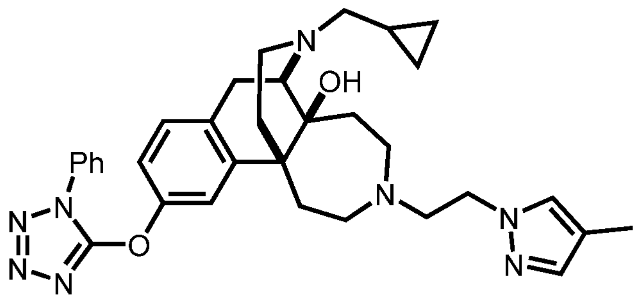

To the compound (25.2 mg, 0.058 mmol) obtained in Example 50 dissolved in N,N-dimethylformamide (1 mL) were added potassium carbonate (23.9 mg, 0.17 mmol) and 5-chloro-1-phenyl-1H-tetrazole (31.3 mg, 0.17 mmol), followed by stirring at room temperature for 19 hours. To the reaction solution, a saturated aqueous sodium bicarbonate solution was added, followed by extraction with ethyl acetate. The organic layer was washed three times with saturated saline, then dried over sodium sulfate, and concentrated under reduced pressure. The obtained crude product was purified by preparative thin layer chromatography (chloroform : methanol = 95 : 5) to yield the title compound (15.3 mg, 46%).

$^1$H-NMR (400 MHz, $CDCl_3$)δ (ppm): 0.05 - 0.16 (m, 2H), 0.46 - 0.58 (m, 2H), 0.78 - 0.91 (m, 1H), 0.99 - 1.06 (m, 1H), 1.42 - 1.51 (m, 1H), 1.68 (ddd, J = 3, 13, 13 Hz, 1H), 1.81 (ddd, J = 3, 3, 16 Hz, 1H), 1.92 - 2.01 (m, 1H), 1.97 (s, 3H), 2.04 - 2.14 (m, 1H), 2.30 - 2.42 (m, 3H), 2.50 (ddd, J = 5, 14, 14 Hz, 1H), 2.57 (dd, J = 4, 12 Hz, 1H), 2.61 - 2.68 (m, 1H), 2.81 - 2.86 (m, 2H), 2.87 - 3.21 (m, 4H), 3.94 - 4.08 (m, 2H), 4.58 (br s, 1H), 6.72 (s, 1H), 7.11 - 7.26 (m, 4H), 7.49 - 7.62 (m, 3H), 7.78 - 7.84 (m, 2H).

(Example 235)

Synthesis of (5aS,6R,11bS)-14-(cyclopropylmethyl)-3-(2-(4-methyl-1H-pyrazol-1-yl)ethyl)-2,3,4,5,6,7-hexahydro-6,11b-(epiminoethano)naphtho[1,2-d]azepin-5a(1H)-ol

[Chemical Formula 282]

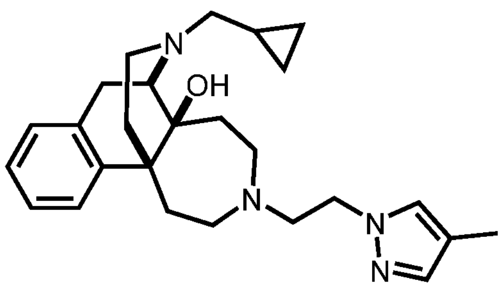

To the compound (14.9 mg, 0.026 mmol) obtained in Reference Example 29 dissolved in methanol (1 mL), 10% palladium-activated carbon (55% wet) (20 mg) was added, followed by stirring under a hydrogen atmosphere at room temperature for 15 hours. The reaction mixture was filtered through a membrane filter, and the filtrate was concentrated under reduced pressure. The obtained crude product was purified by preparative thin layer chromatography (chloroform : 2 M ammonia-methanol solution = 15 : 1) to yield the title compound (4.3 mg, 40%) as a colorless oily matter.

$^1$H-NMR (400 MHz, $CDCl_3$)δ (ppm): 0.06 - 0.15 (m, 2H), 0.46 - 0.53 (m, 2H), 0.77 - 0.90 (m, 1H), 0.98 - 1.04 (m, 1H), 1.45 (ddd, J = 2, 5, 14 Hz, 1H), 1.74 (ddd, J = 3, 3, 13 Hz, 1H), 1.91 (ddd, J = 4, 4, 16 Hz, 1H), 1.94 - 2.00 (m, 1H), 1.98 (s, 3H), 2.08 (ddd, J = 5, 13, 13 Hz, 1H), 2.30 - 2.49 (m, 4H), 2.51 - 2.58 (m, 1H), 2.63 (ddd, J = 4, 4, 13 Hz, 1H), 2.74 - 3.01 (m, 5H), 3.04 (d, J = 18 Hz, 1H), 3.17 (ddd, J = 2, 12, 12 Hz, 1H), 3.85 - 4.01 (m, 2H), 4.63 (br s, 1H), 6.63 (s, 1H), 7.10 - 7.17 (m, 4H), 7.20 (s, 1H).

(Reference Example 30)

Synthesis of (1S,3S)-3-(4-methyl-1H-pyrazol-1-yl)cyclopentan-1-ol

[Chemical Formula 283]

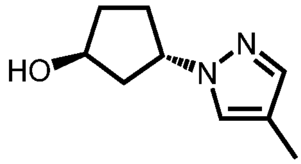

To a solution of 4-methylpyrazole (136 mg, 1.66 mmol) in N,N-dimethylformamide (2 mL) was added sodium hydride (55% oil dispersion, 76.4 mg, 1.75 mmol), followed by stirring at 60°C for 10 minutes. After allowed to cool to room temperature, to the reaction mixture was added a solution of (1S,3R)-3-((methylsulfonyl)oxy)cyclopentyl acetate (synthesized by the method described in Journal of Medicinal Chemistry 1996, 39, 2615, 196 mg, 0.882 mmol) in N,N-dimethylformamide (1.5 mL), followed by stirring at 60°C for 19 hours. After allowed to cool to room temperature, water was added to the reaction mixture, followed by extraction three times with ethyl acetate. The combined extracts were washed with saturated brine, dried over sodium sulfate, and then concentrated under reduced pressure to yield a crude product of the title compound (173 mg) as a yellow oily matter.

$^1$H-NMR (400 MHz, $CDCl_3$)$\delta$ (ppm): 0.83 - 0.90 (m, 1H), 1.68 - 1.76 (m, 1H), 1.97 - 2.05 (m, 1H), 2.06 (s, 3H), 2.14 - 2.41 (m, 3H), 4.57 - 4.61 (m, 1H), 4.81 - 4.88 (m, 1H), 7.18 (s, 1H), 7.31 (s, 1H).

(Reference Example 31)

Synthesis of (1S,3S)-3-(4-methyl-1H-pyrazol-1-yl)cyclopentyl methanesulfonate

[Chemical Formula 284]

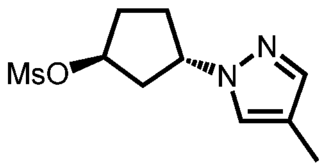

To a solution of the compound (84.2 mg, 0.395 mmol) obtained in Reference Example 30 in tetrahydrofuran (2 mL) were added N,N-diisopropylethylamine (136 pL, 0.789 mmol) and methanesulfonic anhydride (105 mg, 0.603 mmol) under ice cooling, followed by stirring at room temperature for 1 hour. Again, N,N-diisopropylethylamine (34.0 μL, 0.197 mmol) and methanesulfonic anhydride (35.0 mg, 0.201 mmol) were added, followed by stirring at room temperature for 1 hour. To the reaction mixture, a saturated aqueous sodium bicarbonate solution was added, followed by extraction three times with ethyl acetate. The combined extracts were washed with saturated saline, dried over sodium sulfate, and then concentrated under reduced pressure. The obtained crude product was purified by silica gel column chromatography (58 to 100% ethyl acetate/heptane) to yield the title compound (47.6 mg, 49%) as a colorless oily matter.

$^1$H-NMR (400 MHz, $CDCl_3$)$\delta$ (ppm): 2.04 - 2.13 (m, 2H), 2.06 (s, 3H), 2.31 - 2.43 (m, 2H), 2.45 - 2.57 (m, 2H), 3.03 (s, 3H), 4.79 - 4.86 (m, 1H), 5.35 - 5.39 (m, 1H), 7.17 (s, 1H), 7.31 (s, 1H).

(Example 236)

Synthesis of diastereomric mixture of (5aS,6R,11bS)-14-(cyclopropylmethyl)-3-((1R,3S)-3-(4-methyl-1H-pyrazol-1-yl)cyclopentyl)-2,3,4,5,6,7-hexahydro-6,11b-(epiminoethano)naphtho[1,2-d]azepine-5a,10(1H)-diol

[Chemical Formula 285]

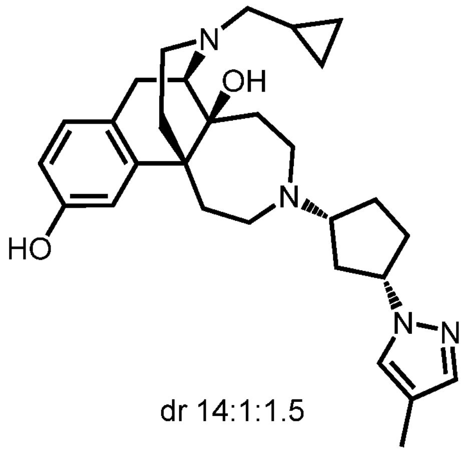

The title compound was obtained as a 14 : 1 : 1.5 diastereomer mixture from the compound E obtained in Example 3 and the compound obtained in Reference Example 31 according to the method described in Example 31.

$^1$H-NMR (400 MHz, $CDCl_3$)δ (ppm): 0.07 - 0.12 (m, 2H), 0.46 - 0.54 (m, 2H), 0.78 - 0.88 (m, 1H), 0.97 - 1.00 (m, 1H), 1.49 - 1.53 (m, 1H), 1.76 - 2.00 (m, 6H), 2.01 (s, 3H), 2.06 - 2.30 (m, 2H), 2.34 (d, J = 6 Hz, 2H), 2.40 - 2.51 (m, 2H), 2.70 - 2.81 (m, 3H), 2.84 - 2.96 (m, 4H), 3.11 - 3.21 (m, 2H), 4.51 - 4.59 (m, 1H), 4.82 (br s, 1H), 6.50 (d, J = 2 Hz, 1H), 6.60 (dd, J = 2, 8 Hz, 1H), 6.90 (d, J = 8 Hz, 1H), 7.11 (s, 1H), 7.25 (s, 1H).

(Reference Example 32)

Synthesis of (1S,3R)-3-(4-methyl-1H-pyrazol-1-yl)cyclopentyl methanesulfonate

[Chemical Formula 286]

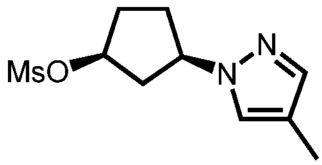

To a solution of 3-(4-methyl-1H-pyrazol-1-yl)cyclopentan-1-one (synthesized by the method described in the Bulletin of the Korian Chemical Society 2012, 33, 3535, 136 mg, 1.66 mmol) in ethanol (2.8 mL) was added sodium borohydride (64.4 mg, 1.70 mmol) under ice cooling, followed by stirring at room temperature for 2.5 hours. Again, sodium borohydride (63.4 mg, 1.67 mmol) was added, followed by stirring at room temperature for 19 hours. Water was added to the reaction mixture, followed by extraction three times with ethyl acetate. The combined extracts were washed with saturated saline, dried over sodium sulfate, and then concentrated under reduced pressure. To a solution of the obtained colorless oily matter (120 mg) in tetrahydrofuran (3.5 mL) were added N,N-diisopropylethylamine (373 pL, 2.16 mmol) and methanesulfonic anhydride (189 mg, 1.08 mmol) under ice cooling, followed by stirring at room temperature for 2 hours. To the reaction mixture, a saturated aqueous sodium bicarbonate solution was added, followed by extraction three times with ethyl acetate. The combined extracts were washed with saturated saline, dried over sodium sulfate, and then concentrated under reduced pressure. The obtained crude product was purified by silica gel column chromatography (67 to 100% ethyl acetate/heptane) to yield the title compound (83.2 mg, 40%) as a colorless oily matter.

$^1$H-NMR (400 MHz, $CDCl_3$)δ (ppm): 2.02 - 2.12 (m, 1H), 2.07 (s, 3H), 2.21 - 2.29 (m, 3H), 2.36 - 2.43 (m, 1H), 2.69 (ddd, J = 7, 9, 15 Hz, 1H), 3.02 (s, 3H), 4.62 - 4.70 (m, 1H), 5.18 - 5.23 (m, 1H), 7.26 (s, 1H), 7.31 (s, 1H).

(Example 237)

Synthesis of (5aS,6R,11bS)-14-(cyclopropylmethyl)-3-((1R,3R)-3-(4-methyl-1H-pyrazol-1-yl)cyclopentyl)-2,3,4,5,6,7-hexahydro-6,11b-(epiminoethano)naphtho[1,2-d]azepine-5a,10(1H)-diol and (5aS,6R,11bS)-14-(cyclopropylmethyl)-3-((1S,3S)-3-(4-methyl-1H-pyrazol-1-yl)cyclopentyl)-2,3,4,5,6,7-hexahydro-6,11b-(epiminoethano)naphtho[1,2-d]azepine-5a,10(1H)-diol

[Chemical Formula 287]

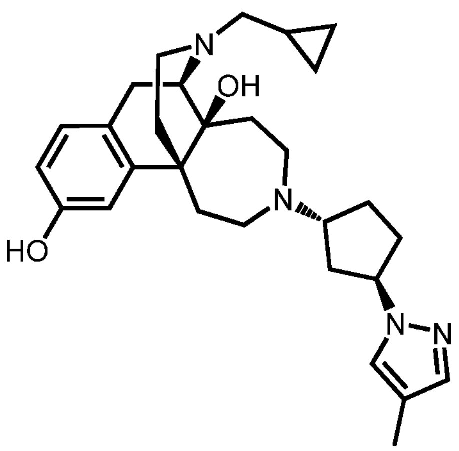

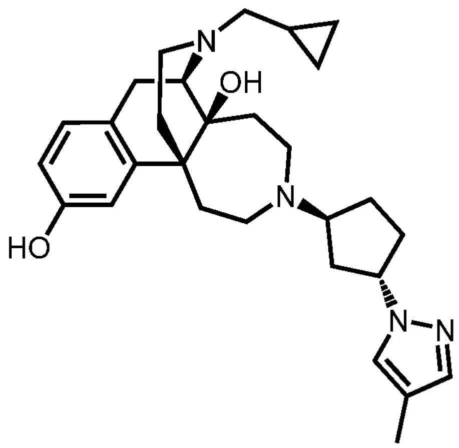

The title compound was obtained as a 1 : 1 diastereomer mixture from the compound E obtained in Example 3 and the compound obtained in Reference Example 32 according to the method described in Example 31.

$^1$H-NMR (400 MHz, $CDCl_3$)δ (ppm): 0.06 - 0.13 (m, 2H), 0.47 - 0.52 (m, 2H), 0.78 - 0.88 (m, 1H), 0.98 - 1.05 (m, 1H), 1.46 - 1.52 (m, 1H), 1.56 - 1.66 (m, 1H), 1.76 - 1.82 (m, 2H), 1.87 - 2.37 (m, 12H), 2.49 - 2.57 (m, 1H), 2.70 - 2.96 (m, 6H), 3.09 - 3.18 (m, 1H), 3.30 - 3.37 (m, 0.5H), 3.39 - 3.47 (m, 0.5H), 4.53 - 4.60 (m, 0.5H), 4.61 - 4.68 (m, 0.5H), 4.79 (br s, 1H), 6.48 (d, J = 2 Hz, 0.5H), 6.53 (d, J = 2 Hz, 0.5H), 6.55 (dd, J = 2, 8 Hz, 0.5H), 6.58 (dd, J = 2, 8 Hz, 0.5H), 6.87 - 6.89 (m, 1H), 7.16 (s, 1H), 7.30 (s, 0.5H), 7.31 (s, 0.5H).

(Example 238)

Synthesis of (5aS,6R,11bS)-14-(cyclopropylmethyl)-5a-hydroxy-3-(2-(4-methyl-1H-pyrazol-1-yl)ethyl)-1,2,3,4,5,5a,6,7-octahydro-6,11b-(epiminoethano)naphtho[1,2-d]azepin-10-yl pivalate

[Chemical Formula 288]

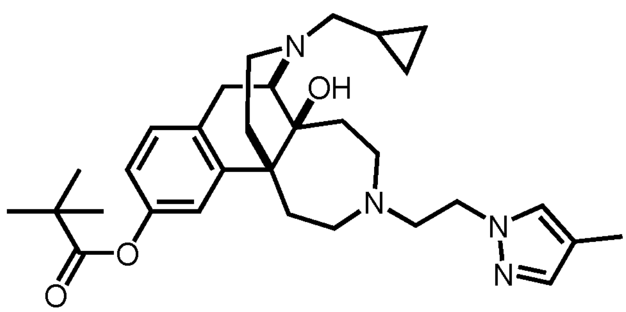

To the compound (11 mg, 0.025 mmol) obtained in Example 50 dissolved in dichloromethane (1 mL) were

added triethylamine (10.5 μL, 0.076 mmol) and pivaloyl chloride (9.3 pL, 0.076 mmol), followed by stirring at room temperature for 2.5 hours. To the reaction solution, a saturated sodium bicarbonate aqueous solution was added, followed by extraction three times with chloroform. The combined extracts were dried over sodium sulfate and concentrated under reduced pressure. The obtained crude product was purified by preparative thin layer chromatography (chloroform : methanol = 95 : 5) to yield the title compound (13.9 mg, quantitative).

$^1$H-NMR (400 MHz, $CDCl_3$)δ (ppm): 0.05 - 0.13 (m, 2H), 0.45 - 0.52 (m, 2H), 0.78 - 0.88 (m, 1H), 0.99 - 1.06 (m, 1H), 1.35 (s, 9H), 1.38 - 1.46 (m, 1H), 1.66 (ddd, J = 3, 13, 13 Hz, 1H), 1.82 (ddd, J = 4, 4, 16 Hz, 1H), 1.92 - 2.01 (m, 1H), 1.98 (s, 3H), 2.02 - 2.12 (m, 1H), 2.32 - 2.39 (m, 3H), 2.45 (ddd, J = 5, 13, 13 Hz, 1H), 2.51 - 2.57 (m, 1H), 2.65 (ddd, J = 4, 13, 13 Hz, 1H), 2.78 - 2.86 (m, 3H), 2.90 - 3.04 (m, 3H), 3.12 - 3.20 (m, 1H), 3.88 - 4.00 (m, 2H), 4.58 (br s, 1H), 6.64 (s, 1H), 6.82 (d, J = 2 Hz, 1H), 6.83 - 6.85 (m, 1H), 7.12 (d, J = 9 Hz, 1H), 7.20 (s, 1H).

(Example 239)

Synthesis of tert-butyl (5aS,6R,11bR)-14-(cyclopropylmethyl)-5a,10-dihydroxy-1,2,5,5a,6,7-hexahydro-6,11b-(epiminoethano)naphtho[1,2-d]azepine-3(4H)-carboxylate (compound K) and tert-butyl (5aS,6R,11bR)-10-((tert-butoxycarbonyl)oxy)-14-(cyclopropylmethyl)-5a-hydroxy-1,2,5,5a,6,7-hexahydro-6,11b-(epiminoethano)naphtho[1,2-d]azepine-3(4H)-carboxylate (compound L)

[Chemical Formula 289]

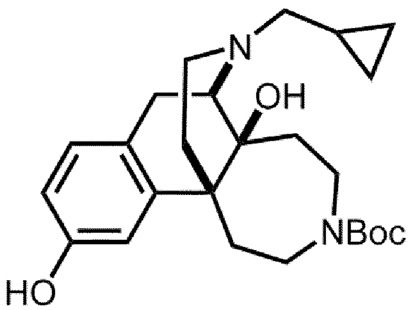

Compound K

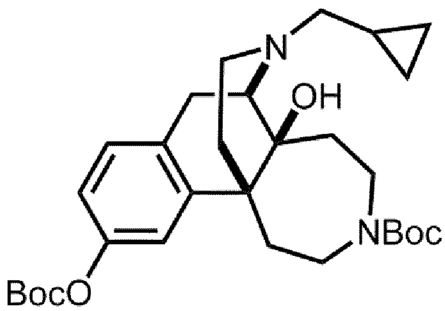

Compound L

To a solution of the compound E (299 mg, 0.911 mmol) obtained in Example 3 in tetrahydrofuran (3.0 mL) was added di-tert-butyl dicarbonate (1.0 mL, 4.35 mmol) under ice cooling, followed by stirring at room temperature for 2 hours. To the reaction mixture, water and a saturated aqueous sodium bicarbonate solution were added under ice cooling, followed by extraction three times with ethyl acetate. The organic layers were combined, washed with water and saturated saline, dried over sodium sulfate, and then concentrated under reduced pressure. The obtained crude product was purified by silica gel column chromatography (amino group-supported silica gel, 50 to 100% ethyl acetate/heptane) to individually yield the title compound K (176 mg, 36%) and the title compound L (190 mg, 49%) as colorless amorphous forms.

(Compound K)

$^1$H-NMR (400 MHz, $CDCl_3$)δ (ppm): 0.06 - 0.14 (m, 2H), 0.47 - 0.55 (m, 2H), 0.76 - 0.88 (m, 1H), 0.99 - 1.09 (m, 1H), 1.29 (s, 4.5H), 1.31 (s, 4.5H), 1.44 - 1.67 (m, 1H), 1.76 - 1.90 (m, 1.5H), 1.91 - 2.11 (m, 2.5H), 2.29 (dd, J = 7, 13 Hz, 0.5H), 2.32 - 2.43 (m, 2H), 2.47 - 2.57 (m, 1.5H), 2.74 - 2.85 (m, 1H), 2.88 - 2.97 (m, 1.5H), 2.98 (d, J = 18 Hz, 0.5H), 3.31 - 3.41 (m, 1.5H), 3.46 - 3.63 (m, 2H), 3.75 (ddd, J = 5, 13, 13 Hz, 0.5H), 4.51 (br s, 0.5H), 4.95 (br s, 0.5H), 5.75 (br s, 0.5H), 6.59 (dd, J = 2, 8 Hz, 0.5H), 6.61 - 6.67 (m, 1.5H), 6.92 (d, J = 8 Hz, 0.5H), 6.94 (d, J = 7 Hz, 0.5H).

(Compound L)

$^1$H-NMR (400 MHz, $CDCl_3$)δ (ppm): 0.05 - 0.14 (m, 2H), 0.42 - 0.55 (m, 2H), 0.77 - 0.90 (m, 1H), 1.00 - 1.11 (m, 1H), 1.30 (s, 4.5H), 1.39 (s, 4.5H), 1.50 - 1.62 (m, 1H), 1.55 (s, 9H), 1.69 - 2.12 (m, 4H), 2.17 - 2.40 (m, 2.5H), 2.44 - 2.58 (m, 1.5H), 2.78 - 2.94 (m, 1.5H), 2.93 (d, J = 6 Hz, 0.5H), 2.97 (d, J = 19 Hz, 0.5H), 3.00 - 3.11 (m, 0.5H), 3.04 (d, J = 18 Hz, 0.5H), 3.26 (ddd, J = 4, 4, 14 Hz, 0.5H), 3.36 - 3.48 (m, 1H), 3.54 - 3.65 (m, 1H), 3.70 (ddd, J = 3, 14, 14

Hz, 0.5H), 3.83 - 3.89 (m, 0.5H), 4.50 (br s, 1H), 6.90 - 6.94 (m, 1H), 6.97 (dd, J = 2, 8 Hz, 1H), 7.07 (d, J = 8 Hz, 0.5H), 7.09 (d, J = 8 Hz, 0.5H).

(Example 240)

Synthesis of tert-butyl (5aS,6R,11bR)-14-(cyclopropylmethyl)-5a-hydroxy-10-(((trifluoromethyl)sulfonyl)oxy)-1,2,5,5a,6,7-hexahydro-6,11b-(epiminoethano)naphtho[1,2-d]azepine-3(4H)-carboxylate

[Chemical Formula 290]

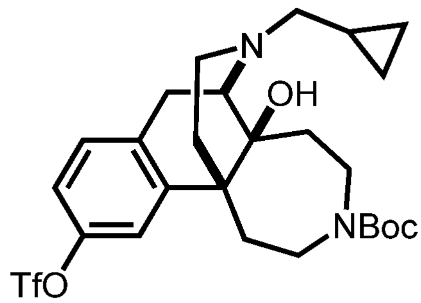

To the compound K (320 mg, 0.746 mmol) obtained in Example 239 dissolved in N,N-dimethylformamide (5.0 mL) were added potassium carbonate (520 mg, 3.76 mmol) and N-phenylbis(trifluoromethanesulfonimide) (804 mg, 2.25 mmol) under ice cooling, followed by stirring at room temperature for 17 hours. To the reaction mixture, a saturated aqueous sodium bicarbonate solution and water were added under ice cooling to stop the reaction, followed by extraction three times with ethyl acetate. The organic layers were combined, washed with water and saturated saline, dried over sodium sulfate, and then concentrated under reduced pressure. The obtained crude product was purified by silica gel column chromatography (amino group-supported silica gel, 20 to 50% ethyl acetate/heptane) to yield the title compound (359 mg, 86%) as a colorless amorphous form.

$^1$H-NMR (400 MHz, $CDCl_3$)$\delta$ (ppm): 0.07 - 0.14 (m, 2H), 0.49 - 0.56 (m, 2H), 0.77 - 0.89 (m, 1H), 0.97 - 1.07 (m, 1H), 1.30 (s, 5.4H), 1.35 (s, 3.6H), 1.50 - 1.81 (m, 2.2H), 1.82 - 1.97 (m, 1.4H), 1.99 - 2.18 (m, 1.4H), 2.30 (dd, J = 7, 13 Hz, 0.6H), 2.32 - 2.41 (m, 1.8H), 2.54 - 2.65 (m, 1.6H), 2.83 - 2.98 (m, 2H), 3.02 (d, J = 19 Hz, 0.6H), 3.07 (d, J = 19 Hz, 0.4H), 3.16 (ddd, J = 2, 12, 14 Hz, 0.4H), 3.31 (ddd, J = 4, 4, 14 Hz, 0.4H), 3.36 - 3.44 (m, 1.2H), 3.55 - 3.68 (m, 1.6H), 3.77 (ddd, J = 4, 5, 15 Hz, 0.4H), 4.47 (br s, 1H), 7.00 - 7.07 (m, 2H), 7.16 (d, J = 8 Hz, 0.6H), 7.17 (d, J = 8 Hz, 0.4H).

(Example 241)

Synthesis of tert-butyl (5aS,6R,11bR)-10-cyano-14-(cyclopropylmethyl)-5a-hydroxy-1,2,5,5a,6,7-hexahydro-6,11b-(epiminoethano)naphtho[1,2-d]azepine-3(4H)-carboxylate

[Chemical Formula 291]

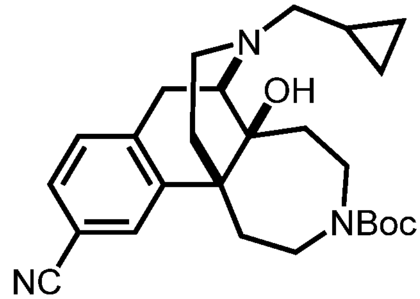

The compound (299 mg, 0.533 mmol) obtained in Example 240, zinc cyanide (190 mg, 1.62 mmol), tris(dibenzylideneacetone)dipalladium (0) (48.8 mg, 53.3 pmol), and (±)-BINAP (66.9 mg, 0.107 mmol) were dissolved in N,N-dimethylformamide (3.0 mL), followed by stirring at 150°C for 1 hour. The reaction mixture was allowed to cool to room temperature and filtered through celite. The filtrate was washed with water and saturated saline, dried over sodium sulfate, and then concentrated under reduced pressure. The obtained crude product was purified by silica gel column chromatography (50 to 100% ethyl acetate/heptane) to yield the title compound (184 mg, 79%) as a pale yellow amorphous form.

$^1$H-NMR (400 MHz, $CDCl_3$)δ (ppm): 0.06 - 0.16 (m, 2H), 0.47 - 0.58 (m, 2H), 0.77 - 0.89 (m, 1H), 0.96 - 1.06 (m, 1H), 1.26 (s, 6.3H), 1.32 (s, 2.7H), 1.44 - 1.75 (m, 2.3H), 1.78 - 1.92 (m, 1.7H), 2.01 - 2.15 (m, 0.3H), 2.14 (ddd, J = 5, 13, 13 Hz, 0.7H), 2.29 (dd, J = 7, 12 Hz, 0.7H), 2.32 - 2.47 (m, 1.6H), 2.59 (dd, J = 4, 12 Hz, 1H), 2.67 (ddd, J = 6, 13, 16 Hz, 0.7H), 2.88 - 3.00 (m, 1H), 2.97 (d, J = 7 Hz, 0.3H), 2.99 (d, J = 6 Hz, 0.7H), 3.04 (d, J = 19 Hz, 0.7H), 3.09 (d, J = 19 Hz, 0.3H), 3.22 - 3.38 (m, 2H), 3.53 - 3.72 (m, 1.3H), 3.80 (ddd, J = 5, 13, 13 Hz, 0.7H), 4.47 (br s, 1H), 7.16 - 7.21 (m, 0.3H), 7.18 (d, J = 8 Hz, 0.7H), 7.39 (d, J = 8 Hz, 1H), 7.46 (s, 1H).

(Example 242)

Synthesis of tert-butyl (5aS,6R,11bR)-10-carbamoyl-14-(cyclopropylmethyl)-5a-hydroxy-1,2,5,5a,6,7-hexahydro-6,11b-(epiminoethano)naphtho[1,2-d]azepine-3(4H)-carboxylate

[Chemical Formula 292]

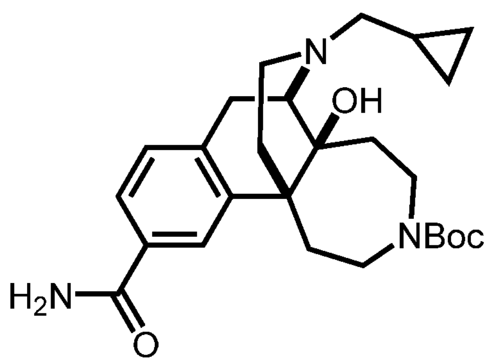

To the compound (222 mg, 0.507 mmol) obtained in Example 241, palladium acetate (24.0 mg, 0.107 mmol), and triphenylphosphine (53.5 mg, 0.204 mmol) dissolved in toluene (3.0 mL) was added acetaldoxime (93.0 μL, 1.52 mmol), followed by heating to 80°C and stirring for 1 hour. The reaction mixture was allowed to cool to room temperature and concentrated under reduced pressure. The obtained crude product was purified by silica gel column chromatography (6 to 50% 10% aqueous ammonia-methanol/50% ethyl acetate-heptane) to yield the title compound (210 mg, 91%) as a yellow amorphous form.

$^1$H-NMR (400 MHz, $CDCl_3$)δ (ppm): 0.06 - 0.17 (m, 2H), 0.46 - 0.58 (m, 2H), 0.77 - 0.89 (m, 1H), 0.98 - 1.13 (m, 1H), 1.06 (s, 7.2H), 1.22 (s, 1.8H), 1.48 (dd, J = 4, 14 Hz, 0.8H), 1.60 (dd, J = 4, 14 Hz, 0.2H), 1.69 - 2.21 (m, 4.2H), 2.26 - 2.41 (m, 0.2H), 2.29 (dd, J = 6, 12 Hz, 0.2H), 2.35 (dd, J = 7, 12 Hz, 0.8H), 2.35 (dd, J = 6, 12 Hz, 0.8H), 2.48 - 2.65 (m, 1.2H), 2.72 (ddd, J = 9, 11, 16 Hz, 0.8H), 2.86 (dd, J = 6, 19 Hz, 0.8H), 2.93 - 3.23 (m, 2H), 3.01 (d, J = 6 Hz, 0.8H), 3.12 (d, J = 19 Hz, 0.8H), 3.30 - 3.38 (m, 0.4H), 3.47 - 3.63 (m, 1H), 3.77 (ddd, J = 4, 13, 13 Hz, 0.2H), 4.28 - 4.39 (m, 0.8H), 4.49 (br s, 0.2H), 4.58 (br s, 0.8H), 5.58 (br s, 1H), 5.82 (br s, 0.2H), 6.20 (br s, 0.8H), 7.13 (d, J = 8 Hz, 0.8H), 7.14 (d, J = 8 Hz, 0.2H), 7.39 - 7.49 (m, 1.8H), 7.73 - 7.75 (m, 0.2H).

(Example 243)

Synthesis of (5aS,6R, 11bS)-14-(cyclopropylmethyl)-5a-hydroxy-1,2,3,4,5,5a,6,7-octahydro-6,11b-(epiminoethano)naphtho[1,2-d]azepine-10-carboxamide

[Chemical Formula 293]

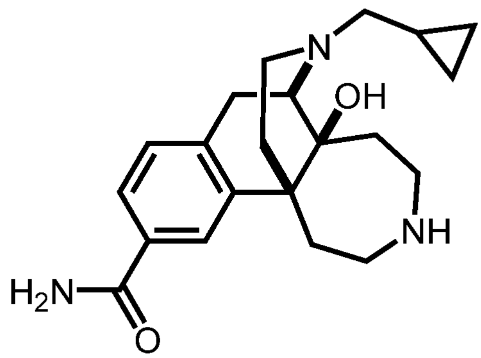

To the compound (210 mg, 0.461 mmol) obtained in Example 242 dissolved in dichloromethane (3.0 mL), trifluoroacetic acid (0.6 mL) was added under cooling, followed by stirring at 0°C for 30 minutes and at room temperature for 1 hour. Water was added to the reaction mixture under cooling to stop the reaction, and the organic layer was separated. To the aqueous layer, a saturated aqueous sodium bicarbonate solution was added to make the aqueous layer basic, followed by extraction with dichloromethane, chloroform, chloroform/methanol (10/1), and chloroform/methanol (5/1). The combined organic layers were dried over sodium sulfate and then concentrated under reduced pressure to yield the title compound (119 mg, 73%) as a pale yellow amorphous form.

$^1$H-NMR (400 MHz, $CDCl_3$)δ (ppm): 0.06 - 0.18 (m, 2H), 0.46 - 0.59 (m, 2H), 0.79 - 0.90 (m, 1H), 1.07 - 1.14 (m, 1H), 1.60 (ddd, J = 5, 5, 15 Hz, 1H), 1.69 (ddd, J = 4, 11, 15 Hz, 1H), 1.92 (ddd, J = 3, 13, 13 Hz, 1H), 2.08 (ddd, J = 5, 13, 13 Hz, 1H), 2.17 (ddd, J = 3, 3, 16 Hz, 1H), 2.30 - 2.41 (m, 3H), 2.54 - 2.60 (m, 1H), 2.80 (ddd, J = 4, 4, 14 Hz, 1H), 2.80 - 2.91 (m, 1H), 2.92 (dd, J = 6, 19 Hz, 1H), 2.99 (d, J = 6 Hz, 1H), 3.05 (ddd, J = 4, 4, 15 Hz, 1H), 3.10 (d, J = 19 Hz, 1H), 3.15 (ddd, J = 3, 11, 14 Hz, 1H), 4.52 (br s, 1H), 5.56 (br s, 1H), 6.08 (br s, 1H), 7.19 (d, J = 8 Hz, 1H), 7.51 (dd, J = 2, 8 Hz, 1H), 7.71 (d, J = 2 Hz, 1H).

(Reference Example 33)

Synthesis of 2-(4-methyl-1H-pyrazol-1-yl)ethyl methanesulfonate

[Chemical Formula 294]

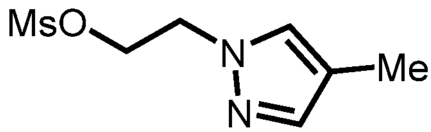

The title compound was obtained from 2-(4-methyl-1H-pyrazol-1-yl)ethane-1-ol according to the method described in Reference Example 31.

$^1$H-NMR (400 MHz, $CDCl_3$)δ (ppm): 2.07 (s, 3H), 2.81 (s, 3H), 4.37 (t, J = 5 Hz, 2H), 4.58 (t, J = 5 Hz, 2H), 7.24 (s, 1H), 7.36 (s, 1H).

(Example 244)

Synthesis of (5aS,6R,11bS)-14-(cyclopropylmethyl)-5a-hydroxy-3-(2-(4-methyl-1H-pyrazol-1-yl)ethyl)-1,2,3,4,5,5a,6,7-octahydro-6,11b-(epiminoethano)naphtho[1,2-d]azepine-10-carboxamide

[Chemical Formula 295]

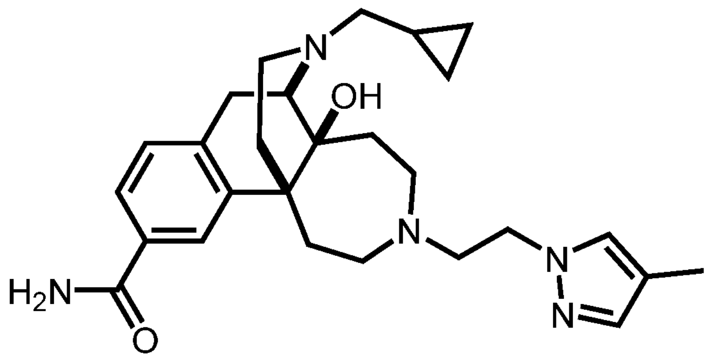

The title compound was obtained from the compounds obtained in Example 243 and Reference Example 33 according to the method described in Example 31.

$^1$H-NMR (400 MHz, $CDCl_3$)δ (ppm): 0.06 - 0.16 (m, 2H), 0.46 - 0.56 (m, 2H), 0.78 - 0.89 (m, 1H), 1.00 - 1.08 (m, 1H), 1.47 (ddd, J = 2, 4, 14 Hz, 1H), 1.68 (ddd, J = 3, 11, 14 Hz, 1H), 1.89 (ddd, J = 3, 13, 13 Hz, 1H), 1.93 - 2.01 (m, 1H), 1.98 (s, 3H), 2.10 (ddd, J = 5, 13, 13 Hz, 1H), 2.34 (d, J = 6 Hz, 2H), 2.40 (ddd, J = 4, 4, 13 Hz, 1H), 2.49 (ddd, J = 5, 12, 16 Hz, 1H), 2.53 - 2.59 (m, 1H), 2.65 (ddd, J = 4, 4, 13 Hz, 1H), 2.78 (ddd, J = 6, 6, 13 Hz, 1H), 2.80 (ddd, J = 6, 6, 13 Hz, 1H), 2.89 (dd, J = 6, 19 Hz, 1H), 2.92 - 3.01 (m, 1H), 2.97 (d, J = 6 Hz, 1H), 3.07 (d, J = 19 Hz, 1H), 3.14 (ddd, J = 2, 11, 13 Hz, 1H), 3.90 (ddd, J = 6, 6, 14 Hz, 1H), 3.93 (ddd, J = 6, 6, 14 Hz, 1H), 4.60 (br s, 1H), 5.57 (br s, 1H), 6.14 (br s, 1H), 6.70 (s, 1H), 7.18 (s, 1H), 7.19 (d, J = 8 Hz, 1H), 7.48 (dd, J = 2, 8 Hz, 1H), 7.69 (d, J = 2 Hz, 1H).

(Example 245)

Synthesis of (2S,3R,5S,6S)-2-(((5aS,6R,11bS)-14-(cyclopropylmethyl)-5a-hydroxy-3-(2-(4-methyl-1H-pyrazol-1-yl)ethyl)-1,2,3,4,5,5a,6,7-octahydro-6,11b-(epiminoethano)naphtho[1,2-d]azepin-10-yl)oxy)-6-(methoxycarbonyl)tetrahydro-2H-pyran-3,4,5-triyl triacetate

[Chemical Formula 296]

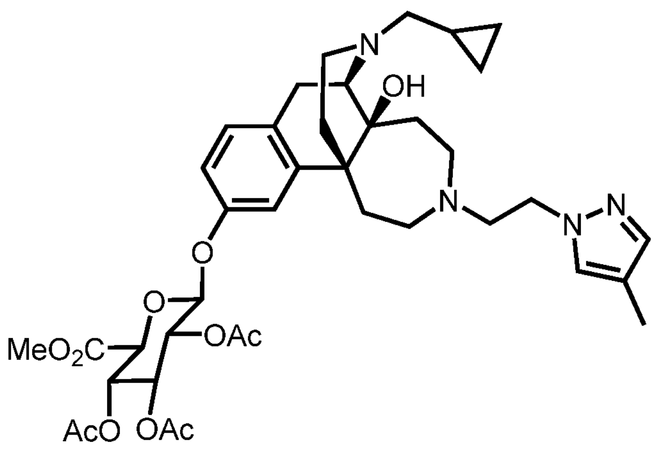

To a solution of the compound (31.2 mg, 0.0717 mmol) obtained in Example 50 and 2,3,4-tri-O-acetyl-α-D-glucuronic acid methyl ester trichloroacetoimidate (68.6 mg, 0.143 mmol) in dichloromethane (1.5 mL) were added molecular sieves 4 Å (157 mg), followed by stirring at room temperature for 3 hours. To the reaction mixture was added a boron trifluoride-diethyl ether complex (36.0 pL, 0.287 mmol), followed by stirring at room temperature for 18 hours. To the reaction mixture, a saturated aqueous sodium bicarbonate solution was added, followed by extraction three times with ethyl acetate. The combined extracts were washed with saturated saline, dried over sodium sulfate, and then concentrated under reduced pressure. The obtained crude product was purified by preparative thin layer chromatography (chloroform : 2 M ammonia-methanol = 95 : 5, twice) to yield the title compound (22.8 mg, 42%) as a pale yellow oily matter.

$^1$H-NMR (400 MHz, $CDCl_3$)δ (ppm): 0.07 - 0.11 (m, 2H), 0.48 - 0.52 (m, 2H), 0.78 - 0.86 (m, 1H), 0.97 - 1.00 (m, 1H), 1.42 - 1.46 (m, 1H), 1.66 - 1.73 (m, 1H), 1.80 (ddd, J = 4, 4, 16 Hz, 1H), 1.90 - 2.11 (m, 15H), 2.33 (d, J = 7 Hz, 2H), 2.37 - 2.48 (m, 2H), 2.54 (dd, J = 4, 12 Hz, 1H), 2.60 (ddd, J = 4, 4, 12 Hz, 1H), 2.76 - 2.83 (m, 3H), 2.91 - 3.00 (m, 3H), 3.10 - 3.16 (m, 1H), 3.72 (s, 3H), 3.85 - 4.01 (m, 2H), 4.11 - 4.13 (m, 1H), 5.07 (d, J = 8 Hz, 1H), 5.22 - 5.36 (m, 3H), 6.67 (s, 1H), 6.79 - 6.81 (m, 2H), 7.04 (d, J = 8 Hz, 1H), 7.19 (s, 1H).

(Example 246)

Synthesis of (2S,3S,5R,6S)-6-(((5aS,6R,11bS)-14-(cyclopropylmethyl)-5a-hydroxy-3-(2-(4-methyl-1H-pyrazol-1-yl)ethyl)-1,2,3,4,5,5a,6,7-octahydro-6,11b-(epiminoethano)naphtho[1,2-d]azepin-10-yl)oxy)-3,4,5-trihydroxytetrahydro-2H-pyran-2-carboxylic acid

[Chemical Formula 297]

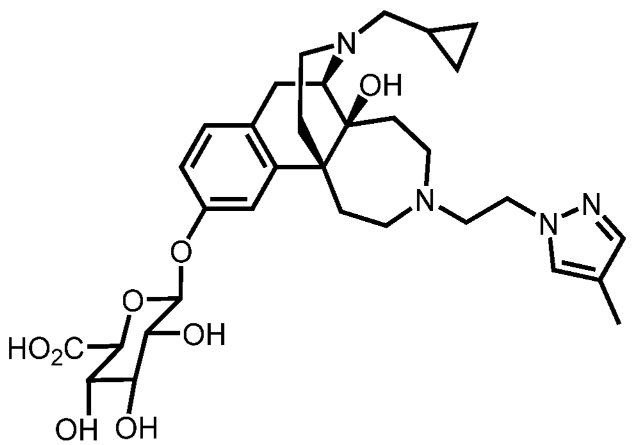

To a mixed solution of the compound (8.3 mg, 0.011 mmol) obtained in Example 245 in tetrahydrofuran/water (v/v = 2/1, 1 mL) was added lithium hydroxide (9.3 mg, 0.22 mmol), followed by stirring at 60°C for 4 hours. After allowed to cool to room temperature, the reaction mixture was concentrated under reduced pressure. The obtained crude product was purified by reverse phase silica gel column chromatography (0 to 100% methanol/water) to yield the title compound (8.1 mg, 100%) as a colorless oily matter.

$^1$H-NMR (400 MHz, $D_2O$)δ (ppm): 0.35 - 0.45 (m, 2H), 0.64 - 0.80 (m, 2H), 1.00 - 1.09 (m, 1H), 1.36 - 1.39 (m, 1H), 1.62 - 1.66 (m, 1H), 1.81 - 1.90 (m, 3H), 2.00 (s, 3H), 2.23 - 2.35 (m, 3H), 2.59 (ddd, J = 4, 13, 13 Hz, 1H), 2.68 - 3.30 (m, 9H), 3.57 - 3.63 (m, 3H), 3.81 - 3.86 (m, 2H), 4.18 - 4.21 (m, 2H), 5.10 (d, J = 7 Hz, 1H), 6.93 (d, J = 2 Hz, 1H), 7.05 (dd, J = 2, 8 Hz, 1H), 7.25 (d, J = 8 Hz, 1H), 7.26 (s, 1H), 7.32 (s, 1H).

(Example 247)

Synthesis of (5aS,6R,11bR)-14-(cyclopropylmethyl)-5a-hydroxy-3-(2-(pyridin-2-yl)acetyl)-1,2,3,4,5,5a,6,7-octahydro-6,11b-(epiminoethano)naphtho[1,2-d]azepine-10-carboxamide

[Chemical Formula 298]

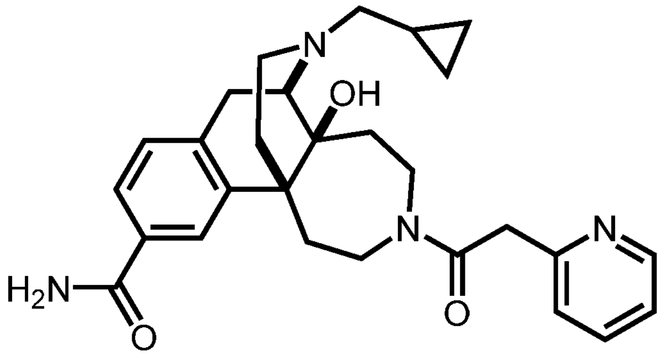

The title compound was obtained from the compound obtained in Example 243 and 2-(pyridin-2-yl)acetic acid hydrochloride according to the method described in Example 5.

$^1$H-NMR (400 MHz, $CDCl_3$)$\delta$ (ppm): 0.05 - 0.17 (m, 2H), 0.45 - 0.59 (m, 2H), 0.76 - 0.89 (m, 1H), 1.01 - 1.11 (m, 1H), 1.52 - 2.15 (m, 5.4H), 2.30 (dd, J = 7, 13 Hz, 0.4H), 2.33 (d, J = 6 Hz, 1.2H), 2.37 (dd, J = 7, 13 Hz, 0.4H), 2.50 - 2.73 (m, 2.6H), 2.86 - 2.99 (m, 0.4H), 2.95 (d, J = 7 Hz, 0.6H), 2.97 (d, J = 6 Hz, 0.4H), 3.04 (d, J = 20 Hz, 1H), 3.27 - 3.39 (m, 1.4H), 3.46 - 3.84 (m, 3.8H), 3.88 - 3.97 (m, 0.4H), 4.36 (br s, 0.6H), 4.51 (br s, 0.4H), 5.75 (br s, 1H), 6.88 (d, J = 8 Hz, 0.6H), 7.00 (d, J = 8 Hz, 0.4H), 7.08 - 7.18 (m, 2H), 7.09 (br s, 1H), 7.39 - 7.44 (m, 0.6H), 7.45 - 7.53 (m, 1.6H), 7.54 - 7.61 (m, 0.4H), 7.65 - 7.68 (m, 0.4H), 8.39 - 8.46 (m, 1H).

(Reference Example 34)

Synthesis of (4bR,8aS,9R)-3-(benzyloxy)-11-(cyclopropylmethyl)-4,8a-dihydroxy-8,8a,9,10-tetrahydro-5H-9,4b-(epiminoethano)phenanthren-6(7H)-one

[Chemical Formula 299]

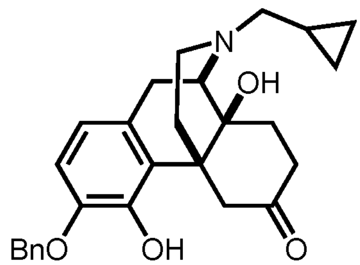

To (4R,4aS,7aR,12bS)-9-(benzyloxy)-3-(cyclopropylmethyl)-4a-hydroxy-2,3,4,4a,5,6-hexahydro-1H-4,12-methanobenzofuro[3,2-e]isoquinoline-7(7aH)-one (synthesized by the method described in Tetrahedron Letters, 2007, 48, 7413) (1.66 g, 3.85 mmol) dissolved in ethanol (165 mL) was added ammonium chloride (2.06 g, 38.5 mmol), and then added zinc powder (2.52 g, 38.5 mmol) in 5 portions, followed by heating under reflux for 1 hour. The reaction mixture was filtered through celite, and the filtrate was concentrated under reduced pressure. A 28% aqueous ammonia solution was added to the obtained residue, followed by extraction once with ethyl acetate. The organic layer was washed with saturated saline and water, then dried over sodium sulfate, and concentrated under reduced pressure. The obtained crude product was used in the next reaction without purification.

$^1$H-NMR (400 MHz, $CDCl_3$)$\delta$ (ppm): 0.09 - 0.15 (m, 2H), 0.49 - 0.58 (m, 2H), 0.80 - 0.92 (m, 1H), 1.49 - 1.63 (m, 1H), 1.78 - 1.86 (m, 1H), 1.97 (ddd, J = 6, 14, 14 Hz, 1H), 2.03 - 2.09 (m, 2H), 2.11 - 2.19 (m, 1H), 2.30 - 2.41 (m, 2H), 2.57 - 2.62 (m, 1H), 2.73 - 2.89 (m, 2H), 2.92 - 3.03 (m, 2H), 3.11 (d, J = 6 Hz, 1H), 3.91 (dd, J = 2, 14 Hz, 1H), 4.72 (br s, 1H), 5.01 (d, J = 11 Hz, 1H), 5.02 (d, J = 11 Hz, 1H), 6.19 (br s, 1H), 6.55 (d, J = 8 Hz, 1H), 6.73 (d, J = 8 Hz, 1H), 7.32 - 7.42 (m, 5H).

(Reference Example 35)

Synthesis of (4bR,8aS,9R)-3-(benzyloxy)-11-(cyclopropylmethyl)-8a-hydroxy-4-methoxy-8,8a,9,10-tetrahydro-5H-9,4b-(epiminoethano)phenanthren-6(7H)-one

[Chemical Formula 300]

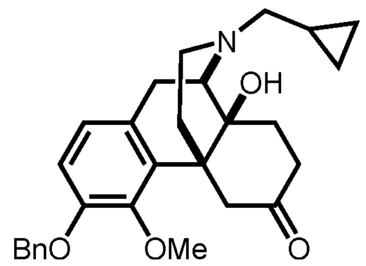

To the compound (98.2 mg, 0.23 mmol) obtained in Reference Example 34 dissolved in N,N-dimethylformamide (4 mL) were added potassium carbonate (62.6 mg, 0.45 mmol) and methyl iodide (17 μL, 0.27 mmol), followed by stirring at room temperature for 40 hours. To the reaction mixture, a saturated aqueous sodium bicarbonate solution was added, followed by extraction once with ethyl acetate. The organic layer was washed three times with water, then dried over sodium sulfate, and concentrated under reduced pressure. The obtained crude product was purified by silica gel column chromatography (0 to 5% methanol/ethyl acetate) to yield the title compound (68.6 mg, 68%) as a colorless amorphous form.

$^1$H-NMR (400 MHz, $CDCl_3$)δ (ppm): 0.06 - 0.17 (m, 2H), 0.49 - 0.59 (m, 2H), 0.80 - 0.91 (m, 1H), 1.50 - 1.60 (m, 1H), 1.81 (dd, J = 7, 13 Hz, 1H), 1.92 - 2.20 (m, 4H), 2.30 - 2.41 (m, 2H), 2.57 - 2.64 (m, 1H), 2.74 - 2.89 (m, 2H), 2.97 (d, J = 19 Hz, 1H), 3.02 (d, J = 14 Hz, 1H), 3.02 (d, J = 14 Hz, 1H), 3.09 (d, J = 6 Hz, 1H), 3.71 (d, J = 14 Hz, 1H), 3.98 (s, 3H), 5.03 (s, 2H), 6.71 (d, J = 8 Hz, 1H), 6.79 (d, J = 8 Hz, 1H), 7.28 - 7.47 (m, 5H).

(Example 248)

Synthesis of (5aS,6R,11bR)-10-(benzyloxy)-14-(cyclopropylmethyl)-5a-hydroxy-11-methoxy-3,4,5,5a,6,7-hexahydro-6,11b-(epiminoethano)naphtho[1,2-d]azepin-2(1H)-one (compound M) and (5aS,6R,11bS)-10-(benzyloxy)-14-(cyclopropylmethyl)-5a-hydroxy-11-methoxy-1,2,5,5a,6,7-hexahydro-6,11b-(epiminoethano)naphtho[1,2-c]azepin-3(4H)-one (compound N)

[Chemical Formula 301]

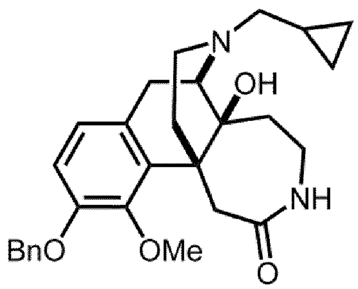

Compound M

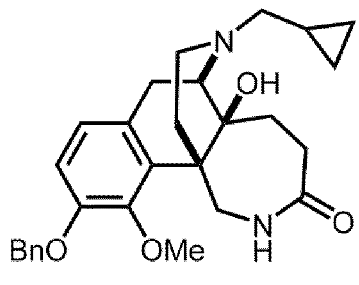

Compound N

The title compounds (M) and (N) were individually obtained from the compound obtained in Reference Example 35 according to the methods described in Reference Example 3 and Example 1.

(Compound M)

$^1$H-NMR (400 MHz, $CDCl_3$)$\delta$ (ppm): 0.07 - 0.13 (m, 2H), 0.45 - 0.56 (m, 2H), 0.75 - 0.88 (m, 1H), 1.53 - 1.62 (m, 2H), 1.84 - 1.94 (m, 1H), 1.96 - 2.11 (m, 2H), 2.26 - 2.38 (m, 2H), 2.57 - 2.66 (m, 1H), 2.78 - 2.92 (m, 4H), 3.20 (dd, J = 2, 14 Hz, 1H), 3.34 (d, J = 14 Hz, 1H), 3.80 (ddd, J = 3, 3, 13 Hz, 1H), 3.96 (s, 3H), 4.50 (br s, 1H), 4.97 (d, J = 12 Hz, 1H), 5.10 (d, J = 12 Hz, 1H), 5.49 - 5.56 (m, 1H), 6.67 (d, J = 8 Hz, 1H), 6.79 (d, J = 8 Hz, 1H), 7.27 - 7.46 (m, 5H).

(Compound N)

$^1$H-NMR (400 MHz, $CDCl_3$)$\delta$ (ppm): 0.07 - 0.16 (m, 2H), 0.46 - 0.58 (m, 2H), 0.77 - 0.88 (m, 1H), 1.10 - 1.26 (m, 1H), 1.49 - 1.57 (m, 1H), 1.81 (dd, J = 14, 14 Hz, 1H), 2.00 (ddd, J = 5, 13, 13 Hz, 1H), 2.07 - 2.17 (m, 2H), 2.29 - 2.40 (m, 2H), 2.65 (dd, J = 4, 11 Hz, 1H), 2.82 (dd, J = 6, 18 Hz, 1H), 2.87 (d, J = 6 Hz, 1H), 2.96 (d, J = 18 Hz, 1H), 3.20 (dd, J = 14, 14 Hz, 1H), 3.46 (dd, J = 7, 15 Hz, 1H), 3.82 (s, 3H), 4.12 (dd, J = 7, 15 Hz, 1H), 4.66 (br s, 1H), 5.00 (d, J = 11 Hz, 1H), 5.07 (d, J = 11 Hz, 1H), 6.10 (dd, J = 6, 6 Hz, 1H), 6.75 (d, J = 8 Hz, 1H), 6.82 (d, J = 8 Hz, 1H), 7.30 - 7.47 (m, 5H).

(Example 249)

Synthesis of tert-butyl (5aS,6R,11bR)-10-(benzyloxy)-14-(cyclopropylmethyl)-5a-hydroxy-11-methoxy-1,2,5,5a,6,7-hexahydro-6,11b-(epiminoethano)naphtho[1,2-d]azepine-3(4H)-carboxylate

[Chemical Formula 302]

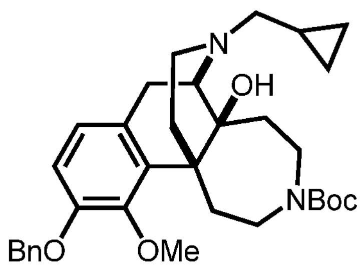

The title compound was obtained from the compound M obtained in Example 248 according to the methods described in Example 2 and Example 239.

$^1$H-NMR (400 MHz, $CDCl_3$)$\delta$ (ppm): 0.06 - 0.12 (m, 2H), 0.48 - 0.53 (m, 2H), 0.78 - 0.86 (m, 1H), 1.24 (s, 6.3H), 1.31 (s, 2.7H), 1.32 - 1.66 (m, 4H), 1.73 - 1.90 (m, 1H), 1.94 - 2.13 (m, 2H), 2.22 - 2.43 (m, 3.3H), 2.46 - 2.62 (m, 2H), 2.81 - 2.95 (m, 2.7H), 3.22 - 3.35 (m, 1.3H), 3.42 - 3.53 (m, 0.7H), 3.55 - 3.62 (m, 0.3H), 3.66 - 3.76 (m, 1H), 3.81 (s, 2.1H), 3.84 (s, 0.9H), 4.45 (br s, 0.7H), 5.00 (d, J = 11 Hz, 0.7H), 5.07 (d, J = 11 Hz, 0.7H), 5.09 (d, J = 12 Hz, 0.3H), 5.13 (d, J = 12 Hz, 0.3H), 6.69 (d, J = 8 Hz, 0.3H), 6.72 (d, J = 8 Hz, 0.7H), 6.77 (d, J = 8 Hz, 0.3H), 6.80 (d, J = 8 Hz, 0.7H), 7.30 - 7.49 (m, 5H).

(Example 250)

Synthesis of tert-butyl (5aS,6R,11bR)-14-(cyclopropylmethyl)-5a-hydroxy-11-methoxy-10-(((trifluoromethyl)sulfonyl)oxy)-1,2,5,5a,6,7-hexahydro-6,11b-(epiminoethano)naphtho[1,2-d]azepine-3(4H)-carboxylate

[Chemical Formula 303]

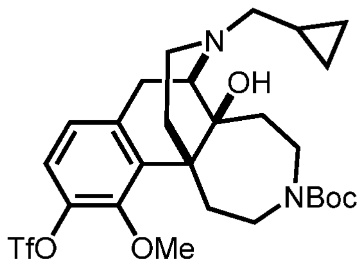

To the compound (64 mg, 0.12 mmol) obtained in Example 249 dissolved in methanol (4 mL), 10% palladium-activated carbon (55% wet) (93 mg) was added, followed by stirring under a hydrogen atmosphere at room temperature for 100 minutes. The reaction mixture was filtered through celite, and then the filtrate was concentrated under reduced pressure. The title compound was obtained from the obtained crude product according to the method described in Example 240.

$^1$H-NMR (400 MHz, $CDCl_3$)δ (ppm): 0.07 - 0.14 (m, 2H), 0.49 - 0.56 (m, 2H), 0.78 - 0.90 (m, 1H), 1.30 - 1.38 (m, 0.7H), 1.34 (s, 6.3H), 1.37 (s, 2.7H), 1.45 - 1.84 (m, 2.3H), 1.93 (ddd, J = 3, 12, 12 Hz, 1H), 2.00 - 2.06 (m, 0.3H), 2.15 (ddd, J = 5, 13, 13 Hz, 0.7H), 2.25 - 2.39 (m, 3H), 2.48 - 2.68 (m, 2H), 2.88 - 3.06 (m, 3.3H), 3.25 - 3.34 (m, 0.7H), 3.39 - 3.49 (m, 1.7H), 3.56 - 3.65 (m, 0.3H), 3.81 - 3.89 (m, 1H), 3.84 (s, 2.1H), 3.87 (s, 0.9H), 6.89 (d, J = 8 Hz, 1H), 7.04 - 7.09 (m, 1H).

(Example 251)

Synthesis of tert-butyl (5aS,6R,11bR)-10-cyano-14-(cyclopropylmethyl)-5a-hydroxy-11-methoxy-1,2,5,5a,6,7-hexahydro-6,11b-(epiminoethano)naphtho[1,2-d]azepine-3(4H)-carboxylate

[Chemical Formula 304]

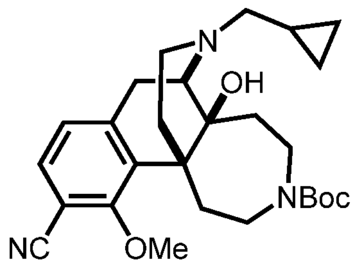

The title compound was obtained from the compound obtained in Example 250 according to the method described in Example 241.

$^1$H-NMR (400 MHz, $CDCl_3$)δ (ppm): 0.07 - 0.15 (m, 2H), 0.49 - 0.55 (m, 2H), 0.76 - 0.88 (m, 1H), 1.26 (s, 6.3H), 1.28 (s, 2.7H), 1.32 - 1.79 (m, 3.7H), 1.86 (ddd, J = 3, 12, 12 Hz, 1H), 1.99 - 2.17 (m, 1.4H), 2.23 - 2.39 (m, 2.3H), 2.43 - 2.53 (m, 0.3H), 2.59 - 2.70 (m, 1.7H), 2.87 - 3.04 (m, 3H), 3.11 - 3.23 (m, 1.3H), 3.37 - 3.56 (m, 1H), 3.69 - 3.80 (m, 1.3H), 4.08 (s, 2.1H), 4.14 (s, 0.9H), 6.81 - 6.86 (m, 1H), 7.29 - 7.34 (m, 1H).

(Example 252)

Synthesis of tert-butyl (5aS,6R,11bR)-10-carbamoyl-14-(cyclopropylmethyl)-5a-hydroxy-11-methoxy-1,2,5,5a,6,7-hexahydro-6,11b-(epiminoethano)naphtho[1,2-d]azepine-3(4H)-carboxylate

[Chemical Formula 305]

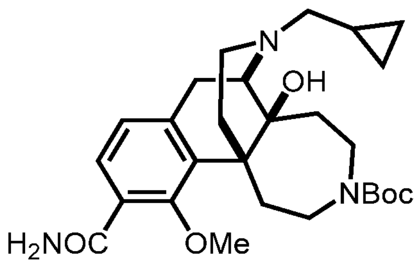

The title compound was obtained from the compound obtained in Example 251 according to the method described in Example 242.

$^1$H-NMR (400 MHz, $CDCl_3$)δ (ppm): 0.07 - 0.14 (m, 2H), 0.48 - 0.55 (m, 2H), 0.78 - 0.89 (m, 1H), 1.09 (s, 9H), 1.30 - 1.37 (m, 1H), 1.43 (dd, J = 4, 14 Hz, 1H), 1.55 - 1.83 (m, 1H), 1.94 - 2.14 (m, 3H), 2.26 - 2.40 (m, 2H), 2.53 - 2.79 (m, 2H), 2.86 - 3.02 (m, 4H), 3.07 - 3.16 (m, 1H), 3.47 - 3.55 (m, 1H), 3.78 (s, 3H), 4.43 (ddd, J = 7, 13, 13 Hz, 1H), 5.62 - 5.82 (m, 1H), 6.82 (d, J = 8 Hz, 1H), 7.26 (d, J = 8 Hz, 1H).

(Example 253)

Synthesis of (5aS,6R,11bS)-14-(cyclopropylmethyl)-5a-hydroxy-11-methoxy-3-(2-(4-methyl-1H-pyrazol-1-yl)ethyl)-1,2,3,4,5,5a,6,7-octahydro-6,11b-(epiminoethano)naphtho[1,2-d]azepine-10-carboxamide

[Chemical Formula 306]

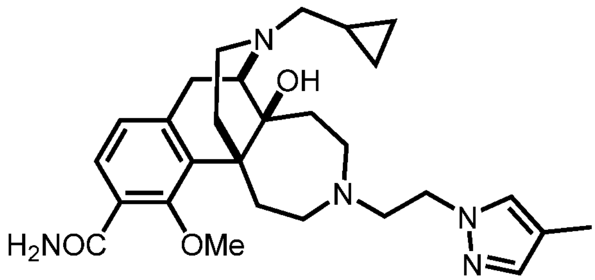

The title compound was obtained from the compound obtained in Example 252 according to the methods described in Example 243 and Example 31.

$^1$H-NMR (400 MHz, $CDCl_3$)δ (ppm): 0.05 - 0.16 (m, 2H), 0.45 - 0.56 (m, 2H), 0.77 - 0.92 (m, 1H), 1.30 - 1.37 (m, 1H), 1.44 (dd, J = 5, 14 Hz, 1H), 1.60 - 1.73 (m, 1H), 1.97 (s, 3H), 1.98 - 2.04 (m, 1H), 2.06 - 2.18 (m, 2H), 2.27 - 2.42 (m, 3H), 2.53 (ddd, J = 5, 13, 13 Hz, 1H), 2.60 - 2.68 (m, 2H), 2.69 - 2.79 (m, 2H), 2.89 - 3.03 (m, 4H), 3.09 - 3.18 (m, 1H), 3.75 (s, 3H), 3.84 - 3.97 (m, 2H), 4.53 (br s, 1H), 5.72 (br s, 1H), 6.76 (s, 1H), 6.96 (d, J = 8 Hz, 1H), 7.08 (br s, 1H), 7.17 (s, 1H), 7.67 (d, J = 8 Hz, 1H).

(Example 254)

Synthesis of (5aS,6R,11bR)-14-(cyclopropylmethyl)-5a-hydroxy-3-(2-(isothiazol-3-yl)acetyl)-1,2,3,4,5,5a,6,7-octahydro-6,11b-(epiminoethano)naphtho[1,2-d]azepine-10-carboxamide

[Chemical Formula 307]

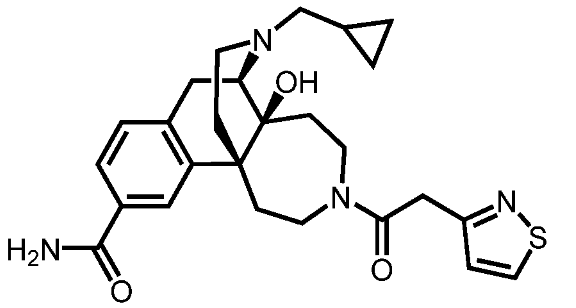

The title compound was obtained from the compound obtained in Example 243 and the compound obtained in Reference Example 21 according to the method described in Example 5.

$^1$H-NMR (400 MHz, $CD_3OD$)δ (ppm): 0.11 - 0.17 (m, 2H), 0.49 - 0.57 (m, 2H), 0.83 - 0.92 (m, 1H), 1.07 - 1.10 (m, 1H), 1.60 - 1.65 (m, 1H), 1.78 - 1.97 (m, 2H), 2.04 - 2.63 (m, 5.5H), 2.84 (dd, J = 6, 19 Hz, 0.5H), 2.95 (dd, J = 6, 19 Hz, 0.5H), 2.99 - 3.18 (m, 2H), 3.43 - 3.55 (m, 2H), 3.59 - 3.71 (m, 2H), 3.78 - 3.86 (m, 2H), 3.94 (d, J = 16 Hz, 0.5H), 6.98 (d, J = 4 Hz, 0.5H), 7.00 (d, J = 4 Hz, 0.5H), 7.23 (d, J = 8 Hz, 0.5H), 7.27 (d, J = 8 Hz, 0.5H), 7.64 (dd, J = 2, 8 Hz, 0.5H), 7.69 (dd, J = 2, 8 Hz, 0.5H), 7.73 (d, J = 2 Hz, 0.5H), 7.78 (d, J = 2 Hz, 0.5H), 8.77 (d, J = 4 Hz, 0.5H), 8.78 (d, J = 4 Hz, 0.5H).

(Reference Example 36)

Synthesis of (4'R,4a'S,7a'R,12b'S)-3'-(cyclopropylmethyl)-4a'-hydroxy-9'-methoxy-1',2',3',4',4a',5'-hexahydrospiro[cyclopentane-1,6'-[4,12]methanobenzofuro[3,2-e]isoquinolin]-7'(7a'H)-one

[Chemical Formula 308]

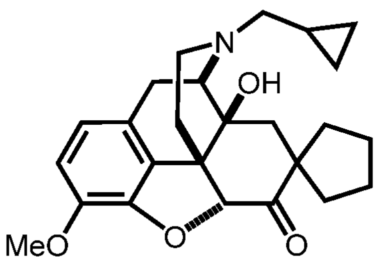

To (4R,4aS,7aR,12bS)-3-(cyclopropylmethyl)-4a-hydroxy-9-methoxy-2,3,4,4a,5,6-hexahydro-1H-4,12-methanobenzofuro[3,2-e]isoquinolin-7(7aH)-one (synthesized by the method described in Organic Letters 2009, 11, 539) (1.50 g, 4.22 mmol) and 1,4 dibromobutane (1.50 mL, 12.7 mmol) dissolved in toluene (42 mL) was added sodium tert-butoxide (2.37 g, 21.1 mmol), followed by heating under reflux for 24 hours under a nitrogen atmosphere. The reaction solution was allowed to cool to room temperature and filtered through celite, and the obtained filtrate was concentrated under reduced pressure. The obtained crude product was purified by silica gel column chromatography (0 to 100% ethyl acetate/chloroform and 0 to 10% methanol/ethyl acetate) to yield the title compound (650 mg, 38%) as a pale yellow oily matter.

$^1$H-NMR (400 MHz, $CDCl_3$)δ (ppm): 0.05 - 0.20 (m, 2H), 0.45 - 0.60 (m, 2H), 0.90 - 1.00 (m, 1H), 1.20 - 1.80 (m, 8H), 2.00 - 2.10 (m, 3H), 2.15 - 2.25 (m, 1H), 2.30 - 2.50 (m, 3H), 2.50 - 2.60 (m, 1H), 2.60 - 2.70 (m, 1H), 3.03 (d, J = 18 Hz, 1H), 3.09 (d, J = 14 Hz, 1H), 3.35 - 3.50 (m, 1H), 3.92 (s, 3H), 4.86 (s, 1H), 6.58 (d, J = 8 Hz, 1H), 6.68 (d, J = 8 Hz, 1H).

(Reference Example 37)

Synthesis of (4b'R,8a'S,9'R)-11'-(cyclopropylmethyl)-4',8a'-dihydroxy-3'-methoxy-8',8a',9', 10'-tetrahydrospiro[cyclopentane-1,7'-[9,4b](epiminoethano)phenanthren]-6'(5'H)-one

[Chemical Formula 309]

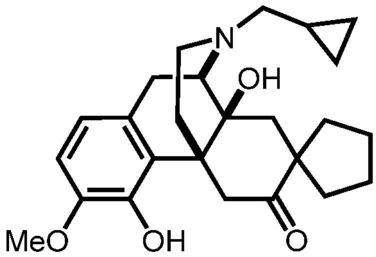

The title compound was obtained from the compound obtained in Reference Example 36 according to the method described in Reference Example 34.

$^1$H-NMR (400 MHz, $CDCl_3$)δ (ppm): 0.05 - 0.20 (m, 2H), 0.45 - 0.60 (m, 2H), 0.90 - 1.00 (m, 1H), 1.20 - 1.80 (m, 8H), 1.80 - 2.25 (m, 4H), 2.25 - 2.50 (m, 3H), 2.50 - 2.65 (m, 1H), 2.70 - 2.90 (m, 1H), 2.96 (d, J = 18 Hz, 1H), 3.00 - 3.10 (m, 1H), 3.21 (d, J = 13 Hz, 1H), 3.74 (d, J = 13 Hz, 1H), 3.82 (s, 3H), 6.06 (s, 1H), 6.53 (d, J = 8 Hz, 1H), 6.65 (d, J = 8 Hz, 1H).

(Reference Example 38)

Synthesis of (4b'R,8a'S,9'R)-11'-(cyclopropylmethyl)-8a'-hydroxy-3'-methoxy-6'-oxo-5',6',8',8a',9',10'-hexahydrospiro[cyclopentane-1,7'-[9,4b](epiminoethano)phenanthren]-4'-yl trifluoromethanesulfonate

[Chemical Formula 310]

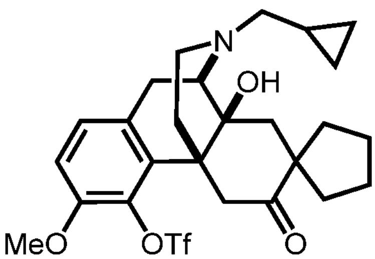

The title compound was obtained from the compound obtained in Reference Example 37 according to the method described in Reference Example 1.

$^1$H-NMR (400 MHz, $CDCl_3$)δ (ppm): 0.05 - 0.20 (m, 2H), 0.45 - 0.60 (m, 2H), 0.90 - 1.00 (m, 1H), 1.20 - 1.80 (m, 8H), 1.90 - 2.00 (m, 1H), 2.00 - 2.15 (m, 3H), 2.15 - 2.25 (m, 1H), 2.30 - 2.45 (m, 2H), 2.60 - 2.70 (m, 1H), 2.80 - 2.90 (m, 1H), 3.02 (d, J = 18 Hz, 1H), 3.09 (d, J = 6 Hz, 1H), 3.38 (d, J = 14 Hz, 1H), 3.42 (d, J = 14 Hz, 1H), 3.80 (s, 3H), 6.83 (d, J = 8 Hz, 1H), 7.03 (d, J = 8 Hz, 1H).

(Reference Example 39)

Synthesis of (4b'R,8a'S,9'R)-11'-(cyclopropylmethyl)-8a'-hydroxy-3'-methoxy-8',8a',9',10'-tetrahydrospiro[cyclopentane-1,7'-[9,4b](epiminoethano)phenanthren]-6'(5'H)-one

[Chemical Formula 311]

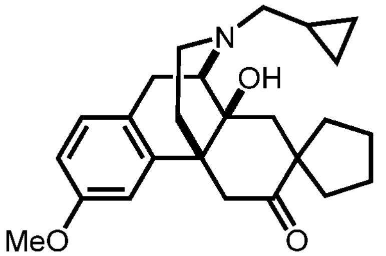

The title compound was obtained from the compound obtained in Reference Example 38 according to the method described in Reference Example 2.

$^1$H-NMR (400 MHz, $CDCl_3$)δ (ppm): 0.05 - 0.20 (m, 2H), 0.45 - 0.60 (m, 2H), 0.75 - 0.90 (m, 2H), 1.10 - 1.20 (m, 1H), 1.35 - 1.70 (m, 4H), 1.67 (d, J = 14 Hz, 1H), 1.79 (d, J = 14 Hz, 1H), 1.85 - 1.95 (m, 1H), 2.00 - 2.20 (m, 4H), 2.37 (d, J = 6 Hz, 2H), 2.50 - 2.60 (m, 1H), 2.65 - 2.80 (m, 2H), 2.95 - 3.10 (m, 2H), 3.32 (d, J = 14 Hz, 1H), 3.76 (s, 3H), 6.66 (dd, J = 3, 8 Hz, 1H), 6.80 (d, J = 3 Hz, 1H), 6.93 (d, J = 8 Hz, 1H).

(Reference Example 40)

Synthesis of (4b'R,8a'S,9'R)-11'-(cyclopropylmethyl)-8a'-hydroxy-3'-methoxy-8',8a',9',10'-tetrahydrospiro[cyclopentane-1,7'-[9,4b](epiminoethano)phenanthren]-6'(5'H)-one oxime

[Chemical Formula 312]

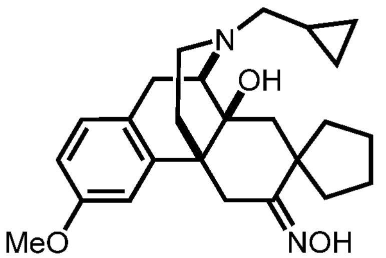

The title compound was obtained from the compound obtained in Reference Example 39 according to the method described in Reference Example 3.

$^1$H-NMR (400 MHz, $CDCl_3$)δ (ppm): 0.05 - 0.20 (m, 2H), 0.45 - 0.60 (m, 2H), 0.75 - 0.90 (m, 1H), 0.90 - 1.00 (m, 1H), 1.00 - 1.20 (m, 1H), 1.40 - 1.80 (m, 6H), 1.80 - 2.20 (m, 5H), 2.25 - 2.45 (m, 2H), 2.50 (d, J = 14 Hz, 1H), 2.50 - 2.60 (m, 1H), 2.60 - 2.80 (m, 1H), 2.90 - 3.10 (m, 2H), 3.55 (s, 3H), 3.69 (d, J = 14 Hz, 1H), 6.69 (dd, J = 3, 8 Hz, 1H), 6.94 (d, J = 8 Hz, 1H), 7.09 (d, J = 3 Hz, 1H), 8.39 (br s, 1H).

(Example 255)

Synthesis of (5a'S,6'R,11b'R)-14'-(cyclopropylmethyl)-5a'-hydroxy-10'-methoxy-5',5a',6',7'-tetrahydro-1'H-spiro[cyclopentane-1,4'-[6,11b](epiminoethano)naphtho[1,2-d]azepin]-2'(3'H)-one

[Chemical Formula 313]

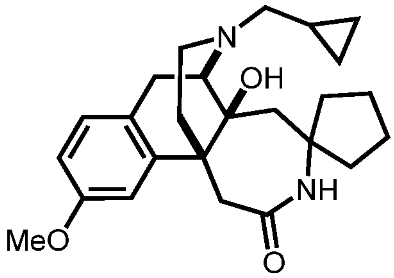

The title compound was obtained from the compound obtained in Reference Example 40 according to the method described in Example 1.

$^1$H-NMR (400 MHz, $CDCl_3$)δ (ppm): 0.05 - 0.20 (m, 2H), 0.45 - 0.60 (m, 2H), 0.75 - 0.90 (m, 1H), 1.00 - 1.15 (m, 1H), 1.40 - 1.85 (m, 8H), 2.00 - 2.25 (m, 4H), 2.30 - 2.40 (m, 2H), 2.55 - 2.60 (m, 1H), 2.66 (dd, J = 2, 15 Hz, 1H), 2.78 (dd, J = 6, 18 Hz, 1H), 2.87 (d, J = 6 Hz, 1H), 3.00 (d, J = 18 Hz, 1H), 3.57 (d, J = 15 Hz, 1H), 3.79 (s, 3H), 4.82 (br s, 1H), 5.40 (s, 1H), 6.70 (dd, J = 3, 8 Hz, 1H), 6.97 (d, J = 8 Hz, 1H), 7.12 (d, J = 3 Hz, 1H).

(Example 256)

Synthesis of (5a'S,6'R,11b'S)-14'-(cyclopropylmethyl)-10'-methoxy-2',3',6',7'-tetrahydro-1'H-spiro[cyclopentane-1,4'-[6,11b](epiminoethano)naphtho[1,2-d]azepin]-5a'(5'H)-ol

[Chemical Formula 314]

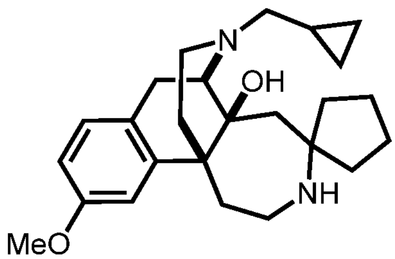

The title compound was obtained from the compound obtained in Example 255 according to the method described in Example 2.

$^1$H-NMR (400 MHz, $CDCl_3$)δ (ppm): 0.05 - 0.20 (m, 2H), 0.45 - 0.60 (m, 2H), 0.75 - 0.90 (m, 1H), 1.00 - 1.15 (m, 2H), 1.40 - 1.70 (m, 5H), 1.70 - 1.85 (m, 2H), 1.85 - 2.10 (m, 3H), 2.25 - 2.45 (m, 3H), 2.50 - 2.65 (m, 2H), 2.76 (dd, J = 6, 18 Hz, 1H), 2.85 (d, J = 6 Hz, 1H), 2.95 - 3.10 (m, 2H), 3.79 (s, 3H), 4.46 (br s, 1H), 6.65 - 6.70 (m, 2H), 7.03 (d, J = 8 Hz, 1H).

(Example 257)

Synthesis of 1-((5a'S,6'R,11b'R)-14'-(cyclopropylmethyl)-5a'-hydroxy-10'-methoxy-1',2',5',5a',6',7'-hexahydro-3'H-spiro[cyclopentane-1,4'-[6,11b](epiminoethano)naphtho[1,2-d]azepin]-3'-yl)-2-(4-methyl-1H-pyrazol-1-yl)ethan-1-one

[Chemical Formula 315]

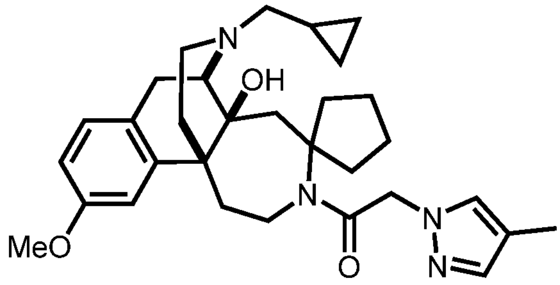

The title compound was obtained from the compound obtained in Example 256 and 4-methylpyrazole according to the method described in Example 132.

$^1$H-NMR (400 MHz, $CDCl_3$)δ (ppm): 0.05 - 0.20 (m, 2H), 0.45 - 0.60 (m, 2H), 0.75 - 0.90 (m, 1H), 1.00 - 1.10 (m, 1H), 1.30 - 2.60 (m, 21H), 2.80 - 3.00 (m, 3H), 3.30 - 3.60 (m, 1H), 3.79 (s, 3H), 4.60 - 4.80 (m, 2H), 6.70 - 6.80 (m, 2H), 7.00 - 7.10 (m, 2H), 7.20 - 7.30 (m, 1H).

(Example 258)

Synthesis of (5a'S,6'R,11b'R)-14'-(cyclopropylmethyl)-10'-methoxy-3'-(2-(4-methyl-1H-pyrazol-1-yl)ethyl)-2',3',6',7'-tetrahydro-1'H-spiro[cyclopentane-1,4'-[6,11b](epiminoethano)naphtho[1,2-d]azepin]-5a'(5'H)-ol

[Chemical Formula 316]

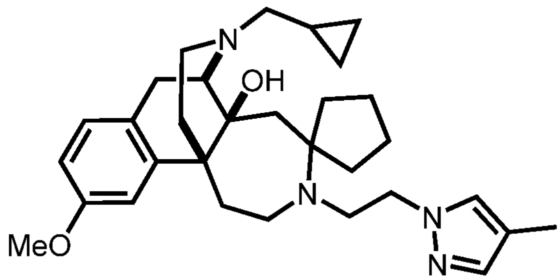

The title compound was obtained from the compound obtained in Example 257 according to the method described in Example 24.

$^1$H-NMR (400 MHz, $CDCl_3$)δ (ppm): 0.05 - 0.30 (m, 2H), 0.50 - 0.70 (m, 2H), 0.80 - 1.00 (m, 1H), 1.00 - 1.30 (m, 2H), 1.30 - 2.10 (m, 18H), 2.09 (s, 3H), 2.10 - 2.30 (m, 1H), 2.30 - 2.45 (m, 1H), 2.45 - 2.70 (m, 2H), 2.70 - 2.90 (m, 2H), 3.00 - 3.15 (m, 1H), 4.11 (br s, 1H), 6.66 (d, J = 8 Hz, 1H), 6.74 (s, 1H), 6.93 (d, J = 8 Hz, 1H), 7.27 (s, 1H), 7.52 (s, 1H).

(Example 259)

Synthesis of (5aS,6R,11bR)-9-bromo-14-(cyclopropylmethyl)-5a-hydroxy-10-methoxy-3,4,5,5a,6,7-hexahydro-6,11b-(epiminoethano)naphtho[1,2-d]azepin-2(1H)-one

[Chemical Formula 317]

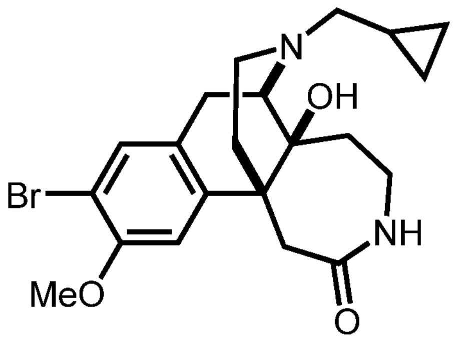

The compound (356 mg, 0.999 mmol) obtained in Example 140 was suspended in water (10 mL), and dissolved by adding trifluoroacetic acid (1.0 mL). Under ice cooling, N-bromosuccinimide (356 mg, 1.99 mmol) was added, followed by stirring at 0°C for 16 hours. To the reaction mixture, a mixed solution of a 2 M aqueous sodium hydroxide solution and a saturated aqueous sodium thiosulfate solution (v/v = 4/1, 10 mL) was added, followed by extraction three times with ethyl acetate. The organic layers were combined, washed with saturated saline, dried over sodium sulfate, and then concentrated under reduced pressure. The obtained crude product was purified by silica gel column chromatography (amino group-supported silica gel, 0 to 10% methanol/ethyl acetate) to yield the title compound (368 mg, 85%) as a white solid.

$^1$H-NMR (400 MHz, $CDCl_3$)$\delta$ (ppm): 0.07 - 0.18 (m, 2H), 0.46 - 0.59 (m, 2H), 0.76 - 0.88 (m, 1H), 1.05 - 1.14 (m, 1H), 1.50 - 1.69 (m, 2H), 2.00 - 2.12 (m, 2H), 2.34 (dd, J = 6, 14 Hz, 1H), 2.35 (dd, J = 6, 14 Hz, 1H), 2.51 - 2.65 (m, 2H), 2.74 (dd, J = 6, 18 Hz, 1H), 2.79 - 2.88 (m, 1H), 2.91 (d, J = 6 Hz, 1H), 2.98 (d, J = 18 Hz, 1H), 3.50 (d, J = 14 Hz, 1H), 3.79 - 3.94 (m, 1H), 3.90 (s, 3H), 4.69 (br s, 1H), 6.04 (br s, 1H), 7.16 (s, 1H), 7.24 (s, 1H).

(Example 260)

Synthesis of (5aS,6R,11bR)-14-(cyclopropylmethyl)-5a-hydroxy-10-methoxy-9-methyl-3,4,5,5a,6,7-hexahydro-6,11b-(epiminoethano)naphtho[1,2-d]azepin-2(1H)-one

[Chemical Formula 318]

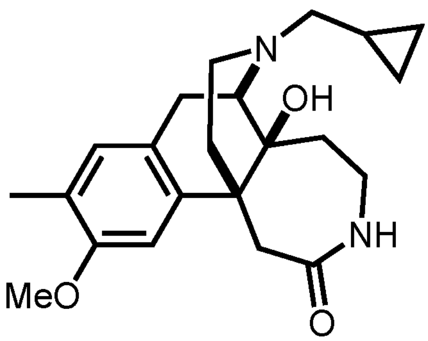

The compound (368 mg, 0.846 mmol) obtained in Example 259, potassium carbonate (351 mg, 2.54 mmol), [1,1'-bis(diphenylphosphino)ferrocene]palladium (II) dichloride dichloromethane adduct (345 mg, 0.423 mmol), and trimethylboroxine (0.590 mL, 4.22 mmol) were suspended in N,N-dimethylformamide (8.0 mL), followed by stirring at 80°C for 15 hours. After allowed to cool to room temperature, a saturated aqueous sodium bicarbonate solution and water were added, followed by extraction three times with ethyl acetate. The organic layers were combined, washed with water and saturated saline, dried over sodium sulfate, and then concentrated under reduced pressure. The obtained crude product was purified by silica gel column chromatography (0 to 20% methanol/ethyl acetate and amino group-supported silica gel, 1 to 30% methanol/ethyl acetate) to yield the title compound (244 mg, 78%) as a pale yellow amorphous form.

$^1$H-NMR (400 MHz, $CDCl_3$)$\delta$ (ppm): 0.05 - 0.18 (m, 2H), 0.45 - 0.56 (m, 2H), 0.77 - 0.89 (m, 1H), 1.08 - 1.17 (m, 1H), 1.53 (dd, J = 6, 14 Hz, 1H), 1.77 (ddd, J = 2, 12, 14 Hz, 1H), 2.02 - 2.13 (m, 2H), 2.14 (s, 3H), 2.35 (dd, J = 6,

13 Hz, 1H), 2.36 (dd, J = 6, 13 Hz, 1H), 2.51 - 2.63 (m, 1H), 2.60 (dd, J = 2, 15 Hz, 1H), 2.72 (dd, J = 6, 18 Hz, 1H), 2.81 - 2.92 (m, 1H), 2.90 (d, J = 6 Hz, 1H), 2.98 (d, J = 18 Hz, 1H), 3.50 (d, J = 15 Hz, 1H), 3.81 - 3.91 (m, 1H), 3.84 (s, 3H), 4.73 (br s, 1H), 5.63 - 5.72 (m, 1H), 6.82 (s, 1H), 7.06 (s, 1H).

(Example 261)

Synthesis of (5aS,6R,11bS)-14-(cyclopropylmethyl)-10-methoxy-9-methyl-2,3,4,5,6,7-hexahydro-6,11b-(epiminoethano)naphtho[1,2-d]azepin-5a(1H)-ol

[Chemical Formula 319]

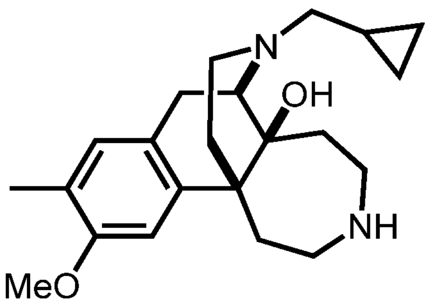

The title compound was obtained from the compound obtained in Example 260 according to the method described in Example 2.

$^1$H-NMR (400 MHz, $CDCl_3$)$\delta$ (ppm): 0.04 - 0.17 (m, 2H), 0.43 - 0.57 (m, 2H), 0.78 - 0.90 (m, 1H), 1.00 - 1.11 (m, 1H), 1.56 (ddd, J = 3, 3, 15 Hz, 1H), 1.75 (ddd, J = 4, 12, 15 Hz, 1H), 1.95 - 2.08 (m, 3H), 2.17 (s, 3H), 2.25 - 2.40 (m, 3H), 2.47 - 2.58 (m, 1H), 2.74 - 2.82 (m, 2H), 2.89 - 3.00 (m, 1H), 2.91 (d, J = 7 Hz, 1H), 2.95 (d, J = 19 Hz, 1H), 3.00 (ddd, J = 3, 6, 14 Hz, 1H), 3.07 (ddd, J = 3, 12, 14 Hz, 1H), 3.79 (s, 3H), 4.52 (br s, 1H), 6.61 (s, 1H), 6.89 (s, 1H).

(Example 262)

Synthesis of (5aS,6R,11bS)-14-(cyclopropylmethyl)-9-methyl-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 320]

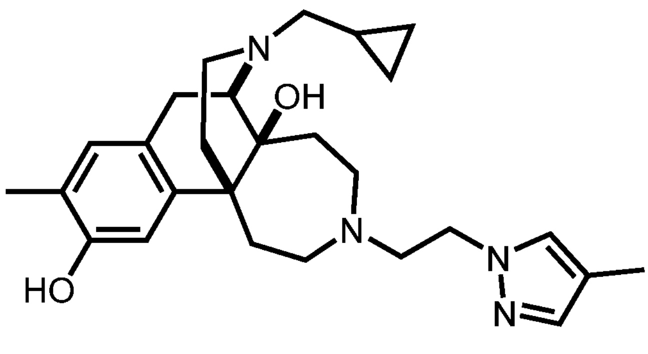

The title compound was obtained from the compound obtained in Example 261 and the compound obtained in Reference Example 33 according to the methods described in Example 31 and Example 6.

$^1$H-NMR (400 MHz, $CDCl_3$)$\delta$ (ppm): 0.05 - 0.16 (m, 2H), 0.46 - 0.55 (m, 2H), 0.76 - 0.88 (m, 1H), 0.95 - 1.05 (m, 1H), 1.39 - 1.46 (m, 1H), 1.72 - 1.82 (m, 2H), 1.95 - 2.07 (m, 2H), 2.00 (s, 3H), 2.19 (s, 3H), 2.28 - 2.40 (m, 3H), 2.45 - 2.61 (m, 3H), 2.73 (dd, J = 6, 18 Hz, 1H), 2.78 - 2.99 (m, 5H), 3.11 - 3.20 (m, 1H), 3.96 - 4.08 (m, 2H), 4.62 (br s, 1H), 6.51 (s, 1H), 6.48 (s, 1H), 6.87 (s, 1H), 7.23 (s, 1H).

(Example 263)

Synthesis of (5aS,6R,11bS)-9-bromo-14-(cyclopropylmethyl)-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 321]

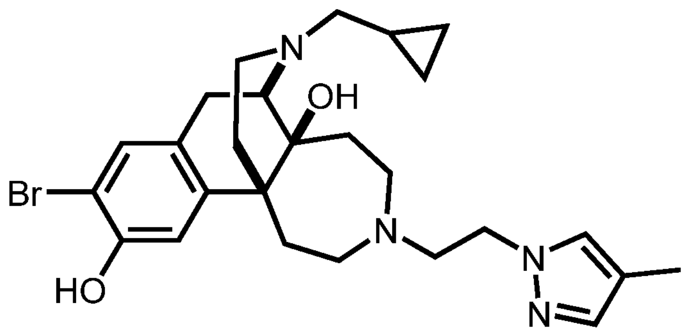

The title compound was obtained from the compound obtained in Example 259 according to the methods described in Example 2, Example 31, and Example 6.

$^1$H-NMR (400 MHz, $CDCl_3$)$\delta$ (ppm): 0.05 - 0.16 (m, 2H), 0.43 - 0.57 (m, 2H), 0.76 - 0.88 (m, 1H), 0.96 - 1.03 (m, 1H), 1.45 (ddd, J = 2, 5, 14 Hz, 1H), 1.72 (ddd, J = 3, 11, 14 Hz, 1H), 1.77 (ddd, J = 4, 4, 16 Hz, 1H), 1.92 - 2.09 (m, 2H), 2.01 (s, 3H), 2.31 - 2.38 (m, 1H), 2.34 (dd, J = 7, 13 Hz, 1H), 2.39 (ddd, J = 4, 11, 16 Hz, 1H), 2.45 (ddd, J = 3, 5, 14 Hz, 1H), 2.51 - 2.58 (m, 1H), 2.59 (ddd, J = 4, 4, 13 Hz, 1H), 2.76 (dd, J = 6, 18 Hz, 1H), 2.80 - 2.95 (m, 3H), 2.89 (d, J = 6 Hz, 1H), 2.94 (d, J = 18 Hz, 1H), 3.13 (ddd, J = 2, 11, 13 Hz, 1H), 3.97 (ddd, J = 6, 6, 14 Hz, 1H), 4.01 (ddd, J = 6, 6, 14 Hz, 1H), 4.58 (br s, 1H), 6.76 (s, 1H), 6.82 (s, 1H), 7.21 (s, 1H), 7.22 (s, 1H).

(Example 264)

Synthesis of (5aS,6R,11bR)-14-(cyclopropylmethyl)-5a-hydroxy-3-(3-(4-(trifluoromethyl)-1H-pyrazol-1-yl)prop anoyl)-1,2,3,4,5,5a,6,7-octahydro-6,11b-(epiminoethano)naphtho[1,2-d]azepine-10-carboxamide

[Chemical Formula 322]

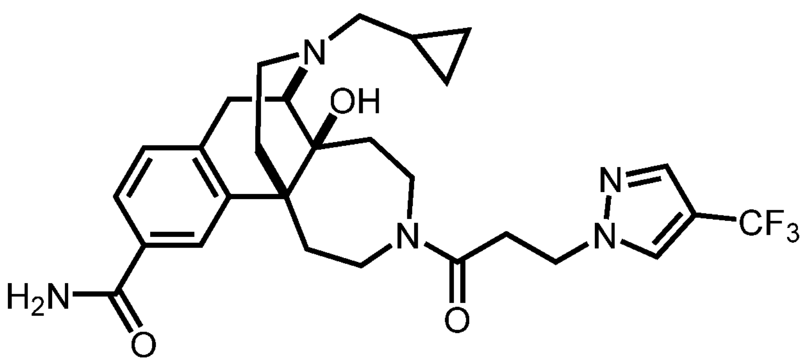

The title compound was obtained from the compound obtained in Example 243 and 3-(4-(trifluoromethyl)-1H-pyrazol-1-yl)propanoic acid according to the method described in Example 5.

$^1$H-NMR (400 MHz, $CDCl_3$)$\delta$ (ppm): 0.04 - 0.18 (m, 2H), 0.46 - 0.58 (m, 2H), 0.76 - 0.88 (m, 1H), 1.04 - 1.13 (m, 1H), 1.57 - 1.76 (m, 1.5H), 1.85 (ddd, J = 4, 12, 12 Hz, 0.5H), 1.91 - 2.20 (m, 2.5H), 2.25 - 2.38 (m, 2.5H), 2.41 -

2.73 (m, 3H), 2.77 - 2.90 (m, 1.5H), 2.95 - 3.00 (m, 0.5H), 2.98 (d, J = 6 Hz, 1H), 2.99 (d, J = 19 Hz, 0.5H), 3.11 (d, J = 19 Hz, 0.5H), 3.23 - 3.40 (m, 1.5H), 3.56 - 3.72 (m, 1.5H), 3.79 (ddd, J = 4, 11, 15 Hz, 0.5H), 3.89 (ddd, J = 3, 3, 14 Hz, 0.5H), 4.15 - 4.24 (m, 1H), 4.25 - 4.34 (m, 1H), 4.47 (br s, 1H), 5.40 (br s, 0.5H), 5.70 (br s, 0.5H), 6.55 (br s, 0.5H), 6.68 (br s, 0.5H), 7.02 (d, J = 8 Hz, 0.5H), 7.14 (d, J = 8 Hz, 0.5H), 7.23 (dd, J = 2, 8 Hz, 0.5H), 7.45 (dd, J = 2, 8 Hz, 0.5H), 7.53 (s, 0.5H), 7.54 (d, J = 2 Hz, 0.5H), 7.63 (s, 1H), 7.69 (s, 0.5H), 7.74 (s, 0.5H).

(Example 265)

Synthesis of (5aS,6R,11bR)-3-(2-(1H-indazol-3-yl)acetyl)-14-(cyclopropylmethyl)-5a-hydroxy-1,2,3,4,5,5a,6,7-octahydro-6,11b-(epiminoethano)naphtho[1,2-d]azepine-10-carboxamide

[Chemical Formula 323]

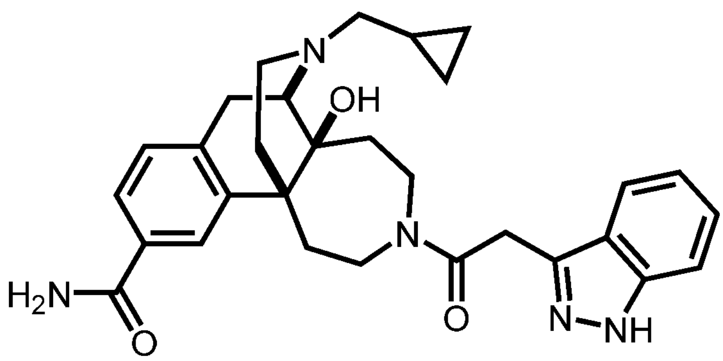

The title compound was obtained from the compound obtained in Example 243 and 2-(1H-indazol-3-yl)acetic acid according to the method described in Example 5.

$^{1}$H-NMR (400 MHz, $CD_3OD$)δ (ppm): 0.08 - 0.20 (m, 2H), 0.46 - 0.58 (m, 2H), 0.81 - 0.92 (m, 1H), 1.02 - 1.12 (m, 1H), 1.53 - 1.66 (m, 1.4H), 1.84 (ddd, J = 3, 12, 15 Hz, 0.6H), 1.92 (ddd, J = 3, 12, 15 Hz, 0.6H), 2.02 - 2.27 (m, 1.4H), 2.29 - 2.65 (m, 3.6H), 2.70 (dd, J = 6, 19 Hz, 0.4H), 2.92 - 3.01 (m, 1H), 3.06 (d, J = 6 Hz, 0.6H), 3.08 (d, J = 19 Hz, 0.4H), 3.16 (d, J = 19 Hz, 0.6H), 3.27 - 3.39 (m, 2H), 3.44 - 3.79 (m, 3H), 3.68 (d, J = 16 Hz, 0.6H), 3.85 (ddd, J = 7, 7, 15 Hz, 0.4H), 3.93 (d, J = 16 Hz, 0.4H), 3.94 (d, J = 16 Hz, 0.6H), 4.05 (d, J = 16 Hz, 0.4H), 6.99 - 7.12 (m, 1.4H), 7.26 - 7.38 (m, 1.6H), 7.41 - 7.50 (m, 2H), 7.52 (dd, J = 2, 8 Hz, 0.4H), 7.69 (dd, J = 2, 7 Hz, 0.6H), 7.71 (dd, J = 2, 8 Hz, 0.4H), 7.81 (d, J = 2 Hz, 0.6H).

(Example 266)

Synthesis of (5aS,6R,11bR)-14-(cyclopropylmethyl)-5a-hydroxy-3-(2-(4-(trifluoromethyl)-1H-pyrazol-1-yl)acetyl)-1,2,3,4,5,5a,6,7-octahydro-6,11b-(epiminoethano)naphtho[1,2-d]azepine-10-carboxamide

[Chemical Formula 324]

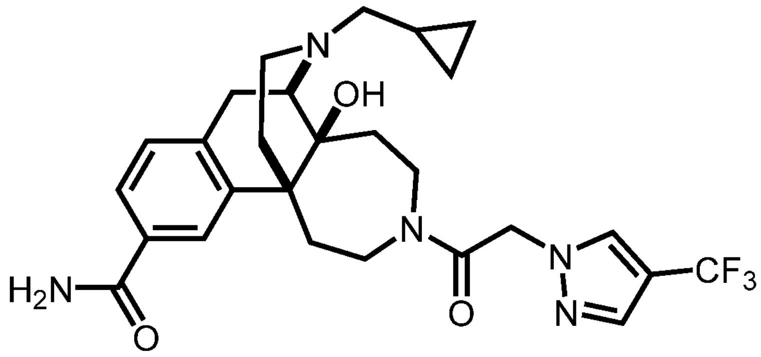

The title compound was obtained from the compound obtained in Example 243 and 2-(4-(trifluoromethyl)-1H-pyrazol-1-yl)acetic acid according to the method described in Example 5.

$^1$H-NMR (400 MHz, $CDCl_3$)δ (ppm): 0.06 - 0.19 (m, 2H), 0.50 - 0.59 (m, 2H), 0.78 - 0.90 (m, 1H), 1.07 - 1.17 (m, 1H), 1.59 - 1.83 (m, 2H), 1.91 (ddd, J = 4, 12, 12 Hz, 0.5H), 1.95 - 2.17 (m, 2H), 2.21 (ddd, J = 4, 4, 16 Hz, 0.5H), 2.31 (dd, J = 7, 13 Hz, 0.5H), 2.36 (d, J = 6 Hz, 1H), 2.38 (dd, J = 7, 13 Hz, 0.5H), 2.47 (ddd, J = 5, 11, 16 Hz, 0.5H), 2.54 - 2.64 (m, 1H), 2.74 - 2.85 (m, 0.5H), 2.86 - 2.90 (m, 0.5H), 2.91 - 2.97 (m, 0.5H), 2.99 (d, J = 6 Hz, 0.5H), 3.00 (d, J = 6 Hz, 0.5H), 3.05 (d, J = 19 Hz, 0.5H), 3.13 (d, J = 19 Hz, 0.5H), 3.26 - 3.50 (m, 1.5H), 3.66 (ddd, J = 4, 12, 15 Hz, 0.5H), 3.71 - 3.82 (m, 1.5H), 3.90 (ddd, J = 3, 4, 14 Hz, 0.5H), 4.50 (br s, 1H), 4.58 (d, J = 16 Hz, 0.5H), 4.75 (d, J = 16 Hz, 0.5H), 4.78 (d, J = 16 Hz, 0.5H), 4.87 (d, J = 16 Hz, 0.5H), 5.64 (br s, 1H), 6.44 (br s, 1H), 7.17 (d, J = 8 Hz, 0.5H), 7.19 (d, J = 7 Hz, 0.5H), 7.44 (d, J = 2, 8 Hz, 0.5H), 7.45 - 7.52 (m, 0.5H), 7.49 (s, 0.5H), 7.55 (d, J = 2 Hz, 0.5H), 7.58 (s, 0.5H), 7.62 (s, 0.5H), 7.64 (s, 0.5H), 7.76 (d, J = 2 Hz, 0.5H).

(Reference Example 41)

Synthesis of (4R,4aS,7aR,12bS)-3-(cyclopropylmethyl)-4a-hydroxy-9-methoxy-6,6-dimethyl-2,3,4,4a,5,6-hexahydro-1H-4,12-methanobenzofuro[3,2-e]isoquinolin-7(7aH)-one

[Chemical Formula 325]

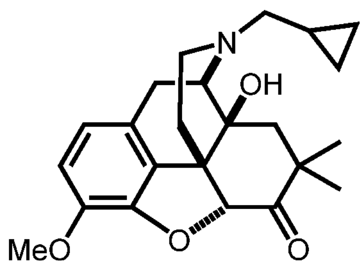

To a solution of (4R,4aS,7aR,12bS)-3-(cyclopropylmethyl)-4a-hydroxy-9-methoxy-2,3,4,4a,5,6-hexahydro-1H-4,12-methanobenzofuro[3,2-e]isoquinolin-7(7aH)-one (synthesized by the method described in Organic Letters 2009, 11, 539) (1.50 g, 4.22 mmol) in tetrahydrofuran (37 mL) cooled to -78°C was added a 1.08 M lithium bis(trimethylsilyl)amide-tetrahydrofuran solution (11.7 mL, 12.6 mmol), followed by stirring for 1 hour. To the reaction mixture, a solution of methyl iodide (1.58 mL, 25.4 mmol) in tetrahydrofuran (3 mL) was added, followed by stirring at -78°C for 1 hour and at room temperature for 18 hours. To the reaction mixture, a saturated aqueous sodium bicarbonate solution was added, followed by extraction three times with ethyl acetate. The combined extracts were washed with saturated saline, dried over sodium sulfate, and then concentrated under reduced pressure. The obtained crude product was purified by silica gel column chromatography (47 to 68% ethyl acetate/heptane) to yield the title compound (574 mg, 36%) as a pale yellow crystal.

$^1$H-NMR (400 MHz, $CDCl_3$)δ (ppm): 0.12 - 0.16 (m, 2H), 0.53 - 0.57 (m, 2H), 0.82 - 0.88 (m, 1H), 0.95 (s, 3H), 1.44 (s, 3H), 1.52 - 1.62 (m, 2H), 1.73 (d, J = 14 Hz, 1H), 2.09 (ddd, J = 4, 12, 12 Hz, 1H), 2.32 - 2.50 (m, 3H), 2.57 (dd, J = 6, 19 Hz, 1H), 2.63 - 2.71 (m, 1H), 3.02 (d, J = 19 Hz, 1H), 3.11 (d, J = 6 Hz, 1H), 3.92 (s, 3H), 4.87 (s, 1H), 5.45 (br s, 1H), 6.60 (d, J = 8 Hz, 1H), 6.69 (d, J = 8 Hz, 1H).

(Reference Example 42)

Synthesis of (4bR,8aS,9R)-11-(cyclopropylmethyl)-4,8a-dihydroxy-3-methoxy-7,7-dimethyl-8,8a,9,10-tetrahydro-5H-9,4b-(epiminoethano)phenanthren-6(7H)-one

[Chemical Formula 326]

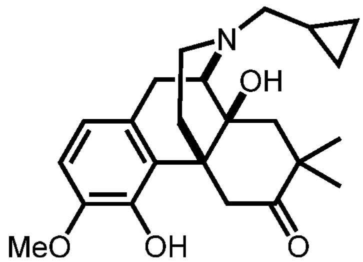

The title compound was obtained from the compound obtained in Reference Example 41 according to the method described in Reference Example 34.

$^1$H-NMR (400 MHz, $CDCl_3$)δ (ppm): 0.09 - 0.13 (m, 2H), 0.50 - 0.54 (m, 2H), 0.82 (s, 3H), 0.82 - 0.88 (m, 1H), 1.40 (s, 3H), 1.65 (d, J = 14 Hz, 1H), 1.81 (d, J = 14 Hz, 1H), 1.97 - 2.11 (m, 2H), 2.31 (dd, J = 6, 13 Hz, 1H), 2.37 (dd, J = 6, 13 Hz, 1H), 2.56 - 2.60 (m, 1H), 2.83 (dd, J = 6, 19 Hz, 1H), 2.96 (d, J = 19 Hz, 1H), 3.06 (d, J = 6 Hz, 1H), 3.23 (d, J = 13 Hz, 1H), 3.73 (d, J = 13 Hz, 1H), 3.82 (s, 3H), 4.87 (br s, 1H), 6.06 (s, 1H), 6.54 (d, J = 8 Hz, 1H), 6.64 (d, J = 8 Hz, 1H).

(Reference Example 43)

Synthesis of (4bR,8aS,9R)-11-(cyclopropylmethyl)-8a-hydroxy-3-methoxy-7,7-dimethyl-6-oxo-6,7,8,8a,9,10-hexahydro-5H-9,4b-(epiminoethano)phenanthren-4-yl trifluoromethanesulfonate

[Chemical Formula 327]

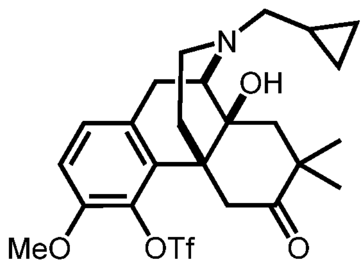

The title compound was obtained from the compound obtained in Reference Example 42 according to the method described in Reference Example 1.

$^1$H-NMR (400 MHz, $CDCl_3$)δ (ppm): 0.10 - 0.14 (m, 2H), 0.52 - 0.57 (m, 2H), 0.78 (s, 3H), 0.81 - 0.89 (m, 1H), 1.38 (s, 3H), 1.61 - 1.70 (m, 3H), 2.06 (ddd, J = 3, 12, 12 Hz, 1H), 2.21 (ddd, J = 5, 12, 12 Hz, 1H), 2.33 (dd, J = 6, 12 Hz, 1H), 2.39 (dd, J = 6, 12 Hz, 1H), 2.66 - 2.69 (m, 1H), 2.87 (dd, J = 6, 18 Hz, 1H), 3.02 (d, J = 18 Hz, 1H), 3.11 (d, J = 6 Hz, 1H), 3.37 (d, J = 14 Hz, 1H), 3.44 (d, J = 14 Hz, 1H), 3.80 (s, 3H), 6.83 (d, J = 8 Hz, 1H), 7.03 (d, J = 8 Hz, 1H).

(Reference Example 44)

Synthesis of (4bR,8aS,9R)-11-(cyclopropylmethyl)-8a-hydroxy-3-methoxy-7,7-dimethyl-8,8a,9,10-tetrahydro-5H-9,4b-(epiminoethano)phenanthren-6(7H)-one

[Chemical Formula 328]

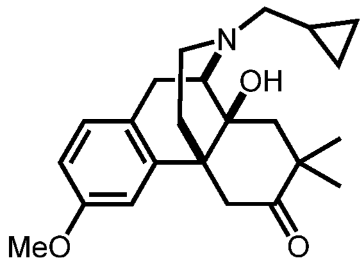

The title compound was obtained from the compound obtained in Reference Example 43 according to the method described in Reference Example 2.

$^1$H-NMR (400 MHz, $CDCl_3$)δ (ppm): 0.11 - 0.15 (m, 2H), 0.51 - 0.56 (m, 2H), 0.79 (s, 3H), 0.80 - 0.91 (m, 1H), 1.14 - 1.17 (m, 1H), 1.40 (s, 3H), 1.64 (d, J = 14 Hz, 1H), 1.71 (d, J = 14 Hz, 1H), 2.07 (ddd, J = 3, 12, 12 Hz, 1H), 2.16 (ddd, J = 4, 13, 13 Hz, 1H), 2.34 (dd, J = 6, 13 Hz, 1H), 2.41 (dd, J = 6, 13 Hz, 1H), 2.56 - 2.60 (m, 1H), 2.72 (d, J = 14 Hz, 1H), 2.75 (dd, J = 6, 18 Hz, 1H), 3.04 (d, J = 18 Hz, 1H), 3.09 (d, J = 6 Hz, 1H), 3.36 (d, J = 14 Hz, 1H), 3.77 (s, 3H), 6.68 (dd, J = 2, 8 Hz, 1H), 6.79 (d, J = 2 Hz, 1H), 6.96 (d, J = 8 Hz, 1H).

(Reference Example 45)

Synthesis of (4bR,8aS,9R)-11-(cyclopropylmethyl)-8a-hydroxy-3-methoxy-7,7-dimethyl-8,8a,9,10-tetrahydro-5H-9,4b-(epiminoethano)phenanthren-6(7H)-one oxime

[Chemical Formula 329]

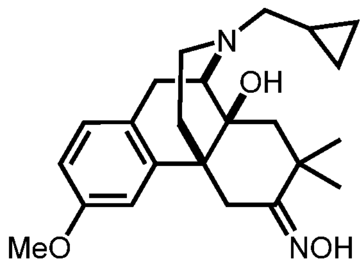

The title compound was obtained from the compound obtained in Reference Example 44 according to the method described in Reference Example 3.

$^1$H-NMR (400 MHz, $CDCl_3$)δ (ppm): 0.09 - 0.13 (m, 2H), 0.49 - 0.53 (m, 2H), 0.80 - 0.89 (m, 1H), 0.90 (s, 3H), 1.12 - 1.15 (m, 1H), 1.42 (s, 3H), 1.43 - 1.60 (m, 2H), 2.04 - 2.17 (m, 2H), 2.32 (dd, J = 6, 13 Hz, 1H), 2.39 (dd, J = 6, 13 Hz, 1H), 2.53 - 2.59 (m, 2H), 2.71 (dd, J = 6, 18 Hz, 1H), 2.99 (d, J = 6 Hz, 1H), 3.01 (d, J = 18 Hz, 1H), 3.69 (d, J = 14 Hz, 1H), 3.74 (s, 3H), 6.68 (dd, J = 2, 8 Hz, 1H), 6.94 (d, J = 8 Hz, 1H), 6.99 (br s, 1H), 7.08 (d, J = 2 Hz, 1H).

(Example 267)

Synthesis of (5aS,6R,11bR)- 14-(cyclopropylmethyl)-5a-hydroxy-10-methoxy-4,4-dimethyl-3,4,5,5a,6,7-hexahydro-6,11b-(epiminoethano)naphtho[1,2-d]azepin-2(1H)-one

[Chemical Formula 330]

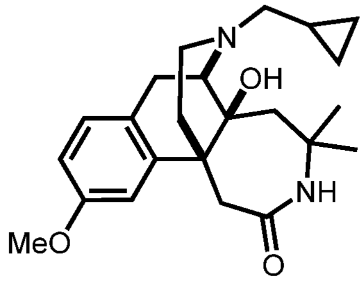

The title compound was obtained from the compound obtained in Reference Example 45 according to the method described in Example 1.

$^1$H-NMR (400 MHz, $CDCl_3$)$\delta$ (ppm): 0.09 - 0.13 (m, 2H), 0.50 - 0.54 (m, 2H), 0.78 - 0.88 (m, 1H), 1.06 - 1.14 (m, 1H), 1.10 (s, 3H), 1.55 (d, J = 14 Hz, 1H), 1.62 (s, 3H), 1.86 (d, J = 14 Hz, 1H), 2.00 - 2.09 (m, 2H), 2.32 (dd, J = 6, 13 Hz, 1H), 2.34 (dd, J = 6, 13 Hz, 1H), 2.52 - 2.61 (m, 1H), 2.72 (dd, J = 2, 15 Hz, 1H), 2.78 (dd, J = 6, 18 Hz, 1H), 2.87 (d, J = 6 Hz, 1H), 3.00 (d, J = 18 Hz, 1H), 3.57 (d, J = 15 Hz, 1H), 3.79 (s, 3H), 4.90 (br s, 1H), 5.39 (s, 1H), 6.71 (dd, J = 2, 8 Hz, 1H), 6.97 (d, J = 8 Hz, 1H), 7.10 (d, J = 2 Hz, 1H).

(Example 268)

Synthesis of (5aS,6R,11bS)-14-(cyclopropylmethyl)-10-methoxy-4,4-dimethyl-2,3,4,5,6,7-hexahydro-6,11b-(epiminoethano)naphtho[1,2-d]azepin-5a(1H)-ol

[Chemical Formula 331]

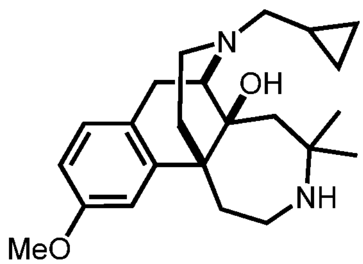

The title compound was obtained from the compound obtained in Example 267 according to the method described in Example 2.

$^1$H-NMR (400 MHz, $CDCl_3$)$\delta$ (ppm): 0.07 - 0.13 (m, 2H), 0.50 - 0.53 (m, 2H), 0.81 - 0.90 (m, 1H), 0.85 (s, 3H), 1.04 - 1.10 (m, 1H), 1.33 (s, 3H), 1.43 (d, J = 15 Hz, 1H), 1.57 - 1.76 (m, 2H), 1.92 - 2.01 (m, 2H), 2.26 - 2.39 (m, 3H), 2.51 - 2.60 (m, 2H), 2.74 (dd, J = 6, 18 Hz, 1H), 2.82 (d, J = 6 Hz, 1H), 2.85 - 2.91 (m, 1H), 2.98 (d, J = 18 Hz, 1H), 3.79 (s, 3H), 4.41 (s, 1H), 6.71 (dd, J = 2, 8 Hz, 1H), 6.74 (d, J = 2 Hz, 1H), 7.02 (d, J = 8 Hz, 1H).

(Example 269)

Synthesis of 1-((5aS,6R,11bR)-14-(cyclopropylmethyl)-5a-hydroxy-10-methoxy-4,4-dimethyl-1,2,5,5a,6,7-hexahydro-6,11b-(epiminoethano)naphtho[1,2-d]azepin-3(4H)-yl)-2-(4-methyl-1H-pyrazol-1-yl)ethan-1-one

[Chemical Formula 332]

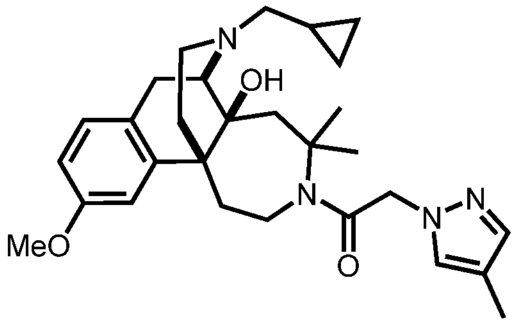

To a solution of the compound (24.0 mg, 0.0648 mmol) obtained in Example 268 in tetrahydrofuran (2 mL) were added N,N-diisopropylethylamine (55.1 pL, 0.324 mmol) and chloroacetyl chloride (12.9 μL, 0.162 mmol) under ice cooling, followed by stirring at room temperature for 1.5 hours. To the reaction mixture, sodium iodide (23.9 mg, 0.159 mmol) and 4-methylpyrazole (13.4 μL, 0.162 mmol) were added, followed by stirring at 60°C for 17 hours. After allowed to cool to room temperature, to the reaction mixture was added a saturated aqueous sodium bicarbonate solution, followed by extraction three times with ethyl acetate. The combined extracts were washed with saturated saline, dried over sodium sulfate, and then concentrated under reduced pressure. The obtained crude product was purified by preparative thin layer chromatography (chloroform : methanol = 95 : 5, twice) to yield the title compound (9.7 mg, 30%) as a pale yellow oily matter.

$^1$H-NMR (400 MHz, $CDCl_3$)δ (ppm): 0.07 - 0.13 (m, 2H), 0.49 - 0.54 (m, 2H), 0.79 - 0.88 (m, 1H), 1.04 - 1.07 (m, 1H), 1.14 (s, 3H), 1.39 (d, J = 15 Hz, 1H), 1.88 (s, 3H), 1.92 - 2.15 (m, 4H), 2.09 (s, 3H), 2.28 - 2.40 (m, 3H), 2.55 - 2.62 (m, 1H), 2.76 (dd, J = 6, 18 Hz, 1H), 2.81 (d, J = 6 Hz, 1H), 2.92 - 2.98 (m, 1H), 3.01 (d, J = 18 Hz, 1H), 3.57 - 3.63 (m, 1H), 3.79 (s, 3H), 4.44 (s, 1H), 4.82 (d, J = 16 Hz, 1H), 4.99 (d, J = 16 Hz, 1H), 6.68 (d, J = 2 Hz, 1H), 6.74 (dd, J = 2, 8 Hz, 1H), 7.05 (d, J = 8 Hz, 1H), 7.33 (s, 1H), 7.34 (s, 1H).

(Example 270)

Synthesis of (5aS,6R,11bR)-14-(cyclopropylmethyl)-4,4-dimethyl-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 333]

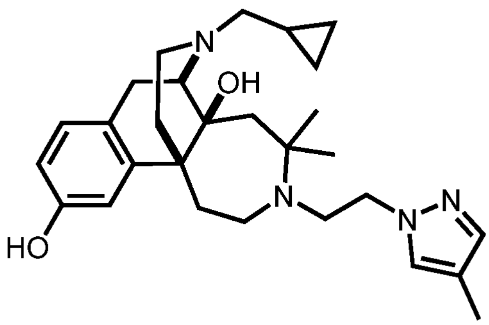

To a solution of the compound (9.7 mg, 0.020 mmol) obtained in Example 269 in tetrahydrofuran (3 mL) was added a 0.9 M boranetetrahydrofuran complex-tetrahydrofuran solution (0.22 mL, 0.20 mmol), followed by heating under reflux for 18 hours. After allowed to cool to room temperature, to the reaction mixture was added 2 M hydrochloric acid (3 mL), followed by stirring at 100°C for 1 hour. After allowed to cool to room temperature, the reaction mixture was added to a saturated aqueous sodium bicarbonate solution, followed by extraction three times with ethyl acetate. The combined extracts were washed with saturated saline, dried over sodium sulfate, and then concentrated under reduced pressure. To a solution of the obtained crude product (4.5 mg) in dichloromethane (1.5 mL) was added a 1 M boron tribrominate-dichloromethane solution (0.050 mL, 0.050 mmol) under ice cooling, followed by stirring at room temperature for 17 hours. To the reaction mixture, a saturated aqueous potassium carbonate solution was added, followed by extraction three times with ethyl acetate. The combined extracts were washed with saturated saline, dried over sodium sulfate, and then concentrated under reduced pressure. The obtained crude product was purified by preparative thin layer

chromatography (chloroform : 2 M $NH_3$-methanol = 95 : 5, twice) to yield the title compound (5.6 mg, 60%) as a colorless oily matter.

$^1H$-NMR (400 MHz, $CDCl_3$)δ (ppm): 0.03 - 0.09 (m, 2H), 0.43 - 0.49 (m, 2H), 0.67 (s, 3H), 0.85 - 0.93 (m, 1H), 0.99 - 1.03 (m, 1H), 1.25 (s, 3H), 1.36 (d, J = 15 Hz, 1H), 1.72 (d, J = 15 Hz, 1H), 1.86 - 1.92 (m, 1H), 1.97 (ddd, J = 3, 12, 12 Hz, 1H), 2.08 (s, 3H), 2.14 (ddd, J = 5, 12, 12 Hz, 1H), 2.28 (dd, J = 5, 15 Hz, 1H), 2.34 - 2.40 (m, 3H), 2.55 - 2.65 (m, 3H), 2.76 (dd, J = 12, 14 Hz, 1H), 2.95 - 2.99 (m, 2H), 3.04 - 3.11 (m, 1H), 4.04 - 4.17 (m, 2H), 4.74 (br s, 1H), 6.61 (d, J = 2, 8 Hz, 1H), 6.71 (d, J = 2 Hz, 1H), 6.90 (d, J = 8 Hz, 1H), 7.28 (s, 1H), 7.33 (s, 1H).

(Reference Example 46)

Synthesis of (4bR,8aS,9R)-11-(cyclopropylmethyl)-4,8a-dihydroxy-3-methoxy-7-methyl-8,8a,9,10-tetrahydro-5H-9,4b-(epiminoethano)phenanthren-6(7H)-one

[Chemical Formula 334]

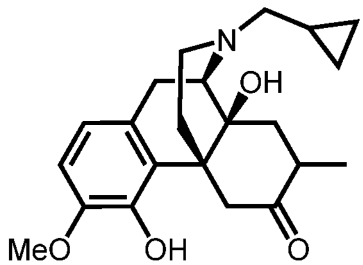

To a solution of (4R,4aS,7aR,12bS)-3-(cyclopropylmethyl)-4a-hydroxy-9-methoxy-2,3,4,4a,5,6-hexahydro-1H-4,12-methanobenzofuro[3,2-e]isoquinolin-7(7aH)-one (synthesized by the method described in Organic Letters 2009, 11, 539) (1.50 g, 4.22 mmol) in tetrahydrofuran (37 mL) cooled to -78°C was added a 1.08 M lithium bis(trimethylsilyl)amide-tetrahydrofuran solution (11.7 mL, 12.6 mmol), followed by stirring for 1 hour. To the reaction mixture, a solution of methyl iodide (1.58 mL, 25.4 mmol) in tetrahydrofuran (3 mL) was added, followed by stirring at -78°C for 1 hour and at room temperature for 18 hours. To the reaction mixture, a saturated aqueous sodium bicarbonate solution was added, followed by extraction three times with ethyl acetate. The combined extracts were washed with saturated saline, dried over sodium sulfate, and then concentrated under reduced pressure. The obtained crude product was purified by silica gel column chromatography (47 to 68% ethyl acetate/heptane). To a mixed solution of the obtained pale yellow crystals (460 mg) in chloroform/water (3 : 2, 2.5 mL), concentrated hydrochloric acid (1.5 mL) was added, followed by stirring at room temperature for 2.5 hours. To the reaction mixture, a saturated sodium bicarbonate aqueous solution was added, followed by extraction twice with chloroform. The combined extracts were washed with saturated saline, dried over sodium sulfate, and then concentrated under reduced pressure. To a solution of the obtained pale yellow oily matter (307 mg) in acetic acid (8 mL), zinc powder (550 mg) was added, followed by heating under reflux for 20.5 hours. After allowed to cool to room temperature, zinc powder (555 mg) was added again, followed by heating under reflux for 8.5 hours. After allowed to cool to room temperature, zinc powder (555 mg) was added again, followed by heating under reflux for 7 hours. The reaction mixture was allowed to cool to room temperature and then filtered through celite, and the filtrate was concentrated under reduced pressure. To the obtained residue, a saturated aqueous potassium carbonate solution was added, followed by extraction three times with ethyl acetate. The combined extracts were washed with saturated saline, dried over sodium sulfate, and then concentrated under reduced pressure. The obtained crude product was purified by silica gel column chromatography (20 to 100% ethyl acetate/heptane) to yield the title compound (219 mg, 14%) as a pale yellow crystal.

$^1H$-NMR (400 MHz, $CDCl_3$)δ (ppm): 0.10 - 0.14 (m, 2H), 0.51 - 0.55 (m, 2H), 0.83 - 0.89 (m, 1H), 0.88 (d, J = 7 Hz, 3H), 1.63 (dd, J = 13, 14 Hz, 1H), 1.87 (dd, J = 6, 13 Hz, 1H), 1.98 - 2.13 (m, 3H), 2.32 - 2.41 (m, 2H), 2.58 - 2.62 (m, 1H), 2.81 (dd, J = 7, 18 Hz, 1H), 2.89 - 3.01 (m, 3H), 3.09 (d, J = 7 Hz, 1H), 3.82 (s, 3H), 3.93 (d, J = 13 Hz, 1H), 6.10 (s, 1H), 6.54 (d, J = 8 Hz, 1H), 6.65 (d, J = 8 Hz, 1H).

(Reference Example 47)

Synthesis of (4bR,8aS,9R)-11-(cyclopropylmethyl)-8a-hydroxy-3-methoxy-7-methyl-6-oxo-6,7,8,8a,9,10-hexahydro-5H-9,4b-(epiminoethano)phenanthren-4-yl trifluoromethanesulfonate

[Chemical Formula 335]

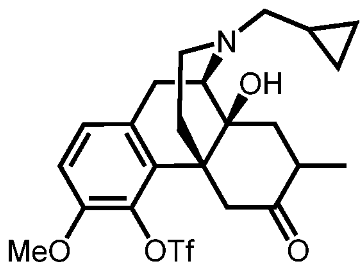

The title compound was obtained from the compound obtained in Reference Example 46 according to the method described in Reference Example 1.

$^1$H-NMR (400 MHz, $CDCl_3$)$\delta$ (ppm): 0.11 - 0.15 (m, 2H), 0.53 - 0.57 (m, 2H), 0.83 (d, J = 6 Hz, 3H), 0.83 - 0.90 (m, 1H), 1.44 (dd, J = 14, 14 Hz, 1H), 1.65 - 1.69 (m, 1H), 1.89 (dd, J = 7, 14 Hz, 1H), 2.09 (dd, J = 3, 12 Hz, 1H), 2.21 (ddd, J = 5, 13, 13 Hz, 1H), 2.38 (d, J = 7 Hz, 2H), 2.67 - 2.71 (m, 1H), 2.85 (dd, J = 7, 18 Hz, 1H), 2.87 - 2.96 (m, 1H), 3.04 (d, J = 18 Hz, 1H), 3.13 (d, J = 7 Hz, 1H), 3.14 (d, J = 14 Hz, 1H), 3.53 (d, J = 14 Hz, 1H), 3.80 (s, 3H), 6.83 (d, J = 8 Hz, 1H), 7.03 (d, J = 8 Hz, 1H).

(Reference Example 48)

Synthesis of (4bR,8aS,9R)-11-(cyclopropylmethyl)-8a-hydroxy-3-methoxy-7-methyl-8,8a,9,10-tetrahydro-5H-9,4b-(epiminoethano)phenanthren-6(7H)-one

[Chemical Formula 336]

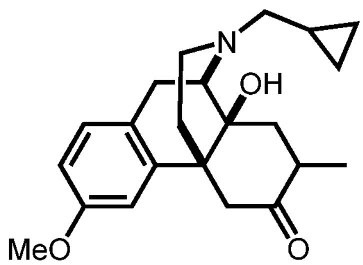

The title compound was obtained from the compound obtained in Reference Example 47 according to the method described in Reference Example 2.

$^1$H-NMR (400 MHz, $CDCl_3$)$\delta$ (ppm): 0.12 - 0.16 (m, 2H), 0.52 - 0.57 (m, 2H), 0.83 - 0.91 (m, 1H), 0.87 (d, J = 6 Hz, 3H), 1.16 - 1.20 (m, 1H), 1.54 (dd, J = 13, 13 Hz, 1H), 1.85 (dd, J = 6, 13 Hz, 1H), 2.05 - 2.20 (m, 2H), 2.36 - 2.44 (m, 2H), 2.57 - 2.61 (m, 1H), 2.74 (dd, J = 6, 18 Hz, 1H), 2.83 (d, J = 14 Hz, 1H), 2.88 - 2.98 (m, 1H), 3.05 (d, J = 18 Hz, 1H), 3.08 (dd, J = 1, 14 Hz, 1H), 3.12 (d, J = 6 Hz, 1H), 3.77 (s, 3H), 6.68 (dd, J = 2, 8 Hz, 1H), 6.81 (d, J = 2 Hz, 1H), 6.97 (d, J = 8 Hz, 1H).

(Reference Example 49)

Synthesis of (4bR,8aS,9R)-11-(cyclopropylmethyl)-8a-hydroxy-3-methoxy-7-methyl-8,8a,9,10-tetrahydro-5H-9,4b-(epiminoethano)phenanthren-6(7H)-one oxime

[Chemical Formula 337]

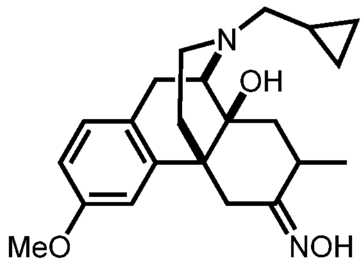

The title compound was obtained from the compound obtained in Reference Example 48 according to the method described in Reference Example 3.

$^1$H-NMR (400 MHz, $CDCl_3$)$\delta$ (ppm): 0.10 - 0.15 (m, 2H), 0.50 - 0.55 (m, 2H), 0.82 - 0.90 (m, 1H), 0.94 (d, J = 6 Hz, 3H), 1.15 - 1.18 (m, 1H), 1.39 (dd, J = 13, 13 Hz, 1H), 1.66 (dd, J = 5, 13 Hz, 1H), 2.06 - 2.16 (m, 2H), 2.33 (d, J = 14 Hz, 1H), 2.33 - 2.42 (m, 2H), 2.55 - 2.60 (m, 1H), 2.71 (dd, J = 6, 18 Hz, 1H), 2.81 - 2.90 (m, 1H), 3.03 (d, J = 6 Hz, 1H), 3.04 (d, J = 18 Hz, 1H), 3.77 (s, 3H), 3.78 (d, J = 14 Hz, 1H), 6.69 (dd, J = 2, 8 Hz, 1H), 6.87 (br s, 1H), 6.95 (d, J = 8 Hz, 1H), 7.11 (d, J = 2 Hz, 1H).

(Example 271)

Synthesis of (5aS,6R,11bR)-14-(cyclopropylmethyl)-5a-hydroxy-10-methoxy-4-methyl-3,4,5,5a,6,7-hexahydro-6,11b-(epiminoethano)naphtho[1,2-d]azepin-2(1H)-one

[Chemical Formula 338]

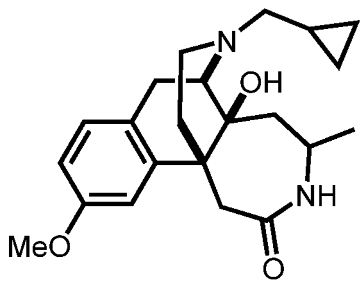

The title compound was obtained from the compound obtained in Reference Example 49 according to the method described in Example 1.

$^1$H-NMR (400 MHz, $CDCl_3$)$\delta$ (ppm): 0.10 - 0.13 (m, 2H), 0.50 - 0.55 (m, 2H), 0.78 - 0.88 (m, 1H), 1.09 - 1.14 (m, 1H), 1.11 (d, J = 6 Hz, 3H), 1.48 (d, J = 14 Hz, 1H), 1.68 (dd, J = 10, 14 Hz, 1H), 2.03 - 2.11 (m, 2H), 2.35 - 2.37 (m, 2H), 2.56 - 2.63 (m, 2H), 2.75 (dd, J = 6, 18 Hz, 1H), 2.90 (d, J = 6 Hz, 1H), 3.01 (d, J = 18 Hz, 1H), 3.50 (d, J = 14 Hz, 1H), 3.80 (s, 3H), 4.06 - 4.15 (m, 1H), 5.23 (s, 1H), 6.71 (dd, J = 2, 8 Hz, 1H), 6.97 (d, J = 8 Hz, 1H), 7.15 (d, J = 2 Hz, 1H).

(Example 272)

Synthesis of (5aS,6R,11bS)-14-(cyclopropylmethyl)-10-methoxy-4-methyl-2,3,4,5,6,7-hexahydro-6,11b-(epiminoethano)naphtho[1,2-d]azepin-5a(1H)-ol

[Chemical Formula 339]

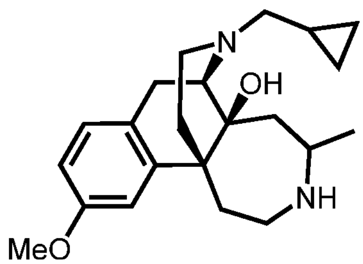

The title compound was obtained from the compound obtained in Example 271 according to the method described in Example 2.

$^1$H-NMR (400 MHz, $CDCl_3$)δ (ppm): 0.07 - 0.13 (m, 2H), 0.47 - 0.55 (m, 2H), 0.78 - 0.88 (m, 1H), 0.96 (d, J = 7 Hz, 3H), 1.03 - 1.07 (m, 1H), 1.51 - 1.60 (m, 2H), 1.95 - 2.08 (m, 3H), 2.30 - 2.38 (m, 3H), 2.50 - 2.53 (m, 1H), 2.81 (dd, J = 6, 18 Hz, 1H), 2.88 - 3.00 (m, 4H), 3.28 - 3.37 (m, 1H), 3.79 (s, 3H), 4.55 (br s, 1H), 6.71 (dd, J = 3, 9 Hz, 1H), 6.72 (d, J = 3 Hz, 1H), 7.02 (d, J = 9 Hz, 1H).

(Example 273)

Synthesis of (5aS,6R,11bS)-14-(cyclopropylmethyl)-10-methoxy-4-methyl-3-(2-(4-methyl-1H-pyrazol-1-yl)ethyl)-2,3,4,5,6,7-hexahydro-6,11b-(epiminoethano)naphtho[1,2-d]azepin-5a(1H)-ol

[Chemical Formula 340]

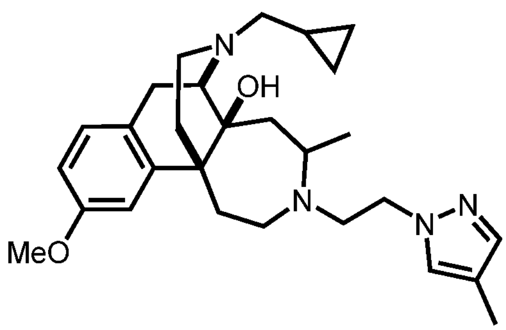

The title compound was obtained from the compound obtained in Example 272 and the compound obtained in Reference Example 33 according to the method described in Example 31.

$^1$H-NMR (400 MHz, $CDCl_3$)δ (ppm): 0.06 - 0.13 (m, 2H), 0.46 - 0.54 (m, 2H), 0.78 - 0.87 (m, 1H), 0.81 (d, J = 6 Hz, 3H), 1.00 - 1.08 (m, 1H), 1.40 (dd, J = 4, 15 Hz, 1H), 1.65 (dd, J = 12, 15 Hz, 1H), 1.86 (dd, J = 6, 16 Hz, 1H), 1.99 - 2.02 (m, 2H), 2.05 (s, 3H), 2.14 (dd, J = 10, 16 Hz, 1H), 2.32 (dd, J = 6, 13 Hz, 1H), 2.38 (dd, J = 6, 13 Hz, 1H), 2.49 - 2.57 (m, 1H), 2.62 (dd, J = 6, 15 Hz, 1H), 2.74 (dd, J = 6, 18 Hz, 1H), 2.83 - 2.89 (m, 2H), 2.98 (d, J = 19 Hz, 1H), 2.98 - 3.11 (m, 2H), 3.16 - 3.24 (m, 1H), 3.78 (s, 3H), 4.03 - 4.15 (m, 2H), 4.69 (br s, 1H), 6.67 (d, J = 2 Hz, 1H), 6.71 (dd, J = 2, 8 Hz, 1H), 7.01 (d, J = 8 Hz, 1H), 7.19 (s, 1H), 7.28 (s, 1H).

(Example 274)

Synthesis of (5aS,6R,11bS)-14-(cyclopropylmethyl)-4-methyl-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 341]

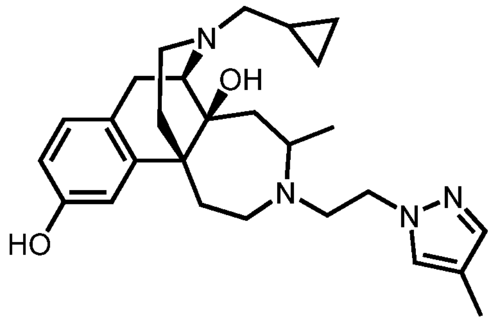

The title compound was obtained from the compound obtained in Example 273 according to the method described in Example 6.

$^1$H-NMR (400 MHz, $CDCl_3$)δ (ppm): 0.05 - 0.13 (m, 2H), 0.45 - 0.53 (m, 2H), 0.78 - 0.90 (m, 1H), 0.84 (d, J = 6 Hz, 3H), 1.00 - 1.07 (m, 1H), 1.38 (dd, J = 4, 15 Hz, 1H), 1.67 (dd, J = 12, 15 Hz, 1H), 1.82 (dd, J = 6, 15 Hz, 1H), 1.99 - 2.01 (m, 2H), 2.05 (s, 3H), 2.13 (dd, J = 10, 15 Hz, 1H), 2.32 (dd, J = 6, 12 Hz, 1H), 2.37 (dd, J = 6, 12 Hz, 1H), 2.48 - 2.56 (m, 1H), 2.62 (dd, J = 6, 15 Hz, 1H), 2.72 (dd, J = 6, 18 Hz, 1H), 2.85 (d, J = 19 Hz, 1H), 2.87 (d, J = 13 Hz, 1H), 2.95 (d, J = 19 Hz, 1H), 2.95 - 3.12 (m, 2H), 3.17 - 3.25 (m, 1H), 4.04 - 4.16 (m, 2H), 4.71 (br s, 1H), 6.61 (d, J = 2 Hz, 1H), 6.64 (dd, J = 2, 8 Hz, 1H), 6.92 (d, J = 8 Hz, 1H), 7.19 (s, 1H), 7.30 (s, 1H).

(Reference Example 50)

Synthesis of (4R,4aS,8aR,13bS)-3-(cyclopropylmethyl)-4a-hydroxy-1,2,3,4,4a,5,6,7-octahydro-4,13-methanobenzofuro[2,3-c]pyrido[4,3-d]azepin-8(8aH)-one

[Chemical Formula 342]

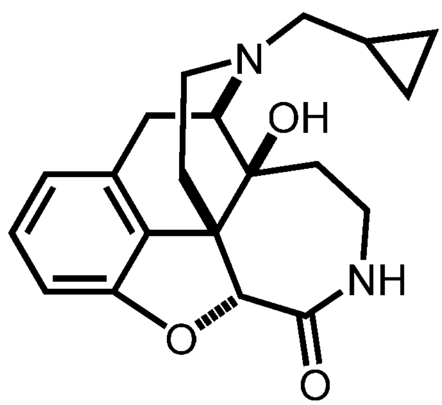

To (4R,4aS,8aR,13bS)-3-(cyclopropylmethyl)-4a-hydroxy-1,2,3,4,4a,5,6,7-octahydro-4,13-methanobenzofuro[2,3-c]pyrido[4,3-d]azepin-8(8aH)-one (synthesized by the method described in Journal of Medicinal Chemistry 2004, 47, 1070) (4.88 g, 15 mmol) suspended in polyphosphoric acid (100 mL) and then heated to an internal temperature of 60 to 80°C was added azidotrimethylsilane (3.95 mL, 30 mmol), followed by stirring at an internal temperature of 60 to 80°C for 30 minutes. Azidotrimethylsilane (7.89 mL, 60 mmol) was added at an internal temperature of 60 to 80°C,

followed by stirring at an internal temperature of 60 to 80°C for 2 hours. The reaction mixture was poured into an ice-cooled 28% aqueous ammonia solution (300 mL), and to the resultant solution, water (1.0 L) and chloroform (500 mL) were added, followed by stirring at room temperature for 18 hours. The mixture was extracted three times with chloroform. The combined extracts were dried over sodium sulfate and then concentrated under reduced pressure. The obtained crude product was purified by silica gel column chromatography (0 to 10% 2 M ammonia-methanol solution/chloroform) to yield the title compound (2.63 g, 52%) as a colorless amorphous form.

$^1$H-NMR (400 MHz, $CDCl_3$)$\delta$ (ppm): 0.10 - 0.17 (m, 2H), 0.52 - 0.59 (m, 2H), 0.80 - 0.92 (m, 1H), 1.27 - 1.36 (m, 1H), 1.57 - 1.66 (m, 1H), 1.87 - 1.98 (m, 1H), 2.27 - 2.44 (m, 3H), 2.46 (ddd, J = 6, 13, 13 Hz, 1H), 2.63 - 2.77 (m, 2H), 2.82 - 2.90 (m, 2H), 3.11 (d, J = 7 Hz, 1H), 3.18 (d, J = 19 Hz, 1H), 4.81 (d, J = 2 Hz, 1H), 4.94 (br s, 1H), 5.93 - 6.02 (m, 1H), 6.66 (d, J = 8 Hz, 1H), 6.71 (d, J = 7 Hz, 1H), 7.12 (dd, J = 8, 8 Hz, 1H).

(Example 275)

Synthesis of (5aS,6R,11bR)-14-(cyclopropylmethyl)-5a,11-dihydroxy-3,4,5,5a,6,7-hexahydro-6,11b-(epiminoethano)naphtho[1,2-d]azepin-2(1H)-one

[Chemical Formula 343]

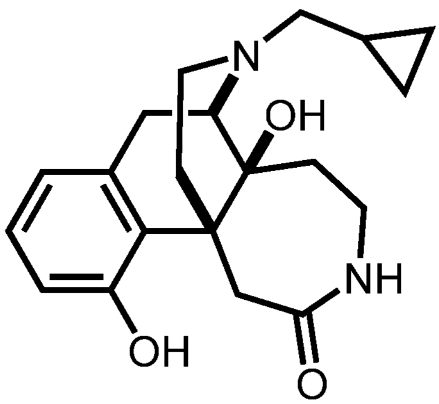

To a solution of the compound (250 mg, 0.73 mmol) obtained in Reference Example 50 in ethylenediamine (10 mL) was added Sodium silica gel Stage I (1 g) under ice cooling, followed by stirring at room temperature for 1 hour. Tetrahydrofuran (100 mL) was added under ice cooling, and then water (150 mL) was slowly added at the same temperature, followed by stirring. The temperature of the mixture was returned to room temperature, and to the mixture, a saturated aqueous sodium bicarbonate solution was added, followed by extraction three times with chloroform. The combined extracts were dried over sodium sulfate and then concentrated under reduced pressure. The obtained crude product was purified by silica gel column chromatography (50 to 100% ethyl acetate/heptane, 0 to 5% methanol/ethyl acetate) to yield the title compound (234 mg, 93%) as a colorless amorphous form.

$^1$H-NMR (400 MHz, $CDCl_3$)$\delta$ (ppm): 0.06 - 0.15 (m, 2H), 0.48 - 0.55 (m, 2H), 0.75 - 0.87 (m, 1H), 1.40 - 1.62 (m, 3H), 1.87 - 2.01 (m, 2H), 2.30 (dd, J = 7, 13 Hz, 1H), 2.35 (dd, J = 6, 12 Hz, 1H), 2.50 - 2.62 (m, 1H), 2.77 - 2.90 (m, 3H), 2.92 - 3.04 (m, 2H), 3.55 (d, J = 14 Hz, 1H), 3.78 - 3.90 (m, 1H), 4.66 (br s, 1H), 6.71 (d, J = 7 Hz, 1H), 6.81 (d, J = 8 Hz, 1H), 6.83 - 6.92 (m, 1H), 7.01 (dd, J = 8, 8 Hz, 1H), 8.53 (br s, 1H).

(Example 276)

Synthesis of (5aS,6R,11bS)-14-(cyclopropylmethyl)-2,3,4,5,6,7-hexahydro-6,11b-(epiminoethano)naphtho[1,2-d]azepine-5a,11(1H)-diol

[Chemical Formula 344]

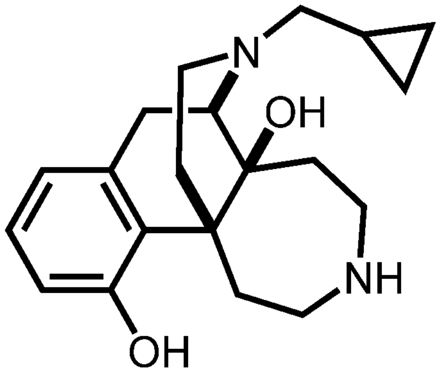

The title compound was obtained from the compound obtained in Example 275 according to the method described in Example 2.

$^1$H-NMR (400 MHz, $CDCl_3$)$\delta$ (ppm): 0.04 - 0.15 (m, 2H), 0.45 - 0.55 (m, 2H), 0.76 - 0.91 (m, 1H), 1.48 - 1.82 (m, 3H), 1.88 - 2.04 (m, 2H), 2.27 - 2.34 (m, 2H), 2.40 - 2.60 (m, 3H), 2.77 - 3.04 (m, 6H), 3.12 - 3.23 (m, 1H), 4.53 (br s, 1H), 5.93 (br s, 1H), 6.31 (d, J = 8 Hz, 1H), 6.57 (d, J = 7 Hz, 1H), 6.88 (dd, J = 8, 8 Hz, 1H).

(Example 277)

Synthesis of (5aS,6R,11bS)-14-(cyclopropylmethyl)-3-(2-(4-methyl-1H-pyrazol-1-yl)ethyl)-2,3,4,5,6,7-hexahydro-6,11b-(epiminoethano)naphtho[1,2-d]azepine-5a,11(1H)-diol

[Chemical Formula 345]

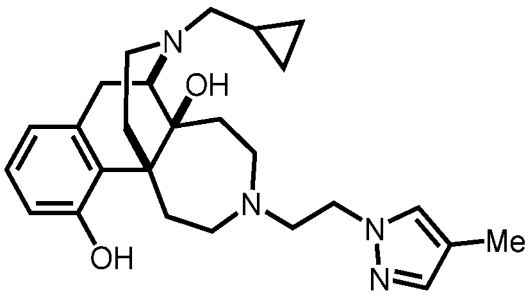

The title compound was obtained from the compound obtained in Example 276 and the compound obtained in Reference Example 33 according to the method described in Example 31.

$^1$H-NMR (400 MHz, $CDCl_3$)$\delta$ (ppm): 0.04 - 0.15 (m, 2H), 0.45 - 0.55 (m, 2H), 0.76 - 0.91 (m, 1H), 1.18 - 2.07 (m, 5H), 2.00 (s, 3H), 2.24 - 2.64 (m, 6H), 2.74 - 3.00 (m, 7H), 3.32 (dd, J = 12, 12 Hz, 1H), 3.98 - 4.16 (m, 2H), 4.61 (br s, 1H), 6.47 (d, J = 8 Hz, 1H), 6.65 (d, J = 7 Hz, 1H), 6.91 (s, 1H), 6.92 (dd, J = 8, 8 Hz, 1H), 7.23 (s, 1H).

(Example 278)

Synthesis of (5aS,6R,11bR)-14-(cyclopropylmethyl)-5a-hydroxy-3-(2-(pyridin-2-yl)acetyl)-1,2,3,4,5,5a,6,7-octahydro-6,11b-(epiminoethano)naphtho[1,2-d]azepin-10-yl trifluoromethanesulfonate

[Chemical Formula 346]

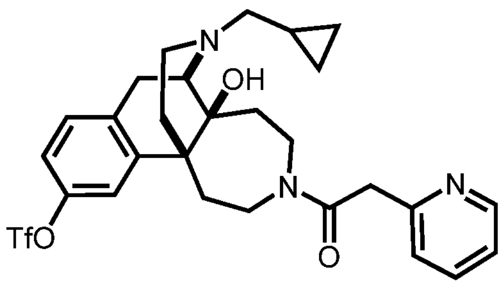

To the compound (26 mg, 0.058 mmol) obtained in Example 22 and N-phenylbis(trifluoromethanesulfonimide) (62 mg, 0.174 mmol) dissolved in dimethylformamide (1 mL) was added potassium carbonate (32 mg, 0.232 mmol), followed by stirring at room temperature for 16 hours under a nitrogen atmosphere. The reaction solution was diluted with ethyl acetate, and then washed with a saturated aqueous sodium bicarbonate solution and saturated saline. The obtained organic layer was dried over sodium sulfate and then concentrated under reduced pressure. The obtained crude product was purified by silica gel column chromatography (amino group-supported silica gel, 1 to 20% methanol/chloroform) to yield the title compound (32 mg, 95%) as a yellow oily matter.

$^1$H-NMR (400 MHz, $CDCl_3$)δ (ppm): 0.05 - 0.20 (m, 2H), 0.45 - 0.60 (m, 2H), 0.70 - 0.90 (m, 1H), 1.00 - 1.10 (m, 1H), 1.60 - 1.80 (m, 4H), 1.80 - 2.20 (m, 3H), 2.20 - 2.40 (m, 2H), 2.45 - 2.65 (m, 1H), 2.70 - 3.15 (m, 2.5H), 3.40 - 3.60 (m, 1.5H), 3.65 - 3.80 (m, 1H), 3.80 - 4.00 (m, 2.5H), 4.10 - 4.25 (m, 0.5H), 4.30 - 4.50 (m, 1H), 6.95 - 7.10 (m, 2H), 7.10 - 7.20 (m, 2.5H), 7.20 - 7.30 (m, 0.5H), 7.50 - 7.60 (m, 1H), 8.40 - 8.55 (m, 1H).

(Example 279)

Synthesis of 1-((5aS,6R,11bR)-14-(cyclopropylmethyl)-5a-hydroxy-10-(6-methoxypyridin-2-yl)-1,2,5,5a,6,7-hexahydro-6,11b-(epiminoethano)naphtho[1,2-d]azepin-3(4H)-yl)-2-(pyridin-2-yl)ethan-1-one

[Chemical Formula 347]

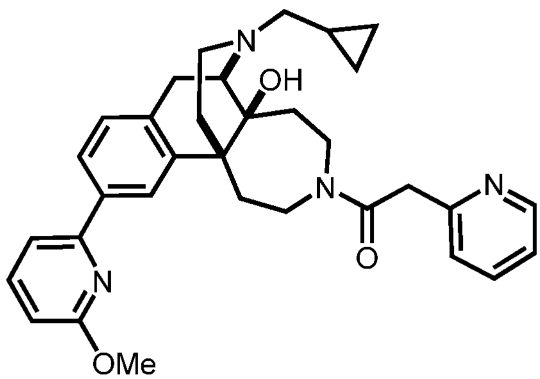

To the compound (16 mg, 0.028 mmol) obtained in Example 278, 2-methoxy-6-(4,4,5,5-tetramethyl-1,3,2-dioxaboran-2-yl)pyridine (20 mg, 0.084 mmol) and tetrakis(triphenylphosphine)palladium (0) (6 mg, 0.0056 mmol) dissolved in toluene/ethanol (v/v = 5/1, 0.6 mL) was added a 2 M aqueous sodium carbonate solution (168 μL, 0.336 mmol), followed by stirring at 100°C for 16 hours under a nitrogen atmosphere. After allowed to cool to room temperature, the reaction solution was diluted with ethyl acetate, and then washed with a saturated aqueous sodium bicarbonate solution and saturated saline. The obtained organic layer was dried over sodium sulfate and then concentrated under reduced pressure. The obtained crude product was purified by preparative thin layer chromatography (chloroform : 2 M ammonia-methanol solution = 10 : 1) to yield the title compound (11 mg, 73%) as a yellow oily matter.

$^1$H-NMR (400 MHz, $CDCl_3$)δ (ppm): 0.05 - 0.20 (m, 2H), 0.45 - 0.60 (m, 2H), 0.75 - 0.90 (m, 1H), 1.05 - 1.20 (m, 1H), 1.50 - 1.70 (m, 1.4H), 1.70 - 1.90 (m, 1.6H), 1.95 - 2.20 (m, 2H), 2.25 - 2.45 (m, 3H), 2.50 - 2.70 (m, 1.4H), 2.70 - 2.85 (m, 0.6H), 2.90 - 3.15 (m, 3H), 3.20 - 3.35 (m, 0.4H), 3.40 - 3.70 (m, 1H), 3.74 (d, J = 16 Hz, 0.6H), 3.80 - 4.00 (m, 2.6H), 4.00 (s, 1.8H), 4.02 (s, 1.2H), 4.15 - 4.20 (m, 0.4H), 6.66 (d, J = 8 Hz, 1H), 6.93 (d, J = 8 Hz, 0.6H), 7.01 (dd, J = 2, 3 Hz, 0.6H), 7.03 (dd, J = 2, 3 Hz, 0.4H), 7.15 - 7.25 (m, 1.4H), 7.25 - 7.30 (m, 1H), 7.46 (dt, J = 2, 7 Hz, 0.6H),

7.56 (dt, J = 2, 7 Hz, 0.4H), 7.60 - 7.70 (m, 1H), 7.73 (dd, J = 2, 8 Hz, 0.6H), 7.78 (dd, J = 2, 8 Hz, 0.4H), 7.80 - 7.00 (m, 1H), 8.39 (dd, J = 1, 4 Hz, 0.6H), 8.49 (dd, J = 1, 4 Hz, 0.4H).

(Example 280)

Synthesis of 1-((5aS,6R,11bR)-14-(cyclopropylmethyl)-5a-hydroxy-10-methoxy-4-methyl-1,2,5,5a,6,7-hexahydro-6,11b-(epiminoethano)naphtho[1,2-d]azepin-3(4H)-yl)-2-(pyridin-2-yl)ethan-1-one

[Chemical Formula 348]

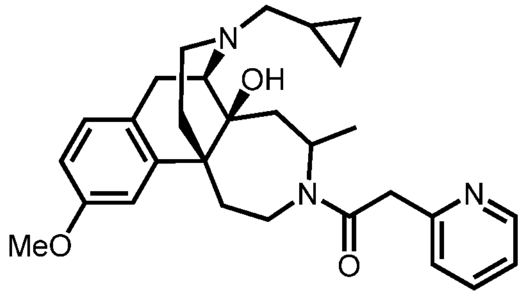

The title compound was obtained from the compound obtained in Example 272 and 2-(pyridin-2-yl)acetic acid hydrochloride according to the method described in Example 5.

$^1$H-NMR (400 MHz, $CD_3D$)δ (ppm): 0.10 - 0.18 (m, 2H), 0.48 - 0.57 (m, 2H), 0.83 - 0.92 (m, 1H), 0.92 (d, J = 6 Hz, 1.5H), 0.97 (d, J = 6 Hz, 1.5H), 1.06 - 1.09 (m, 1H), 1.63 - 1.80 (m, 2H), 1.88 - 2.09 (m, 3H), 2.28 (dd, J = 6, 15 Hz, 0.5H), 2.33 - 2.46 (m, 2.5H), 2.52 - 2.58 (m, 1H), 2.62 - 2.68 (m, 0.5H), 2.80 (dd, J = 6, 19 Hz, 1H), 2.92 - 3.10 (m, 2.5H), 3.76 (s, 3H), 3.90 - 4.12 (m, 3H), 4.27 - 4.37 (m, 0.5H), 4.58 - 4.66 (m, 0.5H), 6.75 - 6.78 (m, 2H), 7.08 (d, J = 8 Hz, 1H), 7.23 - 7.32 (m, 1H), 7.43 (d, J = 8 Hz, 0.5H), 7.45 (d, J = 8 Hz, 0.5H), 7.77 - 7.81 (m, 1H), 8.45 - 8.49 (m, 1H).

(Example 281)

Synthesis of 1-((5aS,6R,11bR)-14-(cyclopropylmethyl)-5a,10-dihydroxy-4-methyl-1,2,5,5a,6,7-hexahydro-6,11b-(epiminoethano)naphtho[1,2-d]azepin-3(4H)-yl)-2-(pyridin-2-yl)ethan-1-one

[Chemical Formula 349]

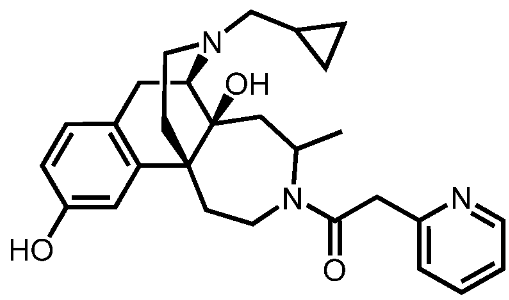

The title compound was obtained from the compound obtained in Example 280 according to the method described in Example 6.

$^1$H-NMR (400 MHz, $CDCl_3$)δ (ppm): 0.08 - 0.11 (m, 2H), 0.49 - 0.52 (m, 2H), 0.76 - 0.81 (m, 1.9H), 0.90 (d, J = 6 Hz, 2.1H), 1.05 - 1.13 (m, 1H), 1.52 - 1.68 (m, 2H), 1.75 - 1.81 (m, 0.6H), 1.85 - 1.95 (m, 1H), 2.02 - 2.15 (m, 1.4H), 2.29 - 2.39 (m, 3H), 2.47 - 2.51 (m, 1H), 2.65 - 2.75 (m, 1.6H), 2.83 - 3.02 (m, 2.4H), 3.79 (dd, J = 6, 16 Hz, 0.3H), 3.86 (d, J = 15 Hz, 0.3H), 3.98 - 4.20 (m, 2.4H), 4.35 - 4.41 (m, 0.7H), 4.67 - 4.73 (m, 0.3H), 6.61 - 6.69 (m, 1.3H), 6.91 (d, J = 8 Hz, 0.3H), 6.94 (d, J = 8 Hz, 0.7H), 7.02 - 7.03 (m, 0.7H), 7.15 - 7.22 (m, 1H), 7.45 (d, J = 8 Hz, 0.7H), 7.49 (d, J

= 8 Hz, 0.3H), 7.64 - 7.71 (m, 1H), 8.52 (d, J = 4 Hz, 0.3H), 8.55 (d, J = 4 Hz, 0.7H).

(Reference Example 51)

Synthesis of (4R,4aS,8aR,13bS)-3-(cyclopropylmethyl)-4a-hydroxy-10-methoxy-1,2,3,4,4a,5,6,7-octahydro-4,13-methanobenzofuro[2,3-c]pyrido[4,3-d]azepin-8(8aH)-one

[Chemical Formula 350]

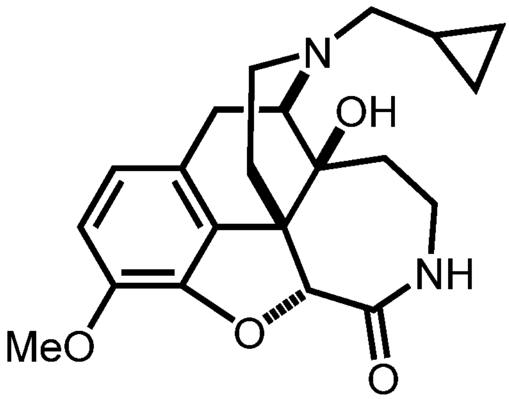

To (4R,4aS,7aR,12bS)-3-(cyclopropylmethyl)-4a-hydroxy-9-methoxy-2,3,4,4a,5,6-hexahydro-1H-4,12-methanobenzofuro[3,2-e]isoquinolin-7(7aH)-one (synthesized by the method described in Organic Letters 2009, 11, 539) (5.00 g, 14 mmol) suspended in polyphosphoric acid (100 mL) and then heated to an internal temperature of 60 to 80°C was added azidotrimethylsilane (5.55 mL, 42 mmol), followed by stirring at an internal temperature of 60 to 80°C for 30 minutes. Azidotrimethylsilane (5.55 mL, 42 mmol) was added at an internal temperature of 60 to 80°C, followed by stirring at an internal temperature of 60 to 80°C for 90 minutes. The reaction mixture was poured into an ice-cooled 28% aqueous ammonia solution (500 mL), and to the resultant solution, chloroform (500 mL) was added, followed by stirring at room temperature for 3 hours. The mixture was separated and then extracted twice with chloroform. The combined extracts were dried over sodium sulfate and then concentrated under reduced pressure. The obtained crude product was purified by silica gel column chromatography (amino group-supported silica gel, 0 to 10% methanol/chloroform) to yield the title compound (2.86 mg, 55%) as a colorless amorphous form.

$^1$H-NMR (400 MHz, $CDCl_3$)δ (ppm): 0.09 - 0.17 (m, 2H), 0.51 - 0.57 (m, 2H), 0.80 - 0.91 (m, 1H), 1.28 - 1.36 (m, 1H), 1.59 - 1.67 (m, 1H), 1.92 (ddd, J = 6, 14, 14 Hz, 1H), 2.28 - 2.42 (m, 3H), 2.46 (ddd, J = 6, 13, 13 Hz, 1H), 2.64 (dd, J = 7, 19 Hz, 1H), 2.72 (dd, J = 5, 11 Hz, 1H), 2.80 - 2.99 (m, 2H), 3.06 - 3.17 (m, 2H), 3.89 (s, 3H), 4.86 (s, 1H), 4.95 (br s, 1H), 5.95 (t, J = 7 Hz, 1H), 6.66 (d, J = 8 Hz, 1H), 6.78 (d, J = 8 Hz, 1H).

(Example 282)

Synthesis of (5aS,6R,11bR)-14-(cyclopropylmethyl)-5a,11-dihydroxy-10-methoxy-3,4,5,5a,6,7-hexahydro-6,11b-(epiminoethano)naphtho[1,2-d]azepin-2(1H)-one

[Chemical Formula 351]

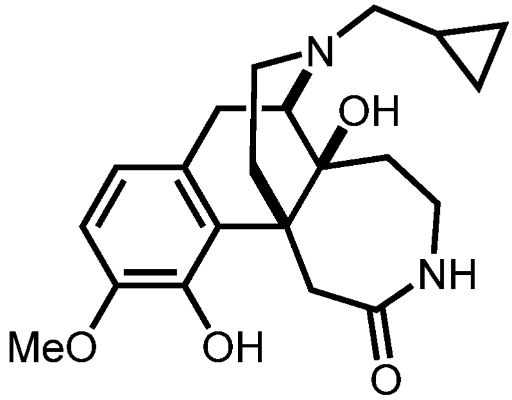

The title compound was obtained from the compound obtained in Reference Example 51 according to the method described in Example 275.

$^1$H-NMR (400 MHz, $CDCl_3$)δ (ppm): 0.06 - 0.15 (m, 2H), 0.47 - 0.55 (m, 2H), 0.75 - 0.88 (m, 1H), 1.43 - 1.71 (m, 2H), 1.80 - 2.05 (m, 3H), 2.32 (dd, J = 6, 13 Hz, 1H), 2.34 (dd, J = 6, 13 Hz, 1H), 2.54 - 2.63 (m, 1H), 2.80 - 3.00 (m, 4H), 3.15 (dd, J = 2, 14 Hz, 1H), 3.52 (d, J = 14 Hz, 1H), 3.81 (s, 3H), 3.84 - 3.96 (m, 1H), 4.63 (s, 1H), 5.85 (br s, 1H), 6.66 (d, J = 8 Hz, 1H), 6.72 (d, J = 8 Hz, 1H).

(Example 283)

Synthesis of (5aS,6R,11bS)-14-(cyclopropylmethyl)-10-methoxy-2,3,4,5,6,7-hexahydro-6,11b-(epiminoethano)naphtho[1,2-d]azepine-5a,11(1H)-diol

[Chemical Formula 352]

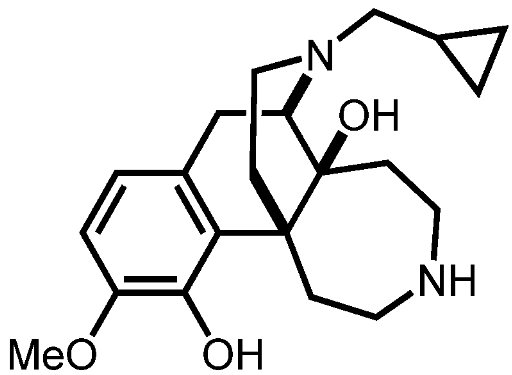

The title compound was obtained from the compound obtained in Example 282 according to the method described in Example 2.

$^1$H-NMR (400 MHz, $CDCl_3$)δ (ppm): 0.05 - 0.14 (m, 2H), 0.45 - 0.54 (m, 2H), 0.76 - 0.89 (m, 1H), 1.44 - 1.80 (m, 3H), 1.86 - 2.04 (m, 2H), 2.26 - 2.58 (m, 5H), 2.72 - 2.80 (m, 1H), 2.87 - 3.02 (m, 5H), 3.10 - 3.20 (m, 1H), 3.86 (s, 3H), 4.47 (br s, 1H), 6.59 (d, J = 8 Hz, 1H), 6.69 (d, J = 8 Hz, 1H).

(Example 284)

Synthesis of (5aS,6R,11bS)-14-(cyclopropylmethyl)-10-methoxy-3-(2-(4-methyl-1H-pyrazol-1-yl)ethyl)-2,3,4,5,6,7-hexahydro-6,11b-(epiminoethano)naphtho[1,2-d]azepine-5a,11(1H)-diol

[Chemical Formula 353]

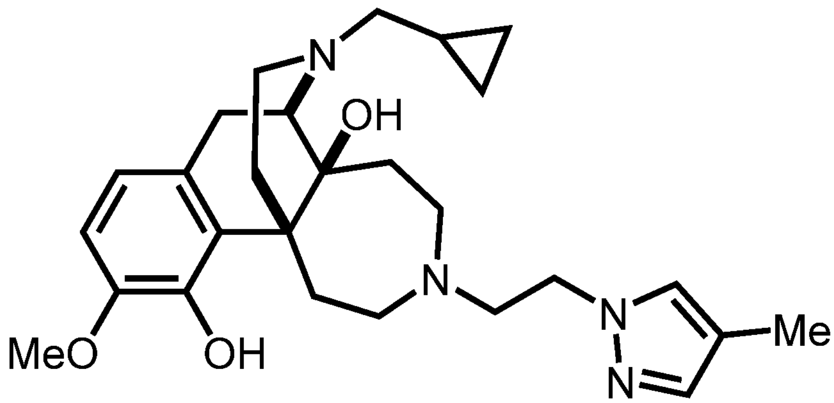

The title compound was obtained from the compound obtained in Example 283 and the compound obtained in Reference Example 33 according to the method described in Example 31.

$^1$H-NMR (400 MHz, $CDCl_3$)δ (ppm): 0.04 - 0.15 (m, 2H), 0.45 - 0.53 (m, 2H), 0.76 - 0.88 (m, 1H), 1.38 - 1.50 (m, 2H), 1.79 - 2.02 (m, 3H), 1.99 (s, 3H), 2.24 - 2.58 (m, 6H), 2.68 - 2.96 (m, 7H), 3.27 (dd, J = 11, 13 Hz, 1H), 3.84 (s, 3H), 3.92 - 4.06 (m, 2H), 4.54 (br s, 1H), 5.89 (s, 1H), 6.60 (d, J = 9 Hz, 1H), 6.69 (d, J = 8 Hz, 1H), 6.82 (s, 1H), 7.20 (s, 1H).

38/38H

(Example 285)

Synthesis of (5aS,6R,11bS)-14-(cyclopropylmethyl)-3-(2-(4-methyl-1H-pyrazol-1-yl)ethyl)-11-phenoxy-2,3,4,5,6,7-hexahydro-6,11b-(epiminoethano)naphtho[1,2-d]azepine-5a,10(1H)-diol

[Chemical Formula 354]

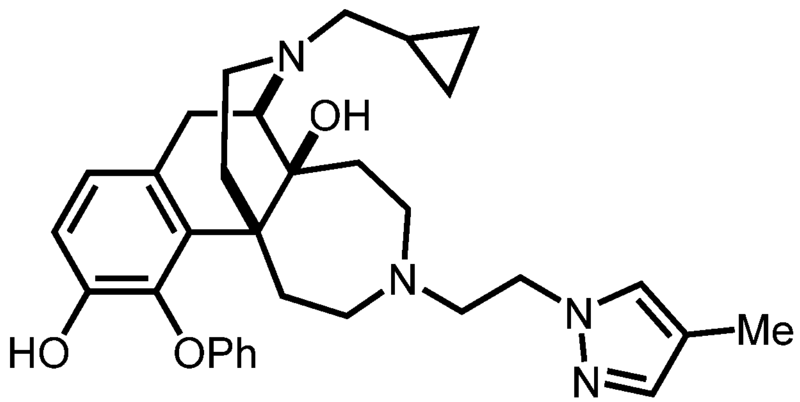

To the compound (23.3 mg, 0.050 mmol) obtained in Example 284, a copper powder (9.5 mg, 0.15 mmol) and potassium carbonate (34.6 mg, 0.25 mmol) suspended in pyridine (2 mL), bromobenzene (26.2 μL, 0.25 mmol) was added, followed by heating under reflux for 18 hours. After allowed to cool, to the reaction mixture was added a saturated sodium bicarbonate aqueous solution, followed by extraction three times with chloroform. The organic layers were combined, dried over sodium sulfate, and then concentrated under reduced pressure. The obtained crude product was used for the next reaction without purification.

To a solution of the obtained crude product in methanol (2 mL) was added sodium borohydride (18.9 mg, 0.50 mmol), followed by stirring at room temperature for 30 minutes. To the reaction mixture, a saturated sodium bicarbonate aqueous solution was added, followed by extraction three times with chloroform. The organic layers were combined, dried over sodium sulfate, and then concentrated under reduced pressure. The obtained crude product was used for the next reaction without purification.

To a solution of the obtained crude product in chloroform (2 mL) was added a 1 M boron tribromide-dichloromethane solution (0.5 mL, 0.5 mmol) under ice cooling, followed by stirring at room temperature for 30 minutes. To the reaction mixture, a 28% aqueous ammonia solution was added under ice cooling, followed by extraction three times with chloroform. The combined extracts were dried over sodium sulfate and then concentrated under reduced pressure. The obtained crude product was purified by preparative thin layer chromatography (chloroform : 2 M ammonia-methanol solution = 10 : 1) to yield the title compound (2.4 mg, 9%) as a colorless amorphous form.

$^1$H-NMR (400 MHz, $CDCl_3$)δ (ppm): 0.05 - 0.12 (m, 2H), 0.44 - 0.52 (m, 2H), 0.73 - 0.92 (m, 2H), 1.22 - 1.86 (m, 5H), 1.92 - 2.10 (m, 2H), 2.02 (s, 3H), 2.26 - 2.51 (m, 4H), 2.68 - 3.21 (m, 7H), 3.95 - 4.01 (m, 2H), 6.65 - 6.73 (m, 2H), 6.84 (s, 1H), 6.88 (d, J = 8 Hz, 1H), 6.94 (d, J = 8 Hz, 1H), 7.01 (t, J = 7 Hz, 1H), 7.22 (s, 1H), 7.24 - 7.29 (m, 2H).

(Reference Example 52)

Synthesis of (4bR,8aS,9R)-3-(benzyloxy)-11-(cyclopropylmethyl)-8a-hydroxy-8,8a,9,10-tetrahydro-5H-9,4b-(epiminoethano)phenanthren-6(7H)-one oxime

[Chemical Formula 355]

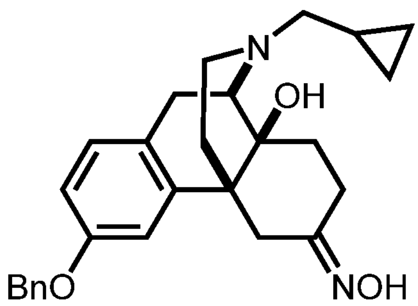

The title compound was obtained from (4bR,8aS,9R)-3-(benzyloxy)-11-(cyclopropylmethyl)-8a-hydroxy-8,8a,9,10-tetrahydro-5H-9,4b-(epiminoethano)phenanthren-6(7H)-one (synthesized by the method described in WO 2015097545) according to the method described in Reference Example 3.

$^1$H-NMR (400 MHz, $CDCl_3$)δ (ppm): 0.04 - 0.20 (m, 2H), 0.45 - 0.60 (m, 2H), 0.79 - 0.93 (m, 1H), 1.07 - 1.20 (m, 1H), 1.54 - 1.65 (m, 2H), 2.07 - 2.24 (m, 3H), 2.31 - 2.45 (m, 2.7H), 2.54 - 2.66 (m, 1.7H), 2.68 - 2.80 (m, 0.6H), 2.72 (dd, J = 7, 19 Hz, 1H), 2.98 - 3.11 (m, 2.3H), 3.70 (d, J = 14 Hz, 0.7H), 5.02 (s, 0.6H), 5.04 (s, 1.4H), 6.76 (dd, J = 3, 8 Hz, 0.3H), 6.77 (dd, J = 3, 8 Hz, 0.7H), 6.92 (d, J = 3 Hz, 0.3H), 6.96 (d, J = 8 Hz, 0.7H), 6.97 (d, J = 8 Hz, 0.3H), 7.17 (d, J = 3 Hz, 0.7H), 7.24 - 7.45 (m, 5H).

(Example 286)Synthesis of (5aS,6R,11bR)-10-(benzyloxy)-14-(cyclopropylmethyl)-5a-hydroxy-3,4,5,5a,6,7-hexahydro-6,11b-(epiminoethano)naphtho[1,2-d]azepin-2(1H)-one (isomer O) and (5aS,6R,11bS)-10-(benzyloxy)-14-(cyclopropylmethyl)-5a-hydroxy-1,2,5,5a,6,7-hexahydro-6,11b-(epiminoethano)naphtho[1,2-c]azepin-3(4H)-one (isomer P)

[Chemical Formula 356]

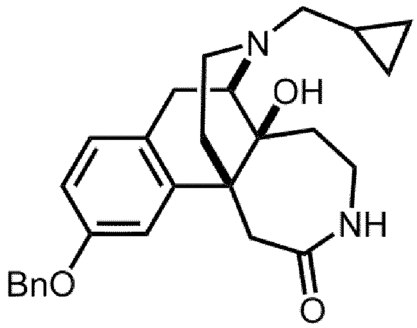

Isomer O

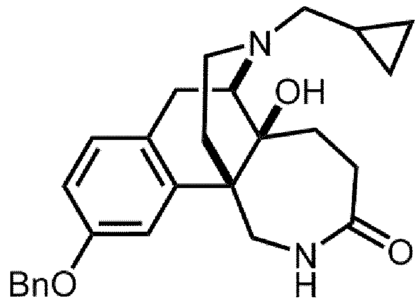

Isomer P

A mixture of the title isomers O and P was obtained from the compound obtained in Reference Example 52 according to the method described in Example 1.

$^1$H-NMR (400 MHz, $CDCl_3$)δ (ppm): 0.05 - 0.18 (m, 2H), 0.45 - 0.59 (m, 2H), 0.76 - 0.89 (m, 1H), 0.90 - 0.97 (m, 0.3H), 1.04 - 1.15 (m, 0.7H), 1.54 - 1.59 (m, 1H), 1.75 - 1.84 (m, 0.3H), 1.79 (ddd, J = 2, 11, 14 Hz, 0.7H), 1.92 - 2.21 (m, 2.3H), 2.29 - 2.41 (m, 2H), 2.52 - 2.64 (m, 1.7H), 2.72 - 2.81 (m, 0.3H), 2.76 (dd, J = 6, 19 Hz, 0.7H), 2.84 - 2.94 (m, 1H), 2.92 (d, J = 6 Hz, 0.7H), 2.95 - 3.05 (m, 0.6H), 3.02 (d, J = 19 Hz, 0.7H), 3.11 - 3.20 (m, 0.3H), 3.51 (d, J = 14 Hz, 0.7H), 3.87 (ddd, J = 4, 12, 15 Hz, 0.7H), 4.08 (dd, J = 6, 16 Hz, 0.3H), 5.01 (d, J = 11 Hz, 0.3H), 5.03 (d, J = 11 Hz, 0.3H), 5.06 (d, J = 12 Hz, 0.7H), 5.06 (d, J = 12 Hz, 0.7H), 5.57 - 5.63 (m, 0.3H), 5.66 - 5.73 (m, 0.7H), 6.53 (d, J = 3 Hz, 0.3H), 6.76 - 6.80 (m, 0.3H), 6.78 (dd, J = 2, 8 Hz, 0.7H), 6.97 (d, J = 8 Hz, 0.7H), 7.01 (d, J = 8 Hz, 0.3H), 7.25 - 7.26 (m, 0.7H), 7.27 - 7.49 (m, 5H).

(Example 287)

Synthesis of (5aS,6R,11bS)-10-(benzyloxy)-14-(cyclopropylmethyl)-2,3,4,5,6,7-hexahydro-6,11b-(epiminoethano)naphtho[1,2-d]azepin-5a(1H)-ol (isomer Q) and (5aS,6R,11bS)-10-(benzyloxy)-14-(cyclopropylmethyl)-2,3,4,5,6,7-hexahydro-6,11b-(epiminoethano)naphtho[1,2-c]azepin-5a(1H)-ol (isomer R)

[Chemical Formula 357]

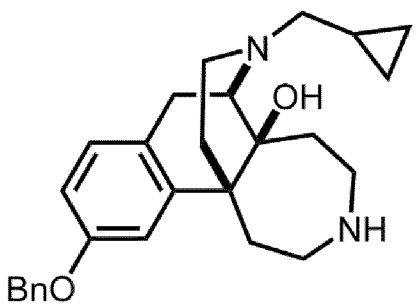

Isomer Q

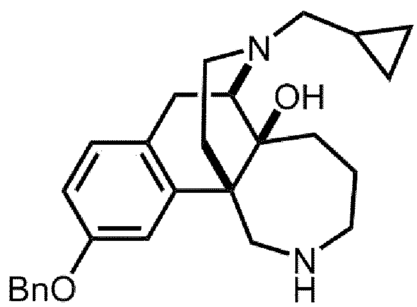

Isomer R

The title isomers Q and R were individually obtained from the compound obtained in Example 286 according to the method described in Example 2.

(Isomer Q)

$^1$H-NMR (400 MHz, $CDCl_3$)δ (ppm): 0.04 - 0.17 (m, 2H), 0.44 - 0.56 (m, 2H), 0.78 - 0.89 (m, 1H), 1.00 - 1.12 (m, 1H), 1.56 (ddd, J = 3, 3, 14 Hz, 1H), 1.73 (ddd, J = 4, 12, 15 Hz, 1H), 1.95 (ddd, J = 3, 3, 15 Hz, 1H), 1.99 - 2.07 (m, 2H), 2.28 (ddd, J = 5, 12, 16 Hz, 1H), 2.34 (dd, J = 7, 13 Hz, 1H), 2.34 (dd, J = 7, 13 Hz, 1H), 2.47 - 2.59 (m, 1H), 2.71 - 2.85 (m, 3H), 2.91 (ddd, J = 4, 4, 14 Hz, 1H), 2.92 (d, J = 7 Hz, 1H), 2.99 (d, J = 18 Hz, 1H)3.08 (ddd, J = 3, 12, 14

Hz, 1H), 4.06 (br s, 1H), 5.02 (d, J = 12 Hz, 1H), 5.03 (d, J = 12 Hz, 1H), 6.76 (d, J = 3 Hz, 1H), 6.79 (dd, J = 3, 8 Hz, 1H), 7.02 (d, J = 8 Hz, 1H), 7.29 - 7.44 (m, 5H).

(Isomer R)

$^1$H-NMR (400 MHz, $CDCl_3$)δ (ppm): 0.04 - 0.17 (m, 2H), 0.45 - 0.55 (m, 2H), 0.78 - 0.90 (m, 1H), 0.92 - 0.97 (m, 1H), 1.47 - 1.67 (m, 2H), 1.90 (ddd, J = 5, 13, 13 Hz, 1H), 1.94 - 2.06 (m, 2H), 2.19 - 2.32 (m, 1H), 2.33 (dd, J = 6, 13 Hz, 1H), 2.38 (dd, J = 6, 13 Hz, 1H), 2.54 - 2.60 (m, 1H), 2.67 (ddd, J = 7, 9, 14 Hz, 1H), 2.80 (dd, J = 6, 18 Hz, 1H), 2.90 (d, J = 6 Hz, 1H), 2.92 - 3.01 (m, 1H), 2.98 (d, J = 18 Hz, 1H), 3.13 (d, J = 15 Hz, 1H), 3.16 (d, J = 15 Hz, 1H), 4.64 (br s, 1H), 5.03 (d, J = 12 Hz, 1H), 5.04 (d, J = 12 Hz, 1H), 6.80 (dd, J = 3, 8 Hz, 1H), 6.83 (d, J = 3 Hz, 1H), 7.03 (d, J = 8 Hz, 1H), 7.29 - 7.46 (m, 5H).

(Example 288)

Synthesis of tert-butyl (5aS,6R,11bR)-10-(benzyloxy)-9-bromo-14-(cyclopropylmethyl)-5a-hydroxy-1,2,5,5a,6,7-hexahydro-6,11b-(epiminoethano)naphtho[1,2-d]azepine-3(4H)-carboxylate

[Chemical Formula 358]

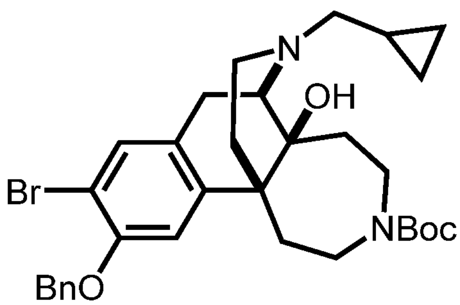

The isomer Q (151 mg, 0.361 mmol) obtained in Example 287 was suspended in water (2.0 mL), and dissolved by adding trifluoroacetic acid (400 pL, 5.23 mmol). After cooled to 0°C, N-bromosuccinimide (129 mg, 0.724 mmol) was added, followed by stirring at 0°C for 17 hours. To the reaction mixture, a mixed solution of 2 M aqueous sodium hydroxide solution/saturated aqueous sodium thiosulfate solution = 4/1 (v/v) (3 mL) and water (2 mL) were added, followed by extraction five times with a mixed solution of chloroform/isopropanol = 4/1 (v/v). The organic layers were combined, dried over sodium sulfate, and then concentrated under reduced pressure.

To a solution of the obtained crude product in chloroform (1.5 mL) were added triethylamine (250 pL, 1.79 mmol) and di-tert-butyl dicarbonate (170 pL, 0.740 mmol) at 0°C, followed by stirring at room temperature for 1.5 hours. After cooled to 0°C, to the reaction mixture were added a saturated aqueous sodium bicarbonate solution (1 mL) and water (2 mL), followed by extraction three times with ethyl acetate. The organic layers were combined, washed with saturated saline, dried over sodium sulfate, and then concentrated under reduced pressure. The obtained crude product was purified by silica gel column chromatography (0 to 5% methanol/ethyl acetate) to yield the title compound (119 mg, 55%) as a pale yellow amorphous form.

$^1$H-NMR (400 MHz, $CDCl_3$)δ (ppm): 0.05 - 0.16 (m, 2H), 0.43 - 0.58 (m, 2H), 0.75 - 0.86 (m, 1H), 0.90 - 0.99 (m, 1H), 1.29 (s, 4.5H), 1.31 (s, 4.5H), 1.43 - 1.60 (m, 2H), 1.74 - 2.10 (m, 3H), 2.28 (dd, J = 6, 12 Hz, 0.5H), 2.30 - 2.41 (m, 2H), 2.45 - 2.59 (m, 1.5H), 2.73 - 2.85 (m, 1H), 2.87 - 2.95 (m, 1.5H), 2.96 (d, J = 18 Hz, 0.5H), 3.27 - 3.42 (m, 2H), 3.46 - 3.61 (m, 2H), 5.10 (d, J = 12 Hz, 0.5H), 5.12 (d, J = 12 Hz, 0.5H), 5.16 (d, J = 12 Hz, 0.5H), 5.20 (d, J = 12 Hz, 0.5H), 6.66 (s, 0.5H), 6.69 (s, 0.5H), 7.27 (s, 1H), 7.29 - 7.51 (m, 5H).

(Example 289)

Synthesis of tert-butyl (5aS,6R,11bR)-10-(benzyloxy)-14-(cyclopropylmethyl)-5a-hydroxy-9-methyl-1,2,5,5a,6,7-hexahydro-6,11b-(epiminoethano)naphtho[1,2-d]azepine-3(4H)-carboxylate

[Chemical Formula 359]

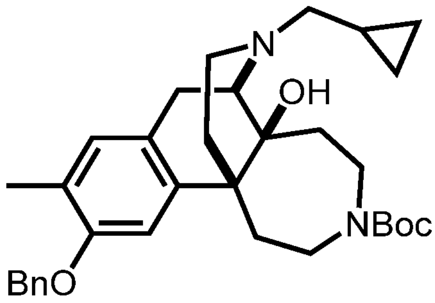

The title compound was obtained from the compound obtained in Example 288 according to the method described in Example 260.

$^1$H-NMR (400 MHz, $CDCl_3$)δ (ppm): 0.04 - 0.16 (m, 2H), 0.44 - 0.55 (m, 2H), 0.75 - 0.89 (m, 1H), 0.96 - 1.03 (m, 1H), 1.27 (s, 4.5H), 1.34 (s, 4.5H), 1.42 - 1.64 (m, 1H), 1.78 - 2.09 (m, 4H), 2.20 (s, 3H), 2.24 - 2.40 (m, 2.5H), 2.43 - 2.57 (m, 1.5H), 2.70 - 2.83 (m, 1H), 2.85 - 3.00 (m, 2H), 3.22 - 3.45 (m, 2H), 3.50 - 3.69 (m, 2H), 4.50 (br s, 1H), 5.02 (d, J = 12 Hz, 0.5H), 5.05 (d, J = 12 Hz, 0.5H), 5.07 (s, 1H), 6.59 (s, 0.5H), 6.61 (s, 0.5H), 6.85 (s, 0.5H), 6.86 (s, 0.5H), 7.27 - 7.47 (m, 5H).

(Example 290)

Synthesis of (5aS,6R,11bS)-14-(cyclopropylmethyl)-9-methyl-2,3,4,5,6,7-hexahydro-6,11b-(epiminoethano)naphtho[1,2-d]azepine-5a,10(1H)-diol

[Chemical Formula 360]

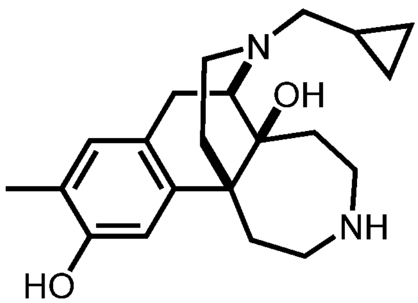

To a solution of the compound (88.1 mg, 0.165 mmol) obtained in Example 289 in methanol (1.5 mL) was added 20% palladium hydroxide-activated carbon (50% wet) (24.4 mg, 10 mol%), followed by stirring under a hydrogen atmosphere at room temperature for 17 hours. The reaction mixture was filtered through celite, and the filtrate was concentrated under reduced pressure.

To a solution of the obtained crude product in chloroform (2.0 mL) was added trifluoroacetic acid (0.30 mL) at 0°C, followed by stirring at room temperature for 1 hour. After cooled to 0°C, to the reaction mixture were added a 2 M aqueous sodium hydroxide solution (3 mL) and water (2 mL), followed by extraction ten times with chloroform/methanol = 10/1 (v/v) and three times with chloroform/isopropanol = 4/1 (v/v). The organic layers were combined, dried over sodium sulfate, and then concentrated under reduced pressure. The obtained crude product was purified by silica gel column chromatography (amino group-supported silica gel, 30 to 55% methanol/ethyl acetate) to yield the title compound (45.3 mg, 80%) as a white solid.

$^1$H-NMR (400 MHz, $CDCl_3$)δ (ppm): 0.03 - 0.16 (m, 2H), 0.42 - 0.57 (m, 2H), 0.77 - 0.88 (m, 1H), 1.03 - 1.11 (m, 1H), 1.48 (ddd, J = 3, 3, 15 Hz, 1H), 1.79 (ddd, J = 5, 12, 15 Hz, 1H), 1.94 (ddd, J = 5, 13, 13 Hz, 1H), 2.03 - 2.16 (m, 3H), 2.14 (s, 3H), 2.34 (d, J = 6 Hz, 2H), 2.48 - 2.56 (m, 1H), 2.70 (dd, J = 6, 18 Hz, 1H), 2.74 - 2.82 (m, 2H), 2.87 (d, J = 6 Hz, 1H), 3.03 - 3.12 (m, 1H), 3.08 (d, J = 18 Hz, 1H), 3.19 (ddd, J = 3, 3, 13 Hz, 1H), 4.57 (br s, 1H), 5.69 (br s, 1H), 6.48 (s, 1H), 6.80 (s, 1H).

(Example 291)

Synthesis of 1-((5aS,6R,11bR)-14-(cyclopropylmethyl)-5a,10-dihydroxy-9-methyl-1,2,5,5a,6,7-hexahydro-6,11b-(epiminoethano)naphtho[1,2-d]azepin-3(4H)-yl)-2-(pyridin-2-yl)ethan-1-one

[Chemical Formula 361]

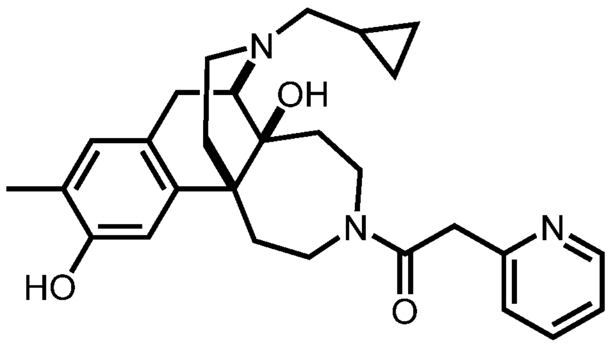

The title compound was obtained from the compound obtained in Example 290 and 2-(pyridin-2-yl)acetic acid hydrochloride according to the method described in Example 26.

$^1$H-NMR (400 MHz, $CDCl_3$)δ (ppm): 0.04 - 0.16 (m, 2H), 0.44 - 0.55 (m, 2H), 0.75 - 0.87 (m, 1H), 0.99 - 1.12 (m, 1H), 1.49 - 1.61 (m, 1H), 1.76 - 2.09 (m, 4H), 2.15 - 2.22 (m, 0.5H), 2.16 (s, 1.5H), 2.17 (s, 1.5H), 2.25 - 2.39 (m, 2H), 2.47 - 2.58 (m, 1.5H), 2.65 (dd, J = 6, 18 Hz, 0.5H), 2.73 (dd, J = 7, 18 Hz, 0.5H), 2.83 (d, J = 6 Hz, 0.5H), 2.89 (d, J = 7 Hz, 0.5H), 2.90 (d, J = 18 Hz, 0.5H), 2.94 (d, J = 18 Hz, 0.5H), 3.12 - 3.21 (m, 0.5H), 3.25 - 3.34 (m, 1H), 3.44 (ddd, J = 4, 4, 13 Hz, 0.5H), 3.71 (d, J = 15 Hz, 0.5H), 3.74 (d, J = 15 Hz, 0.5H), 3.78 - 3.98 (m, 1.5H), 3.90 (d, J = 15 Hz, 0.5H), 3.95 (d, J = 15 Hz, 0.5H), 4.14 - 4.22 (m, 0.5H), 4.42 (br s, 0.5H), 4.51 (br s, 0.5H), 6.66 (s, 0.5H), 6.77 (s, 0.5H), 6.79 (s, 0.5H), 6.79 (s, 0.5H), 7.11 - 7.19 (m, 1H), 7.21 - 7.25 (m, 1H), 7.59 (ddd, J = 2, 8, 8 Hz, 0.5H), 7.61 (ddd, J = 2, 8, 8 Hz, 0.5H), 8.45 - 8.51 (m, 1H).

(Example 292)

Synthesis of tert-butyl (5aS,6R,11bR)-10-(benzyloxy)-14-(cyclopropylmethyl)-5a-hydroxy-9-iodo-1,2,5,5a,6,7-hexahydro-6,11b-(epiminoethano)naphtho[1,2-d]azepine-3(4H)-carboxylate

[Chemical Formula 362]

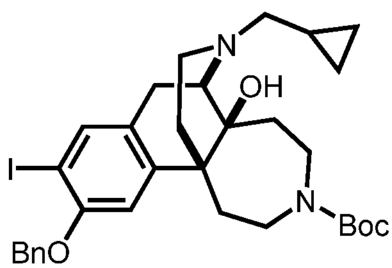

The title compound was obtained from the isomer Q obtained in Example 287 and N-iodosuccinimide according to the method described in Example 288.

$^1$H-NMR (400 MHz, $CDCl_3$)δ (ppm): 0.06 - 0.12 (m, 2H), 0.47 - 0.54 (m, 2H), 0.75 - 0.85 (m, 1H), 0.85 - 0.99 (m, 1H), 1.27 (s, 5H), 1.31 (s, 4H), 1.41 - 1.56 (m, 1H), 1.71 - 2.12 (m, 4H), 2.23 - 2.41 (m, 2.5H), 2.45 - 2.58 (m, 1.5H), 2.69 - 2.85 (m, 1H), 2.86 - 3.00 (m, 2H), 3.25 - 3.65 (m, 4H), 4.45 (br s, 1H), 5.00 - 5.24 (m, 2H), 6.58 (s, 0.5H), 6.61 (s,

0.5H), 7.28 - 7.35 (m, 1H), 7.35 - 7.45 (m, 2H), 7.46 - 7.55 (m, 3H).

(Example 293)

Synthesis of 3-(tert-butyl) 9-(2,4,6-trichlorophenyl) (5aS,6R,11bR)-10-(benzyloxy)-14-(cyclopropylmethyl)-5a-hydroxy-1,2,5,5a,6,7-hexahydro-6,11b-(epiminoethano)naphtho[1,2-d]azepine-3,9(4H)-dicarboxylate

[Chemical Formula 363]

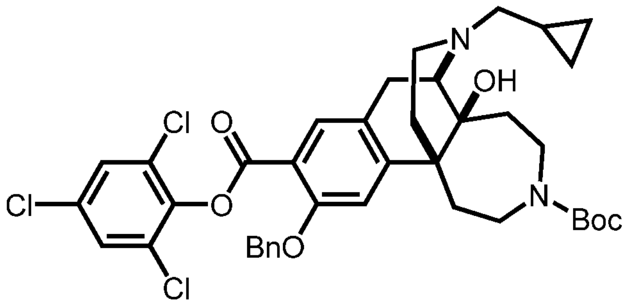

To the compound (40.0 mg, 0.062 mmol) obtained in Example 292 dissolved in toluene (1 mL) were added triethylamine (18.0 pL, 0.12 mmol), 2,4,6-trichlorophenyl formate, Xantphos (14.4 mg, 0.025 mmol), and palladium acetate (2.8 mg, 0.012 mmol), followed by stirring at room temperature for 16 hours. To the reaction solution, a saturated aqueous sodium bicarbonate solution was added, followed by extraction three times with ethyl acetate. The organic layer was dried over sodium sulfate and concentrated under reduced pressure. The obtained crude product was purified by preparative thin layer chromatography (chloroform : methanol = 20 : 1) to yield the title compound (25.6 mg, 56%) as a brown amorphous form.

$^1$H-NMR (400 MHz, $CDCl_3$)$\delta$ (ppm): 0.06 - 0.16 (m, 2H), 0.49 - 0.56 (m, 2H), 0.77 - 0.90 (m, 1H), 0.97 - 1.06 (m, 1H), 1.27 (s, 4.5H), 1.29 (s, 4.5H), 1.50 - 1.66 (m, 1H), 1.75 - 2.20 (m, 4H), 2.27 - 2.53 (m, 2.5H), 2.54 - 2.68 (m, 1.5H), 2.83 - 2.97 (m, 1H), 2.98 - 3.12 (m, 2H), 3.26 - 3.64 (m, 3.5H), 3.73 (ddd, J = 4, 14, 14 Hz, 0.5H), 5.17 (d, J = 12 Hz, 0.5H), 5.23 (d, J = 12 Hz, 0.5H), 5.25 (d, J = 12 Hz, 0.5H), 5.32 (d, J = 12 Hz, 0.5H), 6.83 (s, 0.5H), 6.86 (s, 0.5H), 7.25 - 7.31 (m, 1H), 7.31 - 7.38 (m, 2H), 7.40 (s, 2H), 7.46 - 7.52 (m, 2H), 7.85 (s, 0.5H), 7.86 (s, 0.5H).

(Example 294)

Synthesis of tert-butyl (5aS,6R,11bR)-10-(benzyloxy)-9-carbamoyl-14-(cyclopropylmethyl)-5a-hydroxy-1,2,5,5a,6,7-hexahydro-6,11b-(epiminoethano)naphtho[1,2-d]azepine-3(4H)-carboxylate

[Chemical Formula 364]

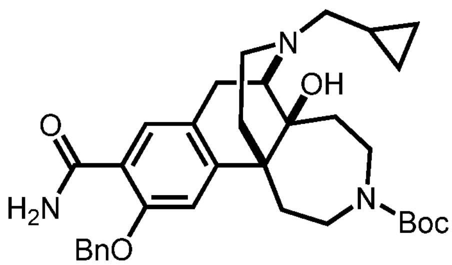

To the compound (62.9 mg, 0.098 mmol) obtained in Example 292 dissolved in toluene (2 mL) were added triethylamine (27 pL, 0.20 mmol), 2,4,6-trichlorophenyl formate (44 mg, 0.20 mmol), Xantphos (22.6 mg, 0.039 mmol), and palladium acetate (4.4 mg, 0.020 mmol), followed by stirring at room temperature for 18 hours. The reaction mixture was concentrated under reduced pressure. The obtained concentrated residue was purified by silica gel column chromatography (70 to 100% ethyl acetate/heptane) to yield a mixture (58.8 mg) of the title compound and the raw material

compound (compound described in Example 292) as a brown amorphous form.

To this mixture dissolved in tetrahydrofuran (1 mL), a 28% aqueous ammonia solution (1 mL) was added, followed by stirring at 45°C for 1 hour. To the reaction solution, a saturated aqueous sodium bicarbonate solution was added, followed by extraction with ethyl acetate. The organic layer was dried over sodium sulfate and concentrated under reduced pressure. The obtained crude product was purified by preparative thin layer chromatography (chloroform : methanol = 20 : 1) to individually yield the title compound (19.5 mg, 36%) and a raw material compound (compound described in Example 292) (32.3 mg, 51%) as colorless amorphous forms.

$^1$H-NMR (400 MHz, $CDCl_3$)δ (ppm): 0.06 - 0.15 (m, 2H), 0.45 - 0.57 (m, 2H), 0.76 - 0.89 (m, 1H), 0.95 - 1.05 (m, 1H), 1.25 (s, 6.3H), 1.30 (s, 2.7H), 1.51 (ddd, J = 3, 3, 15 Hz, 0.7H), 1.57 - 1.64 (m, 0.3H), 1.71 - 2.14 (m, 5H), 2.23 - 2.42 (m, 2H), 2.46 - 2.61 (m, 2H), 2.79 - 3.11 (m, 3H), 3.31 - 3.49 (m, 2.7H), 3.51 - 3.76 (m, 1.3H), 5.14 (d, J = 11 Hz, 0.3H), 5.18 (d, J = 11 Hz, 0.3H), 5.23 (d, J = 11 Hz, 0.7H), 5.33 (d, J = 11 Hz, 0.7H), 5.63 - 5.75 (m, 1H), 6.79 (s, 0.3H), 6.82 (s, 0.7H), 7.34 - 7.49 (m, 5H), 7.66 - 7.77 (m, 1H), 7.93 (s, 0.7H), 7.95 (s, 0.3H).

(Example 295)

Synthesis of (5aS,6R, 11bS)-14-(cyclopropylmethyl)-5a,10-dihydroxy-3-(2-(4-methyl-1H-pyrazol-1-yl)ethyl)-1,2,3,4,5,5a,6,7-octahydro-6,11b-(epiminoethano)naphtho[1,2-d]azepine-9-carboxamide

[Chemical Formula 365]

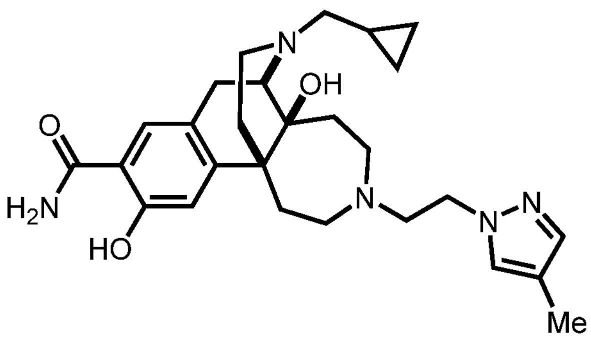

To the compound (19 mg, 0.034 mmol) obtained in Example 294 dissolved in methanol (5 mL), 20% palladium hydroxide-activated carbon (50% wet, 24 mg) was added, followed by stirring under a hydrogen atmosphere at room temperature for 21 hours. The reaction mixture was filtered through a membrane filter, and the filtrate was concentrated under reduced pressure. To the obtained concentrated residue dissolved in chloroform (2 mL), trifluoroacetic acid (0.4 mL) was added, followed by stirring at room temperature for 2 hours. The reaction mixture was concentrated under reduced pressure. To the obtained concentrated residue dissolved in acetonitrile (2 mL) were added N,N-diisopropylethylamine (35.4 pL, 0.20 mmol) and the compound (10.4 mg, 0.051 mmol) obtained in Reference Example 33, followed by stirring at 60°C for 13 hours. To the reaction solution, a saturated sodium bicarbonate aqueous solution was added, followed by extraction three times with chloroform. The organic layer was dried over sodium sulfate and concentrated under reduced pressure. The obtained crude product was purified by preparative thin layer chromatography (chloroform : 10% aqueous ammonia-methanol solution = 20 : 1) to yield the title compound (8.2 mg, 51%) as a colorless amorphous form.

$^1$H-NMR (400 MHz, $CDCl_3$)δ (ppm): 0.06 - 0.12 (m, 2H), 0.48 - 0.55 (m, 2H), 0.77 - 0.89 (m, 1H), 1.01 - 1.08 (m, 1H), 1.48 (ddd, J = 2, 4, 14 Hz, 1H), 1.70 (ddd, J = 3, 11, 14 Hz, 1H), 1.85 (ddd, J = 3, 3, 16 Hz, 1H), 1.92 - 2.01 (m, 1H), 1.99 (s, 3H), 2.08 (ddd, J = 4, 13, 13 Hz, 1H), 2.35 (d, J = 6 Hz, 2H), 2.36 - 2.47 (m, 2H), 2.53 - 2.60 (m, 1H), 2.63 (ddd, J = 4, 4, 13 Hz, 1H), 2.74 - 2.88 (m, 3H), 2.89 - 3.02 (m, 3H), 3.07 - 3.18 (m, 1H), 3.90 - 4.06 (m, 2H), 6.80 (s, 1H), 6.85 (s, 1H), 7.15 (s, 1H), 7.21 (s, 1H).

(Example 296)

Synthesis of (5aS,6R,11bR)-14-(cyclopropylmethyl)-5a,10-dihydroxy-9-iodo-3,4,5,5a,6,7-hexahydro-6,11b-(epiminoethano)naphtho[1,2-d]azepin-2(1H)-one

[Chemical Formula 366]

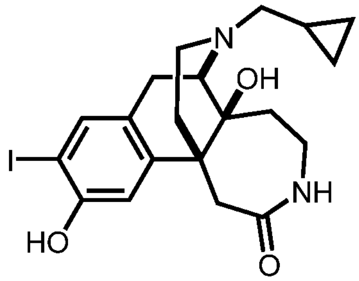

The title compound was obtained from the compound obtained in Example 140 and N-iodosuccinimide according to the methods described in Example 6 and Example 259.

$^1$H-NMR (400 MHz, $CDCl_3$)δ (ppm): 0.05 - 0.20 (m, 2H), 0.45 - 0.60 (m, 2H), 0.70 - 0.90 (m, 1H), 0.95 - 1.10 (m, 1H), 1.50 - 2.20 (m, 4H), 2.20 - 2.40 (m, 2H), 2.40 - 2.65 (m, 2H), 2.65 - 2.80 (m, 1H), 2.80 - 3.00 (m, 3H), 3.40 - 3.60 (m, 1H), 3.70 - 3.90 (m, 1H), 6.14 (br s, 0.1H), 6.39 (br s, 0.9H), 6.98 (s, 0.1H), 7.10 (s, 0.9H), 7.36 (s, 0.9H), 7.45 (s, 0.1H), 8.94 (br s, 1H).

(Example 297)

Synthesis of (5aS,6R,11bS)-14-(cyclopropylmethyl)-9-iodo-2,3,4,5,6,7-hexahydro-6,11b-(epiminoethano)naphtho[1,2-d]azepine-5a,10(1H)-diol

[Chemical Formula 367]

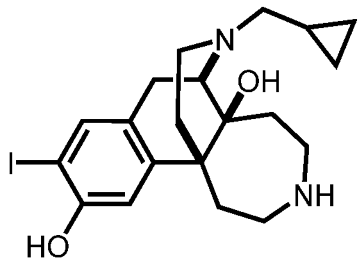

The title compound was obtained from the compound obtained in Example 296 according to the method described in Example 2.

$^1$H-NMR (400 MHz, $CDCl_3$)δ (ppm): 0.05 - 0.20 (m, 2H), 0.45 - 0.60 (m, 2H), 0.70 - 0.85 (m, 1H), 1.00 - 1.10 (m, 1H), 1.30 - 1.50 (m, 1H), 1.50 - 1.65 (m, 2H), 1.65 - 1.90 (m, 1H), 1.90 - 2.25 (m, 4H), 2.25 - 2.40 (m, 2H), 2.45 - 2.60 (m, 1H), 2.65 - 3.40 (m, 5H), 6.63 (s, 1H), 7.42 (s, 1H).

(Example 298)

Synthesis of (5aS,6R,11bS)-14-(cyclopropylmethyl)-9-iodo-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 368]

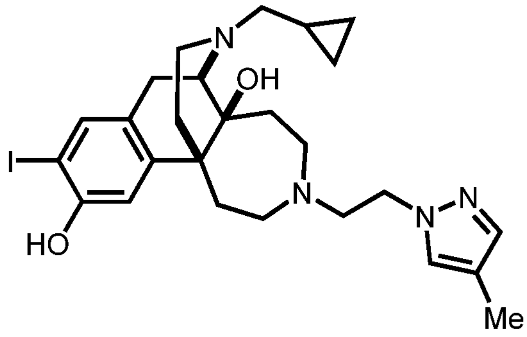

The title compound was obtained from the compound obtained in Example 297 and the compound obtained in Reference Example 33 according to the method described in Example 31.

$^1$H-NMR (400 MHz, $CDCl_3$)$\delta$ (ppm): 0.04 - 0.16 (m, 2H), 0.44 - 0.56 (m, 2H), 0.76 - 0.88 (m, 1H), 0.95 - 1.03 (m, 1H), 1.42 - 1.50 (m, 1H), 1.69 - 1.81 (m, 2H), 1.92 - 2.09 (m, 2H), 2.02 (s, 3H), 2.28 - 2.42 (m, 3H), 2.47 - 2.57 (m, 2H), 2.60 (ddd, J = 4, 4, 13 Hz, 1H), 2.76 (dd, J = 6, 18 Hz, 1H), 2.82 - 2.98 (m, 5H), 3.11 - 3.20 (m, 1H), 3.98 - 4.10 (m, 2H), 6.67 (s, 1H), 6.90 (s, 1H), 7.23 (s, 1H), 7.41 (s, 1H).

(Example 299)

Synthesis of tert-butyl (5aS,6R,11bR)-14-(cyclopropylmethyl)-5a-hydroxy-10-(6-oxo-1,6-dihydropyridin-2-yl)-1,2,5,5a,6,7-hexahydro-6,11b-(epiminoethano)naphtho[1,2-d]azepine-3(4H)-carboxylate

[Chemical Formula 369]

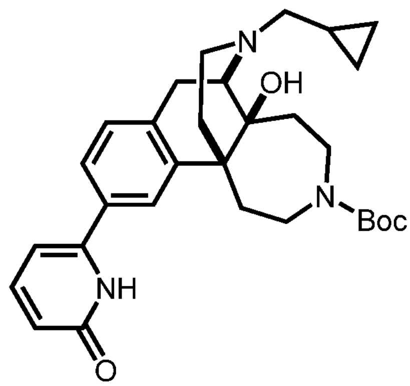

The title compound was obtained from the compound obtained in Example 240 and 6-(4,4,5,5-tetramethyl-1,3,2-dioxaboran-2-yl)pyridin-2(1H)-one according to the method described in Example 279.

$^1$H-NMR (400 MHz, $CDCl_3$)$\delta$ (ppm): 0.05 - 0.20 (m, 2H), 0.50 - 0.70 (m, 2H), 0.80 - 1.00 (m, 1H), 1.08 (s, 6.3H), 1.16 (s, 2.7H), 1.40 - 2.20 (m, 10H), 2.30 - 2.40 (m, 2H), 2.50 - 2.65 (m, 1H), 2.80 - 3.10 (m, 1.7H), 3.10 - 3.30 (m, 1.3H), 3.30 - 3.40 (m, 0.3H), 3.50 - 3.60 (m, 0.7H), 4.25 - 4.40 (m, 1H), 6.20 - 6.35 (m, 1H), 6.50 - 6.65 (m, 2H), 7.10 - 7.30 (m, 2H), 7.30 - 7.40 (m, 1H).

(Example 300)

Synthesis of 6-((5aS,6R,11bR)-14-(cyclopropylmethyl)-5a-hydroxy-3-(2-(pyridin-2-yl)acetyl)-1,2,3,4,5,5a,6,7-octahydro-6,11b-(epiminoethano)naphtho[1,2-d]azepin-10-yl)pyridin-2(1H)-one

[Chemical Formula 370]

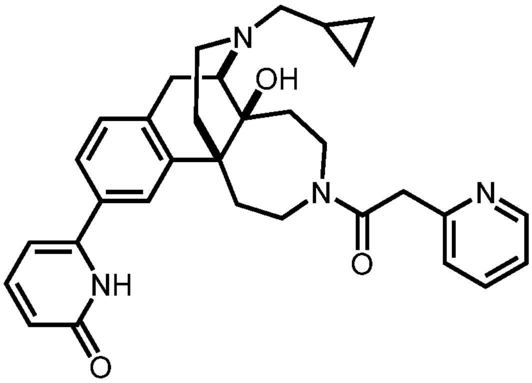

To the compound (11 mg, 0.022 mmol) obtained in Example 299 was added 2 M hydrochloric acid (1 mL), followed by stirring at room temperature for 3 hours. The reaction solution was neutralized with a 1 M aqueous sodium hydroxide solution under ice cooling, and then the aqueous layer was extracted three times with chloroform. The obtained organic layer was dried over sodium sulfate and then concentrated under reduced pressure. To a solution of the obtained crude product in N,N-dimethylformamide (0.5 mL) were added 2-(pyridin-2-yl)acetic acid hydrochloride (5 mg, 0.033 mmol), N,N-diisopropylethylamine (15 μL, 0.088 mmol), and O-(7-azabenzotriazol-1-yl)-N,N,N',N'-tetramethyluronium hexafluorophosphate (HATU) (9 mg, 0.023 mmol), followed by stirring at room temperature for 16 hours. To the reaction mixture, a saturated aqueous sodium bicarbonate solution was added, followed by extraction three times with ethyl acetate. The combined extracts were dried over sodium sulfate and then concentrated under reduced pressure. The obtained crude product was purified by preparative thin layer chromatography (chloroform : 2 M ammonia-methanol solution = 10 : 1) to yield the title compound (1.0 mg, 8%) as a pale yellow oily matter.

$^1$H-NMR (400 MHz, $CDCl_3$)δ (ppm): 0.05 - 0.30 (m, 2H), 0.50 - 0.70 (m, 2H), 0.80 - 1.00 (m, 1H), 1.00 - 1.90 (m, 5H), 1.90 - 2.20 (m, 2.5H), 2.20 - 2.50 (m, 2.5H), 2.50 - 2.80 (m, 2H), 2.80 - 3.20 (m, 3H), 3.20 - 3.80 (m, 4H), 3.80 - 4.00 (m, 1H), 4.20 - 4.50 (m, 1H), 6.31 (d, J = 7 Hz, 0.5H), 6.40 - 6.60 (m, 1.5H), 7.00 - 7.30 (m, 4H), 7.30 - 7.55 (m, 2.5H), 7.60 (dt, J = 2, 8 Hz, 0.5H), 8.35 - 8.45 (m, 0.5H), 8.75 - 8.80 (m, 0.5H).

(Example 301)

Synthesis of tert-butyl (5aS,6R,11bR)-10-(benzyloxy)-9-cyclopropyl-14-(cyclopropylmethyl)-5a-hydroxy-1,2,5,5a,6,7-hexahydro-6,11b-(epiminoethano)naphtho[1,2-d]azepine-3(4H)-carboxylate

[Chemical Formula 371]

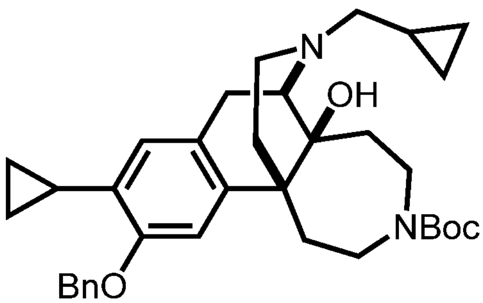

The title compound was obtained from the compound obtained in Example 288 and cyclopropylboronic acid according to the method described in Example 260.

$^1$H-NMR (400 MHz, $CDCl_3$)δ (ppm): 0.06 - 0.13 (m, 2H), 0.46 - 0.60 (m, 3H), 0.63 - 0.72 (m, 1H), 0.76 - 0.91 (m, 3H), 0.94 - 1.02 (m, 1H), 1.28 (s, 4.5H), 1.33 (s, 4.5H), 1.42 - 1.50 (m, 0.5H), 1.76 - 1.89 (m, 1.5H), 1.90 - 2.09 (m, 3H), 2.13 - 2.23 (m, 1H), 2.24 - 2.39 (m, 2.5H), 2.42 - 2.57 (m, 1.5H), 2.68 - 2.81 (m, 1H), 2.84 - 2.92 (m, 1.5H), 2.93 (d, J = 18 Hz, 0.5H), 3.24 - 3.45 (m, 2H), 3.49 - 3.67 (m, 2H), 4.48 (br s, 1H), 5.05 (d, J = 11 Hz, 0.5H), 5.08 (d, J = 11

Hz, 0.5H), 5.11 (s, 1H), 6.52 (s, 0.5H), 6.53 (s, 0.5H), 6.61 (s, 0.5H), 6.63 (s, 0.5H), 7.28 - 7.51 (m, 5H).

(Example 302)

Synthesis of (5aS,6R,11bS)-9-cyclopropyl-14-(cyclopropylmethyl)-2,3,4,5,6,7-hexahydro-6,11b-(epiminoethano)naphtho[1,2-d]azepine-5a,10(1H)-diol

[Chemical Formula 372]

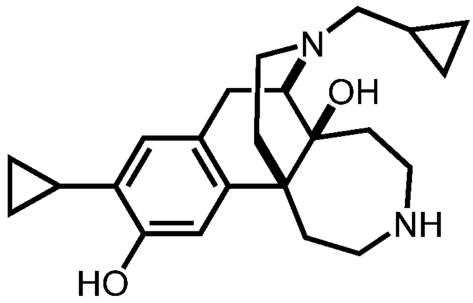

The title compound was obtained from the compound obtained in Example 301 according to the method described in Example 290.

$^1$H-NMR (400 MHz, $CDCl_3$)δ (ppm): 0.06 - 0.16 (m, 2H), 0.47 - 0.54 (m, 2H), 0.56 - 0.67 (m, 2H), 0.77 - 0.97 (m, 3H), 1.02 - 1.11 (m, 1H), 1.49 (ddd, J = 3, 3, 15 Hz, 1H), 1.77 (ddd, J = 5, 12, 15 Hz, 1H), 1.95 (ddd, J = 5, 12, 12 Hz, 1H), 1.99 - 2.21 (m, 4H), 2.33 (d, J = 6 Hz, 2H), 2.47 - 2.56 (m, 1H), 2.69 (dd, J = 6, 18 Hz, 1H), 2.72 - 2.85 (m, 2H), 2.89 (d, J = 6 Hz, 1H), 2.92 (d, J = 18 Hz, 1H), 3.06 (ddd, J = 4, 4, 14 Hz, 1H), 3.17 (ddd, J = 3, 13, 13 Hz, 1H), 4.50 (br s, 1H), 6.54 (s, 1H), 6.55 (s, 1H).

(Example 303)

Synthesis of 1-((5aS,6R,11bR)-9-cyclopropyl-14-(cyclopropylmethyl)-5a,10-dihydroxy-1,2,5,5a,6,7-hexahydro-6,11b-(epiminoethano)naphtho[1,2-d]azepin-3(4H)-yl)-2-(pyridin-2-yl)ethan-1-one

[Chemical Formula 373]

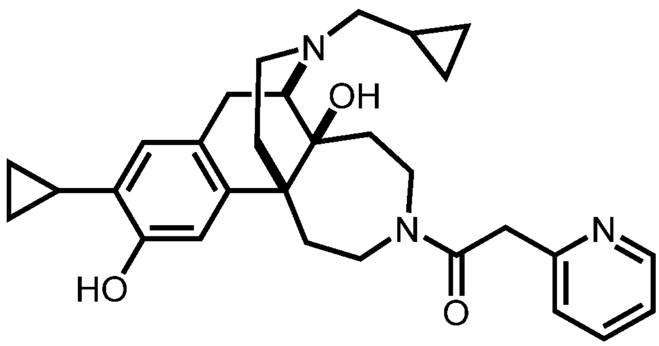

The title compound was obtained from the compound obtained in Example 302 and 2-(pyridin-2-yl)acetic acid hydrochloride according to the method described in Example 26.

$^1$H-NMR (400 MHz, $CDCl_3$)δ (ppm): 0.05 - 0.15 (m, 2H), 0.44 - 0.54 (m, 2H), 0.55 - 0.70 (m, 2H), 0.75 - 0.98 (m, 3H), 0.99 - 1.09 (m, 1H), 1.49 - 1.62 (m, 1H), 1.74 - 2.06 (m, 4.6H), 2.08 - 2.26 (m, 1H), 2.27 - 2.38 (m, 0.8H), 2.30 (dd, J = 7, 13 Hz, 0.6H), 2.34 (dd, J = 6, 13 Hz, 0.6H), 2.44 - 2.56 (m, 1.6H), 2.64 (dd, J = 6, 18 Hz, 0.4H), 2.73 (dd, J = 6, 18 Hz, 0.6H), 2.82 (d, J = 6 Hz, 0.4H), 2.89 (d, J = 18 Hz, 0.6H), 2.89 (d, J = 6 Hz, 0.6H), 2.92 (d, J = 18 Hz, 0.4H), 3.12 - 3.22 (m, 0.4H), 3.31 - 3.41 (m, 1H), 3.45 (ddd, J = 4, 4, 14 Hz, 0.4H), 3.68 (d, J = 15 Hz, 0.6H), 3.73 - 3.82 (m,

0.4H), 3.78 (d, J = 15 Hz, 0.6H), 3.84 - 3.95 (m, 1.2H), 3.90 (s, 0.8H), 4.09 - 4.17 (m, 0.4H), 4.43 (br s, 0.4H), 4.52 (br s, 0.6H), 6.64 (s, 0.4H), 6.65 (s, 0.6H), 6.71 (s, 0.6H), 6.72 (s, 0.4H), 7.11 - 7.17 (m, 1H), 7.20 (d, J = 8 Hz, 0.6H), 7.22 (d, J = 8 Hz, 0.4H), 7.58 (ddd, J = 2, 8, 8 Hz, 0.4H), 7.60 (ddd, J = 2, 8, 8 Hz, 0.6H), 8.46 - 8.50 (m, 1H).

(Example 304)

Synthesis of (5aS,6R,11bS)-9-cyclopropyl-14-(cyclopropylmethyl)-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 374]

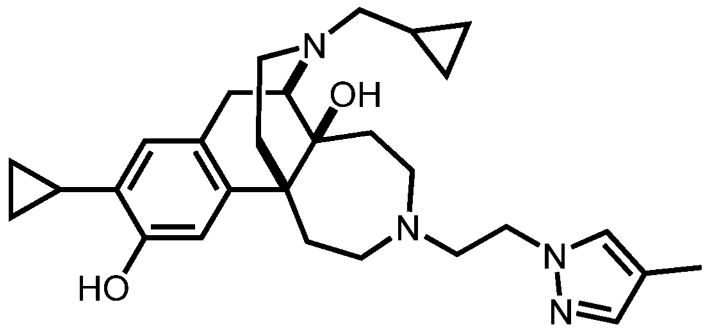

The title compound was obtained from the compound obtained in Example 302 and the compound obtained in Reference Example 33 according to the method described in Example 31.

$^1$H-NMR (400 MHz, $CDCl_3$)δ (ppm): 0.02 - 0.15 (m, 2H), 0.43 - 0.54 (m, 2H), 0.58 - 0.67 (m, 2H), 0.76 - 1.05 (m, 4H), 1.41 (ddd, J = 2, 5, 14 Hz, 1H), 1.75 (ddd, J = 3, 11, 14 Hz, 1H), 1.77 - 1.86 (m, 2H), 1.94 - 2.08 (m, 2H), 2.00 (s, 3H), 2.29 - 2.39 (m, 1H), 2.33 (d, J = 7 Hz, 2H), 2.44 (ddd, J = 4, 4, 13 Hz, 1H), 2.49 - 2.54 (m, 1H), 2.61 (ddd, J = 4, 4, 13 Hz, 1H), 2.72 (dd, J = 6, 18 Hz, 1H), 2.82 (ddd, J = 6, 6, 13 Hz, 1H), 2.84 (ddd, J = 6, 6, 13 Hz, 1H), 2.86 - 2.98 (m, 1H), 2.88 (d, J = 6 Hz, 1H), 2.92 (d, J = 18 Hz, 1H), 3.17 (ddd, J = 2, 11, 14 Hz, 1H), 3.98 (ddd, J = 6, 6, 14 Hz, 1H), 4.01 (ddd, J = 6, 6, 14 Hz, 1H), 4.60 (br s, 1H), 6.61 (s, 1H), 6.75 (s, 1H), 6.79 (s, 1H), 7.22 (s, 1H).

(Example 305)

Synthesis of (5aS,6R,11bR)-9-chloro-14-(cyclopropylmethyl)-5a-hydroxy-10-methoxy-3,4,5,5a,6,7-hexahydro-6,11b-(epiminoethano)naphtho[1,2-d]azepin-2(1H)-one (isomer S) and (5aS,6R,11bR)-11-chloro-14-(cyclopropylmethyl)-5a-hydroxy-10-methoxy-3,4,5,5a,6,7-hexahydro-6,11b-(epiminoethano)naphtho[1,2-d]azepin-2(1H)-one (isomer T)

[Chemical Formula 375]

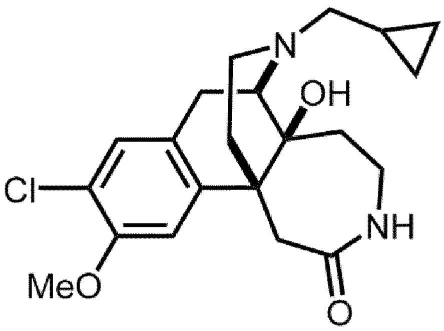

Isomer S

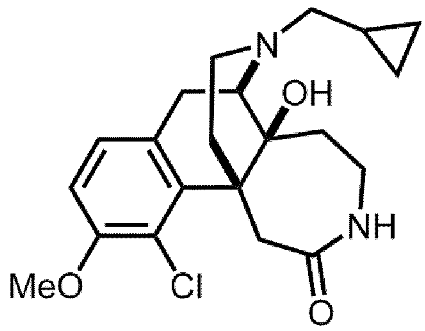

Isomer T

To the compound (300 mg, 0.84 mmol) obtained in Example 140 dissolved in a 0.2 M trifluoroacetic acid aqueous solution (20 mL), N-chlorosuccinimide (225 mg, 1.68 mmol) was added, followed by stirring at 80°C for 4 hours. To the reaction solution, sodium thiosulfate (380 mg) and a saturated sodium bicarbonate aqueous solution were added, followed by extraction three times with chloroform. The organic layer was dried over sodium sulfate and concentrated under reduced pressure. The obtained crude product was purified by silica gel column chromatography (amino group-supported silica gel, 0 to 20% methanol/ethyl acetate) to individually yield an isomer S (151 mg, 46%) and an isomer T (109 mg, 33%) as white solids.

(Isomer S)

$^1$H-NMR (400 MHz, $CDCl_3$)δ (ppm): 0.07 - 0.16 (m, 2H), 0.48 - 0.57 (m, 2H), 0.76 - 0.87 (m, 1H), 1.02 - 1.11 (m, 1H), 1.29 - 1.44 (m, 1H), 1.48 - 1.58 (m, 1H), 1.95 - 2.12 (m, 2H), 2.29 - 2.40 (m, 2H), 2.46 - 2.60 (m, 2H), 2.67 - 2.82 (m, 2H), 2.86 - 2.99 (m, 2H), 3.44 - 3.51 (m, 1H), 3.74 - 3.85 (m, 1H), 3.87 (s, 3H), 4.66 (br s, 1H), 6.61 - 6.91 (m, 1H), 7.03 (s, 1H), 7.15 (s, 1H).

(Isomer T)

$^1$H-NMR (400 MHz, $CDCl_3$)δ (ppm): 0.05 - 0.15 (m, 2H), 0.45 - 0.57 (m, 2H), 0.76 - 0.88 (m, 1H), 1.53 - 1.63 (m, 1H), 1.78 - 2.07 (m, 4H), 2.26 - 2.38 (m, 2H), 2.58 - 3.02 (m, 5H), 3.37 - 3.59 (m, 2H), 3.71 - 3.85 (m, 1H), 3.81 (s, 3H), 4.54 (br s, 1H), 5.74 - 6.04 (m, 1H), 6.75 (d, J = 8 Hz, 1H), 6.94 (d, J = 8 Hz, 1H).

(Example 306)

Synthesis of (5aS,6R,11bS)-9-chloro-14-(cyclopropylmethyl)-10-methoxy-2,3,4,5,6,7-hexahydro-6,11b-(epiminoethano)naphtho[1,2-d]azepin-5a(1H)-ol

[Chemical Formula 376]

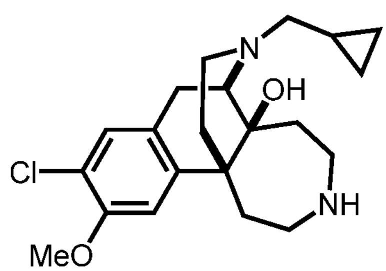

The title compound was obtained from the isomer S obtained in Example 305 according to the method described in Example 2.

$^1$H-NMR (400 MHz, $CDCl_3$)δ (ppm): 0.06 - 0.14 (m, 2H), 0.47 - 0.56 (m, 2H), 0.77 - 0.89 (m, 1H), 1.02 - 1.10 (m, 1H), 1.58 (ddd, J = 3, 3, 15 Hz, 1H), 1.66 - 1.76 (m, 1H), 1.94 - 2.10 (m, 3H), 2.28 - 2.44 (m, 3H), 2.52 - 2.59 (m, 1H), 2.74 - 3.14 (m, 7H), 3.86 (s, 3H), 4.49 (brs, 1H), 6.72 (s, 1H), 7.11 (s, 1H).

(Example 307)

Synthesis of 1-((5aS,6R,11bR)-9-chloro-14-(cyclopropylmethyl)-5a-hydroxy-10-methoxy-1,2,5,5a,6,7-hexahydro-6,11b-(epiminoethano)naphtho[1,2-d]azepin-3(4H)-yl)-2-(pyridin-2-yl)ethan-1-one

[Chemical Formula 377]

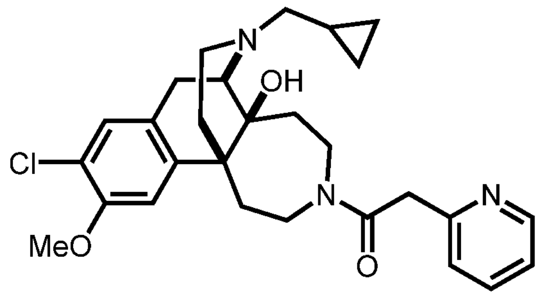

The title compound was obtained from the compound obtained in Example 306 and 2-(pyridin-2-yl)acetic acid hydrochloride according to the method described in Example 5.

$^1$H-NMR (400 MHz, $CDCl_3$)δ (ppm): 0.06 - 0.13 (m, 2H), 0.47 - 0.55 (m, 2H), 0.75 - 0.86 (m, 1H), 0.96 - 1.09 (m, 1H), 1.51 - 1.59 (m, 0.5H), 1.61 - 1.80 (m, 1.5H), 1.87 - 2.12 (m, 3H), 2.25 - 2.46 (m, 2.5H), 2.48 - 2.58 (m, 1.5H), 2.62 (dd, J = 6, 18 Hz, 0.5H), 2.77 (dd, J = 6, 18 Hz, 0.5H), 2.84 - 2.95 (m, 2H), 3.44 - 3.67 (m, 2H), 3.74 - 3.96 (m, 3H), 3.81 (s, 1.5H), 3.88 (s, 1.5H), 4.40 - 4.51 (m, 1H), 6.53 (s, 0.5H), 6.67 (s, 0.5H), 7.03 (s, 0.5H), 7.08 (s, 0.5H), 7.09 - 7.16 (m, 2H), 7.52 (ddd, J = 2, 8, 8 Hz, 0.5H), 7.57 (ddd, J = 2, 8, 8 Hz, 0.5H), 8.40 - 8.50 (m, 1H).

(Example 308)

Synthesis of 1-((5aS,6R,11bR)-9-chloro-14-(cyclopropylmethyl)-5a,10-dihydroxy-1,2,5,5a,6,7-hexahydro-6,11b-(epiminoethano)naphtho[1,2-d]azepin-3(4H)-yl)-2-(pyridin-2-yl)ethan-1-one

[Chemical Formula 378]

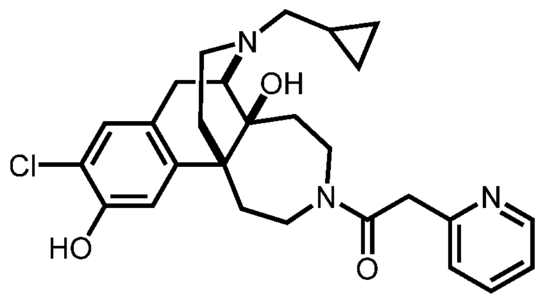

To the compound (18.7 mg, 0.038 mmol) obtained in Example 307 dissolved in chloroform (2 mL), a 1 M boron tribromide-dichloromethane solution (0.57 mL, 0.57 mmol) was added, followed by stirring at room temperature for 6 hours. To the reaction solution, a 28% aqueous ammonia solution and a saturated sodium bicarbonate aqueous solution were added, followed by extraction three times with chloroform. The organic layer was dried over sodium sulfate and concentrated under reduced pressure. The obtained crude product was purified by preparative thin layer chromatography (chloroform : 10% aqueous ammonia-methanol solution = 15 : 1) to yield the title compound (15.8 mg, 87%) as a colorless amorphous form.

$^1$H-NMR (400 MHz, $CDCl_3$)δ (ppm): 0.04 - 0.12 (m, 2H), 0.45 - 0.54 (m, 2H), 0.72 - 0.84 (m, 1H), 0.87 - 0.96 (m, 1H), 1.42 - 1.62 (m, 1.5H), 1.70 - 2.04 (m, 3.5H), 2.23 - 2.38 (m, 2.5H), 2.40 - 2.54 (m, 2H), 2.67 - 2.91 (m, 2.5H), 3.27 - 3.57 (m, 2H), 3.61 - 3.95 (m, 4H), 6.75 (s, 0.5H), 6.76 (s, 0.5H), 6.86 (s, 0.5H), 6.96 - 7.03 (m, 1H), 7.06 - 7.18 (m, 1.5H), 7.51 - 7.60 (m, 1H), 8.39 - 8.47 (m, 1H).

(Example 309)

Synthesis of 1-((5aS,6R,11bR)-9,11-dibromo-14-(cyclopropylmethyl)-5a,10-dihydroxy-1,2,5,5a,6,7-hexahydro-6,11b-(epiminoethano)naphtho[1,2-d]azepin-3(4H)-yl)-2,2,2-trifluoroethan-1-one

[Chemical Formula 379]

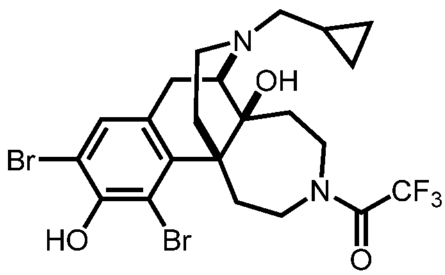

To a solution of the compound E (65.7 mg, 0.2 mmol) obtained in Example 3 in chloroform (2 mL) were added trifluoroacetic anhydride (141 pL, 1.0 mmol) and triethylamine (139 pL, 1.0 mmol), followed by stirring at room temperature for 1 hour. To the reaction mixture, methanol (2 mL) was added under ice cooling, followed by stirring at room temperature for 1 hour. To the reaction mixture, a saturated sodium bicarbonate aqueous solution was added, followed by extraction three times with chloroform. The combined extracts were dried over sodium sulfate and then concentrated under reduced pressure. The obtained crude product was used for the next reaction without purification.

To a solution of the obtained crude product in methanol (1 mL) were added 10% hydrobromic acid (1 mL) and iodobenzene diacetate (257 mg, 0.80 mmol), followed by stirring at room temperature for 18 hours. To the reaction mixture, a saturated aqueous sodium thiosulfate solution was added, followed by stirring at room temperature for 30 minutes, and then a saturated aqueous sodium bicarbonate solution was added, followed by extraction three times with chloroform. The organic layers were combined, dried over sodium sulfate, and then concentrated under reduced pressure. The obtained crude product was purified by silica gel column chromatography (amino group-supported silica gel, 0 to 10% methanol/chloroform) to yield the title compound (66.1 mg, 53%) as a colorless amorphous form.

$^1$H-NMR (400 MHz, $CDCl_3$)δ (ppm): 0.06 - 0.14 (m, 2H), 0.49 - 0.57 (m, 2H), 0.76 - 0.90 (m, 1H), 1.62 - 2.06 (m, 5H), 2.24 - 2.40 (m, 2H), 2.58 - 3.10 (m, 6H), 3.31 - 4.04 (m, 5H), 7.23 (s, 1H).

(Example 310)

Synthesis of (5aS,6R, 11bS)-9,11-dibromo-14-(cyclopropylmethyl)-10-methoxy-2,3,4,5,6,7-hexahydro-6,11b-(epiminoethano)naphtho[1,2-d]azepin-5a(1H)-ol

[Chemical Formula 380]

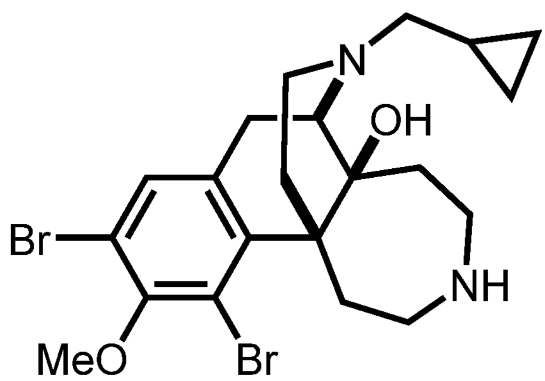

To a solution of the compound (58.2 mg, 0.10 mmol) obtained in Example 309 in N,N-dimethylformamide (1 mL) were added methyl iodide (7.5 μL, 0.12 mmol) and potassium carbonate (69.1 mg, 0.50 mmol), followed by stirring at room temperature for 1 hour. To the reaction mixture, a saturated sodium bicarbonate aqueous solution was added, followed by extraction three times with chloroform. The combined extracts were dried over sodium sulfate and then concentrated under reduced pressure. The obtained crude product was used for the next reaction without purification.

To a solution of the obtained crude product in ethanol (2 mL) was added potassium carbonate (69.1 mg, 0.50 mmol), followed by stirring at 80°C for 3 hours. After allowed to cool, water was added to the reaction mixture, followed by extraction three times with chloroform. The combined extracts were dried over sodium sulfate and then concentrated under reduced pressure. The obtained crude product was purified by silica gel column chromatography (amino group-supported silica gel, 0 to 50% methanol/chloroform) to yield the title compound (44.2 mg, 88%) as a colorless amorphous form.

$^1$H-NMR (400 MHz, $CDCl_3$)δ (ppm): 0.06 - 0.14 (m, 2H), 0.48 - 0.56 (m, 2H), 0.76 - 0.90 (m, 1H), 1.55 - 1.73 (m, 2H), 1.84 - 2.04 (m, 3H), 2.25 - 2.38 (m, 2H), 2.52 - 2.80 (m, 4H), 2.82 - 3.13 (m, 6H), 3.85 (s, 3H), 4.47 (br s, 1H), 7.29 (s, 1H).

(Example 311)

Synthesis of (5aS,6R,11bS)-9,11-dibromo-14-(cyclopropylmethyl)-10-methoxy-3-(2-(4-methyl-1H-pyrazol-1-yl)ethyl)-2,3,4,5,6,7-hexahydro-6,11b-(epiminoethano)naphtho[1,2-d]azepin-5a(1H)-ol

[Chemical Formula 381]

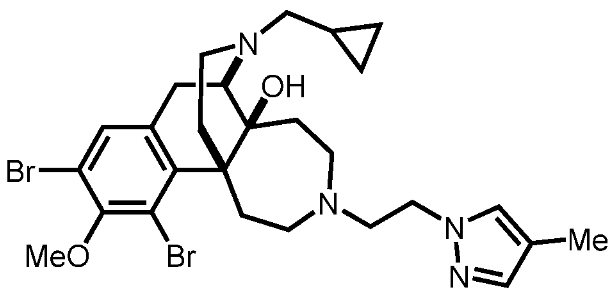

The title compound was obtained from the compound obtained in Example 310 and the compound obtained in Reference Example 33 according to the method described in Example 31.

$^1$H-NMR (400 MHz, $CDCl_3$)δ (ppm): 0.05 - 0.15 (m, 2H), 0.46 - 0.56 (m, 2H), 0.75 - 0.87 (m, 1H), 1.46 (dd, J = 6, 14 Hz, 1H), 1.63 - 2.05 (m, 4H), 2.02 (s, 3H), 2.29 (dd, J = 7, 13 Hz, 1H), 2.31 (dd, J = 7, 13 Hz, 1H), 2.33 - 3.03 (m, 11H), 3.08 - 3.20 (m, 1H), 3.76 - 4.06 (m, 2H), 3.80 (s, 3H), 6.77 (s, 1H), 7.21 (s, 1H), 7.30 (s, 1H).

(Example 312)

Synthesis of (5aS,6R, 11bS)-9, 11-dibromo-14-(cyclopropylmethyl)-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 382]

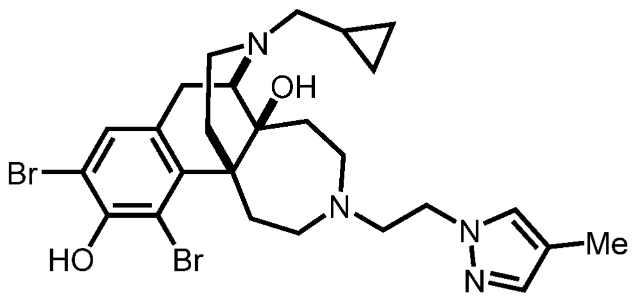

The title compound was obtained from the compound obtained in Example 311 according to the method described in Example 6.

$^1$H-NMR (400 MHz, $CDCl_3$)δ (ppm): 0.05 - 0.12 (m, 2H), 0.47 - 0.54 (m, 2H), 0.74 - 0.85 (m, 1H), 1.51 (dd, J = 6, 15 Hz, 1H), 1.73 - 1.99 (m, 4H), 2.03 (s, 3H), 2.23 - 2.46 (m, 3H), 2.55 - 3.03 (m, 10H), 3.15 - 3.25 (m, 1H), 4.06 - 4.26 (m, 2H), 6.98 (s, 1H), 7.21 (s, 1H), 7.25 (s, 1H).

(Example 313)

Synthesis of (5aS,6R,11bS)-11-bromo-14-(cyclopropylmethyl)-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 383]

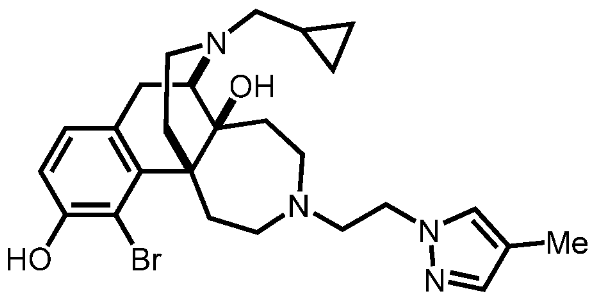

To a solution of the compound (15 mg, 0.030 mmol) obtained in Example 310 in methanol (1 mL) were added potassium formate (126 mg, 1.5 mmol) and 10% palladium-activated carbon (1.5 mg), followed by stirring at room temperature for 1 hour. Then, 10% palladium-activated carbon (3.0 mg) was added, followed by stirring at room temperature for 1 hour. The reaction mixture was filtered through celite, and to the filtrate, a 28% aqueous ammonia solution was added, followed by extraction three times with chloroform. The organic layers were combined, dried over sodium sulfate, and then concentrated under reduced pressure. Although the obtained crude product was purified by preparative thin layer chromatography (chloroform : 2 M ammonia-methanol solution = 10 : 1), it was so difficult to separate the crude product from the by-product that the crude product was used for the next reaction without further purification.

To a solution of the obtained crude product in acetonitrile (1 mL) were added the compound (12.1 mg, 0.060 mmol) obtained in Reference Example 33 and N,N-diisopropylethylamine (15.7 μL, 0.090 mmol), followed by stirring at 60°C for 18 hours. After allowed to cool, to the reaction mixture was added a saturated sodium bicarbonate aqueous solution, followed by extraction three times with chloroform. The combined extracts were dried over sodium sulfate and then concentrated under reduced pressure. The obtained crude product was used for the next reaction without purification.

To a solution of the obtained crude product in chloroform (2 mL) was added a 1 M boron tribromide-dichloromethane solution (0.50 mL, 0.50 mmol) under ice cooling, followed by stirring at room temperature for 30 minutes. To the reaction mixture, a 28% aqueous ammonia solution was added under ice cooling, followed by extraction three times with chloroform. The combined extracts were dried over sodium sulfate and then concentrated under reduced pressure. The obtained crude product was purified by preparative thin layer chromatography (chloroform : 2 M ammonia-methanol solution = 10 : 1) to yield the title compound (2.3 mg, 15%) as a colorless amorphous form.

$^1$H-NMR (400 MHz, $CDCl_3$)δ (ppm): 0.04 - 0.14 (m, 2H), 0.46 - 0.55 (m, 2H), 0.76 - 0.90 (m, 1H), 1.40 - 2.04 (m, 5H), 2.00 (s, 3H), 2.25 - 2.40 (m, 3H), 2.49 - 3.00 (m, 10H), 3.14 (dd, J = 11, 14 Hz, 1H), 3.82 - 3.99 (m, 2H), 4.55

(s, 1H), 6.76 (s, 1H), 6.87 (d, J = 9 Hz, 1H), 6.98 (d, J = 8 Hz, 1H), 7.19 (s, 1H).

(Example 314)

Synthesis of 1-((5aS,6R,11bR)-14-(cyclopropylmethyl)-5a,10-dihydroxy-9-methyl-1,2,5,5a,6,7-hexahydro-6,11b-(epiminoethano)naphtho[1,2-d]azepin-3(4H)-yl)-2-(imidazo[1,2-a]pyridin-2-yl)ethan-1-one

[Chemical Formula 384]

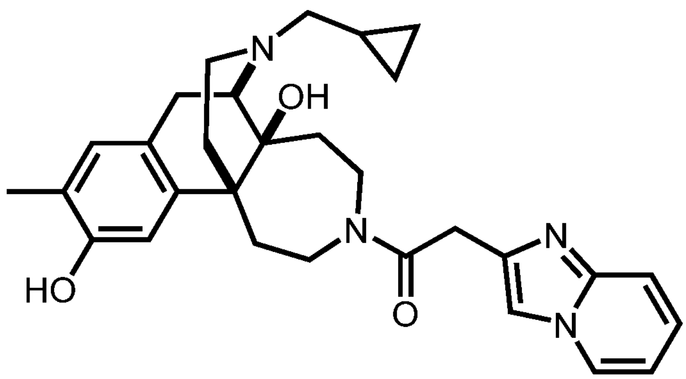

The title compound was obtained from the compound obtained in Example 290 and 2-(imidazo[1,2-a]pyridin-2-yl)acetic acid according to the method described in Example 26.

$^1$H-NMR (400 MHz, $CDCl_3$)δ (ppm): 0.02 - 0.15 (m, 2H), 0.41 - 0.56 (m, 2H), 0.74 - 0.91 (m, 1H), 0.95 - 1.08 (m, 1H), 1.50 - 1.61 (m, 1H), 1.75 - 2.24 (m, 5H), 2.11 (s, 3H), 2.25 - 2.39 (m, 2H), 2.46 - 2.62 (m, 1H), 2.61 (dd, J = 6, 18 Hz, 0.5H), 2.66 (dd, J = 6, 18 Hz, 0.5H), 2.82 (d, J = 6 Hz, 0.5H), 2.88 (d, J = 18 Hz, 0.5H), 2.89 (d, J = 6 Hz, 0.5H), 2.91 (d, J = 18 Hz, 0.5H), 3.10 - 3.30 (m, 1.5H), 3.45 - 3.55 (m, 0.5H), 3.68 (d, J = 16 Hz, 0.5H), 3.71 (d, J = 16 Hz, 0.5H), 3.87 (d, J = 16 Hz, 0.5H), 3.89 - 4.03 (m, 1.5H), 3.93 (d, J = 16 Hz, 0.5H), 4.06 - 4.16 (m, 0.5H), 4.51 (br s, 1H), 6.70 - 6.80 (m, 3H), 7.07 - 7.18 (m, 1H), 7.42 - 7.54 (m, 1.5H), 7.45 (s, 0.5H), 7.98 - 8.04 (m, 1H).

(Example 315)

Synthesis of 1-((5aS,6R,11bR)-14-(cyclopropylmethyl)-5a,10-dihydroxy-9-methyl-1,2,5,5a,6,7-hexahydro-6,11b-(epiminoethano)naphtho[1,2-d]azepin-3(4H)-yl)-2-((R)-2-methylpyrrolidin-1-yl)ethan-1-one

[Chemical Formula 385]

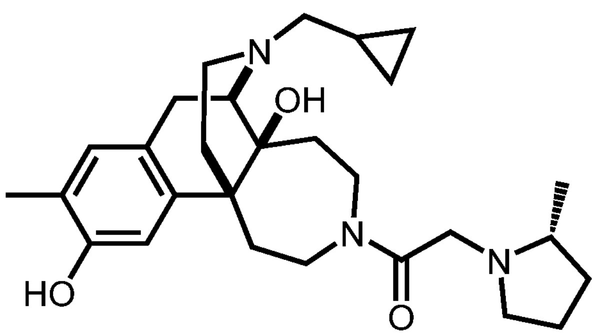

The title compound was obtained from the compound obtained in Example 290 and (R)-2-methylpyrrolidine hydrochloride according to the method described in Example 132.

$^1$H-NMR (400 MHz, $CDCl_3$)δ (ppm): 0.05 - 0.17 (m, 2H), 0.45 - 0.56 (m, 2H), 0.76 - 0.90 (m, 1H), 0.99 - 1.13 (m, 1.6H), 1.05 (d, J = 6 Hz, 2.4H), 1.22 - 1.44 (m, 1H), 1.50 - 2.20 (m, 9H), 2.17 (s, 3H), 2.21 - 2.40 (m, 4H), 2.42 - 2.65

(m, 2H), 2.72 (dd, J = 6, 18 Hz, 0.8H), 2.75 - 2.83 (m, 0.2H), 2.79 (d, J = 13 Hz, 0.8H), 2.85 - 2.93 (m, 0.4H), 2.87 (d, J = 6 Hz, 0.8H), 2.96 (d, J = 18 Hz, 0.8H), 2.97 - 3.06 (m, 0.2H), 3.36 - 3.52 (m, 1.2H), 3.53 - 3.65 (m, 0.2H), 3.61 (ddd, J = 4, 4, 14 Hz, 0.8H), 3.66 - 3.84 (m, 1.2H), 3.69 (d, J = 13 Hz, 0.8H), 3.93 - 4.04 (m, 0.8H), 4.40 - 4.67 (m, 1H), 6.61 (s, 0.2H), 6.79 (s, 0.8H), 6.81 (s, 0.2H), 6.85 (s, 0.8H).

(Example 316)

Synthesis of tert-butyl (5aS,6R,11bR)-5a-acetoxy-10-(benzyloxy)-14-(cyclopropylmethyl)-9-iodo-1,2,5,5a,6,7-hexahydro-6,11b-(epiminoethano)naphtho[1,2-d]azepine-3(4H)-carboxylate

[Chemical Formula 386]

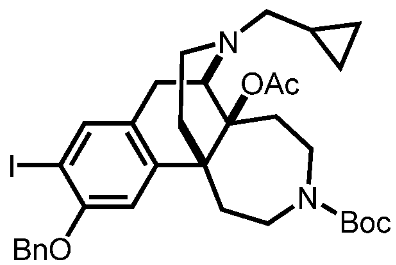

The compound (106 mg, 0.16 mmol) obtained in Example 292 was dissolved in acetic anhydride (2.5 mL), followed by heating under reflux for 1.5 hours. The reaction mixture was concentrated under reduced pressure. To the obtained concentrated residue was added a saturated aqueous sodium bicarbonate solution, followed by extraction three times with ethyl acetate. The organic layer was dried over sodium sulfate and concentrated under reduced pressure. The obtained crude product was purified by silica gel column chromatography (30 to 100% ethyl acetate/heptane) to yield the title compound (79.4 mg, 70%) as a colorless amorphous form.

$^1$H-NMR (400 MHz, $CDCl_3$)$\delta$ (ppm): 0.01 - 0.09 (m, 2H), 0.39 - 0.51 (m, 2H), 0.68 - 0.79 (m, 1H), 0.93 - 1.03 (m, 1H), 1.41 (s, 3.6H), 1.44 (s, 5.4H), 1.80 - 1.90 (m, 1H), 1.92 - 2.07 (m, 2H), 2.08 - 2.18 (m, 2H), 2.10 (s, 1H), 2.12 (s, 2H), 2.19 - 2.45 (m, 3H), 2.48 - 2.56 (m, 1H), 2.60 - 2.78 (m, 2H), 2.92 - 3.16 (m, 2H), 3.42 (ddd, J = 4, 13, 13 Hz, 0.4H), 3.50 (ddd, J = 2, 5, 15 Hz, 0.6H), 3.60 (ddd, J = 4, 13, 13 Hz, 0.6H), 3.74 (ddd, J = 2, 5, 15 Hz, 0.4H), 4.18 (d, J = 6 Hz, 0.4H), 4.26 (d, J = 6 Hz, 0.6H), 5.00 - 5.20 (m, 2H), 6.58 (s, 0.4H), 6.59 (s, 0.6H), 7.28 - 7.35 (m, 1H), 7.36 - 7.43 (m, 2H), 7.44 - 7.51 (m, 2H), 7.54 (s, 0.4H), 7.55 (s, 0.6H).

(Example 317)

Synthesis of tert-butyl (5aS,6R,11bR)-5a-acetoxy-10-(benzyloxy)-14-(cyclopropylmethyl)-9-(trifluoromethyl)-1,2,5,5a,6,7-hexahydro-6,11b-(epiminoethano)naphtho[1,2-d]azepine-3(4H)-carboxylate

[Chemical Formula 387]

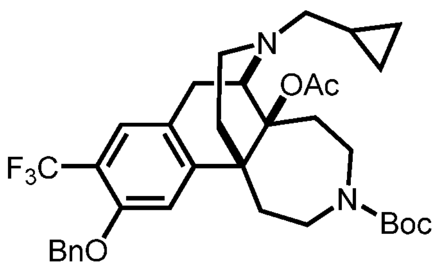

To the compound (78 mg, 0.11 mmol) obtained in Example 316 dissolved in N,N-dimethylformamide (2.5 mL) were added copper (I) iodide (38.2 mg, 0.20 mmol) and methyl 2,2-difluoro 2-(fluorosulfonyl)acetate (73 μL, 0.58 mmol), followed by stirring at 80°C for 1 hour. To the reaction solution, a saturated aqueous sodium bicarbonate solution was added, followed by extraction with ethyl acetate. The organic layer was dried over sodium sulfate and concentrated

under reduced pressure. The obtained crude product was purified by silica gel column chromatography (26 to 47% ethyl acetate/heptane) to yield the title compound (51.0 mg, 71%) as a white amorphous form.

$^{1}$H-NMR (400 MHz, $CDCl_3$)δ (ppm): 0.01 - 0.10 (m, 2H), 0.41 - 0.51 (m, 2H), 0.68 - 0.80 (m, 1H), 0.94 - 1.04 (m, 1H), 1.39 (s, 3.6H), 1.43 (s, 5.4H), 1.75 - 1.86 (m, 1H), 1.95 (ddd, J = 3, 12, 12 Hz, 1H), 1.98 - 2.09 (m, 1H), 2.11 (s, 1.2H), 2.13 (s, 1.8H), 2.14 - 2.34 (m, 4H), 2.35 - 2.49 (m, 1H), 2.50 - 2.58 (m, 1H), 2.64 - 2.84 (m, 2H), 2.97 - 3.18 (m, 2H), 3.40 (ddd, J = 4, 13, 13 Hz, 0.4H), 3.49 (ddd, J = 3, 5, 15 Hz, 0.6H), 3.58 (ddd, J = 4, 13, 13 Hz, 0.6H), 3.69 (ddd, J = 3, 5, 15 Hz, 0.4H), 4.23 (d, J = 6 Hz, 0.6H), 4.30 (d, J = 6 Hz, 0.4H), 5.08 - 5.24 (m, 2H), 6.76 (s, 1H), 7.28 - 7.50 (m, 6H).

(Example 318)

Synthesis of (5aS,6R, 11bS)-10-(benzyloxy)-14-(cyclopropylmethyl)-9-(trifluoromethyl)-2,3,4,5,6,7-hexahydro-6,11b-(epiminoethano)naphtho[1,2-d]azepin-5a(1H)-ol

[Chemical Formula 388]

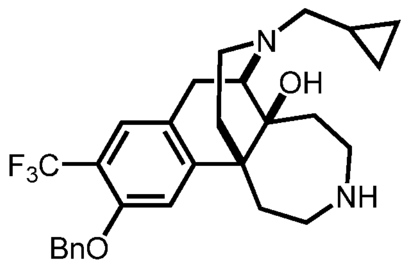

To the compound (22.3 mg, 0.036 mmol) obtained in Example 317 dissolved in ethanol (3 mL), a 6 M aqueous sodium hydroxide solution (2 mL) was added, followed by stirring at 80°C for 1.5 hours. To the reaction solution, 2M hydrochloric acid and a saturated aqueous sodium bicarbonate solution were added, followed by extraction with ethyl acetate. The organic layer was dried over sodium sulfate and concentrated under reduced pressure. To the obtained concentrated residue dissolved in chloroform (1.5 mL), trifluoroacetic acid (0.5 mL) was added, followed by stirring at room temperature for 2.5 hours. To the reaction solution, a saturated aqueous sodium bicarbonate solution was added, followed by extraction with a mixed solvent of chloroform/isopropanol (4/1). The organic layer was dried over sodium sulfate and concentrated under reduced pressure. The obtained crude product was purified by preparative thin layer chromatography (chloroform : 10% aqueous ammonia-methanol solution = 8 : 1) to yield the title compound (12.5 mg, 72%) as a colorless amorphous form.

$^{1}$H-NMR (400 MHz, $CDCl_3$)δ (ppm): 0.06 - 0.14 (m, 2H), 0.47 - 0.56 (m, 2H), 0.76 - 0.88 (m, 1H), 0.95 - 1.03 (m, 1H), 1.51 - 1.68 (m, 2H), 1.84 (ddd, J = 3, 3, 16 Hz, 1H), 1.89 - 2.09 (m, 2H), 2.26 - 2.39 (m, 3H), 2.44 - 2.58 (m, 2H), 2.74 (ddd, J = 4, 4, 14 Hz, 1H), 2.78 - 2.86 (m, 2H), 2.87 - 3.10 (m, 3H), 4.47 (br s, 1H), 5.12 (d, J = 13 Hz, 1H), 5.21 (d, J = 13 Hz, 1H), 6.75 (s, 1H), 7.28 - 7.48 (m, 6H).

(Example 319)

Synthesis of (5aS,6R, 11bS)-14-(cyclopropylmethyl)-9-(trifluoromethyl)-2,3,4,5,6,7-hexahydro-6,11b-(epiminoethano)naphtho[1,2-d]azepine-5a,10(1H)-diol

[Chemical Formula 389]

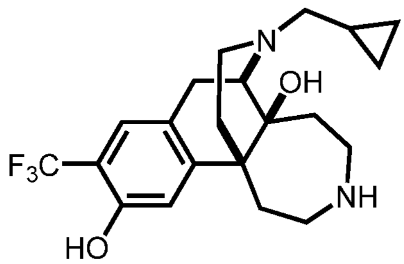

To the compound (21.4 mg, 0.044 mmol) obtained in Example 318 dissolved in methanol (2.5 mL), 20% palladium hydroxide-activated carbon (50% wet, 21.4 mg) was added, followed by stirring under a hydrogen atmosphere at room temperature for 6 hours. The reaction mixture was filtered through a membrane filter, and the filtrate was concentrated under reduced pressure to yield the title compound (13.4 mg, 77%) as a colorless amorphous form.

$^1H$-NMR (400 MHz, $CDCl_3$)δ (ppm): 0.05 - 0.14 (m, 2H), 0.46 - 0.56 (m, 2H), 0.75 - 0.87 (m, 1H), 0.98 - 1.07 (m, 1H), 1.47 - 1.57 (m, 1H), 1.69 - 1.81 (m, 1H), 1.88 - 2.07 (m, 2H), 2.10 - 2.18 (m, 2H), 2.34 (d, J = 6 Hz, 2H), 2.48 - 2.57 (m, 1H), 2.62 - 2.76 (m, 2H), 2.79 - 3.02 (m, 3H), 3.08 - 3.20 (m, 1H), 3.23 - 3.36 (m, 1H), 6.59 (s, 1H), 7.18 (s, 1H).

(Example 320)

Synthesis of 1-((5aS,6R,11bR)-14-(cyclopropylmethyl)-5a,10-dihydroxy-9-(trifluoromethyl)-1,2,5,5a,6,7-hexahydro-6,11b-(epiminoethano)naphtho[1,2-d]azepin-3(4H)-yl)-2-(pyridin-2-yl)ethan-1-one

[Chemical Formula 390]

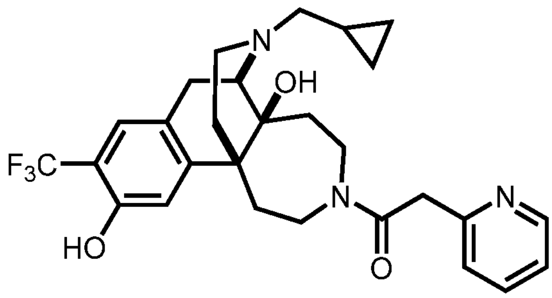

The title compound was obtained from the compound obtained in Example 319 and 2-(pyridin-2-yl)acetic acid hydrochloride according to the method described in Example 26.

$^1H$-NMR (400 MHz, $CDCl_3$)δ (ppm): 0.06 - 0.13 (m, 2H), 0.47 - 0.54 (m, 2H), 0.75 - 0.86 (m, 1H), 0.96 - 1.09 (m, 1H), 1.49 - 1.85 (m, 2H), 1.88 - 2.15 (m, 3H), 2.25 - 2.39 (m, 2H), 2.47 - 2.67 (m, 1.7H), 2.76 (dd, J = 6, 18 Hz, 0.3H), 2.83 - 2.99 (m, 2.3H), 3.10 - 3.21 (m, 0.7H), 3.46 - 3.72 (m, 2.7H), 3.77 - 4.04 (m, 3.3H), 6.88 (s, 0.3H), 6.98 (s, 0.7H), 7.07 - 7.18 (m, 2.7H), 7.24 - 7.28 (m, 0.3H), 7.57 (ddd, J = 2, 8, 8 Hz, 0.7H), 7.62 (ddd, J = 2, 8, 8 Hz, 0.3H), 8.36 - 8.40 (m, 0.3H), 8.42 - 8.46 (m, 0.7H).

(Example 321)

Synthesis of (5aS,6R, 11bS)-14-(cyclopropylmethyl)-3-(2-(4-methyl-1H-pyrazol-1-yl)ethyl)-9-(trifluoromethyl)-2,3,4,5,6,7-hexahydro-6,11b-(epiminoethano)naphtho[1,2-d]azepine-5a,10(1H)-diol

[Chemical Formula 391]

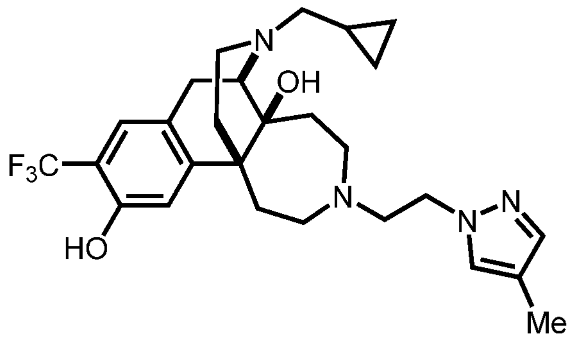

The title compound was obtained from the compound obtained in Example 319 and the compound obtained in Reference Example 33 according to the method described in Example 31.

$^1$H-NMR (400 MHz, $CDCl_3$)δ (ppm): 0.06 - 0.13 (m, 2H), 0.47 - 0.54 (m, 2H), 0.75 - 0.86 (m, 1H), 0.95 - 1.03 (m, 1H), 1.42 - 1.51 (m, 1H), 1.61 - 1.79 (m, 2H), 1.90 - 2.04 (m, 2H), 1.97 (s, 3H), 2.27 - 2.39 (m, 3H), 2.47 - 2.57 (m, 2H), 2.62 (ddd, J = 2, 5, 13 Hz, 1H), 2.69 - 2.82 (m, 2H), 2.86 - 3.03 (m, 4H), 3.05 - 3.15 (m, 1H), 4.03 - 4.11 (m, 2H), 6.42 (s, 1H), 7.03 (s, 1H), 7.15 (s, 1H), 7.24 (s, 1H).

(Example 322)

Synthesis of 1-((5aS,6R,11bR)-14-(cyclopropylmethyl)-5a,10-dihydroxy-9-methyl-1,2,5,5a,6,7-hexahydro-6,11b-(epiminoethano)naphtho[1,2-d]azepin-3(4H)-yl)-2-(6-(trifluoromethyl)pyridin-2-yl)ethan-1-one

[Chemical Formula 392]

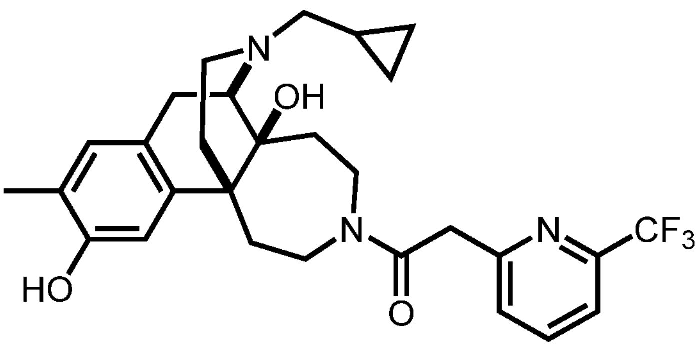

The title compound was obtained from the compound obtained in Example 290 and 2-(6-(trifluoromethyl)pyridin-2-yl)acetic acid according to the method described in Example 26.

$^1$H-NMR (400 MHz, $CDCl_3$)δ (ppm): 0.05 - 0.16 (m, 2H), 0.43 - 0.56 (m, 2H), 0.76 - 0.87 (m, 1H), 0.99 - 1.12 (m, 1H), 1.53 - 1.63 (m, 0.7H), 1.79 - 2.09 (m, 4H), 2.12 (s, 2.1H), 2.16 - 2.23 (m, 1.4H), 2.18 (s, 0.9H), 2.26 - 2.39 (m, 2H), 2.46 (ddd, J = 5, 12, 16 Hz, 0.3H), 2.48 - 2.56 (m, 1H), 2.66 (dd, J = 6, 18 Hz, 0.7H), 2.75 (dd, J = 6, 18 Hz, 0.3H), 2.84 (d, J = 6 Hz, 0.7H), 2.90 (d, J = 6 Hz, 0.3H), 2.91 (d, J = 18 Hz, 0.3H), 2.94 (d, J = 18 Hz, 0.7H), 3.12 - 3.21 (m, 0.7H), 3.38 - 3.54 (m, 1.3H), 3.67 - 3.77 (m, 0.3H), 3.73 (d, J = 15 Hz, 0.3H), 3.82 (ddd, J = 3, 4, 14 Hz, 0.3H), 3.87 (d, J = 15 Hz, 0.3H), 3.90 - 4.01 (m, 0.3H), 3.96 (d, J = 15 Hz, 0.7H), 4.02 (d, J = 15 Hz, 0.7H), 4.15 (ddd, J = 4, 4, 14 Hz, 0.7H), 4.41 (br s, 0.7H), 4.51 (br s, 0.3H), 6.58 (s, 0.3H), 6.77 (s, 1.4H), 6.83 (s, 0.3H), 7.38 (d, J = 8 Hz, 0.3H), 7.46 (d, J = 8 Hz, 0.7H), 7.52 - 7.56 (m, 0.3H), 7.54 (d, J = 8 Hz, 0.7H), 7.75 (dd, J = 8, 8 Hz, 0.3H), 7.77 (dd, J = 8, 8 Hz, 0.7H).

(Example 323)

Synthesis of 1-((5aS,6R,11bR)-14-(cyclopropylmethyl)-5a,10-dihydroxy-9-methyl-1,2,5,5a,6,7-hexahydro-6,11b-(epiminoethano)naphtho[1,2-d]azepin-3(4H)-yl)-2-(5-methylisothiazol-3-yl)ethan-1-one

[Chemical Formula 393]

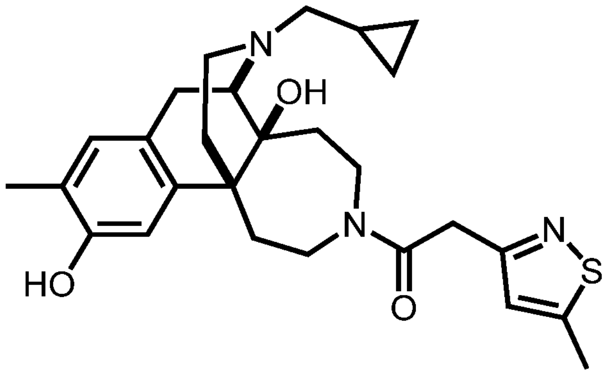

The title compound was obtained from the compound obtained in Example 290 and 2-(5-methylisothiazol-3-yl)acetic acid according to the method described in Example 26.

$^1$H-NMR (400 MHz, $CDCl_3$)δ (ppm): 0.04 - 0.17 (m, 2H), 0.45 - 0.55 (m, 2H), 0.76 - 0.88 (m, 1H), 1.00 - 1.12 (m, 1H), 1.51 - 1.61 (m, 1H), 1.76 - 2.10 (m, 3.4H), 2.14 - 2.23 (m, 1H), 2.18 (s, 3H), 2.28 (dd, J = 6, 12 Hz, 0.6H), 2.32 - 2.39 (m, 0.8H), 2.36 (dd, J = 6, 12 Hz, 0.6H), 2.45 - 2.59 (m, 1.6H), 2.51 (s, 1.2H), 2.52 (s, 1.8H), 2.67 (dd, J = 6, 18 Hz, 0.6H), 2.73 (dd, J = 6, 18 Hz, 0.4H), 2.84 (d, J = 6 Hz, 0.6H), 2.89 (d, J = 6 Hz, 0.4H), 2.90 (d, J = 18 Hz, 0.4H), 2.95 (d, J = 18 Hz, 0.6H), 3.19 (ddd, J = 4, 10, 14 Hz, 0.6H), 3.30 - 3.42 (m, 0.8H), 3.42 (ddd, J = 4, 4, 13 Hz, 0.6H), 3.63 (d, J = 15 Hz, 0.4H), 3.69 (d, J = 15 Hz, 0.4H), 3.73 - 3.97 (m, 1.4H), 3.83 (d, J = 16 Hz, 0.6H), 3.91 (d, J = 16 Hz, 0.6H), 4.11 - 4.19 (m, 0.6H), 4.44 (br s, 0.6H), 4.52 (br s, 0.4H), 6.64 (s, 0.4H), 6.79 (s, 0.6H), 6.81 (s, 1H), 6.83 (s, 0.4H), 6.87 (s, 0.6H).

(Example 324)

Synthesis of 1-((5aS,6R,11bR)-10-(benzyloxy)-14-(cyclopropylmethyl)-5a-hydroxy-1,2,5,5a,6,7-hexahydro-6,11b-(epiminoethano)naphtho[1,2-d]azepin-3(4H)-yl)-2,2,2-trifluoroethan-1-one

[Chemical Formula 394]

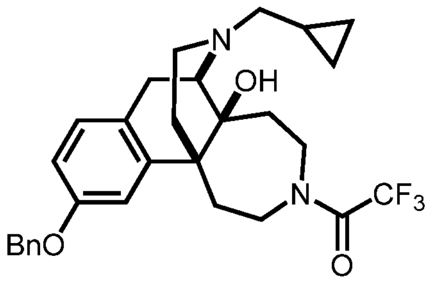

To the compound Q (99.8 mg, 0.24 mmol) obtained in Example 287 dissolved in chloroform (4 mL) were added N,N-diisopropylethylamine (250 μL, 1.44 mmol) and trifluoroacetic anhydride (152 μL, 1.08 mmol), followed by stirring at room temperature for 1 hour. To the reaction solution, a saturated sodium bicarbonate aqueous solution was added, followed by extraction three times with chloroform. The organic layer was dried over sodium sulfate and concentrated under reduced pressure. The obtained crude product was purified by silica gel column chromatography (5 to 20%

methanol/ethyl acetate) to yield the title compound (100.1 mg, 81%) as a pale yellow amorphous form.

$^1$H-NMR (400 MHz, $CDCl_3$)δ (ppm): 0.05 - 0.15 (m, 2H), 0.47 - 0.56 (m, 2H), 0.76 - 0.86 (m, 1H), 1.01 - 1.13 (m, 1H), 1.66 (ddd, J = 4, 4, 15 Hz, 1H), 1.82 - 1.93 (m, 1H), 1.96 - 2.15 (m, 3H), 2.27 - 2.41 (m, 3H), 2.52 - 2.62 (m, 1H), 2.74 - 2.84 (m, 1H), 2.88 - 2.94 (m, 1H), 2.97 - 3.05 (m, 1H), 3.13 - 3.23 (m, 0.5H), 3.28 - 3.38 (m, 0.5H), 3.46 - 3.56 (m, 1H), 3.59 - 3.67 (m, 0.5H), 3.78 - 4.06 (m, 1.5H), 4.98 - 5.08 (m, 2H), 6.66 (d, J = 2 Hz, 0.5H), 6.72 (d, J = 2 Hz, 0.5H), 6.77 - 6.83 (m, 1H), 7.03 (d, J = 8 Hz, 1H), 7.30 - 7.45 (m, 5H).

(Example 325)

Synthesis of (5aS,6R,11bS)-9-chloro-14-(cyclopropylmethyl)-2,3,4,5,6,7-hexahydro-6,11b-(epiminoethano)naphtho[1,2-d]azepine-5a,10(1H)-diol (compound U), (5aS,6R,11bS)-11-chloro-14-(cyclopropylmethyl)-2,3,4,5,6,7-hexahydro-6,11b-(epiminoethano)naphtho[1,2-d]azepine-5a,10(1H)-diol (compound V) and (5aS,6R, 11bS)-9,11-dichloro-14-(cyclopropylmethyl)-2,3,4,5,6,7-hexahydro-6,11b-(epiminoethano)naphtho[1,2-d]azepine-5a, 10(1H)-diol (compound UV)

[Chemical Formula 395]

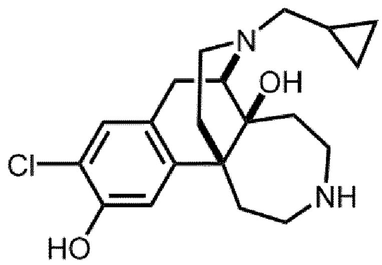

Compound U

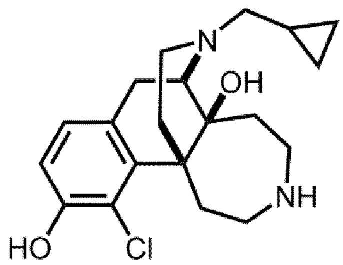

Compound V

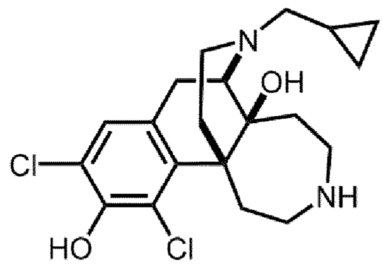

Compound UV

To the compound (100 mg, 0.19 mmol) obtained in Example 324 dissolved in a 2 M trifluoroacetic acid aqueous solution (4 mL), N-chlorosuccinimide (200 mg, 1.50 mmol) was added, followed by stirring at room temperature for 18.5 hours. To the reaction solution, a saturated sodium bicarbonate aqueous solution was added, followed by extraction three times with chloroform. To the obtained concentrated residue dissolved in ethanol (6 mL), potassium carbonate (300 mg, 2.17 mmol) was added, followed by stirring at 80°C for 4 hours. The reaction mixture was filtered through a membrane filter, and the filtrate was concentrated under reduced pressure. To the obtained residue, 6 M hydrochloric acid (6 mL) was added, followed by stirring at 80°C for 15.5 hours. To the reaction solution, a saturated aqueous sodium bicarbonate solution was added, followed by extraction with a mixed solvent of chloroform/isopropanol (4/1). The organic layer was dried over sodium sulfate and concentrated under reduced pressure. The obtained crude product was purified by preparative thin layer chromatography (chloroform : 10% aqueous ammonia-methanol solution = 5 : 1) to individually yield an isomer U (6.1 mg, 8%), an isomer V (4.2 mg, 6%) and an isomer UV (7.1 mg, 9%) as white amorphous forms.

(Compound U)

$^1$H-NMR (400 MHz, $CD_3OD$)δ (ppm): 0.09 - 0.18 (m, 2H), 0.46 - 0.56 (m, 2H), 0.81 - 0.91 (m, 1H), 1.04 - 1.12 (m, 1H), 1.53 - 1.62 (m, 1H), 1.84 - 1.94 (m, 1H), 1.96 - 2.14 (m, 3H), 2.17 - 2.27 (m, 1H), 2.33 - 2.43 (m, 2H), 2.53 - 2.61 (m, 1H), 2.74 (dd, J = 6, 18 Hz, 1H), 2.79 - 2.87 (m, 1H), 2.90 - 3.06 (m, 4H), 3.24 - 3.34 (m, 1H), 6.59 (s, 1H), 6.98 (s, 1H).

(Compound V)

$^1$H-NMR (400 MHz, $CD_3OD$)δ (ppm): 0.07 - 0.17 (m, 2H), 0.46 - 0.55 (m, 2H), 0.80 - 0.92 (m, 1H), 1.60 - 2.05 (m, 5H), 2.29 - 2.41 (m, 2H), 2.45 - 2.66 (m, 2H), 2.73 - 2.87 (m, 2H), 2.88 - 3.11 (m, 5H), 3.26 - 3.33 (m, 1H), 6.66 (d, J = 8 Hz, 1H), 6.81 (d, J = 8 Hz, 1H).

(Compound UV)

$^1$H-NMR (400 MHz, $CD_3OD$)δ (ppm): 0.07 - 0.17 (m, 2H), 0.44 - 0.55 (m, 2H), 0.78 - 0.90 (m, 1H), 1.58 - 1.72

(m, 2H), 1.86 - 2.04 (m, 2H), 2.19 - 2.49 (m, 4H), 2.56 - 2.63 (m, 1H), 2.76 - 2.94 (m, 5H), 3.01 (dd, J = 6, 13 Hz, 1H), 3.10 - 3.18 (m, 1H), 3.41 - 3.50 (m, 1H), 6.84 (s, 1H).

(Example 326)

Synthesis of (5aS,6R, 11bS)-9-chloro-14-(cyclopropylmethyl)-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 396]

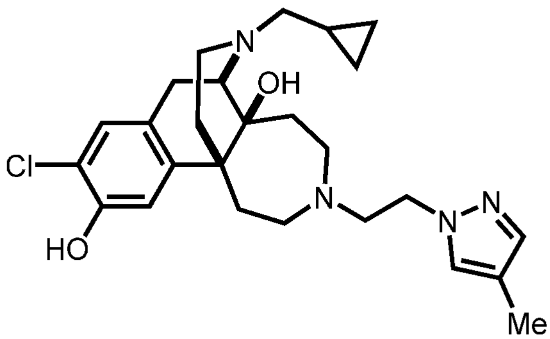

The title compound was obtained from the compound U obtained in Example 325 and the compound obtained in Reference Example 33 according to the method described in Example 31.

$^1$H-NMR (400 MHz, $CDCl_3$)$\delta$ (ppm): 0.05 - 0.12 (m, 2H), 0.46 - 0.52 (m, 2H), 0.75 - 0.85 (m, 1H), 0.92 - 1.00 (m, 1H), 1.40 - 1.48 (m, 1H), 1.66 - 1.77 (m, 2H), 1.92 - 2.05 (m, 2H), 1.99 (s, 3H), 2.27 - 2.40 (m, 3H), 2.41 - 2.60 (m, 3H), 2.68 - 2.96 (m, 6H), 3.07 - 3.16 (m, 1H), 3.92 - 4.06 (m, 2H), 6.71 (s, 1H), 6.84 (s, 1H), 7.04 (s, 1H), 7.21 (s, 1H).

(Example 327)

Synthesis of 1-((5aS,6R,11bR)-11-chloro-14-(cyclopropylmethyl)-5a,10-dihydroxy-1,2,5,5a,6,7-hexahydro-6,11b-(epiminoethano)naphtho[1,2-d]azepin-3(4H)-yl)-2-(pyridin-2-yl)ethan-1-one

[Chemical Formula 397]

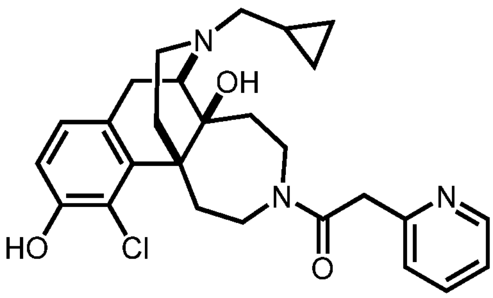

The title compound was obtained from the compound V obtained in Example 325 and 2-(pyridin-2-yl)acetic acid hydrochloride according to the method described in Example 26.

$^1$H-NMR (400 MHz, $CDCl_3$)$\delta$ (ppm): 0.05 - 0.12 (m, 2H), 0.47 - 0.54 (m, 2H), 0.74 - 0.86 (m, 1H), 1.49 - 1.55 (m, 0.3H), 1.58 - 1.73 (m, 1.7H), 1.76 - 2.06 (m, 2.7H), 2.17 - 2.39 (m, 2.3H), 2.48 - 2.75 (m, 2.7H), 2.76 - 2.97 (m, 3.3H), 3.16 - 3.26 (m, 0.7H), 3.40 (ddd, J = 3, 5, 13 Hz, 0.7H), 3.48 (d, J = 16 Hz, 0.7H), 3.58 - 3.81 (m, 3.2H), 4.18 (ddd, J = 2, 5, 14 Hz, 0.7H), 4.49 (br s, 1H), 6.80 - 6.85 (m, 1.3H), 6.90 (d, J = 8 Hz, 0.7H), 7.03 - 7.14 (m, 2H), 7.50 - 7.57 (m, 1H), 8.44 - 8.48 (m, 1H).

(Example 328)

Synthesis of 1-((5aS,6R,11bR)-14-(cyclopropylmethyl)-5a,10-dihydroxy-9-methyl-1,2,5,5a,6,7-hexahydro-6,11b-(epiminoethano)naphtho[1,2-d]azepin-3(4H)-yl)-2-(4-methyl-1H-pyrazol-1-yl)ethan-1-one

[Chemical Formula 398]

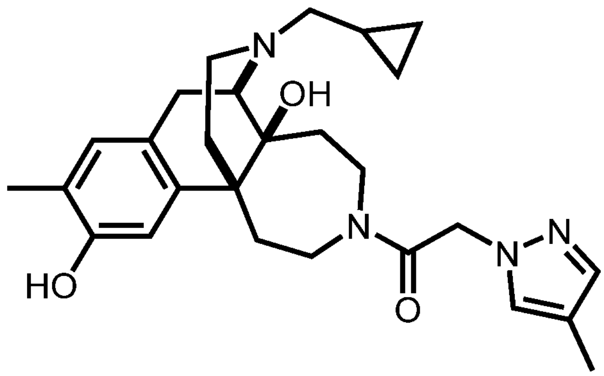

The title compound was obtained from the compound obtained in Example 290 and 2-(4-methyl-1H-pyrazol-1-yl)acetic acid according to the method described in Example 26.

$^1$H-NMR (400 MHz, $CDCl_3$)δ (ppm): 0.03 - 0.16 (m, 2H), 0.44 - 0.57 (m, 2H), 0.75 - 0.88 (m, 1H), 0.99 - 1.11 (m, 1H), 1.50 - 1.61 (m, 1H), 1.78 - 2.11 (m, 4H), 2.04 (s, 1.5H), 2.07 (s, 1.5H), 2.11 - 2.21 (m, 0.5H), 2.18 (s, 1.5H), 2.19 (s, 1.5H), 2.28 (dd, J = 6, 13 Hz, 0.5H), 2.28 - 2.39 (m, 1H), 2.35 (dd, J = 6, 13 Hz, 0.5H), 2.48 - 2.77 (m, 2.5H), 2.86 (d, J = 7 Hz, 0.5H), 2.89 (d, J = 19 Hz, 0.5H), 2.90 (d, J = 6 Hz, 0.5H), 2.96 (d, J = 18 Hz, 0.5H), 3.06 (ddd, J = 3, 13, 14 Hz, 0.5H), 3.17 - 3.28 (m, 1H), 3.33 (ddd, J = 4, 4, 13 Hz, 0.5H), 3.73 (ddd, J = 5, 12, 14 Hz, 0.5H), 3.80 - 3.90 (m, 1H), 4.07 - 4.16 (m, 0.5H), 4.45 (br s, 0.5H), 4.53 (br s, 0.5H), 4.66 (s, 1H), 4.91 (d, J = 16 Hz, 0.5H), 4.92 (d, J = 16 Hz, 0.5H), 6.65 (s, 1H), 6.81 (s, 0.5H), 6.83 (s, 0.5H), 7.10 (s, 0.5H), 7.19 (s, 0.5H), 7.28 (s, 0.5H), 7.30 (s, 0.5H).

(Example 329)

Synthesis of 1-((5aS,6R,11bR)-14-(cyclopropylmethyl)-5a,10-dihydroxy-9-methyl-1,2,5,5a,6,7-hexahydro-6,11b-(epiminoethano)naphtho[1,2-d]azepin-3(4H)-yl)-2-(4-(trifluoromethyl)-1H-pyrazol-1-yl)ethan-1-one

[Chemical Formula 399]

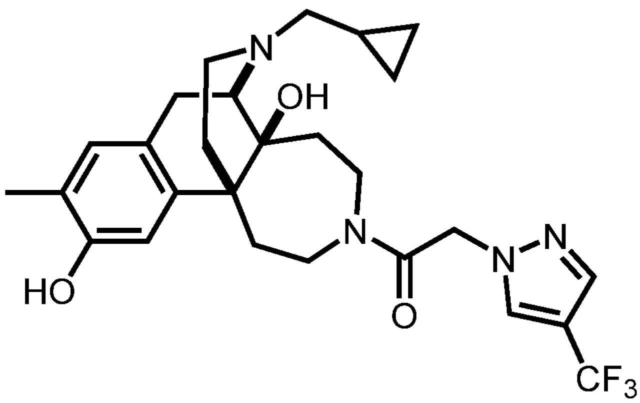

The title compound was obtained from the compound obtained in Example 290 and 2-(4-(trifluoromethyl)-1H-pyrazol-1-yl)acetic acid hydrochloride according to the method described in Example 26.

$^1$H-NMR (400 MHz, $CDCl_3$)δ (ppm): 0.05 - 0.18 (m, 2H), 0.47 - 0.57 (m, 2H), 0.76 - 0.89 (m, 1H), 1.04 - 1.13

(m, 1H), 1.55 - 1.65 (m, 1H), 1.83 (ddd, J = 2, 12, 15 Hz, 0.4H), 1.88 - 2.24 (m, 4.2H), 2.17 (s, 1.8H), 2.19 (s, 1.2H), 2.29 (dd, J = 6, 13 Hz, 0.4H), 2.35 (d, J = 6 Hz, 1.2H), 2.36 (dd, J = 6, 13 Hz, 0.4H), 2.49 - 2.59 (m, 1H), 2.61 - 2.80 (m, 1.4H), 2.86 - 2.93 (m, 1.4H), 2.96 - 3.03 (m, 0.6H), 2.99 (d, J = 18 Hz, 0.6H), 3.30 - 3.40 (m, 1H), 3.44 (ddd, J = 2, 6, 14 Hz, 0.4H), 3.72 (ddd, J = 5, 11, 14 Hz, 0.4H), 3.81 - 3.91 (m, 1H), 4.17 (ddd, J = 2, 5, 14 Hz, 0.6H), 4.76 (d, J = 16 Hz, 0.4H), 4.81 (d, J = 16 Hz, 0.4H), 4.99 (d, J = 16 Hz, 0.6H), 5.04 (d, J = 16 Hz, 0.6H), 6.62 (s, 0.4H), 6.65 (s, 0.6H), 6.84 (s, 0.6H), 6.85 (s, 0.4H), 7.55 (s, 0.4H), 7.66 (s, 0.4H), 7.71 (s, 0.6H), 7.80 (s, 0.6H).

(Reference Example 53)

Synthesis of (4R,4aS,8aR,13bS)-3-(cyclopropylmethyl)-4a,10-dihydroxy-1,2,3,4,4a,5,6,7-octahydro-4,13-methanobenzofuro[2,3-c]pyrido[4,3-d]azepin-8(8aH)-one

[Chemical Formula 400]

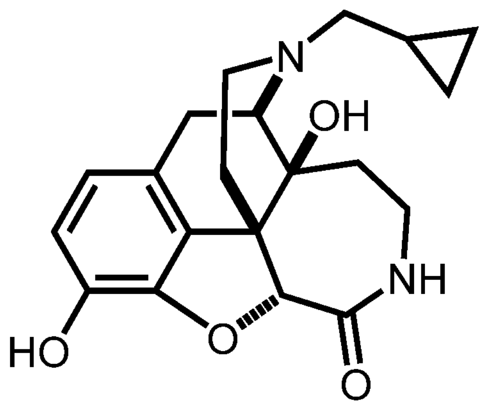

The title compound was obtained from the compound obtained in Reference Example 51 according to the method described in Example 6.

$^1$H-NMR (400 MHz, $CD_3OD$)δ (ppm): 0.13 - 0.20 (m, 2H), 0.50 - 0.59 (m, 2H), 0.84 - 0.95 (m, 1H), 1.35 - 1.43 (m, 1H), 1.55 - 1.62 (m, 1H), 1.79 - 1.90 (m, 1H), 2.29 - 2.47 (m, 4H), 2.67 (dd, J = 7, 19 Hz, 1H), 2.69 - 2.75 (m, 1H), 2.80 - 2.91 (m, 2H), 3.11 (d, J = 7 Hz, 1H), 3.15 (d, J = 5 Hz, 1H), 4.71 (s, 1H), 6.60 (d, J = 8 Hz, 1H), 6.68 (d, J = 8 Hz, 1H).

Synthesis of (5aS,6R,11bR)-14-(cyclopropylmethyl)-5a,11-dihydroxy-10-(methoxymethoxy)-3,4,5,5a,6,7-hexahydro-6,11b-(epiminoethano)naphtho[1,2-d]azepin-2(1H)-one

[Chemical Formula 401]

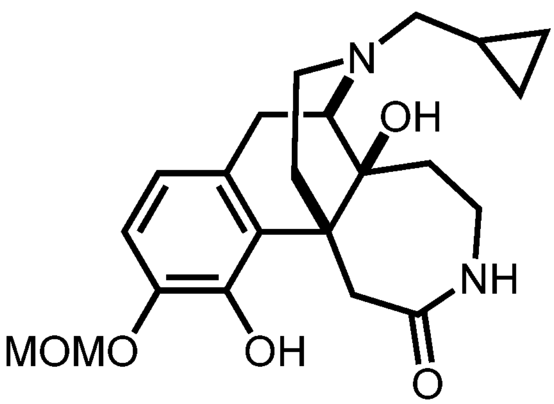

To a solution of the compound (107 mg, 0.30 mmol) obtained in Reference Example 53 in N,N-dimethylformamide (6 mL) were added potassium carbonate (249 mg, 1.8 mmol) and chloromethyl methyl ether (91.1 pL, 1.2 mmol), followed by stirring at 40°C for 6 hours. To the reaction mixture, a saturated sodium bicarbonate aqueous solution was added, followed by extraction three times with chloroform. The combined extracts were dried over sodium sulfate and then concentrated under reduced pressure. The obtained crude product was used for the next reaction without purification.

To a solution of the obtained crude product in ethylenediamine (10 mL) was added Sodium silica gel Stage I (1 g) under ice cooling, followed by stirring at room temperature for 1 hour. To the reaction mixture, tetrahydrofuran (50 mL) was added under ice cooling, and then water (100 mL) was slowly added at the same temperature, followed by stirring. The temperature of the reaction mixture was returned to room temperature, and to the reaction mixture, a saturated aqueous sodium bicarbonate solution was added, followed by extraction three times with chloroform. The combined extracts were dried over sodium sulfate and then concentrated under reduced pressure. The obtained crude product was purified by silica gel column chromatography (amino group-supported silica gel, 0 to 30% methanol/chloroform) to yield the title compound (93.0 mg, 77%) as a colorless amorphous form.

$^1$H-NMR (400 MHz, $CDCl_3$)$\delta$ (ppm): 0.06 - 0.13 (m, 2H), 0.47 - 0.55 (m, 2H), 0.76 - 0.88 (m, 1H), 1.52 - 1.68 (m, 1H), 1.81 - 1.90 (m, 1H), 1.93 - 2.05 (m, 2H), 2.32 (dd, J = 6, 12 Hz, 1H), 2.34 (dd, J = 7, 13 Hz, 1H), 2.54 - 2.64 (m, 1H), 2.80 - 3.00 (m, 4H), 3.11 (dd, J = 2, 14 Hz, 1H), 3.51 (s, 3H), 3.54 (d, J = 14 Hz, 1H), 3.86 - 3.96 (m, 1H), 4.65 (br s, 1H), 5.15 (d, J = 12 Hz, 1H), 5.16 (d, J = 12 Hz, 1H), 5.83 - 5.92 (m, 1H), 6.64 (d, J = 8 Hz, 1H), 6.95 (d, J = 9 Hz, 1H), 7.61 (s, 1H).

(Example 331)

Synthesis of (5aS,6R, 11bS)-14-(cyclopropylmethyl)-11-methoxy-10-(methoxymethoxy)-2,3,4,5,6,7-hexahydro-6,11b-(epiminoethano)naphtho[1,2-d]azepin-5a(1H)-ol (compound W) and (5aS,6R,11bS)-14-(cyclopropylmethyl)-11-methoxy-2,3,4,5,6,7-hexahydro-6,11b-(epiminoethano)naphtho[1,2-d]azepine-5a,10(1H)-diol (compound X)

[Chemical Formula 402]

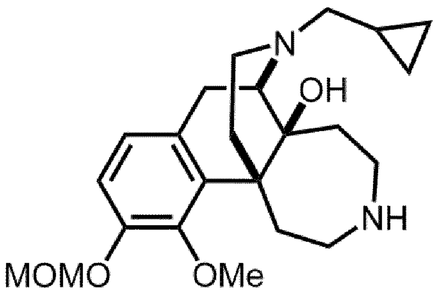

Compound W

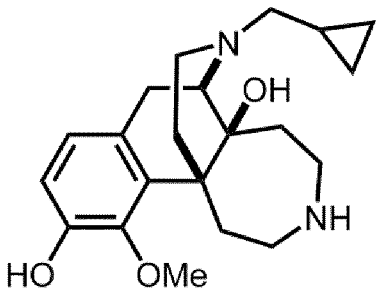

Compound X

To a solution of the compound (40.2 mg, 0.10 mmol) obtained in Example 330 in N,N-dimethylformamide (1 mL) were added methyl iodide (7.5 μL, 0.12 mmol) and potassium carbonate (69.1 mg, 0.50 mmol), followed by stirring at room temperature for 1 hour. To the reaction mixture, a saturated sodium bicarbonate aqueous solution was added, followed by extraction three times with chloroform. The combined extracts were dried over sodium sulfate and then concentrated under reduced pressure. The obtained crude product was used for the next reaction without purification.

To a solution of the obtained crude product in tetrahydrofuran (1 mL) was added a 0.91 M borane-tetrahydrofuran complex-tetrahydrofuran solution (1.0 mL, 0.91 mmol), followed by heating under reflux for 2 hours. After allowed to cool, the reaction mixture was concentrated under reduced pressure. To the residue, a 2 M aqueous sodium hydroxide solution (2 mL) was added, followed by stirring at room temperature for 1 hours. Water was added to the reaction mixture, followed by extraction three times with chloroform. The combined extracts were dried over sodium sulfate and then concentrated under reduced pressure. The obtained crude product was purified by silica gel column chromatography (amino group-supported silica gel, 0 to 50% methanol/chloroform) to individually yield the title compound W (12.1 mg, 30%) and the crude product as colorless amorphous forms.

To the obtained crude product was added 2 M hydrochloric acid (5.0 mL, 10 mmol), followed by stirring at room temperature for 30 minutes. To the reaction mixture, a 28% aqueous ammonia solution was added under ice cooling, followed by extraction three times with chloroform. The organic layers were combined, dried over sodium sulfate, and then concentrated under reduced pressure. The obtained crude product was purified by preparative thin layer chromatography (chloroform : 2 M ammonia-methanol solution = 5 : 1) to yield the title compound X (21.0 mg, 59%) as a colorless amorphous form.

(Compound W)

$^1$H-NMR (400 MHz, $CDCl_3$)δ (ppm): 0.05 - 0.14 (m, 2H), 0.46 - 0.55 (m, 2H), 0.76 - 0.90 (m, 1H), 1.31 - 1.40 (m, 1H), 1.55 - 1.85 (m, 2H), 1.94 - 2.14 (m, 2H), 2.25 - 2.39 (m, 3H), 2.41 - 2.51 (m, 1H), 2.54 - 2.63 (m, 1H), 2.71 - 2.80 (m, 1H), 2.86 - 3.02 (m, 5H), 3.08 - 3.18 (m, 1H), 3.51 (s, 3H), 3.79 (s, 3H), 4.47 (br s, 1H), 5.16 (s, 2H), 6.77 (d, J = 9 Hz, 1H), 6.97 (d, J = 9 Hz, 1H).

(Compound X)

$^1$H-NMR (400 MHz, $CDCl_3$)δ (ppm): 0.06 - 0.13 (m, 2H), 0.47 - 0.54 (m, 2H), 0.77 - 0.88 (m, 1H), 1.29 - 1.38 (m, 1H), 1.55 - 1.70 (m, 2H), 2.02 - 2.14 (m, 2H), 2.20 - 2.63 (m, 6H), 2.75 - 3.02 (m, 5H), 3.06 - 3.16 (m, 1H), 3.71 (s, 3H), 4.53 (br s, 1H), 6.69 (d, J = 8 Hz, 1H), 6.73 (d, J = 8 Hz, 1H).

(Example 332)

Synthesis of (5aS,6R,11bS)-14-(cyclopropylmethyl)-11-methoxy-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 403]

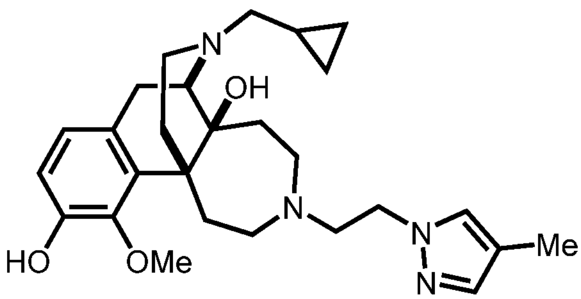

To a solution of the compound W (10 mg, 0.025 mmol) obtained from Example 331 in acetonitrile (0.5 mL) were added the compound (10.2 mg, 0.050 mmol) obtained in Reference Example 33 and N,N-diisopropylethylamine (13.1 μL, 0.075 mmol), followed by stirring at 70°C for 18 hours. After allowed to cool, to the reaction mixture was added a 28% aqueous ammonia solution, followed by extraction three times with chloroform. The combined extracts were dried over sodium sulfate and then concentrated under reduced pressure. The obtained crude product was used for the next reaction without purification.

To the obtained crude product was added 2 M hydrochloric acid (2.0 mL, 4.0 mmol), followed by stirring at room temperature for 30 minutes. To the reaction mixture, a 28% aqueous ammonia solution was added under ice cooling, followed by extraction three times with chloroform. The organic layers were combined, dried over sodium sulfate, and then concentrated under reduced pressure. The obtained crude product was purified by preparative thin layer chromatography (chloroform : 2 M ammonia-methanol solution = 10 : 1) to yield the title compound (3.5 mg, 30%) as a colorless amorphous form.

$^1$H-NMR (400 MHz, $CDCl_3$)δ (ppm): 0.07 - 0.13 (m, 2H), 0.47 - 0.53 (m, 2H), 0.77 - 0.89 (m, 1H), 1.29 - 1.44 (m, 2H), 1.70 - 1.80 (m, 1H), 2.00 (s, 3H), 2.02 - 2.24 (m, 3H), 2.27 - 2.49 (m, 4H), 2.55 - 2.97 (m, 8H), 3.14 - 3.24 (m, 1H), 3.74 (s, 3H), 3.89 - 4.05 (m, 2H), 6.75 (d, J = 8 Hz, 1H), 6.77 (d, J = 8 Hz, 1H), 6.83 (s, 1H), 7.21 (s, 1H).

(Example 333)

Synthesis of 1-((5aS,6R,11bR)-14-(cyclopropylmethyl)-5a,10-dihydroxy-11-methoxy-1,2,5,5a,6,7-hexahydro-6,11b-(epiminoethano)naphtho[1,2-d]azepin-3(4H)-yl)-2-(pyridin-2-yl)ethan-1-one

[Chemical Formula 404]

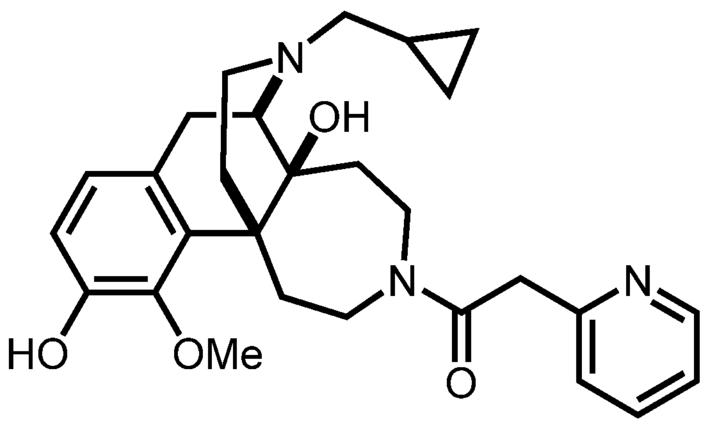

The title compound was obtained from the compound X obtained in Example 331 and 2-(pyridin-2-yl)acetic acid hydrochloride according to the method described in Example 5.

$^1$H-NMR (400 MHz, $CDCl_3$)δ (ppm): 0.06 - 0.15 (m, 2H), 0.47 - 0.55 (m, 2H), 0.77 - 0.89 (m, 1H), 1.20 - 1.60 (m, 2H), 1.72 - 1.88 (m, 0.7H), 2.00 - 4.10 (m, 16.3H), 3.71 (s, 0.9H), 3.80 (s, 2.1H), 4.48 (br s, 1H), 6.65 - 6.70 (m, 0.3H), 6.69 (d, J = 8 Hz, 0.7H), 6.76 (d, J = 8 Hz, 0.7H), 6.80 (d, J = 8 Hz, 0.3H), 7.04 - 7.16 (m, 2H), 7.51 - 7.60 (m, 1H), 8.42 - 8.46 (m, 1H).

(Example 334)

Synthesis of tert-butyl (5aS,6R,11bR)-14-(cyclopropylmethyl)-5a-hydroxy-10-(methylcarbamoyl)-1,2,5,5a,6,7-hexahydro-6,11b-(epiminoethano)naphtho[1,2-d]azepine-3(4H)-carboxylate

[Chemical Formula 405]

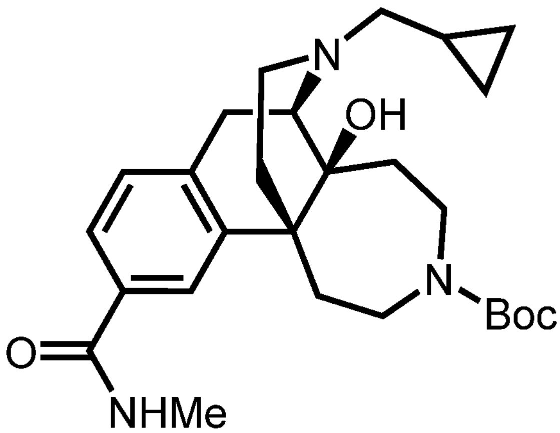

To a solution of the compound (24 mg, 0.055 mmol) obtained in Example 241 in methanol (1 mL) was added 4 M aqueous sodium hydroxide solution (1 mL), followed by heating under reflux for 16 hours. The reaction solution was allowed to cool to room temperature, then neutralized with 2 M hydrochloric acid (2 mL) under ice cooling, and then concentrated under reduced pressure. The obtained residue was lyophilized. To a solution of the obtained crude product in N,N-dimethylformamide (0.5 mL) were added methylamine hydrochloride (19 mg, 0.27 mmol), N,N-diisopropylethylamine (77 μL, 0.44 mmol), and O-(7-azabenzotriazol-1-yl)-N,N,N',N'-tetramethyluronium hexafluorophosphate (HATU) (22 mg, 0.058 mmol), followed by stirring at room temperature for 16 hours. To the reaction mixture, a saturated aqueous sodium bicarbonate solution was added, followed by extraction three times with ethyl acetate. The combined extracts

were dried over sodium sulfate and then concentrated under reduced pressure. The obtained crude product was purified by preparative thin layer chromatography (chloroform : 2 M ammonia-methanol solution = 10 : 1) to yield the title compound (26 mg, quantitative) as a colorless oily matter.

$^1$H-NMR (400 MHz, $CDCl_3$)δ (ppm): 0.05 - 0.30 (m, 2H), 0.45 - 0.60 (m, 2H), 0.80 - 1.00 (m, 1H), 1.00 - 1.10 (m, 9H), 1.30 - 2.10 (m, 5H), 2.30 - 2.40 (m, 2H), 2.50 - 2.60 (m, 1H), 2.60 - 2.80 (m, 1H), 2.80 - 3.30 (m, 10H), 3.50 - 3.60 (m, 1H), 4.25 - 4.40 (m, 1H), 7.05 - 7.15 (m, 1H), 7.20 - 7.30 (m, 1H), 7.35 - 7.45 (m, 1H).

(Example 335)

Synthesis of (5aS,6R,11bR)-14-(cyclopropylmethyl)-5a-hydroxy-N-methyl-3-(2-(pyridin-2-yl)acetyl)-1,2,3,4,5,5a,6,7-octahydro-6,11b-(epiminoethano)naphtho[1,2-d]azepine-10-carboxamide

[Chemical Formula 406]

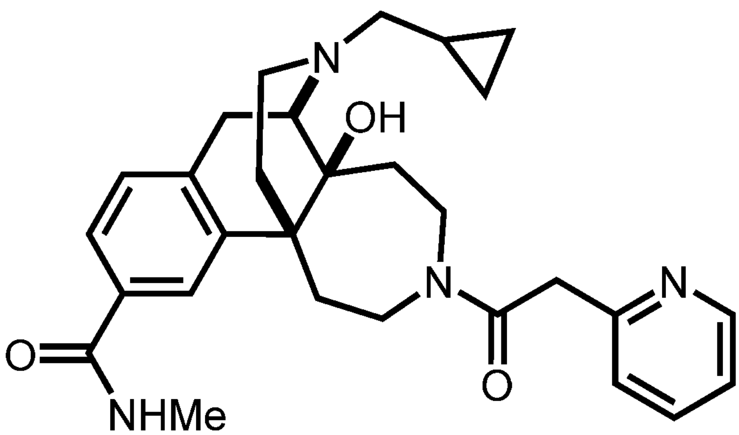

To the compound (26 mg, 0.055 mmol) obtained in Example 334 was added trifluoroacetic acid (0.5 mL), followed by stirring at room temperature for 1 hour. The reaction solution was concentrated under reduced pressure. The obtained residue was roughly purified by silica gel column chromatography (amino group-supported silica gel, 0 to 20% methanol/chloroform). To a solution of half (6.5 mg) of the obtained crude product (13 mg, 64%) in N,N-dimethylformamide (1 mL) were added 2-(pyridin-2-yl) acetic acid hydrochloride (5 mg, 0.019 mmol), N,N-diisopropylethylamine (12 μL, 0.070 mmol), and O-(7-azabenzotriazol-1-yl)-N,N,N',N'-tetramethyluronium hexafluorophosphate (HATU) (7 mg, 0.019 mmol), followed by stirring at room temperature for 16 hours. To the reaction mixture, a saturated aqueous sodium bicarbonate solution was added, followed by extraction three times with ethyl acetate. The combined extracts were dried over sodium sulfate and then concentrated under reduced pressure. The obtained crude product was purified by preparative thin layer chromatography (chloroform : 2 M ammonia-methanol solution = 10 : 1) to yield the title compound (1.9 mg, 22%) as a colorless oily matter.

$^1$H-NMR (400 MHz, $CDCl_3$)δ (ppm): 0.05 - 0.30 (m, 2H), 0.50 - 0.70 (m, 2H), 0.80 - 1.00 (m, 1H), 1.00 - 1.30 (m, 2H), 1.30 - 2.10 (m, 3H), 2.20 - 2.45 (m, 3H), 2.45 - 2.80 (m, 3H), 2.80 - 3.10 (m, 5H), 3.20 - 3.40 (m, 2H), 3.45 - 4.00 (m, 4H), 4.20 - 4.60 (m, 1H), 5.00 - 5.50 (m, 1H), 6.85 (d, J = 7 Hz, 0.5H), 7.00 - 7.70 (m, 5.5H), 8.30 - 8.50 (m, 1H).

(Example 336)

Synthesis of 1-((5aS,6R,11bR)-14-(cyclopropylmethyl)-5a,10-dihydroxy-9-methyl-1,2,5,5a,6,7-hexahydro-6,11b-(epiminoethano)naphtho[1,2-d]azepin-3(4H)-yl)-2-((R)-3-methylpyrrolidin-1-yl)ethan-1-one

[Chemical Formula 407]

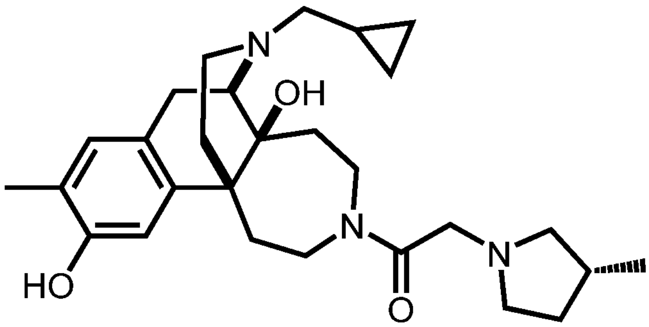

The title compound was obtained from the compound obtained in Example 290 and (R)-3-methylpyrrolidine hydrochloride according to the method described in Example 132.

$^1$H-NMR (400 MHz, $CDCl_3$)δ (ppm): 0.04 - 0.16 (m, 2H), 0.44 - 0.56 (m, 2H), 0.76 - 0.89 (m, 1H), 0.96 - 1.12 (m, 1H), 0.99 (d, J = 6 Hz, 3H), 1.19 - 1.38 (m, 1H), 1.53 - 1.62 (m, 1H), 1.82 (ddd, J = 2, 12, 15 Hz, 0.3H), 1.90 - 2.39 (m, 10.3H), 2.17 (s, 3H), 2.45 - 2.57 (m, 1.4H), 2.59 - 2.80 (m, 3H), 2.85 - 2.93 (m, 0.6H), 2.87 (d, J = 6 Hz, 0.7H), 2.96 (d, J = 18 Hz, 0.7H), 3.15 (d, J = 15 Hz, 0.3H), 3.18 (d, J = 14 Hz, 0.7H), 3.20 (d, J = 15 Hz, 0.3H), 3.32 (d, J = 14 Hz, 0.7H), 3.35 - 3.50 (m, 1.7H), 3.55 (ddd, J = 4, 5, 14 Hz, 0.3H), 3.63 (ddd, J = 4, 11, 14 Hz, 0.3H), 3.72 - 3.83 (m, 1H), 3.96 - 4.03 (m, 0.7H), 4.51 (br s, 1H), 6.61 (s, 0.3H), 6.80 (s, 0.7H), 6.81 (s, 0.3H), 6.84 (s, 0.7H).

(Example 337)

Synthesis of (5aS,6R, 11bS)-14-(cyclopropylmethyl)-9-methoxy-3-(2-(4-methyl-1H-pyrazol-1-yl)ethyl)-2,3,4,5,6,7-hexahydro-6,11b-(epiminoethano)naphtho[1,2-d]azepine-5a,10(1H)-diol

[Chemical Formula 408]

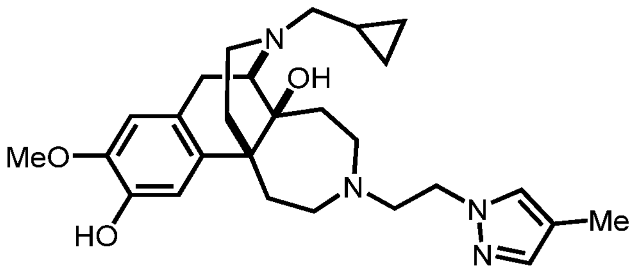

To the compound (32.2 mg, 0.050 mmol) obtained in Example 292 and a copper powder (3.2 mg, 0.050 mmol) suspended in pyridine (1 mL), a 5 M sodium methoxide-methanol solution (200 μL, 1.0 mmol) was added, followed by heating under reflux for 1 hour. Thereafter, a copper powder (3.2 mg, 0.050 mmol) was added, followed by heating under reflux for 1 hour. Thereafter, a copper powder (3.2 mg, 0.050 mmol) was added, followed by heating under reflux for 1 hour. After allowed to cool, the reaction mixture was filtered through celite, and to the filtrate, a 28% aqueous ammonia solution was added, followed by extraction three times with chloroform. The organic layers were combined, dried over sodium sulfate, and then concentrated under reduced pressure. Although the obtained crude product was purified by silica gel column chromatography (amino group-supported silica gel, 0 to 10% methanol/chloroform), it was so difficult to separate the crude product from the by-product that the crude product was used for the next reaction without further purification.

To the obtained crude product was added trifluoroacetic acid (1 mL), followed by stirring at room temperature for 30 minutes. The reaction mixture was concentrated under reduced pressure, and to the concentrated reaction mixture, a 28% aqueous ammonia solution was added, followed by extraction three times with chloroform. The organic layers were combined, dried over sodium sulfate, and then concentrated under reduced pressure. The obtained crude product was used for the next reaction without purification.

To a solution of the obtained crude product in acetonitrile (1 mL) were added the compound (20.4 mg, 0.10

mmol) obtained in Reference Example 33 and N,N-diisopropylethylamine (26.1 μL, 0.15 mmol), followed by stirring at 70°C for 18 hours. After allowed to cool, to the reaction mixture was added a 28% aqueous ammonia solution, followed by extraction three times with chloroform. The combined extracts were dried over sodium sulfate and then concentrated under reduced pressure. The obtained crude product was used for the next reaction without purification.

To a solution of the obtained crude product in methanol (1 mL) were added 10% palladium-activated carbon (32.2 mg) and potassium formate (84.1 mg, 1.0 mmol), followed by stirring at room temperature for 30 minutes. The reaction mixture was filtered through celite, and to the filtrate, a 28% aqueous ammonia solution was added, followed by extraction three times with chloroform. The organic layers were combined, dried over sodium sulfate, and then concentrated under reduced pressure. The obtained crude product was purified by preparative thin layer chromatography (chloroform : 2 M ammonia-methanol solution = 10 : 1) to yield the title compound (3.5 mg, 13%) as a colorless amorphous form.

$^1$H-NMR (400 MHz, $CDCl_3$)δ (ppm): 0.06 - 0.14 (m, 2H), 0.46 - 0.54 (m, 2H), 0.77 - 0.90 (m, 1H), 0.96 - 1.04 (m, 1H), 1.38 - 1.48 (m, 1H), 1.76 - 1.88 (m, 2H), 1.94 - 2.10 (m, 2H), 2.00 (s, 3H), 2.27 - 2.41 (m, 3H), 2.48 - 2.59 (m, 2H), 2.64 - 2.72 (m, 1H), 2.77 (dd, J = 6, 18 Hz, 1H), 2.85 - 2.99 (m, 5H), 3.18 - 3.26 (m, 1H), 3.86 (s, 3H), 3.98 - 4.18 (m, 2H), 6.57 (s, 1H), 6.66 (s, 1H), 6.90 (s, 1H), 7.23 (s, 1H).

(Reference Example 54)

Synthesis of (4'R,4a'S,7a'R,12b'S)-3'-(cyclopropylmethyl)-4a'-hydroxy-9'-methoxy-1',2',3',4',4a',5'-hexahydrospiro[cyclopropane-1,6'-[4a,12]methanobenzofuro[3,2-e]isoquinolin]-7'(7a'H)-one

[Chemical Formula 409]

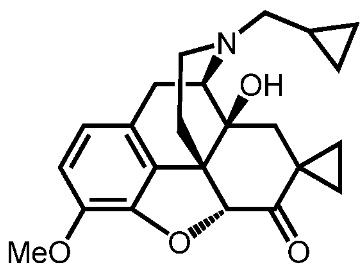

To a solution of (4R,4aS,7aR,12bS)-3-(cyclopropylmethyl)-4a-hydroxy-9-methoxy-2,3,4,4a,5,6-hexahydro-1H-4,12-methanobenzofuro[3,2-e]isoquinolin-7(7aH)-one (synthesized by the method described in Organic Letters 2009, 11, 539) (854 mg, 3.38 mmol) in tert-butanol (28 mL) were added potassium tert-butoxide (1.20 g, 10.7 mmol) and (2-chloroethyl) dimethylsulfonium iodide (synthesized by the method described in WO 2007092681) (1.00 g, 2.81 mmol), followed by stirring at room temperature for 24 hours. Water was added to the reaction mixture, followed by extraction three times with ethyl acetate. The combined extracts were washed with saturated saline, dried over sodium sulfate, and then concentrated under reduced pressure. The obtained crude product was purified by silica gel column chromatography (28 to 100% ethyl acetate/heptane) to yield the title compound (843 mg, 79%) as a pale yellow crystal.

$^1$H-NMR (400 MHz, $CDCl_3$)δ (ppm): 0.13 - 0.17 (m, 2H), 0.43 - 0.48 (m, 1H), 0.53 - 0.58 (m, 2H), 0.82 - 0.91 (m, 1H), 1.08 - 1.19 (m, 3H), 1.57 - 1.62 (m, 2H), 2.14 (d, J = 14 Hz, 1H), 2.25 (ddd, J = 3, 12, 12 Hz, 1H), 2.33 - 2.47 (m, 3H), 2.58 (dd, J = 6, 18 Hz, 1H), 2.71 (dd, J = 5, 12 Hz, 1H), 3.10 (d, J = 18 Hz, 1H), 3.15 (d, J = 6 Hz, 1H), 3.87 (s, 3H), 4.53 (s, 1H), 6.64 (d, J = 8 Hz, 1H), 6.72 (d, J = 8 Hz, 1H).

(Reference Example 55)

Synthesis of (4b'R,8a'S,9'R)-11'-(cyclopropylmethyl)-4',8a'-dihydroxy-3'-methoxy-8',8a',9',10'-tetrahydrospiro[cyclopropane-1,7'-[9,4b](epiminoethano)phenanthren]-6'(5'H)-one (compound Y) and (4bR,8aS,9R)-11-(cyclopropylmethyl)-7-ethyl-4,8a-dihydroxy-3-methoxy-8,8a,9,10-tetrahydro-5H-9,4b-(epiminoethano)phenanthren-6(7H)-one (compound Z)

[Chemical Formula 410]

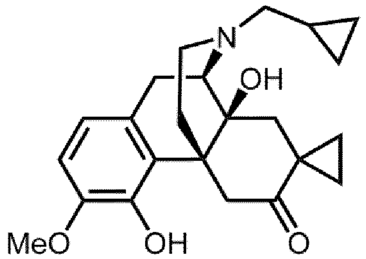

Compound Y

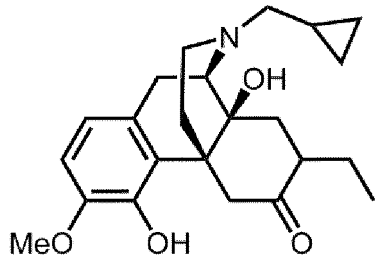

Compound Z

The title compounds Y and Z were individually obtained from the compound obtained in Reference Example 54 according to the method described in Reference Example 34.

(Compound Y)

$^1$H-NMR (400 MHz, $CDCl_3$)δ (ppm): 0.10 - 0.14 (m, 2H), 0.20 - 0.25 (m, 1H), 0.50 - 0.55 (m, 2H), 0.82 - 0.88 (m, 2H), 0.93 - 0.98 (m, 1H), 1.29 (d, J = 14 Hz, 1H), 1.37 - 1.42 (m, 1H), 1.59 - 1.62 (m, 1H), 2.06 - 2.09 (m, 2H), 2.32 - 2.41 (m, 3H), 2.61 - 2.64 (m, 1H), 2.85 (dd, J = 6, 19 Hz, 1H), 3.00 - 3.09 (m, 3H), 3.83 (s, 3H), 3.98 (d, J = 16 Hz, 1H), 4.71 (br s, 1H), 6.05 (s, 1H), 6.58 (d, J = 8 Hz, 1H), 6.69 (d, J = 8 Hz, 1H).

(Compound Z)

$^1$H-NMR (400 MHz, $CDCl_3$)δ (ppm): 0.10 - 0.14 (m, 2H), 0.51 - 0.56 (m, 2H), 0.82 (t, J = 8 Hz, 3H), 0.82 - 0.89 (m, 2H), 0.94 - 1.05 (m, 1H), 1.53 - 1.60 (m, 1H), 1.67 - 1.78 (m, 1H), 1.92 (dd, J = 6, 13 Hz, 1H), 1.99 - 2.12 (m, 2H), 2.33 - 2.41 (m, 2H), 2.58 - 2.61 (m, 1H), 2.67 - 2.75 (m, 1H), 2.82 (dd, J = 6, 18 Hz, 1H), 2.96 (d, J = 13 Hz, 1H), 2.98 (d, J = 18 Hz, 1H), 3.10 (d, J = 6 Hz, 1H), 3.82 (s, 3H), 3.91 (d, J = 13 Hz, 1H), 4.77 (br s, 1H), 6.09 (s, 1H), 6.53 (d, J = 8 Hz, 1H), 6.65 (d, J = 8 Hz, 1H).

(Reference Example 56)

Synthesis of (4b'R,8a'S,9'R)-11'-(cyclopropylmethyl)-8a'-hydroxy-3'-methoxy-6'-oxo-5',6',8',8a',9',10'-hexahydrospiro[cyclopropane-1,7'-[9,4b](epiminoethano)phenanthren]-4'-yl trifluoromethanesulfonate

[Chemical Formula 411]

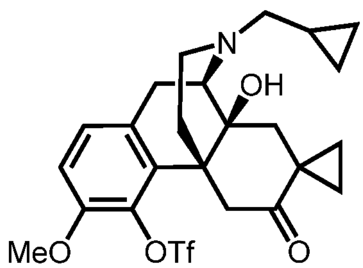

The title compound was obtained from the compound Y obtained in Reference Example 55 according to the method described in Reference Example 1.

$^1$H-NMR (400 MHz, $CDCl_3$)δ (ppm): 0.11 - 0.19 (m, 3H), 0.53 - 0.57 (m, 2H), 0.79 - 0.89 (m, 2H), 0.94 - 0.99 (m, 1H), 1.30 - 1.35 (m, 1H), 1.36 (d, J = 14 Hz, 1H), 1.70 - 1.73 (m, 1H), 2.10 - 2.24 (m, 3H), 2.33 - 2.43 (m, 2H), 2.68 - 2.72 (m, 1H), 2.90 (dd, J = 7, 18 Hz, 1H), 3.08 (d, J = 18 Hz, 1H), 3.13 (d, J = 7 Hz, 1H), 3.23 (d, J = 16 Hz, 1H), 3.60 (d, J = 16 Hz, 1H), 3.80 (s, 3H), 6.87 (d, J = 8 Hz, 1H), 7.07 (d, J = 8 Hz, 1H).

(Reference Example 57)

Synthesis of (4b'R,8a'S,9'R)-11'-(cyclopropylmethyl)-8a'-hydroxy-3'-methoxy-8',8a',9',10'-tetrahydrospiro[cyclopropane-1,7'-[9,4b](epiminoethano)phenanthren]-6'(5'H)-one

[Chemical Formula 412]

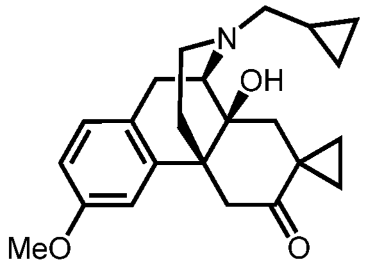

The title compound was obtained from the compound obtained in Reference Example 56 according to the method described in Reference Example 2.

$^1$H-NMR (400 MHz, $CDCl_3$)$\delta$ (ppm): 0.11 - 0.15 (m, 2H), 0.20 - 0.25 (m, 1H), 0.52 - 0.56 (m, 2H), 0.83 - 0.93 (m, 3H), 1.17 - 1.28 (m, 2H), 1.43 - 1.48 (m, 1H), 2.10 - 2.19 (m, 2H), 2.27 (d, J = 14 Hz, 1H), 2.37 (dd, J = 6, 12 Hz, 1H), 2.42 (dd, J = 6, 12 Hz, 1H), 2.60 - 2.62 (m, 1H), 2.77 (dd, J = 7, 19 Hz, 1H), 2.92 (d, J = 16 Hz, 1H), 3.04 - 3.14 (m, 3H), 3.75 (s, 3H), 6.71 (dd, J = 2, 8 Hz, 1H), 6.77 (d, J = 2 Hz, 1H), 7.01 (d, J = 8 Hz, 1H).

(Reference Example 58)

Synthesis of (4b'R,8a'S,9'R)-11'-(cyclopropylmethyl)-8a'-hydroxy-3'-methoxy-8',8a',9',10'-tetrahydrospiro[cyclopropane-1,7'-[9,4b](epiminoethano)phenanthren]-6'(5'H)-one oxime

[Chemical Formula 413]

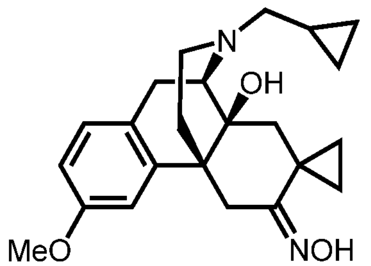

The title compound was obtained from the compound obtained in Reference Example 57 according to the method described in Reference Example 3.

$^1$H-NMR (400 MHz, $CDCl_3$)$\delta$ (ppm): 0.10 - 0.17 (m, 3H), 0.50 - 0.54 (m, 2H), 0.59 - 0.63 (m, 1H), 0.82 - 0.88 (m, 2H), 0.93 (d, J = 14 Hz, 1H), 1.18 - 1.29 (m, 2H), 2.10 - 2.18 (m, 3H), 2.34 (dd, J = 6, 13 Hz, 1H), 2.40 (dd, J = 6, 13 Hz, 1H), 2.55 (d, J = 14 Hz, 1H), 2.59 - 2.61 (m, 1H), 2.70 (dd, J = 6, 18 Hz, 1H), 3.01 (d, J = 6 Hz, 1H), 3.04 (d, J = 18 Hz, 1H), 3.74 (d, J = 16 Hz, 1H), 3.76 (s, 3H), 6.69 (dd, J = 3, 8 Hz, 1H), 6.73 (br s, 1H), 6.96 (d, J = 8 Hz, 1H), 7.05 (d, J = 3 Hz, 1H).

(Example 338)

Synthesis of (5a'S,6'R,11b'R)-14'-(cyclopropylmethyl)-5a'-hydroxy-10'-methoxy-5',5a',6',7'-tetrahydro-1'H-spiro[cyclopropane-1,4'-[6,11b](epiminoethano)naphtho[1,2-d]azepin]-2'(3'H)-one

[Chemical Formula 414]

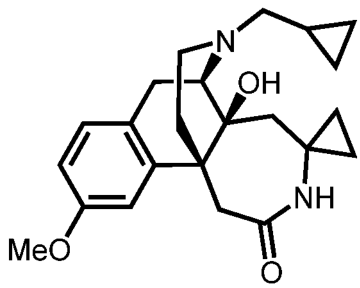

The title compound was obtained from the compound obtained in Reference Example 58 according to the method described in Example 1.

$^1$H-NMR (400 MHz, $CDCl_3$)δ (ppm): 0.09 - 0.12 (m, 2H), 0.47 - 0.55 (m, 4H), 0.78 - 0.85 (m, 1H), 0.90 - 0.95 (m, 1H), 0.98 (d, J = 14 Hz, 1H), 1.14 - 1.17 (m, 1H), 1.43 - 1.49 (m, 1H), 2.01 - 2.17 (m, 2H), 2.30 - 2.39 (m, 2H), 2.40 (dd, J = 2, 14 Hz, 1H), 2.57 - 2.61 (m, 2H), 2.77 (dd, J = 6, 18 Hz, 1H), 2.85 (d, J = 6 Hz, 1H), 3.00 (d, J = 18 Hz, 1H), 3.76 (d, J = 15 Hz, 1H), 3.80 (s, 3H), 4.73 (br s, 1H), 5.42 (br s, 1H), 6.71 (dd, J = 2, 8 Hz, 1H), 6.97 (d, J = 8 Hz, 1H), 7.16 (d, J = 2 Hz, 1H).

(Example 339)

Synthesis of (5a'S,6'R, 11b'S)-14'-(cyclopropylmethyl)-10'-methoxy-2',3',6',7'-tetrahydro-1'H-spiro[cyclopropane-1,4'-[6,11b](epiminoethano)naphtho[1,2-d]azepin]-5a'(5'H)-ol

[Chemical Formula 415]

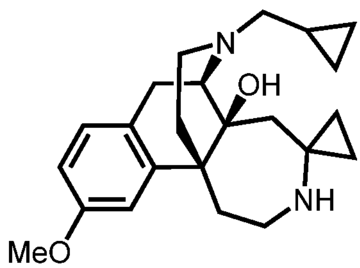

The title compound was obtained from the compound obtained in Example 338 according to the method described in Example 2.

$^1$H-NMR (400 MHz, $CDCl_3$)δ (ppm): 0.07 - 0.15 (m, 3H), 0.34 - 0.39 (m, 1H), 0.48 - 0.53 (m, 2H), 0.72 - 0.76 (m, 2H), 0.79 - 0.87 (m, 1H), 1.06 - 1.13 (m, 2H), 2.00 - 2.06 (m, 3H), 2.27 - 2.45 (m, 4H), 2.51 - 2.54 (m, 1H), 2.79 (dd, J = 6, 18 Hz, 1H), 2.84 - 3.02 (m, 4H), 3.79 (s, 3H), 4.52 (br s, 1H), 6.72 (dd, J = 2, 8 Hz, 1H), 6.77 (d, J = 2 Hz, 1H), 7.03 (d, J = 8 Hz, 1H).

(Example 340)

Synthesis of 1-((5a'S,6'R,11b'R)-14'-(cyclopropylmethyl)-5a'-hydroxy-10'-methoxy-1',2',5',5a',6',7'-hexahydro-3'H-spiro[cyclopropane-1,4'-[6,11b](epiminoethano)naphtho[1,2-d]azepin]-3'-yl)-2-(pyridin-2-yl)ethan-1-one

[Chemical Formula 416]

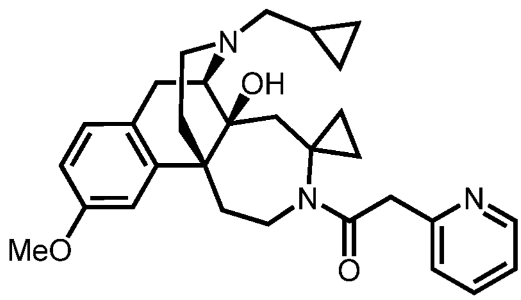

The title compound was obtained from the compound obtained in Example 339 and 2-(pyridin-2-yl)acetic acid hydrochloride according to the method described in Example 5.

$^1$H-NMR (400 MHz, $CDCl_3$)$\delta$ (ppm): 0.07 - 0.10 (m, 2H), 0.46 - 0.53 (m, 2H), 0.66 - 1.18 (m, 6H), 1.48 - 1.54 (m, 0.7H), 1.78 - 1.86 (m, 1H), 1.90 - 1.97 (m, 1H), 2.07 - 2.17 (m, 1H), 2.25 - 2.36 (m, 2.3H), 2.48 - 2.60 (m, 2H), 2.72 (dd, J = 6, 19 Hz, 0.7H), 2.83 - 2.90 (m, 3H), 2.98 - 3.24 (m, 1.3H), 3.54 (d, J = 15 Hz, 0.3H), 3.68 (d, J = 15 Hz, 0.7H), 3.78 - 3.86 (m, 4H), 3.95 - 4.03 (m, 0.3H), 4.71 - 4.79 (m, 0.7H), 6.55 (dd, J = 3, 8 Hz, 0.7H), 6.63 - 6.82 (m, 3.3H), 7.03 - 7.09 (m, 1H), 7.42 (ddd, J = 2, 8, 8 Hz, 0.7H), 7.51 (ddd, J = 2, 8, 8 Hz, 0.3H), 8.37 - 8.40 (m, 0.7H), 8.42 - 8.45 (m, 0.3H).

(Example 341)

Synthesis of 1-((5a'S,6'R,11b'R)-14'-(cyclopropylmethyl)-5a',10'-dihydroxy-1',2',5',5a',6',7'-hexahydro-3'H-spiro[cyclopropane-1,4'-[6,11b](epiminoethano)naphtho[1,2-d]azepin]-3'-yl)-2-(pyridin-2-yl)ethan-1-one

[Chemical Formula 417]

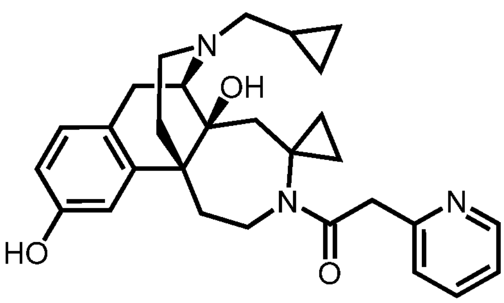

The title compound was obtained from the compound obtained in Example 340 according to the method described in Example 6.

$^1$H-NMR (400 MHz, $CDCl_3$)$\delta$ (ppm): 0.06 - 0.09 (m, 2H), 0.46 - 0.51 (m, 2H), 0.66 - 0.82 (m, 2H), 0.92 - 1.06 (m, 3H), 1.14 - 1.26 (m, 1H), 1.52 - 1.55 (m, 0.7H), 1.68 - 1.81 (m, 1H), 1.94 - 2.13 (m, 2.3H), 2.23 - 2.35 (m, 2H), 2.45 - 2.71 (m, 2.6H), 2.82 - 3.04 (m, 4.4H), 3.38 (d, J = 15 Hz, 0.3H), 3.61 (d, J = 15 Hz, 0.3H), 3.71 (d, J = 15 Hz, 0.7H), 3.83 (d, J = 15 Hz, 0.7H), 3.95 - 4.04 (m, 0.3H), 4.72 - 4.80 (m, 0.7H), 6.50 (dd, J = 3, 9 Hz, 0.7H), 6.61 - 6.68 (m, 2.4H), 6.80 (d, J = 2 Hz, 0.3H), 6.91 (d, J = 8 Hz, 0.3H), 7.00 - 7.05 (m, 1H), 7.14 - 7.17 (m, 0.3H), 7.40 (ddd, J = 2, 8, 8 Hz, 0.7H), 7.59 (ddd, J = 2, 8, 8 Hz, 0.3H), 8.37 - 8.40 (m, 0.7H), 8.45 - 8.47 (m, 0.3H).

(Example 342)

Synthesis of 1-((5aS,6R,11bR)-14-(cyclopropylmethyl)-5a-hydroxy-10-methoxy-4,4-dimethyl-1,2,5,5a,6,7-hexahydro-6,11b-(epiminoethano)naphtho[1,2-d]azepin-3(4H)-yl)-2-(pyridin-2-yl)ethan-1-one

[Chemical Formula 418]

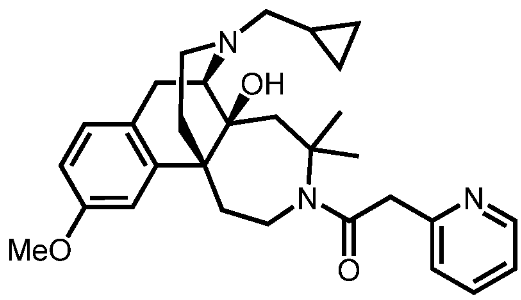

The title compound was obtained from the compound obtained in Example 268 and 2-(pyridin-2-yl)acetic acid hydrochloride according to the method described in Example 5.

$^1$H-NMR (400 MHz, $CDCl_3$)δ (ppm): 0.07 - 0.13 (m, 2H), 0.48 - 0.54 (m, 2H), 0.80 - 0.87 (m, 1H), 1.02 - 1.05 (m, 1H), 1.14 (s, 3H), 1.38 (d, J = 15 Hz, 1H), 1.91 (s, 3H), 1.95 - 1.98 (m, 2H), 2.11 (dd, J = 10, 15 Hz, 1H), 2.27 - 2.40 (m, 3H), 2.58 - 2.60 (m, 1H), 2.73 - 2.90 (m, 3H), 3.00 (d, J = 18 Hz, 1H), 3.72 - 3.78 (m, 1H), 3.78 (s, 3H), 3.88 (d, J = 15 Hz, 1H), 3.99 (d, J = 15 Hz, 1H), 4.46 (br s, 1H), 6.67 (d, J = 3 Hz, 1H), 6.72 (dd, J = 3, 8 Hz, 1H), 7.03 (d, J = 8 Hz, 1H), 7.16 (ddd, J = 1, 5, 8 Hz, 1H), 7.40 (d, J = 8 Hz, 1H), 7.64 (ddd, J = 2, 8, 8 Hz, 1H), 8.52 - 8.56 (m, 1H).

(Example 343)

Synthesis of 1-((5aS,6R,11bR)-14-(cyclopropylmethyl)-5a,10-dihydroxy-4,4-dimethyl-1,2,5,5a,6,7-hexahydro-6,11b-(epiminoethano)naphtho[1,2-d]azepin-3(4H)-yl)-2-(pyridin-2-yl)ethan-1-one

[Chemical Formula 419]

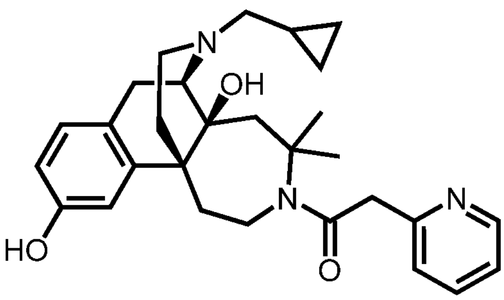

The title compound was obtained from the compound obtained in Example 342 according to the method described in Example 6.

$^1$H-NMR (400 MHz, $CDCl_3$)δ (ppm): 0.06 - 0.13 (m, 2H), 0.47 - 0.53 (m, 2H), 0.78 - 0.87 (m, 1H), 1.00 (s, 3H), 1.05 - 1.08 (m, 1H), 1.34 (d, J = 15 Hz, 1H), 1.89 (s, 3H), 1.90 - 2.08 (m, 4H), 2.29 (dd, J = 6, 12 Hz, 1H), 2.36 (dd, J = 6, 12 Hz, 1H), 2.42 (dd, J = 7, 15 Hz, 1H), 2.57 (dd, J = 4, 12 Hz, 1H), 2.71 (dd, J = 6, 18 Hz, 1H), 2.78 (d, J = 6 Hz, 1H), 2.86 (dd, J = 10, 16 Hz, 1H), 2.97 (d, J = 18 Hz, 1H), 3.82 (d, J = 14 Hz, 1H), 3.85 (dd, J = 7, 17 Hz, 1H), 4.12 (d, J = 14 Hz, 1H), 4.49 (br s, 1H), 6.67 (d, J = 2, 8 Hz, 1H), 6.80 (d, J = 2 Hz, 1H), 6.91 (d, J = 8 Hz, 1H), 7.22 (ddd, J = 1, 5, 8 Hz, 1H), 7.54 (d, J = 8 Hz, 1H), 7.71 (ddd, J = 2, 8, 8 Hz, 1H), 8.51 - 8.53 (m, 1H).

(Reference Example 59)

Synthesis of (4bR,8aS,9R)-11-(cyclopropylmethyl)-7-ethyl-8a-hydroxy-3-methoxy-6-oxo-6,7,8,8a,9,10-hexahydro-5H-9,4b-(epiminoethano)phenanthren-4-yl trifluoromethanesulfonate

[Chemical Formula 420]

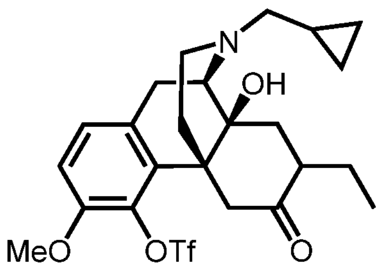

The title compound was obtained from the compound Z obtained in Reference Example 55 according to the method described in Reference Example 1.

$^1$H-NMR (400 MHz, $CDCl_3$)δ (ppm): 0.12 - 0.15 (m, 2H), 0.53 - 0.58 (m, 2H), 0.80 (t, J = 7 Hz, 3H), 0.83 - 1.00 (m, 2H), 1.39 (dd, J = 13, 13 Hz, 1H), 1.60 - 1.71 (m, 2H), 1.92 (dd, J = 6, 13 Hz, 1H), 2.08 (ddd, J = 3, 12, 12 Hz, 1H), 2.21 (ddd, J = 5, 13, 13 Hz, 1H), 2.38 - 2.40 (m, 2H), 2.68 - 2.75 (m, 2H), 2.86 (dd, J = 7, 19 Hz, 1H), 3.04 (d, J = 19 Hz, 1H), 3.13 - 3.17 (m, 2H), 3.50 (d, J = 14 Hz, 1H), 3.79 (s, 3H), 6.83 (d, J = 8 Hz, 1H), 7.03 (d, J = 8 Hz, 1H).

(Reference Example 60)

Synthesis of (4bR,8aS,9R)-11-(cyclopropylmethyl)-7-ethyl-8a-hydroxy-3-methoxy-8,8a,9,10-tetrahydro-5H-9,4b-(epiminoethano)phenanthren-6(7H)-one

[Chemical Formula 421]

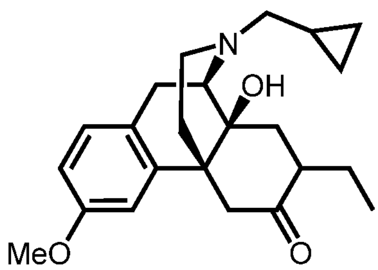

The title compound was obtained from the compound obtained in Reference Example 59 according to the method described in Reference Example 2.

$^1$H-NMR (400 MHz, $CDCl_3$)δ (ppm): 0.11 - 0.18 (m, 2H), 0.51 - 0.59 (m, 2H), 0.79 (t, J = 7 Hz, 3H), 0.84 - 1.04 (m, 2H), 1.17 - 1.21 (m, 1H), 1.48 (dd, J = 13, 13 Hz, 1H), 1.64 - 1.74 (m, 1H), 1.91 (dd, J = 6, 13 Hz, 1H), 2.06 - 2.21 (m, 2H), 2.41 - 2.42 (m, 2H), 2.58 - 2.63 (m, 1H), 2.68 - 2.78 (m, 2H), 2.81 (d, J = 14 Hz, 1H), 3.05 (d, J = 18 Hz, 1H), 3.09 (d, J = 14 Hz, 1H), 3.16 (d, J = 6 Hz, 1H), 3.77 (s, 3H), 6.68 (dd, J = 2, 8 Hz, 1H), 6.82 (d, J = 2 Hz, 1H), 6.97 (d, J = 8 Hz, 1H).

(Reference Example 61)

Synthesis of (4bR,8aS,9R)-11-(cyclopropylmethyl)-7-ethyl-8a-hydroxy-3-methoxy-8,8a,9,10-tetrahydro-5H-9,4b-(epiminoethano)phenanthren-6(7H)-one oxime

[Chemical Formula 422]

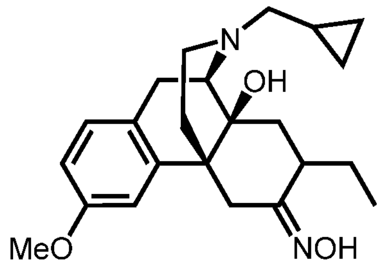

The title compound was obtained from the compound obtained in Reference Example 60 according to the method described in Reference Example 3.

$^1$H-NMR (400 MHz, $CDCl_3$)δ (ppm): 0.11 - 0.16 (m, 2H), 0.49 - 0.57 (m, 2H), 0.81 - 0.90 (m, 1H), 0.87 (t, J = 8 Hz, 3H), 0.99 - 1.10 (m, 1H), 1.15 - 1.18 (m, 1H), 1.30 (dd, J = 13, 13 Hz, 1H), 1.71 - 1.80 (m, 2H), 2.07 - 2.16 (m, 2H), 2.32 (d, J = 14 Hz, 1H), 2.37 - 2.39 (m, 2H), 2.55 - 2.67 (m, 2H), 2.72 (dd, J = 6, 18 Hz, 1H), 3.03 (d, J = 18 Hz, 1H), 3.05 (d, J = 6 Hz, 1H), 3.71 (s, 3H), 3.78 (d, J = 14 Hz, 1H), 6.68 (dd, J = 2, 8 Hz, 1H), 6.94 (d, J = 8 Hz, 1H), 7.12 (d, J = 2 Hz, 1H).

(Example 344)

Synthesis of (5aS,6R,11bR)-14-(cyclopropylmethyl)-4-ethyl-5a-hydroxy-10-methoxy-3,4,5,5a,6,7-hexahydro-6,11b-(epiminoethano)naphtho[1,2-d]azepin-2(1H)-one

[Chemical Formula 423]

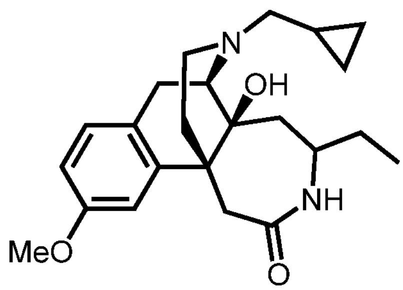

The title compound was obtained from the compound obtained in Reference Example 61 according to the method described in Example 1.

$^1$H-NMR (400 MHz, $CDCl_3$)δ (ppm): 0.10 - 0.13 (m, 2H), 0.50 - 0.55 (m, 2H), 0.80 - 0.86 (m, 1H), 0.94 (t, J = 7 Hz, 3H), 1.12 - 1.14 (m, 1H), 1.34 - 1.46 (m, 1H), 1.49 (d, J = 14 Hz, 1H), 1.57 - 1.66 (m, 2H), 2.02 - 2.11 (m, 2H), 2.34 - 2.36 (m, 2H), 2.56 - 2.63 (m, 2H), 2.75 (dd, J = 6, 18 Hz, 1H), 2.89 (d, J = 6 Hz, 1H), 3.01 (d, J = 18 Hz, 1H), 3.49 (d, J = 15 Hz, 1H), 3.81 (s, 3H), 3.82 - 3.89 (m, 1H), 5.22 (br s, 1H), 6.70 (dd, J = 2, 8 Hz, 1H), 6.96 (d, J = 8 Hz, 1H), 7.16 (d, J = 2 Hz, 1H).

(Example 345)

Synthesis of (5aS,6R, 11bS)-14-(cyclopropylmethyl)-4-ethyl-10-methoxy-2,3,4,5,6,7-hexahydro-6,11b-(epiminoethano)naphtho[1,2-d]azepin-5a(1H)-ol

[Chemical Formula 424]

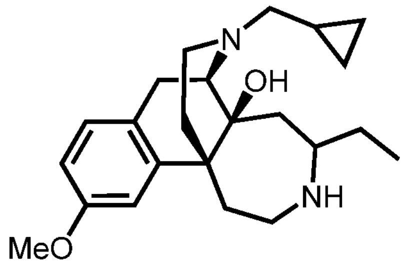

The title compound was obtained from the compound obtained in Example 344 according to the method described in Example 2.

$^1$H-NMR (400 MHz, $CDCl_3$)δ (ppm): 0.09 - 0.12 (m, 2H), 0.47 - 0.55 (m, 2H), 0.79 - 0.89 (m, 1H), 0.87 (t, J = 7 Hz, 3H), 1.04 - 1.08 (m, 1H), 1.24 - 1.35 (m, 3H), 1.48 - 1.61 (m, 2H), 1.95 - 2.08 (m, 3H), 2.31 - 2.39 (m, 3H), 2.49 - 2.58 (m, 1H), 2.81 (dd, J = 6, 18 Hz, 1H), 2.89 - 3.04 (m, 4H), 3.08 - 3.15 (m, 1H), 3.77 (s, 3H), 4.59 (br s, 1H), 6.70 - 6.73 (m, 2H), 7.02 (d, J = 8 Hz, 1H).

(Example 346)

Synthesis of 1-((5aS,6R,11bR)-14-(cyclopropylmethyl)-4-ethyl-5a-hydroxy-10-methoxy-1,2,5,5a,6,7-hexahydro-6,11b-(epiminoethano)naphtho[1,2-d]azepin-3(4H)-yl)-2-(pyridin-2-yl)ethan-1-one

[Chemical Formula 425]

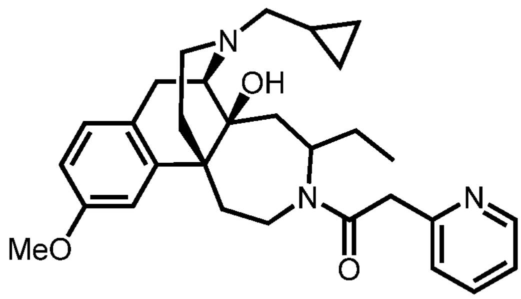

The title compound was obtained from the compound obtained in Example 345 and 2-(pyridin-2-yl)acetic acid hydrochloride according to the method described in Example 5.

$^1$H-NMR (400 MHz, $CDCl_3$)δ (ppm): 0.07 - 0.12 (m, 2H), 0.48 - 0.55 (m, 2H), 0.71 (t, J = 7 Hz, 1.5H), 0.80 (t, J = 7 Hz, 1.5H), 0.80 - 0.85 (m, 1H), 1.06 - 1.09 (m, 1H), 1.16 - 1.37 (m, 2.5H), 1.55 - 1.76 (m, 2.5H), 1.88 - 2.24 (m, 3.5H), 2.32 - 2.37 (m, 2.5H), 2.49 - 2.58 (m, 1.5H), 2.74 - 2.92 (m, 2.5H), 3.03 (dd, J = 6, 18 Hz, 1H), 3.78 (s, 1.5H), 3.79 (s, 1.5H), 3.79 - 3.86 (m, 0.5H), 3.91 - 4.16 (m, 3H), 4.58 - 4.66 (m, 0.5H), 6.68 - 6.77 (m, 2H), 7.05 (d, J = 8 Hz, 1H), 7.13 - 7.17 (m, 1H), 7.43 (d, J = 8 Hz, 0.5H), 7.47 (d, J = 8 Hz, 0.5H), 7.61 - 7.66 (m, 1H), 8.52 - 8.54 (m, 1H).

(Example 347)

Synthesis of 1-((5aS,6R,11bR)-14-(cyclopropylmethyl)-4-ethyl-5a,10-dihydroxy-1,2,5,5a,6,7-hexahydro-6,11b-(epiminoethano)naphtho[1,2-d]azepin-3(4H)-yl)-2-(pyridin-2-yl)ethan-1-one

[Chemical Formula 426]

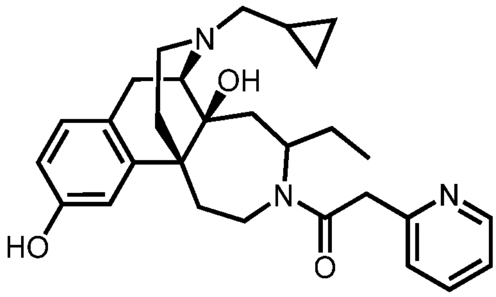

The title compound was obtained from the compound obtained in Example 346 according to the method described in Example 6.

$^1$H-NMR (400 MHz, $CDCl_3$)δ (ppm): 0.08 - 0.12 (m, 2H), 0.48 - 0.52 (m, 2H), 0.63 (t, J = 7 Hz, 0.9H), 0.78 (t, J = 7 Hz, 2.1H), 0.82 - 0.91 (m, 1H), 1.07 - 1.34 (m, 3H), 1.50 - 1.77 (m, 2H), 1.85 - 1.98 (m, 1H), 2.03 - 2.17 (m, 2H), 2.29 - 2.40 (m, 3H), 2.47 - 2.51 (m, 1H), 2.63 - 2.86 (m, 3H), 2.95 - 3.02 (m, 1H), 3.83 (d, J = 14 Hz, 0.3H), 3.87 (dd, J = 5, 16 Hz, 0.3H), 3.98 - 4.27 (m, 3.1H), 4.49 - 4.57 (m, 0.3H), 6.59 (dd, J = 2, 8 Hz, 0.3H), 6.64 (dd, J = 2, 8 Hz, 0.7H), 6.70 (d, J = 2 Hz, 0.3H), 6.89 (d, J = 8 Hz, 0.3H), 6.94 (d, J = 8 Hz, 0.7H), 7.00 (d, J = 2 Hz, 0.7H), 7.15 - 7.18 (m, 0.7H), 7.21 - 7.24 (m, 0.3H), 7.43 (d, J = 8 Hz, 0.7H), 7.54 (d, J = 8 Hz, 0.3H), 7.66 (ddd, J = 2, 8, 8 Hz, 0.7H), 7.71 (ddd, J = 2, 8, 8 Hz, 0.3H), 7.85 (br s, 0.7H), 8.04 (br s, 0.3H), 8.51 - 8.56 (m, 1H).

(Example 348)

Synthesis of 1-((5aS,6R,11bR)-9,11-dichloro-14-(cyclopropylmethyl)-5a,10-dihydroxy-1,2,5,5a,6,7-hexahydro-6,11b-(epiminoethano)naphtho[1,2-d]azepin-3(4H)-yl)-2-(pyridin-2-yl)ethan-1-one

[Chemical Formula 427]

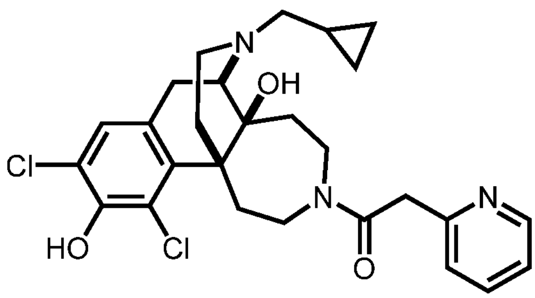

The title compound was obtained from the compound UV obtained in Example 325 according to the method described in Example 26.

$^1$H-NMR (400 MHz, $CDCl_3$)δ (ppm): 0.03 - 0.10 (m, 2H), 0.45 - 0.53 (m, 2H), 0.73 - 0.85 (m, 1H), 1.43 - 1.58 (m, 1H), 1.61 - 1.97 (m, 3H), 2.04 - 2.34 (m, 4H), 2.38 - 2.91 (m, 5.7H), 3.07 - 3.20 (m, 0.3H), 3.33 - 3.49 (m, 2H), 3.53 - 3.74 (m, 1.7H), 3.79 - 3.94 (m, 0.3H), 4.08 - 4.17 (m, 0.3H), 4.44 (br s, 1H), 4.60 - 4.75 (m, 0.7H), 6.69 (s, 0.7H), 6.74 (s, 0.3H), 6.79 - 7.02 (m, 1.7H), 7.09 - 7.15 (m, 0.3H), 7.36 - 7.45 (m, 0.7H), 7.50 - 7.57 (m, 0.3H), 8.26 - 8.32 (m, 1H).

(Example 349)

Synthesis of (5aS,6R,11bR)-11-(benzyloxy)-14-(cyclopropylmethyl)-5a-hydroxy-10-(methoxymethoxy)-3,4,5,5a,6,7-hexahydro-6,11b-(epiminoethano)naphtho[1,2-d]azepin-2(1H)-one

[Chemical Formula 428]

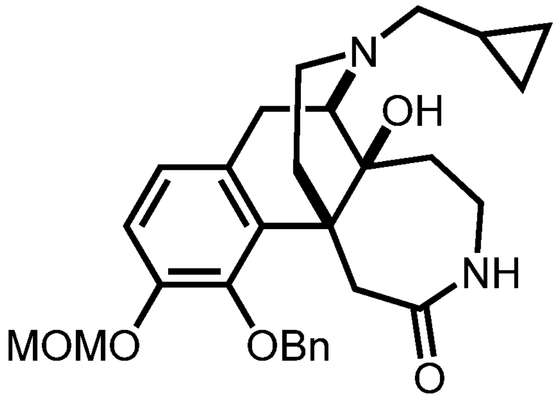

The title compound was obtained from the compound obtained in Example 330 and benzyl bromide according to the method described in Reference Example 35.

$^{1}$H-NMR (400 MHz, $CDCl_3$)δ (ppm): 0.06 - 0.13 (m, 2H), 0.47 - 0.54 (m, 2H), 0.74 - 0.86 (m, 1H), 1.36 - 1.62 (m, 3H), 1.82 - 2.04 (m, 3H), 2.25 - 2.38 (m, 2H), 2.52 - 2.64 (m, 1H), 2.75 - 2.97 (m, 3H), 3.16 - 3.28 (m, 2H), 3.43 (s, 3H), 3.72 - 3.83 (m, 1H), 4.98 (d, J = 12 Hz, 1H), 4.99 (d, J = 6 Hz, 1H), 5.08 (d, J = 6 Hz, 1H), 5.42 - 5.49 (m, 1H), 5.56 (d, J = 12 Hz, 1H), 6.72 (d, J = 8 Hz, 1H), 6.97 (d, J = 9 Hz, 1H), 7.22 - 7.40 (m, 3H), 7.55 - 7.60 (m, 2H).

(Example 350)

Synthesis of (5aS,6R,11bS)-11-(benzyloxy)-14-(cyclopropylmethyl)-2,3,4,5,6,7-hexahydro-6,11b-(epiminoethano)naphtho[1,2-d]azepine-5a,10(1H)-diol

[Chemical Formula 429]

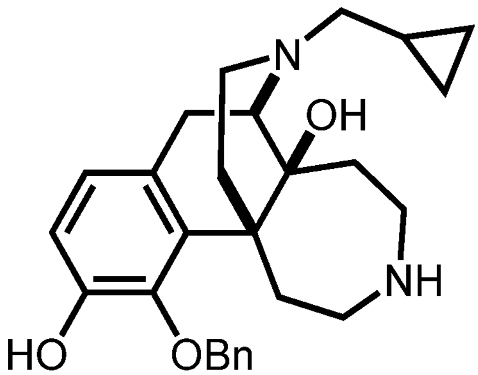

The title compound was obtained from the compound obtained in Example 349 according to the method described in Example 2.

$^{1}$H-NMR (400 MHz, $CDCl_3$)δ (ppm): 0.06 - 0.12 (m, 2H), 0.46 - 0.53 (m, 2H), 0.74 - 0.86 (m, 1H), 1.23 - 1.32 (m, 1H), 1.45 - 1.55 (m, 1H), 1.57 - 1.69 (m, 1H), 1.95 (ddd, J = 5, 12, 12 Hz, 1H), 2.04 - 2.22 (m, 2H), 2.26 - 2.38 (m, 3H), 2.54 (dd, J = 3, 12 Hz, 1H), 2.67 - 2.95 (m, 6H), 3.04 (dd, J = 12, 12 Hz, 1H), 4.69 (d, J = 11 Hz, 1H), 5.05 (d, J = 11 Hz, 1H), 6.68 (d, J = 8 Hz, 1H), 6.72 (d, J = 8 Hz, 1H), 7.32 - 7.44 (m, 5H).

(Example 351)

Synthesis of (5aS,6R,11bR)-11-(benzyloxy)-14-(cyclopropylmethyl)-5a-hydroxy-3-(2-(pyridin-2-yl)acetyl)-1,2,3,4,5,5a,6,7-octahydro-6,11b-(epiminoethano)naphtho[1,2-d]azepin-10-yl trifluoromethanesulfonate

[Chemical Formula 430]

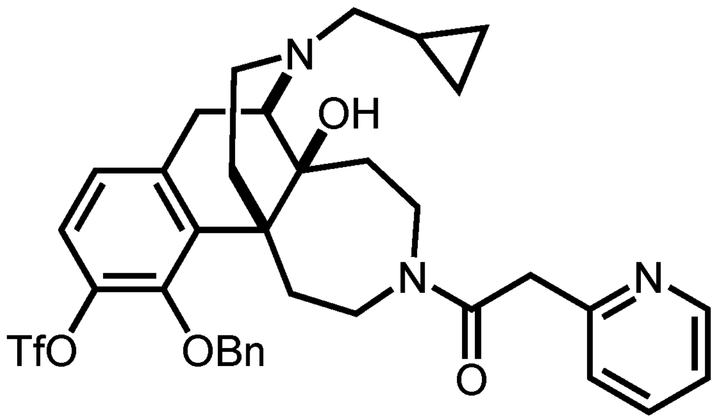

To the compound (43.5 mg, 0.10 mmol) obtained in Example 350, 2-(pyridin-2-yl)acetic acid hydrochloride (20.8 mg, 0.12 mmol), and O-(7-azabenzotriazol-1-yl)-N,N,N',N'-tetramethyluronium hexafluorophosphate (45.6 mg, 0.12 mmol) dissolved in N,N-dimethylformamide (1 mL) was added N,N-diisopropylethylamine (52.3 μL, 0.30 mmol), followed by stirring at room temperature for 1 hour. To the reaction mixture, a saturated sodium bicarbonate aqueous solution was added, followed by extraction three times with chloroform. The combined extracts were dried over sodium sulfate and then concentrated under reduced pressure. The obtained crude product was used for the next reaction without purification.

To a solution of the obtained crude product in N,N-dimethylformamide (1 mL) were added potassium carbonate (55.3 mg, 0.40 mmol) and N-phenylbis (trifluoromethanesulfonimide) (143 mg, 0.40 mmol), followed by stirring at room temperature for 1 hour. To the reaction mixture, a saturated sodium bicarbonate aqueous solution was added, followed by extraction three times with chloroform. The organic layers were combined, dried over sodium sulfate, and then concentrated under reduced pressure. The obtained crude product was purified by silica gel column chromatography (amino group-supported silica gel, 0 to 30% methanol/chloroform) to yield the title compound (56.1 mg, 82%) as a colorless amorphous form.

$^1$H-NMR (400 MHz, $CDCl_3$)δ (ppm): 0.06 - 0.15 (m, 2H), 0.46 - 0.56 (m, 2H), 0.75 - 0.90 (m, 1H), 1.20 - 1.30 (m, 1H), 1.45 - 1.68 (m, 2H), 1.90 - 2.23 (m, 3H), 2.25 - 2.42 (m, 2H), 2.43 - 2.55 (m, 1H), 2.56 - 2.70 (m, 1H), 2.71 - 3.46 (m, 5H), 3.50 - 3.90 (m, 3H), 3.92 - 4.02 (m, 0.3H), 4.15 - 4.24 (m, 0.7H), 4.65 (d, J = 11 Hz, 0.7H), 4.74 (d, J = 11 Hz, 0.3H), 5.26 (d, J = 11 Hz, 0.3H), 5.37 (d, J = 11 Hz, 0.7H), 6.90 (d, J = 9 Hz, 0.3H), 6.97 (d, J = 9 Hz, 0.7H), 7.06 - 7.20 (m, 3H), 7.30 - 7.44 (m, 5H), 7.53 (ddd, J = 2, 8, 8 Hz, 0.3H), 7.55 (ddd, J = 2, 8, 8 Hz, 0.7H), 8.44 - 8.51 (m, 1H).

(Example 352)

Synthesis of (5aS,6R,11bR)-11-(benzyloxy)-14-(cyclopropylmethyl)-5a-hydroxy-3-(2-(pyridin-2-yl)acetyl)-1,2,3,4,5,5a,6,7-octahydro-6,11b-(epiminoethano)naphtho[1,2-d]azepine-10-carbonitrile

[Chemical Formula 431]

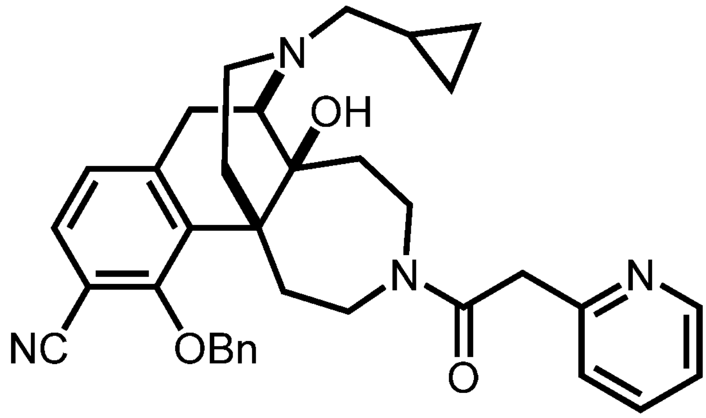

The title compound was obtained from the compound obtained in Example 351 according to the method described in Example 241.

$^1$H-NMR (400 MHz, $CDCl_3$)δ (ppm): 0.06 - 0.14 (m, 2H), 0.46 - 0.56 (m, 2H), 0.75 - 0.92 (m, 1H), 1.20 - 4.40 (m, 19H), 5.01 (d, J = 11 Hz, 0.7H), 5.12 (d, J = 11 Hz, 0.3H), 5.52 (d, J = 11 Hz, 0.3H), 5.54 (d, J = 11 Hz, 0.7H), 6.85 (d, J = 8 Hz, 0.3H), 6.93 (d, J = 8 Hz, 0.7H), 7.00 - 7.22 (m, 2H), 7.32 - 7.60 (m, 7H), 8.42 - 8.54 (m, 1H).

(Example 353)

Synthesis of (5aS,6R,11bR)-14-(cyclopropylmethyl)-5a,11-dihydroxy-3-(2-(pyridin-2-yl)acetyl)-1,2,3,4,5,5a,6,7-octahydro-6,11b-(epiminoethano)naphtho[1,2-d]azepine-10-carboxamide

[Chemical Formula 432]

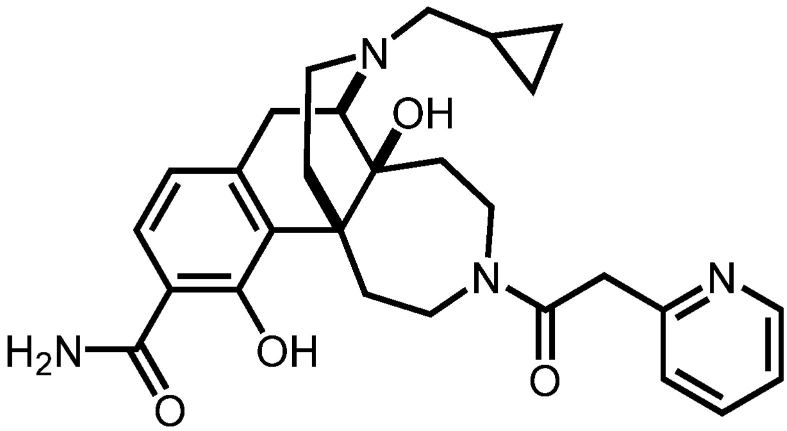

To the compound (5.0 mg, 0.0089 mmol) obtained in Example 352, palladium (2.0 mg, 0.0089 mmol) acetate, and triphenylphosphine (2.3 mg, 0.0089 mmol) dissolved in toluene (200 μL) was added acetaldoxime (1.6 μL, 0.0267 mmol), followed by stirring at 80°C for 2 hours. After allowed to cool, to the reaction mixture was added a 28% aqueous ammonia solution, followed by extraction three times with chloroform. The organic layers were combined, dried over sodium sulfate, and then concentrated under reduced pressure. The obtained crude product was used for the next reaction without purification.

To a solution of the obtained crude product in methanol (0.5 mL) were added 10% palladium-activated carbon (15.0 mg) and potassium formate (15.0 mg, 0.178 mmol), followed by stirring at room temperature for 30 minutes. The reaction mixture was filtered through celite, and to the filtrate, a 28% aqueous ammonia solution was added, followed by extraction three times with chloroform. The organic layers were combined, dried over sodium sulfate, and then concentrated under reduced pressure. The obtained crude product was purified by preparative thin layer chromatography

(chloroform : 2 M ammonia-methanol solution = 10 : 1) to yield the title compound (1.7 mg, 39%) as a colorless amorphous form.

$^1$H-NMR (400 MHz, $CDCl_3$)$\delta$ (ppm): 0.03 - 0.15 (m, 2H), 0.45 - 0.56 (m, 2H), 0.75 - 0.92 (m, 1H), 1.01 - 2.08 (m, 6H), 2.20 - 3.00 (m, 8H), 3.20 - 4.85 (m, 5.3H), 4.10 - 4.61 (m, 1.7H), 6.54 (d, J = 8 Hz, 0.3H), 6.60 (d, J = 8 Hz, 0.7H), 6.98 - 7.21 (m, 3H), 7.35 - 7.79 (m, 2H), 8.40 - 8.48 (m, 1H).

(Example 354)

Synthesis of 1-((5aS,6R,11bR)-14-(cyclopropylmethyl)-5a-hydroxy-10-methoxy-9-methyl-1,2,5,5a,6,7-hexahydro-6,11b-(epiminoethano)naphtho[1,2-d]azepin-3(4H)-yl)-2-(4-(trifluoromethyl)pyridin-2-yl)ethan-1-one

[Chemical Formula 433]

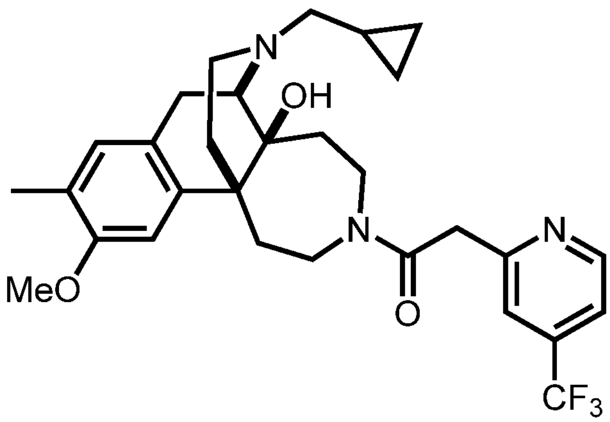

The title compound was obtained from the compound obtained in Example 261 and 2-(4-(trifluoromethyl)pyridin-2-yl)acetic acid according to the method described in Example 5.

$^1$H-NMR (400 MHz, $CDCl_3$)$\delta$ (ppm): 0.04 - 0.17 (m, 2H), 0.44 - 0.56 (m, 2H), 0.75 - 0.91 (m, 1H), 1.01 - 1.11 (m, 1H), 1.59 (ddd, J = 4, 4, 15 Hz, 0.5H), 1.60 - 1.67 (m, 0.5H), 1.83 (ddd, J = 3, 12, 15 Hz, 0.5H), 1.89 - 2.11 (m, 3H), 2.15 (s, 1.5H), 2.15 (s, 1.5H), 2.17 - 2.25 (m, 1H), 2.26 - 2.40 (m, 2H), 2.50 - 2.60 (m, 1.5H), 2.72 (dd, J = 6, 18 Hz, 0.5H), 2.77 (dd, J = 6, 18 Hz, 0.5H), 2.87 (d, J = 6 Hz, 0.5H), 2.90 (d, J = 18 Hz, 0.5H), 2.91 (d, J = 6 Hz, 0.5H), 2.97 (d, J = 18 Hz, 0.5H), 3.11 (ddd, J = 2, 10, 14 Hz, 0.5H), 3.46 (ddd, J = 4, 4, 13 Hz, 0.5H), 3.52 (ddd, J = 2, 12, 14 Hz, 0.5H), 3.62 (ddd, J = 4, 4, 14 Hz, 0.5H), 3.67 - 3.98 (m, 1.5H), 3.73 (d, J = 15 Hz, 0.5H), 3.78 (s, 1.5H), 3.80 (s, 1.5H), 3.84 (d, J = 15 Hz, 0.5H), 3.93 (d, J = 15 Hz, 0.5H), 3.99 (d, J = 15 Hz, 0.5H), 4.20 (ddd, J = 2, 6, 14 Hz, 0.5H), 6.49 (s, 0.5H), 6.55 (s, 0.5H), 6.85 (s, 1H), 7.32 - 7.38 (m, 1.5H), 7.56 - 7.59 (m, 0.5H), 8.64 (d, J = 5 Hz, 0.5H), 8.68 (d, J = 5 Hz, 0.5H).

(Example 355)

Synthesis of 1-((5aS,6R,11bR)-14-(cyclopropylmethyl)-5a,10-dihydroxy-9-methyl-1,2,5,5a,6,7-hexahydro-6,11b-(epiminoethano)naphtho[1,2-d]azepin-3(4H)-yl)-2-(4-(trifluoromethyl)pyridin-2-yl)ethan-1-one

[Chemical Formula 434]

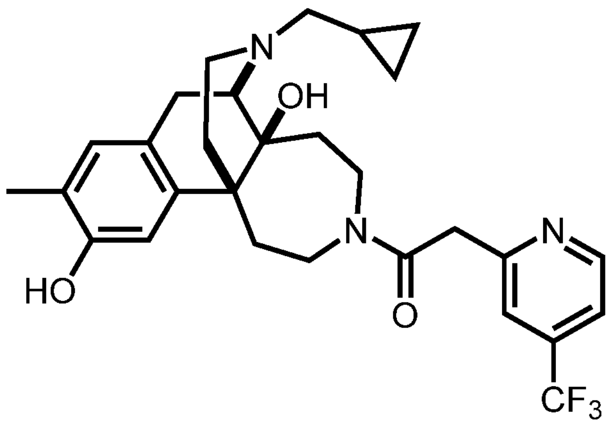

The title compound was obtained from the compound obtained in Example 354 according to the method described in Example 6.

$^1$H-NMR (400 MHz, $CDCl_3$)δ (ppm): 0.05 - 0.16 (m, 2H), 0.43 - 0.56 (m, 2H), 0.75 - 0.88 (m, 1H), 1.01 - 1.15 (m, 1H), 1.51 - 1.63 (m, 1H), 1.77 - 1.86 (m, 1H), 1.87 - 1.96 (m, 0.3H), 1.92 (ddd, J = 4, 13, 13 Hz, 0.7H), 1.98 - 2.19 (m, 2H), 2.10 (s, 2.1H), 2.17 (s, 0.9H), 2.23 - 2.39 (m, 2.7H), 2.49 - 2.62 (m, 1.3H), 2.70 (dd, J = 6, 18 Hz, 0.7H), 2.71 - 2.78 (m, 0.3H), 2.84 (d, J = 6 Hz, 0.7H), 2.90 (d, J = 6 Hz, 0.3H), 2.91 (d, J = 18 Hz, 0.3H), 2.94 - 3.02 (m, 0.7H), 2.96 (d, J = 18 Hz, 0.7H), 3.32 - 3.48 (m, 1.3H), 3.77 - 3.86 (m, 0.3H), 3.78 (d, J = 15 Hz, 0.3H), 3.81 (d, J = 15 Hz, 0.3H), 3.89 (ddd, J = 3, 5, 14 Hz, 0.3H), 3.97 (ddd, J = 4, 13, 13 Hz, 0.7H), 4.01 (d, J = 15 Hz, 0.7H), 4.03 (d, J = 15 Hz, 0.7H), 4.27 - 4.34 (m, 0.7H), 4.42 (br s, 0.7H), 4.52 (br s, 0.3H), 6.63 (s, 0.3H), 6.79 (s, 1.4H), 6.81 (s, 0.3H), 7.35 - 7.43 (m, 1.3H), 7.58 - 7.61 (m, 0.7H), 8.64 (d, J = 5 Hz, 0.3H), 8.70 (d, J = 5 Hz, 0.7H).

(Example 356)

Synthesis of 1-((5aS,6R,11bR)-14-(cyclopropylmethyl)-5a-hydroxy-10-methoxy-9-methyl-1,2,5,5a,6,7-hexahydro-6,11b-(epiminoethano)naphtho[1,2-d]azepin-3(4H)-yl)-2-(1H-indazol-3-yl)ethan-1-one

[Chemical Formula 435]

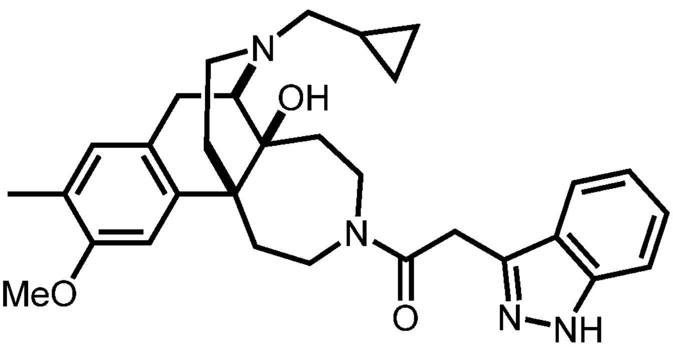

The title compound was obtained from the compound obtained in Example 261 and 2-(1H-indazol-3-yl)acetic acid according to the method described in Example 5.

$^1$H-NMR (400 MHz, $CDCl_3$)δ (ppm): 0.03 - 0.15 (m, 2H), 0.44 - 0.57 (m, 2H), 0.75 - 0.91 (m, 1H), 0.99 - 1.11 (m, 1H), 1.50 - 1.64 (m, 1H), 1.76 (ddd, J = 3, 9, 12 Hz, 0.5H), 1.80 (ddd, J = 3, 8, 11 Hz, 0.5H), 1.91 - 2.19 (m, 3H), 2.15 (s, 1.5H), 2.16 (s, 1.5H), 2.26 - 2.35 (m, 1.5H), 2.28 (dd, J = 6, 13 Hz, 0.5H), 2.35 (dd, J = 6, 13 Hz, 0.5H), 2.45 - 2.56 (m, 1.5H), 2.64 (dd, J = 6, 18 Hz, 0.5H), 2.73 (dd, J = 6, 18 Hz, 0.5H), 2.85 (d, J = 6 Hz, 0.5H), 2.88 (d, J = 18 Hz, 0.5H), 2.89 (d, J = 6 Hz, 0.5H), 2.92 (d, J = 18 Hz, 0.5H), 3.27 (ddd, J = 2, 12, 14 Hz, 0.5H), 3.44 - 3.56 (m, 1H), 3.60 (ddd, J = 4, 4, 14 Hz, 0.5H), 3.72 - 3.84 (m, 0.5H), 3.77 (s, 1.5H), 3.81 (s, 1.5H), 3.85 - 3.98 (m, 1H), 3.85 (d, J = 15 Hz, 0.5H), 3.92 (d, J = 15 Hz, 0.5H), 4.03 (d, J = 16 Hz, 0.5H), 4.06 (d, J = 16 Hz, 0.5H), 4.07 - 4.14 (m, 0.5H), 4.50 (br s, 1H), 6.52 (s, 0.5H), 6.53 (s, 0.5H), 6.79 (s, 0.5H), 6.81 (s, 0.5H), 7.06 (ddd, J = 1, 6, 6 Hz, 0.5H), 7.07 (ddd, J = 1, 6, 6 Hz, 0.5H), 7.27 - 7.37 (m, 2H), 7.62 - 7.69 (m, 1H), 10.1 (br s, 1H).

(Example 357)

Synthesis of 1-((5aS,6R,11bR)-14-(cyclopropylmethyl)-5a, 10-dihydroxy-9-methyl-1,2,5,5a,6,7-hexahydro-6,11b-(epiminoethano)naphtho[1,2-d]azepin-3(4H)-yl)-2-(1H-indazol-3-yl)ethan-1-one

[Chemical Formula 436]

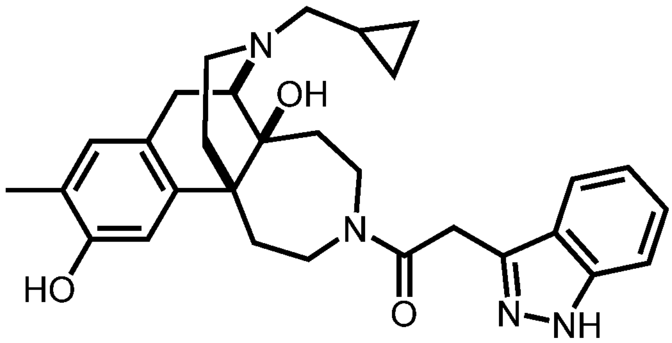

The title compound was obtained from the compound obtained in Example 356 according to the method described in Example 6.

$^1$H-NMR (400 MHz, $CDCl_3$)δ (ppm): 0.03 - 0.16 (m, 2H), 0.40 - 0.56 (m, 2H), 0.73 - 1.00 (m, 2H), 1.47 - 2.06 (m, 5.5H), 2.17 (s, 1.5H), 2.21 (s, 1.5H), 2.22 - 2.40 (m, 1H), 2.26 (dd, J = 7, 13 Hz, 0.5H), 2.31 (d, J = 6 Hz, 1H), 2.33 (dd, J = 7, 13 Hz, 0.5H), 2.42 - 2.52 (m, 1H), 2.59 (dd, J = 6, 18 Hz, 0.5H), 2.68 (dd, J = 6, 18 Hz, 0.5H), 2.81 - 2.89 (m, 1H), 2.83 (d, J = 6 Hz, 0.5H), 2.90 (d, J = 18 Hz, 0.5H), 2.97 - 3.10 (m, 0.5H), 3.13 - 3.27 (m, 0.5H), 3.40 - 3.59 (m, 1H), 3.70 - 4.00 (m, 3.5H), 4.54 (br s, 1H), 6.63 (s, 0.5H), 6.69 (s, 0.5H), 6.74 (s, 0.5H), 6.76 (s, 0.5H), 6.93 - 7.06 (m, 1H), 7.16 - 7.35 (m, 2H), 7.49 - 7.57 (m, 0.5H), 7.60 - 7.69 (m, 0.5H).

(Example 358)

Synthesis of 1-((5aS,6R,11bR)-14-(cyclopropylmethyl)-5a-hydroxy-10-methoxy-9-methyl-1,2,5,5a,6,7-hexahydro-6,11b-(epiminoethano)naphtho[1,2-d]azepin-3(4H)-yl)-2-(3-fluoropyridin-2-yl)ethan-1-one

[Chemical Formula 437]

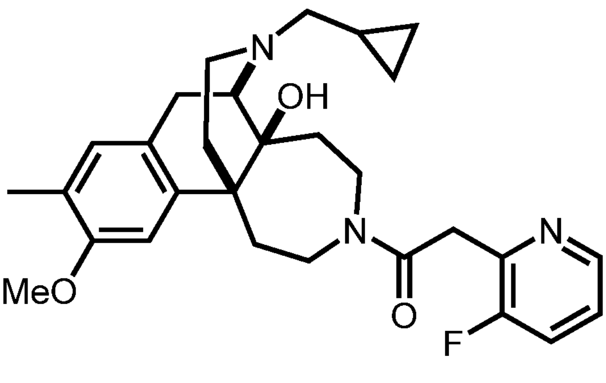

The title compound was obtained from the compound obtained in Example 261 and 2-(3-fluoropyridin-2-yl)acetic acid according to the method described in Example 5.

$^1$H-NMR (400 MHz, $CDCl_3$)δ (ppm): 0.05 - 0.17 (m, 2H), 0.46 - 0.54 (m, 2H), 0.76 - 0.88 (m, 1H), 1.03 - 1.11 (m, 1H), 1.56 - 1.65 (m, 1H), 1.84 (ddd, J = 2, 12, 15 Hz, 0.4H), 1.91 - 2.39 (m, 5.4H), 2.16 (s, 1.8H), 2.18 (s, 1.2H), 2.37 (dd, J = 7, 13 Hz, 0.6H), 2.50 - 2.59 (m, 0.8H), 2.61 (ddd, J = 4, 12, 16 Hz, 0.6H), 2.73 - 2.82 (m, 0.4H), 2.74 (dd,

J = 6, 18 Hz, 0.6H), 2.89 (d, J = 6 Hz, 0.6H), 2.91 (d, J = 6 Hz, 0.4H), 2.91 (d, J = 18 Hz, 0.4H), 2.98 (d, J = 18 Hz, 0.6H), 3.04 - 3.13 (m, 0.6H), 3.44 (ddd, J = 4, 4, 13 Hz, 0.6H), 3.45 - 3.54 (m, 0.4H), 3.57 (ddd, J = 4, 4, 14 Hz, 0.4H), 3.67 (dd, J = 2, 16 Hz, 0.4H), 3.68 - 3.82 (m, 0.6H), 3.77 (s, 1.8H), 3.80 (s, 1.2H), 3.81 (dd, J = 2, 16 Hz, 0.4H), 3.88 - 4.01 (m, 1H), 3.89 (dd, J = 2, 16 Hz, 0.6H), 3.98 (dd, J = 2, 16 Hz, 0.6H), 4.24 (ddd, J = 2, 6, 15 Hz, 0.6H), 6.52 (s, 0.4H), 6.55 (s, 0.6H), 6.86 (s, 1H), 7.16 (ddd, J = 4, 4, 9 Hz, 0.4H), 7.19 (ddd, J = 4, 4, 8 Hz, 0.6H), 7.32 (ddd, J = 1, 9, 10 Hz, 0.4H), 7.34 (ddd, J = 1, 8, 10 Hz, 0.6H), 8.29 (ddd, J = 1, 1, 4 Hz, 0.4H), 8.33 (ddd, J = 1, 1, 4 Hz, 0.6H).

(Example 359)

Synthesis of 1-((5aS,6R,11bR)-14-(cyclopropylmethyl)-5a, 10-dihydroxy-9-methyl-1,2,5,5a,6,7-hexahydro-6,11b-(epiminoethano)naphtho[1,2-d]azepin-3(4H)-yl)-2-(3-fluoropyridin-2-yl)ethan-1-one

[Chemical Formula 438]

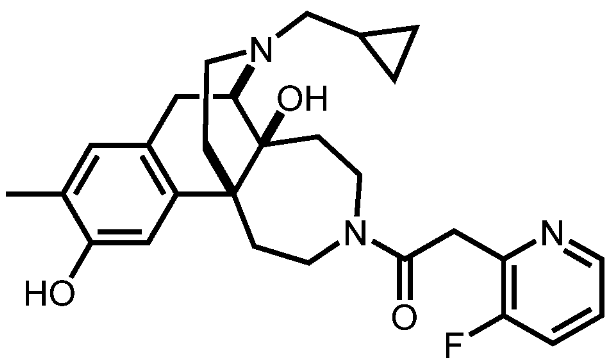

The title compound was obtained from the compound obtained in Example 358 according to the method described in Example 6.

$^1$H-NMR (400 MHz, $CDCl_3$)$\delta$ (ppm): 0.03 - 0.16 (m, 2H), 0.44 - 0.57 (m, 2H), 0.76 - 0.90 (m, 1H), 1.01 - 1.11 (m, 1H), 1.52 - 1.64 (m, 1H), 1.79 - 2.07 (m, 2.7H), 2.08 - 2.26 (m, 2H), 2.09 (s, 2.1H), 2.19 (s, 0.9H), 2.29 (dd, J = 6, 12 Hz, 0.3H), 2.32 - 2.39 (m, 0.3H), 2.34 (d, J = 6 Hz, 1.4H), 2.48 - 2.56 (m, 1H), 2.59 (ddd, J = 5, 11, 16 Hz, 0.3H), 2.70 (dd, J = 6, 18 Hz, 0.7H), 2.74 (dd, J = 6, 18 Hz, 0.3H), 2.86 (d, J = 6 Hz, 0.7H), 2.90 (d, J = 6 Hz, 0.3H), 2.90 (d, J = 18 Hz, 0.3H), 2.94 - 3.03 (m, 0.7H), 2.95 (d, J = 18 Hz, 0.7H), 3.30 - 3.47 (m, 1.3H), 3.74 (dd, J = 2, 16 Hz, 0.3H), 3.78 (dd, J = 2, 16 Hz, 0.3H), 3.79 - 3.92 (m, 1H), 3.93 - 3.99 (m, 0.3H), 3.94 (dd, J = 2, 16 Hz, 0.7H), 4.01 (dd, J = 2, 16 Hz, 0.7H), 4.30 (ddd, J = 2, 6, 14 Hz, 0.7H), 4.48 (br s, 1H), 6.64 (s, 0.3H), 6.75 (s, 0.7H), 6.78 (s, 0.7H), 6.82 (s, 0.3H), 7.19 (ddd, J = 4, 4, 8 Hz, 0.3H), 7.21 (ddd, J = 4, 4, 8 Hz, 0.7H), 7.34 (ddd, J = 1, 8, 10 Hz, 0.3H), 7.37 (ddd, J = 1, 8, 10 Hz, 0.7H), 8.29 (ddd, J = 1, 1, 4 Hz, 0.3H), 8.35 (ddd, J = 1, 1, 4 Hz, 0.7H).

(Example 360)

Synthesis of (5aS,6R,11bS)-14-(cyclopropylmethyl)-5a-hydroxy-N-methyl-3-(2-(4-methyl-1H-pyrazol-1-yl)ethyl)-1,2,3,4,5,5a,6,7-octahydro-6,11b-(epiminoethano)naphtho[1,2-d]azepine-10-carboxamide

[Chemical Formula 439]

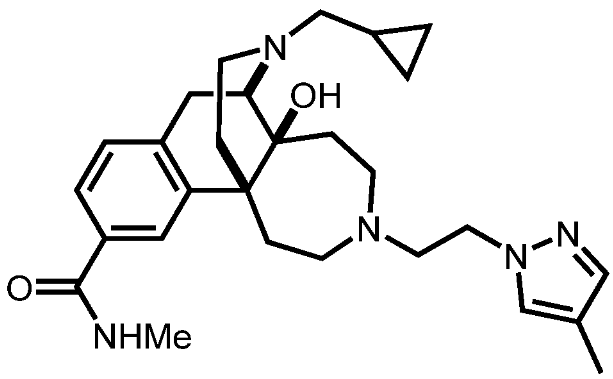

The title compound was obtained from the compound obtained in Example 334 according to the methods described in Example 243 and Example 31.

$^1$H-NMR (400 MHz, $CDCl_3$)δ (ppm): 0.05 - 0.20 (m, 2H), 0.45 - 0.60 (m, 2H), 0.80 - 1.00 (m, 2H), 1.00 - 1.10 (m, 1H), 1.20 - 1.40 (m, 1H), 1.40 - 1.55 (m, 1H), 1.55 - 2.20 (m, 7H), 2.20 - 2.65 (m, 5H), 2.65 - 2.80 (m, 1H), 2.80 - 3.15 (m, 7H), 3.15 - 3.30 (m, 1H), 3.90 - 4.10 (m, 2H), 6.20 - 6.40 (m, 1H), 6.80 (s, 1H), 7.17 (d, J = 8 Hz, 1H), 7.21 (s, 1H), 7.43 (dd, J = 2, 8 Hz, 1H), 7.60 (d, J = 2 Hz, 1H).

(Example 361)

Synthesis of phenyl (5aS,6R,11bR)-14-(cyclopropylmethyl)-5a, 10-dihydroxy-1,2,5,5a,6,7-hexahydro-6,11b-(epiminoethano)naphtho[1,2-d]azepine-3(4H)-carboxylate

[Chemical Formula 440]

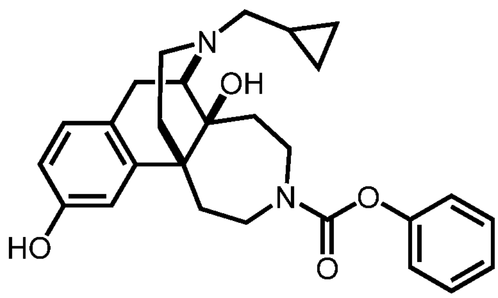

To the compound E (10 mg, 0.030 mmol) obtained in Example 3 dissolved in tetrahydrofuran (2 mL) were added triethylamine (20 μL, 0.14 mmol) and phenyl chloroformate (14 μL, 0.11 mmol), followed by stirring at room temperature for 30 minutes. Thereafter, to the reaction solution, a 2 M aqueous sodium hydroxide solution (1 mL) was added, followed by further stirring at room temperature for 3 hours. To the reaction solution, a saturated ammonium chloride aqueous solution was added, followed by extraction three times with chloroform. The organic layer was dried over sodium sulfate and concentrated under reduced pressure. The obtained crude product was purified by silica gel column chromatography (amino group-supported silica gel, 40 to 100% ethyl acetate/hexane) to yield the title compound (12.2 mg, 89%) as a colorless amorphous form.

$^1$H-NMR (400 MHz, $CDCl_3$)δ (ppm): 0.06 - 0.16 (m, 2H), 0.48 - 0.55 (m, 2H), 0.77 - 0.88 (m, 1H), 1.02 - 1.11 (m, 1H), 1.56 - 1.66 (m, 1H), 1.86 - 2.12 (m, 4H), 2.28 - 2.49 (m, 2.5H), 2.51 - 2.64 (m, 1.5H), 2.76 - 2.85 (m, 1H), 2.91 - 3.03 (m, 2H), 3.50 - 3.74 (m, 3H), 3.76 - 3.96 (m, 1H), 6.60 - 6.68 (m, 2H), 6.85 - 6.91 (m, 2H), 6.92 - 6.98 (m, 1H), 7.09 - 7.17 (m, 1H), 7.24 - 7.32 (m, 2H).

(Example 362)

Synthesis of (5aS,6R,11bR)-N-(4-chlorophenyl)-14-(cyclopropylmethyl)-5a,10-dihydroxy-1,2,5,5a,6,7-hexahydro-6,11b-(epiminoethano)naphtho[1,2-d]azepine-3(4H)-carboxamide

[Chemical Formula 441]

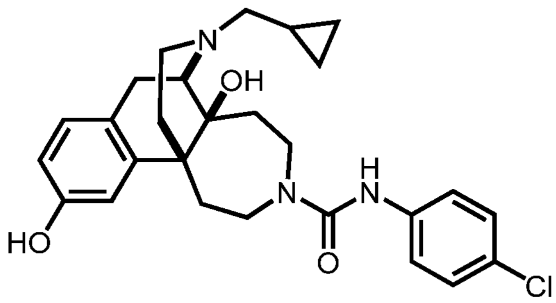

To the compound E (30 mg, 0.091 mmol) obtained in Example 3 dissolved in chloroform (2 mL) were added triethylamine (25 μL, 0.18 mmol) and 4-chlorophenyl isocyanate (28 mg, 0.18 mmol), followed by stirring at room temperature for 1 hour. To the reaction solution, a saturated sodium bicarbonate aqueous solution was added, followed by extraction three times with chloroform. The organic layer was dried over sodium sulfate and concentrated under reduced pressure. To the obtained concentrated residue dissolved in tetrahydrofuran (3 mL), a 2 M aqueous sodium hydroxide solution (1 mL) was added, followed by stirring at room temperature for 16 hours. To the reaction solution, a saturated ammonium chloride aqueous solution was added, followed by extraction with chloroform. The organic layer was dried over sodium sulfate and concentrated under reduced pressure. The obtained crude product was purified by preparative thin layer chromatography (chloroform : 10% aqueous ammonia-methanol solution = 12 : 1) to yield the title compound (36.4 mg, 83%) as a colorless amorphous form.

$^1$H-NMR (400 MHz, $CDCl_3$)δ (ppm): 0.05 - 0.14 (m, 2H), 0.46 - 0.55 (m, 2H), 0.75 - 0.87 (m, 1H), 0.97 - 1.05 (m, 1H), 1.53 - 1.62 (m, 1H), 1.85 - 2.07 (m, 4H), 2.16 - 2.40 (m, 3H), 2.47 - 2.57 (m, 1H), 2.69 - 2.79 (m, 1H), 2.85 - 3.00 (m, 2H), 3.11 - 3.31 (m, 1H), 3.38 - 3.55 (m, 1H), 3.60 - 3.92 (m, 2H), 6.49 (br s, 1H), 6.58 - 6.68 (m, 2H), 6.89 (d, J = 8 Hz, 1H), 7.11 - 7.20 (m, 4H).

(Example 363)

Synthesis of (5aS,6R,11bR)-N-(4-chlorophenyl)-14-(cyclopropylmethyl)-5a,10-dihydroxy-N-methyl-1,2,5,5a,6,7-hexahydro-6,11b-(epiminoethano)naphtho[1,2-d]azepine-3(4H)-carboxamide

[Chemical Formula 442]

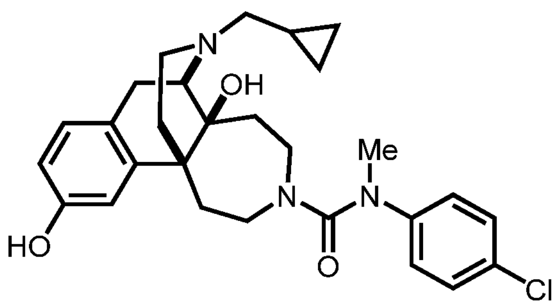

To the compound (29 mg, 0.060 mmol) obtained in Example 362 dissolved in tetrahydrofuran (2 mL) were added methyl iodide (15 μL, 0.24 mmol) and sodium hydride (55% oil dispersion) (11 mg, 0.24 mmol), followed by stirring at room temperature for 17 hours. To the reaction solution was added a saturated aqueous ammonium chloride solution,

followed by extraction three times with ethyl acetate. The organic layer was dried over sodium sulfate and concentrated under reduced pressure. The obtained crude product was purified by preparative thin layer chromatography (chloroform : 10% aqueous ammonia-methanol solution = 12 : 1) to yield the title compound (3.3 mg, 11%) as a colorless amorphous form.

$^1$H-NMR (400 MHz, $CDCl_3$)δ (ppm): 0.05 - 0.12 (m, 2H), 0.45 - 0.52 (m, 2H), 0.74 - 0.85 (m, 1H), 1.05 - 1.15 (m, 1H), 1.27 - 1.36 (m, 1H), 1.64 - 1.76 (m, 1H), 1.93 - 2.13 (m, 3H), 2.26 - 2.36 (m, 2H), 2.44 - 2.57 (m, 2H), 2.66 (dd, J = 6, 18 Hz, 1H), 2.77 - 2.83 (m, 1H), 2.89 - 3.00 (m, 2H), 3.09 (s, 3H), 3.21 - 3.33 (m, 2H), 3.51 - 3.63 (m, 1H), 4.46 (br s, 1H), 6.66 (dd, J = 2, 8 Hz, 1H), 6.79 - 6.88 (m, 3H), 6.94 (d, J = 8 Hz, 1H), 7.16 - 7.22 (m, 2H).

(Example 364)

Synthesis of 1-((5aS,6R,11bR)-14-(cyclopropylmethyl)-5a-hydroxy-10-methoxy-9-methyl-1,2,5,5a,6,7-hexahydro-6,11b-(epiminoethano)naphtho[1,2-d]azepin-3(4H)-yl)-2-(4-fluoro-1H-pyrazol-1-yl)ethan-1-one

[Chemical Formula 443]

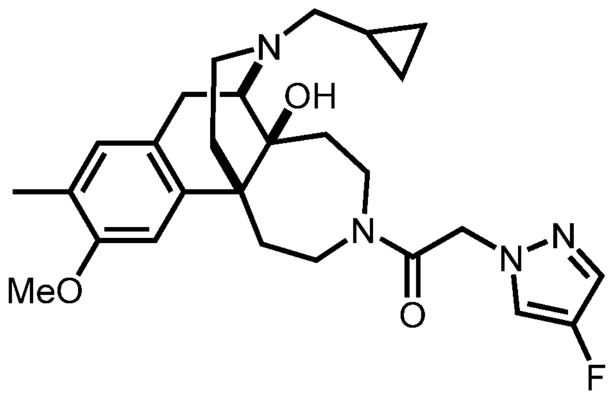

The title compound was obtained from the compound obtained in Example 261 and 4-fluoro-1H-pyrazole according to the method described in Example 132.

$^1$H-NMR (400 MHz, $CDCl_3$)δ (ppm): 0.05 - 0.18 (m, 2H), 0.45 - 0.59 (m, 2H), 0.76 - 0.90 (m, 1H), 1.03 - 1.12 (m, 1H), 1.55 - 1.65 (m, 1H), 1.80 - 1.91 (m, 1H), 1.92 - 2.09 (m, 2.4H), 2.10 - 2.29 (m, 1.2H), 2.17 (s, 1.8H), 2.17 (s, 1.2H), 2.30 - 2.40 (m, 2H), 2.49 - 2.61 (m, 1.4H), 2.71 (dd, J = 6, 18 Hz, 0.6H), 2.77 (dd, J = 6, 19 Hz, 0.4H), 2.88 (d, J = 6 Hz, 0.6H), 2.91 (d, J = 18 Hz, 0.4H), 2.91 (d, J = 6 Hz, 0.4H), 2.97 (d, J = 18 Hz, 0.6H), 3.16 (ddd, J = 2, 11, 14 Hz, 0.6H), 3.34 (ddd, J = 4, 4, 13 Hz, 0.6H), 3.46 - 3.55 (m, 0.8H), 3.64 (ddd, J = 4, 12, 14 Hz, 0.4H), 3.76 - 3.88 (m, 1.2H), 3.79 (s, 1.8H), 3.82 (s, 1.2H), 4.10 (ddd, J = 3, 6, 14 Hz, 0.4H), 4.52 (d, J = 16 Hz, 0.4H), 4.77 (d, J = 16 Hz, 0.4H), 4.86 (s, 1.2H), 6.51 (s, 0.4H), 6.55 (s, 0.6H), 6.87 (s, 0.6H), 6.87 (s, 0.4H), 7.16 (dd, J = 1, 4 Hz, 0.4H), 7.27 - 7.29 (m, 0.6H), 7.31 (dd, J = 1, 4 Hz, 0.4H), 7.32 - 7.34 (m, 0.6H).

(Example 365)

Synthesis of 1-((5aS,6R,11bR)-14-(cyclopropylmethyl)-5a, 10-dihydroxy-9-methyl-1,2,5,5a,6,7-hexahydro-6,11b-(epiminoethano)naphtho[1,2-d]azepin-3(4H)-yl)-2-(4-fluoro-1H-pyrazol-1-yl)ethan-1-one

[Chemical Formula 444]

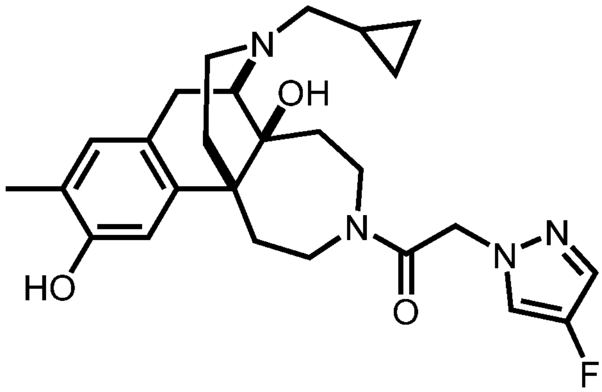

The title compound was obtained from the compound obtained in Example 364 according to the method described in Example 6.

$^1$H-NMR (400 MHz, $CDCl_3$)δ (ppm): 0.03 - 0.16 (m, 2H), 0.43 - 0.57 (m, 2H), 0.76 - 0.90 (m, 1H), 1.02 - 1.11 (m, 1H), 1.54 - 1.62 (m, 1H), 1.78 - 2.24 (m, 4.6H), 2.18 (s, 1.8H), 2.19 (s, 1.2H), 2.29 (dd, J = 6, 13 Hz, 0.4H), 2.30 - 2.39 (m, 1.2H), 2.39 (dd, J = 6, 12 Hz, 0.4H), 2.51 - 2.70 (m, 1.4H), 2.70 (dd, J = 6, 18 Hz, 0.6H), 2.74 (dd, J = 6, 19 Hz, 0.4H), 2.89 (d, J = 6 Hz, 0.6H), 2.90 (d, J = 19 Hz, 0.4H), 2.91 (d, J = 6 Hz, 0.4H), 2.97 (d, J = 18 Hz, 0.6H), 3.08 - 3.17 (m, 0.6H), 3.27 - 3.39 (m, 1.4H), 3.71 (ddd, J = 5, 12, 14 Hz, 0.4H), 3.79 - 3.89 (m, 1H), 4.08 (ddd, J = 2, 5, 14 Hz, 0.6H), 4.46 (br s, 0.4H), 4.52 (br s, 0.6H), 4.65 (d, J = 16 Hz, 0.4H), 4.67 (d, J = 16 Hz, 0.4H), 4.87 (d, J = 17 Hz, 0.6H), 4.87 (d, J = 17 Hz, 0.6H), 6.11 (br s, 0.4H), 6.26 (br s, 0.6H), 6.62 (s, 0.4H), 6.64 (s, 0.6H), 6.83 (s, 0.4H), 6.84 (s, 0.6H), 7.19 (d, J = 5 Hz, 0.4H), 7.30 (d, J = 5 Hz, 0.6H), 7.31 (d, J = 5 Hz, 0.4H), 7.34 (d, J = 5 Hz, 0.6H).

(Test Example 1)

Opioid receptor function test

The function activity of the compound of the present invention on κ opioid receptors was examined.

Method: Using a Lance Ultra cAMP kit (Perkin Elmer), the examination was performed according to a predetermined method. In evaluation of agonist activity, CHO cells expressing human κ opioid receptors (Catalog No. CT6606, accession No. NM_000912) and a test compound were reacted in an assay buffer (1 × HBSS, 5 mM HEPES, pH 7.4, 0.5 mM IBMX (isobutylmethylxanthine), 0.1% BSA) in the presence of 10 pM forskolin for 30 minutes. Subsequently, a cAMP detection reagent in the kit was added. One hour later, time-resolved fluorescence measurement was performed using an EnVision plate reader (Perkin Elmer). The evaluation of the test compound was performed in a concentration range of $10^{-14}$ to $10^{-7}$ M for the κ opioid receptor function test.

[Table 1]

| Examples | $EC_{50}$ value (nM) | Examples | $EC_{50}$ value (nM) |
|---|---|---|---|
| Example 3 | 0.83 | Example 84 | 0.47 |
| Example 22 | 0.13 | Example 88 | 0.75 |
| Example 27 | 0.13 | Example 90 | 0.87 |
| Example 31 | 0.11 | Example 100 | 0.26 |
| Example 32 | 0.13 | Example 102 | 0.13 |
| Example 33 | 0.92 | Example 105 | 0.088 |
| Example 34 | 0.040 | Example 108 | 0.066 |
| Example 36 | 0.025 | Example 112 | 0.037 |
| Example 37 | 0.051 | Example 113 | 0.56 |
| Example 41 | 0.13 | Example 114 | 0.043 |

(continued)

| Examples | $EC_{50}$ value (nM) | Examples | $EC_{50}$ value (nM) |
|---|---|---|---|
| Example 45 | 0.17 | Example 115 | 0.15 |
| Example 47 | 0.23 | Example 116 | 0.021 |
| Example 49 | 0.089 | Example 117 | 0.40 |
| Example 50 | 0.027 | Example 118 | 0.016 |
| Example 52 | 0.15 | Example 120 | 0.053 |
| Example 54 | 0.36 | Example 122 | 0.15 |
| Example 56 | 0.97 | Example 123 | 0.064 |
| Example 58 | 0.14 | Example 124 | 0.040 |
| Example 60 | 0.056 | Example 126 | 0.74 |
| Example 61 | 0.028 | Example 128 | 0.10 |
| Example 66 | 0.14 | Example 130 | 0.36 |
| Example 69 | 0.28 | Example 132 | 0.16 |
| Example 77 | 0.026 | Example 133 | 0.033 |
| Example 83 | 0.37 | Example 137 | 0.14 |

[Table 2]

| Examples | $EC_{50}$ value (nM) | Examples | $EC_{50}$ value (nM) |
|---|---|---|---|
| Example 143 | 0.033 | Example 225 | 0.11 |
| Example 144 | 0.019 | Example 226 | 0.017 |
| Example 149 | 0.023 | Example 227 (I) | 0.015 |
| Example 159 | 0.77 | Example 227 (II) | 0.16 |
| Example 160 | 0.045 | Example 228 | 0.010 |
| Example 162 | 0.39 | Example 229 | 0.025 |
| Example 163 | 0.076 | Example 230 | 0.066 |
| Example 164 | 0.26 | Example 232 | 0.17 |
| Example 165 | 0.28 | Example 234 | 0.087 |
| Example 166 | 0.31 | Example 236 | 0.012 |
| Example 168 | 0.047 | Example 237 | 0.29 |
| Example 169 | 0.094 | Example 238 | 0.45 |
| Example 177 | 0.019 | Example 244 | 0.47 |
| Example 179 | 0.024 | Example 246 | 2.6 |
| Example 181 | 0.32 | Example 262 | 0.085 |
| Example 182 | 0.22 | Example 263 | 0.89 |
| Example 183 | 0.030 | Example 264 | 0.29 |
| Example 184 | 0.095 | Example 265 | 0.12 |
| Example 185 | 0.39 | Example 266 | 0.074 |
| Example 186 | 0.99 | Example 270 | 0.052 |

(continued)

| Examples | $EC_{50}$ value (nM) | Examples | $EC_{50}$ value (nM) |
|---|---|---|---|
| Example 188 | 0.69 | Example 274 | 0.070 |
| Example 189 | 0.39 | Example 281 | 0.14 |
| Example 190 | 0.32 | Example 291 | 0.55 |
| Example 191 | 0.48 | Example 308 | 0.60 |
| Example 193 | 0.035 | Example 314 | 0.27 |
| Example 195 | 0.023 | Example 323 | 0.18 |
| Example 196 | 0.22 | Example 326 | 0.18 |
| Example 198 | 0.59 | Example 328 | 0.33 |
| Example 209 | 0.050 | Example 329 | 0.084 |
| Example 210 | 0.72 | Example 332 | 0.64 |
| Example 214 | 0.13 | Example 336 | 0.18 |
| Example 217 | 0.18 | Example 343 | 0.56 |
| Example 218 | 0.16 | Example 347 | 0.12 |
| Example 219 | 0.23 | Example 355 | 0.11 |
| Example 220 | 0.26 | Example 357 | 0.062 |
| Example 221 | 0.034 | | |

As shown in Table 1 and Table 2, it has been confirmed that the compound of the present invention exhibits potent agonist activity on κ opioid receptors.

(Test Example 2)

Metabolic stability test

To determine the unchanged product-residual ratio of the test compound in the reaction sample, human liver microsomes and the test compound were reacted for a predetermined time (0 to 30 minutes). The unchanged product-residual ratio at a reaction time of 0 hours was considered 100%, the residual ratio after incubation was log-linear plotted with respect to the time, a regression line (y = 100 $e^{-kt}$, k = slope of the line: elimination rate constant) was determined, and the metabolic clearance $CL_{int}$ (mL/min/kg) was calculated using the following formula.

$$CL_{int}^{*} = k\ (\text{- min}) \times 45\ (\text{mg MS protein/g liver}) \times 21\ (\text{g liver/kg})/\text{MS protein (mg MS protein/mL)}$$

*: Yamazaki S.; Skaptason J.; Romero D, Vekich S.;Jones HM.; Tan W.; Wilner KD.; Koudriakova T. Drug Metab. Dispos. 2011 Mar;39 (3): 383 - 393.

[Table 3]

| Test compound[1]) | $CL_{int}$(mL/min/kg) |
|---|---|
| Example 20 | 3.9 |
| Example 22 | 7.2 |
| Example 31 | 7.5 |
| Example 45 | 4.5 |
| Example 50 | 8.7 |

(continued)

| Test compound[1]) | $CL_{int}$(mL/min/kg) |
|---|---|
| Example 61 | 26.0 |
| Example 77 | 6.7 |
| Example 90 | 11.6 |
| Example 124 | 14.3 |
| Example 139 | 29.7 |
| Example 176 | 8.2 |
| Example 177 | 9.5 |
| Example 229 | 5.1 |
| Example 244 | 7.7 |
| Example 247 | 4.8 |
| Example 262 | 5.8 |
| Example 263 | 18.0 |
| Example 266 | 4.4 |
| Example 277 | 6.4 |
| Example 281 | 13.7 |
| Example 291 | 8.0 |
| Example 295 | 17.2 |
| Example 298 | 22.5 |
| Example 308 | 9.0 |
| Example 320 | 22.9 |
| Example 322 | 6.6 |
| Example 326 | 10.9 |
| Example 332 | 9.2 |
| Example 353 | 7.9 |
| [1])A hydrochloride was used. | |

As shown in Table 3, the compound of the present invention exhibits excellent metabolic stability.

(Test Example 3)

Mouse acetic acid writhing test

The analgesic effect of the test compound was evaluated by an acetic acid writhing test. For the experiment, ICR male mice were used and acclimated to a plastic open field for 30 minutes before starting the test. After acclimation, the mice were administered with the test compound (30 to 3000 μg/kg) or water for injection subcutaneously or orally, and once returned to the open field. Thirty minutes after the administration of the test compound, a 0.6% aqueous acetic acid solution was additionally administered to the same mice by intraperitoneal administration. From 10 minutes after administration of the 0.6% aqueous acetic acid solution, the number of writhing reactions (writhing reactions in which the mouse stretches while pressing the abdominal cavity against the floor) induced in the mice was measured for 10 minutes, and the number of writhing reactions was compared with that of a control group (water for injection-administrated group) to evaluate the analgesic effect of the test compound. The results are shown in Table 4.

[Table 4]

| Test compound[1)] | Writhing suppressing effect | | | |
|---|---|---|---|---|
| | Administration route | Dose | Writhing suppression rate[2)] | $ED_{50}$ value (μg/kg) |
| Example 22 | Oral | 30 | + | 15.2 |
| | | 300 | ++ | |
| Example 34 | Subcutaneous | 30 | ++ | N.C.[3)] |
| Example 45 | Subcutaneous | 30 | ++ | N.C.[3)] |
| Example 50 | Oral | 300 | + | 210 |
| | | 1000 | +++ | |
| Example 77 | Subcutaneous | 30 | + | 12.5 |
| | | 300 | +++ | |
| Example 308 | Oral | 1000 | + | 1420 |
| | | 3000 | ++ | |

1)A hydrochloride was used.
2)The writhing suppressing effect was classified as follows according to the suppression rate.
40 ≤ % < 60 : +, 60 ≤ % < 80 : ++, 80 ≤ % : +++
3)N.C.: not calculated

As shown in Table 4, it has been confirmed that the compound of the present invention exhibits a potent analgesic effect.

(Test Example 4)

Rotarod test

The sedative effect (motor coordination disorder effect) of the test compound was evaluated by a rotarod test. For the experiment, ICR male mice that had learned a motion task in advance by rotarod training were used. Rotarod training was repeated while providing a moderate rest period until the mice acclimatized for 60 seconds on a rotating shaft rod rotating at 3 rpm (diameter 3 cm, KN-75, Natsume Seisakusho Co.,Ltd.) and acquired motor learning. Thereafter, a total of three training units of 3 rpm for 180 seconds, 4 rpm for 180 seconds, and 5 rpm for 180 seconds were provided. After all training, a 90 to 120 minute rest was provided to reduce the burden on the mice. A mouse that failed to acquire motor learning by training was not used for the experiment.

In the rotarod test, the number of times of falling at 5 rpm for 300 seconds (cut-off value 300 seconds) was measured as a pre-value before administration of the test compound. After the measurement of the pre-value, the mice were treated with the test compound (3 to 500 μg/kg) or water for injection by subcutaneous administration (s.c.) or oral administration (p.o.), and the number of times of falling at 5 rpm for 300 seconds was measured at each of four points of 30 minutes, 60 minutes, 90 minutes, and 120 minutes after administration of the test compound, in the same manner as measurement of the pre-value. The total number of times of falling at the four points was defined as the total number of times of falling (total falls), and the sedative effect of the test compound was evaluated. The results are shown in Figs. 1 to 5. As the test compound, a hydrochloride was used. In Figs. 1 to 5, Mean ± SEM ns; not significant (Welch's t test), Mean ± SEM *; $p < 0.05$, ***; $p < 0.001$ (Bonferroni's test).

As shown in Figs. 1 to 5, it has been confirmed that the therapeutic range is wide, since the compound of the present invention does not exhibit a sedative effect even at a dose higher than that of the comparative compound Nalfurafine, and the dose is 30 times or more higher than the $ED_{50}$ value (Test Example 3) in the mouse acetic acid writhing test.

Although preferred examples of the present invention have been described above, the present invention is not limited to these examples. Addition, omission, substitution and other changes of the configuration can be made without departing from the spirit of the present invention. The present invention is not limited by the foregoing description, but only by the scope of the appended claims.

## Claims

**1.** An azepane derivative represented by a following formula (I):

[Chemical Formula 1]

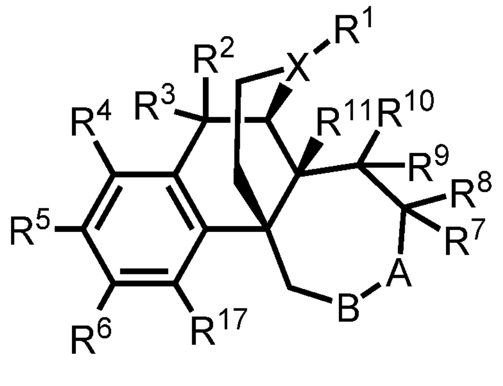

(I)

wherein $R^1$ represents a hydrogen atom, a $C_{1-6}$ alkyl group optionally having a substituent, a $C_{3-6}$ cycloalkyl group optionally having a substituent, a $C_{3-6}$ cycloalkyl $C_{1-6}$ alkyl group optionally having a substituent, a $C_{6-10}$ aryl group optionally having a substituent, a heteroaryl group optionally having a substituent, an aralkyl group optionally having a substituent, a heteroarylalkyl group optionally having a substituent, a $C_{2-6}$ alkenyl group optionally having a substituent, a $C_{2-6}$ alkynyl group optionally having a substituent, an acyl group optionally having a substituent, or an amino protecting group,

$R^2$ and $R^3$ are the same or different and each represent a hydrogen atom, a $C_{1-6}$ alkyl group optionally having a substituent, a $C_{1-6}$ alkoxy group optionally having a substituent, a halogen atom, an optionally protected hydroxy group, a carbonyl group in which $R^2$ and $R^3$ are together with each other, or a $C_{3-6}$ saturated hydrocarbon ring optionally having a substituent or a cyclic ketal optionally having a substituent in which $R^2$ and $R^3$ are bonded to each other,

$R^4$ and $R^5$ are the same or different and each represent a hydrogen atom, a $C_{1-6}$ alkyl group optionally having a substituent, a $C_{1-6}$ alkoxy group optionally having a substituent, a $C_{3-6}$ cycloalkyl group optionally having a substituent, an amino group optionally having a substituent, a protected amino group, a halogen atom, an optionally protected hydroxy group, a carboxy group, a carboxylic acid ester group, or a carbamoyl group optionally having a substituent,

$R^6$ represents a hydrogen atom, a $C_{1-6}$ alkoxy group optionally having a substituent, an amino group optionally having a substituent, a protected amino group, a halogen atom, an optionally protected hydroxy group, a tetrazolyloxy group optionally having a substituent, a cyano group, a carboxy group, a carboxylic acid ester group, a carbamoyl group optionally having a substituent, a $C_{6-10}$ aryl group optionally having a substituent, a heteroaryl group optionally having a substituent, a $C_{6-10}$ aryloxy group, or a saccharide,

$R^7$ and $R^8$ are the same or different and each represent a hydrogen atom, a $C_{1-6}$ alkyl group optionally having a substituent, a $C_{1-6}$ alkoxy group optionally having a substituent, a halogen atom, an optionally protected hydroxy group, a carbonyl group or a thiocarbonyl group in which $R^7$ and $R^8$ are together with each other, or a $C_{3-6}$ saturated hydrocarbon ring optionally having a substituent or a cyclic ketal optionally having a substituent in which $R^7$ and $R^8$ are bonded to each other,

$R^9$ and $R^{10}$ are the same or different and each represent a hydrogen atom, a $C_{1-6}$ alkyl group optionally having a substituent, a $C_{1-6}$ alkoxy group optionally having a substituent, a halogen atom, an optionally protected hydroxy group, a carbonyl group or a thiocarbonyl group in which $R^9$ and $R^{10}$ are together with each other, or a $C_{3-6}$ saturated hydrocarbon ring optionally having a substituent or a cyclic ketal optionally having a substituent in which $R^9$ and $R^{10}$ are bonded to each other,

$R^{11}$ represents a hydrogen atom, a $C_{1-6}$ alkyl group optionally having a substituent, a $C_{1-6}$ alkoxy group optionally having a substituent, an aralkyloxy group optionally having a substituent, an amino group optionally having a substituent, a protected amino group, a halogen atom, an optionally protected hydroxy group, or a cyano group,

A and B are different from each other and each represent $NR^{18}$ ($R^{18}$ represents a hydrogen atom or an amino protecting group), a methylene group, a carbonyl group, or a group represented by a following formula (II):

[Chemical Formula 2]

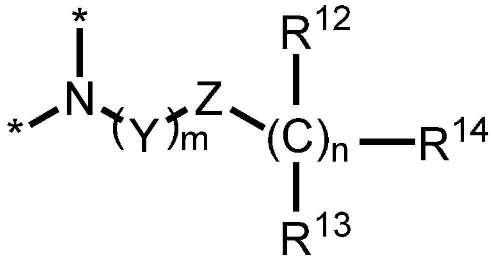

(II)

wherein $R^{12}$ and $R^{13}$ are the same or different and each represent a hydrogen atom, a halogen atom or a $C_{1-6}$ alkyl group optionally having a substituent, or a $C_{3-6}$ saturated hydrocarbon ring optionally having a substituent or a saturated heterocyclic ring optionally having a substituent in which $R^{12}$ and $R^{13}$ on a same carbon are bonded to each other, or may form a $C_{3-6}$ saturated hydrocarbon ring optionally having a substituent or a saturated heterocyclic ring optionally having a substituent in which a pair of adjacent $R^{12}$s are bonded to each other when n is 2 to 3,
$R^{14}$ represents a hydrogen atom, a $C_{1-6}$ alkyl group optionally having a substituent, a $C_{3-6}$ cycloalkyl group optionally having a substituent, a $C_{2-6}$ alkenyl group optionally having a substituent, a $C_{2-6}$ alkynyl group optionally having a substituent, a $C_{1-6}$ alkoxy group optionally having a substituent, an amino group optionally having a substituent, a protected amino group, a halogen atom, an optionally protected hydroxy group, a $C_{6-10}$ aryl group optionally having a substituent, a heteroaryl group optionally having a substituent, a saturated heterocyclic group optionally having a substituent, or a cyclic amino group optionally having a substituent, and * represents a bond, provided that one of A or B represents the formula (II),
X represents a nitrogen atom or an N-oxide,
Y represents a methylene group optionally having a substituent, a carbonyl group, or a thiocarbonyl group,
Z represents $NR^{15}$, an oxygen atom, a bond, an ethenylene group optionally having a substituent, or an ethynylene group, provided that when m is 0, n does not represent 0 and Z does not represent $NR^{15}$,
$R^{15}$ represents a hydrogen atom, a $C_{1-6}$ alkyl group optionally having a substituent, or a nitrogen-containing saturated heterocyclic ring optionally having a substituent in which $R^{15}$ and $R^{14}$ are bonded to each other,
$R^{17}$ represents a hydrogen atom, a halogen atom, an optionally protected hydroxy group, an alkoxy group optionally having a substituent, or a tetrazolyloxy group optionally having a substituent,
m represents an integer of 0 to 1, and
n represents an integer of 0 to 3,
provided that when Z is a bond, m and n do not simultaneously represent 0,
and a pharmaceutically acceptable salt thereof.

2. The azepane derivative and the pharmaceutically acceptable salt thereof according to claim 1, wherein $R^6$ is a hydroxy group, a $C_{1-6}$ alkoxy group optionally having a substituent, or a carbamoyl group optionally having a substituent.

3. The azepane derivative and the pharmaceutically acceptable salt thereof according to claim 1 or 2, wherein $R^6$ is a hydroxy group.

4. The azepane derivative and the pharmaceutically acceptable salt thereof according to any one of claims 1 to 3, wherein $R^1$ is a $C_{1-6}$ alkyl group optionally having a substituent or a $C_{3-6}$ cycloalkyl $C_{1-6}$ alkyl group optionally having a substituent.

5. The azepane derivative and the pharmaceutically acceptable salt thereof according to any one of claims 1 to 4, wherein Z is a bond.

6. The azepane derivative and the pharmaceutically acceptable salt thereof according to any one of claims 1 to 5, wherein n is 1 to 3.

7. The azepane derivative and the pharmaceutically acceptable salt thereof according to any one of claims 1 to 6, wherein $R^{14}$ is a heteroaryl group optionally having a substituent.

**8.** The azepane derivative and the pharmaceutically acceptable salt thereof according to any one of claims 1 to 7, wherein A represents a group represented by the formula (II), and B represents a methylene group.

**9.** The azepane derivative and the pharmaceutically acceptable salt thereof according to any one of claims 1 to 8, wherein $R^{11}$ is a hydroxy group.

**10.** A pharmaceutical composition comprising the azepane derivative and the pharmaceutically acceptable salt thereof according to any one of claims 1 to 9 as an active ingredient.

**11.** The pharmaceutical composition according to claim 10, being a treating, improving, or preventing agent for a disease related to a κ opioid receptor.

**12.** The pharmaceutical composition according to claim 10 or 11, being an analgesic.

**13.** The pharmaceutical composition according to claim 10 or 11, being an antipruritic drug.

[FIG. 1]

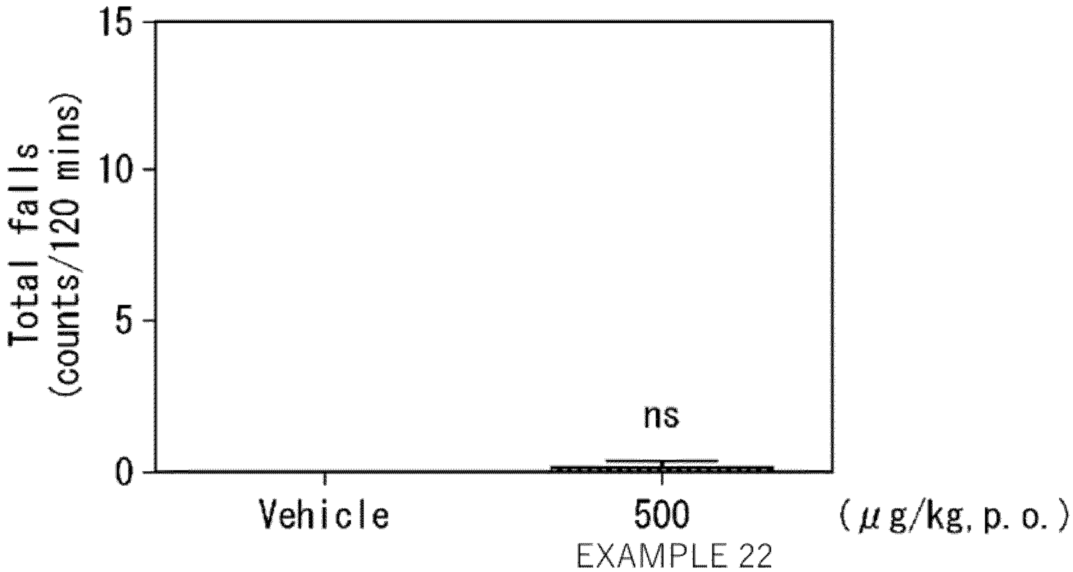
15
10
5
0
Total falls
(counts/120 mins)
ns
Vehicle
500
(μg/kg, p. o.)
EXAMPLE 22

[FIG. 2]

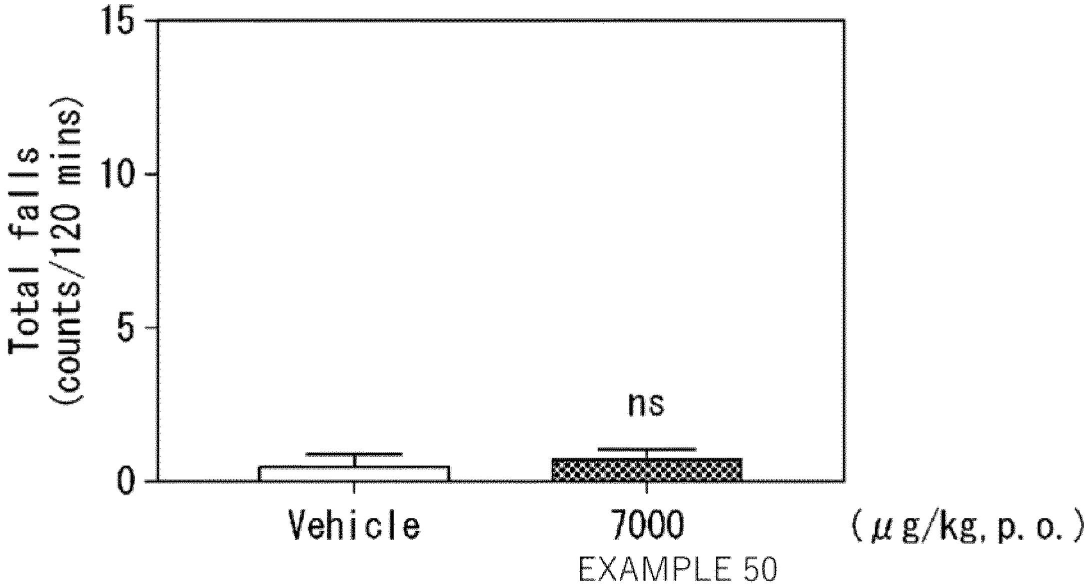
15
10
5
0
Total falls
(counts/120 mins)
ns
Vehicle
7000
(μg/kg, p. o.)
EXAMPLE 50

[FIG. 3]

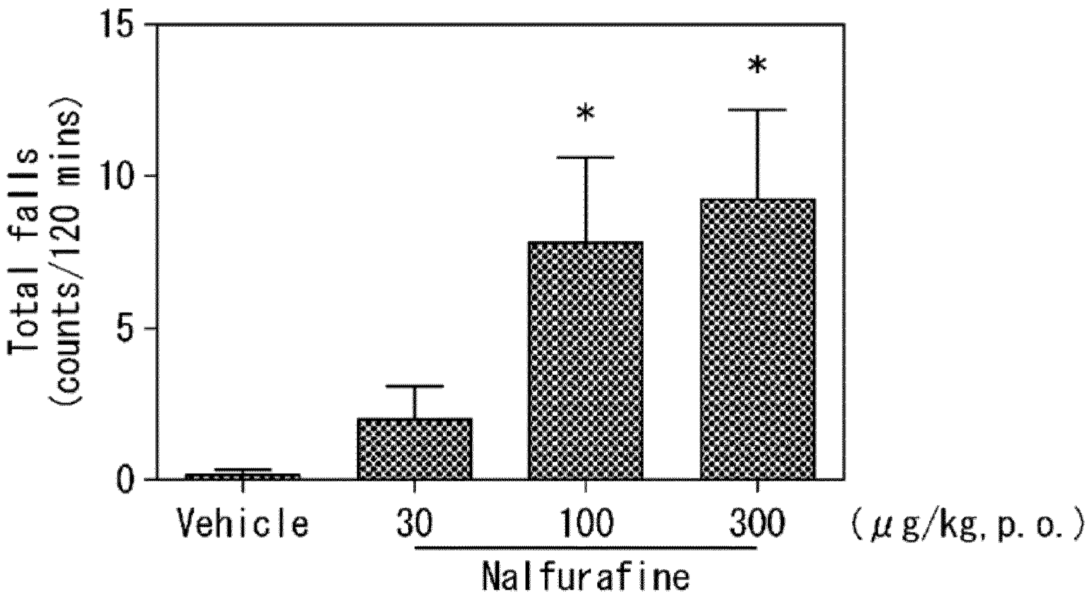
15
10
5
0
Total falls
(counts/120 mins)
*
*
Vehicle
30
100
300
(μg/kg, p. o.)
Nalfurafine

[FIG. 4]

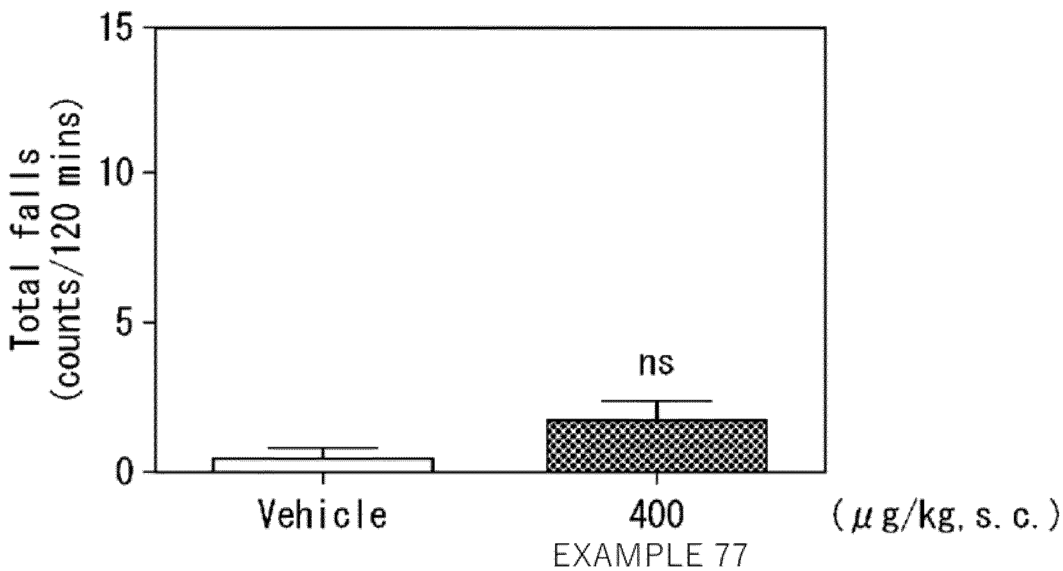
15
10
5
0
Total falls
(counts/120 mins)
ns
Vehicle
400
(μg/kg, s. c.)
EXAMPLE 77

[FIG. 5]

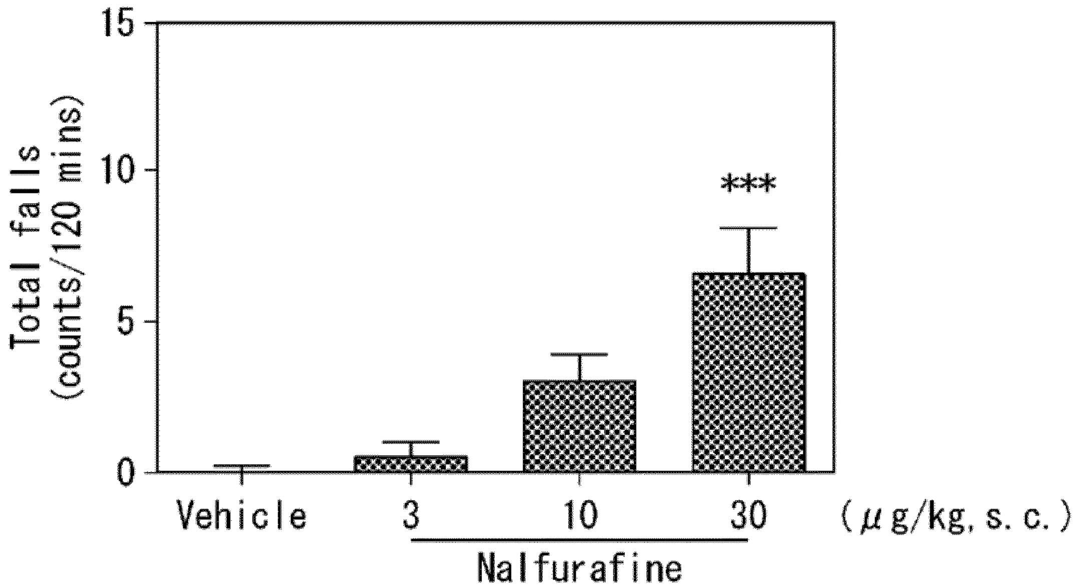
15
10
5
0
Total falls
(counts/120 mins)
***
Vehicle
3
10
30
(μg/kg, s. c.)
Nalfurafine

**INTERNATIONAL SEARCH REPORT**

International application No. PCT/JP2020/036868

A. CLASSIFICATION OF SUBJECT MATTER

A61P 25/04(2006.01)i; A61P 43/00(2006.01)i; C07D 471/08(2006.01)i; A61P 17/04(2006.01)i; C07F 7/10(2006.01)i; C07D 519/00(2006.01)i; A61K 31/55(2006.01)i; A61K 31/695(2006.01)i

FI: C07D471/08 CSP; A61P25/04; A61P17/04; A61K31/55; C07D519/00 311; A61K31/695; C07F7/10 S; A61P43/00 111

According to International Patent Classification (IPC) or to both national classification and IPC

B. FIELDS SEARCHED

Minimum documentation searched (classification system followed by classification symbols)

A61P25/04; A61P43/00; C07D471/08; A61P17/04; C07F7/10; C07D519/00; A61K31/55; A61K31/695

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| P, A | WO 2020/085234 A1 (NIPPON CHEMIPHAR CO., LTD.) 30 April 2020 (2020-04-30) claims | 1-13 |
| A | JP 2015-500245 A (MALLINCKRODT, LLC.) 05 January 2015 (2015-01-05) claims | 1-13 |
| A | SUZUKI, Shinya et al., "Discovery of highly selective kappa-opioid receptor agnists: 10 alpha-hydroxy TRK-820 derivatives", Bioorganic & Medicinal Chemistry Letters, 2017, 27, 3920-3924 TRK-820 compounds, fig. 1, 2 | 1-13 |
| A | WO 2008/143263 A1 (ASTELLAS PHARMA INC.) 27 November 2008 (2008-11-27) claims | 1-13 |
| A | JP 2010-511717 A (NEUROGESX, INC.) 15 April 2010 (2010-04-15) claims | 1-13 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search: 11 November 2020 (11.11.2020)

Date of mailing of the international search report: 08 December 2020 (08.12.2020)

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**

International application No.
PCT/JP2020/036868

C (Continuation). DOCUMENTS CONSIDERED TO BE RELEVANT

| Category* | Citation of document, with indication, where appropriate, of the relevant passages | Relevant to claim No. |
|---|---|---|
| A | ISHIKAWA, Kyoko et al, "C-homomorphinan derivatives as lead compounds to obtain safer and more clinically useful analgesics", Chem. Pharm. Bull., 2017, 65, 920-929 fig. 1 intermediate compound | 1-13 |

Form PCT/ISA/210 (continuation of second sheet) (January 2015)

**INTERNATIONAL SEARCH REPORT**
Information on patent family members

International application No.
PCT/JP2020/036868

| Patent Documents referred in the Report | Publication Date | Patent Family | Publication Date |
|---|---|---|---|
| WO 2020/085234 A1 | 30 Apr. 2020 | (Family: none) | |
| JP 2015-500245 A | 05 Jan. 2015 | US 2013/0144053 A1 claims<br>WO 2013/085937 A1<br>CA 2856727 A<br>AU 2012348029 A<br>MX 2014006268 A<br>KR 10-2014-0099525 A<br>CN 104039795 A<br>EP 2788360 A1<br>RU 2014127179 A<br>BR 112014013515 A | |
| WO 2008/143263 A1 | 27 Nov. 2008 | US 2010/0168154 A1 claims<br>AU 2008254061 A<br>TW 200914015 A<br>MX 2009012610 A<br>EP 2149560 A1<br>KR 10-2010-0014570 A<br>CN 101679273 A<br>RU 2009147451 A<br>IL 201987 A<br>CA 002687958 A | |
| JP 2010-511717 A | 15 Apr. 2010 | WO 2008/070149 A2 claims<br>CA 2671737 A<br>AU 2007328007 A<br>US 2008/0318905 A1<br>MX 2009006007 A<br>KR 10-2009-0086627 A<br>EP 2121029 A1<br>CN 101678120 A<br>RU 2009125597 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019179727 A
- WO 19935081 A
- JP 2008179554 A
- JP 2525552 B
- US 963460 A
- WO 2010105179 A
- WO 2017223239 A
- WO 2010021149 A
- WO 9905095 A
- WO 2011045383 A
- WO 2018148576 A
- WO 2016040515 A
- WO 2018160878 A
- WO 2015097545 A
- WO 2007092681 A


### Non-patent literature cited in the description


- Symposium Program and Abstract of 36th Annual Meeting of Japanese Narcotics Research Conference (JNRC 2016). *Japanese Narcotics Research Conference,* 37
- *Tetrahedron Lett.,* 2010, vol. 51, 2359
- *Bioorganic Medicinal Chemistry,* 2012, vol. 20, 949
- *Journal of Medicinal Chemistry,* 2018, vol. 61, 8797
- *Journal of Medicinal Chemistry,* 1996, vol. 39, 2615
- *Tetrahedron Letters,* 2007, vol. 48, 7413
- *Organic Letters,* 2009, vol. 11, 539
- *Journal of Medicinal Chemistry,* 2004, vol. 47, 1070
- **YAMAZAKI S. ; SKAPTASON J. ; ROMERO D ; VEKICH S. ; JONES HM. ; TAN W. ; WILNER KD. ; KOUDRIAKOVA T.** *Drug Metab. Dispos.,* March 2011, vol. 39 (3), 383-393